# EUROPEAN PATENT SPECIFICATION

(11) **EP 3 311 172 B1**
(45) Date of publication and mention of the grant of the patent: **19.05.2021**
(21) Application number: 16736395.1
(22) Date of filing: 17.06.2016
(51) Int. Cl.: G01N 33/68

(54) **METHODS FOR IDENTIFYING COMPOUNDS THAT DIRECTLY BIND TO INSECT TRANSIENT RECEPTOR POTENTIAL CHANNELS**
VERFAHREN ZUR IDENTIFIZIERUNG VON VERBINDUNGEN, DIE DIREKT AN ÜBERGANGSREZEPTORPOTENZIALKANÄLEN VON INSEKTEN BINDEN
PROCÉDÉS D'IDENTIFICATION DE COMPOSÉS SE LIANT DIRECTEMENT À DES CANAUX À POTENTIEL DE RÉCEPTEUR TRANSITOIRE D'INSECTES

(30) Priority: 19.06.2015 US 201562182037 P
(43) Date of publication of application: 25.04.2018
(73) Proprietor: BASF SE, 67056 Ludwigshafen am Rhein (DE)
(72) Inventor: VON DEYN, Wolfgang, 67435 Neustadt (DE); NESTEROV, Alexandre, Chapel Hill, NC 27514 (US); KANDASAMY, Ramani, Chapel Hill, NC 27516 (US); LONDON, Damian, Wake Forest, NC 27587 (US); SALGADO, Vincent L., Durham, NC 27703 (US); LELITO, Katherine, Research Triangle Park, NC 27709 (US)
(74) Representative: BASF IP Association
(86) International application number: PCT/EP2016/064078
(87) International publication number: WO 2016/203014

(56) References cited:
- WO-A2-2015/097560
- CN-A- 103 898 122
- US-A1- 2009 209 633
- CHERYL A. LEICHTER ET AL: "The high potency of ME-5343 to aphids is due to a unique mechanism of action", PESTICIDE BIOCHEMISTRY AND PHYSIOLOGY, vol. 107, no. 2, 1 October 2013 (2013-10-01), pages 169-176, XP055218037, ISSN: 0048-3575, DOI: 10.1016/j.pestbp.2013.06.009
- ALEXANDRE NESTEROV ET AL: "TRP Channels in Insect Stretch Receptors as Insecticide Targets", NEURON, vol. 86, no. 3, 1 May 2015 (2015-05-01), pages 665-671, XP055201614, ISSN: 0896-6273, DOI: 10.1016/j.neuron.2015.04.001
- KEIICHI NAGATA: "TRP channels as target sites for insecticides: physiology, pharmacology and toxicology", INVERTEBRATE NEUROSCIENCE, SPRINGER, BERLIN, DE, vol. 7, no. 1, 7 February 2007 (2007-02-07), pages 31-37, XP019476061, ISSN: 1439-1104, DOI: 10.1007/S10158-007-0044-4
- G. R. SEABROOK: "Functional Properties of the High-Affinity TRPV1 (VR1) Vanilloid Receptor Antagonist (4-Hydroxy-5-iodo-3-methoxyphenylacetate ester) Iodo-Resiniferatoxin", JOURNAL OF PHARMACOLOGY AND EXPERIMENTAL THERAPEUTICS, vol. 303, no. 3, 1 December 2002 (2002-12-01), pages 1052-1060, XP055305053, US ISSN: 0022-3565, DOI: 10.1124/jpet.102.040394
- E. PHILLIPS ET AL: "Identification of Species-specific Determinants of the Action of the Antagonist Capsazepine and the Agonist PPAHV on TRPV1", JOURNAL OF BIOLOGICAL CHEMISTRY, vol. 279, no. 17, 23 April 2004 (2004-04-23), pages 17165-17172, XP055305059, US ISSN: 0021-9258, DOI: 10.1074/jbc.M313328200
- Janghwan Kim ET AL: "A TRPV family ion channel required for hearing in Drosophila", Nature, vol. 424, no. 6944, 3 July 2003 (2003-07-03), pages 81-84, XP055538237, London ISSN: 0028-0836, DOI: 10.1038/nature01733

### SEQUENCE LISTING

<110> BASF SE
<120> METHODS FOR IDENTIFYING COMPOUNDS THAT DIRECTLY BIND TO INSECT TRANSIENT RECEPTOR POTENTIAL CHANNELS
<130> 0000078630WO01
<150> US 62/182037
   <151> 2015-06-19
<160> 112
<170> PatentIn version 3.5
<210> 1
   <211> 2502
   <212> DNA
   <213> Drosophila melanogaster
<400> 1
   atggggaata cggagagcaa tgttaccagc ggggtgaaga agcaggccgg agtctccacc      60
   caggctctat acaagttcgt gaatctcaag ggcggtggac tcctggtgga catgatgaaa     120
   agagcctgtc aaaccaaaca gtttgctgag atcgatcatg ctatcaaaac caaagtggag     180
   ccctttctgt acaacaaggg cgctggacgg tattttccca tctcgaagct cgtactgctg     240
   cgaaatcggg atcgcccacg cactcgccaa ttgcccgaga tccgagccct cgagaatcct     300
   gacgacgatt tcaacatcca tgactactgc cccgaggttt cggaggccga gtatatctcc     360
   aatcctacgg cctatcgatt cgtctgctgg gatcttaata tgcgtggcgc tgtgggcgaa     420
   actattctcc atttgtgcct gctaaatgca tcttccttgc acgcagatct cgccaaaaga     480
   ttgctaaaat tctacccgaa actcatcctg gacatttaca tgagcgacga gtattatgga     540
   gagagtgtcc tgcacattgc catcgtcaat gaagatccgg caatggtgaa gtatctgctg     600
   gatgccaacg cagatgttca agagcggtgc tgtggagcct tcatgtcggc ggaggatact     660
   aagttttctc gcacggattc gccggatcac gagtatgtgg cactttgtcc catgaccaac     720
   tacgatggtt atgtttactg gggagaatac ccattgagtt ttgccgcttg cctgtcgcag     780
   gaggagtgct tccgcttagt tttggccaga ggtgccgatc ccgacttcca ggacaccaac     840
   ggaaacaccg ttctccacat gctggtcatt tacgaaaaga tcgaaatgtt cgatgttggc     900
   tacgaggttg gaacaaacat acacataaaa aacattcaga atctcacgcc ccttactttg     960
   gcggccaagt tgggcagagt tgagatgttc ttccacgtga tgagcatcga acgggagatc    1020
   tattggcaat tgggtagcat cacatgtgcc gcatatccgc ttttgatgat agacactata    1080
   aatgaggaaa ccggcaatat caacaaggac tccgtgctga actttgtggt atttggcgat    1140
   aagctggagc acttggagct gctagatggg gtggtcatag atctgctaaa gaccaaatgg    1200
   gacacgttct gcaaatcgcg attttacaag caattttaca tgttcgccct ctactttctc 1260
   atctcgctgt tcagttttat actgcgtcca ggtccggatg ccaaggacga agacgaggat 1320
   ggcgccaata gcactacggc taaaagtgat ctgtataggc aaaatggatc ggattcttac 1380
   catctgcaca gcaagcgggc tacaatgact accgaataca aaacattttg gcttaatttt 1440
   actgagtact acgatccatc ggaagttgag gtcctgcccg cctggtggga aagttatgct 1500
   cagtgtcctt tgatgaatct cgaatccgat ttggccaagc ttcgtattat ggcagaactg 1560
   ttgaattttg taggagctat cctgtatctg ctggttgctt tgcgagaagc tcgattcctg 1620
   ggccttaaaa tgtttatcga aaacttgatg acggcacctt cgcgagttat gtttctgttt 1680
   tcgtgtgctt tgatgatgac tataccctgg ttgagggttt cctgtctcac cgaaatagat 1740
   gatcacgtca ccgttgtgat aatgctaacc actgctccct attttctatt cttttgtcgc 1800
   ggcttcaaaa ctgtcggtcc cttcgtagtg atgatatacc gcatggtcat gggagatctg 1860
   ctccgattcg tctccatcta cttggtgttc gtcatgggtt tctcccaggc attttacata 1920
   atttttctaa ccttcgacaa cccatcgtca ccagaggatc aggacgcgga atccaaccca 1980
   atgccctcgc ccatggaatc gatagtggcc atgttcctaa tgtcgctaac aaatttcggt 2040
   gactactatg gagcgatggt gtctacgcag cacgaatacg aggcgaagat cctcttcttt 2100
   ctgttcatgg tgatcgtgag tgttctgctg gtgaacatgt tgatcgccat gatgggtaat 2160
   acgtatcaga agatcgccga gatccgaaac gagtggcagc gccaatgggc caggatcgtc 2220
   ctcgtcgtgg agcgaagtgt tcctcccgcc gagcggttga agaactttat gcagtacagt 2280
   cagtcgatgt cggatggcag aagggcactg gtcctccggt tgaatatgac tgaggaaaag 2340
   gaggagatga aggaggtgca ggagatgaag cgcatccatc aacggttcgc caagaagcgg 2400
   caaatggagc gcgaggctcg cgcccttcga cgtcaacagg agtacgagaa gttcttcggc 2460
   accgccccca aatcggaatg ttccgataac aacaactttt ga 2502
<210> 2
   <211> 2505
   <212> DNA
   <213> Drosophila melanogaster
<400> 2
   atggggaata cggagagcaa tgttaccagc ggggtgaaga agcaggccgg agtctccacc      60
   caggctctat acaagttcgt gaatctcaag ggcggtggac tcctggtgga catgatgaaa     120
   agagcctgtc aaaccaaaca gtttgctgag atcgatcatg ctatcaaaac caaagtggag     180
   ccctttctgt acaacaaggg cgctggacgg tattttccca tctcgaagct cgtactgctg     240
   cgaaatcggg atcgcccacg cactcgccaa ttgcccgaga tccgagccct cgagaatcct     300
   gacgacgatt tcaacatcca tgactactgc cccgaggttt cggaggccga gtatatctcc     360
   aatcctacgg cctatcgatt cgtctgctgg gatcttaata tgcgtggcgc tgtgggcgaa 420
   actattctcc atttgtgcct gctaaatgca tcttccttgc acgcagatct cgccaaaaga 480
   ttgctaaaat tctacccgaa actcatcctg gacatttaca tgagcgacga gtattatgga 540
   gagagtgtcc tgcacattgc catcgtcaat gaagatccgg caatggtgaa gtatctgctg 600
   gatgccaacg cagatgttca agagcggtgc tgtggagcct tcatgtcggc ggaggatact 660
   aagttttctc gcacggattc gccggatcac gagtatgtgg cactttgtcc catgaccaac 720
   tacgatggtt atgtttactg ggagaatacc ccattgagtt ttgccgcttg cctgtcgcag 780
   gaggagtgct tccgcttagt tttggccaga ggtgccgatc ccgacttcca ggacaccaac 840
   ggaaacaccg ttctccacat gctggtcatt tacgaaaaga tcgaaatgtt cgatgttggc 900
   tacgaggttg gaacaaacat acacataaaa aacattcaga atctcacgcc ccttactttg 960
   gcggccaagt tgggcagagt tgagatgttc ttccacgtga tgagcatcga acgggagatc 1020
   tattggcaat tgggtagcat cacatgtgcc gcatatccgc ttttgatgat agacactata 1080
   aatgaggaaa ccggcaatat caacaaggac tccgtgctga actttgtggt atttggcgat 1140
   aagctggagc acttggagct gctagatggg gtggtcatag atctgctaaa gaccaaatgg 1200
   gacacgttct gcaaatcgcg attttacaag caattttaca tgttcgccct ctactttctc 1260
   atctcgctgt tcagttttat actgcgtcca ggtccggatg ccaaggacga agacgaggat 1320
   ggcgccaata gcactacggc taaaagtgat ctgtataggc aaaatggatc ggattcttac 1380
   catctgcaca gcaagcgggc tacaatgact accgaataca aaacattttg gcttaatttt 1440
   actgagtact acgatccatc ggaagttgag gtcctgcccg cctggtggga aagttatgct 1500
   cagtgtcctt tgatgaatct cgaatccgat ttggccaagc ttcgtattat ggcagaactg 1560
   ttgaattttg taggagctat cctgtatctg ctggttgctt tgcgagaagc tcgattcctg 1620
   ggccttaaaa tgtttatcga aaacttgatg acggcacctt cgcgagttat gtttctgttt 1680
   tcgtgtgctt tgatgatgac tataccctgg ttgagggttt cctgtctcac cgaaatagat 1740
   gatcacgtca ccgttgtgat aatgctaacc actgctccct attttctatt cttttgtcgc 1800
   ggcttcaaaa ctgtcggtcc cttcgtagtg atgatatacc gcatggtcat gggagatctg 1860
   ctccgattcg tctccatcta cttggtgttc gtcatgggtt tctcccaggc attttacata 1920
   atttttctaa ccttcgacaa cccatcgtca ccagaggatc aggacgcgga atccaaccca 1980
   atgccctcgc ccatggaatc gatagtggcc atgttcctaa tgtcgctaac aaatttcggt 2040
   gactactatg gagcgatggt gtctacgcag cacgaatacg aggcgaagat cctcttcttt 2100
   ctgttcatgg tgatcgtgag tgttctgctg gtgaacatgt tgatcgccat gatgggtaat 2160
   acgtatcaga agatcgccga gatccgaaac gagtggcagc gccaatgggc caggatcgtc 2220
   ctcgtcgtgg agcgaagtgt tcctcccgcc gagcggttga agaactttat gcagtacagt 2280
   cagtcgatgt cggatggcag aagggcactg gtcctccggt tgaatatgac tgaggaggaa 2340
   aaggaggaga tgaaggaggt gcaggagatg aagcgcatcc atcaacggtt cgccaagaag 2400
   cggcaaatgg agcgcgaggc tcgcgccctt cgacgtcaac aggagtacga gaagttcttc 2460
   ggcaccgccc ccaaatcgga atgttccgat aacaacaact tttga 2505
<210> 3
   <211> 2517
   <212> DNA
   <213> Musca domestica
<400> 3
   atgggaaata ctgaaagtaa tgttaccagt ggtgtcaaga aacaggcggg agtttccacc 60
   caggccctgt acaaatttgt caatctaaag ggtggtggcc ttctggtgga catgatgaaa 120
   agggcctgtc aaacgaaaca attcgccgaa atagatcatg ccattaaaac caaggtggaa 180
   ccatttctct ataacaaagg agctggtcgt tattttccga tttcgaaaat ggttttgtta 240
   cgcaacaagg agagggcacg cacgaaacaa ctgcctgaaa tacgggccct tgaaaatccc 300
   gatgaagatt tcaatattca cgactactgt tccgaggtgt cggaggcgga gtacatatcc 360
   aatccgagtg cctatcgttt tgtttgttgg aatttgaatg aaagaggtgc tgtgggtgaa 420
   acaattctac acttgtgcct gctcaatgcc tcctcgttgc atgcggattt ggccaagagg 480
   ctattgaaat tttatcccaa actcattatg gatatttact tgagcgatga atactatgga 540
   gaatcggtgt tacacattgc cattgtgaac gaggatccgg caatggtgaa atatctattg 600
   gatgccaatg ccgatgtcca ggagagatgc tgtggtgcct ttatgtcggc cgaagatcaa 660
   aaggcttcgc gatatgactc ccccgaacat gaatatgttg gtgttcatcc catgaccaat 720
   tatgatggct acgtttattg gggtgaatat ccactgtcat ttgcagcctg tttatcgcag 780
   gaagaatgtt ttcgtttggt gttggcccgt ggtgctgatc ccgatagcca agataccaat 840
   ggcaataccg tgttgcatat gttggtgatc tatgagaaaa tcgacatgtt cgatgtggcc 900
   tatgaggttg gcaccaatat ccacatacgg aatgtacaaa atcttacacc cctgacattg 960
   gcggccaagt tggcgcgggt ggaaatgttt ttccacattt tgagtataga gagggaaatt 1020
   tactggcagt tgggtagcat aacatgtgcc gcatatcctt tgtcaaaaat cgataccatt 1080
   gatgttaaga ctgggaatat aaacaaggat tcggtgttga attttgtggt gtttggggac 1140
   aaattggaac atttggagtt gttggatggc gtggtcattg atttgcttaa aacgaaatgg 1200
   gaaacatttg tcaagtcccg tttctacaaa cagttttata tgttttcctt ctatttcctc 1260
   ttctccttgg tgagttttat atcccggcca ggacccaaaa ttgaggatga agatgaggag 1320
   ggggatgcga atgattcggc gggtaaagga aatgattcta ccacaaatgc cagacttttg 1380
   tatgccacac ctgccagtag agtaacaggc attgaaaagt taccctacaa aacattttgg 1440
   cttaacttta gtgagtacat tgatgatgat gctaatgctg aaaatatgcc ctcctggtgg 1500
   gcaagctatg aggagtgtcc cctgatggat atggaaacga atttggcaaa gttccgcatt 1560
   gtgtccgaaa tcgtaatttt ctttggagcg attttgtatt tgttatcggc cctgagagag 1620
   gccaagtttt tgggttataa aatgtttgtt gaaaatctga tgacggcacc ctcaagggtc 1680
   atgttcttgt tctcctgctg cctaatgatg accataccct ggctaaggct atcctgtctc 1740
   accgaactgg atgatcatgt ggcggtgatg ataatgctta ccacggctcc ctactttttg 1800
   ttcttttgtc gaggcttcaa gaccgttggt cccttcgttg tgatgatcta tcgcatggtt 1860
   atgggagatt tgatacgttt cgtatcgatt tatttggtct ttgtgatggg cttttcccag 1920
   gcctattaca ttatcttttt gaccttcgat aatcctgcca caccggagga aattgatgat 1980
   accggcacaa atcccatgcc atcgcccatg gaatcagtgg tggccatgtt tctaatgtcc 2040
   ctgacaaatt tcggtgatta ttacggggcc atgggttcga cgcaacatga atatgaagcg 2100
   aaaattttat tcttcctttt tatggtgatc gtcagtgtgt tgctggttaa catgttgatt 2160
   gccatgatgg gtaatacgta tcagaagatt gccgagatac gcaatgaatg gcagcgccaa 2220
   tgggcccgca ttgttttggt tgtggaacgt agtgtgccac cggcagagcg tttgaagaat 2280
   ttcatgcagt attcacaacc gatgtcggat ggacgcaggg ctttggtgtt gcgtttaaat 2340
   atgagtgatg aggacaaaga ggaaatgaaa gaggtgcagg aaatgaaacg catacatcaa 2400
   cgcttttcca agaaacgcca aatggagcgg gaggccagag ccaaaaagcg ccaggaggaa 2460
   tatgagaaat tctttggcgc aacaccaacg tccgataatg aaaataataa tttctaa 2517
<210> 4
   <211> 2535
   <212> DNA
   <213> Ceratitis capitata
<400> 4
   atggggaata ccgagagcaa tgttaccagc ggcgttaaga aacaagctgg cgtctccacg 60
   caagcactct acaaattcgt taatctcaaa ggcggcggcc tgctggtgga catgatgaag 120
   cgcgcttgtc agacaaagca gtttgcagag atcgatcatg ccattaagac gaaggtggaa 180
   ccttttctct acaataaagg cgctggtcgc tactttccca tatcgaagtt ggtgctgttg 240
   cgtaataaag agcgtgcgcg taccaagcag ctgcctgaga ttagagcaat ggagaatccc 300
   gatgaagatt ttaatataca cgatcattgt accgaggttt cggaggctga gtacatatcg 360
   aatccgagtg cttatcgtta tgtttgctgg aatttgaatg aacgtggcgc tgtcggtgag 420
   actatcttgc atctctgtct gttgaatgcc tcatcgctgc atgccgatct ggcgaaacgt 480
   ctgctcaagt tttatcccaa actcatactg gatgtgtata tgtgtgatga gtattatggt 540
   gagagcgttt tgcatatcgc gatcgttaac gaagatccag caatggttaa gtatctgctc 600
   gacgcaggcg ctgatgtgca agaacgctgc tgtggcgcct tcatgtccgc ggaggatcag 660
   aaagcatcac gctacgactc acccgatcat gaatatgtcg gcgtacaacc gatgaccaac 720
   tatgatggct acgtttattg gggcgagtat ccgatgagtt ttgctgcttg tctctcgcaa 780
   gaagactgtt ttcgtttggt attggcgcgc ggcgctgatc cgaatctgca ggacaccaac 840
   ggtaatacct cgctgcatat gttggtgatc tatgaaaaga tcgaaatgtt cgatgtggcc 900
   tacgaggtgg gtactaatat acatttgcgt aatgtacaaa acctgacagc tttgacgttg 960
   gcggcgaaat tgggtcgcgt cgagatgttc tttcacatat tgagtattga acgcgagatc 1020
   tactggcagt tgggtagcat aacgtgtgcg gcatatccac tctcgatgat cgacacaatc 1080
   gatgtggaga cgggtaatat caataaggac tcggttttga attttgtcgt gtttggtgac 1140
   aaactggagc acttggagct gctggatggc gtagtgatcg atttgttgaa gaccaaatgg 1200
   gagtccttcg tcaagtcgcg tttctacaag cagttttata tgttctcctt ctattttctc 1260
   ttctcattaa tcagtttcat ctcgcgacca ggaccgactg aaaagaatga ggacgatgat 1320
   gatgatgatg atgctgatgc tagcgttgag gctaccgtaa cgcgcgcagc gaatagtacg 1380
   gctccgctac aacgtggcga tcagtggttc aagcgcttga tcaagcgtgc tatcgacaag 1440
   atggaataca aaaccttttg gctgaacttc acagactact ttgatgacaa cgacgtggaa 1500
   tcggtgccac cgtggtgggc gagctatgcc gagtgcccgc ttatgaacat ggaatcggac 1560
   ttagcgaagc tacgcattgt ggcggaaata attatatttt ttggctcggt tctatattta 1620
   ttcgccgcgc tgagagaggc acgctttctg ggctataaaa tgtttgtcga aaatatgatg 1680
   accgcacctt cgcgtgtcat gtttcttttc tcttgcgcac tgatgatgtc tataccttgg 1740
   ctgagggtgt tttgtctgac tgagatcgat gaccacgtgg cggtatttat tatgcttaca 1800
   acagcaccct attttctgtt cttctgtcgc ggcttcaaaa ctgtcggtcc cttcgttgtg 1860
   atgatctatc gcatggtcat gggtgatctt atacgttttg tctccattta tttggttttt 1920
   gtaatgggtt tctcgcaagc ttactatatt atttttctca cctttgacaa tcccgctaca 1980
   ccggaggaaa tcgatgactc cgagtcgaat ccaatgccct caccaatgga gtctatcgtt 2040
   gcaatgtttc tgatgtcact cacgaatttc ggtgactatt tcggcggcat ggcctcgaca 2100
   cagcatgaat atgaagcgaa gacactcttc tttctcttca tggtaatcgt cagtgtgttg 2160
   ttggtgaaca tgttgatcgc catgatgggt aatacctatc agaagattgc cgaaatacgc 2220
   aacgaatggc aacgtcagtg ggcgcgtata gttttggtgg tggagcgcag tgtaccacca 2280
   gcggagcggc tcaagaactt catgcactac agtcaaccaa tgtcggacgg caggcgtgcg 2340
   ttggtgttgc gtttaaatat gagcgatgaa gttaaggagg aaatgaagga ggtacaagag 2400
   accaaacgca tacatcagcg cttctcgaag aaacgacaaa ttgagcgcga ggcgagagca 2460
   caaaagagac aagaggagta tgagaaattc tttggcaaga agccgtcggg caacagtgat 2520
   aacaataatg cctaa 2535
<210> 5
   <211> 2502
   <212> DNA
   <213> Anopheles gambiae
<400> 5
   atgggaaata ccgagagcaa tgtgaccagt ggcgttaaga agcaggctgg agtgtccacc 60
   caggcgctgt acaagttcgt caacctgaag ggcggcggcc tgctggtgga catgatgaag 120
   cgcgcgatac agaacaagca gtacgccgag attgatcatg cgatcaagac gaaggtggaa 180
   ccgtttctgt acaacaaggg caaggggaag tacatccccg tgtcggtgct ggtgctgtta 240
   cgaaaccggg agcggccgcg ccacaagcag ctgcccgaga tccgggcgat ggagaacccg 300
   gaggaggact ttgacatcga tgcggtctgt ccggaagtga gcgaagccga gtactaccta 360
   aacccgtccg gctatcggga ggtgtgctgg aacataaagg aacgtggcgc tgtgggagag 420
   acaattctac acctttgcct actgaacgcc acttccctgc atgcggatct agccaaacga 480
   ctgctgcgct tctatcccaa gctcatcaac gacgtgtaca tgtgcgacga gtactacggt 540
   gagaatgtcc tgcatctcgc aatcgtgaac gaagatcctg ccatggtgaa gtatctgctc 600
   gatcacaacg cggacgtaaa cgagcgctgc tgcggtacgt tcatgtgccc ggaagaccag 660
   aaagcctccc gctacgatac gcccgaaacg gaggtcgtcc agatggtgca ggtgacgaac 720
   tacgacggct acgtgtactg gggcgagtac ccgctcacct ttgccgcctg tctcgggcag 780
   gaggagtgct accggctggt gctggcccgc ggcgccgacc cggacaacaa agacttcaac 840
   ggcaacacgg tgctgcacat gctggtgata tacgagaaga ttgccacgtt cgacatgggg 900
   tacgaggtgg gttcgtcgct tagcatccgc aaccaccaga acctgacgcc gctgacgctg 960
   gcggccaagc tcggccgggt ggagatgttc tttcacatca tgaacatcga gcgcgagatc 1020
   tactggcagc tgggcagcat cacgtgcgcg gcctatccgc tcggcctgat cgacacgatc 1080
   gacgtggaga cgggaaacat taacaaagat tccgccctca atctggtggc gttcggcgac 1140
   aaggacgagc atctcgatct gctagacggg gtgctgatcg atctgctcaa gacgaagtgg 1200
   aacacgttcg tgaaggacaa gttctatcgg cagtttttca tgttcttctg ctacttttgc 1260
   gtgtcgctgg tgagcttcac gctgcgcaat ggaccaccac cggcggagga tgacgatggg 1320
   aaggagagcg aggctggggg gaataaaacg gcagctgtca cgaaggaaga gcttcgtcgg 1380
   cagctgtgga acgaaacgac gctagaggat gcgctggctg cgcttgccgg caatggaagc 1440
   ttcgctggac ggttgtcgaa tctggagcag agcaacggaa gtagcgacgc gatgggtgag 1500
   tttgatcctg ccgagatgga cgacgagggt ctgttcttca actccaactg tcacacgatg 1560
   gattacgacg gtgtggaggg caaggtgcgt ctcatatcgg agatgatcat actggtcggt 1620
   tcgtttctgt atctgctcgc tgccttgcgg gagttgaagt ttcttgggcg gaaaatgttc 1680
   ttcgaaaatc ttatgacagc accatcgagg gttatgtttc tgttttcctg ctgcataatg 1740
   atgatcgtac cgtttctgaa ggttctctgc tttacggagc tggaagatca cgtagccgta 1800
   acgatcatgc tgacgacggc gccctatttt ttattcttct gtcgtggctt caaaacggtc 1860
   ggaccgttcg tggtgatgat ataccgcatg gtgatgggcg atctgttgcg gttcgtcgtc 1920
   atctatctgg tatttgtgat gggcttctcg caggcgtact acattgtgtt tttgtcctac 1980
   aaaccggacg aggaaggcga cccaaacccg atgccgtcgc cgatcgagtc gatcgtagcg 2040
   atgtttctga tgtcgctgac caactttggc gattactatg gcgcactgga gaacaccgac 2100
   cacgagatgt gcgcaaagat cttgttcgtg ctgttcatgg tgatcgttgc cgtgctgttg 2160
   gtgaacatgt tgatcgctat gatgggcaac acgtaccaga agattgcaga aacgaagaac 2220
   gagtggcagc gccagtgggc ccgcatcgtg ctcgtcgtcg agcgtggcgt cccaccaaag 2280
   gagcggctca agaacctgat gtcgtacagc caaccgatgt cggacgggcg tcgtgcgctg 2340
   gtgctacgat taaacatgac ggaagataag gaagagatga aggagatact cgaaatgaag 2400
   cgcgtccacg agcggctctc tataaagcgg cagatcgagc gggatgcacg ggccgagcag 2460
   cgtcggctgc tgcgcgagaa gtacttgaat atgtcttcat ga 2502
<210> 6
   <211> 2448
   <212> DNA
   <213> Aedes aegypti
<400> 6
   atgggaaata ccgagagcaa tgtcaccagc ggagtcaaga aacaggccgg agtgtccacc 60
   caggctctgt acaagttcgt caatctgaag ggtggtggtc ttttggtgga catgatgaaa 120
   agagcaatcc aaaacaaaca atatgcagaa atagatcatg cgatcaaaac gaaagtcgaa 180
   cccttcctgt acaataaggg caaagggaag tatatcccag tggcccagtt ggtgctgctg 240
   aggaatcgag aacgacctcg atccaagcag ttgccagaga ttcgtgcctt ggaaaaccct 300
   gaagaggatt tcgacattga cgcagtatgt ccggacgtga ccgagggcga ataccagagg 360
   aatccatcgg ggtacaggga ggtgtgctgg aacatcaagg aacgaggagc agtcggcgaa 420
   actattcttc atctgtgctt gctgaacgca acatctttgc atgctgatct tgccaagcgc 480
   ctactacgtt tctacccgaa gctcatcaac gatatctaca tgtgcgatga gtattacggg 540
   gaaagtgtgc tccacgtggc aattgtcaac gaagatccgg ccatggttaa gtatttattg 600
   gacagtggtt ccgatgtaga cgagagatgt tgcggaacgt tcatgtgtcc agaggaccaa 660
   aaggcaacgc gatacgattc tatcgaaacg gaaatcattt gcgtacttcc catgaccaac 720
   tacgatgggt atgtgtattg gggcgaatat ccgttgagtt ttgccgcttg cttaggccag 780
   gaggaatgct accggttggt gttggctcgg ggagctgatc cagataatca ggattcgaac 840
   ggaaacaccg ttttgcacat gttggtcatc tacgagaaaa tctcaacatt cgacatggcg 900
   tatgaagtcg gctcatcgct ggacataaga aacctgcaga atttgacgcc gttgactttg 960
   gcagccaagt tgggtcgggt tgagatgttt ttccacatta tgaacatcga acgagaaatc 1020
   tactggcagt tgggagctat cacttgtgca gcctatccgc ttgctttgat cgacacgatc 1080
   gacatcgaaa cagggaacat taacaaagat tcagcattaa atttagtggt attcggcgac 1140
   aaagcggagc atttagacct actagaaggt gtactgattg atttgctcaa gaccaagtgg 1200
   aactcgttcg tcaaagacaa gttctatcgc caattcttcg tgttcttttg gtatttttgc 1260
   gtttcactag taagcttcac tctgagaagg ggaccaatga atttggagga gacggcagcc 1320
   aaccgcacca caaccagcat tactttgaaa aatttgacat tttctgattc gactaacgca 1380
   acattaccca ctttactggc tgcggatgtg aacgaaaaaa tcgttcaatt tattgcaaac 1440
   gaaacgttat tcgatccggc agttatggaa gaaaacactt tctttaattc taaatgtgcc 1500
   accatggcat acgacacact gcaggataag ataaggttgg tttcggagat catcattctc 1560
   atcggatcat tcttgtacct agttggagcg ttgagagaat ttcgattctt gggacgaaaa 1620
   atgttctttg agaatttgat gatttcccct tctcgagtta tgtttctgtt ctcgtgctgc 1680
   ataatgatga tcgtaccgtt tctgaaagtc ctttgcttaa tcgagcttga ggatcacgtt 1740
   gccgtgacga ttatgttgac tactgctccg tactttttgt tcttctgtcg tggtttcaaa 1800
   accgttggac cgttcgttgt gatgatctac cgcatggtga tgggagactt gctgcgattc 1860
   gtcgtcatct atctggtgtt cgtgatggga ttttcccagg cctactacat tgtgttcttg 1920
   tcgttcaagg gtgaagagga aggcgatgaa aacccgatgc cttcgccgat ggagtcgata 1980
   gtggcgatgt ttttgatgtc gctgacgaat ttcggcgact actacggtgc actggagaaa 2040
   accgagcacg agggatgcgc taaggttctt ttcgtcattt tcatggttat tgtggccgtc 2100
   ctgttggtca acttgctcat cgccatgatg ggtaacacct acaccatgat tgccgaaacg 2160
   aagaacgaat ggcaacggca atgggcacga atcgttctgg tagtggaacg aggagttcct 2220
   ccgagcgagc gactcaaaaa cttcatgagc tacagccaac ccatgtcgga cgggagacga 2280
   gccctcgtgt tgcgattgca tatgacggac gaagacaagg aagagatgaa ggaaatcctc 2340
   gagatgaaac gtgtccatga acgcttgaag aagaaacgtc aggtggaacg agaattaaga 2400
   gccgagcagc gacgactaat catggagaaa tatgcaaacg taccttga 2448
<210> 7
   <211> 2451
   <212> DNA
   <213> Culex quinquefasciatus
<400> 7
   atgggaaata ccgaaagtaa cgtcaccagt ggcgtcaaga agcaggccgg agtgtccacc 60
   caggcactgt acaagtttgt caacctgaaa ggtggcggcc tgctggtgga catgatgaag 120
   cgggcgatcc aaaacaaaca gtacgccgaa atagaccacg caataaagac caaggtggaa 180
   ccgtttctgt acaacaaggg caaggggaag tacattccgg tggcacagtt ggtgtaccta 240
   aggaaccggg aacggccacg ccacaagcaa ctgccggaaa ttcgtgccct cgaaaatccc 300
   gaggaggact acgatataga caaagagtgc ccggatgtga ccgagggcga gtacatgcgg 360
   aaccccgggg ggtacaggga ggtgtgctgg aacatcaagg aacgtggcgc agtaggcgag 420
   accattttgc atttgtgcct tctgaacgca acttcactgc acgcagatct ggcgaagcgc 480
   ctgctgcgat tctaccctaa actgattaac gatgtgtaca tgtgcgatga gtactacggc 540
   gaaagtgtgc tgcatttggc gatcgtcaac gaagatccgg ccatggtgaa atacctgctg 600
   gatgccgggt ccgacgtgaa tgaacggtgc tgcgggacgt tcatgtgtcc ggaggatcaa 660
   aaagcatccc ggttcgattc actggaaacg gaaataatct gtgtgatgcc acaaaccaac 720
   tacgacggat acgtctattg gggagagtac ccgttgagct tcgccgcttg tttgggccag 780
   gaagagtgct accggttggt gttggcacgg ggagccgacc cggataacca ggattcaaac 840
   atgaacaacg ttctacacat gatggtgatt tacgaaaagt taacgacgtt cgacatggcg 900
   tacgaagtgg gttcttcgct gagcataaga aatctccaaa atctaacacc gttgacgttg 960
   gccgccaagc tgggtcgcgt cgaaatgttc ttccacatca tgaacattga gcgggagatc 1020
   tactggcaat tgggaagtat tacgtgtgcc gcctacccgc tggcattgct cgataccatc 1080
   gacatcgaaa ccggcaacat tagcaaagat tccgcgctga atctggtcgt tttcggcgac 1140
   aaagacgagc atttagatct cctcgaagga gtcctcatcg atctcctgaa aaccaaatgg 1200
   aacacgttcg tcaaggacaa gttctacagc caatttttcg tgttcttttg ctatttttgt 1260
   gtgtcactga ttagcttcac actgcgcgag ggaccaatga agctagagga cggagaggcc 1320
   aacaaaacta ccacgagtat tcagctaaaa aatatgaccc tggccgacct caccaacgcc 1380
   agtacgctgt cctcgctact ggtcaccgac gtgagcggca agatcgtcgg actcaacggg 1440
   agtgaaacgt tcttcgaccc gtcgacgatc gagtccgaca tgttcttcaa ctccaagtgt 1500
   ccaacgatga tctacaaggg tatcgagggg aaaattcgac tggtttcgga agcaattatt 1560
   ctggtgggat catttctcta tctggtttcg gctttgagag aatctcgctt tcttgggagg 1620
   aaaatgttta tcgaaaattt gatgaccgct ccttccagag taatgttcct gttttcctgc 1680
   tgtatcatga tggtagttcc ttgcctcaaa gtgctatgtt tcaccgagtt ggaagatcac 1740
   gttgcggtcg tgattatgct gacaactgcg ccctacttcc tgttcttctg tcgcggcttc 1800
   aaaacggtcg gaccgttcgt ggtgatgatt taccgcatgg tgatgggaga tctgctccgg 1860
   ttcgtggcca tttactcggt gttcgtgatg ggcttctccc aggcctacta catcgcgttc      1920
   ctgtcgtaca agggcgagga ggagggcgac gagaatccga tgccctcgcc gatggagtcg      1980
   atcgtggcca tgtttctgat gtcgttgacc aactttggcg attactacgc cgcgctcgag      2040
   gataccgccc acgagggttg cgcaaaggta ctctttgtga catacatggt aattgtggcg      2100
   gtgctgttgg tcaacttgct gatcgctatg atgggtaata cgtacaccaa gattgccgag      2160
   acgaaaaacg agtggcaacg gcagtgggct cgcatcgttc tggtcgtcga gcgaggcgtt      2220
   ccaccggcag aacgtctgaa gaacctaatg aactacagtc aacccatgtc ggacggtaga      2280
   agggccttgg ttttgcgatt gaatatgacg gaagaggata aggaagagat gaaggagatc      2340
   cttgacatga aacgggtgca cgaacggctg tccaagaagc gagcagttga gcgggaagca      2400
   cgcgccgagc agagacgtct cattgcggag aaatacgcaa acgtaccgta g               2451
<210> 8
   <211> 2385
   <212> DNA
   <213> Tribolium castaneum
<400> 8
   atgggtaata ccgaaagtaa cgttactagt ggtgttaaga agcaagctgg tgcttcagtg      60
   caaccaattt ataaattatg cgatttgaaa gggggagggc tcctagtgga attaatgaaa     120
   cgggccacac aaaacaagca atatgcagaa ctagaccacg caataaaaac caaggtggag     180
   cactttttgt acaataaagg ggctggacgg tatttcacaa tatcggactt ggttcttttg     240
   aggaataagg aaaggtctcg ccagaaatgg ttaccacaat tgaaagcaat ggaaaaccct     300
   gaagattttg aaattgatga tgaaggcccc gaaataacag aagaacagta tcaaaaaaat     360
   ccgcatttgt accgtcacgt ttgctggaaa atcaaggaac gcggagccgt gggcgaaagc     420
   ataatgcact tgtgtttgct aaatgccact tccttgcacg ctgatatcgc aaagaggctt     480
   ttacgctttt accccaaatt gattaacgat atttacatgt cggatgaata ttacggagaa     540
   aacgttttgc acattgctat cgtaaacgag gatccgtcaa tggtaaaatt ccttctagat     600
   tcgggtgtaa atatccaaga acgctgctgt ggcaacttta tgtgtcccga agatcagaaa     660
   tcgtctcgtt atgactcctt ggaccacgag tgggtcaacg tttgcccggt cacgaattac     720
   gaaggctatg tatactgggg cgaatatcct ctaactttcg ccgcgtgttt aggacaggaa     780
   gagagctttc gcttgatgct gtccaggggg gctgatcccg atgctcaaga caccaacggg     840
   aacacggtcc tacacttgct tgtcattttg cagaaattgg aggctttcga tatggcctac     900
   gaagtggggg ccaaattatc aatccgaaac gtgttgagtc tgactccact aactctggcc     960
   gcaaaacttg ccagaatcga tatgtttttc cacattttga acttggaacg cgaaatttat    1020
   tggcaaattg gcagcatcac gtgcgctgct taccctctat cgcagattga tacaattgac    1080
   attgaaacgg gccaaataag caaaacttca gcccttaatc ttgtggtgtt tggtgacaaa 1140
   gacgagcact tggagttgat ggacggtgtt ttgatcgact tgcttaatgc gaaatggaac 1200
   acttttgtca agtttaaatt ctacaaacag tttttcacgt ttgcgttcta ttttttgatc 1260
   tcccttgttg ctttcacgct acggccaggg cccccacaca aggaggccaa actggtgaat 1320
   gtcacaataa acgccactgt ttcaaatact tccaagtggg aaaatctcac ttctataccg 1380
   ttcggtaaaa ccccggatga tgatgacagc gacatggaag aatggtggga caatttgcaa 1440
   gaagaatgtc gcttgatgca actagaatca cccgaatcta aaattcgact cactgcagag 1500
   gttttgatgg ttgtgggggc ttttgcatat ctggcagccg cagttcgcga ggctaggttt 1560
   ttaggggggc gcatgttttt cgaaaatctt atgactgcac cgtcaagagt catgttttta 1620
   ttttcttgta ttttaatgtt aacggtgcca tgtttgagac tggcttgttt ggatgagttt 1680
   gaagatattg tagctgtggt gattatgttg accactgcac cgtattttct cttcttctgt 1740
   cgaggtttta aaacagtggg acccttcgtt gtgatgatct acaggatggt gatgggtgat 1800
   ttgataagat ttgcttcaat ttatctagtg tttgtcatgg ggttttctca agctttttac 1860
   atcatttttc tctcgtttga taacccacta acccctgacg acgttgacga ttccgcaact 1920
   aaccccatct ccacccccat cgagtcaatt atggcgatgt ttctgatgtc aatgaccaat 1980
   ttcggtgact attatgcggc cttcgaaaaa actgaccatg aatacgaagc taaaatgttg 2040
   tttgttgtat ttatggtaat cgtttcaatt ttactgatca acatgttgat tgccatgatg 2100
   ggcaacactt accaaaaaat cgcagagaca agaaacgagt ggcaaagaca atgggcgcga 2160
   attgttttgg ttgttgaaag gggggtgagt ccctctgagc gtttgaaaca acttatggtt 2220
   tattcacaac ctatgtccga tggaagaagg gctttagtgt tgagattaaa ccagtctgac 2280
   gaggacaagg aggaaatgaa ggaaattcta gaaatgcggc gcaggcatga aagacacata 2340
   aagaaaagaa gcgaaaagtt gaaagagaat gcaaaaaaga attga 2385
<210> 9
   <211> 2526
   <212> DNA
   <213> Bombyx mori
<400> 9
<210> 10
   <211> 2379
   <212> DNA
   <213> Anopheles darlingi
<400> 10
   atgggaaata cggagagtaa tgtgaccagt ggcgtgaaga agcaggccgg agtgtccacc 60
   caggccctgt acaagttcgt caacctgaag ggcggtgggc tgctggtgga tatgatgaaa 120
   cgggcgatcc agaacaagca gtacgccgag attgaccacg cgatcaagac gaaggtcgaa 180
   cccttcctgt acaacaaagg caaaggaaag tacatcccgg tgtcggtgct ggtgctgttg 240
   cgaaacagtg agcgaccgag gcacaagcag ctgcccgaaa tccgagcgtt ggagaacccg 300
   gaggaggact ttgatatcga tgcagtctgc ccggaagtga gcgaagcgga gtactacctc 360
   aacccatccg ggtaccggga ggtgtgctgg aacgtcaagg aacgtggagc agtcggtgag 420
   acgattctgc atctttgcct gctcaacgct acgtcactac atgccgatct ggcgaagcgg 480
   cttctacgct tctatccaaa gctcatcaac gacgtgtaca tgtgcgacga gtactacggc 540
   gagagtgttc tccatctggc catcgtaaac gaggatcccg cgatggtgaa atacctgctc 600
   gatcacaact cggacgtaaa cgagcgctgc tgcgggacgt tcatgtgccc ggaggatcaa 660
   aaggcttccc gctacgatac tcccgagacg gaagtcgtcc agatggtgca gctcacgaac 720
   tacgatgggt acgtgtattg gggcgagtat ccccttacat ttgccgcctg tctaggacaa 780
   gaagagtgct accggctggt gttggcccgc ggtgccgacc cggacaacaa ggactttaac 840
   ggcaacacgg tactgcatat gctggtgata tacgagaaga ttaccacgtt cgatatgggc 900
   tacgaggtag gatcatcgtt aagcatacgg aatcaccaga atctaacgcc gctaacgttg 960
   gcggcgaagc ttggccgcgt cgagatgttc ttccacatta tgaacattga gcgagagatt 1020
   tactggcagc tgggtagcat tacctgtgcc gcctatccgc tcgtcctgat cgacacgatc 1080
   gacgtagaaa ccggtaacat cagcaaagac tcggccctga atttggtcgt gtttggtgac 1140
   aaagatgagc atctggatct actggacggt gtgctgatcg atctgctcaa gacgaagtgg 1200
   aacacgttcg tgaaggacaa attctatcga cagttcttca tgtttttctg ctacttctgt 1260
   gtttcgctga tcggcttcac gctacgcaat ggaccaccac cgcccgagga agacgacgaa 1320
   aatcgtgccg gtggtggcag tggcaacaaa acgtcctcag cttcgaagca ggatcttcgg 1380
   caacgattat ggaaccgaac ggtaacatta gaggacgcgg acaatgtaag cagcctttta 1440
   agtgacatta tggggattga tgcgcttggc aaactggccg agcagcttct taacggaagc 1500
   gaaggatcgg gtgcattcaa acccgaaacg gtcgaagagg aagaggatgg gctgttcttc 1560
   aactccaagt gtcacaccat ggattatgat ggcgttgagg gaaaggtacg attcgtggcc 1620
   gaagtcatca tactgatcgg ttccttcctc tatctcctgt cggcgttgcg cgagctaaag 1680
   tttcttggcc ggaagatgtt cttcgaaaat ttggtaagcc gtggcttcaa gacggtcggt 1740
   cctttcgtgg tgatgatcta ccgtatggtg atgggtgatc tgctgcggtt cgtcgtcatc 1800
   tatctggtgt ttgtgatggg tttctcgcag gcgtactata tcgtgtttct ctcctacaaa 1860
   ccggaggagg acggtgacgc caatccgatg ccatcgccca tcgaatcgat cgtcgccatg 1920
   tttctgatgt cgctgaccaa ctttggggat tactatggag cgctcgaaaa cacggatcac 1980
   gagatgtgcg ccaagatcct gttcgtgctc tttatggtga ttgtggcggt gctgttggtg 2040
   aacatgctga tcgctatgat gggtaacacc taccagaaga tcgccgaaac caagaacgaa 2100
   tggcaacgcc agtgggcacg catcgtgctg gtggtagaac gtggtgtgcc accgaaggag 2160
   cggctcaaaa atctcatgtc ctacagtcaa ccgatgtcgg atggtcggcg agcactggtg 2220
   ctccggttaa acatgacgga cgaagacaaa gaggaaatga aggagatcct cgagatgaaa 2280
   cgtgttcacg agcggctgtc gaagaagcgg caggtcgagc gggatgcccg ggccgagcag 2340
   cgacgtttgc tgcgcgaaaa atatctcaac atgacgtag 2379
<210> 11
   <211> 2703
   <212> DNA
   <213> Acyrthosiphon pisum
<400> 11
   atgggtaaca ccgagagcaa tgtggccagt ggtgtcaaga agcaagtcga cacctcgtca        60
   ctgcagatat acaaccttgt cgacctgaaa ggttgtggtc ttctcgtgga tttgatgaag       120
   aaagccgtgc agacgaaaaa ttttaccgag ctggacaacg cgatcaaaac ccaagtggaa       180
   cccatgctgt acaacaaagg tgaaggtaga atgataccta tcgccaaact cgtgttgctg       240
   agaaatcgcg accgttcccg ggcaagatgg cttccaccgc taaagaacat ggaagacacc       300
   aatgattacg acatagaaaa agacattccc accattgaag aactggaaaa gctgggggaa       360
   gtgcagtaca gggaagtgtg ctgggatctg aaggaaagag gcaccgtcgg cgaaacggcg       420
   ttacatctat gtttgctgaa cgctacgtca atacacgcag acttggccaa aaggctgttg       480
   catttctatc caaagctcgt aaatgacatt tacatgattg acgagtatta cggagagagc       540
   gttttgcaca tggccatcgt caacgaagat ccggccatgg tgaaggtact gatggacagc       600
   ggggctaacc tcaacgaacg gtgtttcggg aacttcatga gcactgaaga tcaaaaggct 660
   tctagatcgg attcgctcga ccacgaatgg gtgaacctat gtcccgacac caattatgaa 720
   gggtacgtgt attggggcga atatccactg tcatttgcag cgtgtctcgg ccaagaggag 780
   agctaccgtt tgatgttggc gagaggcgct gacccaaata accaggacac caacggcaac 840
   acggttctgc atatgcttgt aatttacgaa aaaatttcga cgttcgacat ggcctacgaa 900
   attggggcgg aactcaacat taggaacgtg caaaatttga cgccactcac actggccgcg 960
   aaactggctc ggatccaaat gttctttcac atactcaaaa tcgaacggga aatttattgg 1020
   caaatcggca gtatcacttg tgctgcgtac cccctgaccc aaatcgacac catcgacaat 1080
   gacaccgggc agatcagcag aaactcggcg ctcaacttgg ttgtatttgg cgaaagtccg 1140
   gaacacttgg agatgatgga cggtgtgttg gtggatctgt taaatgccaa atggaacgct 1200
   ttcgtgaaat tccggttcta taggcagttc atcttgttcg ccttttactt ttccatatct 1260
   atggtatgct tcgtgctgcg tcccggaccg ccacccactg gtcttaagcc accattaatc 1320
   aactacactt ccacgtcgac cacggtggcc accacgccgt ttgacttcat ggacaacgag 1380
   tcgatgttgc ccgacatgta cgacgagatg atgatcggcg gtggcggtcc gaccgagttc 1440
   gagacggtgc aggtgaccac ggaaatggcg acgaccagcg acaacgcgac gacgaccaac 1500
   aacggtagcg gtatcggaac gccactgatc tacgaggacg gcggcggtgg aggtggtgga 1560
   ggtggtaccg tgagcttgca cgggaagagt tcgggttcgg acaggggcaa acacaggagc 1620
   acgtggagtc cggccaacca tacgttcaag accaactaca cgtacaacta caacaaatca 1680
   agaaaatttt ggtggaacaa gagacaaaag gtatgcagat taatggctgt gcactcgact 1740
   gacgatttaa tcagaatggg atcggaagtg ggaatgttta tgggagcatt cctctatctt 1800
   ctggccgcgg tcagagaagc tggttttttg ggcacacaga tgttcattga aaacttggcc 1860
   atagcaccgg caagagtgat gtttctgttc tcgtgcctgc tcatgatgac cataccgccg 1920
   ctgagactga catgctatga caaagccgaa gacataatag ccgtaatcat tatgttgacc 1980
   acttgtccgt actttttgtt cttctgcagg ggtttcaaaa ccgtcgggcc attcgtggtg 2040
   atgatctata ggatggtgat gggagacttg ttgaggttcg tttccattta catggtgttc 2100
   gtgatgggtt tctcgcaagc ttattacgtg attttcctgt cttacgacaa tccgctgacg 2160
   ccagagggta tcgatgactc ggtactgaac ccgatgccca cgccgaccga gtccatcatg 2220
   gcgatgttcc ttatgtccgt caacacgttc acagactact acacggcgtt tgacaagact 2280
   agtcacacgt tggtcgcaaa gttttgcttt atagttttca tggtaatcgt ggccatattg 2340
   ctggtgaaca tgttgatcgc catgatgggc aacacttatc aaaagattgc cgagaccaga 2400
   aacgaatggc agagacaatg ggctcggatc gtactggtgg tagagagagg tgtcagtccg 2460
   tcgcaaaggc tcataaagct catgtactac tcgcaaccga tgtcagatgg ccggagggcg 2520
   ttggtgttgc gactcaatca gacggatgaa gacaaggagc aaatgaaaga gatattggaa 2580
   atgaaacgca ttcacaatcg ctatgtagaa agaatgcgcg ccagagacct gagcagcaaa 2640
   ctatcgtctt cgtgcggaaa aaatgcaaac aatgggacat ttaatctaaa agcaaatgaa 2700
   taa 2703
<210> 12
   <211> 2124
   <212> DNA
   <213> Dendroctonus ponderosae
<400> 12
   atgggtaata ccgagagtaa tgttactagt ggcgtcaaaa aacaggcggg agccgccatg 60
   cagccgatat ataaactttg tgatttgaaa gggggcggtc tgctggtgga acttatgaaa 120
   agggcagccc agaataagca gtacgcggaa atagatcacg ccatcaaaac taaggtggaa 180
   cattttctgt acaataaagg tgccgggcgt tatatcccaa tatctcaaat ggtgctttta 240
   aggaacaagg aacgcgggaa gcataaactg ctgccccaac ttagaggaat ggaaaatcct 300
   gaggattttg aagtcgatga tgaaggaccc gaaataactg aagaagagta caataaagat 360
   ccatcattat atcggcatgt ttgttggaag ctgagagaaa gaggagcggt tggagaaaca 420
   attttacatc tttgtttatt gaatgccact tctctacatg cagacatcgc caagaggcta 480
   ttgaggttct atcccaaatt gatcaacgat atttacatat cagatgaata ctatggtgaa 540
   aatgttctac atatcgccat agttaacgag gatccttcca tggtaaagtt tcttcttgcc 600
   gcaaatgtga actttcagga aagatgtttc ggtaatttca tgtgtccaga agatcagaaa 660
   tcatcgagaa ctgattctct agatcacgaa tgggtgaatg tttatcacga aacaaattat 720
   gatggatatg tgtactgggg tgaatatcca ttgtcatttg ctgcatgttt aggtcaagaa 780
   gaaagttttc gattaatttt ggcaaagggt gccaatttag atgcacaaga cactaatgga 840
   aacactgtcc tgcatttatt agttatttat tccaaagtgg aagctttcga tatggcatat 900
   gaattaggtg ctcacctttc aataaggaac attctgaaaa tgacaccttt aacgctggcc 960
   gccaagctag caagaatgga tatatttttc catattttaa agctagagag ggaaatatac 1020
   tggcagatag gaagcattac atgtgccgcc tatcctctat ctcagattga tacgatagat 1080
   gttaagactg gacaaatcag taaaatgtca gcgctgaatc tggtagtatt tggtgataaa 1140
   gatgaacatt tagaactgct cgatggagta ttagttgatc tattaaatgc caagtggaat 1200
   acgtttgtga aatctaaatt ttaccggcaa ttttttactt ttgcgtttta tttcaccgta 1260
   tcgcttgtgg ataccagcaa tgatgattat gatgttgagg aatggtggga taatttacga 1320
   gaggaatgtc gattaatgaa tttagacacg actgagtcac tagtgagatt tactgctgaa 1380
   cttggaatca cgtttggtgc atttctttat ttggcggccg caatgactgc tccttcgaga 1440
   gttatgttct tgttttcctg cattttggtt tttggagtaa ttcctagcct gcgcttggta     1500
   tgcatggatg aagttgaaga tataatcgca gtagtagtta tgctaacgac ggctccatat     1560
   ttcctatttt tctgtagagg ctttaaaaca gtaggtccgt ttgtggtaat gatctatcgc     1620
   atggttctag gcgacttgct acgtttcgca actatataca tggtttttgt catgggattt     1680
   tctcaagcat actacataat tttcttgtct tttgataacc cattgacacc agaagacgtc     1740
   gatgactcgg ctacgaatcc aatgtcaact ccatccgaat ccattatggc aatgttttta     1800
   atgtccatga ccaacttcgg agattattac acagcatttg ctaggaccga gcacgaatat     1860
   gaggctaaaa ttcttttcgt aatatttatg gccattgtgg caattttgtt aattaatatg     1920
   ttaatcgcta tgatgggtaa cacataccaa aagatagcgg aaacgcgtaa cgaatggcag     1980
   cgacagtggg ctcgcattgt tttggtcgta gaaagaggtg ttagcccacc tgaacggctc     2040
   aagcaattga atgtttattc acaaccaatg agcgatggca aaagagctct cgttttaagg     2100
   ctcaaccagt cggtaagata ttag                                            2124
<210> 13
   <211> 2718
   <212> DNA
   <213> Harpegnathos saltator
<400> 13
   atgggcaata ccgagagcaa cgtcacgagc ggcgtgaaaa agcagacgga cacgtcgtcc      60
   atcatgcttt ataagttggt cgatctgaaa ggcggtgggt tgttcgtaga catgatgaag     120
   agagcagcgc aaactaagca gttcgcagag ttggatcacg cggtgagaac aaaggtcgag     180
   ccctacctgt acaacaaagg aaagggcaag tggatacccg tggacaaact ggttcttcta      240
   agaaacaaag atcgtccgcg acataaaatg ctggcacctt tgaaagccat ggaaaatccg     300
   gcggattacg acatagataa ggacatgggt gatgaagatg ttgacgagac taaaatagac     360
   aaaagcaaat acagactggt ctgctggagc ctgagcgaga gaggcgctgt cggtgaaact     420
   atcctgcact tgtgcatgtt aaatgccact gctctccacg cggatctggc gaaacgattg     480
   ctacgcttct atccgaatct cataaacgac atttacataa gcgacgaata ctacggagag     540
   aacgtgctgc acatcgcgat cgtaaacgag gacccagcga tggtgaagta tcttctagac     600
   agcggtgcgg atgttcacga gaggtgcttc ggtaacttca tgtgtccgga ggatcagaaa     660
   gcgtcaaggg ttgatagtct ggaccacgag tgggtgtgcg ttgcaccgga gaccaattac     720
   agcggatacg tgtactgggg agaatacccg ttgagcttcg ccgcgtgtct gggccaggag      780
   gagtgctaca ggcttatgct cgccagaggc gcggatcctg acaagcagga caccaacggc     840
   aacacggttt tgcacatgtt ggtgatatat gagaagctga ctacgttcga catggcgtac     900
   gaagtgggag cctcgttggc cataagaaac gtccaacacc tgactccgct gacgttgtca     960
   gcaaagctgg ccaagatcga gatgttcttt cacattctga acatcgagag agagatatac 1020
   tggcagatcg gtagcataac ttgcgcagct tacccgttgt cgcaaataga cacgatcgat 1080
   attaacatcg ggactatcaa caagaactcc gccctgaatt tggttgtttt tggggaaaaa 1140
   gacgagcatc tagaattgat ggacggtatg ttagtcgacc ttctgaacgc caagtggaac 1200
   actttcgtta agtctcgatt ctaccgtcaa ttcttcctct tctgcttcta cttcgtgctg 1260
   tcgctgatca gcttcactct tcgcccgggc ccgttgccgc aggagaccga aaatgcgaac 1320
   gatacgagaa cgaattcatc gaacgcaacg gcgatggaga ttcttaggtt gttccaaagc 1380
   tcggacctgt cgaacctcgt cacgaacagc ctcgccgcgg cttttgcctc gaacctcaaa 1440
   tcgtccttaa acgtgacgca agaatcgttg gagaagatcc agctgcaagt gacgtcgaac 1500
   attacatcgg ccctgaagaa tattttgcct tcgctgagcg acaacgaggg catcctgagt 1560
   gcccctgacg aagccgccat tcgtacgctg tggtatacca gtagtccgta caatgagtca 1620
   gattacttga cagatgctta caacgagaat gtcacgagcg atgcgaattc gaccgcgggg 1680
   acgatcatcg gcagcatcga ggtgtcgaag tccaacgata aggacaattg gtgggaagat 1740
   ctgacggaag aatgcaggct gatgcagcta acgacgataa gtgcgaagat ccggctgact 1800
   gcggaagtgc tgatggaaat cgccgcggtg ctctacattt tagccgcact gcgggaggcg 1860
   aggtttttgg ggctcaacat gttcgtcgag aatctaatga cagcgccgtc gcgcgtcatg 1920
   tttctcttct cgtgctgcat cttactctcg tttccatttc tgagattagc ctgcgccgac 1980
   gaagtcgaag acatgttggc ggtcgtggtg atgctgacaa cggcaccgta tttcttattc 2040
   ttctgcagag gcttcaagac ggtcggtccg ttcgtcgtga tgatttacag gatgatcatg 2100
   ggcgacctcc tcagattcgt gtccatctat ctggtcttcg tcatgggctt ctctcaagcg 2160
   tactacatta tatttctgtc gttcgacaac ccgaataccc cggaaggcgt cgacgattcg 2220
   gtatcgaacc cgatgccgtc gccgatggag agcatcatgg cgatgttcct gatgagcatg 2280
   acgaacttcg gcgactacta cggcgcgttc gagcgaaccg aacacgagtc cgaggctaag 2340
   ctgctgtttg tgttgtacat ggcgatcgtg gcgatcctgc tcgtaaacat gctaattgct 2400
   atgatgggca acacttacca gaagatcgcc gagaccagaa atgaatggca aagacaatgg 2460
   gcgcgcatag ttctggtcgt ggagagagga gtcagccccg ccgagaggct gaagaagctc 2520
   atggactact ctcagccgat gtccggcggc cgtcgagcgt tggtcctccg gttgaatcaa 2580
   agtgaggagg acaaggagga gatgaaggaa atactcgaga tgaagaggac ccacgataga 2640
   ctgctgaaga agaggcaagg gagaatgtca aaggagaaag ttctggccga gagcgaaatt 2700
   attattaccc gaaattga 2718
<210> 14
   <211> 2756
   <212> DNA
   <213> Nasonia vitripennis
<400> 14
   atgggtaacg ccgagagcaa cgtcaccagc ggcgtgaaaa aacagacgga tgcaggtgcg      60
   atcgccctgt acaaactcgt tgatctcaaa ggtggtggtc tgctggtgga catgatgaag     120
   cgggcctcgc aaacgaaaca gtacgctgag atagatcacg caattaggac aaaagtcgag     180
   ccgtttctgt acaacggagg caaaggcaag tggataccca tagcgaaact ggtgctcttg     240
   aggaataaag aaagggggag acacaaaatg ttacccgtgc tcaaagccat ggagaagccc     300
   gaggactacg atatcgataa ggacatggct aacgacccgg agccggatga aaacacaatc     360
   gacaagagta aatacaagct cgtctgttgg accctgagcg acagaggagc agtgggcgag     420
   acgattctgc atctctgcat gctcaacgcc acgcagctcc acgcggatct ggcgaagagg     480
   ctgctgcgct tctatccgaa cctcatcaac gacatttaca tcgatgacga atactacgga     540
   gagaacgtcc tgcacatcgc gatagtgaac gaagacccgt cgatggtgaa attcctgctg     600
   gacagcggag cgaacgtgaa cgagcgttgc tgcggcaact tcatgtgtcc ggaagatcaa     660
   aaggcttcta ggaacgacag cgtcgagcat gaatgggtct gcgtatgctc ggaaaccaac     720
   tacgatggtt acgtctactg gggcgagtat cctttgagct tcgcagcctg tttggatcag     780
   gaggaatgct accggctgat actagccaaa ggcgccgatc cagatagcca ggacacgaac     840
   ggcaacaccg ttctccacat gctcgtcatt tacgaaaagc tcgaaacctt cgacatggcc     900
   tacgaggtcg gttcttcctt gtcgatacga aacgtcctgc aactgacacc tttaacgcta     960
   gccgccaaac tcgcgagggt ggagatgttc ttccatattc tgaacatcga gcgcgagatc    1020
   tactggcaga tcggtagcat cacctgcgcg gcttatccgc tttctcagat cgacactatc    1080
   gacgtggaca ccggaaagat cagcaacaac tcggcgttga atttggtcgt tttcggtgat    1140
   aaagaggagc atctcaaact gctggaaggc gtgctgatcg atctactgaa cgcaaagtgg    1200
   aatactttcg tcaagtccag gttctaccag cagttctacc tcttcttctg ttacttcatc    1260
   ctctcgctga taagctgcac cctccgacct ggtcccataa cgaagaccga cgctccaacc    1320
   acgacaccac atcccatgaa cgacacgagc atcgagtcca tcactccgac ctcggaacct    1380
   ttagagctgc agaccttgtc gtcgcttctg gcgaaaaact tggccgaaca actcgtcatg    1440
   ggtatgaaga tgtacggcaa cgtctccgag gactcactcg cgaggataag tctccaggtg    1500
   gcttcaaaca tcagcggctc gatcaaggac atctggacga ggcagaacct cacgagcaac    1560
   gacaccgact acgtcgatat cgcaggaggt ctgtacgcgg acgtcaacga gaccagcgcg    1620
   agtgatccct tcgatttgaa cgcgagcgat atcgtgattc ttaagaaggg aaagaaggaa    1680
   ctcgatgact ggtgggatga cctaaccgaa gactgccgac tcatgcagat gaacagtacc    1740
   tcggcgaaaa tccgcctgac ggccgagata ttcatggaat tcggcgctat actttacata 1800
   tgcgctgcgc tccgagaggc gaggttcctc gggctcaaca tgttcatcga gaatctcatg 1860
   acggctccgt cgcgcgtcat gtttctcttc tcctgctgca tcctcctgtc ctttccgttt 1920
   ctgaggatgt cctgcgccga cgaggtcgag gacatactcg ctgtagtggt gatgctcacg 1980
   accgcgccct acttcctctt cttctgcaga ggcttcaaga ccgtcgggcc cttcgtcgtc 2040
   atgatctaca ggatgatcat gggtgacctg ctcagattcg tctcgatcta tctggtcttc 2100
   gtcatgggct tctctcaagc ttactacata atcttcctgt cgttcgacaa tccgatcacg 2160
   cccgagggtg tcgacgacag caaagctaat ccactaccgt cagccatgga gagcatcatg 2220
   gcgatgttcc tcatgagcat gacgaatttc ggcgattact acgacgcttt cgagaacacc 2280
   gagcacgaga tgttggccaa gtgtctcttc gtcgtgtata tggccattgt gtcggttcta 2340
   ctggtgaaca tgctcatcgc tatgatgggc aatacctacc agaagatcgc tgaaacgagg 2400
   aacgagtggc agagacagtg ggctaggata gttctcgtgg tggagagagg agtagccccg 2460
   gcggagagat tgaagaaact gaacgtttac tcgcaaccga tgtccgatgg gcgtaaggca 2520
   ttggtactga ggttgaatca aactgacaaa gataaggagg agatgaagga catcctggaa 2580
   atgaagagga tacacataaa atcgataaag agacgcaagg acaagctgca gaaggcgaaa 2640
   gaagacgctg aaaagctcca gcagcagatg gccgataagg acaataagga caaagccgtt 2700
   attatacaat gagaattttt ctatgtatat aataaagcgc tcttgtaaat atattc 2756
<210> 15
   <211> 2712
   <212> DNA
   <213> Megachile rotundata
<400> 15
   atggggaaca cggagagtaa cgtaactagc ggcgtgaaga aacaaacaga ctcctcttct 60
   atcttacttt acaagctggt cgatttaaaa ggtggtggtt tattagtgga catgatgaaa 120
   agagcaacgc aaactaaaca gtacgctgaa ctagatcacg ctctaagaac aaaagtggag 180
   ccgtatttat acaataaggg taaaggtaaa tggatcccga tagaaaagtt ggttctactg 240
   cggaataaag atcgtccgaa gcataaaatg cttccaccat tgagggctat ggagaatccg 300
   gctgattacg acatagacaa ggatatgggc gaggaagaag tggacgaaac gaagatagat 360
   aaaagcaagt acagattggt ctgctggagc ttgagtcaaa gaggagcagt aggagagacg 420
   attatgcatt tatgcatgtt acatgcgact gctattcaca ccgatctggc taaacgttta 480
   ctacgtttct atcctaagct catcaatgac atctatatca gtgacgaata ctatggtgaa 540
   aatgcattac acatcgctat agtgaacgaa gatccagcta tggtaaagtt ccttctagac 600
   agcggcgccg acgtgcacga aagatgcgta ggaaatttta tgtgtccgga ggatcagaaa 660
   gcgtcgagaa cagacagcgt ggaccacgaa tgggtttgcg tggctcccga aacgaattac 720
   agcggatacg tttactgggg tgaatatcca ttaaacttcg ccgcgtgttt aggccaagaa 780
   gagtgctaca gattgatact cgctagagga gcagatcccg acaagcaaga caccaatgga 840
   aacacggttc tgcacatgtt ggtgatctat gaaaagatgg ccacgttcga tatggcgtac 900
   gaggtaggag catcgttatc gatcagaaac gcgcaacatt tgacaccgct gacgttatcg 960
   gcgaagctgg caaggatcga aatgtttttt cacattttga acatcgagag ggagatttat 1020
   tggcagattg gcagcattac gtgtgcggct tatcccctct cgcaagtgga cactatcgat 1080
   gtgaacactg gtagcatcag ccacacttcc gccctgaatc tggtggtctt tggagaaaag 1140
   gatgagcact tggaactaat ggatggtacc atggttgacc tattaaacgc gaagtggaac 1200
   acctttgtca agtccagatt ttatcgccag ttcttcctat tttgttttta ctttattctt 1260
   tacttgatca gtttcactct tcgacctggt ccttcggtca catcgactgc tacaactgct 1320
   ccgactacag aatcgaccac accgaaattg ccagatcctc ctccaaattt tattccagtt 1380
   acgaatcaaa aaacgtttct cgaagcgagc ctctcgtcgt ccttggatga aattgtgaca 1440
   aacgccctga atttaaattt cccttcgaac gcgacggctc gctttgacga tctaaaactc 1500
   gatctcgtaa cccacataat gtctaatctg aagaacatat tgaatgggaa cgagaccaaa 1560
   aacgagccat ttaacagctc ccaactcgcc aggtttgaca aacatttatt ccgcgaaaat 1620
   gacactgatt attctactag catcttgaac gagaccgccg acgttgattc cacgacgaag 1680
   ttttcgtttg acaattctgt actgtttgag gctgataata aaagcgattg gtggactgat 1740
   cttaccacag aatgccgatt aatgcaactg accaccgcat ccgcgaagat ccgattaatc 1800
   gcagaaatat ttatggaaat cgcagctatt ctttacattc ttgcagcgct aagagaggcc 1860
   aggtttctag gtctcaacat gttcatcgaa aatctgatga ctgtaccatc gagagtaatg 1920
   tttctcttct cctgttgcat actgctctct tttccgtttc tgagattatc ctgtgctgac 1980
   gaagtggaag atgtgctagc agtggtagta atgttgacta cagcaccata ttttttgttc 2040
   ttctgcagag gtttcaaaac ggtcggccct ttcgtcgtga tgatttatag aatgatcatg 2100
   ggcgacctac ttcggttcgt atccatctat ttggtctttg taattggatt ttctcaagct 2160
   tattacatca tatttctatc ttttgataat ccaaacacgc cagaaggtgt tgatgattca 2220
   gtgagcaatc caatgccatc accaatagaa agcatcatgg caatgtttct aatgagtatg 2280
   acgaactttg gagattacta tggtgcattt gagaggaccc gacatgaaat ggaagccaag 2340
   tttctgtttg ttgtatacat ggctattgtt gctatcttac ttgtgaacat gttgattgct 2400
   atgatgggga atacgtatca aaaaattgca gaaactagga atgaatggca gagacaatgg 2460
   gcacgtatag ttttggtagt agaaagagga gtaagtcctc aagaaaggct taaaaaattg 2520
   atggattatt ctcaaccaat gtctgatggc cgcagagcat tggtgctcag attaaatcaa 2580
   tcggaagaag ataaggaaga aatgaaggag attttggaaa tgaagaggac tcatgagaga 2640
   ttatataaaa agaggcaact taaaacaaca aaagaaaaga ctacaataac agaggagaat 2700
   gttactttat ag 2712
<210> 16
   <211> 2664
   <212> DNA
   <213> Apis mellifera
<400> 16
   atgggaaata cggaaagcaa cgtagccagc ggagtgaaga aacagacgga cgcatcgtcc 60
   attttgcttt acaaattggt cgacttgaaa ggtggtggcc tattggtgga tatgatgaaa 120
   agggcaacgc aaactaaaca gtacgcggaa ttggaccatg ccctgagaac aaaagtagag 180
   ccatatttat ataacaaggg taaaggtaaa tggatcccga tcgagaaatt ggttttgctg 240
   agaaacaaag atcgtccaaa gcacaaaatg cttccaccgt tgcgagctat ggaaaatccg 300
   gccgattacg acatagacaa agatatgggc gaagacgagg tggacgagac gaaaatagat 360
   aaaagcaagt acagattagt gtgctggagc ttgagcgaga gaggagcggt cggtgaaacg 420
   attctacatt tgtgcatgct acacgcaact gctattcaca tcgatttggc taaacgtttg 480
   ctacgttttt atcccaagct tatcaacgat gtgtacataa gcgacgagta ttatggcgag 540
   agcgcgttac acatcgcgat tgtaaacgag gacccgtcaa tggtaaaatt ccttttggat 600
   agcggtgcgg acgtgcacga gagatgcata ggaaacttta tgtgtccaga ggatcaaaaa 660
   gcgtcgaggg cggacagtct tgatcacgaa tgggtttgcg taactccgga aaccaattac 720
   aacgggtacg tttactgggg cgagtatcct ttaaattttg ctgcgtgcct gggacaggaa 780
   gagtgttaca gattgatact cgcaagggga gccgaccccg acaagcaaga tacgaacgga 840
   aataccgttt tacacatgtt ggtcatttac gaaaaattgg ccacgttcga catggcgtac 900
   gaggtgggag catcgttggc catcaggaat gcccaacact taacgccctt gacattgtcg 960
   gcgaaattgg cgaagatcga gatgtttttc cacattttaa acatcgagcg agagatttat 1020
   tggcagatcg gaagcataac gtgcgcagct tatcctctct cgcaagtcga caccatcgat 1080
   gtcgacacgg gaagcattag tcataattcc gctctaaatc tggtcgtatt cggcgaaaaa 1140
   gatgagcatt tggaattgat ggacggaatc ctggtcgatc tgttgaacgc caaatggaac 1200
   acgttcgtca aatttcgctt ttaccgccaa tttttcctct tttgcttcta cttcgtcctg 1260
   tcgttgatca gtttcaccct tcgacccggc ccagcaacaa catcgtccgt atccaatcct 1320
   cagatcacgt ccaccgaatt gcccacaatt cccccaaaaa tggatccacc cgtccacaat 1380
   tccgatctcc ccctcctcct tgacaaaatt ctttccaccg ctctcgtctc gaaaaggtat 1440
   ccttggaatt tgacgagaac gcgtctcgac aagctgaagc tcgatatcgt ggagaacttg 1500
   acatccgctt tgagcgatat gctcgggaca taccgaaacg agatcaaatt gtttaaccgt 1560
   tctgacaaaa cgtccatcta ctcgtctaga aacgacgcta aaccgatcga cgagactgtc 1620
   ctccggataa attcgacgat cctgttcaac ggcgatggga tatcgtttac cagcaagtac 1680
   aattggtgga acaatcttac agaggaatgc agactcatgc atctagatac gttatcgaca 1740
   aagattagat tgactgccga ggtattaatg gaaattgcgg ccactcttta tatttttgca 1800
   gcattgcgcg aagccagatt tctaggtctt aatatgttta tagaaaatct aatgaccgca 1860
   ccgtccagag tgatgttcct cttctcatgc tgtatattgt taaccttccc gtttctaaga 1920
   ttgatctgcg ccgacgaaat tgaagatatg ttagccgtgg tcgtcatgct caccacggca 1980
   ccgtattttc tattcttctg cagaggtttc aaaacggtcg gcccattcgt cgtgatgatt 2040
   tacagaatga tcatgggcga tctgctacgt ttcgtctcca tttacttggt cttcgtgatg 2100
   ggattttccc aagcatacta cattatattt ttatctttcg ataatccaaa cacaccggaa 2160
   ggagtagacg attcgatgag caatccgatg ccatcaccaa tagaaagtat aatggcgatg 2220
   tttcttatga gcatgacaaa ctttggagat tattatggtg cttttgaaag aacgcaacac 2280
   gagatggaag ccaaattctt atttgtagtt tacatggcta tcgttgctat attattggta 2340
   aatatgttaa ttgccatgat ggggaataca tatcagaaaa ttgcagaaac gagaaatgaa 2400
   tggcaaagac aatgggcacg catagtttta gtagttgaaa gaggagtgag ccctgatgaa 2460
   agacttaaaa aattaatgga ttattctcaa ccaatgtctg atggtcgaag agcattggta 2520
   cttcgattga atcaatcgga agaggacaaa gaagaaatga aggaaattct agagatgaaa 2580
   agaactcatg ataaattata caaaagaaga cagagcaaaa caatgaaaaa gattttaaca 2640
   tcagaagata atgttatttt ataa 2664
<210> 17
   <211> 2739
   <212> DNA
   <213> Apis florea
<220>
   <221> modified_base
   <222> (1240)..(1240)
   <223> a, c, t, g, unknown or other
<400> 17
   atgggaaata cggagagcaa cgtggccagc ggtgtgaaga aacagacgga cgcgtcgtcc      60
   attttgcttt acaaattagt cgacttgaaa ggaggtggtt tattggtaga tatgatgaaa     120
   agggcaacgc aaactaaaca atacgcggaa ttggatcacg cactgagaac aaaagtagaa     180
   ccgtatttat ataacaaggg taaaggtaaa tggatcccga tcgagaaatt ggttttgttg     240
   aggaacaagg atcgtccaaa gcacaaaatg cttccaccgt tgcgggctat ggaaaatccg     300
   gccgattacg acatagacaa agatacgggc gaagatgaag tgagggacga gacgagaata 360
   gataaaagca agtacagatt agtatgctgg agcttgagcg agagaggagc agtcggtgaa 420
   acaattttac atttgtgtat gctacatgca actgctattc acatcgattt ggctaagcgt 480
   ttgctacgtt tttatcccaa gcttatcaac gatgtttaca taagcgacga gtattatggc 540
   gagagcgcat tacacatcgc aatcgtgaac gaggatccgt caatggtgaa attccttttg 600
   gatagcggtg cggacgtgca cgaaagatgc ataggaaact ttatgtgtcc agaggatcaa 660
   aaagcgtcga gagcggacag tctggatcac gaatgggttt gcgtaactcc ggaaactaat 720
   tacaacgggt acgtttactg gggcgagtat cctttaaatt ttgccgcgtg tttgggacag 780
   gaagagtgtt acagattgat actcgcgagg ggggctgacc ccgacaaaca agatacgaac 840
   gggaacaccg ttttacacat gctggtcatt tacgaaaaat tggcgacatt cgatatggcg 900
   tacgaagtgg gcgcatcgtt agcgatcagg aatgctcaac acttaacgcc cttgacgttg 960
   tcggccaaat tggcaaagat cgaaatgttt tttcacattt tgaacatcga gcgagagatt 1020
   tattggcaga tcggaagtat aacgtgcgca gcttatcctc tctcgcaagt cgacaccatc 1080
   gatgtcgaca cgggaagcat tagccacaat tccgctctaa atctggtcgt gttcggcgaa 1140
   aaagacgagc atttggaatt gatggacggg attctggttg atctgttgaa cgccaagtgg 1200
   aacacattcg tcaaatttcg gttttatcgt caatttttcn ctttttgttt ttactttgtc 1260
   ctgtcgttga tcagtttcac ccttcgacct ggcccagcga cgacatcatc cgttcccaac 1320
   cccgtcgctc aaacgcccct tacgtccacc gaattgtcca caatttcccc aaaagtggat 1380
   cctcccgtct cgaacgttac tgtcaatcga agctcgaatg ctttccaaca cgattccaat 1440
   ctctccctcc ttcttgataa aattctttcc actgcaagcg taaaatatcc ttggaacgtg 1500
   acaagaactc gtctcgataa gctgaagctc gatatcatcg agaatttgac aaccgctttg 1560
   aacaatctac tgtcgacaca ccgagacgag atcgaatttt ttaaccactc ttacaaaaca 1620
   accgaatatt tccattctac gagaaacgat accacgccga tcgataattt gaaaattacg 1680
   atcaatgaaa ctgtcctttc ggtaaattcg acgattctgt tcaacgatgg gatatcgttt 1740
   gccactggca aatacaactg gtggaacaat cttacagaag aatgtagact aatgcatgta 1800
   gatacgttat cgacaaagat tagattgact gctgaagtat taatggaaat tgcggccact 1860
   ctttacattt ttgcagcatt gcgagaagcc agattccttg gtcttaatat gttcattgaa 1920
   aatctaatga ctgcaccgtc cagagtgatg tttctctttt cctgctgtat attgttaacc 1980
   ttcccatttt taagattgat ttgcgccgac caagttgaag atatgctagc tgttgtcgtt 2040
   atgttaacca cggcaccgta tttcttattt ttctgtagag gtttcaaaac ggtcggccca 2100
   ttcgtcgtga tgatttacag aatgatcatg ggcgatctcc tccgtttcgt ctccatttac 2160
   ttggtcttcg tgatgggatt ttcccaagca tactatatta tatttttatc tttcgataat     2220
   ccaaatacac cggaaggagt agatgattcg atgagcaatc caatgccgtc accaatagaa     2280
   agtataatgg caatgtttct tatgagcatg acaaactttg gagattatta tggtgctttt     2340
   gaaagaacgc aacacgagat agaagccaag tttttatttg tagtatacat ggcgatcgtt     2400
   gctatattat tggtaaatat gttaattgcc atgatgggaa atacgtatca gaaaattgca     2460
   gaaacaagaa atgaatggca aagacaatgg gcacgtatag ttttagtagt tgaaagagga     2520
   gtgagccctg aggaaagact taagaaatta atggattatt ctcaaccaat gtccgatggt     2580
   cgaagagcat tagtacttcg attgaatcaa tcggaagagg acaaagaaga aatgaaagaa     2640
   attctagaga tgaaaagaac tcatgataaa ttatacaaaa aaagacagag caaaataatg     2700
   aaaaagattt taacatcaga ggataatgtt attttataa                          2739
<210> 18
   <211> 2472
   <212> DNA
   <213> Pediculus humanus
<220>
   <221> source
   <223> /note="Pediculus humanus corporis"
<400> 18
   atgggaaata cagagagtaa cgttacgagt ggtgtaaaaa aacaagcggg aacatccgaa      60
   caaccattgt acaaattagt cggtttaaaa ggtggaggtc ttttggtcga catgatgaaa     120
   agagcgacac aaacgaaaca atatgctgaa ttagatcacg cgatcaaaac aaaaattgaa     180
   ccttttttat ataacaaagg aaaaggaaga ttgataccca tatcacagtt ggtactttta     240
   agaaactgcg aaagatctag aacaaaattg cttccgcttc ttaaaaacat ggaagatcca     300
   gaatctgaat ttgacatgga tcgtgatgga aatcctggag attttcaacc ggctccagat     360
   ggcgtacaag aagattttca cgattacaat aaaaaatata gagacgtttg ttgggatttg     420
   aaaaaaaggg gagccgtggg agaaacatgc cttcacctgt gcatgcttaa tgccacgtca     480
   atccacgcgg atttagctaa aagattatta aaattttatc cgaaacttat aaatgacatt     540
   tacatgtcgg acgaatatta cggtgaaagc gttcttcacg tggcaatagt aaatgaagat     600
   cctgccatgg ttaaatattt actcgatagc ggttctaatt ttcacgaacg ttgttttgga     660
   aatttcatgt gtccagaaga tcaaaaagca acaagaacgg attctctcga tcatgaatat     720
   gttaatttat caacggaaac taattacgaa ggatacgttt attggggaga atatccgtta     780
   agttttgcag cttgtttggg acaagaagaa agttatcgtt tgatgcttgc caagggtgcc     840
   aatcccgata atcaagatac aaatggcaac actgttttac acatgctcgt catacataat     900
   aaattgccca tgtttgacgt ggcttatgaa gttggcgcca atttatcgtt aaaaaatgca     960
   aaatgtctaa cgccacttac acttgcggca aaattagcaa gagtcgaact tttttttcat 1020
   attttaaaca tagaaagaga aatttattgg caaataggga gcataacgtg tgcggcatat 1080
   ccgttatcac aaatcgatac gattgatatc gaaaccggaa acataagtaa aaattcagcg 1140
   ttaaatttag tcgttttcgg agaaaaagac gaacatttag aactcatgga tggagtattg 1200
   gtagatttat tagtcgcaaa atggaacacc tacgttaaat tcagattcta caggcaattt 1260
   atatcttttt ttttctattt tcttatctct gttatctgtt ttacacttcg acccggtcct 1320
   ccgacaggaa caacaacaac aacaaacggt atcgtcgttt taaataataa taacaacaac 1380
   ataacgtccg ttgttgataa ttcaaataac gttaccacca ccaccgccac aactactgcc 1440
   acgtcatatt cttttcctgg tcacgtgata gtgacacttt tatcgtatga taaatacgaa 1500
   gataaaatac gtataatatc agaattagcg atggaaatcg gatctgtttt gtatttgtta 1560
   ggagcattga gagaagctaa atttttaggg attcacatgt ttattgaaaa tttgatgaca 1620
   gctccgtcga gagttttatt tttaggttct tgttgcataa tgcaattcgt accttggctc 1680
   aggatcacgt gtaaagaaga aacggaagat atcgttgccg ttatcattat gttaacgacg 1740
   gctccatatt ttttattttt ttgcagaaaa atgaacgacc ccgtttttct ttttcttttt 1800
   tttttaataa aaaaaatttt ttttctaatt tttttttttt ttttttttac aaagccaact 1860
   tattatataa tattcctttc gtttgataat ccaaaaactc ccgatggagt cgacgattct 1920
   ggaacaaatc caatgcaaag cccagttgaa tccataatgg caatgttttt aatgtctctg 1980
   acaaattttg gagaatatta cggtgcattc gaaaagactg aacacgaatt tgttgcaaag 2040
   ttaatgtttg tcgtttacat ggctatcgtt gctatattac tcgttaacat gttgatcgcc 2100
   atgatgggta atacgtatca aaaaatagca gaaacgagaa acgaatggca aagacaatgg 2160
   gcaagaattg ttttggttgt tgaaagaggt gtgagtcctc aagaaaggtt aaaaaaatta 2220
   atgctttatt ctcagcctat gtctgacgga agaagagcat tagttttacg attgcatcaa 2280
   acgccagaag acaaagaaga aatgaaagaa atattagata tgaaaagaat acacaataga 2340
   acggttcaaa ggcgaaaagc taaagaaaat aaaaatgtca cgtttgctgg taccccctaa 2400
   aatggcatta aaaatcgttt gctcggtccc gttcccccat taaaaagcat accgtcaaaa 2460
   cctaaaattt aa 2472
<210> 19
   <211> 1068
   <212> DNA
   <213> Danaus plexippus
<400> 19
   atgctggagt caggaggtgg tttacttgtg gacatgatga agagggcggt tcagaataag 60
   cagtacgctg agatagacca cgctatcaag actaaggtgg agccctttct ctacaataag 120
   ggcaagggca ggcatatccc tatatcgcat cttgtgctgt tgaggaataa ggaacgaggg 180
   aggcataaat tgctgccacc gttacgtggc atggagaatc ctgacgaaga gtttgatgta 240
   gagaaggatt ggccagttgt gacccaggaa gaatacgagg cgaatccctc ggcttacagg 300
   gagctttgct gggacctgaa ggaacgcggt gccgtcggtg agacaatcct tcacctttgt 360
   cttctaaacg ctacctcact tttagctgat ttagccaaga gattgcttcg tttttaccca 420
   aaacttataa acgacattta catgagcgac gagtattacg gtgagagcgt acttcatatg 480
   actattgtaa acgaggaccc gactatggtg aaatttcttt tggattccgg agctgattat 540
   catgaacgtt gcttcgggaa cttcatgtgt cctgaggatc agaaggcctc ccgaacagat 600
   tccttcgacc acgagtgggt caacgtccag ccggacacca actatgacgg gtacgtatat 660
   tggggggaat accctttaag tttcgcggcc tgtctcggtc aggaggagtg ctacaggctc 720
   atactggcaa gaggagcgaa ccccgacaaa caggacacca atggcaacac cgtactgcat 780
   atgctgatca tttatgataa aataagcaca ttcgatatgg cgtatgaagt tggtgcatcc 840
   ctaaacatca ggaacgttca gaacctaaca ccgctcaccc tggccgctaa actggcgaga 900
   gttgagctgt tcttccatat cctgaacatt gaaagggaga tctattggca aatcggagcc 960
   accacctgtg cagcgtatcc tctgggacaa gttgacacta ttgacaccga gaccggaagg 1020
   attagtaaag attcggcgct gaatttagtt gtatttgggg ttagttag 1068
<210> 20
   <211> 2583
   <212> DNA
   <213> Solenopsis invicta
<400> 20
   ggcggattgt tagtggatat gatgaagaga gcctctcaga ccaagcagta cgcggagttg      60
   gatcacgcga tgagaacgaa ggtcgagccg tttttgtaca acaaaggaaa gggcaaatgg     120
   atacccatag aaaaattggt cctcctacga aataaggatc gtccgcgaca taaaatgcta     180
   ccacctttga aagccatgga aaatccagcg gattacgata tcgataagga tatgggcgat     240
   gaggaagtcg acgaagctaa gatagataaa agcaaatata ggctggtgtg ctggaatctg     300
   agcgacagag gtgccgtcgg cgaaaccatt ttgcatttgt gcatgttgaa cgcgacagcc     360
   atccacgccg atttggcgaa acgactgttg cgtttctatc ctctgcttat atacgatatt     420
   tatttgtgcg acgagtatta cggggagaat gtcttgcaca tcgctatcgt taacgaggac     480
   ccgtcgttgg tcaagtttct tctggatagt ggagcggacg atccgcgcat tatccacgaa     540
   aggtgcttcg gcaccttcat gtgtccggag gaccagaagg cgtcgaggtt tgacagtatg     600
   gaccacgagt gggtgtgcgt cacaccggag accgattaca atggctatgt gtactgggga     660
   gaatatccgt tgagtttcgc cgcatgcctc ggtcaggaag agtgctacag gcttatacta     720
   gccagaggcg cggatcctga taagcaggac acgaatggca acaccgtttt gcacatgctg 780
   gctacgttcg acatggctta tgaggtagga gcttctctcg acataagaaa cgttcaacac 840
   ctaactccgt tgaccttagc agcaaagttg gccaggatcg agatgttctt tcacatcctg 900
   aacatcgaaa gagagatata ctggcaaatc ggtagtataa cttgcgcagc ctacccttta 960
   tcgcaaatag atacgatcga tgttaacacg gggacaatta acaaaacttc tgccctgaat 1020
   ctggttgttt ttggcgacaa agatgaacat ttagagttga tggacggaat gctagtcgat 1080
   ctgctaaacg ccaagtggaa cactttcgtt aaatctcgat tttaccgtca attcttcctc 1140
   ttctgcttcc acttcgtgct gtcgctgatt agcttcactc ttcgccctgg cccgtcgacc 1200
   atggaaacag atgaaaacgc gaacgatacg tcgacgaaga caaattcttc aaatataaca 1260
   gagcccataa ttctcaagcc cctccaaagc tcggatctgt cggaactggt tacgaatagc 1320
   ttcgtggcgg cttttgcgtc gaatctcaag tcatccttaa acatgacgca ggaatcattg 1380
   gagaaaatcc agctgcaagt aacatcgaat atcacatcgg ccttgaagag cattttgctc 1440
   ttgacgttga acgaaaacga gggcattctg agtcctcctg acgaggccac catccatacg 1500
   ccgtggtata ccgacagctc tccaaatacg tcggactatt tcacagatct ctacaataaa 1560
   actgtcgtag ccgaaaattc gactgtcaag gcagttgtcg atgacgtcgc gcaaaccgat 1620
   tactggtggg acgatttcgc cgaggagtgt agactgatgc agctaacaga aacgagtgcg 1680
   aagattcgtc taactgcgga aacttttatg tacatcgcag cggtgcttta tatcttggct 1740
   gcattgaggg aggcgagatt tttaggtctc aacatgttca tcgaaaatct cgcgacggcg 1800
   ccatcgcgcg tgatgtttct gttctcgtgc tgtatcctgc tgtcgttccc atttttgcga 1860
   tttgtctgcg cggacgagat cgaagacata ctggcggtcg tggtgatgct gacgaccgcg 1920
   ccgtactttc tgttcttctg ccggggcttc aagacggtcg ggccgttcgt cgtgatgatt 1980
   tacaggatga tcatgggcga cctcctcaga ttcgtctgca tctaccttgt cttcgttatg 2040
   ggattctccc aagcgtacta catcatattc ctgtcgttcg acaatccgaa cactccggag 2100
   ggcaccgacg actcgataac gaatccgatg ccgtcgccga tggagagcgt catggcgatg 2160
   ttcctgatga gcatgacgaa cttcggagat tactttggcg cgtttgaaag aaccgagcac 2220
   gagttcgagg ccaagctgct gttcgtgttg tacatggtga tcgtggcgat cctgctcgta 2280
   aacatgttga tcgccatgat gggcaacact taccagaaga tcgccgaaac caggaatgaa 2340
   tggcagcggc aatgggcgcg gatagttttg gtcgtggaga gaggtgtcag tcccgctgaa 2400
   aggctgaaaa aattgacaga ctattctcag cccatgtccg acggccgtcc tgcgttggtt 2460
   cttcgattac atcaaactga ggaggacaag gaagagatga aggagatact cgagatgaag 2520
   agaacccacg aaaaattgct gaagaaaaga caatgcagga tgtcaaagga gaagattctg 2580
   tct 2583
<210> 21
   <211> 2586
   <212> DNA
   <213> Acromyrmex echinatior
<400> 21
   atgggtaata ccgagagcaa tgttgcgagc agcgtgaaga aacaaacgga tacgtcgtcc      60
   atcctgctct ataaattagt cgacttgaag ggtggcggat tgttggtgga catgatgaaa     120
   agagccacgc agactaagca gtttgcagag ttggatcacg cgatgagaac caaagtagaa     180
   ccgtttctgt acaacaaggg aaaaggcaag tggataccag tggacaaatt agttcttcta     240
   cgaaataaag atcgtccgcg gcataaaatg ctaccgcctt taaaagctat ggaaaatcca     300
   gctgattacg atatcgataa agacatgggc gacgaggaag ttgatgaggc caagatcgat     360
   aaaagcaaat atagactggt atgttggaat ttaagcgaca gaggtgctgt tggcgaaact     420
   atcttgcatt tgtgcatgtt gcacgcgacc gccattcatg ctgatttggc aaaacgactg     480
   ttgcgtttct atccgaagct tataaacgat atttatctaa gcgatgagta ctatggagag     540
   aatgtcttgc acatcgctat tgtaaacgag gatccatcga tggttaagtt ccttctcgac     600
   agcggagcag acgatccgtg cattatccac gagaggtgct tcggcaactt catgtgccca     660
   gaagatcaga aggcgtcgag gtttgacaat atggaccacg agtgggtgtg tgtttcaccg     720
   gaaaccaatt acaacggtta tgtgtactgg ggagaatatc cgttaagctt cgccgcatgt     780
   ctcggtcagg aagagtgcta caggcttata ttagccagag gtgcagatcc tgataagcag     840
   gacacgaatg gcaataccgt tatgcatatg ctagccacgt tcgacatggc ttatgaagta     900
   ggagcttctc tcggcataag aaacgttcaa catctgactc cgttgacttt ggctgcaaag     960
   ttggccagga ttgagatatt ctttcacatc atgaatatcg aaagagagat atactggcaa    1020
   atcggtagca taacttgcgc agcctaccct ttatcgcaaa tagatacgat cgacgttaat    1080
   acgggcacaa tcaacaataa ttccgccctg aatctggttg tttttggtga taaagatgaa    1140
   catttagagt taatggacgg tgtgttagtc gatcttttaa atgccaagtg gaacaccttc    1200
   gttaaatccc gattctacaa tcaattcttt cttttctgct tctacttcgt actatcgctg    1260
   attagcttca cccttcgacc tggcccatca accatagaaa cagacgaaaa tgcaaatgat    1320
   acgtcgacga ggacaaattc ctccaacata acggagccta taattcacaa gactctccaa    1380
   agctcggatc tgtcagaatt ggttacgaat agtctcgtag cggcttttac ctcgaatttt    1440
   aaatcatcct taaatatgac gcaggaatcg ctagaaaaga tccagctaca agtaacgtca    1500
   aacatcacgt cagccctgaa gagcattttg ctcttgacgt tgaacgaaaa tgagggcatt    1560
   ctgagtcctc ctgacgaggc caccattcat acgctgtggt ataccgatag ccctccaaac    1620
   gcgtcggatt attttatgaa tacttacaac aaaaccgacg cgaattcaac tgtcaagaca    1680
   attatcgacg gcgtcgcgcg accaaaattt aaaagcaatt attggtggga cgatttctcg 1740
   gaggagtgca gactcatgca gctatcagaa atgagtgcaa aaattcgtct aactgcggaa 1800
   atttttatgt tcatcgcagc ggtgctctac attttggctg cgttgcggga ggcgaaattc 1860
   ttaggtcttc atatgttcat cgaaaatctc atgacggtgc cgtcacgcgt gatgttcctg 1920
   atctcgtgct gcatcctgat gtggttcccg gttctgcgat ttacttgctc cgacgaggtc 1980
   gaagatatgc tagcagtcgt agtgatgctg acgacggcgc catacttttt attcttctgc 2040
   cggggcttca agacggtcgg accgttcgtc gtgatgattt acaggatgat catgggtgat 2100
   ctcatcagat tcgtctctat ctacctcgtt tttgtcatgg gattctctca agcatattac 2160
   atcatattcc tgtcattcga caacccgaat actccagagg gcatcgacga ctctatcacg 2220
   aatccgatgc catcgccgat ggaaagtgtt atggcgatgt tcctgatgag catgacgaat 2280
   ttcggtgatt actttggcgc gtttgacaga accgagcatg aattcgaggc caagctgctg 2340
   tttgtattgt acatggcgat tgtggcaatc ttgctcgtga acatgttgat cgctatgatg 2400
   ggcaacactt accagaagat cgctgaaacc aggaacgaat ggcaacggca agaggaagac 2460
   aaggaagaga tgaaggagat actcgagatg aagagaaccc atgatagatt gctgaagaaa 2520
   agacaaaata gaatgtcaaa ggaaaagatt atatttgaaa cagaacctat tattaccaag 2580
   aattga 2586
<210> 22
   <211> 2715
   <212> DNA
   <213> Camponotus floridanus
<400> 22
   atgggtaata cagagagtaa tgttacgagc ggtgtaaaga aacaaacgga cacatcgtcc      60
   atcgtgcttt ataaattagt cgatttaaaa ggtggtggtt tgttagttga tatgatgaaa     120
   agagccactc agacaaagca gtttgcggaa ttagaccatg cgatgagaac gaaagtcgag     180
   ccctttttat ataacaaggg ggaaggcaaa tggataccta tagacaaatt ggtcctctta     240
   cgaaataaag atcgtccgcg acacaaaatg ctagcaccgt taaaagctat ggaaaatcca     300
   gcggattacg atatagacaa agatatgggc gatgaggaag ttgatgaaac taagatagat     360
   aaaagcaaat atagattagt gtgctggaat ttgagtgaga gaggtgctgt cggcgaaact     420
   attttgcatt tatgtatgtt aaacgctact gctctccacg ctgatttggc aaaaagacta     480
   ttacgtttct atccgaagct tataaacgat atttatctat gcgacgaata ttacggtgaa     540
   aatgtattac atattgctat tgtgaacgag gatccatcga tggtcaagta tcttctcgac     600
   agcggcgcgg acgacccgcg cattatccac gagagatgct tcggtaattt tatgtgtccg     660
   gaagatcaga aagcatcgag gtttgatagt caagatcacg aatgggtgtg cgtcacacca     720
   gaaaccaatt ataacggata cgtgtattgg ggagagtatc ccttgagctt tgccgcatgc 780
   cttggtcagg aagaatgcta cagacttata cttgccagag gtgcggatcc ggataaacag 840
   gatacgaatg ggaacaccgt tttacatatg ctggctacgt tcgatatggc ctatgaagtg 900
   ggggcttctc tagacataag aaacgttcaa catctgactc cattgacttt ggcagcaaag 960
   ctagctagga tagagatgtt ctttcacatt ctgaatatcg agagagagat atactggcaa 1020
   ataggtagca taacttgcgc agcctatcct ttgtcgcaaa tagatacaat agacgttaat 1080
   gcaggaacaa tcaacaataa ttctgctcta aatttggttg tttttggcga gaaagatgaa 1140
   catttagagt taatggacgg cgtcctaatc gatcttttaa atgccaagtg gaacactttt 1200
   gttaaattcc gattctatcg tcaattcatt ctcttctact tctactttgt gctatcgctg 1260
   attagtttca ctcttcgtcc tggcccgcaa acagatgaaa atgcgaatga tacattgaca 1320
   aactcattca acacgacgag ggaatccatg attctcaagt ctcttcacag ttcggatcta 1380
   tcagaactgg ttacaaatag tttcacagca gcttttactt cgaacttcaa atcatcctta 1440
   aacgtaacgc aagagtcatt ggagaagatc cagctgcaag tgacgtcgaa tgttacatct 1500
   gctctgagaa gcattttgct tttaacgttg agcaacaatg aggatctttt gagttcccct 1560
   gatgaggccg ccattcatac actgtggtat accgataatc ctcaaaacgc gtcggattat 1620
   ttcataaata tgtataataa aactgttaca attgatacta actcaaccgc caaaactacc 1680
   atcgacgatg tcacactatt aggaaaattt aacaatcacg aaaaatggtg ggatgatctt 1740
   acagaagaat gcagactgat gcaattgaca acaatgagcg cgaagatccg tcttactgcg 1800
   gaaatcgtga tgtattttgc ggcagtgctc tacatcttgg ctgcgttgcg agaagcgaga 1860
   tttttaggtc tcaacatgtt cattgaaaat ctcatgacgg cgccgtcgcg cgtcatgttt 1920
   ctgttctcgt gttgcatctt aatgtcgttt ccggttctga gactttcctg ctccgatgag 1980
   gtcgaagacg tgctggcagt tgtagtgatg ctaacaacgg cgccatattt tttattcttc 2040
   tgccgaggtt tcaagacagt cggcccgttt gtcgtgatga tttacagaat gattacgggc 2100
   gatctcctca gattcgtctc tatctatctc gtcttcgtga tgggattctc tcaagcgtat 2160
   tatatcatat ttttgtcgtt tgacaatccg aatactccgg aaggcgttga cgattcgata 2220
   acaaatccta tgccatcgcc gatggagagc gttatggcaa tgtttctgat gagcatgaca 2280
   aatttcggcg attattatgc cacgtttgac agaaccgagc atgaattcga agccaagctg 2340
   ctgttcgtgt tatatatggc gatcgtggca atcctgctcg taaacatgtt gattgctatg 2400
   atgggcaaca cttaccagaa gatcgctgaa accagaaatg agtggcagag acaatgggcg 2460
   cgcatagtct tggttgtgga gagaggcgta agtcctgctg aaagattaaa aaaattgatg 2520
   gactattctc agcccatgtc tgacggtcgt aaagcgttgg ttctccgatt aaatcaaact 2580
   gaagaagaca aggaagaaat gaaagagata cttgaaatga aaaggacgca cgataaatta 2640
   ataaagaaaa gacaagacaa aatattaaag gagaaagctt tagctgaaac agaagctgtt 2700
   attactagaa aataa 2715
<210> 23
   <211> 2550
   <212> DNA
   <213> Myzus persicae
<220>
   <221> modified_base
   <222> (210)..(638)
   <223> a, c, t, g, unknown or other
<220>
   <221> modified_base
   <222> (899)..(984)
   <223> a, c, t, g, unknown or other
<220>
   <221> modified_base
   <222> (1863)..(1883)
   <223> a, c, t, g, unknown or other
<400> 23
   cgcgatcaaa acacaagtgg aacccatgct gtacaacaag ggtgaaggca gaatgatacc       60
   gattgccaaa ctcgtgttac tgagaaatcg cgaccgttcc cgggcaagat ggcttccacc      120
   gttaaagaac atggaagacg ccaatgaata cgatatcgaa aaagacattc ccaccatcga      180
   agaactggaa aagctggggg aagtgcagtn nnnnnnnnnn nnnnnnnnnn nnnnnnnnnn      240
   nnnnnnnnnn nnnnnnnnnn nnnnnnnnnn nnnnnnnnnn nnnnnnnnnn nnnnnnnnnn      300
   nnnnnnnnnn nnnnnnnnnn nnnnnnnnnn nnnnnnnnnn nnnnnnnnnn nnnnnnnnnn      360
   nnnnnnnnnn nnnnnnnnnn nnnnnnnnnn nnnnnnnnnn nnnnnnnnnn nnnnnnnnnn      420
   nnnnnnnnnn nnnnnnnnnn nnnnnnnnnn nnnnnnnnnn nnnnnnnnnn nnnnnnnnnn      480
   nnnnnnnnnn nnnnnnnnnn nnnnnnnnnn nnnnnnnnnn nnnnnnnnnn nnnnnnnnnn      540
   nnnnnnnnnn nnnnnnnnnn nnnnnnnnnn nnnnnnnnnn nnnnnnnnnn nnnnnnnnnn      600
   nnnnnnnnnn nnnnnnnnnn nnnnnnnnnn nnnnnnnntg gcaagaggag ccgatccaaa      660
   taaccaggat accaacggca atacagttct gcacatgctc gtaatttacg aaaaaatctc      720
   gacgttcgac atggcctacg aaatcggggc ggagctcaac attaggaacg tgcaaaattt      780
   gacaccactc acgctggccg cgaaactggc tcggatccaa atgttctttc acatactcaa      840
   aatcgaacgg gaaatttatt ggcaaatcgg cagtatcact tgtgccgcgt accccctgnn      900
   nnnnnnnnnn nnnnnnnnnn nnnnnnnnnn nnnnnnnnnn nnnnnnnnnn nnnnnnnnnn      960
   nnnnnnnnnn nnnnnnnnnn nnnnacactt ggagatgatg gacggtgtgt tggtggatct     1020
   gctaaatgct aaatggaacg ctttcgtgaa attccggttc tataggcagt tcatcttgtt 1080
   cgcgttttac ttttccatat ccatggtgtg cttcgttttg cgtcccgggc cgccacccac 1140
   tggtcttaag ccgccattaa tcaactacac ttcgacgtcg accacggtgg ccaccacgcc 1200
   gtttgacttc atggacaacg agtcgatgtt gcccgacaac gtcatgtacg acgaaatgat 1260
   gatcggcggt ggcggtccga ccgaattcga aacgccggtg gtaaccacgg agatggcgac 1320
   ggtcaatgac aacgccacaa ccaacaacgg taccggtatc atccgtacgc cactgatcta 1380
   cgaggacggt gctggaggtg gtggtggaag tggtaccgta agcttgcacg gtaagagttc 1440
   cggttcggac aggggcaaac acaggagcac gtggaatccc gccaaccata cgttcaagac 1500
   caactacacg tacaactaca acaaatcgag aaaattttgg tggaacaaga gacaaaaggt 1560
   gtgcagatta atggcagtgc attcgactga tgatctaatc agaatggggt cggaagtggg 1620
   aatgtttgtg ggagccttcc tctatctact ggccgcagtc agagaagctg gatttttggg 1680
   ctcccagatg ttcattgaaa acctggccat agcaccggca agagtgatgt ttctgttctc 1740
   gtgcctgctc atgatgacca taccaccact gagactgacg tgctatgaca aagccgaaga 1800
   cataatagcc gtaatcatca tgttgaccac ttgtccgtac tttttgttct tctgcagggg 1860
   ttnnnnnnnn nnnnnnnnnn nnntggtgat gatctacagg ttggtgatgg gagacttgct 1920
   gagattcgtt tccatttaca tggtgttcgt gatggggttc tcgcaagctt attacgtgat 1980
   tttcctatct tacgacaacc cgctgacacc agagggcatt gacgattccg tgctgaaccc 2040
   gatgcccacg ccgaccgagt ccatcatggc gatgttcctt atgtccgtca atacgttcac 2100
   agattactac acggcgttcg acaagactag tcacacgttg gtcgcaaagt tttgctttat 2160
   agttttcatg gtgatcgtgg ccatattgcc ggtgaacatg ttaatcgcca tgatggggga 2220
   cacttatcaa aagatcgccg agaccagaaa cgaatggcag agacaatggg ctcggatcgt 2280
   actggtggta gagagaggtg tcagcccgtc acagaggctc aaaaagctca tgtactactc 2340
   gcaaccgatg tcagatggtc ggagggcgtt ggtgttgcga ctcaatcaga cggatgaaga 2400
   caaggagcaa atgaaagaga tattggaaat gaaacgcatt cacaatcgct atgttgaaag 2460
   aatgcgcgca agagacctga gcagcaaact atcgtcttcg tgcggaaaaa atgcaaacga 2520
   tgggacaatt aatttaaaag caaataaata 2550
<210> 24
   <211> 2502
   <212> DNA
   <213> Bemisia tabaci
<220>
   <221> modified_base
   <222> (877)..(1717)
   <223> a, c, t, g, unknown or other
<400> 24
   gatcctgaga aaattgatcc gaagaaatgc cgagaggtat gctgggattt gatggaacgt      60
   ggagcggtgg gtgaaactat tctccatctg tgcctcctca acgccacatc catccatgca     120
   gacttggcca agcgactcct ccgcttttat ccgaaactta tcaacgatat atacatgtgc     180
   gacgaatatt acggtgaaag tgtgctacat attgccatag tgaacgaaga tccagcgatg     240
   gtgaagttcc ttttagacag tggcgctgac taccacgagc gttgtaccgg gaacttcatg     300
   tgtcctgagg atcagaaggc tacaaggcag gacacagttg agcatgaatg ggtgaatctg     360
   tcgccactca ccaactacga gggatacgtg tactggggtg agtacccact tagttttgcg     420
   gcttgtctgg gccaagaaga gtgcttccga ctcatgctcg cgcgaggagc aaatcccgat     480
   aatcaagaca caaatggaaa taccgttctt catatgctcg tcatctatga gaaactcgaa     540
   acatttgaca tggcatacga agttggcgcc aacatttctg ttcggaatgt ccagaatttg     600
   actcctctca ctttggcagc aaaactcgcg agaattgaga tgttcttcca cattttaaac     660
   atcgaacgcg aaatctattg gcaaattggc agtattgcat gtgcagctta tcctctaggg     720
   caaatcgaca caattgacat cgaaacagga cacatcagca aaaattcggc tcttaatctt     780
   gtggtctttg gggacaaaga cgaacatctg gagttgatgg atggcgtttt ggtggacctt     840
   ttgaacgcca agtggaacgc ttttgtcaaa ttgaggnnnn nnnnnnnnnn nnnnnnnnnn     900
   nnnnnnnnnn nnnnnnnnnn nnnnnnnnnn nnnnnnnnnn nnnnnnnnnn nnnnnnnnnn     960
   nnnnnnnnnn nnnnnnnnnn nnnnnnnnnn nnnnnnnnnn nnnnnnnnnn nnnnnnnnnn    1020
   nnnnnnnnnn nnnnnnnnnn nnnnnnnnnn nnnnnnnnnn nnnnnnnnnn nnnnnnnnnn    1080
   nnnnnnnnnn nnnnnnnnnn nnnnnnnnnn nnnnnnnnnn nnnnnnnnnn nnnnnnnnnn    1140
   nnnnnnnnnn nnnnnnnnnn nnnnnnnnnn nnnnnnnnnn nnnnnnnnnn nnnnnnnnnn    1200
   nnnnnnnnnn nnnnnnnnnn nnnnnnnnnn nnnnnnnnnn nnnnnnnnnn nnnnnnnnnn    1260
   nnnnnnnnnn nnnnnnnnnn nnnnnnnnnn nnnnnnnnnn nnnnnnnnnn nnnnnnnnnn    1320
   nnnnnnnnnn nnnnnnnnnn nnnnnnnnnn nnnnnnnnnn nnnnnnnnnn nnnnnnnnnn    1380
   nnnnnnnnnn nnnnnnnnnn nnnnnnnnnn nnnnnnnnnn nnnnnnnnnn nnnnnnnnnn    1440
   nnnnnnnnnn nnnnnnnnnn nnnnnnnnnn nnnnnnnnnn nnnnnnnnnn nnnnnnnnnn    1500
   nnnnnnnnnn nnnnnnnnnn nnnnnnnnnn nnnnnnnnnn nnnnnnnnnn nnnnnnnnnn    1560
   nnnnnnnnnn nnnnnnnnnn nnnnnnnnnn nnnnnnnnnn nnnnnnnnnn nnnnnnnnnn    1620
   nnnnnnnnnn nnnnnnnnnn nnnnnnnnnn nnnnnnnnnn nnnnnnnnnn nnnnnnnnnn    1680
   nnnnnnnnnn nnnnnnnnnn nnnnnnnnnn nnnnnnntga taatgttgct tacgtcacca    1740
   tactttctct tcttttgccg gggattcaag acggtcggac cattcgtcgt tatgatctac    1800
   aggatggtca tgggggatct actccgattc gtttccatct acatggtctt cgtcatggga    1860
   ttctctcaag cctactacat catatttcta tccttcgaca accctagtac accagacgga      1920
   gtggacgaca cggtgagcaa tccaatgcca aacccagttg aggctggaat ggccatgttc      1980
   ctcatgtcac tcaacacttt cggtgactat tttagcgcct ttgaaaaaac cgaacacgag      2040
   ctagaggcta aggtatgctt tgtaatattc atgggtatcg ttgccattct actggttaac      2100
   atgttgattg ctatgatggg taacacttac caaaaaatcg cagaaacccg gaatgagtgg      2160
   cagagacagt gggccagaat tgttttggtt gttgaaagag gagtcccacc gaaagagcgg      2220
   ctaactaaac tcatgtggta ctcacaacct atgtccgatg gcagacgtgc gttggtcttg      2280
   cgcctcaatc agacggagca agacaaagag gagatgaaag agattttaga gatgaagcga      2340
   gtgcacaacc gaatcgtgga gaagcggcaa aagcgactgg caaagctgtc caacaatgca      2400
   agcccctcta acaagcctct agttgacctg ccaaaccttt cgaaccatga ttcccctaca      2460
   aagcctctcc tcgaccttgc cagtaacctc agcaaatttt ga                        2502
<210> 25
   <211> 2428
   <212> DNA
   <213> Euschistus heros
<400> 25
   ctcaggttct accccaagct catcaatgac gtctacatgt ccgatgaata ctatggtgaa      60
   agtgtcctgc atattgccat agtcaacgaa gatcctgcga tggtgaaatt cctactagac     120
   agcggtgtca atgtcaatga acgctgtttt ggcaacttta tgtgtcctga ggaccagaag     180
   gcatcgagga ctgattcact cgatcatgaa tgggtcaact tgcaatcatt tactacttat     240
   gaggggtatg tatattgggg tgagtaccca ctatcctttg cggcctgcct tggacaggaa     300
   gaaygctaca gattgatgct tgcacgagga gctaatcctg acaatcagga taccaacgga     360
   aataccgttc ttcatatgct agtcatatac tgtaaaatac aaactttcga catggcctat     420
   gaagttggtg gagacctatc aataagaaat gtccaatatc taacaccatt gacattggca     480
   gctaaattag ctaggattga actattcttc cacatattaa atattgaaag agaaatatac     540
   tggcagattg gcagcataac ttgtgcagct tacccacttt cacaaatcga tactattgat     600
   attgtgactg gaaacatcag caaaaattct gctttaaatc ttgtcgtttt tggcgaaaag     660
   gatgaacatt tggaacttat ggatggtgtc ctgatcgacc ttttaaacgc aaaatggaat     720
   gcctttgtaa aatttaggtt ttatagacag tttttcttat ttctctttta cttcttgatt     780
   tcgcttattt gtttcactct acgacctgga cctccaccag ttaaaccaat atcaagtttc     840
   aacagtacca aacccagcgg aaataacatt actgctgaat tgtcgccaga aagcaatgca     900
   actactgttc caaaatggat ttacaaaaca gaaattaaaa agtatgatcc cgaagacaac     960
   agtagtcaaa acagcagact taatgtaata ttacgtgaag tcattttgac atctttgaaa    1020
   gttgaagata ttgataaagt agtttctttt tcaaatgcag agccgcaaga caattccagg 1080
   gaagatgcag atggattggt ttgcaatggc gattctgttc attcctgttt aggaaatgga 1140
   tctgatgttg ttgctgtatt tggaaaaggc aagagcccaa aagtaccaaa acctggagcc 1200
   tccgccgatg attttgatta tgacgacact tggtgggaag aatttggaca atgcagatta 1260
   cttcaggtca cttcctacat tgaaatgacc cgccttatat cagaagtctt gttggacatc 1320
   ggagctttgc tatacatatt agcagcattg agagaagcga gatttctagg atggagcatg 1380
   tttgtggaga atttgatgac tgctccttca agagttatgt ttttgttttc atgctgtttg 1440
   atgctaacga tgccattttt aagatttacc tgcaatgaag aaatagaaga catgatggca 1500
   gttattataa tgctcaccac tgctccttat tttctgtttt tctgcagagg tttcaagacc 1560
   gttggtccat ttgttgtgat gatatatagg atgataatgg gagacttgtt gagatttgct 1620
   acaatatatc ttgtctttgt gatggggttt gctcaagcat attatatcat tttcctgtca 1680
   tttgataacc cactgacacc cgaaggagtg gatgattctg taagtaatcc tattccgaac 1740
   ccaatggaag cagtcatggc aatgtttttt atgtcaatga caagttttgg cgattactat 1800
   ccagctttag aaaggacagc tcatgaattc tgtgctaagc tttgttttgt gatatacatg 1860
   gcaatagtag caattctact ggttaatatg ttaattgcta tgatgggaaa tacttatcaa 1920
   aaaatagctg aaacaagaaa tgaatggcag agacagtggg ccagaatagt attagtggtt 1980
   gaaagaggag taagtccatc agagaggtta actaaactaa tgtggtactc acagcctatg 2040
   tcagatggaa gaagagcgct ggtactgagg ctaaaccaat cggaggaaga taaagaggaa 2100
   atgaaagaaa ttctagagat gaaaagaatt cacaacagaa tggtacaaaa aaggaaagaa 2160
   agggaaaagt cgatgaacaa aagcccaaat ttgatcaatt taaacagtcc ggctgaaaat 2220
   gttaaaaata caaatccttt cagttaattc agttaacaga acaaagtgaa ttaaacacaa 2280
   cagaataatc ttcttatgaa actatgatat cagcccctga agtctttgta tcaaatcaag 2340
   caccctgtga gcagatgggc aataaacaaa aaatatgttt tctatggact agccccaaaa 2400
   tagatctatt gatttcaatg aaacttgc 2428
<210> 26
   <211> 2205
   <212> DNA
   <213> Nilaparvata lugens
<400> 26
   ctgcatatgg ccattgtgaa tgaggaccca ggcatggtga agttcctgct gaactcggga      60
   gccgacttcc acgaacgctg cttcggcaac ttcatgtgtc ccgaggacca gaaggcatcc     120
   cgctccgatt ccttcgatca cgaatgggtc aatctctggc ctgtcaccaa ctacgagggt     180
   tatgtgtact ggggcgaata cccgctatct tttgctgcgt gtttgggtca ggaggaatgc     240
   tatcggctca tgctagccag gggggccaac cccgacaacc aggacactaa tggcaacact 300
   gttcttcaca tgcttgtcat atacgagaaa atgggaacgt ttgacacagc ttacgagctg 360
   ggtgccaacg tatcggtgag gaacattcag aacttgacac ctctgacact ggcagccaag 420
   ctggccagaa ttgaaatgtt cttccacata atgaacatcg aacgagaaat ttactggcaa 480
   attggtagta ttacttgtgc ggcctatccc ttgatgctra tcgatacaat agatattgaa 540
   acagggrata ttagcaaaga ttcggctctc aatcttgtcg tatttgggga caaagatgag 600
   catttggatt tgatggaagg cgtgctggta gatttgctcc atgccaaatg gaatgcattt 660
   gtcaagttca ggttttaccg ccagttcata atgttcttcc agtactttct gatatcgtgc 720
   gtgtgcttca tactgcggcc tggtcccccc gaagcgcacg acggtatgaa catcaccgcc 780
   aatgctacac tcaacctgag cttcggggtg ctygaagtcc gacctgargc gttgcaagcg 840
   ytgctctaym ggctcgagaa gctgtcactg ccccagtcgg gtgatctgga aaggttcatt 900
   ctggaatctc trgctgatga gaagtcaatt tcagaagctg cattagagaa cgatgagaat 960
   cgggraccag gctggaacaa tagtgtrttt tcaacgtatg aaacgctggc tcgtgatacg 1020
   ggctaccaaa ctgatgagag tccrcatgac ggattgtcga gtagtatgga ttaygccgag 1080
   gtggccgagc gattgaccaa actgatgatt gatgtcaayc tgactgagag gagtggagct 1140
   gtcaatgaga ttatactgaa agctttgcct agtcttgagg agaaagagga tggtttggag 1200
   gagtggtgga gcggaatagc cggtgactgt atcctgttgc aggtgaatga cctctacgac 1260
   ttggcacgcc tgtgcgccga ggtggcgctt gaaattggcg cctttctcta cctgctggca 1320
   gcactgcgcg aggcccgctt cctcggactc aaaatgatga tcgagaatct gatgacggcg 1380
   ccgtcgcgtg tgatgttcct gttctcatgc tgcctgatga tgacgatgcc cttcttccgc 1440
   ttcacctgcc aggacgagac cgaggacatc ctggccgttg tcatcatgct cacaacgggg 1500
   ccttacttcc tcttcttttg cagagggttc aaaacagtgg gacctttcgt agtgatgatt 1560
   tacagaatgg taatgggtga tcttctgaga ttcgtgtcta tttacatggt gtttgtgatg 1620
   ggattttcac aagcgtatta cataattttc ttgacgtacg acaacccgac tacaccagaa 1680
   ggtacggacg acactacgtc gaatccactg cccagtccaa ttgaagccat tatggccatg 1740
   ttcttcatgt cactcaccaa ctttggagat tactactctg cgttcgagaa cactgaacat 1800
   gaaatcgaag ctaaggcttt ctttgtgatt tacatggtga ttgtggcgat tcttctaatc 1860
   aacatgttga tcgctatgat gggtaacact taccagaaga ttgctgaaac aaagaacgaa 1920
   tggcaaagac aatgggccag aatagttctg gttgtggaga gaggtgttag tcctaaggaa 1980
   cgcttgaaaa agctcatgtc ctattcacag cctatgtccg atggagggag ggctctggtt 2040
   ctcaggctga acatgacgga agaagacaag gaagaaatga aagaaatcct ggaaatgaaa 2100
   cgtgtacgtg ataaaattgt catgaagcgc aaaaaaggct tgaaattgtt ggggactaca 2160
   actcctagtc caaagaggat gaaaggccga gaaactccta tttaa 2205
<210> 27
   <211> 487
   <212> DNA
   <213> Schistocerca americana
<400> 27
   ctgcatatgg ccattgtgaa tgaggacccg gccatggtga agttcctgct ggacagtggt 60
   gccaacgtgc acgagcgctg cttcggcacg ttcatgagcc cggaggatca gaaggcccag 120
   cgctcagact ccctggacca cgagtgggtc aacgtcaact cggacacaaa ctatgaaggc 180
   tacgtgtact ggggggaata cccgctgagc ttcgctgcct gcctgggaca ggaggagtgc 240
   tatcggctgg tgctcgccaa gggtgccaac ccagatagcc aagacaccaa cggaaacacg 300
   acactgcaca tgctcgtcat ccacaggaag atgacccagt tcgacatggc ttatgaagtt 360
   ggctcttcac tcgacattaa gaataacctc aacctcacac ctctcacgtt agccgctaag 420
   ctggcacgaa tagagatgtt tttccatatc ttaaacatag aaagagagat ctactggcaa 480
   ataggtt 487
<210> 28
   <211> 3372
   <212> DNA
   <213> Drosophila melanogaster
<400> 28
   atgaagttcc tcctgaagaa atgcctgcgc aagaaggcgc ccgagatgaa acccggggcc        60
   atcctggatg ccgtcatctc gcaatcatcg gccaccgcgt gcaagtgcct gctgtacaaa       120
   ctggcggatt acaaaagagg tggtgatctc atcgatgcca tcaactcggg cggactgatt       180
   gccgtggagc agctgatccg ggagcagttt ggcgtcttca tgtacaacga tggcaagggc       240
   caggtgataa accgtgctga gtttctgcgc tggaagtacc gcgaccacac cgaggtgacc       300
   atacccattg aggcatccct gtccatccac gatccgctgg gcaaatggga ggaccacaag       360
   gcctgctggc agatgcagta ccgcggcgcc ctgggcgaga gtctgctcca tgtgctcatc       420
   atctgcgatt ccaaggtgca caccaagttg gcgcgcgtgc tgctccgcgt gttccccaat       480
   ctggcgctgg atgtgatgga gggcgaggag tatctgggtg ctagtgccct gcacctgtcg       540
   attgcctaca gcaacaatga gctggtggcg gatctgatcg aggccggagc ggatatccat       600
   cagcgagcga ttggtagctt ctttctgcca cgtgaccaac agcgagcgaa tccggccaag       660
   agcaccgact acgagggact ggcctacatg ggtgaatatc cgctggcttg ggccgcctgc       720
   tgtgccaacg agagtgtcta caacctgctg gtggactgcg gatccgatcc ggatgcccag       780
   gactccttcg gcaatatgat cctgcacatg gtggtcgtgt gcgacaaact ggacatgttc       840
   ggctatgccc tgcgacatcc caagacgccg gccaagaatg gcattgtcaa ccagacggga 900
   ctgactccac tcactctggc ctgcaagctg ggacgggcgg aggtctttcg tgaaatgttg 960
   gagctgtccg cccgcgagtt ttggcgctac agtaacatca cctgttccgg ctatccactg 1020
   aatgcactgg acactcttct gccggacgga aggactaatt ggaattccgc cttgttcatc 1080
   attctgaacg gcaccaagcc ggagcatctg gacatgttgg acggcggcat tatccagcgc 1140
   ctgctggagg agaagtggaa gacctttgcg cagaaccagt ttctgaagcg tctgctcatc 1200
   ctgtccaccc atctgctatg cctgtccgta tcggtgtatt tgcgtccagc gcacgatggt 1260
   gaagccgagg atgaggactc cgagggatcg gatgcctcgg cagccgctct gctggacata 1320
   cagtcggatg agggcgacag tggtggtggt gactacaatg cccagacggt ggcccgttac 1380
   tgcgccgagt ttgccaccct ggtgggagtt ttaagctatg tgatcttcca gcaaggcgac 1440
   gagataaaga accagggttt gtccgccttt ctcaagcagc tgtcccatgc gccggctaag 1500
   gccatcttcc tgttctccaa cttgctgatc ctggcgtgca ttccatttcg tttgattggc 1560
   gacaccgata ccgaggaggc catcctgatt ttcgctgtgc cgggctcctg gtttctccta 1620
   atgttctttg ctggtgccat tcggctgacg ggtccgtttg tgaccatgat ctattccatg 1680
   atcacgggcg acatgttcac cttcggcatc atctactgca tcgtcctgtg cggattctcg 1740
   caggccttct acttcctcta caagggacat ccccaggtgc agtccaccat gttcaatacc 1800
   tacaccagca cttggatggc cctgttccaa accacactgg gtgattataa ctatcccgat 1860
   ctcaatcaga ccacatatcc caatctctcc aagaccgtgt ttgtcatctt tatgatcttc 1920
   gtacccatcc tgttgctcaa catgttgatt gccatgatgg gcaacaccta tgtgactgtg 1980
   atcgagcagt cggagaagga atggatgaag cagtgggcca agattgtcgt caccctggag 2040
   cgcgctgttc cgcaggcgga tgccaagggc tatctggagg catactccat cccgctgggt 2100
   ccgtccgatg actccggttt cgaagtgcgc ggcgtgatgg tcatcaagag caagagcaag 2160
   acgagggcca agcagcgaaa aggagccgtc tccaactgga agcgcgtggg tcgtgtgacg 2220
   ctaacggcgc tcaagaaacg cggaatgacc ggcgaggaga tgcgtcgcct tatgtggggc 2280
   cgtgcctcca tctccagtcc cgtgaaagtg acgaagcaga agcttaagga tccctacaat 2340
   ctgcacacgg actccgactt caccaacgcc atggacatgc tgaccttcgc tagcaacccg 2400
   gcttcctcca atggcgtcac cctgcgatcg gtaacagcgc ctccgcccgc accgccagca 2460
   ccggatccct tcagggagct gatcatgatg tcggaccagc gaccggagac ccatgatccc 2520
   cactactttg ccggcctgca gcagctggcc aataaggctt tcgatttggt ggagcaaacg 2580
   atgaagacac agccgcaagc gccggtggcc aagaaggtgg atccactgcc agtggcttcg 2640
   gtggccaaag catctccagc cgctcctgca acacaagcta ctgctactgc tgctgctgct 2700
   tccgatctaa tggccatgcc actgcccatt tcgaacttga gcaatctttt ccaagacccc 2760
   aaggacattg tcgatcccaa aaagctggag gagttcatgg ccatgctggc cgaggtggaa 2820
   accgaggaga gcgacagcgg cggacccatt ctcggcaagc tatcgctggc caaacggaca 2880
   cacaatgccc tatccaaggc ggaaattcga cgggatcagc agggattcga gggtcacagc 2940
   catggacagt ttcagcccat gtcctcggtg tgggcaccac ctggcctgga tgtggacacg 3000
   ggcttccact tcgatgaagc cgtcgcggag gaggtgctga ccatcgaaca ggaagccgaa 3060
   gtggagaccg aggacggcaa tggtggacag gacagcgagg atatacccac ggcggaggag 3120
   gtgcacgcca ctatgaagca gtttcacctg cgcaagtgcc agccagcaca ggatgaggcc 3180
   gctcgtcgcg ccaaaagtgc tcgtgttcgt cgccgaaaca aagtttcgcc ggagcaaagc 3240
   gatgatccgg acgagcgcag ccagcgggga cgttccgctt acacgcggcg aacgcaatcg 3300
   ccgcccgatc cgctggaacc atggagcacc cgcgaactgc aggacatcaa caagatcctg 3360
   gccagaaagt ga 3372
<210> 29
   <211> 3387
   <212> DNA
   <213> Musca domestica
<400> 29
   atgaggctgt tcatgaaaaa gtgtttgaga aaaaagcctc aggaattgaa acctggagcg 60
   attttagatg ctgtcatttc acagtcatcg gcaacagcta gcaaatgttt gctctacaag 120
   ttggcggact acaaacgggg aggcgacctc attgatgcca tcaacaccgg agggctggtg 180
   gcggtggaac aactgattcg cgaacaattt ggcatattca tgtataacga tggcaagggt 240
   caggtcatta atcgtgccga attcttgcgt tggaaatatc gtgaccacac cgaagtgacc 300
   atacccatag aagcctcttt atccatacac gatccattgg gtaaatggaa ggatcacagt 360
   gcctgttggc agatgcagta tcgtggcgcc ttgggagaga gtctgctgca cgtcttgatt 420
   atttgcgatt ccaaaatgca taccaaattg gcgcgtatct tgttgagggt gtttcctcgc 480
   ctggcattgg atgtcatcga gggcgaggag tatttgggtg caagtgcatt gcatttggcc 540
   atagcttata gcaataatga gttggttgcc gatttaattg aggcaggagc tgacatcaat 600
   cagagagcca ttggaagttt ctttttgccc aaggatcaac agagagccaa tccggcaaag 660
   agtacagatt acgagggttt ggcttatatg ggcgaatatc cattgtcatg ggctgcctgc 720
   tgcgccaatg aaagtgtcta taatctgttg attgatcatg atgccgatcc cgatgcccag 780
   gatagttttg gaaacatgat cctgcacatg gtggtggtgt gtgataagtt ggacatgttt 840
   ggatatgctc tacgccaccc caagactccg gccaaaaatg gaattgttaa tcattctggt 900
   ctgacacctc taacattggc ttgcaaattg ggtcgggctg aggtgttcag ggaaatgttg 960
   gaactatcgg ccagagaatt ttggcgttat agtaacatta cctgttcggg ttatcctttg 1020
   aatgctttgg acacgctgct accagatggg aggaccaatt ggaattcggc cttgtttatt 1080
   attttgaacg gtaccaagga ggaacatttg gatatgttag atggtgggat tatacagcgt 1140
   ctgctggagg agaaatggaa gacctttgcc cagaatcaat ttctaaagcg tttgctgatt 1200
   ttggtggtgc atttgttgtg cctctcaacg tcggtgtatc tgaggccagc ccatgatggc 1260
   gatgatgacg aagaagatga aacggctcag gctaatgcag gactacagac caatgatcca 1320
   gaaaacgaag aatatgatat tcaaacaata gtgagatatt gtgctgagct gtgtaccatt 1380
   gcgggggtcc ttagtttcct gattttccaa caaggcgatg aaataaagaa tcagggattg 1440
   tcagccttcc tcaaacaact gtcccatgct ccagctaagg taatattctt ggtctcgaat 1500
   ctgatgattt tggcctgcat accctttcgc ctaatgggtg acacggacac agaagaggcc 1560
   atacttatat ttgctgtgcc gggaagttgg ttcttgctga tgttctttgc gggtgcaatt 1620
   cgtttgaccg gtcccttcgt aaccatgatc tactccatga taacaggtga catgtttacg 1680
   tttggcatta tttactccat tgttctgtgt ggcttttccc aggcgtttta tttcctttac 1740
   aaaggacatc cccaattgca gtcgactatg tttgatacct ttcccagcac ctggatggcc 1800
   ctgtttcaaa ccactttggg agattacaat tatcccgatt tgaatcaaac aacctacccg 1860
   aacttgtcga aaaccgtttt cgttattttc atgattttcg ttcccattct gttgctgaac 1920
   atgttgatcg ccatgatggg caacacctac gcccatgtca ttgaacaatc cgaaaaggaa 1980
   tggatgaaac agtgggccaa gattgtggtc acactggaaa gggcagttcc ccaagcggat 2040
   gcaaaaaatt atctggaagc ctattccata cccttgggtc cttcggatga ttccggattt 2100
   gaggttaggg gagtaatggt cattaagagc aaaagtaaga caagagccaa acaacgcaag 2160
   ggagcagtat cgaattggaa gagagtgggc cgtgtaacgc tcaatgcctt gaagaaacgt 2220
   ggtatgacgg gtgaggagat gcgtcgtctt atgtggggcc gagcatccat atcgagtccc 2280
   attaaagtgg ccaagaagaa actgaaggat ccctacaatc taaatcaaga gcctgggggt 2340
   caatccaatt tgaccagtgc catggacatg cttaattttg ccaatgaccc agcctccagc 2400
   tcgggattgg cttttagaac ttctggtact caggacccca aggagaagaa ggatgtgcca 2460
   cacattcccg caccagatcc cctgcgtgat ttggttctgt tagccgaaga gaagccggaa 2520
   gtacacgatc cacaattcta ttcagcattg attcagttgg ccaacaaggc ctatgatttg 2580
   gtgaagaaaa ccctggaaac ccaacatcag ccacaagttg aggagccgat accactcaca 2640
   gaggccacca gtttgaagga cgtcacactg acaaaacaac ccgatcccgt tgctgctgat 2700
   gctggactcc ttggcgacat ggccatgggt gccaatctta ccaatctctt tcaagacccc 2760
   aaagatattg tggacccaaa gaaactcgaa caatttctga aaatggtagc tgatgttgag 2820
   accgaggaaa gtgatggagg tggtccaatt ttgggaaaac tttcattggc ccgcaggaca 2880
   cgcaatgccc tatttaaggt acccataccc aagagtactc ttggcgacag ttcggataag 2940
   ttaattaatt cggtgtggga caaggtgcca gccgacaatg atgaggacag tattccggcc 3000
   tttgatttcc aagaagctct ggctgaacat gtcctgacag tggaacaaga ggccgatgtg 3060
   gagaccgaag atgcaaacga tgattcgggt gagaccatgg acgatatacc cacagcggag 3120
   gaagtacacg ctaccatgaa gcaatttcat ttgcgtaagc gtcaatatca gcaggacgat 3180
   gcagcacgtc gtgctaagac ggctcgaata aggcgcaaga ataaagttgc tccagattat 3240
   gctgggagtg aagagcaata tggacgtggc cgttcggcac ccatgaaaaa gaaacatgat 3300
   gaggatgaaa tgtcacctcc tgatcccttg gaaccatgga gtactcggga attgcaaaat 3360
   attaataaaa tcctggcaaa aaagtaa 3387
<210> 30
   <211> 3435
   <212> DNA
   <213> Ceratitis capitata
<400> 30
   atgaaattct ttttgaaaaa atgtctacgc aaaaaaccgg aggagatgaa accaggcgcc      60
   atactggacg ccgtcatttc gcaatcctca ccaatggcca acaaatgtct actctacaag     120
   ctggccgact acatgcgcgg cggtgacctc atcgacgcac tgaacactgg cggcctggtc     180
   gccgtggaac aactgatacg cgagcagttc ggcatattta tgtataacga tggcaaggga     240
   caggtgatta atcgtgccga gtttttacgc tggaaatatc gcgatcacac tgaagttact     300
   ataccgattg aggcttcgct ctcacgtcac gatccgctgg gcaagtggga ggatcataaa     360
   gcctgttggc aaatgcaatt tcgtggggca ctcggtgaga gtttactgca cgtgctcatc     420
   atttgcgatt cgaaaataca cacaaagcta gcgcgggtac tgatacgagt ctttccgaat     480
   ctggcacaag atgtgatgga gggtgaggag tatttgggtg ctagtgcgtt gcatttggcg     540
   attgcttata gcaataatga gcttgtggcc gatttgattg aagcaggcgc tgatataaat     600
   cagcgtgcta ttggcagttt ctttctgccg cgtgaccagc aacgtcgaaa tccagccaag     660
   tcgacggact atgaaggatt ggcgtatctg ggcgagtatc cgttggcatg ggcggcatgt     720
   tgcgccaatg aaagtgtgta caatttgctg gtggactgtg gtgcagaccc agatgcacaa     780
   gactcgtttg gtaatatgat actgcatatg gtggtggtgt gcgataaatt ggatatgttc     840
   ggttatgctc ttcgccatcc aaaaacgcca gccaaaaatg gtatcgccaa ccacagtgga     900
   ctcacaccgc tgacactttc ttgcaaactg ggacgcgctg aagtttttcg tgagatgttg     960
   gagctttccg cacgtgagtt ttggcgttac agcaacatca cctgctcggg ttatccgctc    1020
   aacgctttag atactttgct gccagatgga cgcacaaatt ggaactcggc gctgtttatc    1080
   atcttgaatg gcaccaaaga ggaacatttg gatatgttgg acggcggcat catacaacgt    1140
   ttactggaag agaaatggaa aacctttgct caaaatcaat tcctcaaacg tctactgatc 1200
   ctagtcatac acttggtttt cctctccatc tcagtttata tgcgtcccgc gcgtgttgca 1260
   gacgatgatg atgaagatga gatagatgcg tcttcgaaag acactaaagc tcttgagctt 1320
   gccacggaat tggatgaaga tgagtacgat gtgcagacta ttgtgcgcta ctgcgcagag 1380
   tttggcacta tagtgggcgt attgagttac gttatctttc agcagggaga tgagatcaaa 1440
   aatcaaggtt tgccagcgtt cttaaagcag ttgactcacg ctccagccaa agctatcttt 1500
   ctagtctcta atctgctaat tctcgcctgt ataccctgtc gactcatggg tgatacggac 1560
   gccgaggagg ctatactcat ctttgccgtg cctggcagtt ggtttctgct aatgtttttt 1620
   gccggcgcca tacgtctgac gggtcctttc gttactatga tatactccat gattacaggc 1680
   gatatgttca cgtttggcat tatttattgt atcgtactat gcggcttctc gcaagctttc 1740
   tactttctct acaaggggca tccacaaata cagtcgacta tgttcaatac ctttcccagt 1800
   acttggatgg ctttgtttca aaccacactc ggagactata attatcccga cttgaacaat 1860
   acaacatatc cgaacctctc gaagactgta ttcgttatat ttatgatttt cgtgccgatt 1920
   ttattgttga acatgttaat cgctatgatg ggcaacacct acgcgcaagt tatcgaaaga 1980
   tcggagaaag agtggatgaa acagtgggca aagatcgtgg tgaccttgga acgcgctgta 2040
   ccacaagctg atgctaagaa ctatctagag gcctattcaa taccactcgg accaatagat 2100
   gactccggat atgaggtgcg cggtgtaatg gttatcaaga gcaaggcgaa gaccagagct 2160
   aaacagcgta agggtgcagt ttctaattgg aagcgcgttg gtcgggtcac attgaatgcg 2220
   ttgaaaaagc gcggcatgac cggcgagcaa atgcgtcgtt taatgtgggg acgtgcatcc 2280
   atatcaagtc cgattaagat tacaaaaaag aagttgaaag atccctacaa tttgaatcca 2340
   caaaatgatc tcacaagcgc tatggatatg ttgacctttg ccaatgatcc cgcagcaagc 2400
   gctggtctac aactacgctc ctcactcatg gactccggtg ccgagggcaa agcagcaaaa 2460
   gtgcaggagg aaaatcagca acccgcacca gatccactgc gcgacttaat tttcctcgcc 2520
   gatcgtcgtc cggaaatgca tgatcctcag tattttgttg gcctacagca attggcaaat 2580
   caggcattgg atttggtaga acaaacaatg ggcatccatc caccacccac tcccgcaact 2640
   gatgctttac ttctatcagc agctgctgct acaacaacta tcggtgcagt gacaatgcaa 2700
   acagccacaa ctgttgccgc accgagcgca gatttaatta ccccattggg cgcgaattta 2760
   acaacgctct ttcaagataa caaagatgtc gtggatccgc aaaagcttca agaattcttg 2820
   aaaatgttgg ccgagatcga gaccgaggag agtgatggcg gtggtagacc aattttgggc 2880
   aagttatcgc tttctcgacg tacaaagagc gctctttcga aggctcagat taagaaagat 2940
   acggtcggtg gtagtcctga taaattgatc ccatcggttt ggtcacataa tttaccgcaa 3000
   ccagatgagg aaccggaatt caacttcgag gctgcactag ccgaggaaca cacactcacc 3060
   attgaacagg aggctgaggt ggagaccgaa accggcaacg agcgtgagga tagcgaagat 3120
   tgtccgacgg tagaggaggt gcatgcaaca atgaaacaat tccacctgcg taagcggcaa 3180
   tatcaacagg acgacgcagc acgacgagcg aaaagtgcac gcatcaagcg caaaaataga 3240
   atttcaccgg aacaatccca tgagtgtgat gacggcagca gcggcggcgg cggaggtaag 3300
   tcaacacacc cacgcggcgc ttcagcgcca ggccgtaagc acacggtgag cgaacgcttg 3360
   tcgccgccgg atcctctgga gccgtggagt acgcgcgaat tgcaaaatat caacaaaata 3420
   ttagcgcgca agtga 3435
<210> 31
   <211> 3363
   <212> DNA
   <213> Aedes aegypti
<400> 31
   atggtccaat tcgactggcg gcgtatgtgt cgcaagaagc gaaaggttcc tcagggcggt 60
   gctatcctgg atcaagtgat ttcccagtcg gccaatgctt ctaatcagtg cttgctgtat 120
   aagatggcta attacaaacg gggtggagat ttgatcgatg cgttccaggt cggagggcag 180
   aaggccgtgg agcagttgat tcgggagcag tttggagttt ttatgtacaa caatggccgc 240
   ggacagatca tcaatagggc cgagtatcta cggtggaagt acatggataa tcatgaggtc 300
   gtcatcccga ttgaggcgtc gttgtctccg catgatcctt tgggcaaatg ggtcgatcat 360
   aaggcctgct ggcagatgca gtatcgtgga ttgctgggcg agagtttgct gcacgtgttg 420
   atcatctgcg atacgaagat tcacacgaag ttggcccgca tcctgctgcg cgtgttcccc 480
   gagctgtcga tcgacgtgat ggaaggcgag gagtacctgg gcgctagtgc actccacttg 540
   gcgatcgcgt acagcaacaa cgagttggtg ggcgatctga tcgatgccgg agcggacgtg 600
   tcccagcgcg ccatcggacg gttcttcctt ccacgggatc agcagggctt gaagccggcc 660
   aagaacaccg actacgaggg gttggcctac ttgggcgagt atccgctggc gtgggccgcc 720
   tgctgcgcca atgagtccgt ctataacctg ctgctggaat gcggggcgga tccgaacgcg 780
   caggacagct tcgggaacat gattctgcac atggtcgtcg tctgcgataa gctggatatg 840
   ttcgggtatg cccttcgtca tccgaagctt ccctgtaaaa atgggatcgt caatgagtcc 900
   ggactcacgc cgttgacctt ggcctgtcga ttgggtcgtg atgaagtgtt ccgtgagatg 960
   ttggagttgt cggcgaggga gttttggcgg tacagtaaca tcacctgttc cggatatccg 1020
   ttgaacgcat tggacacgtt gatgcccgac ggaactacca attggaattc ggcgctgttt 1080
   atcatcctca acggaaccaa ggaagagcat ttgaacatgc tggacggtgg catcgtggaa 1140
   cgtctgctgg acgagaagtg gaagacgttc gctcggaacc agttcatcaa gcgactgctg 1200
   atcctcgctc tgcatctgtt ctgcttgtcg tgttcggtct acttgagacc ggtgcgagtt 1260
   ttcaaggagg agagtgaaga gggaagcgat ggaggagatg gagcgggtac agctgctccg 1320
   gacggtgtcg atgatgatga ggacattgat ttgacgacct ggttccggta tggattcgag 1380
   atcgcaaccg tgatgggagt actgagctac gtggttcttc aacagggtga tgagatcaag 1440
   aatcagggat tgatctcgtt tataaagtca cttggaaatg ctccggcgaa agctatcttc 1500
   ctgatctcca atctgatgat cttggcttgc atcccgttcc gaatgatggg tgacgttgaa 1560
   cacgaggagg ccattctgtt gttcgcagtc ccaggatcat ggttcctgct gatgttcttt 1620
   gctggcgcca tcggcttaac aggtcccttc gtcaccatga tcttctctat gatcacgggt 1680
   gacatgttca ccttcggcat catctacatg atcgttctgt tcggattttc ccaggcgttc 1740
   tacttcctgt acaaaggtca tccggaagcg gaggattctc catttgggag ctacttcggc 1800
   acttggatgg gcctgttcca gacaaccctc ggcgattacg attacgccga cttgaatctt 1860
   acgacctacc cgaacctggc gaaaaccgtg tttatcatat tcatgatctt cgtgccgatc 1920
   ctgttgttga acatgttgat cgctatgatg ggcaacacct acgcgtacgt cattgagcaa 1980
   gctgaaaagg aaggcatgaa acagtgggcc aagatcgtgg tcaacctgga gcgggcggtc 2040
   aagcaggagg acgccaaaaa gtacctggag gagtactcga tcgggttggg accgtcggat 2100
   gatccgaggt acgagatccg tggagtcatg gtcatcaaga gcaagagtaa gacccgtgcc 2160
   aggcagcgca agggagcggt aagtaactgg aagagcgttc tgcgtgtcac gttgaacgag 2220
   ctgaagaagc gtaacatgac cggcgaggag ctgcggagga tcatgtgggg acgatcgtcg 2280
   atcacttctc cggccaagat ctccaagaag aaaaagatct acgaagagga ggaccccttc 2340
   gccatcacca atgctatcga tgtgatgtcc ttcacgcagg acatcgtcat ggtaagtagc 2400
   gaaccgaccg gaccgcccac aatcgacccc tccaaaccgg cgacgatcgc cgtcaagccc 2460
   caaacggcac ccgcagcgca accgatcccg gagattcctc ctcaaccgcc gctggtcaaa 2520
   cggcagtccg tggttagtgc tccgccggcc tacgacgatt tccccccgat gaaggactac 2580
   aaggatcccc tgcgggagct ggtcatcatc tcggagtcgc cctcggtgga cgaccattac 2640
   gtgcagagct gcaagaccct cgcgaatgat gcgtccaccc tggatcacga caccttgggt 2700
   cagttaaatc ccttcctgga ggcgaaggac gtggtcgatc cggtgaagga acgcgagttt 2760
   ctcaaaacac tggaggccct ggaggacacc gacagcgaag cggccgaaaa gccggtcctg 2820
   ggcaagatct ccctgatacg acgcgccaag tcagccgtct cgagaaccac ttccaggaag 2880
   aagaaaaccg accagcatcc gctgtttacg atcgcttggg atgacaagaa cctgacccgg 2940
   acgcatccgg aggactttgg agcgctgaac acggcctacg agtacagtgc cgaagatttc 3000
   aagcaagacg tggacgagga agcgagggag gaggaggaag acgagggtgt gacggtggag 3060
   gaagtgcacc gccgaatgga agagctacac catcgcggcc gggcttcctt ggagcgggac 3120
   agcaactcga ccgagagcag caagaaacat ccgcacagga agcggcaagg tatgggccga 3180
   ggtcggaaca acaaggtgtc gccggatacg tccaacgaga gcgtatccgg caagaaggac 3240
   aaacggatga agtcggcgcc gacgggtgga gggacctcgc caccggatcc actggaacca 3300
   tggagcacaa gggatatttg caacattaat aaacttttgg acaccgatac gcaggatgag 3360
   taa 3363
<210> 32
   <211> 3414
   <212> DNA
   <213> Culex quinquefasciatus
<400> 32
   atggttaagt tcgactggcg gcggatgtgc cgcaagaaga agaacgttcc gcagggtggt      60
   gctatcctgg atcaagtgat ttcgcagagc acgaacgtcc agggccagtg tctgctgtac     120
   aagatggcca attacaaggg tggtggcgat ttgatcgatg cgttcaagat gggcggccag     180
   aaggcggtgg agcagctgat ccgggaacag tttggggtgt ttatgtataa tgctggccgg     240
   ggtcagatta tcaatcgggc ggagtatctg cggtggaagt atatggataa ccatgaggtt     300
   gtgattccga ttgaggcgtc gctgtccccg catgatcctt tggggaagtg ggtcgatcat     360
   aaggcctgtt ggcagatgca gtatcgtggg ttgctggggg agagtctact tcatgtgttg     420
   atcatctgcg acactaagat tcataccaaa ctgtcccgca ttctgctgcg cgtgtttccg     480
   gagctgtcga tcgacgtgat ggaaggcgag gagtacctgg gcgctagcgc actccacctg     540
   gcgatcgcgt acagcaacaa cgaactcgtt ggcgatctga tcgacgccgg ggcgaatgtg     600
   tcccagcggg ccatcgggcg cttcttcctt ccacgggacc agcagaagat gcatccggcc     660
   aagaccaccg actacgaggg gttggcctac ctgggcgagt acccactggc ttgggccgct     720
   tgctgctcga acgaatcggt ctacaacctg ctgctcgagt gtggcgcaga tccgaacgcg     780
   caagacagct tcggaaacat gatcctgcac atggtcgtcg tgtgcgacaa gctggacatg     840
   tttgggtacg cgctgcggca cccgaagcta ccatgcaaga acgggatcgt caacgaggcc     900
   ggactgaccc cgttgacgct ggcctgccgc atcggtcgag acgaggtgtt ccgtgagatg     960
   ttggagctgt cggcgaggga gttttggcgg tacagtaaca tcacctgttc cgggtatccg    1020
   ttgaacgcgc tggacacgtt gatgccggat ggttcgacga actggaactc tgcgctgttc    1080
   atcatactta acggaacaaa ggaggaacat ttgaacatgc tggacggtgg tatcgtggag    1140
   cgtctgctgg acgagaagtg gaaaactttc gccaggaatc agttcttgaa gcgtttgctt    1200
   atccttgcac tgcacttgtt ttgtctgtcg tgctcgattt acctgcgacc ggtacatgtc    1260
   ttcaaggatg atgacgatgc agatgacacc gatggggtta ctgattcccc tgatacaacc    1320
   gacgacgagg ggattgacct gaccacgtgg ttccggtacg ggtttgaagt tgccaccgtg    1380
   atgggcgtgc tgagctatgt cgtagttcag cagggtgaag aaatcaagaa tcaggggttt 1440
   atatcgttcc tcaaatcgct ggctggagct cccgctaaag cgattttctt gatttccaac 1500
   ctgatgattt tgtgctgcat acctctgcgg attctcggcg atagagaagc tgaggaagcg 1560
   gtgttgttgt ttgccgtccc cggcagctgg tttctgttga tgttcttcgc cggtgcaatc 1620
   ggactaactg gacccttcgt aaccatgatc ttttctatga taaccggtga catgttcacc 1680
   tttggtatca tttacacgat cgtactgttc ggattctcgc aagcgtttta cttcctgtac 1740
   aagggtcatc ccaacgcgga cgaatcacct tttggcacgt actttggaac ctggatggga 1800
   ctgttccaga cgaccctggg cgattacgat tacgccgatt tgaacctgac cacgtacccc 1860
   aacctcgcca aaacggtctt tatcacgttc atgatcttcg tgccgattct gctgcttaac 1920
   atgttgatcg ccatgatggg taacacctac gcgtacgtca ttgagcaagc cgaaaaggaa 1980
   ggtatgaagc agtgggccaa gattgtggtc aacctggagc gtgccgtcaa gcaggaggat 2040
   gccaaaaagt atctggaaga gtactcgatc gggctgggac cttcggacga tccacggttt 2100
   gagattcgcg gcgtgatggt catcaagagt aagagcaaga cgcgagcgcg gcagcgcaag 2160
   ggagcggtca gcaactggaa gagtgtgctg cgtgtgacgt tgaacgagtt gaagaagcgc 2220
   agcatgactg gcgaggagct gcgccggatt atgtggggtc gatcgtcgat cacttccccg 2280
   gctaagattt ccaaaaagaa gaagatctac gacgaagacg aggatccgtt cgcaatcacg 2340
   gcagccattg acgtgatgtc gttcacgcaa gatatcgtga tggtcagtac ggaaccggtt 2400
   ggtcccctca tgatcgaccc gacgaaacct cccgtcacca ccgtcaaacc taccgctccg 2460
   accgttcctt ctgcgcaacc tgctccaggc gttccgatga caacagcgca gactgcggct 2520
   cctacggcac cccagctggt caagcgccag tccgtggtca gtgctccgcc ggcttacgat 2580
   gacttcctcc cggcgaaagg cttcaaggat ccgctgcggg agctgctcat cctttcagaa 2640
   tcgacctcga tagacgagca ctacgcgcaa acgtgccgtt cgctggcgac ggatgcctct 2700
   accctggacc acgaggaaca ccagcaacag caacagcaga ccgttggcca gttgaatccc 2760
   ttcctggacg cgaaagacgt ggtcgaccca gtgaaggaac gcgagttcct caaaaccctg 2820
   gaagccctcg aagacaccga cagtgaagcg gccgaaaagc ccgtcctcgg caagatctcg 2880
   ttgatccggc gtgccagatc ggccgtttcg agaacaacct ccaggaagaa gaaaaccgac 2940
   cagcatccac tgtttatgat tgcctgggac gacaaaacga cgaccatccc cggtgggggt 3000
   ccccaagcgg tatcggccac cgccggaact gctgctccgg aggatgactt tggggcactg 3060
   aacacggcgt acgagtgctc ggcggaagat ttgaagcacg acgtggacga agaagcacgg 3120
   gaggacgatc cagacgaggg cgtgacggtg gaggaggttc accgacggat ggagcggttc 3180
   caccagcggc cgggcagatc ctcgccggaa cgggacagca actcgacgga aagcagcaag 3240
   aagcaggcca agggagacaa acgcatgaag tcggcaccca gcggccatgg gccgcgccat 3300
   cggcagatga tggacactgg tggtggatcg ccgccggatc cactcgagcc gtggagcacg 3360
   aaggaaattg taccgatcaa taagctgctc gatacggata cccaggacga gtag 3414
<210> 33
   <211> 3651
   <212> DNA
   <213> Anopheles gambiae
<400> 33
   atggtgcatc tcgatccgct gcggctatgc cgcaaaaagc gcaaactacc gcagggaggc 60
   gccatcctcg accaggtgat atcgcagtcc gcgtccgcct cgaaccagtg tctcctgtac 120
   aagatggcca actacaagcg gggcggcgat ctgatcgacg cgttccagat cggcggccag 180
   aaggcggtgg agcagctgat ccgcgagcag ttcggcgtgt tcatgtacaa caagggccgg 240
   ggccagatca ttaaccgggc cgagtacctg cgctggaagt acatggacaa ccatgaggtc 300
   atcataccga tcgaggcctc cctgtcgccg cacgacccgc tcagcaagtg ggacgaccat 360
   aaggcctgct ggcagatgca gtaccggggc ctgctgggcg aaagtctgct gcacgtgctc 420
   atcatctgcg acacgaaggt gcacaccaag ctggcccgca tcctgctgcg cgtcttcccg 480
   gagcagtcga tcgacgtgat ggagggcgag gagtatcttg gcgcgtccgc cctccatcta 540
   gccatcgcgt acagcaacaa cgagctggtc ggcgacctga tcgatgccgg cgcggacgtg 600
   tcccagcggg cgaccgggcg cttctttctg ccccgcgacc agcagggcct gcggccggcc 660
   aagacgaccg actacgaggg gctggcgtac ctcggcgagt acccactggc ctgggcggcc 720
   tgctgcgcga acgagtcggt gtacaacctg ctgctcgagt gtggcgccga cccgaacgcc 780
   caggacagct tcggcaacat gatcctgcac atggtggtcg tgtgcgacaa gctggacatg 840
   ttcgggtacg ccctgcgcca cccgaagctg ccgtgcaaga acggcatcgt gaatgcggcc 900
   ggcctgaccc cgcttacgct cgcctgccgg ctcggccgcg acgaggtgtt ccgcgagatg 960
   ttggagcttt ctgcgcgcga attctggcgg tacagtaaca tcacctgctc gggctacccg 1020
   ctaaacgcgc tggacacgct catgccggac ggcagcacca actggaattc ggccctgttc 1080
   atcatcctga acggcacaaa ggaggagcat ctgaacatgc tggacggcgg catcgtcgag 1140
   cggctgctgg acgagaagtg gaaaacgttc gcccggaacc agttcatcaa gcggctgctc 1200
   atcctcgcga tacacctgtt ctgtctgtcc tgctcggtct acctgcgccc ggtccgcgtg 1260
   tttgctgatg acgagggcga ggagggcgga gacgcaaccg atgacgatgg tgcggacgat 1320
   ggtgggccga tggcggtgga cgcggtcgac gacgacatcg acctgacgac ctgggtgcgg 1380
   tacggcttcg aggtggccac ggtgatgggc gtgctgagct acgtcgtact gcagcagggc 1440
   gacgagatca agaatcaggg cttcttctcc tttctcaagt cgctgagtca agcaccggcg 1500
   aaggcgattt ttctgatatc caacatcctg atcctggcct gcataccgct gcgcatgatg 1560
   ggcgacaccg agacggagga agccatcctg ctgtttgccg taccgggcag ctggtttctg 1620
   ctaatgttct ttgctggtgc gatcggactg accggaccgt tcgtgacgat gatcttctcc 1680
   atgattacgg gcgacatgtt cacgttcggc atcatctaca tgatcgtgct gtttggcttt 1740
   tcgcaagcgt tctacttcct ctacaagggc cacccgaacg cggaggacag cccgttcggc 1800
   agctatttcg gcacgtggat ggcgctgttc cagaccacgc tcggcgacta cgattacgcc 1860
   gatctgaacc tcaccaccta cccgaacctg gcgaagacgg tgtttgtcat ctttatgatc 1920
   ttcgtgccga tcctgctgct gaacatgctg atcgcgatga tgggcaacac gtacgcgtac 1980
   gtgatcgagc aggcggagaa ggagggcatg aagcagtggg cgaagatagt ggtcaatctc 2040
   gagcgggcgg tgacgcagga cgacgcgaag cgctacctgg aggagtactc gatcgggctc 2100
   gggccgtcgg acgatccgcg gtacgagacg cgcggcgtca tggtgatcaa gagcaagagc 2160
   aagacgcggg cccgccagcg caagggcgca gtgagcaact ggaagagcgt gctgcgcgtc 2220
   acgctgaacg agctgaagaa gcgcagcatg acgggcgagg agctgcgacg catcatgtgg 2280
   ggccgctcgt ccatcacgtc gccggcgaag atcgcgaaga agaagcggcc gggcgaggtg 2340
   gacgatctgc tcggcgatcc gttcgcgata acggccgcga tcgacgtgat gtcgttcacg 2400
   caggacatcg tgatggttag cacggacacg gtctgcccga tcacgatacc gccagcggct 2460
   gccccgggca cggcggcccc gaaggctggt ggcaccaaac cggtagtagt ggcaccgccg 2520
   ccgccagcag cagcaacagc agtacagcaa ccaccgccgc cagcgacgac gcggcgccac 2580
   tcgacggcaa gcgcaccggc cacgatgggg ctgggcgtgg aggcggccct gcccggctac 2640
   aaggatccgc tgcgcgagct cgtcatcatc tccgagtcgg cctcggtcga cgagaactac 2700
   gcgcagaacg tgaaaacgct cgcgatcgac gcgtccacgc tcgaccacgt gcacgagatc 2760
   gacatcagcc agcaacagca gcaacagcag caacagcagc agcaacagcc ccagcaaccc 2820
   cagcagcagc agaatctcgg ccagctgacc ctcttccaga acccgaagga cgtcgtggat 2880
   ccggtgcggg agcgcgagtt tctcaaaacg ctcgaagcgc tcgaggacac ggacagcgag 2940
   gcgggcgaga agcccgtcct cggcaagatc tcgctgatcc ggcgggccaa atcggccgtc 3000
   tcgcgcagca cgtcgcgcaa gcgcaaaacc gaccagcacc cgctgttcat gatcgcgtgg 3060
   gaggacaagg gcgaccagca gcggcacagc acagcgctgt acgatggcgc gacgggtgcg 3120
   gccccgtcgg tcggtgcgcc cggcgagctg gggcagccgg agcagcccga ggacgccgtc 3180
   acggtcgagg agctgcaccg gcggatggag cagttccacc agcgggcgtc cgtgcgcgaa 3240
   cgggacgcga actcgagcga gagcggcagc ggcaagcagc acgccggccc gaaggcacgg 3300
   aagccgtccc accatcggga cggtggcggc tcccatcatc accatgggct cgggcggggc 3360
   aagcacaaca agatatcgcc cgacaactcg aacgaaagta cgggcggtgg cggcggtggc 3420
   ggcgccagtg gccacgggcg caagcaggac aagcgcatga agtcggcccc gatactgggc 3480
   agcggcggtg cgggcagtgc cggtagcagt gccggcgggc ggacggtggc gcccggtggt 3540
   gcccgaccgg acgatggtac cgggtcgccg ccggacccgc tcgaaccatg gagcaccaag 3600
   aacatcatga acatcaacaa gctgctggac caggacacga ccgaagagta g 3651
<210> 34
   <211> 3261
   <212> DNA
   <213> Tribolium castaneum
<400> 34
   atgggtgcaa aagtgtgcaa accgtgtaaa aagcgcaaag ccaacacctt ccaagggggc 60
   tccatcctcg accgggtcat cagccaagcc tcaaaccaag accagtgcct cctttacaaa 120
   ttagcaaatt acaagaaagg cggcgaactg atcgacgcgt acaaccaagg gggccaagcc 180
   gaggtggaaa aattaatacg cgagcaattc ggccagttga tgtaccagga aggcaaaggc 240
   cagataatca accgctcgga gtatctcagg tggaaatttc gcgaccacga gcaggtcatt 300
   ctcccaatcg aggcctcctt gagccgatac gaccccctgg ccaagtggaa cgaccacgag 360
   gcgtgctggc agatgcagtt cagggggtct ttaggcgagt ctttgctgca cgtcctcata 420
   atttgcgaca cgaaaattca cacaagactg gcgcggactc tgatcaagtg tttcccgaag 480
   ctggccctcg acgttgtcga gggcgaggag tatctagggg cgagtgcttt gcacctagcg 540
   atcgcttaca acaacaatga gctagtccag gacttggtcg aagctggtgc taacgtgaac 600
   cagcgcgcca ttgggagctt tttcctcccg agggaccaac aacgccaaaa acccgcaaaa 660
   cacaccgact acgaggggct cgcctacttg gggaataccc ctctggcctg ggccgcgtgt 720
   tgcgccaacg aaagcgtcta caatctgttg ctggacagtg gggcgcaccc cgactatcag 780
   gacaattttg gaaacatgat cttgcacatg gtggtggttt gcgacaagtt ggacatgttc 840
   ggctacgcct tgagacaccc gaaactaccg gcaagcaacg gaattgtgaa taaagcaggt 900
   ctaactcccc taacgctggc ttgtaagctg ggtcgggctg aggttttccg cgaaatgttg 960
   gagctttcag ccaaggagtt ttggaggtat agtaacatca cctgctcggc gtattttttg 1020
   aacgctttgg atacgctgct accggacggg cgcacaaact ggaattcggc ccttttcatt 1080
   attttaaacg gaaccaagga agagcattta gccatgcttg acggtgggat tatccaacga 1140
   ctgttggagg aaaaatggaa aacattcgca cggaaccaat ttttgaaacg gcttcttatc 1200
   ctcgttgtgc atttgttgtt tctgtctttg gcggtttact tgcgaccaga cgacccagac 1260
   gagtctcttc tcacctggtc tgatgatgtt acactgatag ctagatatgt gtgtgaagtg 1320
   ggcacgattt tgggcgtcct cagttatctg gttttgcaac aaggggacga aatccgtaat 1380
   cagggactta ctgcgttctt gaaacaacaa ctgaattcac ccccgaaatt aattttcctc 1440
   atttcgaatt tcctaatttt ggcgtgcatt ccatgcaggt tgtatggcga taaggaaacc 1500
   gaggaggcta ttctgtgttt tgcggtacct gggtcttggt ttcttctcat gttttttgca 1560
   ggggccgttc gtttgactgg tccttttgtc acaatgattt acagcatgat taccggcgat 1620
   atgttgactt ttggcatcat ttacaccgtt ttcttatttg gtttttcgca gtcgttctat 1680
   ttcctgtata agggattccc cggggtcaaa acttccttat acaacacgta catgtcgact 1740
   tggatggcct tattccaaat tactttgggc aattatgaat attctgagtt gagtgccacc 1800
   acatacccag cggtcagcaa aacagtcttt gccatcttca tggtgttcgt cccaattttg 1860
   ctgctcaaca tgttaatcgc catgatgggc aacacttacg cccatgtaat cgagcaaagc 1920
   gagaaagaat gggtcaaaca atgggcaaaa atcgtcatcg ccctggaacg ggcaatccca 1980
   caatccgacg cccaacatta cttacaagag tacagtattt cgctggggcc cagtgaacag 2040
   gaccccagta ccgaaaaacg cggagttttg gtcataaaat caaagagtaa aacgcgggca 2100
   aaacaacgaa aaggggccgt ggccaattgg aagcgggtgg gcaaagtaac gataaacgcg 2160
   ctcaaaaaac gcggcttaac cggtgaagaa atgcgctgct tgatgtgggg ccgcgagtct 2220
   attaatacac cggtcaaaac aaagaagcct gtgaaggacc cactgctgga cccgcaaggg 2280
   ccgaatttaa cgggtggctt cggcgatgct ttaaccactg ctttggatgt gatgacgttc 2340
   acgcacgatt tggatattgt gggggcttcc cagggcctga atttggccac gaaccctaaa 2400
   cccgtccctc ccacaacaac agcgagtgcc gtgactgtga ataaccagat aaattcggct 2460
   ctgaacgcgc agcaaaaggc cgtagctggg gctggagttg gtgctttggc tatgataggc 2520
   acagtgaccc aactcaccga ctctcaaggc tacattatgc aaaacagtgt gaaaaaagaa 2580
   gaaaaaattg tcgcaacttt ggaggaccct ttcagggaac ttgtcataaa cgctaatgac 2640
   agtaactgcg acccggaaaa attaaaaatg cttgcgctat cggccgccaa tttgaaagac 2700
   gttgaagagt tatcggtggc caagccacaa acgaaaagtg ttaagagtct agcggggatt 2760
   ttcgccggga ctgagacttt tgtgaggaaa gttgaagaaa caataaagaa gaaatatgcg 2820
   gctttggatc caagtgatag tgaaggtttt ggcgaacctc cactattagg gaaaatatcc 2880
   aggactagaa gggccaaatc ggcaaacttg cgcaactctt cagcgcgatc taaagcttca 2940
   gataagaaaa aactggtcgc ggggtcacag tcaagctcca ctgatacggt caataacgaa 3000
   attaatgaaa agaatatcga aaattctgac ttagactatg ccgaagagag gatcaagctg 3060
   gtgaaagagt cgttaaagca ggtggttgac gttgcgcaaa ttcggccgat taacatcgat 3120
   gtggccctcg aggagcaagt gtcggtcaca atttcggaaa cgatgcgagt gcagggctcc 3180
   ggggacgggg ccgaagtcca gtcatccaac gtcaaaccaa agcgcaaaaa acggagcaaa 3240
   acagccaaaa acaacaagta a 3261
<210> 35
   <211> 3306
   <212> DNA
   <213> Megachile rotundata
<400> 35
   atgggtggtg tctgctcctg tcgaggtctc ggtagccagg ttaacgctgg ctcgattctc 60
   gatcgtgtga tcagtcaagc gagcgacgag gatcaatgtt tgctatatcg tctggccaat 120
   tacaagaaga gtggcgagct gatagacgcg tacaatcaag gtggccaggc agaggtggaa 180
   aaattgatca gggaacagtt tggtgtactg atgtacgcgg acggcaaggg tcagatgatc 240
   aatcgggcag agtatctgcg ctggaaattc cgggacctgg aacaggtcgt gcttcctata 300
   gaagcttcct tgtcacgatt cgatcctctg gctcaatgga acgatcacca ggcatgctgg 360
   cagatgcaat acagaggttc cctcggagaa accctcttgc atgttctgat catatgcgat 420
   accaggttac atacacggct ggcgcgtatc ctgctcaagt gctttccacg attggccatc 480
   gacgtagtag agggcgagga atacctcggt gctagtgctc tacacctggc gatcgcgtac 540
   aataataacg aactggtgca agacctcgtt gaagtgggtg cgattatttc tcaacgcgcg 600
   atcggcagtt ttttcctacc cagagatcaa caacgaatga acccggccaa gaataccgat 660
   tacgaaggat tagcctactt aggagagtat cctcttgcct gggccgcctg ctgcgcgaac 720
   gagagcgttt ataacttgct gctcgactcc ggtgcggatc ctgacgaaca ggattctttt 780
   ggcaatatga tactgcacat ggtggtagtt tgtgataaac tggacatgtt cgggtatgcc 840
   ctccgccatc caaagcttcc agctcgcaat gggatcgtga atgccgctgg actgacccca 900
   ttgaccctcg cctgccagct gggtcgtgca gaggtcttcc gggagatgtt ggaactgtct     960
   gcacgagaat tctggcgcta cagtaatatc acctgctcgg cgtaccccct caatgcactc    1020
   gacacgctgt tacccgacgg cagaacaaac tggaactctg cgctatttat cattctgaac 1080
   ggtaccaagg aggaacactt gaacatgctg gacggtggta ttattcagcg attactcgag 1140
   gaaaaatgga agacctttgc gcggttacag ttcttgaagc gtttgatcat cctggtattt 1200
   cacctgacat cactgtctct ggccgtctac ttaagaccct caaacctcga tacggttctc 1260
   ctgaagtggc cagaggaagt aacagaagtt ggtcgaacca tagctgagtg tatcaccgtg 1320
   ctaggcgtcc tgagttatat tttagtgcaa ctgggcggtg aaatcatcaa catcggtgtt 1380
   ctctcgttcc tgaagcagct gagtcacgag cccgccaaac tgatatttct gatcagcaac 1440
   ttgctcatac tggcgtgcat tccgtgtcgt cttgctggaa acaggcatgc ggaagacgca 1500
   atcctcgtcg ttgctgttcc tggttcctgg ttccttttga tgttttttgc tggcgctatc 1560
   cggctgaccg gtccattcgt caccatggtc tacagcatga taaccggcga catgttgacc 1620
   ttcggaataa tctacatggt tgtgctgttc ggtttttgcc agtcgttcta ctttttgtac 1680
   aaaggctttc caggagtgaa atcatccctc tatagttcct atcactcgac atggatggca 1740
   ctgtttcaag taaccctcgg cgattataac tacacagatc ttagttacac aacgtatccg 1800
   aatttatcga agatggtgtt cactatattc atggtcctgg tgcctatact actcctgaac 1860
   atgctaattg ccatgatggg caacacttac gcacacgtga ttgagcaaag cgagaaggag 1920
   tgggtgaagc aatgggcgaa aatcgtggtc tctctggagc gagcagtatc tcaaaaagac 1980
   gctcagaatt atttgcaaga atacagtatt aaattaggac ctggagatga cccgaacaac 2040
   ccagcgtcgg agcaacgtgg tgttctggtg attaaaagta aatcgaaaac caaggcaaag 2100
   caacgcaaag gtgctgttgc taactggaag cgtgtcggaa aagtgacgat caacgaatta 2160
   agaaaacgtg gaatgacggg agaagagtta cgacgaataa tgtggggtcg agcctctttt 2220
   tcgacaccag ttagagtaag tccaaatgta gatgaacctc aggtttcagc ggtaactgct 2280
   ggttttgggg atgcattaac agcagcactg gacgttatgg cgtttgccca tgacttggac 2340
   ctctccactg ccacagaatc gattcctact aatattgacg taaaacaatc gaaaccaaag 2400
   gttgtcagca gcgaacaaac caaattaacg acaaataatc aagaaaaacc atcgataaca 2460
   gaaaaagctg ccacggagat cgcgaataaa caagtaaacg ctaaagatga gaccaataag 2520
   aaacctaatg aagttgtcga cgttgagaaa atgaatgtga agaatgcaaa caattcaagc 2580
   acagttgaag agtatcatga tctgtttttg gaatttgtga ttgcttcgga aagtatgaac 2640
   gatcctgaaa cgctactaaa aatggcggaa cgtgttgctg cagaatttga ttcgtctgcg 2700
   aatcccaaaa taaatttgca aattttggaa cagttcacga tgacgaaaat accgatggag 2760
   gaacaagctg cagctgttaa gaaacaatat tttatcgaat ccagcgataa cgattttggc 2820
   ggcgacaact tgctaggaac ggtggcccgt ttacgtagaa taagatcagc taacagcaga 2880
   tttatcacgg cacggagaca ttcacggcat gtcaatgacg acatgtcatc gacgtcttcc 2940
   gcatccggcg acggaaatcc tcgataccag cagttgctaa acgacactga agaggtaccg 3000
   tcgaatcaac ggaccgaaaa tcgagatgag tcaatcgtgg cgaacaaatc ggaagtaaaa 3060
   caaaacgatg ctaactgcgg atcgaggtgc aaagctcaga agcgtcgacc aaaaactgct 3120
   agaaacaggg tatcgccgaa ggaaacggag gattccggcc aaaggagaag agaatcgatg 3180
   gagcggaata aggcggtttc accggcgaca tcgcccaccg atccgctcga gccatggagc 3240
   acacgcggaa ttaaagacat gaacacgata ttggcttggg aggaaaatgt gccggacagt 3300
   ccttaa 3306
<210> 36
   <211> 3306
   <212> DNA
   <213> Apis mellifera
<400> 36
   atgggtggtg tctgctcgtt tcgaggtcgc ggtagccagg tgaacgctgg ctcgatcctc 60
   gatcgtgtga tcagtcaagc gagcgacgag gatcaatgcc tgttgtatcg tctggccaat 120
   tacaaaaagg gtggcgagct gatcgagtcg tacaatcagg gtggccagtt cgaggtagaa 180
   aaattgatca gggaacagtt cggtgtgttg atgtatgcgg acggtaaggg gcaggtgatc 240
   aatcgtgccg agtatctacg ctggaaattc cgagacctcg agcaagttgt gctccctatc 300
   gaggcttctt tgtcgcagtt cgatcctctg gctcaatgga acgatcacga ggcgtgctgg 360
   caaatgcagt acaggggctc gctgggagag accctccttc atgtcctgat tatatgcgat 420
   accaggatac acacgcgagt ggcccgtatt ctgctcaagt gctttccacg attggccatt 480
   gacgtagtcg agggcgagga gtaccttggc gctagcgcgc tccatctagc gatcgcgtac 540
   aacaataatg aactggtgca agacctggtt gaggcggggg cgatcatttc tcagcgggcg 600
   ataggcagct ttttcctacc gagggaccaa caacgaacga atccggctaa gaatacggat 660
   tacgagggat tagcttatct gggggaatat cccctcgcct gggccgcttg ttgtgcaaac 720
   gagagcgttt acaacttatt actcgactct ggcgctgatc ccgacgagca agactctttt 780
   ggaaacatga tattgcatat ggtcgtggtt tgtgataaat tggacatgtt cggatatgct 840
   ctccgtcatc cgaagcttcc tgctcgcaat gggattgtga atgctgccgg actgacccca 900
   ttgacgctcg cctgccagct gggccgtgct gaggtctttc gggagatgtt ggaactgtcc 960
   gcacgagaat tctggcgcta cagtaacatc acctgctcgg cgtatcccct gaatgcgctc 1020
   gacacgctgt tgcccgatgg cagaacaaac tggaactcgg cgctatttat cattctgaac 1080
   ggtacgaagg aggagcactt ggacatgctg gacggtggta ttattcagcg attactcgag 1140
   gaaaaatgga agacctttgc gcggttgcag ttcttaaaac gactgatcat cttggcattc 1200
   catctgacgt ccttgtcgct ggcagtatat ctgaggcctt ccaacacaga tgctcagctc 1260
   ttgaaatggc cggaagaaat cacggaagtc gcacgcacga tcgccgagtg catcacggtg 1320
   ctgggcgtgc tcagttacat tctggtccaa ctaggcggtg agatcatcaa cattggtctc 1380
   ttgtccttca tgaaacagtt gagtcacgag cccgccaagc tcatattctt gatcagcaac 1440
   ctgctcatac tcgcgtgcat tccatgtcgt cttgccggta ataggcatgc ggaagacgcg 1500
   attctcatag tcgctgtgcc cggctcttgg ttccttctca tgttcttcgc tggggctgtc 1560
   cggctgaccg gtccattcgt caccatggtg tacagcatga taacgggaga catgttgacc 1620
   ttcggtataa tatacatggt ggtgctgttc ggcttctgcc agtcgttcta ctttttgtac 1680
   aaaggattcc cgggagtgaa atcatccctc tacagttcct atcactcgac ctggatggca 1740
   ctgtttcaga taaccctcgg cgattacaac tacacggatc tcagctacac gacctatcct 1800
   aatttatcga agatggtgtt cgccatattt atggtactgg tgcctatact attgttgaac 1860
   atgctaatcg ccatgatggg caacacatac gctcatgtga tcgagcagag tgaaaaggag 1920
   tgggtgaaac aatgggctaa aatcgtggtt tctctggaac gtgcagtctc acagaaggat 1980
   gcacagaatt atttgcaaga atatagcatt aaactgggac ctggagatga cccaaacaat 2040
   cctgccgcgg agcagcgagg agttttgatt attaaaagta aatcgaaaac caaggcgaag 2100
   caacgcaaag gcgcggtagc taattggaag cgtgtgggca aagtgacgat aaacgaatta 2160
   aaaaaacgtg gtttaacagg agaggaattg cgacgaatta tgtggggacg agcttctttt 2220
   tctacacctg tcagagtaag tccaaaaggg gtcgagcctc aagtttctgt ggtgaccgcc 2280
   ggtttcgggg acgcattgac cacagctttg gatgtcatga cgttcgccca cgatctcgac 2340
   ttgtccaccg caacggaagg aattccaacc aacatcgatg ccaagcagtc gaaaccaaag 2400
   agtgctacca aggagacgaa atcgacggtg aataatcagc agaacgtgac gacaaacata 2460
   gaaccattga aatcgaccac ggaaaatgtg gacgatcagg cgaacaatcc tagggaaaaa 2520
   aaagtaacta gccaagctgc gaccgtacac gagatgaatt tgaagaatgc gaatcattcg 2580
   agcgtgaccg aggattttca ggatccgctt ttggaacttg tgattgcttc cgaaaatacg 2640
   aacgatcccg aaaccctgct cgaaatagcg aaacgtgccg cggcaggctt cgaaacagaa 2700
   accagctcta aaataaatct tcaaatttta gaacaattca cgatgacgaa gatacccatg 2760
   gatgagaagg tcaacgttac gaggaaacaa tatttcgtcg aatccagtga caatgacttt 2820
   ggaggcgaca acttgctcgg gactgaggcc cgtctacgta gaataagatc agcgaacaac 2880
   agatttatca cgacacgtag acggtcacgg aacgtcgacg acgacctgtc atccacatct 2940
   tccacgtcta tggacagaaa tcctcgatat cagtcgttgc tcaacggcca tgaaaactcg 3000
   atcgaccgtc caatcgagag tcgagagtgt tccatagaag caattaactc gcaaataaag 3060
   cagaacggac cgtgcgaaac gatgaaggcc aaggtccaga agaaaagacc aaaaacggcc 3120
   agaaataggg tatcgccgaa ggaaatagag aaggctggtc cgaggagaag agaatcgatc 3180
   gagcggaata aggcggtttc accggcaaca tcgcccacgg atccgctcga gccatggagc 3240
   acacgcggca ttaaggacat gaacacgata ttggcttggg aagagaccgc gccggacagt 3300
   ccttaa 3306
<210> 37
   <211> 3318
   <212> DNA
   <213> Apis florea
<400> 37
   atgggtggtg tctgctcgtt tcgaggtcgc ggtagccagg tgaactctgg ctcgatcctt 60
   gatcgtgtga tcagtcaagc gagcgacgag gatcaatgtc tgttgtatcg tctggccaat 120
   tacaaaaagg gtggcgagct gatcgagtcg tacaatcaag gtggccagtt cgaggtagaa 180
   aaattgatca gggaacaatt cggtgtattg atgtatgcga acggtaaggg ccaggtgatt 240
   aatcgtgccg agtatctacg ctggaaattc cgagacctcg agcaagttgt gctccctatc 300
   gaagcttctt tgtcacagtt cgaccctttg gctcaatgga acgatcacga agcgtgctgg 360
   caaatgcagt acagaggttc gctaggagag actctcctgc atgttctgat tatatgcgat 420
   accaggatac acacacgagt ggcgcgtatt ctgctcaagt gctttccacg attggccatt 480
   gacgtagtcg agggtgagga gtaccttggc gctagcgcgc tccatctagc gatcgcgtac 540
   aacaataatg aactggtgca agacctcgtt gaggcgggag cgatcatttc tcagcgggcg 600
   ataggcagct ttttcctacc cagggaccaa caacgaatga atccggctaa gaatacggat 660
   tacgagggat tagcttatct gggggaatat cccctcgcct gggccgcttg ttgtgcaaac 720
   gagagcgttt acaacttatt actcgactct ggcgctgatc ccgacgagca agactctttc 780
   ggaaacatga tactgcatat ggtagtggtt tgtgataaat tggacatgtt cggatatgct 840
   ctccgtcatc cgaagcttcc tgctcgcaat gggattgtga atgctgccgg actgacccca 900
   ttgacgctcg cctgccagtt gggccgtgct gaagtctttc gggagatgtt ggaactgtcc 960
   gcacgagaat tctggcgcta cagtaacatc acctgctcgg catatcccct gaatgcgctc 1020
   gatacgctgt tgcccgatgg tagaacaaac tggaactcgg cgctatttat cattctgaac 1080
   ggtacgaagg aggagcactt ggacatgctg gacggtggta ttattcagcg attactcgag 1140
   gaaaaatgga agacctttgc gcggttgcag tttttaaaac gactaatcat cttggcattc 1200
   catctgacgt ccttgtcgct ggcagtgtac ctgagacctt ccaacacaga tgctcaactc 1260
   ttgaaatggc cggaagaaat cacggaagtc gcacgcacga tcgccgagtg catcacggtg 1320
   ctaggcgtgc tcagttatat tctggtccaa ctaggtggcg agatcatcaa cattggtctc 1380
   ctgtccttca tgaaacagtt gagtcacgag cccgccaagc tcatattctt gatcagcaac 1440
   ctgctcatac tcgcgtgcat tccatgtcgt cttgccggta ataggcatgc ggaagacgcg 1500
   atactcatag tcgctgtgcc cggctcttgg ttccttctca tgttcttcgc tggggctgtc 1560
   cggctgaccg gtccattcgt caccatggtg tatagcatga taacgggaga catgttgacc 1620
   ttcggtataa tatacatggt ggtactgttc ggcttctgtc agtcgttcta ctttttgtac 1680
   aaaggattcc cgggagtgaa atcatccctc tacagttcct atcactcgac ctggatggca 1740
   ctgtttcaga taaccctcgg cgattacaac tacacggatc tcagctacac gacctatcct 1800
   aatttatcga agatggtgtt cgctatattt atggtactgg tgcctatact attgttgaac 1860
   atgctaatcg ccatgatggg caacacgtat gcccacgtga ttgagcagag tgaaaaggaa 1920
   tgggtgaaac aatgggcgaa aatcgtggtt tctctggaac gtgcagtttc acagaaggat 1980
   gcacagaatt atttgcaaga atatagcatt aaactgggac ctggagatga cccaaacaat 2040
   cctgcctcgg agcaacgagg agtcttgatt attaaaagta aatcgaaaac caaggcgaag 2100
   caacgcaaag gcgcggtggc taattggaag cgtgtgggca aggtgacgat aaatgaatta 2160
   aagaaacgtg gcttaacagg agaggaattg cgacgaatta tgtggggtcg agcttctttt 2220
   tctacacctg taagagtaag tccaaaaggg gtcgagcctc aagtttctgt ggtgactgcc 2280
   ggtttcggag acgcgttgac cacagctttg gatgtcatgg cgttcgccca tgatctcgac 2340
   ttgtctaccg caacggaagg aattccaacc aacatcgatg ccaagcagtc gaaaccgaag 2400
   agtgctatca aggagcagat gaaattgacg acgaataatc aggagaacgt gtcgactaac 2460
   gtagaaccgc agctgaaatc gaccacggaa aatgcgaacg atcaggcgaa caatcctagg 2520
   gaaattaaaa aagtaactaa ccaggttaca ggcatacacg agatgaattt gaagaacgcg 2580
   aatcattcga gcgtgaccga ggattttcag gatccacttt tggaacttgt gattgcttcc 2640
   gaaaatacga acgatcccga aaccctgctc gaaatagcga aacgtgccgc ggcaggcttc 2700
   gaaacagaag ccagctctaa aataaatctt caaattttag aacaattcac gatgacgaag 2760
   atacctatgg aggagaaggt cagcgttaca aggaaacaat atttcgtcga gtctagtgac 2820
   aatgactttg gaggcgacaa cctgctcggg actgaggccc gtctacgtag aataagatca 2880
   gcgaacaaca gatttatcac gacacgtaga cggtcacgga acgtcgacga cgacctgtca 2940
   tccacatctt ccacgtccat ggacagaaat cctcgatatc agtcgttgtt caacggccat 3000
   gaaaactcga tcgaccgtcc aatcgagagt cgagagtgtt ccatggaagc aattaactcg 3060
   caaataaagc agaacgggcc accgtgcgaa acagtgaagg ccaaggtcca gaagaaaaga 3120
   ccaaaaacgg ccaaaaatag ggtatcgccg aaggaaatag agaaggctgg tccgaggaga 3180
   agagaatcga tcgagcggaa taaggcggtt tcaccggcaa catcgcccac ggatccgctc 3240
   gagccatgga gcacacgcgg cattaaggac atgaacacga tattggcttg ggaagagacc 3300
   gcgccggaca gtccttaa 3318
<210> 38
   <211> 3168
   <212> DNA
   <213> Pediculus humanus
<220>
   <221> source
   <223> /note="Pediculus humanus corporis"
<400> 38
   atgggagctg gattatgtgg aacttctcaa gatcctcaaa atcaaggttc ggttttggat 60
   agagtcattt ctcaagcttc taacaaagat gattgtcttt tatataaact tgccaactac 120
   aaaaacagtg gagaattgat cgaagcatac aatattggag gtcaagctga ggttgaaaaa 180
   ctgattaaag aacaatttgg agttttaatg tatgccgatg gcaaaggtga agtaattaaa 240
   cgtgcagaat accttaggtg gaaatttcga gatcaagctc aggtcgtttt acctatcgaa 300
   gcttctttat ctatttacga tcctttggca aaatgggaag accacgaagc ttgttggcaa 360
   atgcagtaca ggggtagttt aggagaaacg cttcttcatg ttttgataat ttgcgattct 420
   aaaatacaca caaaacttgc tcgtaccctt ttaaaatgct ttcctaaact tgctttggat 480
   atagtagaag gagaagaata tttgggagct agtgctcttc atttagccat tgcttataat 540
   aataacgaat tagttgaaga tttggtagat gctggagcca acattaatca aagagcagta 600
   ggtagttttt ttcttcctaa agatcaacaa cgagcaaaac ctttaaaaac cacagattac 660
   gaaggtttgg catatttagg agaatatcct ttatcgtggg cagcttgctg ctcgaatgaa 720
   agcgtctaca atttacttct tgatgtcggt gccgatcctg acagtcagga ttcttttgga 780
   aatatgatat tgcacatggt ggtcgtttgt gataaactgg acatgtttgg ttatgctctt 840
   cgtcatccaa aagtaccagc tagtaatgga ataattaaca acgagggact tactccccta 900
   acattggcat gtaaattggg tagagcagat gtttttaaag aaatgttaga actgagtgct 960
   aaagaatttt ggcgttacag caacatcact tgttcggcat atccactaaa tgcacttgac 1020
   actctattac cagatggaag aaccaattgg aattctgcaa tttttattat tttaaacggt 1080
   accaaagaag aacatttaga catgttagat ggtggaataa tccaaaggct tttggaagaa 1140
   aaatggaaaa cttttgcaag aaaccaattt ttaaaacgtt tggttatttt cttcttacat 1200
   atattttgcc taagtggttc tgtatattta agaccggacg atagaaataa accacttttg 1260
   ggtggtactt ccgttcaaga tgttgtcagg tattgtttcg aaatcggtac catactcgga 1320
   gttctttgtt atttatgttt tcaacaagga gatgaaatta gaaatcaagg attgattagt 1380
   tttttaaaac aattgccgca tgatcctgca aagtttatat ttctcatatc aaatctttta 1440
   attttggcct gtatacctta ccgtgtggct ggtgatacgg atacggaaga agccatactt 1500
   gtatttgcgg tacctagttc gtggtttctg ttaatgtttt ttgccggagc gattcgatta 1560
   actggtccat ttgtcaccat gatttacagt atgatcactg gagatatgtt aacctttggt 1620
   attatatatt cggtattttt atttggtttt tctcaaagtt ttttttccct atacaaaggc 1680
   tctaaaaatg tgagcagttc tctatttact tcatatccat caacttggat ggctcttttt 1740
   caagtcacaa tgggagatta taactacaat gatttatctc taacagcata tccagccatt 1800
   agcaaaatgg tattcacaat atttatggtt ttggtaccca ttcttttgtt aaacatgttg 1860
   attgccatga tgggcaatac ttatgctcat gttattgaac aaagcgaaaa agaatggatg 1920
   aaacagtggg ctaaaattgt tgtggctttg gaacgtgcgg taaatcagga agattgtcat 1980
   cgttacttac aagaatattc tattaaatta ggtcctggtg atgatcctag caccgagcaa 2040
   agaggtgttt tggtaattaa atctaaaagt aaaacaagag caaaacaaag aaaaggcgca 2100
   ctttgtaatt ggaagagggt tggaaaagta accattcgag aattgcataa gagaggaatg 2160
   accggagaac aattgcgacg tcttatgtgg ggaagatcat caatttcaac ccccgtcaaa 2220
   cctgcaccaa taaaattagg ccacgttacg agcatctcgg gtgtggctga tataaccgtt 2280
   ccagaaacga caggaaacac tgttggaacg ggaggaggcg gtttgttagc tgctttggac 2340
   gttatggcat ttacaaatga tttggaattc agttcggaaa atcaaacttc gaccagtttc 2400
   aaaactcaac ctcaaccatc agaagtttct tgcgttgaat cggattcgaa ttcagatcct 2460
   ttatataatg atcctctgag gcaattgaat gaagaactca ataaaactcg ggaagaaatt 2520
   ttaactttag ctcgagttgc agcaaataat gatggaattg aagagatacc accgcaaata 2580
   tgtcaatacg gttggattaa taattataat tttttcggta atacatttgc acaaaatcaa 2640
   aaagttgagg atcctgttca acagcctaca tttcaatcta caccaaatca tagcgacagt 2700
   gatggtttag gagatggatt actccttgga agtaatcgaa gattaaaaag aactcgatcg 2760
   gcaaatataa aaagaaagtt ttctggaagt tcgacaaatg aaaaaaattt attagaacct 2820
   gaggattcaa gttcaactaa ttccgaggat ttttcatgtt taaccgaaaa ttacataaat 2880
   aaaaatatta aagttaatgc aaatttttcg gagaattctt tacgaagtaa tagaagttct 2940
   gccaatctcg tggtgaaaaa atttggaaga aggtcgggtt tgaaatcctc taccaacaga 3000
   attgctcctg ctgatttaag tccagttggt aatgtttcca aatactctga aactttgtgt 3060
   aactcatatt gtattacttc ttcatcggat gttttgtatc aatggagtat aaaaggaata 3120
   acaaatatga atacactttt aggattggaa aatgaggatt caatgtaa 3168
<210> 39
   <211> 3330
   <212> DNA
   <213> Acromyrmex echinatior
<400> 39
   atgggtggtg tttgctcgtg ccgcgttggt cgcaacagcc aggtcaacgc cggttcgatc      60
   ctcgaccgcg tgatcagcca ggcaagcaac gaggaccagt gtttattgta tcgattggcg     120
   aactacaaga agggcggtga gctaatcgag gcgtacaacc agggcggtca gcctgaggtg     180
   gagaagctga tccgcgagca atttggtatc ctgatgtata tggatggcaa aggccagata     240
   atcaatcgtg ccgagtatct acgttggaaa tttcgtgacc tcgagcaagt cgtgttgccg     300
   atcgaggcct cgctgtcgcg ttttgatcca ctagcgcagt ggaatgatca tgaagcctgc     360
   tggcagatgc agtacagagg ctcgctgggc gagaccttgt tgcatgttct tatcatatgt     420
   gacacgcgga tgcatactag aatagcgcgc atactgctca agtgttttcc gcggctagct     480
   attgacgtcg tcgagggcga agagtatctg ggtgcgagtg ctcttcatct ggcgatcgcc     540
   tatgcgaaca acgagctcgt gcaggatctg gtcgaggcgg gcgcgattat ctcgcagcgg     600
   gccatcggta gcttcttcct gccgcgggat cagcaaagga tgaatcccgc cagaaatacc     660
   gattacgagg gtctcgcgta tctgggagag tatcctctcg cctgggccgc ctgctgcgct     720
   aatgagagcg tctacaacct gctgcttgat tccggcgctg atcctgacga gcaggatact 780
   tttggaaata tgattttgca catggtcgta gtttgcgaca aattggacat gttcggctac 840
   gcattgcgac atccgaagct tcctgctcgc aatggaattg tgaatgccgc gggcctgacc 900
   ccgttgacac tcgcctgcca gctggggcgt gccgaggtct tccgtgagat gttggagctg 960
   tccgcgcggg agttttggcg ttacagtaat atcacttgct cggcgtaccc tctcaatgct 1020
   ctcgacacgc tgttgcccga tggcagaaca aactggaact ctgcgctatt tatcattctg 1080
   aacggtacga aggaggagca cttggacatg ctggacggtg gtattattca gcgattactt 1140
   gaggaaaaat ggaagacctt cgcgcgattg cagttcctga agcgattgat tatcttggtg 1200
   tttcacctgg cctcgctgtc cttagccatg tacttcaggc ccgcggacat ggaggctgtg 1260
   ctgctgcagt ggcccgagga gataacggac gtcgtccgca ccgcggcgga gtgcatcacc 1320
   gtgctgggcg tgctgaatta catcctggtg cagctgggat gcgaaatgat caacgtcggt 1380
   ccgctatcat tcctgaagca actgagccac gaaccggcca agttgatatt cgtgatcagc 1440
   aatctgctca ttctcgcgtg cattccatgt cgtatcgctg gcgatcggca cgcggaggat 1500
   gcgatactga tgttcgctgt gccgggctca tggtttcttc ttatgttttt cgccggagcg 1560
   attcgattga ctggtccatt tgtcacaatg gtatacagca tgataacggg cgatatgctg 1620
   acctttggta ttatctacac ggttatgctc tttggattct cgcaatcatt ttactttctt 1680
   tataagggat tccccggagt gaaatcatct ctgtatcatt cctatccttc gacctggatg 1740
   gcactgtttc agataaccct cggcgattat aactacgcgg atttgagtaa cacgacctat 1800
   cctaacttga gcaagaccgt ctttgcgatt ttcatggtac ttgtgcccat actgctgctc 1860
   aacatgctaa ttgccatgat gggtaataca tatgcacatg ttatcgagca aagcgaaaag 1920
   gaattcgtca agcagtgggc aaaaatcgtg gtgtctttgg aacgcgccgt atctcagaaa 1980
   gatgctcata attacctaca ggaatacagc attaaactgg gacccggcga tgatccgaac 2040
   aaccccgcgt cggaacaaag gggcgtgttg gttattaaaa gtaaatcgaa aaccagagca 2100
   aagcagcgta agggcgctgt gtctaattgg aagcgagtcg gaaaagtcac gatactcgaa 2160
   ttgagaaaac gaggtatgac tggcgaggaa ctccgtcgta taatgtgggg tcgcgcgtcc 2220
   ttttccaccc ctgtgcgagt cagtcccaat gccggtcaac cgcaagtgtc ggcagttact 2280
   gcaggttttg atgatgcgtt aaccgcagct ttggacgtaa tggcctttgc acatgacttt 2340
   gacttatcca ctgcgacgga aggcatacct accaatatcg acgcaaaaca gataaagaca 2400
   gcgcagtcga aagccgctct caacgaccag cagacgaaaa caacccaaaa taatccagaa 2460
   gtggtgataa atgtgcaacc gcataatcaa cccattaaag aggaaactaa attatcaggt 2520
   ctcacagatt taatcgataa gtctatcgat cccgtaaagg atgtggaggc aatgaatttg 2580
   agaaatatga atcaaacgaa agcgctcgaa gaaaccgtcg aagatccatt tttggaactc 2640
   gctatcgctt ctgagactac aacggattac aaaactctac tgcaaatagc gaagtccgct 2700
   ttagccacaa gcgaagcttc aatgaaaacc acaatcgacc cgcaaattct agcacacttc 2760
   gccatggtcg cgccgccgac cattgagaaa tcttctatcg ttaagaaaca gtatttcatg 2820
   gagtccagcg acaatgacct cggcggcgat aatttacttg gcaccgaagc tcgccttcga 2880
   aggataaaat ccgcgaataa cagattcact acatcgaaac ggtcacgccg tgatgacgat 2940
   ggcgacagtt cctcatctac aacatccgtc gaacaaagtc tgcgatatcg accactactg 3000
   aatgatccgg aacagagacc gatgaatcgt atcgagactg aaggacgaaa gaccaccgtg 3060
   gaaacaatta agccggaggt caaacagaat ggcagcattc ctaccaaacc actcgataaa 3120
   atccaaaaac gtcgaccaaa aacggccaag aatagggtat cgcctaaaga aactgaggaa 3180
   ccagcgagga gaagggaatc gattgagcgg aataaggcgg cttcaccacc tacgtcgtcc 3240
   tcggatccgc tcgagccatg gagcacgcgt ggcattaccg acatgaacac aatattggct 3300
   tggcgggaaa acgcaccgga cagtccttaa 3330
<210> 40
   <211> 3339
   <212> DNA
   <213> Harpegnathos saltator
<400> 40
   atgggtggtg tttgctcgtg tcgcggccgc ggcagccagg tcaacgccgg ctcgatcctc      60
   gaccgcgtga tcagccaggc gagcaacgag gaccagtgtt tgttgtatcg gctggccaac     120
   tacaagaagg gcggcgagct gatagaggct tacaaccagg gcggccagcc ggaggtcgag     180
   agactgataa gagagcaatt cggcatcctg atgtacgcgg acggcaaggg tcaggtgatc     240
   aatcgcgccg agtatctacg ctggaagttt cgcgacctgg agcaggtcgt gctgccgatc     300
   gaggcgtcgt tgtcgcgctt cgacccgttg gcacagtggt acgaccacga agcctgctgg     360
   cagatgcagt acagaggctc gctgggagag agcctgctgc acgtgctcat catatgcgac     420
   acgcgtatgc acacgaggat agcgcgcacg ttgctcaagt gcttcccacg gctggccatc     480
   gacgtggtcg agggcgagga atacctcggc gctagtgccc ttcatctggc gatcgcctac     540
   aacaacaacg agctcgtgca ggatctggtc gaggcgggcg cgatcatctc gcaacgggct     600
   atcggcagct tcttcctgcc cagagaccag cagaggatga atcccgtaag gaacaccgat     660
   tacgagggac tcgcgtatct cggggagtac ccgctcgcgt gggccgcgtg ctgcgccaac     720
   gaaagcgttt acaacctgct gctcgactcc ggcgccgatc ctgacgagca ggattctttc     780
   ggaaatatga ttttgcacat ggttgtagtg tgcgacaaat tggacatgtt cggctatgcc     840
   ctgcgacatc ccaagcttcc cactcgcaac ggaatcgtga atgccgcagg cctgacccca     900
   ttgacactcg cctgccagct ggggcgtgcc gaggtcttcc gtgagatgct ggagctgtcc 960
   gcacgggagt tctggcgtta cagtaatatc acctgctcgg cgtatccact caatgctctc 1020
   gacacgctgc tcccggacgg tagaacaaac tggaactctg cgctatttat cattctgaac 1080
   ggtacgaagg aggagcactt ggacatgctg gacggtggtg ttattcagcg attacttgag 1140
   gaaaaatgga agacctttgc gcgattgcag ttcctgaagc gattgattat cttggtgttt 1200
   cacctggcct cgctgtcctt agccgtgtac ttcaggcccg cggacatgga cgccgagttg 1260
   ctgcagtggc ccgacgagat tacggacgcc gcccgcatcg cggcggaatg cagtaccgtg 1320
   ctgggtgtgc taagttacat cctggtgcaa ctgggcggcg agatgatcaa catcggtccg 1380
   ctgtcgttcc tgaagcaact gagccacgaa ccggccaagt tgatattcgt gatcagcaat 1440
   ctgctcattc tcgcgtgcat tccatgtcgt atcgccggca atcggcacgc ggaggatgcg 1500
   atactggtgt tcgccgtgcc gggctcgtgg tttcttctca tgtttttcgc cggagctatc 1560
   cgactgaccg gtccgttcgt cacgatggtg tacagcatga taaccggcga catgctgacc 1620
   ttcggcatca tttacacggt catgctcttt ggattttctc aatcgtttta tttcctctac 1680
   aagggattcc cgggcgtgaa gtcgtccctc taccattcct atccttcgac ctggatggcg 1740
   ttatttcaga taaccttggg cgattataac tacgcagacc taagtaacac gacctatccc 1800
   aacttgagta agacggtctt tgcgatcttc atggtgctcg tgccgatatt gctgctcaat 1860
   atgctgatcg ccatgatggg taatacgtac gcgcacgtta tcgagcagag cgagaaggaa 1920
   tttgtgaagc agtgggccaa aattgtggta tctttggaac gcgctgtgtc gcagaaggac 1980
   gcccataatt atctacagga atacagtatc aagttggggc cgggcgacga tccgaataat 2040
   cccgcatcgg agcagagagg tgtgctgatt attaaaagca aatcgaaaac tagagcgaaa 2100
   cagcgcaagg gcgccgtggc taattggaag cgagtcggaa aagtcacgat aaacgagctg 2160
   agaaaacgag gtatgactgg cgaggagcta cgccgtataa tgtgggatcg tgcgtccatc 2220
   tccactcctg tgcgagtcag tcctaatgtc aacgagccac aagtgtcggc agttactgca 2280
   ggttttggtg acgcgctaac cgcagctttg gacgtaatgg cctttgcgca cgactttgac 2340
   ttatccactg cgacggaagg cgtaccgagc aatatcgaca cgaggcagac gaaagccatc 2400
   cccgacgagc agacgagaac ggctcggaat aatccaaagg cggtgacgga tgtccaaccg 2460
   cacaaacaag ccatcggggg ggaggagacc aagttgtcag gtctcacgga tctaattgat 2520
   atgtccgccg ccgaaccggc gcgcgtggaa gcgatgaact tgaagaacgc gagtcaagca 2580
   aaggcgcacg agggagctgt cgaggatcca ttcctggagc tcgctatcgc ctccgagact 2640
   acggaagatt acgatacttt attgcaaatg gccaagtccg ccctagctac cagtgaggct 2700
   tcaaccacgg tcgacccaca aattctagca cacttcacta tgatcgcccc gccgcccgtt 2760
   gaaaaaccca tcgttgttaa gaaacaatac ttggtggagt ccagtgacaa cgatctcggt 2820
   ggcgacaatt tgctcggtac cgaagctcgc atcaggagga taaagtcggc gaatgataga 2880
   ttcatcagca catccaaacg atcacaccat gacgacgata acgacagttc ctcgtccgtc 2940
   acttccgtcg accggagtca gccgcagtat cgaccgctgc tgaatgattc ggaagagagc 3000
   ctggcgaacc gcgtcgagaa cgaaggacga agaacttccg acgtcgaaac aattaagtcg 3060
   gaagtcaagc agaacggccg cggcggcggc ggcggcggtg tttccaagcc gtcggataaa 3120
   cgcgtccaga aacgccggcc gaaaacggcc aagaacaggg tatcgcctaa ggaaatagag 3180
   gaaccggcga ggagacgaga agaatcgatt gagcggaata aggcggctct ttcacctccg 3240
   gcgtcgccct cggatccgct cgagccatgg agcacgcgtg gcattaccga catgaacacg 3300
   atattggcct ggcgggagaa tgcaccggac agtccttaa 3339
<210> 41
   <211> 3315
   <212> DNA
   <213> Bombus impatiens
<400> 41
   atgggtggtg tctgctcgtg tcgaggtcgc ggtagccagg tgaatgctgg ctcgatcctt      60
   gatcgagtga tcagtcatgc gagcgacgag gatcaatgtc tattgtatcg tctggccaat     120
   tataagaagg gcggcgagct gatcgagtcg tacaatcaag gtggccaagc ggaagtggaa     180
   aaattgatca gggaacagtt cggtgtactg atgtacgcag atgggaaggg tcaggcgatc     240
   aatcgggcgg aatatctacg ttggaaattc cgtgacctcg agcaggttgt gctaccaata     300
   gaggcttctt tgtcacagtt tgatcctctg gctcaatgga acgatcatga agcatgttgg     360
   cagatgcaat atagaggatc actcggagag actctcctgc atgtcctgat tatatgcgat     420
   accaggatac acacacgact agcgcgtatt ctgcttaagt gctttccacg attggctatt     480
   gacgtagtcg agggcgagga gtacctcggc gctagcgctc tgcatctcgc tatcgcgtac     540
   aacaataatg aactggttca agatctcgtg gaggcgggag cgattatttc tcagagggcg     600
   ataggcagct ttttcctgcc tagggaccaa caacgaacga atccagctaa gaatacggat     660
   tacgagggat tagcatacct gggagagtat cccctcgcct gggctgcttg ctgtgcaaac     720
   gagagtgttt acaacttatt actcgactct ggcgctgatc ccgacgagca ggactctttt     780
   gggaacatga tactacatat ggtagtcgtt tgtgataaat tggacatgtt cgggtatgcc     840
   ctccgtcatc cgaagcttcc tgctcgcaat gggatcgtga atgccgccgg actgacccca     900
   ttgacgctcg cctgccagct gggtcgcgca gaggtctttc gggagatgtt ggaactgtct     960
   gcacgagaat tctggcgtta cagtaatatc acctgctcgg catatccgct gaatgcactc    1020
   gacacgctgt tacctgacgg cagaacaaac tggaactccg cgctatttat cattctgaac    1080
   ggtacgaagg aggagcactt ggacatgctg gacggtggta ttattcagcg attactcgag    1140
   gaaaaatgga agacctttgc gcggttgcag ttcttgaaac ggttgatcat cctggtgttc 1200
   catctgacat cactctcgct agccgtgtat atgagaccgt caaacatcga tgccaccctc 1260
   ctggaatggc cggaagaaat cacggaagtt ggacgcatga tcgcggagtg caccacggta 1320
   ataggtgtgc tgagctacat tttggtgcaa ctaggcggcg aggtagtcaa cattggtctt 1380
   ctatcgttca tgaaacaatt gagtcacgag cctgccaagc tcatattctt gatcagcaat 1440
   ctgctcatac tcgcgtgcat accatgtcgt cttgctggta ataggcatgc tgaagacgcg 1500
   atactcgtgg ttgctgttcc cggctcttgg ttcctcctca tgttctttgc tggcgctatc 1560
   cggttgaccg gtccattcgt taccatggtc tacagtatga taaccggtga catgttgacc 1620
   ttcgggataa tctacatggt ggtgctgttc ggattttgcc agtcgttcta ctttttgtac 1680
   aaaggcttcc cgggagtgaa atcatccctc tacagttcct atcactcgac ctggatggca 1740
   ctgtttcaga taaccctcgg cgattataac tatacggacc tcggctacac aacctaccct 1800
   aatttatcga agatggtgtt cgccatattt atggtcctgg tgcccatact gctgttgaat 1860
   atgctgattg cgatgatggg caacacttac gcgcatgtga tcgagcagag cgagaaggaa 1920
   tgggtgaagc aatgggcgaa aatcgtggtt tctctggagc gtgcagtcgc gcagaaagat 1980
   gcgcagaatt atttgcaaga atacagtatt aaactgggac ctggagatga cccgaacaac 2040
   cccgcctcgg aacagcgcgg agtcctgatt attaaaagta aatcgaaaac caaggcgaag 2100
   caacgcaaag gcgcggtggc taattggaag cgcgtaggca aagtgacgat aaacgaattg 2160
   aagaaacgtg gtataacagg agaggcgttg cgacgagtca tgtggggccg agcatctttc 2220
   tccactcctg ttagaacgag tccaaacact gccgagcctc aagtttcagc ggttacggcc 2280
   ggttttggag acgcattgac tgcagcgctg gatgtgatgg cgttcgccca cgatctcgac 2340
   ttgtctactg ctacggaagg aattccgacc aacatcggcg cgaagcagtc gaaatcgaag 2400
   accgcctgca aggatcaagc gaaatcggcg acgaataatc aggagaaagg gtcgacggac 2460
   gttcagcaac aacggaaact aaccacggaa aatctggata atctggcgag cagtcataat 2520
   gatattgaca aaacatccag cgaatttgtg gccgttcgcg agatgaattc gaagaacgcg 2580
   agtcattcga gcatggccga ggattttcag gatccgcttt tggagcttgt gattgcttcc 2640
   gaaaatacgg acgatcctga aacgctgctg aaaatagcgg agcgggccgc agcgaatttc 2700
   gaagtggaga gcagttcgaa aataaatctg caaattttgg agcaattcac gatgactaag 2760
   ctacctatgg aggagaaagt tgccgctaca aagaagcagt actttgttga atcgagtgac 2820
   aacgattttg gtggcgacaa cctactcgga accgaggccc gtctacgtag aataagatca 2880
   gcgaacaaca gatttatcac gacacggaga cggtcgcggc acaccgatga cgacctgtca 2940
   tctacgtcgt ccacatccgc cgacagaaat cctcaatatc agccattgct caacggccct 3000
   gagaacttgg cgagccgtca aatcgaaagt cgagagagct ctatcgaagc aattaagccg 3060
   caagtaaagc agaacgggtc atgcgattca gtgaagatca aggttcagaa gcgtcgaccg 3120
   aaaacggcga aaaatagggt atcaccgaag gaaatagagg aggctggtcc gaggagaaga 3180
   gaatcgatcg agcggaataa ggcggtttcg ccggcgacgt cgcctactga tccgctcgaa 3240
   ccatggagca cacgcggcat taaggacatg aacacgatat tggcttggga ggagaatacg 3300
   ccggacagtc cttaa 3315
<210> 42
   <211> 3075
   <212> DNA
   <213> Bombus terrestris
<400> 42
   atgggtggtg tctgctcgtg tcgaggtcgc ggtagccagg tgaatgctgg ctcgatcctt 60
   gatcgagtga tcagtcatgc aagcgacgag gatcaatgtc tattgtatcg tctggccaat 120
   tacaagaagg gtggcgagct gatcgagtcg tacaatcaag gtggccaagc ggaagtggaa 180
   aaattgatca gggaacagtt cggtgtactg atgtacgcag atgggaaggg tcaggcgatc 240
   aatcgggcgg aatatctacg ttggaaattc cgtgacctcg agcaggttgt gctgccaata 300
   gaggcttctt tgtcacagtt cgatcctcta gctcaatgga acgatcatga agcatgttgg 360
   cagatgcaat atagaggatc actcggagag actctcctgc atgtcctgat tatatgcgat 420
   accaggatac acacacgact agcgcgtatt ctgcttaagt gctttccacg attggctatt 480
   gacgtagtcg agggcgagga gtacctcggc gctagcgctc tgcatctcgc tatcgcgtac 540
   aacaataatg aactggttca agatctcgtg gaggcgggcg cgattatttc tcagagggcg 600
   ataggcagct ttttcctgcc tagggaccaa caacgaacga atccagctaa gaatacggat 660
   tacgagggat tagcatacct gggagagtat cccctcgcct gggctgcttg ctgtgcaaac 720
   gagagtgttt acaacttatt actcgactct ggcgctgatc ccgacgagca ggactctttt 780
   gggaacatga tactacatat ggtagtcgtt tgtgataaat tggacatgtt cgggtatgcc 840
   ctccgtcatc cgaagcttcc tgctcgcaat gggatcgtga atgccgccgg actgacccca 900
   ttgacgctcg cctgccagct gggtcgcgca gaggtctttc gggagatgtt ggaactgtct 960
   gcacgagaat tctggcgtta cagtaatatc acctgctcgg catatccgct gaatgcactc 1020
   gacacgctgt tacctgacgg cagaacaaac tggaactccg cgctatttat cattctgaac 1080
   ggtacgaagg aggagcactt ggacatgctg gacggtggta ttattcagcg attactcgag 1140
   gaaaaatgga agacctttgc gcggttgcag ttcttgaaac ggttgatcat cctggtgttc 1200
   catctgacat cactctcgct agccgtgtat atgagaccgt cgaacatcga tgccactctc 1260
   ctggaatggc cagaagaaat cacggaggtt ggacgcatga ttgcggagtg caccacggtg 1320
   ataggtgtgc tgagctacat tttggtgcaa ctaggcggcg aggtcgtcaa cattggtctt 1380
   ctatcgttca tgaaacaatt gagtcacgag cctgccaagc tcatattctt gatcagcaat 1440
   ctgctcatac tcgcgtgcat accatgtcgt cttgctggta ataggcatgc tgaagacgcg 1500
   atactcgtgg ttgctgttcc cggctcttgg ttcctcctca tgttctttgc tggcgctatc 1560
   cggttgaccg gtccattcgt taccatggtc tacagtatga taaccggtga catgttgacc 1620
   tttgggataa tctacatggt ggtgctgttc ggattttgcc agtcgttcta ctttttgtac 1680
   aaaggcttcc cgggagtgaa atcatccctc tacagttcct atcattcgac ctggatggca 1740
   ctgtttcaga taaccctcgg cgattataac tatacggacc ttggttacac gacctaccct 1800
   aatttatcga agatggtgtt cgccatattt atggtcctgg tgcccatact gcttttgaat 1860
   atgctgattg cgatgatggg caacacttac gcgcatgtga tcgagcagag cgagaaggaa 1920
   tgggtgaagc aatgggcgaa aatcgtggtt tctctggagc gtgcagtcgc gcaaaaagat 1980
   gcgcagaatt atttgcaaga atacagtatt aaactggggc ctggagatga cccgaacaac 2040
   cccgcctcgg aacagcgcgg agtcctggtt attaaaagta aatcgaaaac caaggcgaag 2100
   caacgcaaag gcgcggtggc taattggaag cgcgtaggca aagtgacgat aaacgaattg 2160
   aagaaacgtg gcataacagg agaggcgttg cgacgagtca tgtggggtcg agcatctttc 2220
   tccactcctc gagacacttt gagagctctc ggatctgtag gtattgtgag agctgtagga 2280
   actctcggac ctgtaggtac tgtgagagct gtaggagctc tcggacctgt aggcactgtg 2340
   agagattcga gcatggccga ggattttcag gatccgcttt tggagcttgt gattgcttcc 2400
   gaaaatacgg acgatcctga aacgctgttg aaaatagcgg agcgggccgc ggcgaatttc 2460
   gaagtggaga gcagttcgaa aataaatctg caaattttgg agcaattcac gatgactaag 2520
   ctacctatgg aggagaaagt tgccgctaca aagaagcagt actttgttga atcgagtgac 2580
   aacgattttg gtggcgacaa cctactcgga accgaggccc gtctacgtag aataagatca 2640
   gcgaacaaca gatttatcac gacacggaga cggtcgcggc acaccgacga cgacctgtca 2700
   tccacgtcgt ccacatccgc cgatagaaat cctcaatatc agccattgct taacggccct 2760
   gagaacttgg tgagccgtca aatcgaaagt cgggagagct ctatcgaagc aattaagccg 2820
   caagtaaagc agaacggatc atgcgattca gtgaagatca aggttcagaa gcgtcgaccg 2880
   aaaacggcga aaaatagggt atcaccgaag gaaatagagg aggctggtcc gaggagaaga 2940
   gaatcgatcg agcggaataa ggcggtttca ccggcgacgt cgcctactga tccgctcgaa 3000
   ccatggagca cacgcggcat taaggacatg aacacgatat tggcttggga ggagaatacg 3060
   ccggacagtc cttaa 3075
<210> 43
   <211> 2898
   <212> DNA
   <213> Dendroctonus ponderosae
<400> 43
   atgggtgggt gccaatgtaa gccatgctgc agacacaaga aatcgcacta cactggaggc      60
   tcaattttgg atcgagtaat tagccaaagc tccaatcaag accagtgttt gttataccga     120
   ttggccaact acaagaaggg aggggaatta attgatgcct acaatgcagg cgggcagatt     180
   gagtgcgaga agctgatccg cgaccaattc gcgcaactga tgtatcagga tggcaaaggg     240
   aagattatca accgatctga gtatctaaga tggaaattca gggatcatga gcatgtgatt     300
   cttccaatcg aagcgtcact aagtcgttac gacccattag gcaaatggac cgaccatgaa     360
   gcctgctggc aaatgcagta cagaggctct ctaggcgaat ccctgctcca tctgctcatt     420
   atttgcgaca gcaaaatcca caccagactg gcaagaacgt taattaaatg cttcccgaaa     480
   ctggcgcttg atgttgtcga gggagaagag tacttagggg ctagtgctct tcacctagct     540
   atagcctaca gcaacaacga actagtgcag gacctagttg aggctggagc taacgtgaac     600
   caacgagcta tcggtaagca aatcgctgat aaaaccggca aaattaacgc aaaaaaccgt     660
   ttttttctgc cgagagatca gcaaaaacag cgaccagcta agcatacaga ctacgaaggt     720
   ttggcttatt tgggcgagta tccactggct tgggcggcgt gttgtgccaa cgaaagcgtc     780
   tacaatctgc ttctggacag cggcgctcat ccagatttgc aggacaattt cggcaacatg     840
   atccttcata tggtggtcgt ttgcgataaa ttggatatgt ttggctacgc gcttcgacat     900
   cccaaactgc cggctagtaa tgggattatt aacaaagctg gattgactcc actcactttg     960
   gcctgcaaac taggtcgtgc ggaagtgttc agggagatgt tggaactgtc ctgcaaagag    1020
   ttttggagat acagcaacat tacatgttcg gcgtatactc tgaacgcttt ggacactttg    1080
   ctgcctgatg gtcgcaccaa ttggaactct gccctgttca ttatcctcaa tggcaccaag    1140
   gaggcgcatc tcgatatgtt ggatggaggc atcattcaaa gactgctgga ggaaaaatgg    1200
   aaagcctttg ctcggaacca gttcctcaaa cgcttaatta tcctgatagt tcatctagct    1260
   tttatgtcac tggctgtgta tatgaggcct gatgatccgg acgaaccgtt actggaatgg    1320
   tccgacgatc ctacagttat cattcggtat cgtcggcttt tccaagagaa tccagaggtt    1380
   ttactggggt tttgtagatt tactgctgaa attgggacgc tactaaatgt gctcagttac    1440
   gttgttgtac agcagggaga cgaaataagg aaccaaggat ttctctcttt tttgaaacaa    1500
   caaacaaatt ccccgccaaa gtgcattttc ttagtgtcca acttgatgat tctggcatgc    1560
   atcccttgta gactggccgg aaaccgaggg ttggaagaaa caattttgat ctttgctgtg    1620
   cccggatcct ggtttttgct catgtttttt gccggtgctg ttcgcttaac cggaccattc    1680
   gtcacaatga tctacagcat gatcaccggg gacatgttaa cttttggaat aatctacaca    1740
   attttccttt tcggtttttc ccagtcgctt ttcttcttgt acaagggaaa tcccagcgtt     1800
   aatggcacgt tgtatcacag ctacagctcc acttggatgg cactgtttca agtcacaatg 1860
   ggaaattacg attactctga actggcgcta acaacatatc caggtgtggc caagtccgta 1920
   ttcggtcttt tcatggtatt cgtcccaata ttgctcttca acatgttgat tgccatgatg 1980
   ggaaacactt acgctcatgt taccgaacag agtgaaaagg aatgggtaaa acaatgggcg 2040
   aaaatcgtaa tttccttgga aagggccatc ccacaaaagg aggctcataa ttacctacag 2100
   gaatatagca tatccttggg accagctgaa gatccagcaa ccgaacaacg aggagtgatg 2160
   gtgataaaat cgaaaaacaa aactagagcc aaacaacgaa agggagcagt cagcaattgg 2220
   aagagagtgg gaaaagttac aataaaagcg ctaaagaaaa aagggatgac tggggaagag 2280
   atgcgctgcc taatgtgggg gcgagagtca attaacacgc cggtaaaagt aaaagggcct 2340
   aaaagagacc ctttggcaaa cccccaaggc ccagacttgg ccaatggctt cggggatgct 2400
   ctaacttctg ctctggatgt aatggcgttt acccacgatt tggacattaa tgctcaaggc 2460
   ctaaatttag caaaacaaac tctaaatcct aagcctttta cggaccctct aagaactctg 2520
   gttttggagt ctctagtgga gaccaattca gccagattga gcgttttggc tgcacaagca 2580
   gccaatttgc ctgatactgc cgagatcgtc tcatgcccaa atacggccac cacttccgtg 2640
   aagaacctcg caggaatttt tgcagggact ggcaattttg tgaagaaaat tgaggagaaa 2700
   gtgaagaaga aaaaatatgc cgttttggaa tgcagcgata gcgagagctt cggaggcaag 2760
   tgccagggca tttcaacgca tttccagtca aagactttga aatattgcag aagtgctgct 2820
   ggggacaata tcacgggtga agagggccag actgatacat atgcgacatt ctgcaaacag 2880
   gaaaaggaat ttagataa 2898
<210> 44
   <211> 3444
   <212> DNA
   <213> Nasonia vitripennis
<220>
   <221> modified_base
   <222> (1051)..(1053)
   <223> a, c, t, g, unknown or other
<400> 44
   atgggtgggg catgctcctg tcagggtcga gacagccagg tcaatgctgg ctcgatcctc        60
   gatcgcgtga tcagccaggc gagcgacgag gaccaatgtc tgctctaccg gctggccaac       120
   tacaagaagg gcggcgagct gatcgaggcc tacaaccagg gcggtcaagc cgaggtcgag       180
   cgtctgatcc gcgagcagtt cggcatcctc atgtacgccg acggcaaggg tcagacgatc       240
   aaccgggcag agtacctccg ctggaagttc cgggacctgg accaggtcat cctgccgatc       300
   gaggcgtccc tctcgcgctt cgatcccttg gcccagtggt cggatcacga ggcctgctgg       360
   cagatgcagt accgcggctc gctgggagag acgctgctcc acgtgctcat tatctgcgac 420
   acccgcaccc acacccggat cgcgaggacg ctgctcaagt gcttccccag actggccata 480
   gacgtggtcg agggagagga gtacctcggc gcgagcgcgc tgcacctggc catagcctac 540
   gctaacaacg agctggtcca ggatctcgtc gaggccgggg ccatagtgtc gcagcgggcc 600
   ataggaagct tcttcctgcc gagggaccag cagaggcccc gaccggctaa gagcaccgac 660
   tacgagggac tggcctatct cggcgagtac ccgctcgcct gggcggcctg ctgcgccaac 720
   gagagcgtct acaacctgct cctcgactcg ggagcccacc ccgacgagca ggacaccttc 780
   ggcaacagca tcctccacat ggtcgtcgtc tgcgacaagc tggtcagatt cggctacgct 840
   ctgcgtcacc cgaagctacc agcgagcaac ggcatagcta acgcagccgg cctgaccccg 900
   ctgacactcg cctgccagct gggacgtgcc gaagtcttcc gcgagatgct cgagctctcc 960
   gcccgggagt tctggcgata cagtaatatc acttgctcgg cgtatccgct caacgcactc 1020
   gacacgctgc tgcccgacgg aaggacgagt nnnaactcgg ccctgttcat cattctcaac 1080
   ggtacgaagg aggagcacct ggacatgctg gacggcggca tcatacagcg gctgctggaa 1140
   gagaagtgga agaccttcgc tcggctgcag ttcttgaagc ggctcgtaat cctcgtcttt 1200
   cacctggtgt cgctgtcgct ggccgtttac tttcggcccg cggacactga cgccgagttg 1260
   gcccagtggc ccgaggagat caccgacgtc gtcagagtca tcgccgagtg cgtcaccgtc 1320
   ctcggcgtct tgggttacat tctactccag ctcggagggg agatcgtcaa catcggcttc 1380
   ttctcctttt tcaaacagct gagccacgaa cctgccaagt tcatatttct catcagcaac 1440
   ctcctgattc tggcctgcat cccgtgcaga ctgtccggcg accgacacac cgaggacgcc 1500
   attctcgtgg tcgccgttcc gggctcctgg tttctcctga tgtttttcgc cggggcagta 1560
   cgtctgaccg gacccttcgt cacgatggtc tatagcatga tactcggcga catgcggact 1620
   tttggcatca tctacatgat cgtcatctgc ggcttcaccc aggcgttcta cttcctctac 1680
   aagggctacc cgggagtgaa gacgaccctc ttccattcct acccctcgac gtggatggcg 1740
   ctatttcaga taactctcgg cgattacagc tactcggacc tcagcatgac gacctacccc 1800
   aatctcagca aagcggtctt caccatcttc atggtcctcg tgccgatcct cctgctcaat 1860
   atgctgatag ccatgatggg aaacacgtac gcgcacgtga tcgagcagag cgagaaagaa 1920
   tggatgaaac agtgggcgaa aatcgtggtt tcgctggaac gagctgtgtc gcaggacgac 1980
   gcgcacaatt acctccagga gtacagcata aaattgggcc caggagacga cccgaacgat 2040
   ccagcctcgg agcaacgggg cgtcatggtc ataaagagca aatcgaagac tcgggctcgt 2100
   cagcggaaag gcgccgttgc taattggaag agagtcggaa aggtaacgat cgccgagctg 2160
   aaaaagcgtg gcatgaccgg ggaggagctt cgtcgcatca tgtggggccg agcgtccttt 2220
   tccacgccgg tcaggtccag cccgatcctg atggagccgg tgtcatcagt gccaggcggt      2280
   ttcggcgacg ccctgacgtc agccctcgac gttatgactt tcgccgccga tgtgccggtg      2340
   cccattgagg gtagtatacc caagccagtc gccgtcgccc cgggccaaca gccgactaca      2400
   acacttgcac tacccaatgg agctgcgaaa cctcaacagc cgatgatgac aacgtcgacg      2460
   acaacgacga cgatcacgac gacagtgcag aagactctcg ttaccggcac tgctacaaca      2520
   ccggcggtgg gcgttacgaa gaccgtcgcg gttccagcgg cgaccaccgt cagcaccaca      2580
   acgactcgag catcagcatc agcagtgccc cccacagttc ggccgtcaga gctcggtcca      2640
   gcctcggccc cttgcagcgc cgacccactg ctggagttga tgctcgcgac ggaggatcca      2700
   ggctcgtcgg aggagactct gcagcgtctg gcgcactcgg cccggatcgt gggcgagagc      2760
   gccaccacca gcgccaagtc cgatatcgat tcgagcttcc tcgagaggct tgcgctcgga      2820
   ggtctcggac tcgccgcgat gcccgaacag cctgacaagc ccgtcgagcc caagaagaag      2880
   cagtatctcg tcgagtccag cgataacgat atctgtgcga acgacggtca gctgggaacg      2940
   gaggctcgac tgcggcgcat caggtcggcc agtagccggc tagccccgac gacgacgacg      3000
   tcgacgtcgc gacggaagtc cggccagcag aagcagcaac gcgaagccag ggacgacgag      3060
   agcacctcgt ccgccgcttc gctcgacaac atttccggct accagccgct cctgaacgag      3120
   ccggacacca ccgcggacca gcctgccacg ggaacagagg acaataagcc ggccaacggc      3180
   aggaacctac gcaagagacc caagaccacg agtaacgaag gcaaggtctt ctgtagtaga      3240
   aggacaattg ttgtacttaa taaaaataag aagaataaaa agctcgagac atctgaagcc      3300
   aacatgaacg ccaggaaaaa ggaaactctt cttgcactgc acgacatgac aagtttgatc      3360
   tcaggccgtg tactcaccct ggaacaggcc aatgaaaatg gctatgaaat gctggagcag      3420
   ttgaagcata agttggaccc ataa                                             3444
<210> 45
   <211> 3330
   <212> DNA
   <213> Camponotus floridanus
<400> 45
   atgggtggtg tttgctcgtg tcgcgttggt cgaggtaacc agcttaacgc aggctcaatt      60
   ctcgatcgcg tcatcagcca ggcaagcaac gaggatcagt gcttgttgta tcggctggcc     120
   aattacaaaa agggcggcga gctgatcgag gcgtacaatc aaggtggcca gccagaagtg     180
   gagaaattgg tgcgcgagca attcggcatt ctgatgtacg ccgatggtaa gggccaggtg     240
   atcaatcgcg ccgagtatct gcgttggaag tttcgtgacc tcgagcaggt cgtgctgccg     300
   atcgaggcat cgctgtcgcg cttcgatcca ctcgcccaat ggaacgatca cgaggcctgc     360
   tggcagatgc aatacagagg ctcgctgggc gaggctctgc ttcatgtcct catcatatgc     420
   gacacgcgga tacacaccag aatagcgcgt atattactca agtgcttccc gcgactcgct 480
   atcgacgtcg tcgagggcga agagtatctc ggagcgagcg cccttcacct ggcgatcgct 540
   tataacaaca acgaactcgt gcaggatctg gtcgatgcgg gcgcgattat ctcgcagcgg 600
   gccatcggta gcttttttct accgcgtgat cagcagggaa tgaatcccgt tagaaacacc 660
   gattacgaag gactcgcgta tttaggcgag tacccgctcg cctgggccgc ttgctgcgct 720
   aatgaaagcg tctacaatct gctgcttgat tccggtgcgg atcctgacga gcaggattct 780
   ttcggaaata tgattttgca tatggtcgta gttggcgata aattggatat gttcggctat 840
   gctctgcgac atccgaagct tcccgctcgc aacggaatcg cgaatgctgc gggtctgacc 900
   ccgttgacac tcgcctgcca acttgggcgt gccgaggtct tccgtgagat gctggaactg 960
   tccgcgcggg agttctggcg ttacagtaat atcacctgct cggcgtatcc cctcaatgcc 1020
   ctcgacacgc tactaccaga cggcagaaca aactggaact ctgcgctatt tatcattctg 1080
   aacggtacga aggaggagca cttggacatg ctggacggtg gtattattca gcgattactt 1140
   gaggaaaaat ggaagacctt cgcgcgattg cagttcctga agcgattgat tatcttggtg 1200
   tttcacctgg cctcgctgtc cttagcggta tacttcagac ccgcggatac ggatgccgtg 1260
   ctgctgcagt ggcccgagga gataacggac gtcatccgta ccgcggcgga gtgcatcacc 1320
   gtgctgggcg tgctcaatta catcctggta caactgggtg gcgagatgat caacgtcgga 1380
   ccgctgtcgt ttctgaagca actgagccac gagccagcca agttgatatt cgtgatcagc 1440
   aatctactca ttctcgcgtg cattccatgt cgtatcgccg gaaatcggca cgcggaggat 1500
   gcgatattgg tgttcgccgt cccgggctcc tggtttcttc tcatgttttt cgccggagca 1560
   attcgattga ccggtccatt cgtcacgatg gtatacagca tgataaccgg tgatatgctg 1620
   actttcggta taatctatac aatcatgctc ttcggatttt cgcagtcgtt ttattttctc 1680
   tacaagggat tcccaggcgt gaaatcatct ctttatcatt cttatgtttc gacctggatg 1740
   gcattgtttc agataaccct cggcgattat aactatgcgg aattgaataa cacgatctat 1800
   cccaacttga gcaaaaccgt cttcgcgatt tttatggtgc ttgtgcccat tctgctactt 1860
   aacatgttga ttgccatgat gggtaatacg tacgcacatg ttatcgagca aagcgagaaa 1920
   gaattcgtta agcagtgggc aaaaatagtg gtgtctctgg aacgtgccgt atcgcaaaaa 1980
   gacgctcata attatctgca agaatacagc atcaagctag gacccggcga cgatccaaac 2040
   aatcctgctt cggagcaaag aggcgtgctc gttattaaaa gtaaatcgaa aaccaaagcg 2100
   aagcagcgta agggcgccgt agctaattgg aagcgagtcg gaaaagttac gataaacgaa 2160
   ctgagaaaac gagggatgac tggggaggag cttcgacata taatgtgggg tcgtgagtcc 2220
   ttctctactc ctgtacgagt cagtcccaat gctggccaac cgcaagtgtc ggcagttact 2280
   gcaggttttg gtgatgcact aaccgcagct ttggacgtaa tggcctttgc acatgacttt 2340
   gacttatccg cggcgacaga aggcataccc accaatatcg atgcgaaaca aacaaaaacg 2400
   acacaatcga aagccattcc caacgatcag atgaaaacga cccagaataa tccagaaaca 2460
   gtgacgaatg tccaaccaca taaacagcct attggggagc caactaaatt gttaggtctt 2520
   acggatttaa tcgataattc cattggtccc gtgaaggatg tggaagcgat gaatttaaaa 2580
   aatacgaacc aaacaaaagc gctcgaagca tctatggagt tcgaagatcc atttttggaa 2640
   ctagccatcg cttctgagac tacgacaaat tacgaaatct tactacaaat agcgaagtct 2700
   gctctagctg ccagtgaagc ttcggtgaaa actgcaattg atccacaaat tctcgcacat 2760
   tttgctatga ttcctccgcc gatcactgag aaattcatcg ttaagaagca atactttatg 2820
   gaatctagtg ataacgatgt cggtggggat aatttattag gcactgaagc tcgtcttcga 2880
   aggataaaat ctgcgaataa caggttcata actacatcaa aacggccaca tcgtgatgat 2940
   gatggcgaca gttcttcgtc cactatatca attgaacgaa atcaacgata tcgaccatta 3000
   ttcaacgatc cggaagaaag atcgataaat catgtcgaaa ctgaaggtca aaaaactccc 3060
   atggaaacaa ttaaatcgga aatccagcag aatggtagca ttccactcaa gccacccgat 3120
   aaaattcaaa agcgtcgacc gaaaacagcc aagaacagag tatcacctaa ggaaatagag 3180
   gagccgagga gaagggaatc gattgagcag aataaggcgg cctcaccacc gacgtcgtcc 3240
   tcggatccgc tcgagccatg gagcacgcgt ggcattaccg acatgaacac aatattggct 3300
   tggagggata acgcaccgga tagtccttaa 3330
<210> 46
   <211> 2974
   <212> DNA
   <213> Danaus plexippus
<400> 46
   atgggtgtac cgttaagtaa gctgtgttca gcaacgagtg tcccagccgt tggctcggtt 60
   ctcgaccgtg tcatatccca gcccagcagc gaagatcaca ctgtcttata taagctcgct 120
   gattataaag aggtttacta ttagaaacct atactaaggg cggcgtggta gccgccgagc 180
   gcctcatcag agaggagttc tcggcatata tgtacgctgg tggacggggc cgggtcatca 240
   acagagctga atacctccga tggaagtttc gagaccagga agttgttctc ccaatagaag 300
   cgtctctctc acctcacgat cctctagcga aatgggaaga tcacacagct tgctggcaga 360
   tgagttaccg cggagcactg ggggagtcac ttcttcatgt tctcattata tgcgacatcc 420
   acacaagatt ggcgagaact ttggtcaaat gttttccgaa attgtccctg gatgtcgtcg 480
   aaggtgaaga atgtgctagt tcgttacatt tggccatcgc ctatagtaat aatgaattag 540
   tccaagactt ggtcgaagct ggcgcggatg ttagtcagaa agctgtagtt tctttctccc 600
   gagagaccaa caaaaaaatc caccagctcg tcagacaaat tacgaaggtt tggcgtattt 660
   aggggagtac ccactagcgt gggctgcttg ttgcgctaac gaggccgtat ataacctcct 720
   tctggactct ggagctgatc cagacgccca ggattcattt ggaaatatga tcctgcatat 780
   ggttgtagtt tgtgataagt tggatatgtt cggctatgcc cttcgtcatc caaaggtacc 840
   agccagtaac ggcagactaa ataaggctgg gttcacaccg ctcacccttg cctgccaatt 900
   ggggcgagcc tcggtgttca gggaaatgtt ggagctttca tccagggaat tttggagata 960
   ttccaacatc acctgttctg catatccatt gaatgctcta gacactttgc tacctgatgg 1020
   acgtacaaat tggaattccg cattattcat catacttaat ggcactaaac aagagcatct 1080
   taatatgttg gacgggggaa tcatacaaag gctcttagaa gagaaatgga aaacatttgc 1140
   cagaacaaaa tttttaaagc gtctattaat cctcatgcta catctgatct tgctatccat 1200
   atctgtgtat ctacgtcaca gcagtctgga agcggatgta gaccccgact ggggtttaga 1260
   ggtcaatgat gcaaggtccg ggataagatt ggcctgcgaa ctaggaacta tcataagcac 1320
   tctttgctat attattctgc agcaaggcga tgagatcaaa aaccaaggtg ttgttgcgta 1380
   cttcaagcaa ctaatccatg aaccagccaa atttatattc ctagcttcaa acatattgtt 1440
   actggcgtgt attccagcaa gaataagcca aagaactact ttggaagaag caatactaat 1500
   attcgtacta cccggttctt ggttccttat gatgtttttt gcaggagccg tgaagttgac 1560
   tggcccgttt gttacaatga tatacagcat gatcactggg gacatgttca cattcggcat 1620
   tatctactgc atagtattgt tcggattctc gcaatctttt tacttcctgt ataaagggtt 1680
   tccgaacgta cattcaactc tgtactccag ctatcccagc acctggatgg ctctttttca 1740
   aatcacgtta ggcgactaca gttacacaga tctcggtctg acgatgtatc ctaatttgtc 1800
   gaagacagtg ttcactgttt tcatggtgtt cgtccccatt ctgctactca acatgctgat 1860
   agccatgatg ggtaacacat acgcacacgt tatagagcaa tcagagaagg aatgggtaaa 1920
   acagtgggcg aagattgttg tgtctctgga gcgttcagta tcccaggaag atgctcatcg 1980
   ttaccttcag gagtattcaa taggtctggg accctcagac gaccctcgtt atgagcaacg 2040
   aggtgttatg gtaattaaga gcaaggccaa aactcgcgcc aagcaaagaa agggcgcgct 2100
   gtctaattgg aagcgtgtgg gtaaagttac tatagcagag ctgcggcgcc gcggcattag 2160
   tggagaagaa ttgagaagac tcatgtgggg gagagtctca atatctactc caaccaaagc 2220
   acccttaaaa tgtgtgcctc ccccggagtt agtgacttca gaaattccag gaagtggagt 2280
   tggaccagcg ttgtcatcag ctcttaacgt gatggcgtac acacaagatc tagacctcac 2340
   caacaccgga tcagaactgc ataaacagat aacgcccgat ttgctcgtca atggcaagac 2400
   gcctgtgaca gcacagaata cagcaaccac tcccaaagta ccacttgtaa atctaccaaa 2460
   taaagcggga ttggacgttc aaagtacggg aagtgatcag agcgtgccaa ctgcattaac 2520
   taagaatatg gacgttttgg gcataaatat gtcaacacag gatttgctaa agaatcaaac 2580
   tataaacaat cccgctacag ccaacgaaat agtgttcaaa gattatctca gggatatcat 2640
   aaaggcggag caacttggtc tagataatat tgacataaag gccctcgcag agaaagcggc 2700
   caacctcacc gacgtaccag agatagacat taacatatct acggctgcaa ggtctgcccg 2760
   tcgtgtggta gccggcgctg tttctggtct gttcggtgtg acagcagaga ctccgcggga 2820
   cgcaggctgg cgcagagaga gacacgaaca tacagacagt gaccctgtcc cagagtgcgt 2880
   catactgggt cgtgcggcgc gcgctcgccg tgcacgatct gcttcccggc gcgcgccccc 2940
   tcccccacct cacctctacg ttcctgcgag gtaa 2974
<210> 47
   <211> 3540
   <212> DNA
   <213> Bombyx mori
<400> 47
   atgggtaatg ccattggaaa atttctaact gcgggcaacg ttcaaggtgc tggctcggtt      60
   ctcgatagag tcatatccca gccgagcagc gaagaccaca ccgtacttta caaattagct     120
   gattataaga aaggcggtct acttttagag acctacgcga aaggtggcat gacagccgct     180
   gaaaagttaa tgagagacga attcgctgca tacatgtacg gcggagggag gggccgggtc     240
   atcaatcgtg ctgaatactt gagatggaag ttcagagacc aagaacaggt tgtacttcca     300
   attgaagcat cgctctcacc ttatgacccg ctcgccaaat gggaagatca caccgcctgt     360
   tggcagatgt gttatagagg agctctcggc gaatcattgc ttcatgtact cattatttgt     420
   gatactaaga ttcatacaag attagctcga actctagtaa aatgttttcc gaagctgtca     480
   ctagatgtcg ttgaaggtga agaatattta ggggcaagct ctttacatct ggcaatagcg     540
   tacagcaata acgaactagt acaagatcta gttgaggccg gagctgatgt caaccagaga     600
   gccattggta gctttttcct accccgcgac caacaaagag ttccgccagc gcgacaaaca     660
   aattacgaag gtctggcata cttgggcgaa taccccctag cctggacggc ttgttgcgcg     720
   aacgaagcag tctacaacct cttactagat tccggggcag atcccgatgc tcaggattcg     780
   ttcgggaaca tgatactaca catggtggtc gtttgtgata aactggacat gtttggatat     840
   gccctgcgtc accctaaagt tccggccagt aacggtcgaa tgaataaagc aggtttcacc     900
   ccactaacct tggcttgtca gttgggtcgg gcctctgtct tcagagagat gctggagcta     960
   tcagccagag agttctggcg ctactccaac attacgtgct ccgcttaccc tttgaacgct    1020
   ttagatacct tgctgcctga tgggcgaaca aattggaatt cggctttatt cattatactg    1080
   aacggaacaa agcaagaaca cttgaacatg ttagacggcg gtatcataca aaggttgctt    1140
   gaagaaaaat ggaagacttt cgccaggacg aaattcctca aacgtctcct catactgatg    1200
   ctgcatcttc tcctgctctc cgtttctgtg tacctacgtc acagcagcgc agaagctgat 1260
   gcccacccca actggggctt ggagatcaac gacgctagaa gcggactgag acttgccagt 1320
   gaacttggga ctatattgag cacattgtgt tatattatac tgcaacaagg cgacgagcta 1380
   aagaatcaag gattggtcgc ttattttaag cagttgattc acgagccggc caaatttata 1440
   tttttggcgt caaacatcct tgtgttagcg tgcataccgg ccagactcct taaggagacc 1500
   aacgtggagg aagccatact gcttttcctg ctgccgggat cgtggttttt gttgatgttt 1560
   tttgctggcg cggtgaaatt gaccggtcca ttcgtgacaa tgatctacag tatgataaca 1620
   ggggacatgt tcacgttcgg tatcatctac tgtattgtcc tcttcggctt ctcgcagtca 1680
   ttctacttct tgtatagagg ttttccaaac gtacagtcga ctttgtactc gagctacccc 1740
   agcacgtgga tggcactatt ccaaatcacc ttaggagatt acagctactc ggatctcagt 1800
   cagacaacat accctaatct ctcgaagact gtgttcacgg tgttcatgat attcgtgcca 1860
   attctactat tgaacatgtt gatcgccatg atggggaaca cgtacgccca cgtcattgag 1920
   caatccgaga aggaatgggt caaacagtgg gcgaaaattg tggtatcact agagcgttca 1980
   gtggcccagg atgacgcaca taagtacttg caggagtatt ctataggact tgggccttcc 2040
   gacgatccga gatacgagca acgagcggtc atggtcatca agagcaaagc taaaactaga 2100
   gcgaaacaga agaaagacgc tttaactaat tggaagcacg tcggtaaagt gacaatagca 2160
   gagttgagaa gacgcggtat cagcggtgaa gagttacgta gattgatgtg ggccgtattt 2220
   tcaatttcga caccaacaaa agctcccttg ccgcgaagag tcccagcccc gcctccagac 2280
   tgcgtggttt cgtcagacgt gggtttagct agtgacgtag ggaacggtgt agcaccagct 2340
   ctctcgtcag ctctcaacgt catggccttc actcacgaat tagacattgg cactactggt 2400
   tctgatcaaa aacaaactac accggacttg ttggtcaacg gcaaaacttc caatgcccca 2460
   atcttaacta ccggtaccca aatgccaaaa gtttcaagca aaactctcgg tacaccagtg 2520
   gcacgtattg aagcatcttc acttaagacg cctttggatg ttaaattaaa cccaacaaca 2580
   ttgacacagt ccacggttca gggtaatata ggtaatgtca atattcctat aagtggagtg 2640
   aaaataacaa caaatgttgg acaaaactcc gcggtagtac ctgagattca acagtcgaat 2700
   gaaaatcaga aacctgagca agtacaccaa gattatctgc gtgaactcat agttcttgct 2760
   gagaaacctg caaccactaa tcttgaactc aaacaacttg ctgagaaagc ggctgatctg 2820
   agagatgtac cggaaattga tattaacatc aatatggctg caaaatctgc gcgtaaaatg 2880
   gtagcgggtg ctgtgtcggg tttattcggt gtcgccgctg acactcctgc ccccgatgct 2940
   ggctggaggc gggatcgtca tgacaacagc gacagtgatc cgatatcgga gtcagtgctg 3000
   ttaagtcgag catcaagagc aagacgcgcg aggtcagcat cgcgacgagc cgcaccaccg 3060
   cccccacatc tatatgtacc ggctaggtct atgtacttag tggcatctga gagcagtgct 3120
   atagaaagtg atgcaccttg ggatgatcaa cccagttcag agaacaattc agctgtaggt      3180
   ggtcgtagag aagagatcgc ccacattaaa gctgcaagac ccttgtgtat tcaacatgct      3240
   gcgtcaggga gaacggctca agtagaacac tcattatttg ttgtaggtcc tggaaccgga      3300
   gccgaagttg aaagtactcc tactgtacaa aaatccgaaa aaaaggctag acctaaaaca      3360
   acgcgatcta gacgaaacag aatatctcca acaccggtac aagaagcggg cagccctttg      3420
   gagccctggg ccacagcaca actatcgaag ctatcacgtc tcatacgcta ttccagctca      3480
   tcagcagctt ctatcacctc gcagacgtcc acggaacagg agcaatcgcc aagatactga      3540
<210> 48
   <211> 2982
   <212> DNA
   <213> Acyrthosiphon pisum
<400> 48
   atgggaaatt cttgtgcgag ttgcacagat ttctcattct cgaaaacagc ttcaggatct      60
   gttctggata gagtgatttc tcaagcgtca aatgaagacg attgtttgtt gtataagtta     120
   gcggattata aaaaaggagg agaattgatc aatgtgtata ataatggtgg tcaggacgaa     180
   gcggaacgat ttattgttga aaaattaccc ggccttatgt ataacaacgg taaaggtcaa     240
   gtgataaatc gaaatgatta tttgagatgg aaatttcaaa atcgtaaaca cgtggctgtt     300
   aatgtcgagg aaaggccggg tcctcacgat ccgttaacaa gatgggtgga ccatgtggcg     360
   tgctggcaaa tgcagtatag aggatcatta ggcgaaagtt tgttgcacgt attaatcata     420
   tgcgataccg tgatacacac tcggctgtcc cgactacttc tcaaacacta cccgatgtta     480
   agtcaagatg ttgtggaagg cgaagaatat ctcggcgcta gtgcattaca tttgtctatt     540
   gcttataaca acaacgattt aatccaagac ctggttgatg ctggtgctaa tatttgtcaa     600
   agagccatag ggagcttttt cttaccacgc gatcaacaga ataaagaaat caataacaaa     660
   cacacggatt acgagggtct agcgtatctc ggggagctgc cattagcgtg ggctgcttgt     720
   tgcgggaacc aaacggtata caacctgttg attgatgctg gagcaaaccc tgacgcccag     780
   gactcatttg gaaatatgat tctccatatg gtggtcgttt gtgataaact gagtatgttt     840
   ggttatgctt tgaaacaccc taaagtgaaa gcaagtaacg gtatcatgaa cgtggcagga     900
   ctaacacctt taacactatc gtgcaaactt gcaagaacta gtgttttccg tgaaatgttg     960
   gaactaagtg cacgggaatt ttggagatat agtaatatta cgtgttcggc atacccttta    1020
   agcgcgttag acacgttact tccagacgga agaacaaatt ggaattcagt attgtttata    1080
   attttggacg gtaccaaaga agaacatttg gacatgctcg atggaggcat tattcaaaaa    1140
   ttattagaag aaaagtggaa aacttttgct cggaaacaat ttatgaaacg tttagttata    1200
   ttatccatcc atctaatcat gttgagtatt tctatatacc taagacccgt ggaccaagac    1260
   aaaccactgt taggagaagc agaagactgg caagacgtgg caaggtattg ttttgaaggc 1320
   ggaacagtag tcggagtgct gtcatattta attgttcagc aaggtggaga aatactcaat 1380
   caaggtctgg taggattttt aaaacaaacg ttcaaagaac cggccaaact gatatttctg 1440
   atttcaaatt tattgatatt ggcttgtata ccacccagga tgatgggcga caaacagacg 1500
   gaggaagcca tattagtatt tgcagttccc ggatcatggt ttttgttaat gttttttgct 1560
   ggcgctatac gcctgactgg gcctttcgta actatggtgt acttgatgat cacaggagac 1620
   atgttgacct ttttcgtcat ctactcagta attttgtctg gttttacgca atcgtttttt 1680
   ttcctctaca aaggatcgcc agacgtcagt acgtcgttat ataaatctta cccttctacg 1740
   tggatggcct tatttcaaat tactatgggc gactacaatt acgcggattt gagttacacc 1800
   gtatatccag cgctcagcaa aactgtgttt acggttttca tggtgttggt gccaatttta 1860
   ttacttaaca tgttaatagc tatgatgggc aacacttatg cgcacgtcat cgaacagagc 1920
   gaaaaggaat ggatgaaaca gtgggccaag attgtagtta cccttgaaag agctgtacca 1980
   caaaaagcag ccaggaatta cctcgaagaa tatagtattc agttggctcc cggcgacgat 2040
   gtaaatccag aacaaagagg attcatggtt atcaaatgta aaagccaaac tagagcgaaa 2100
   cagcgaaaag gcgccgtcat aaattggaag aaaagtggaa agttggtcat tcaagagctt 2160
   cgtagattag aaggtactgg ccagaatcta agagatatga tttggaaacg atcgtctcta 2220
   tcagcgtctt ctccggttgc taaaagtgta tcaataacaa aaaataaaag caagtcctat 2280
   tctgaagcaa aagatgacca aattcggctt tctggtgcac taggagcagc actagacgcg 2340
   attgcagtag ctcatgactt agacatgact ctgacaaaag atcaatcgat tgatgaatta 2400
   cacgaccctt taagacaatt agtaataatg tcagaaagtg ataaaaatat tgataaaagt 2460
   caagtcgaaa tagtggcgaa tgcagcagca aaaattgcaa atcaagattc tataaaaccg 2520
   atatcgatta atttagagca acaaaatgac gtaaaaaatg aaaaaaccaa aattccaact 2580
   tccagtaaca caataacaaa tagaaaactc ttaaacaagc aatctaaaaa tttaagcatg 2640
   tatggttttg agagtcagga catgtcagta gataaataca gcaaagcaat tgcaatcaca 2700
   agaagtatat caactcaaac tggaggtatt ggtatcatcg aaaaaaacga acgacagata 2760
   attcgaccga aaactgcaaa aatcaatcgc gtgacgccca cgcagaattt cgcagtgaaa 2820
   agaggccaat cggcacagcc ggacaaacgg gaagaccaca gggataacaa ggagcaacag 2880
   tgttattgca tcagtggcaa caacatgttg cggccgtgga gcacgcaaga cctggcgccg 2940
   ttgaacacga tcatggcgtg gggtcccaac gacgatttct ga 2982
<210> 49
   <211> 825
   <212> DNA
   <213> Solenopsis invicta
<400> 49
   caatttatcg ccaactacga ccatgtgtaa aatcatattt ccgaaagaat cctgctcgtc      60
   aggatccgcg ccggaatcaa gcagcagatt gtagacgctc tcgttagcgc agcaggctgc     120
   ccaggcgagc gggtactctc ccagataggc gagaccctcg taatcggtat ttctggcggg     180
   attcatcctt tgctgatcgc gcggtaggaa gaagctaccg atggcgcgct gcgagataat     240
   cgcgcccgcc tcaaccagat cctgcacgag ctcgttgttg ttgtaggcga tcgccaggtg     300
   aagggcgctc gcaccgagat actcctcgcc ctcgacgacg tcgatagaca gccgcggaaa     360
   gcacttgagc agtatgcgcg ctatcctcgt gtgcatccgc gtgtcgcata tgatcagtac     420
   gtgcaacaag gtctcgccca gcgagcccct gtactgcatc tgccagcagg cctcgtggtc     480
   attccactgc gccagaggat cgaagcgcgt cagcgaggcc tcgatcggca gcacgacttg     540
   ctccaggtcg cggaacttcc aacgtaaata ctcggcgcga ttgattgtct ggcccttacc     600
   gtccgcatac atcaggatac cgaattgctc gcgaattagc ttctccacct ccggttgacc     660
   gccctgattg tacgcctcga tcagctcgcc gcccttcttg tagttggcca gccgatacaa     720
   taaacattga tcctcgttgc tcgcctggct gatcacgcgg tcgaggattg aaccgggatt     780
   gacctggctg ccgcgaccaa agcggcacga gcacgcacca cccat                    825
<210> 50
   <211> 426
   <212> DNA
   <213> Schistocerca americana
<400> 50
   caccacaatt ttggcccayt gtttcatcca ctccttctcg ctctgctcga tracgtgcgc      60
   gtacgtgttt cccatcatgg cgatcagcat rttgagcagc aggatgggca ccagcaccat     120
   gaaaatggcg aacactgtct tggccagcgt tggatatgtc gtgtggctca gttctggrta     180
   gttatagtcg cccatggtga tctggaagag cgccatccag gtggtggggt aggagtggta     240
   gagcgacgtc ttcacgccgg ggaagccctt gtacagaaag tagaaggact gagagaagcc     300
   gaacagcaca accgtgtaga tgatgccgaa cgtcaacatg tcaccggtga tcatcgagta     360
   caccatcgtc acgaacggac cagtcagccg grtggccccc gcgaaaaaca tcagcaggaa     420
   ccagga                                                                426
<210> 51
   <211> 2670
   <212> DNA
   <213> Myzus persicae
<220>
   <221> modified_base
   <222> (865)..(886)
   <223> a, c, t, g, unknown or other
<220>
   <221> modified_base
   <222> (1112)..(1306)
   <223> a, c, t, g, unknown or other
<220>
   <221> modified_base
   <222> (2234)..(2277)
   <223> a, c, t, g, unknown or other
<400> 51
   gaaaggccgg gccctcacga tccgttgaca agatgggtgg accacgtggc gtgctggcaa 60
   atgcaataca gaggatcatt aggcgaaagt ctgttgcacg tattaatcat atgcgatact 120
   gtgatacaca ctcggctctc ccgactactt ctcaaacact accctatgtt aagtcaagat 180
   gttgtggaag gtgaagaata tctcggcgcc agtgcattac atttgtctat tgcttacaac 240
   aacaacgatt taatccaaga cttggttgat gctggagcaa atatttgtca aagagccata 300
   gggagctttt tcttaccacg cgatcaacag aataaagaaa tcaataacaa acacacggat 360
   tacgagggtc tagcgtatct cggagagcta ccgttagcgt gggctgcttg ttgcggaaac 420
   caaacggtat ataacctgtt gattgatgct ggagcaaacc ctgacgccca ggactcgttt 480
   ggaaatatga ttctccatat ggtggtcgtt tgtgacaaac tgagtatgtt tggttatgct 540
   ttgaagcacc ctaaagtgcc ggcaagtaat ggtataatga acgtggcagg actaacacct 600
   ttaactctat cgtgtaaact tgcaagaact agtgttttcc gtgaaatgtt ggaactaagt 660
   gcaagggaat tttggagata tagtaatatt acatgttcgg cgtatccttt aagtgcttta 720
   gacacattac ttccagacgg aagaacaaat tggaattcag tattgtttat aattttggat 780
   ggtacaaaag aagaacattt agacatgctc gatggaggca taattcaaaa attattagaa 840
   gaaaagtgga aaacttttgc tcggnnnnnn nnnnnnnnnn nnnnnnttat attatccatc 900
   catttaatca tgttgagtat ttctatatac ctaagacccg tggaccagga caaaccattg 960
   ttaggagaag cagaagactg gcaagacgtg gcaaggtatt gttttgaagg cggaacagta 1020
   gtcggagtgc tgtcatattt aatcgttcag caaggtggag aaatactcaa tcaaggtctg 1080
   gtaggatttt taaaacaaac gttcaaagaa cnnnnnnnnn nnnnnnnnnn nnnnnnnnnn 1140
   nnnnnnnnnn nnnnnnnnnn nnnnnnnnnn nnnnnnnnnn nnnnnnnnnn nnnnnnnnnn 1200
   nnnnnnnnnn nnnnnnnnnn nnnnnnnnnn nnnnnnnnnn nnnnnnnnnn nnnnnnnnnn 1260
   nnnnnnnnnn nnnnnnnnnn nnnnnnnnnn nnnnnnnnnn nnnnnngaga catgttaacc 1320
   tttttcgtca tctactcagt aattttgtct ggttttacgc aatcgttttt ttttctctac 1380
   aaaggatcgc cagacgtcag tacgtcgtta tataaatctt acccttctac atggatggcc 1440
   ttatttcaaa ttactatggg tgactacaat tacgcggatt tgagttacac cgtatacccc 1500
   gctctcagca aaactgtgtt tacggttttc atggtgttgg tgcccatttt attacttaac 1560
   atgttaatag ctatgatggg aaacacttat gcgcacgtca tcgaacagag cgaaaaagaa 1620
   tggatgaaac agtgggctaa gattgtagtt actctcgaga gagctgtacc acaaaaagca 1680
   gccaggaatt acctcgaaga atatagcatt cagctggcac ccggtgacga cgtaaatcca 1740
   gaacaaagag gattcatggt aattaaatgt aaaagtcaaa cacgagcgaa acagcgaaaa 1800
   ggcgcagtca taaattggaa gaaaagtgga aagttggtca ttcaagagct tcgtagatta 1860
   gaaggcactg gccaaaattt aagagatatg atttggaaac gatcgtctct atcagcgtct 1920
   tctccggtcg ctaaaagtgt atcaataaca aaaaataaaa gcaagtctta ttctgaagca 1980
   aaagatgacc aaattcggct ctctggtgca ctaggagcag cactagacgc gattgctgta 2040
   gctcatgatt tagacatgac cctgacaaaa gatcaatcga ttgatgaatt acatgatcct 2100
   ttaagacagt tagtaataat gtcggaaagt gataaaaata ttgataaaag ccaagtcgaa 2160
   atagtggcga atgcagcagc aaaaattgca aaccaagatt ctataaaacc gatatcggtt 2220
   aatttagagc aacnnnnnnn nnnnnnnnnn nnnnnnnnnn nnnnnnnnnn nnnnnnnaca 2280
   attacaaata gaaaactgtt aaacaagcaa tctaaaaatt taagcatgta tggttttgag 2340
   agtcaggaca tttcagtaga taaatacagc aaagaaattg taatcacaag aagtatttca 2400
   actcaaactg gaggtattgg tatcatcgaa aaaaacgaac gaccgataat aagaccaaaa 2460
   actgcaaaaa tcaatcgcgt gacgcccaca cagaatttcg cggtgaaaag aggccaatca 2520
   gcacagccgg acaaacggga agaccacagg gataacaagg agcaacagtg ttattgcatc 2580
   agtggcaaca acatgttgag gccgtggagc actcaagacc tggcaccgtt aaacacgatc 2640
   atggcttggg gtcccaacga cgatttctga 2670
<210> 52
   <211> 2904
   <212> DNA
   <213> Bemisia tabaci
<400> 52
   atgtacaatg atggcaaagg acagatcatc aaaagagcag agtatttacg ttggaagttc 60
   agagatcagc aacaggtaac tctacctatt gaagcatctc tgagtcctca tgatcccctc 120
   gccaaatggg aggaccatga agcttgctgg caaatgcagt acagaggctc cttgggtgaa 180
   accttattac acgtcttgat aatctgtgac acaaaaacca acactcgcct ggcccgcaca 240
   ttgcttaagt gcttccctcg attggctcaa gatattgtag aaggagaaga gtatcttggt 300
   gccagtgctc ttcaccttgc tattgcatac aataataacg aattagtcca ggatttggta 360
   gaagcaggag caaatgtcaa tcaaagagca ataggtagct ttttcctgcc caaagatcag 420
   caaagaccga gacctgctaa acacacagat tacgaaggtt tggcttacct cggagaattt 480
   cctctttcgt gggcagcctg ttgcgccaat gaatcagttt acaatctact tctagattca 540
   ggagcccatc cagacatgca ggacactttt gggaatatga ttctgcacat ggttgtcgtt 600
   tgtgacaagt tggacatgtt tggttatgct ttgagacatc caaaactgcc agccagtaat 660
   ggtattacca acacagctgg cctcacgcct ctcacattag cctgcaagtt gggccgtgca 720
   gaagtttttc gtgaaatgtt agaactgagc gcaagggaat tttggaggta cagcaacata 780
   acatgctcag cgtacccttt aaatgctctg gataccctgt tacctgacgg acgaactaac 840
   tggaattctg ctctgttcat cattcttaat ggtaccaaag aagaacatct agacatgtta 900
   gatggtggaa tcattcaacg attactggag gagaagtgga aaacgtttgc aagacgccag 960
   tttttaaagc ggctggcaat tttgtttctc caccttctca tgctaagtat ggccgtatac 1020
   ctgagaccag tggacaggga gcagcctctt ataggaggag atgactggtc ggatattgcg 1080
   cgatacggtt ttgaagttgg cactatcatg ggcgttttat cgtatctagt tgtacaacaa 1140
   ggtggggaga ttaagaatca aggtcttgga agtttcttga agcaattgat ggcagaacca 1200
   gccaaagcaa tattccttgt ttcaaatctt ctaattctgg cttgcatccc ttttcggatg 1260
   gcaggggact cgcaaacaga agaggctatt ttaattttag ccgttcctgg gtcatggttt 1320
   ctactaatgt tttttgctgg ggctgtgcgg ctgactggtc cctttgttac aatgatctac 1380
   agtatgatta ctggtgatat gtttacgttc ggaattatct actcaattct tttgtttggg 1440
   ttctctcagt cattctattt cttgtacaaa ggctcgccaa atgtgaagca aactttattt 1500
   cactcttatc caacaacttg gatggctctc ttccaaatta ctttgggtga ctataattat 1560
   ccagagttgg ctttcaccgt gtatccaact ctgagcaaga tagttttcac tagtttcatg 1620
   gttctggtgc cgatcttgct gctgaacatg ttgatcgcta tgatgggtaa cacctacgct 1680
   catgtcatag aacgcagtga aaaggaatgg atgaagcaat gggcgaaaat tgttgttgct 1740
   ctggagcgag ctgttcccgc agcagatgcg cataactatt tgcaagagta cagcataaaa 1800
   ctagggcctg gcgatgatcc atcaacggaa caaagaggtg tcatggtaat caaaagtaag 1860
   agcaaaacac gtgccaagca aagaaaagga gctgtatcca actggaagcg agttggtaaa 1920
   gtcaccatca atgaattacg gaagcgagga atgacaggtg aacagctgaa tcgaatcatg 1980
   tggggacgag cttccatatc gacaccagtt cgacagcctg ccatggctct attacttgaa 2040
   gatagtgaag aagaaaatgc tgcaaacaat actggtccat ttggtggagc gctgacagct 2100
   gccttggacg taatggcctt cacccacgac atagacttaa atattggtca aacgccggtc 2160
   acagtttctg gccttggtgg tgtttccgtt cgcagtggag ctgaaggtct tcctccagaa 2220
   caaccattcc atgacccatt ccggcagctg gtgattgaag cagaaaatcc tcatattgat 2280
   gaatccgaat tagcagctct tgcaaatgct gctgcaatgt ccgttgttgc gcctgatgat 2340
   attccgactc ctgtggttac acctgagcct gtatctgcat tacctcgatt gccctctctt 2400
   catctagaac caagtgacag tgatggactt ggagatggac ctcttcttgg aagaggtagt 2460
   cgtgtgcgca gagttcgttc tgcacatctg aagcagcaaa aagcatcttc tacgaatggg 2520
   aatggctatt cagacagaca aagacttttt tcatctaaga atgaagactc aagttcaaat 2580
   acttcattag atttcgacct gacaaatcct ccttctcaat tgaggctcac tccacctcct 2640
   agaccggtaa cagctgaaat tgtggctgta aatccgaaga aatacatagg tggaaacagt 2700
   caagagaggt taaggccaaa aacagctaaa gtcaacaggg ttgcgccaac ccccgatgtt 2760
   cctgtgcgtc gagggatttc tgctaatggt atcccagaat cactgccgtc tccacctgat 2820
   ccactagagc catggagcac tcgtaatatc gtcaatatga atgcaattct tgcatggcag 2880
   caaagtgatc aggatagttt gtaa 2904
<210> 53
   <211> 2955
   <212> DNA
   <213> Euschistus heros
<400> 53
   atgggcatca tctggggcag tggtgcttct tcagtgaatg ctgggacagt acttgaccga      60
   gtgatctccc aagcatcaaa taaggatgaa tgccttcttt acaaattggc caactacaag     120
   aagggaggag agctgataga tgcctataat gcaggaggcc aaagtgaggt tgagaaactg     180
   ataagggaac agttcggcca attgatgtat aatgaaggaa agggtgctct catcaacaga     240
   gctgaatatc tgcgatggaa atttagggac atgcaacagg tacagatccc tatagaggca     300
   tctttatcca gccaagatcc attgtcaaaa tgggaggatc accaagcttg ctggcagatg     360
   cagtatcgag gttcactggg agaaacattg ctgcatgtac ttataatttg tgatacaaag     420
   atacatacga gactagcaag gacactatta aagtgtttcc ctaatcttgc catagacgtt     480
   gtggaaggag aagaataytt aggtgctagt gccctccatt tggctattgc atatttcaac     540
   aatgaattag ttcaagatct tgttgaagct ggagcgaatg tagaacaaag ggcaataggc     600
   agtttcttcc ttccaaggga ccagcaaggg cagagaccat caaagcacac agattatgag     660
   ggcttagcct acctcgggga gtayccattg gcctgggcag cctgctgtgc taatgagagt     720
   atatacaatt tacttctgga caatggagct aatcctgatc acagggacac attcggaaac     780
   atgattcttc atatggttgt ggtctgtgac aaacttgata tgtttggata tgctctcagg     840
   catcccaaga tgcctgcttc aaatgggata gctaatgtcg caggcttaac acctcttact     900
   cttgcctgca aactagggcg tgcgaaggtc tttcgagaaa tgttggaatt gagtgccaga     960
   gaattctgga gatacagcaa tattacatgc tctgcttatc cattgaatgc gcttgataca    1020
   ctgctccctg atggcagaac aaactggaat tcagctctct tcataattct aaatggaaca    1080
   aaagaagagc acttagatat gttagacgga ggtataattc aaaggttgct agaagaaaaa 1140
   tggaaarcat ttgcaaggag gcagttttta aaacggctag taatcctaat gcttcattta 1200
   ctctgcctga gcggagcagt ttatcttcgc ccaacagaca gaactaaacc actcttagga 1260
   ggagatgatt ggaaatcagt agcgaggcaa ggttttgaag tagcaactgt tttaggtgtt 1320
   ctttcgtatg ttatagtaca gcaaggagga gaaataagga accagggatt tatatctttc 1380
   atcaaacaac tggacycagc taaagccatc ttccttgtat caaatatctt gatattaata 1440
   tgtatcccat ttcgattgat ggatgacaaa agaactgagg aggcaatatt agtatttgca 1500
   gtgcctggat catggttctt actcatgttt tttgcaggtg cagtgagatt aacgggacca 1560
   tttgtaacaa tggtatatag tatgatagtt ggagacatgt ttacttttgg tataatttat 1620
   tctattgtgc tctttggatt ttcccaatca ttctattttc tgtacaaagg atttccagga 1680
   gttaaaaata ctctttacag ctcataccac agcacatgga tggctttatt tcagatcact 1740
   ctcggtgatt ataattatgc tgaattgtcg catacttcat acccgacatt aagtaagaca 1800
   gtgtttgcta tcttcatgat tctagtccct atacttctgc ttaacatgtt aatagctatg 1860
   atgggaaata cttatgccca tgtgattgaa caaagcgaga aagaatggat gaaacagtgg 1920
   gccaaaattg tggtttcact ggaaaragcg gtaaaccagg aagattgcaa acaatattta 1980
   caagaataca gcataaagct aggacctgga gatgacccat cgacagaaca gcgtggagta 2040
   atggttataa aaagtaaaag taaaaccaga gctaaacaaa ggaaaggagc tgtagctaat 2100
   tggaagagag tgggcaaggt aacaatcaat gagcttagaa aacgagggat gaacggagaa 2160
   caattaagaa gaataatgtg ggatagagct tcaatatcaa caccagttaa agtacctcaa 2220
   aaccctgtag tagatgtatt aatggaaaac acggcagaac aacaagccca acaaactcca 2280
   ggaagttttg gaggagcatt aactgcagca cttgatgtaa tggctttcac acatgattta 2340
   gatttatcag catctggaat cacttcttcc ggcttaaatg caaaagaact tattgttagc 2400
   gatcccttcc gtgatctggt gttgagctcc gaaatagatg tgggagagga agaattggca 2460
   acacttgcgg aagctgctgt tctatcggtg atgtcaggcc aatctgatga atcagaaaaa 2520
   ctaaaaatag ctaaatttaa taagcaaatc agttttgaac agaatgcagt tcttgaagtg 2580
   agtgacagcg atggattcct agatggccag ccattgggtc aaggatcacg agcaagaaaa 2640
   gttaaatcag ctcaagagag gcaaagagca gagaggacgt ggggtggaac aagcatatcg 2700
   tcaatagagg aaccaccacc atacctccct ttacctcccc caccaccact gcattcccat 2760
   caacgaccaa ggcccaaaac tgcgaaacct aacagagtcg tcccagaagc agtggtagca 2820
   aagcgtccac agagctcagc tttaggggac agggttaagt ccccaccaga actccttgaa 2880
   ccatggagca caagaggcat tgcaaccata aatacaatac ttgcttggca accttctgat 2940
   caagatagca tgtag 2955
<210> 54
   <211> 833
   <212> PRT
   <213> Drosophila melanogaster
<400> 54
   Met Gly Asn Thr Glu Ser Asn Val Thr Ser Gly Val Lys Lys Gln Ala
   1               5                   10                  15
   Gly Val Ser Thr Gln Ala Leu Tyr Lys Phe Val Asn Leu Lys Gly Gly
               20                  25                  30
   Gly Leu Leu Val Asp Met Met Lys Arg Ala Cys Gln Thr Lys Gln Phe
           35                  40                  45
   Ala Glu Ile Asp His Ala Ile Lys Thr Lys Val Glu Pro Phe Leu Tyr
       50                  55                  60
   Asn Lys Gly Ala Gly Arg Tyr Phe Pro Ile Ser Lys Leu Val Leu Leu
   65                  70                  75                  80
   Arg Asn Arg Asp Arg Pro Arg Thr Arg Gln Leu Pro Glu Ile Arg Ala
                   85                  90                  95
   Leu Glu Asn Pro Asp Asp Asp Phe Asn Ile His Asp Tyr Cys Pro Glu
               100                 105                 110
   Val Ser Glu Ala Glu Tyr Ile Ser Asn Pro Thr Ala Tyr Arg Phe Val
           115                 120                 125
   Cys Trp Asp Leu Asn Met Arg Gly Ala Val Gly Glu Thr Ile Leu His
       130                 135                 140
   Leu Cys Leu Leu Asn Ala Ser Ser Leu His Ala Asp Leu Ala Lys Arg
   145                 150                 155                 160
   Leu Leu Lys Phe Tyr Pro Lys Leu Ile Leu Asp Ile Tyr Met Ser Asp
                   165                 170                 175
   Glu Tyr Tyr Gly Glu Ser Val Leu His Ile Ala Ile Val Asn Glu Asp
               180                 185                 190
   Pro Ala Met Val Lys Tyr Leu Leu Asp Ala Asn Ala Asp Val Gln Glu
           195                 200                 205
   Arg Cys Cys Gly Ala Phe Met Ser Ala Glu Asp Thr Lys Phe Ser Arg
       210                 215                 220
   Thr Asp Ser Pro Asp His Glu Tyr Val Ala Leu Cys Pro Met Thr Asn
   225                 230                 235                 240
   Tyr Asp Gly Tyr Val Tyr Trp Gly Glu Tyr Pro Leu Ser Phe Ala Ala
                   245                 250                 255
   Cys Leu Ser Gln Glu Glu Cys Phe Arg Leu Val Leu Ala Arg Gly Ala
               260                 265                 270
   Asp Pro Asp Phe Gln Asp Thr Asn Gly Asn Thr Val Leu His Met Leu
           275                 280                 285
   Val Ile Tyr Glu Lys Ile Glu Met Phe Asp Val Gly Tyr Glu Val Gly
       290                 295                 300
   Thr Asn Ile His Ile Lys Asn Ile Gln Asn Leu Thr Pro Leu Thr Leu
   305                 310                 315                 320
   Ala Ala Lys Leu Gly Arg Val Glu Met Phe Phe His Val Met Ser Ile
                   325                 330                 335
   Glu Arg Glu Ile Tyr Trp Gln Leu Gly Ser Ile Thr Cys Ala Ala Tyr
               340                 345                 350
   Pro Leu Leu Met Ile Asp Thr Ile Asn Glu Glu Thr Gly Asn Ile Asn
           355                 360                 365
   Lys Asp Ser Val Leu Asn Phe Val Val Phe Gly Asp Lys Leu Glu His
       370                 375                 380
   Leu Glu Leu Leu Asp Gly Val Val Ile Asp Leu Leu Lys Thr Lys Trp
   385                 390                 395                 400
   Asp Thr Phe Cys Lys Ser Arg Phe Tyr Lys Gln Phe Tyr Met Phe Ala
                   405                 410                 415
   Leu Tyr Phe Leu Ile Ser Leu Phe Ser Phe Ile Leu Arg Pro Gly Pro
               420                 425                 430
   Asp Ala Lys Asp Glu Asp Glu Asp Gly Ala Asn Ser Thr Thr Ala Lys
           435                 440                 445
   Ser Asp Leu Tyr Arg Gln Asn Gly Ser Asp Ser Tyr His Leu His Ser
       450                 455                 460
   Lys Arg Ala Thr Met Thr Thr Glu Tyr Lys Thr Phe Trp Leu Asn Phe
   465                 470                 475                 480
   Thr Glu Tyr Tyr Asp Pro Ser Glu Val Glu Val Leu Pro Ala Trp Trp
   485 490 495
   Glu Ser Tyr Ala Gln Cys Pro Leu Met Asn Leu Glu Ser Asp Leu Ala
   500 505 510
   Lys Leu Arg Ile Met Ala Glu Leu Leu Asn Phe Val Gly Ala Ile Leu
   515 520 525
   Tyr Leu Leu Val Ala Leu Arg Glu Ala Arg Phe Leu Gly Leu Lys Met
   530 535 540
   Phe Ile Glu Asn Leu Met Thr Ala Pro Ser Arg Val Met Phe Leu Phe
   545 550 555 560
   Ser Cys Ala Leu Met Met Thr Ile Pro Trp Leu Arg Val Ser Cys Leu
   565 570 575
   Thr Glu Ile Asp Asp His Val Thr Val Val Ile Met Leu Thr Thr Ala
   580 585 590
   Pro Tyr Phe Leu Phe Phe Cys Arg Gly Phe Lys Thr Val Gly Pro Phe
   595 600 605
   Val Val Met Ile Tyr Arg Met Val Met Gly Asp Leu Leu Arg Phe Val
   610 615 620
   Ser Ile Tyr Leu Val Phe Val Met Gly Phe Ser Gln Ala Phe Tyr Ile
   625 630 635 640
   Ile Phe Leu Thr Phe Asp Asn Pro Ser Ser Pro Glu Asp Gln Asp Ala
   645 650 655
   Glu Ser Asn Pro Met Pro Ser Pro Met Glu Ser Ile Val Ala Met Phe
   660 665 670
   Leu Met Ser Leu Thr Asn Phe Gly Asp Tyr Tyr Gly Ala Met Val Ser
   675 680 685
   Thr Gln His Glu Tyr Glu Ala Lys Ile Leu Phe Phe Leu Phe Met Val
   690 695 700
   Ile Val Ser Val Leu Leu Val Asn Met Leu Ile Ala Met Met Gly Asn
   705 710 715 720
   Thr Tyr Gln Lys Ile Ala Glu Ile Arg Asn Glu Trp Gln Arg Gln Trp
   725 730 735
   Ala Arg Ile Val Leu Val Val Glu Arg Ser Val Pro Pro Ala Glu Arg
   740 745 750
   Leu Lys Asn Phe Met Gln Tyr Ser Gln Ser Met Ser Asp Gly Arg Arg
   755 760 765
   Ala Leu Val Leu Arg Leu Asn Met Thr Glu Glu Lys Glu Glu Met Lys
   770 775 780
   Glu Val Gln Glu Met Lys Arg Ile His Gln Arg Phe Ala Lys Lys Arg
   785 790 795 800
   Gln Met Glu Arg Glu Ala Arg Ala Leu Arg Arg Gln Gln Glu Tyr Glu
   805 810 815
   Lys Phe Phe Gly Thr Ala Pro Lys Ser Glu Cys Ser Asp Asn Asn Asn
   820 825 830
   Phe
<210> 55
   <211> 834
   <212> PRT
   <213> Drosophila melanogaster
<400> 55
   Met Gly Asn Thr Glu Ser Asn Val Thr Ser Gly Val Lys Lys Gln Ala
   1               5                   10                  15
   Gly Val Ser Thr Gln Ala Leu Tyr Lys Phe Val Asn Leu Lys Gly Gly
               20                  25                  30
   Gly Leu Leu Val Asp Met Met Lys Arg Ala Cys Gln Thr Lys Gln Phe
           35                  40                  45
   Ala Glu Ile Asp His Ala Ile Lys Thr Lys Val Glu Pro Phe Leu Tyr
       50                  55                  60
   Asn Lys Gly Ala Gly Arg Tyr Phe Pro Ile Ser Lys Leu Val Leu Leu
   65                  70                  75                  80
   Arg Asn Arg Asp Arg Pro Arg Thr Arg Gln Leu Pro Glu Ile Arg Ala
                   85                  90                  95
   Leu Glu Asn Pro Asp Asp Asp Phe Asn Ile His Asp Tyr Cys Pro Glu
               100                 105                 110
   Val Ser Glu Ala Glu Tyr Ile Ser Asn Pro Thr Ala Tyr Arg Phe Val
           115                 120                 125
   Cys Trp Asp Leu Asn Met Arg Gly Ala Val Gly Glu Thr Ile Leu His
       130                 135                 140
   Leu Cys Leu Leu Asn Ala Ser Ser Leu His Ala Asp Leu Ala Lys Arg
   145                 150                 155                 160
   Leu Leu Lys Phe Tyr Pro Lys Leu Ile Leu Asp Ile Tyr Met Ser Asp
                   165                 170                 175
   Glu Tyr Tyr Gly Glu Ser Val Leu His Ile Ala Ile Val Asn Glu Asp
               180                 185                 190
   Pro Ala Met Val Lys Tyr Leu Leu Asp Ala Asn Ala Asp Val Gln Glu
           195                 200                 205
   Arg Cys Cys Gly Ala Phe Met Ser Ala Glu Asp Thr Lys Phe Ser Arg
       210                 215                 220
   Thr Asp Ser Pro Asp His Glu Tyr Val Ala Leu Cys Pro Met Thr Asn
   225                 230                 235                 240
   Tyr Asp Gly Tyr Val Tyr Trp Gly Glu Tyr Pro Leu Ser Phe Ala Ala
                   245                 250                 255
   Cys Leu Ser Gln Glu Glu Cys Phe Arg Leu Val Leu Ala Arg Gly Ala
               260                 265                 270
   Asp Pro Asp Phe Gln Asp Thr Asn Gly Asn Thr Val Leu His Met Leu
           275                 280                 285
   Val Ile Tyr Glu Lys Ile Glu Met Phe Asp Val Gly Tyr Glu Val Gly
       290                 295                 300
   Thr Asn Ile His Ile Lys Asn Ile Gln Asn Leu Thr Pro Leu Thr Leu
   305                 310                 315                 320
   Ala Ala Lys Leu Gly Arg Val Glu Met Phe Phe His Val Met Ser Ile
                   325                 330                 335
   Glu Arg Glu Ile Tyr Trp Gln Leu Gly Ser Ile Thr Cys Ala Ala Tyr
               340                 345                 350
   Pro Leu Leu Met Ile Asp Thr Ile Asn Glu Glu Thr Gly Asn Ile Asn
   355 360 365
   Lys Asp Ser Val Leu Asn Phe Val Val Phe Gly Asp Lys Leu Glu His
   370 375 380
   Leu Glu Leu Leu Asp Gly Val Val Ile Asp Leu Leu Lys Thr Lys Trp
   385 390 395 400
   Asp Thr Phe Cys Lys Ser Arg Phe Tyr Lys Gln Phe Tyr Met Phe Ala
   405 410 415
   Leu Tyr Phe Leu Ile Ser Leu Phe Ser Phe Ile Leu Arg Pro Gly Pro
   420 425 430
   Asp Ala Lys Asp Glu Asp Glu Asp Gly Ala Asn Ser Thr Thr Ala Lys
   435 440 445
   Ser Asp Leu Tyr Arg Gln Asn Gly Ser Asp Ser Tyr His Leu His Ser
   450 455 460
   Lys Arg Ala Thr Met Thr Thr Glu Tyr Lys Thr Phe Trp Leu Asn Phe
   465 470 475 480
   Thr Glu Tyr Tyr Asp Pro Ser Glu Val Glu Val Leu Pro Ala Trp Trp
   485 490 495
   Glu Ser Tyr Ala Gln Cys Pro Leu Met Asn Leu Glu Ser Asp Leu Ala
   500 505 510
   Lys Leu Arg Ile Met Ala Glu Leu Leu Asn Phe Val Gly Ala Ile Leu
   515 520 525
   Tyr Leu Leu Val Ala Leu Arg Glu Ala Arg Phe Leu Gly Leu Lys Met
   530 535 540
   Phe Ile Glu Asn Leu Met Thr Ala Pro Ser Arg Val Met Phe Leu Phe
   545 550 555 560
   Ser Cys Ala Leu Met Met Thr Ile Pro Trp Leu Arg Val Ser Cys Leu
   565 570 575
   Thr Glu Ile Asp Asp His Val Thr Val Val Ile Met Leu Thr Thr Ala
   580 585 590
   Pro Tyr Phe Leu Phe Phe Cys Arg Gly Phe Lys Thr Val Gly Pro Phe
   595 600 605
   Val Val Met Ile Tyr Arg Met Val Met Gly Asp Leu Leu Arg Phe Val
   610 615 620
   Ser Ile Tyr Leu Val Phe Val Met Gly Phe Ser Gln Ala Phe Tyr Ile
   625 630 635 640
   Ile Phe Leu Thr Phe Asp Asn Pro Ser Ser Pro Glu Asp Gln Asp Ala
   645 650 655
   Glu Ser Asn Pro Met Pro Ser Pro Met Glu Ser Ile Val Ala Met Phe
   660 665 670
   Leu Met Ser Leu Thr Asn Phe Gly Asp Tyr Tyr Gly Ala Met Val Ser
   675 680 685
   Thr Gln His Glu Tyr Glu Ala Lys Ile Leu Phe Phe Leu Phe Met Val
   690 695 700
   Ile Val Ser Val Leu Leu Val Asn Met Leu Ile Ala Met Met Gly Asn
   705 710 715 720
   Thr Tyr Gln Lys Ile Ala Glu Ile Arg Asn Glu Trp Gln Arg Gln Trp
   725 730 735
   Ala Arg Ile Val Leu Val Val Glu Arg Ser Val Pro Pro Ala Glu Arg
   740 745 750
   Leu Lys Asn Phe Met Gln Tyr Ser Gln Ser Met Ser Asp Gly Arg Arg
   755 760 765
   Ala Leu Val Leu Arg Leu Asn Met Thr Glu Glu Glu Lys Glu Glu Met
   770 775 780
   Lys Glu Val Gln Glu Met Lys Arg Ile His Gln Arg Phe Ala Lys Lys
   785 790 795 800
   Arg Gln Met Glu Arg Glu Ala Arg Ala Leu Arg Arg Gln Gln Glu Tyr
   805 810 815
   Glu Lys Phe Phe Gly Thr Ala Pro Lys Ser Glu Cys Ser Asp Asn Asn
   820 825 830
   Asn Phe
<210> 56
   <211> 838
   <212> PRT
   <213> Musca domestica
<400> 56
   Met Gly Asn Thr Glu Ser Asn Val Thr Ser Gly Val Lys Lys Gln Ala
   1               5                   10                  15
   Gly Val Ser Thr Gln Ala Leu Tyr Lys Phe Val Asn Leu Lys Gly Gly
               20                  25                  30
   Gly Leu Leu Val Asp Met Met Lys Arg Ala Cys Gln Thr Lys Gln Phe
           35                  40                  45
   Ala Glu Ile Asp His Ala Ile Lys Thr Lys Val Glu Pro Phe Leu Tyr
       50                  55                  60
   Asn Lys Gly Ala Gly Arg Tyr Phe Pro Ile Ser Lys Met Val Leu Leu
   65                  70                  75                  80
   Arg Asn Lys Glu Arg Ala Arg Thr Lys Gln Leu Pro Glu Ile Arg Ala
                   85                  90                  95
   Leu Glu Asn Pro Asp Glu Asp Phe Asn Ile His Asp Tyr Cys Ser Glu
               100                 105                 110
   Val Ser Glu Ala Glu Tyr Ile Ser Asn Pro Ser Ala Tyr Arg Phe Val
           115                 120                 125
   Cys Trp Asn Leu Asn Glu Arg Gly Ala Val Gly Glu Thr Ile Leu His
       130                 135                 140
   Leu Cys Leu Leu Asn Ala Ser Ser Leu His Ala Asp Leu Ala Lys Arg
   145                 150                 155                 160
   Leu Leu Lys Phe Tyr Pro Lys Leu Ile Met Asp Ile Tyr Leu Ser Asp
                   165                 170                 175
   Glu Tyr Tyr Gly Glu Ser Val Leu His Ile Ala Ile Val Asn Glu Asp
               180                 185                 190
   Pro Ala Met Val Lys Tyr Leu Leu Asp Ala Asn Ala Asp Val Gln Glu
           195                 200                 205
   Arg Cys Cys Gly Ala Phe Met Ser Ala Glu Asp Gln Lys Ala Ser Arg
       210                 215                 220
   Tyr Asp Ser Pro Glu His Glu Tyr Val Gly Val His Pro Met Thr Asn
   225                 230                 235                 240
   Tyr Asp Gly Tyr Val Tyr Trp Gly Glu Tyr Pro Leu Ser Phe Ala Ala
   245 250 255
   Cys Leu Ser Gln Glu Glu Cys Phe Arg Leu Val Leu Ala Arg Gly Ala
   260 265 270
   Asp Pro Asp Ser Gln Asp Thr Asn Gly Asn Thr Val Leu His Met Leu
   275 280 285
   Val Ile Tyr Glu Lys Ile Asp Met Phe Asp Val Ala Tyr Glu Val Gly
   290 295 300
   Thr Asn Ile His Ile Arg Asn Val Gln Asn Leu Thr Pro Leu Thr Leu
   305 310 315 320
   Ala Ala Lys Leu Ala Arg Val Glu Met Phe Phe His Ile Leu Ser Ile
   325 330 335
   Glu Arg Glu Ile Tyr Trp Gln Leu Gly Ser Ile Thr Cys Ala Ala Tyr
   340 345 350
   Pro Leu Ser Lys Ile Asp Thr Ile Asp Val Lys Thr Gly Asn Ile Asn
   355 360 365
   Lys Asp Ser Val Leu Asn Phe Val Val Phe Gly Asp Lys Leu Glu His
   370 375 380
   Leu Glu Leu Leu Asp Gly Val Val Ile Asp Leu Leu Lys Thr Lys Trp
   385 390 395 400
   Glu Thr Phe Val Lys Ser Arg Phe Tyr Lys Gln Phe Tyr Met Phe Ser
   405 410 415
   Phe Tyr Phe Leu Phe Ser Leu Val Ser Phe Ile Ser Arg Pro Gly Pro
   420 425 430
   Lys Ile Glu Asp Glu Asp Glu Glu Gly Asp Ala Asn Asp Ser Ala Gly
   435 440 445
   Lys Gly Asn Asp Ser Thr Thr Asn Ala Arg Leu Leu Tyr Ala Thr Pro
   450 455 460
   Ala Ser Arg Val Thr Gly Ile Glu Lys Leu Pro Tyr Lys Thr Phe Trp
   465 470 475 480
   Leu Asn Phe Ser Glu Tyr Ile Asp Asp Asp Ala Asn Ala Glu Asn Met
   485 490 495
   Pro Ser Trp Trp Ala Ser Tyr Glu Glu Cys Pro Leu Met Asp Met Glu
               500                 505                 510
   Thr Asn Leu Ala Lys Phe Arg Ile Val Ser Glu Ile Val Ile Phe Phe
           515                 520                 525
   Gly Ala Ile Leu Tyr Leu Leu Ser Ala Leu Arg Glu Ala Lys Phe Leu
       530                 535                 540
   Gly Tyr Lys Met Phe Val Glu Asn Leu Met Thr Ala Pro Ser Arg Val
   545                 550                 555                 560
   Met Phe Leu Phe Ser Cys Cys Leu Met Met Thr Ile Pro Trp Leu Arg
                   565                 570                 575
   Leu Ser Cys Leu Thr Glu Leu Asp Asp His Val Ala Val Met Ile Met
               580                 585                 590
   Leu Thr Thr Ala Pro Tyr Phe Leu Phe Phe Cys Arg Gly Phe Lys Thr
           595                 600                 605
   Val Gly Pro Phe Val Val Met Ile Tyr Arg Met Val Met Gly Asp Leu
       610                 615                 620
   Ile Arg Phe Val Ser Ile Tyr Leu Val Phe Val Met Gly Phe Ser Gln
   625                 630                 635                 640
   Ala Tyr Tyr Ile Ile Phe Leu Thr Phe Asp Asn Pro Ala Thr Pro Glu
                   645                 650                 655
   Glu Ile Asp Asp Thr Gly Thr Asn Pro Met Pro Ser Pro Met Glu Ser
               660                 665                 670
   Val Val Ala Met Phe Leu Met Ser Leu Thr Asn Phe Gly Asp Tyr Tyr
           675                 680                 685
   Gly Ala Met Gly Ser Thr Gln His Glu Tyr Glu Ala Lys Ile Leu Phe
       690                 695                 700
   Phe Leu Phe Met Val Ile Val Ser Val Leu Leu Val Asn Met Leu Ile
   705                 710                 715                 720
   Ala Met Met Gly Asn Thr Tyr Gln Lys Ile Ala Glu Ile Arg Asn Glu
                   725                 730                 735
   Trp Gln Arg Gln Trp Ala Arg Ile Val Leu Val Val Glu Arg Ser Val
               740                 745                 750
   Pro Pro Ala Glu Arg Leu Lys Asn Phe Met Gln Tyr Ser Gln Pro Met
           755                 760                 765
   Ser Asp Gly Arg Arg Ala Leu Val Leu Arg Leu Asn Met Ser Asp Glu
       770                 775                 780
   Asp Lys Glu Glu Met Lys Glu Val Gln Glu Met Lys Arg Ile His Gln
   785                 790                 795                 800
   Arg Phe Ser Lys Lys Arg Gln Met Glu Arg Glu Ala Arg Ala Lys Lys
                   805                 810                 815
   Arg Gln Glu Glu Tyr Glu Lys Phe Phe Gly Ala Thr Pro Thr Ser Asp
               820                 825                 830
   Asn Glu Asn Asn Asn Phe
           835
<210> 57
   <211> 844
   <212> PRT
   <213> Ceratitis capitata
<400> 57
   Met Gly Asn Thr Glu Ser Asn Val Thr Ser Gly Val Lys Lys Gln Ala
   1               5                   10                  15
   Gly Val Ser Thr Gln Ala Leu Tyr Lys Phe Val Asn Leu Lys Gly Gly
               20                  25                  30
   Gly Leu Leu Val Asp Met Met Lys Arg Ala Cys Gln Thr Lys Gln Phe
           35                  40                  45
   Ala Glu Ile Asp His Ala Ile Lys Thr Lys Val Glu Pro Phe Leu Tyr
       50                  55                  60
   Asn Lys Gly Ala Gly Arg Tyr Phe Pro Ile Ser Lys Leu Val Leu Leu
   65                  70                  75                  80
   Arg Asn Lys Glu Arg Ala Arg Thr Lys Gln Leu Pro Glu Ile Arg Ala
                   85                  90                  95
   Met Glu Asn Pro Asp Glu Asp Phe Asn Ile His Asp His Cys Thr Glu
               100                 105                 110
   Val Ser Glu Ala Glu Tyr Ile Ser Asn Pro Ser Ala Tyr Arg Tyr Val
           115                 120                 125
   Cys Trp Asn Leu Asn Glu Arg Gly Ala Val Gly Glu Thr Ile Leu His
       130                 135                 140
   Leu Cys Leu Leu Asn Ala Ser Ser Leu His Ala Asp Leu Ala Lys Arg
   145                 150                 155                 160
   Leu Leu Lys Phe Tyr Pro Lys Leu Ile Leu Asp Val Tyr Met Cys Asp
                   165                 170                 175
   Glu Tyr Tyr Gly Glu Ser Val Leu His Ile Ala Ile Val Asn Glu Asp
               180                 185                 190
   Pro Ala Met Val Lys Tyr Leu Leu Asp Ala Gly Ala Asp Val Gln Glu
           195                 200                 205
   Arg Cys Cys Gly Ala Phe Met Ser Ala Glu Asp Gln Lys Ala Ser Arg
       210                 215                 220
   Tyr Asp Ser Pro Asp His Glu Tyr Val Gly Val Gln Pro Met Thr Asn
   225                 230                 235                 240
   Tyr Asp Gly Tyr Val Tyr Trp Gly Glu Tyr Pro Met Ser Phe Ala Ala
                   245                 250                 255
   Cys Leu Ser Gln Glu Asp Cys Phe Arg Leu Val Leu Ala Arg Gly Ala
               260                 265                 270
   Asp Pro Asn Leu Gln Asp Thr Asn Gly Asn Thr Ser Leu His Met Leu
           275                 280                 285
   Val Ile Tyr Glu Lys Ile Glu Met Phe Asp Val Ala Tyr Glu Val Gly
       290                 295                 300
   Thr Asn Ile His Leu Arg Asn Val Gln Asn Leu Thr Ala Leu Thr Leu
   305                 310                 315                 320
   Ala Ala Lys Leu Gly Arg Val Glu Met Phe Phe His Ile Leu Ser Ile
                   325                 330                 335
   Glu Arg Glu Ile Tyr Trp Gln Leu Gly Ser Ile Thr Cys Ala Ala Tyr
               340                 345                 350
   Pro Leu Ser Met Ile Asp Thr Ile Asp Val Glu Thr Gly Asn Ile Asn
           355                 360                 365
   Lys Asp Ser Val Leu Asn Phe Val Val Phe Gly Asp Lys Leu Glu His
       370                 375                 380
   Leu Glu Leu Leu Asp Gly Val Val Ile Asp Leu Leu Lys Thr Lys Trp
   385 390 395 400
   Glu Ser Phe Val Lys Ser Arg Phe Tyr Lys Gln Phe Tyr Met Phe Ser
   405 410 415
   Phe Tyr Phe Leu Phe Ser Leu Ile Ser Phe Ile Ser Arg Pro Gly Pro
   420 425 430
   Thr Glu Lys Asn Glu Asp Asp Asp Asp Asp Asp Asp Ala Asp Ala Ser
   435 440 445
   Val Glu Ala Thr Val Thr Arg Ala Ala Asn Ser Thr Ala Pro Leu Gln
   450 455 460
   Arg Gly Asp Gln Trp Phe Lys Arg Leu Ile Lys Arg Ala Ile Asp Lys
   465 470 475 480
   Met Glu Tyr Lys Thr Phe Trp Leu Asn Phe Thr Asp Tyr Phe Asp Asp
   485 490 495
   Asn Asp Val Glu Ser Val Pro Pro Trp Trp Ala Ser Tyr Ala Glu Cys
   500 505 510
   Pro Leu Met Asn Met Glu Ser Asp Leu Ala Lys Leu Arg Ile Val Ala
   515 520 525
   Glu Ile Ile Ile Phe Phe Gly Ser Val Leu Tyr Leu Phe Ala Ala Leu
   530 535 540
   Arg Glu Ala Arg Phe Leu Gly Tyr Lys Met Phe Val Glu Asn Met Met
   545 550 555 560
   Thr Ala Pro Ser Arg Val Met Phe Leu Phe Ser Cys Ala Leu Met Met
   565 570 575
   Ser Ile Pro Trp Leu Arg Val Phe Cys Leu Thr Glu Ile Asp Asp His
   580 585 590
   Val Ala Val Phe Ile Met Leu Thr Thr Ala Pro Tyr Phe Leu Phe Phe
   595 600 605
   Cys Arg Gly Phe Lys Thr Val Gly Pro Phe Val Val Met Ile Tyr Arg
   610 615 620
   Met Val Met Gly Asp Leu Ile Arg Phe Val Ser Ile Tyr Leu Val Phe
   625 630 635 640
   Val Met Gly Phe Ser Gln Ala Tyr Tyr Ile Ile Phe Leu Thr Phe Asp
   645 650 655
   Asn Pro Ala Thr Pro Glu Glu Ile Asp Asp Ser Glu Ser Asn Pro Met
   660 665 670
   Pro Ser Pro Met Glu Ser Ile Val Ala Met Phe Leu Met Ser Leu Thr
   675 680 685
   Asn Phe Gly Asp Tyr Phe Gly Gly Met Ala Ser Thr Gln His Glu Tyr
   690 695 700
   Glu Ala Lys Thr Leu Phe Phe Leu Phe Met Val Ile Val Ser Val Leu
   705 710 715 720
   Leu Val Asn Met Leu Ile Ala Met Met Gly Asn Thr Tyr Gln Lys Ile
   725 730 735
   Ala Glu Ile Arg Asn Glu Trp Gln Arg Gln Trp Ala Arg Ile Val Leu
   740 745 750
   Val Val Glu Arg Ser Val Pro Pro Ala Glu Arg Leu Lys Asn Phe Met
   755 760 765
   His Tyr Ser Gln Pro Met Ser Asp Gly Arg Arg Ala Leu Val Leu Arg
   770 775 780
   Leu Asn Met Ser Asp Glu Val Lys Glu Glu Met Lys Glu Val Gln Glu
   785 790 795 800
   Thr Lys Arg Ile His Gln Arg Phe Ser Lys Lys Arg Gln Ile Glu Arg
   805 810 815
   Glu Ala Arg Ala Gln Lys Arg Gln Glu Glu Tyr Glu Lys Phe Phe Gly
   820 825 830
   Lys Lys Pro Ser Gly Asn Ser Asp Asn Asn Asn Ala
   835 840
<210> 58
   <211> 833
   <212> PRT
   <213> Anopheles gambiae
<400> 58
   Met Gly Asn Thr Glu Ser Asn Val Thr Ser Gly Val Lys Lys Gln Ala
   1               5                   10                  15
   Gly Val Ser Thr Gln Ala Leu Tyr Lys Phe Val Asn Leu Lys Gly Gly
               20                  25                  30
   Gly Leu Leu Val Asp Met Met Lys Arg Ala Ile Gln Asn Lys Gln Tyr
           35                  40                  45
   Ala Glu Ile Asp His Ala Ile Lys Thr Lys Val Glu Pro Phe Leu Tyr
       50                  55                  60
   Asn Lys Gly Lys Gly Lys Tyr Ile Pro Val Ser Val Leu Val Leu Leu
   65                  70                  75                  80
   Arg Asn Arg Glu Arg Pro Arg His Lys Gln Leu Pro Glu Ile Arg Ala
                   85                  90                  95
   Met Glu Asn Pro Glu Glu Asp Phe Asp Ile Asp Ala Val Cys Pro Glu
               100                 105                 110
   Val Ser Glu Ala Glu Tyr Tyr Leu Asn Pro Ser Gly Tyr Arg Glu Val
           115                 120                 125
   Cys Trp Asn Ile Lys Glu Arg Gly Ala Val Gly Glu Thr Ile Leu His
       130                 135                 140
   Leu Cys Leu Leu Asn Ala Thr Ser Leu His Ala Asp Leu Ala Lys Arg
   145                 150                 155                 160
   Leu Leu Arg Phe Tyr Pro Lys Leu Ile Asn Asp Val Tyr Met Cys Asp
                   165                 170                 175
   Glu Tyr Tyr Gly Glu Asn Val Leu His Leu Ala Ile Val Asn Glu Asp
               180                 185                 190
   Pro Ala Met Val Lys Tyr Leu Leu Asp His Asn Ala Asp Val Asn Glu
           195                 200                 205
   Arg Cys Cys Gly Thr Phe Met Cys Pro Glu Asp Gln Lys Ala Ser Arg
       210                 215                 220
   Tyr Asp Thr Pro Glu Thr Glu Val Val Gln Met Val Gln Val Thr Asn
   225                 230                 235                 240
   Tyr Asp Gly Tyr Val Tyr Trp Gly Glu Tyr Pro Leu Thr Phe Ala Ala
                   245                 250                 255
   Cys Leu Gly Gln Glu Glu Cys Tyr Arg Leu Val Leu Ala Arg Gly Ala
   260 265 270
   Asp Pro Asp Asn Lys Asp Phe Asn Gly Asn Thr Val Leu His Met Leu
   275 280 285
   Val Ile Tyr Glu Lys Ile Ala Thr Phe Asp Met Gly Tyr Glu Val Gly
   290 295 300
   Ser Ser Leu Ser Ile Arg Asn His Gln Asn Leu Thr Pro Leu Thr Leu
   305 310 315 320
   Ala Ala Lys Leu Gly Arg Val Glu Met Phe Phe His Ile Met Asn Ile
   325 330 335
   Glu Arg Glu Ile Tyr Trp Gln Leu Gly Ser Ile Thr Cys Ala Ala Tyr
   340 345 350
   Pro Leu Gly Leu Ile Asp Thr Ile Asp Val Glu Thr Gly Asn Ile Asn
   355 360 365
   Lys Asp Ser Ala Leu Asn Leu Val Ala Phe Gly Asp Lys Asp Glu His
   370 375 380
   Leu Asp Leu Leu Asp Gly Val Leu Ile Asp Leu Leu Lys Thr Lys Trp
   385 390 395 400
   Asn Thr Phe Val Lys Asp Lys Phe Tyr Arg Gln Phe Phe Met Phe Phe
   405 410 415
   Cys Tyr Phe Cys Val Ser Leu Val Ser Phe Thr Leu Arg Asn Gly Pro
   420 425 430
   Pro Pro Ala Glu Asp Asp Asp Gly Lys Glu Ser Glu Ala Gly Gly Asn
   435 440 445
   Lys Thr Ala Ala Val Thr Lys Glu Glu Leu Arg Arg Gln Leu Trp Asn
   450 455 460
   Glu Thr Thr Leu Glu Asp Ala Leu Ala Ala Leu Ala Gly Asn Gly Ser
   465 470 475 480
   Phe Ala Gly Arg Leu Ser Asn Leu Glu Gln Ser Asn Gly Ser Ser Asp
   485 490 495
   Ala Met Gly Glu Phe Asp Pro Ala Glu Met Asp Asp Glu Gly Leu Phe
   500 505 510
   Phe Asn Ser Asn Cys His Thr Met Asp Tyr Asp Gly Val Glu Gly Lys
           515                 520                 525
   Val Arg Leu Ile Ser Glu Met Ile Ile Leu Val Gly Ser Phe Leu Tyr
       530                 535                 540
   Leu Leu Ala Ala Leu Arg Glu Leu Lys Phe Leu Gly Arg Lys Met Phe
   545                 550                 555                 560
   Phe Glu Asn Leu Met Thr Ala Pro Ser Arg Val Met Phe Leu Phe Ser
                   565                 570                 575
   Cys Cys Ile Met Met Ile Val Pro Phe Leu Lys Val Leu Cys Phe Thr
               580                 585                 590
   Glu Leu Glu Asp His Val Ala Val Thr Ile Met Leu Thr Thr Ala Pro
           595                 600                 605
   Tyr Phe Leu Phe Phe Cys Arg Gly Phe Lys Thr Val Gly Pro Phe Val
       610                 615                 620
   Val Met Ile Tyr Arg Met Val Met Gly Asp Leu Leu Arg Phe Val Val
   625                 630                 635                 640
   Ile Tyr Leu Val Phe Val Met Gly Phe Ser Gln Ala Tyr Tyr Ile Val
                   645                 650                 655
   Phe Leu Ser Tyr Lys Pro Asp Glu Glu Gly Asp Pro Asn Pro Met Pro
               660                 665                 670
   Ser Pro Ile Glu Ser Ile Val Ala Met Phe Leu Met Ser Leu Thr Asn
           675                 680                 685
   Phe Gly Asp Tyr Tyr Gly Ala Leu Glu Asn Thr Asp His Glu Met Cys
       690                 695                 700
   Ala Lys Ile Leu Phe Val Leu Phe Met Val Ile Val Ala Val Leu Leu
   705                 710                 715                 720
   Val Asn Met Leu Ile Ala Met Met Gly Asn Thr Tyr Gln Lys Ile Ala
                   725                 730                 735
   Glu Thr Lys Asn Glu Trp Gln Arg Gln Trp Ala Arg Ile Val Leu Val
               740                 745                 750
   Val Glu Arg Gly Val Pro Pro Lys Glu Arg Leu Lys Asn Leu Met Ser
           755                 760                 765
   Tyr Ser Gln Pro Met Ser Asp Gly Arg Arg Ala Leu Val Leu Arg Leu
   770 775 780
   Asn Met Thr Glu Asp Lys Glu Glu Met Lys Glu Ile Leu Glu Met Lys
   785 790 795 800
   Arg Val His Glu Arg Leu Ser Ile Lys Arg Gln Ile Glu Arg Asp Ala
   805 810 815
   Arg Ala Glu Gln Arg Arg Leu Leu Arg Glu Lys Tyr Leu Asn Met Ser
   820 825 830
   Ser
<210> 59
   <211> 815
   <212> PRT
   <213> Aedes aegypti
<400> 59
   Met Gly Asn Thr Glu Ser Asn Val Thr Ser Gly Val Lys Lys Gln Ala
   1 5 10 15
   Gly Val Ser Thr Gln Ala Leu Tyr Lys Phe Val Asn Leu Lys Gly Gly
   20 25 30
   Gly Leu Leu Val Asp Met Met Lys Arg Ala Ile Gln Asn Lys Gln Tyr
   35 40 45
   Ala Glu Ile Asp His Ala Ile Lys Thr Lys Val Glu Pro Phe Leu Tyr
   50 55 60
   Asn Lys Gly Lys Gly Lys Tyr Ile Pro Val Ala Gln Leu Val Leu Leu
   65 70 75 80
   Arg Asn Arg Glu Arg Pro Arg Ser Lys Gln Leu Pro Glu Ile Arg Ala
   85 90 95
   Leu Glu Asn Pro Glu Glu Asp Phe Asp Ile Asp Ala Val Cys Pro Asp
   100 105 110
   Val Thr Glu Gly Glu Tyr Gln Arg Asn Pro Ser Gly Tyr Arg Glu Val
   115 120 125
   Cys Trp Asn Ile Lys Glu Arg Gly Ala Val Gly Glu Thr Ile Leu His
   130 135 140
   Leu Cys Leu Leu Asn Ala Thr Ser Leu His Ala Asp Leu Ala Lys Arg
   145 150 155 160
   Leu Leu Arg Phe Tyr Pro Lys Leu Ile Asn Asp Ile Tyr Met Cys Asp
   165 170 175
   Glu Tyr Tyr Gly Glu Ser Val Leu His Val Ala Ile Val Asn Glu Asp
   180 185 190
   Pro Ala Met Val Lys Tyr Leu Leu Asp Ser Gly Ser Asp Val Asp Glu
   195 200 205
   Arg Cys Cys Gly Thr Phe Met Cys Pro Glu Asp Gln Lys Ala Thr Arg
   210 215 220
   Tyr Asp Ser Ile Glu Thr Glu Ile Ile Cys Val Leu Pro Met Thr Asn
   225 230 235 240
   Tyr Asp Gly Tyr Val Tyr Trp Gly Glu Tyr Pro Leu Ser Phe Ala Ala
   245 250 255
   Cys Leu Gly Gln Glu Glu Cys Tyr Arg Leu Val Leu Ala Arg Gly Ala
   260 265 270
   Asp Pro Asp Asn Gln Asp Ser Asn Gly Asn Thr Val Leu His Met Leu
   275 280 285
   Val Ile Tyr Glu Lys Ile Ser Thr Phe Asp Met Ala Tyr Glu Val Gly
   290 295 300
   Ser Ser Leu Asp Ile Arg Asn Leu Gln Asn Leu Thr Pro Leu Thr Leu
   305 310 315 320
   Ala Ala Lys Leu Gly Arg Val Glu Met Phe Phe His Ile Met Asn Ile
   325 330 335
   Glu Arg Glu Ile Tyr Trp Gln Leu Gly Ala Ile Thr Cys Ala Ala Tyr
   340 345 350
   Pro Leu Ala Leu Ile Asp Thr Ile Asp Ile Glu Thr Gly Asn Ile Asn
   355 360 365
   Lys Asp Ser Ala Leu Asn Leu Val Val Phe Gly Asp Lys Ala Glu His
   370 375 380
   Leu Asp Leu Leu Glu Gly Val Leu Ile Asp Leu Leu Lys Thr Lys Trp
   385 390 395 400
   Asn Ser Phe Val Lys Asp Lys Phe Tyr Arg Gln Phe Phe Val Phe Phe
   405 410 415
   Trp Tyr Phe Cys Val Ser Leu Val Ser Phe Thr Leu Arg Arg Gly Pro
   420 425 430
   Met Asn Leu Glu Glu Thr Ala Ala Asn Arg Thr Thr Thr Ser Ile Thr
   435 440 445
   Leu Lys Asn Leu Thr Phe Ser Asp Ser Thr Asn Ala Thr Leu Pro Thr
   450 455 460
   Leu Leu Ala Ala Asp Val Asn Glu Lys Ile Val Gln Phe Ile Ala Asn
   465 470 475 480
   Glu Thr Leu Phe Asp Pro Ala Val Met Glu Glu Asn Thr Phe Phe Asn
   485 490 495
   Ser Lys Cys Ala Thr Met Ala Tyr Asp Thr Leu Gln Asp Lys Ile Arg
   500 505 510
   Leu Val Ser Glu Ile Ile Ile Leu Ile Gly Ser Phe Leu Tyr Leu Val
   515 520 525
   Gly Ala Leu Arg Glu Phe Arg Phe Leu Gly Arg Lys Met Phe Phe Glu
   530 535 540
   Asn Leu Met Ile Ser Pro Ser Arg Val Met Phe Leu Phe Ser Cys Cys
   545 550 555 560
   Ile Met Met Ile Val Pro Phe Leu Lys Val Leu Cys Leu Ile Glu Leu
   565 570 575
   Glu Asp His Val Ala Val Thr Ile Met Leu Thr Thr Ala Pro Tyr Phe
   580 585 590
   Leu Phe Phe Cys Arg Gly Phe Lys Thr Val Gly Pro Phe Val Val Met
   595 600 605
   Ile Tyr Arg Met Val Met Gly Asp Leu Leu Arg Phe Val Val Ile Tyr
   610 615 620
   Leu Val Phe Val Met Gly Phe Ser Gln Ala Tyr Tyr Ile Val Phe Leu
   625 630 635 640
   Ser Phe Lys Gly Glu Glu Glu Gly Asp Glu Asn Pro Met Pro Ser Pro
   645 650 655
   Met Glu Ser Ile Val Ala Met Phe Leu Met Ser Leu Thr Asn Phe Gly
   660 665 670
   Asp Tyr Tyr Gly Ala Leu Glu Lys Thr Glu His Glu Gly Cys Ala Lys
   675 680 685
   Val Leu Phe Val Ile Phe Met Val Ile Val Ala Val Leu Leu Val Asn
   690 695 700
   Leu Leu Ile Ala Met Met Gly Asn Thr Tyr Thr Met Ile Ala Glu Thr
   705 710 715 720
   Lys Asn Glu Trp Gln Arg Gln Trp Ala Arg Ile Val Leu Val Val Glu
   725 730 735
   Arg Gly Val Pro Pro Ser Glu Arg Leu Lys Asn Phe Met Ser Tyr Ser
   740 745 750
   Gln Pro Met Ser Asp Gly Arg Arg Ala Leu Val Leu Arg Leu His Met
   755 760 765
   Thr Asp Glu Asp Lys Glu Glu Met Lys Glu Ile Leu Glu Met Lys Arg
   770 775 780
   Val His Glu Arg Leu Lys Lys Lys Arg Gln Val Glu Arg Glu Leu Arg
   785 790 795 800
   Ala Glu Gln Arg Arg Leu Ile Met Glu Lys Tyr Ala Asn Val Pro
   805 810 815
<210> 60
   <211> 816
   <212> PRT
   <213> Culex quinquefasciatus
<400> 60
   Met Gly Asn Thr Glu Ser Asn Val Thr Ser Gly Val Lys Lys Gln Ala
   1 5 10 15
   Gly Val Ser Thr Gln Ala Leu Tyr Lys Phe Val Asn Leu Lys Gly Gly
   20 25 30
   Gly Leu Leu Val Asp Met Met Lys Arg Ala Ile Gln Asn Lys Gln Tyr
   35 40 45
   Ala Glu Ile Asp His Ala Ile Lys Thr Lys Val Glu Pro Phe Leu Tyr
   50 55 60
   Asn Lys Gly Lys Gly Lys Tyr Ile Pro Val Ala Gln Leu Val Tyr Leu
   65                  70                  75                  80
   Arg Asn Arg Glu Arg Pro Arg His Lys Gln Leu Pro Glu Ile Arg Ala
                   85                  90                  95
   Leu Glu Asn Pro Glu Glu Asp Tyr Asp Ile Asp Lys Glu Cys Pro Asp
               100                 105                 110
   Val Thr Glu Gly Glu Tyr Met Arg Asn Pro Gly Gly Tyr Arg Glu Val
           115                 120                 125
   Cys Trp Asn Ile Lys Glu Arg Gly Ala Val Gly Glu Thr Ile Leu His
       130                 135                 140
   Leu Cys Leu Leu Asn Ala Thr Ser Leu His Ala Asp Leu Ala Lys Arg
   145                 150                 155                 160
   Leu Leu Arg Phe Tyr Pro Lys Leu Ile Asn Asp Val Tyr Met Cys Asp
                   165                 170                 175
   Glu Tyr Tyr Gly Glu Ser Val Leu His Leu Ala Ile Val Asn Glu Asp
               180                 185                 190
   Pro Ala Met Val Lys Tyr Leu Leu Asp Ala Gly Ser Asp Val Asn Glu
           195                 200                 205
   Arg Cys Cys Gly Thr Phe Met Cys Pro Glu Asp Gln Lys Ala Ser Arg
       210                 215                 220
   Phe Asp Ser Leu Glu Thr Glu Ile Ile Cys Val Met Pro Gln Thr Asn
   225                 230                 235                 240
   Tyr Asp Gly Tyr Val Tyr Trp Gly Glu Tyr Pro Leu Ser Phe Ala Ala
                   245                 250                 255
   Cys Leu Gly Gln Glu Glu Cys Tyr Arg Leu Val Leu Ala Arg Gly Ala
               260                 265                 270
   Asp Pro Asp Asn Gln Asp Ser Asn Met Asn Asn Val Leu His Met Met
           275                 280                 285
   Val Ile Tyr Glu Lys Leu Thr Thr Phe Asp Met Ala Tyr Glu Val Gly
       290                 295                 300
   Ser Ser Leu Ser Ile Arg Asn Leu Gln Asn Leu Thr Pro Leu Thr Leu
   305                 310                 315                 320
   Ala Ala Lys Leu Gly Arg Val Glu Met Phe Phe His Ile Met Asn Ile
                   325                 330                 335
   Glu Arg Glu Ile Tyr Trp Gln Leu Gly Ser Ile Thr Cys Ala Ala Tyr
               340                 345                 350
   Pro Leu Ala Leu Leu Asp Thr Ile Asp Ile Glu Thr Gly Asn Ile Ser
           355                 360                 365
   Lys Asp Ser Ala Leu Asn Leu Val Val Phe Gly Asp Lys Asp Glu His
       370                 375                 380
   Leu Asp Leu Leu Glu Gly Val Leu Ile Asp Leu Leu Lys Thr Lys Trp
   385                 390                 395                 400
   Asn Thr Phe Val Lys Asp Lys Phe Tyr Ser Gln Phe Phe Val Phe Phe
                   405                 410                 415
   Cys Tyr Phe Cys Val Ser Leu Ile Ser Phe Thr Leu Arg Glu Gly Pro
               420                 425                 430
   Met Lys Leu Glu Asp Gly Glu Ala Asn Lys Thr Thr Thr Ser Ile Gln
           435                 440                 445
   Leu Lys Asn Met Thr Leu Ala Asp Leu Thr Asn Ala Ser Thr Leu Ser
       450                 455                 460
   Ser Leu Leu Val Thr Asp Val Ser Gly Lys Ile Val Gly Leu Asn Gly
   465                 470                 475                 480
   Ser Glu Thr Phe Phe Asp Pro Ser Thr Ile Glu Ser Asp Met Phe Phe
                   485                 490                 495
   Asn Ser Lys Cys Pro Thr Met Ile Tyr Lys Gly Ile Glu Gly Lys Ile
               500                 505                 510
   Arg Leu Val Ser Glu Ala Ile Ile Leu Val Gly Ser Phe Leu Tyr Leu
           515                 520                 525
   Val Ser Ala Leu Arg Glu Ser Arg Phe Leu Gly Arg Lys Met Phe Ile
       530                 535                 540
   Glu Asn Leu Met Thr Ala Pro Ser Arg Val Met Phe Leu Phe Ser Cys
   545                 550                 555                 560
   Cys Ile Met Met Val Val Pro Cys Leu Lys Val Leu Cys Phe Thr Glu
   565 570 575
   Leu Glu Asp His Val Ala Val Val Ile Met Leu Thr Thr Ala Pro Tyr
   580 585 590
   Phe Leu Phe Phe Cys Arg Gly Phe Lys Thr Val Gly Pro Phe Val Val
   595 600 605
   Met Ile Tyr Arg Met Val Met Gly Asp Leu Leu Arg Phe Val Ala Ile
   610 615 620
   Tyr Ser Val Phe Val Met Gly Phe Ser Gln Ala Tyr Tyr Ile Ala Phe
   625 630 635 640
   Leu Ser Tyr Lys Gly Glu Glu Glu Gly Asp Glu Asn Pro Met Pro Ser
   645 650 655
   Pro Met Glu Ser Ile Val Ala Met Phe Leu Met Ser Leu Thr Asn Phe
   660 665 670
   Gly Asp Tyr Tyr Ala Ala Leu Glu Asp Thr Ala His Glu Gly Cys Ala
   675 680 685
   Lys Val Leu Phe Val Thr Tyr Met Val Ile Val Ala Val Leu Leu Val
   690 695 700
   Asn Leu Leu Ile Ala Met Met Gly Asn Thr Tyr Thr Lys Ile Ala Glu
   705 710 715 720
   Thr Lys Asn Glu Trp Gln Arg Gln Trp Ala Arg Ile Val Leu Val Val
   725 730 735
   Glu Arg Gly Val Pro Pro Ala Glu Arg Leu Lys Asn Leu Met Asn Tyr
   740 745 750
   Ser Gln Pro Met Ser Asp Gly Arg Arg Ala Leu Val Leu Arg Leu Asn
   755 760 765
   Met Thr Glu Glu Asp Lys Glu Glu Met Lys Glu Ile Leu Asp Met Lys
   770 775 780
   Arg Val His Glu Arg Leu Ser Lys Lys Arg Ala Val Glu Arg Glu Ala
   785 790 795 800
   Arg Ala Glu Gln Arg Arg Leu Ile Ala Glu Lys Tyr Ala Asn Val Pro
   805 810 815
<210> 61
   <211> 794
   <212> PRT
   <213> Tribolium castaneum
<400> 61
   Met Gly Asn Thr Glu Ser Asn Val Thr Ser Gly Val Lys Lys Gln Ala
   1               5                   10                  15
   Gly Ala Ser Val Gln Pro Ile Tyr Lys Leu Cys Asp Leu Lys Gly Gly
               20                  25                  30
   Gly Leu Leu Val Glu Leu Met Lys Arg Ala Thr Gln Asn Lys Gln Tyr
           35                  40                  45
   Ala Glu Leu Asp His Ala Ile Lys Thr Lys Val Glu His Phe Leu Tyr
       50                  55                  60
   Asn Lys Gly Ala Gly Arg Tyr Phe Thr Ile Ser Asp Leu Val Leu Leu
   65                  70                  75                  80
   Arg Asn Lys Glu Arg Ser Arg Gln Lys Trp Leu Pro Gln Leu Lys Ala
                   85                  90                  95
   Met Glu Asn Pro Glu Asp Phe Glu Ile Asp Asp Glu Gly Pro Glu Ile
               100                 105                 110
   Thr Glu Glu Gln Tyr Gln Lys Asn Pro His Leu Tyr Arg His Val Cys
           115                 120                 125
   Trp Lys Ile Lys Glu Arg Gly Ala Val Gly Glu Ser Ile Met His Leu
       130                 135                 140
   Cys Leu Leu Asn Ala Thr Ser Leu His Ala Asp Ile Ala Lys Arg Leu
   145                 150                 155                 160
   Leu Arg Phe Tyr Pro Lys Leu Ile Asn Asp Ile Tyr Met Ser Asp Glu
                   165                 170                 175
   Tyr Tyr Gly Glu Asn Val Leu His Ile Ala Ile Val Asn Glu Asp Pro
               180                 185                 190
   Ser Met Val Lys Phe Leu Leu Asp Ser Gly Val Asn Ile Gln Glu Arg
           195                 200                 205
   Cys Cys Gly Asn Phe Met Cys Pro Glu Asp Gln Lys Ser Ser Arg Tyr
       210                 215                 220
   Asp Ser Leu Asp His Glu Trp Val Asn Val Cys Pro Val Thr Asn Tyr
   225 230 235 240
   Glu Gly Tyr Val Tyr Trp Gly Glu Tyr Pro Leu Thr Phe Ala Ala Cys
   245 250 255
   Leu Gly Gln Glu Glu Ser Phe Arg Leu Met Leu Ser Arg Gly Ala Asp
   260 265 270
   Pro Asp Ala Gln Asp Thr Asn Gly Asn Thr Val Leu His Leu Leu Val
   275 280 285
   Ile Leu Gln Lys Leu Glu Ala Phe Asp Met Ala Tyr Glu Val Gly Ala
   290 295 300
   Lys Leu Ser Ile Arg Asn Val Leu Ser Leu Thr Pro Leu Thr Leu Ala
   305 310 315 320
   Ala Lys Leu Ala Arg Ile Asp Met Phe Phe His Ile Leu Asn Leu Glu
   325 330 335
   Arg Glu Ile Tyr Trp Gln Ile Gly Ser Ile Thr Cys Ala Ala Tyr Pro
   340 345 350
   Leu Ser Gln Ile Asp Thr Ile Asp Ile Glu Thr Gly Gln Ile Ser Lys
   355 360 365
   Thr Ser Ala Leu Asn Leu Val Val Phe Gly Asp Lys Asp Glu His Leu
   370 375 380
   Glu Leu Met Asp Gly Val Leu Ile Asp Leu Leu Asn Ala Lys Trp Asn
   385 390 395 400
   Thr Phe Val Lys Phe Lys Phe Tyr Lys Gln Phe Phe Thr Phe Ala Phe
   405 410 415
   Tyr Phe Leu Ile Ser Leu Val Ala Phe Thr Leu Arg Pro Gly Pro Pro
   420 425 430
   His Lys Glu Ala Lys Leu Val Asn Val Thr Ile Asn Ala Thr Val Ser
   435 440 445
   Asn Thr Ser Lys Trp Glu Asn Leu Thr Ser Ile Pro Phe Gly Lys Thr
   450 455 460
   Pro Asp Asp Asp Asp Ser Asp Met Glu Glu Trp Trp Asp Asn Leu Gln
   465 470 475 480
   Glu Glu Cys Arg Leu Met Gln Leu Glu Ser Pro Glu Ser Lys Ile Arg
   485 490 495
   Leu Thr Ala Glu Val Leu Met Val Val Gly Ala Phe Ala Tyr Leu Ala
   500 505 510
   Ala Ala Val Arg Glu Ala Arg Phe Leu Gly Gly Arg Met Phe Phe Glu
   515 520 525
   Asn Leu Met Thr Ala Pro Ser Arg Val Met Phe Leu Phe Ser Cys Ile
   530 535 540
   Leu Met Leu Thr Val Pro Cys Leu Arg Leu Ala Cys Leu Asp Glu Phe
   545 550 555 560
   Glu Asp Ile Val Ala Val Val Ile Met Leu Thr Thr Ala Pro Tyr Phe
   565 570 575
   Leu Phe Phe Cys Arg Gly Phe Lys Thr Val Gly Pro Phe Val Val Met
   580 585 590
   Ile Tyr Arg Met Val Met Gly Asp Leu Ile Arg Phe Ala Ser Ile Tyr
   595 600 605
   Leu Val Phe Val Met Gly Phe Ser Gln Ala Phe Tyr Ile Ile Phe Leu
   610 615 620
   Ser Phe Asp Asn Pro Leu Thr Pro Asp Asp Val Asp Asp Ser Ala Thr
   625 630 635 640
   Asn Pro Ile Ser Thr Pro Ile Glu Ser Ile Met Ala Met Phe Leu Met
   645 650 655
   Ser Met Thr Asn Phe Gly Asp Tyr Tyr Ala Ala Phe Glu Lys Thr Asp
   660 665 670
   His Glu Tyr Glu Ala Lys Met Leu Phe Val Val Phe Met Val Ile Val
   675 680 685
   Ser Ile Leu Leu Ile Asn Met Leu Ile Ala Met Met Gly Asn Thr Tyr
   690 695 700
   Gln Lys Ile Ala Glu Thr Arg Asn Glu Trp Gln Arg Gln Trp Ala Arg
   705 710 715 720
   Ile Val Leu Val Val Glu Arg Gly Val Ser Pro Ser Glu Arg Leu Lys
   725 730 735
   Gln Leu Met Val Tyr Ser Gln Pro Met Ser Asp Gly Arg Arg Ala Leu
   740 745 750
   Val Leu Arg Leu Asn Gln Ser Asp Glu Asp Lys Glu Glu Met Lys Glu
   755 760 765
   Ile Leu Glu Met Arg Arg Arg His Glu Arg His Ile Lys Lys Arg Ser
   770 775 780
   Glu Lys Leu Lys Glu Asn Ala Lys Lys Asn
   785 790
<210> 62
   <211> 841
   <212> PRT
   <213> Bombyx mori
<400> 62
   Met Gly Asn Thr Glu Ser Asn Val Thr Ser Gly Val Lys Lys Gln Ala
   1 5 10 15
   Gly Thr Ser Glu Gln Lys Leu Tyr Lys Leu Val Asp Ile Lys Gly Gly
   20 25 30
   Gly Leu Leu Val Asp Met Met Lys Arg Ala Leu Gln Asn Lys Gln Tyr
   35 40 45
   Ala Glu Ile Asp His Ala Ile Lys Thr Lys Val Glu Pro Phe Leu Tyr
   50 55 60
   Asn Arg Gly Arg Gly Arg Tyr Ile Pro Ile Ser His Leu Val Leu Leu
   65 70 75 80
   Arg Asn Lys Glu Arg Pro Arg His Lys Leu Leu Pro Pro Leu Arg Gly
   85 90 95
   Met Glu Asn Pro Asp Glu Glu Phe Asp Val Asp Lys Asp Trp Pro Val
   100 105 110
   Val Thr Gln Glu Glu Tyr Asp Ala Asn Pro Ser Gly Tyr Arg Glu Leu
   115 120 125
   Cys Trp Asp Val Lys Glu Arg Gly Ala Val Gly Glu Thr Ile Leu His
   130 135 140
   Leu Cys Leu Leu Asn Ala Thr Ser Leu Leu Ala His Leu Ala Lys Arg
   145 150 155 160
   Leu Leu Arg Phe Tyr Pro Lys Met Ile Asn Asp Ile Tyr Ile Ser Asp
                   165                 170                 175
   Glu Tyr Tyr Gly Glu Thr Val Leu His Ile Thr Ile Val Asn Glu Asp
               180                 185                 190
   Pro Thr Met Ala Lys Phe Leu Leu Asp Ala Gly Ala Asp Tyr His Glu
           195                 200                 205
   Arg Cys Tyr Gly Asn Phe Met Cys Pro Glu Asp Gln Lys Ala Ser Arg
       210                 215                 220
   Thr Asp Ser Phe Asp His Glu Trp Val Asn Val Gln Pro Asp Thr Asn
   225                 230                 235                 240
   Tyr Asn Gly Tyr Val Tyr Trp Gly Glu Tyr Pro Leu Ser Phe Ala Ala
                   245                 250                 255
   Cys Leu Gly Gln Glu Glu Cys Tyr Arg Leu Ile Leu Ala Arg Gly Ala
               260                 265                 270
   Asp Pro Asp Lys Gln Asp Thr Asn Gly Asn Thr Val Leu His Met Leu
           275                 280                 285
   Val Ile Tyr Glu Lys Met Ser Thr Phe Asp Met Ala Tyr Glu Val Gly
       290                 295                 300
   Ala Ser Leu Asn Ile Arg Asn Val Gln Asn Leu Thr Pro Leu Thr Leu
   305                 310                 315                 320
   Ala Ala Lys Leu Ala Arg Thr Glu Met Phe Phe His Ile Leu Asn Ile
                   325                 330                 335
   Glu Arg Glu Ile Tyr Trp Gln Ile Gly Ala Thr Thr Cys Ala Ala Tyr
               340                 345                 350
   Pro Leu Gly Gln Val Asp Thr Ile Asp Thr Glu Thr Gly Leu Ile Ser
           355                 360                 365
   Lys Asp Ser Ala Leu Asn Leu Val Val Phe Gly Glu Lys Asp Glu His
       370                 375                 380
   Leu Gly Leu Leu Glu Gly Met Leu Ile Asp Leu Leu Lys Thr Lys Trp
   385                 390                 395                 400
   Asn Thr Phe Val Lys Phe Arg Phe Tyr Arg Gln Phe Ile Leu Phe Ser
                   405                 410                 415
   Cys Tyr Phe Leu Val Ser Leu Ile Cys Phe Thr Leu Arg Pro Gly Pro
               420                 425                 430
   Pro Asp Arg Ala Leu Asn Thr Thr Val Leu Asn Ser Thr Ile Gly Pro
           435                 440                 445
   Asn Val Thr Asp Ala Glu Leu Val Ser Asp Val Glu Asn Cys Thr Met
       450                 455                 460
   Thr Pro Asn Ala Asp Phe Asp Thr Asn Ala Val Glu Val Leu Asn Gly
   465                 470                 475                 480
   Thr Lys Phe Gly Gly Ser His Cys Ala Arg Phe Lys Ser His Pro Lys
                   485                 490                 495
   Glu Lys Ser Thr Glu Ala Pro Arg Glu Asn Asp Val Glu Gly Trp Trp
               500                 505                 510
   Glu Asp Leu Thr Glu Glu Cys Arg Leu Met Asn Leu Asp Thr Trp Gln
           515                 520                 525
   Ala Lys Leu Arg Ile Ser Ala Glu Leu Leu Leu Trp Met Gly Ala Leu
       530                 535                 540
   Ala Tyr Phe Gly Ala Ala Leu Arg Glu Ala Lys Phe Leu Gly Ile Lys
   545                 550                 555                 560
   Met Phe Ile Glu Asn Leu Ser Thr Val Pro Ser Arg Val Met Phe Leu
                   565                 570                 575
   Phe Ser Cys Leu Leu Met Leu Ile Leu Pro Thr Leu Arg Leu Trp Cys
               580                 585                 590
   Ala Asp Glu Ala Glu Asp His Leu Ala Val Met Ile Met Leu Thr Thr
           595                 600                 605
   Ala Pro Tyr Phe Leu Phe Phe Cys Arg Gly Phe Lys Thr Val Gly Pro
       610                 615                 620
   Phe Val Val Met Ile Tyr Arg Met Val Met Gly Asp Leu Leu Arg Phe
   625                 630                 635                 640
   Val Cys Ile Tyr Leu Val Phe Val Met Gly Phe Ser Gln Ala Tyr Tyr
                   645                 650                 655
   Val Ile Phe Leu Ser Phe Asp Asn Pro Asn Thr Pro Glu Gly Val Asp
               660                 665                 670
   Asp Ser Val Ser Asn Pro Met Pro Ser Pro Met Glu Ser Ile Met Ala
   675 680 685
   Met Phe Leu Met Ser Leu Thr Ser Phe Ser Asp Tyr Tyr Thr Ala Phe
   690 695 700
   Asp Arg Thr Asp His Glu Ile Glu Ala Lys Leu Leu Phe Val Ile Tyr
   705 710 715 720
   Met Ile Ile Val Ala Ile Leu Leu Val Asn Met Leu Ile Ala Met Met
   725 730 735
   Gly Asn Thr Tyr Gln Lys Ile Ala Glu Thr Arg Asn Glu Trp Gln Arg
   740 745 750
   Gln Trp Ala Arg Ile Val Leu Val Val Glu Arg Gly Val Pro Pro Ala
   755 760 765
   Gln Arg Leu Lys Gln Leu Met Thr Tyr Ser Gln Pro Met Ala Asn Gly
   770 775 780
   Lys Arg Ala Leu Val Leu Arg Ile Asn Gln Lys Asp Glu Asp Lys Glu
   785 790 795 800
   Glu Met Lys Glu Ile Leu Glu Met Lys Arg Thr His Glu Arg Ile Val
   805 810 815
   Ala Lys Arg Lys Gln Arg Glu Ala Leu Lys Pro Gly Glu Ile Pro Pro
   820 825 830
   Pro Ala Arg Asp Tyr Pro Ile Arg Lys
   835 840
<210> 63
   <211> 792
   <212> PRT
   <213> Anopheles darlingi
<400> 63
   Met Gly Asn Thr Glu Ser Asn Val Thr Ser Gly Val Lys Lys Gln Ala
   1 5 10 15
   Gly Val Ser Thr Gln Ala Leu Tyr Lys Phe Val Asn Leu Lys Gly Gly
   20 25 30
   Gly Leu Leu Val Asp Met Met Lys Arg Ala Ile Gln Asn Lys Gln Tyr
   35 40 45
   Ala Glu Ile Asp His Ala Ile Lys Thr Lys Val Glu Pro Phe Leu Tyr
       50                  55                  60
   Asn Lys Gly Lys Gly Lys Tyr Ile Pro Val Ser Val Leu Val Leu Leu
   65                  70                  75                  80
   Arg Asn Ser Glu Arg Pro Arg His Lys Gln Leu Pro Glu Ile Arg Ala
                   85                  90                  95
   Leu Glu Asn Pro Glu Glu Asp Phe Asp Ile Asp Ala Val Cys Pro Glu
               100                 105                 110
   Val Ser Glu Ala Glu Tyr Tyr Leu Asn Pro Ser Gly Tyr Arg Glu Val
           115                 120                 125
   Cys Trp Asn Val Lys Glu Arg Gly Ala Val Gly Glu Thr Ile Leu His
       130                 135                 140
   Leu Cys Leu Leu Asn Ala Thr Ser Leu His Ala Asp Leu Ala Lys Arg
   145                 150                 155                 160
   Leu Leu Arg Phe Tyr Pro Lys Leu Ile Asn Asp Val Tyr Met Cys Asp
                   165                 170                 175
   Glu Tyr Tyr Gly Glu Ser Val Leu His Leu Ala Ile Val Asn Glu Asp
               180                 185                 190
   Pro Ala Met Val Lys Tyr Leu Leu Asp His Asn Ser Asp Val Asn Glu
           195                 200                 205
   Arg Cys Cys Gly Thr Phe Met Cys Pro Glu Asp Gln Lys Ala Ser Arg
       210                 215                 220
   Tyr Asp Thr Pro Glu Thr Glu Val Val Gln Met Val Gln Leu Thr Asn
   225                 230                 235                 240
   Tyr Asp Gly Tyr Val Tyr Trp Gly Glu Tyr Pro Leu Thr Phe Ala Ala
                   245                 250                 255
   Cys Leu Gly Gln Glu Glu Cys Tyr Arg Leu Val Leu Ala Arg Gly Ala
               260                 265                 270
   Asp Pro Asp Asn Lys Asp Phe Asn Gly Asn Thr Val Leu His Met Leu
           275                 280                 285
   Val Ile Tyr Glu Lys Ile Thr Thr Phe Asp Met Gly Tyr Glu Val Gly
       290                 295                 300
   Ser Ser Leu Ser Ile Arg Asn His Gln Asn Leu Thr Pro Leu Thr Leu
   305                 310                 315                 320
   Ala Ala Lys Leu Gly Arg Val Glu Met Phe Phe His Ile Met Asn Ile
                   325                 330                 335
   Glu Arg Glu Ile Tyr Trp Gln Leu Gly Ser Ile Thr Cys Ala Ala Tyr
               340                 345                 350
   Pro Leu Val Leu Ile Asp Thr Ile Asp Val Glu Thr Gly Asn Ile Ser
           355                 360                 365
   Lys Asp Ser Ala Leu Asn Leu Val Val Phe Gly Asp Lys Asp Glu His
       370                 375                 380
   Leu Asp Leu Leu Asp Gly Val Leu Ile Asp Leu Leu Lys Thr Lys Trp
   385                 390                 395                 400
   Asn Thr Phe Val Lys Asp Lys Phe Tyr Arg Gln Phe Phe Met Phe Phe
                   405                 410                 415
   Cys Tyr Phe Cys Val Ser Leu Ile Gly Phe Thr Leu Arg Asn Gly Pro
               420                 425                 430
   Pro Pro Pro Glu Glu Asp Asp Glu Asn Arg Ala Gly Gly Gly Ser Gly
           435                 440                 445
   Asn Lys Thr Ser Ser Ala Ser Lys Gln Asp Leu Arg Gln Arg Leu Trp
       450                 455                 460
   Asn Arg Thr Val Thr Leu Glu Asp Ala Asp Asn Val Ser Ser Leu Leu
   465                 470                 475                 480
   Ser Asp Ile Met Gly Ile Asp Ala Leu Gly Lys Leu Ala Glu Gln Leu
                   485                 490                 495
   Leu Asn Gly Ser Glu Gly Ser Gly Ala Phe Lys Pro Glu Thr Val Glu
               500                 505                 510
   Glu Glu Glu Asp Gly Leu Phe Phe Asn Ser Lys Cys His Thr Met Asp
           515                 520                 525
   Tyr Asp Gly Val Glu Gly Lys Val Arg Phe Val Ala Glu Val Ile Ile
       530                 535                 540
   Leu Ile Gly Ser Phe Leu Tyr Leu Leu Ser Ala Leu Arg Glu Leu Lys
   545 550 555 560
   Phe Leu Gly Arg Lys Met Phe Phe Glu Asn Leu Val Ser Arg Gly Phe
   565 570 575
   Lys Thr Val Gly Pro Phe Val Val Met Ile Tyr Arg Met Val Met Gly
   580 585 590
   Asp Leu Leu Arg Phe Val Val Ile Tyr Leu Val Phe Val Met Gly Phe
   595 600 605
   Ser Gln Ala Tyr Tyr Ile Val Phe Leu Ser Tyr Lys Pro Glu Glu Asp
   610 615 620
   Gly Asp Ala Asn Pro Met Pro Ser Pro Ile Glu Ser Ile Val Ala Met
   625 630 635 640
   Phe Leu Met Ser Leu Thr Asn Phe Gly Asp Tyr Tyr Gly Ala Leu Glu
   645 650 655
   Asn Thr Asp His Glu Met Cys Ala Lys Ile Leu Phe Val Leu Phe Met
   660 665 670
   Val Ile Val Ala Val Leu Leu Val Asn Met Leu Ile Ala Met Met Gly
   675 680 685
   Asn Thr Tyr Gln Lys Ile Ala Glu Thr Lys Asn Glu Trp Gln Arg Gln
   690 695 700
   Trp Ala Arg Ile Val Leu Val Val Glu Arg Gly Val Pro Pro Lys Glu
   705 710 715 720
   Arg Leu Lys Asn Leu Met Ser Tyr Ser Gln Pro Met Ser Asp Gly Arg
   725 730 735
   Arg Ala Leu Val Leu Arg Leu Asn Met Thr Asp Glu Asp Lys Glu Glu
   740 745 750
   Met Lys Glu Ile Leu Glu Met Lys Arg Val His Glu Arg Leu Ser Lys
   755 760 765
   Lys Arg Gln Val Glu Arg Asp Ala Arg Ala Glu Gln Arg Arg Leu Leu
   770 775 780
   Arg Glu Lys Tyr Leu Asn Met Thr
   785 790
<210> 64
   <211> 900
   <212> PRT
   <213> Acyrthosiphon pisum
<400> 64
   Met Gly Asn Thr Glu Ser Asn Val Ala Ser Gly Val Lys Lys Gln Val
   1 5 10 15
   Asp Thr Ser Ser Leu Gln Ile Tyr Asn Leu Val Asp Leu Lys Gly Cys
   20 25 30
   Gly Leu Leu Val Asp Leu Met Lys Lys Ala Val Gln Thr Lys Asn Phe
   35 40 45
   Thr Glu Leu Asp Asn Ala Ile Lys Thr Gln Val Glu Pro Met Leu Tyr
   50 55 60
   Asn Lys Gly Glu Gly Arg Met Ile Pro Ile Ala Lys Leu Val Leu Leu
   65 70 75 80
   Arg Asn Arg Asp Arg Ser Arg Ala Arg Trp Leu Pro Pro Leu Lys Asn
   85 90 95
   Met Glu Asp Thr Asn Asp Tyr Asp Ile Glu Lys Asp Ile Pro Thr Ile
   100 105 110
   Glu Glu Leu Glu Lys Leu Gly Glu Val Gln Tyr Arg Glu Val Cys Trp
   115 120 125
   Asp Leu Lys Glu Arg Gly Thr Val Gly Glu Thr Ala Leu His Leu Cys
   130 135 140
   Leu Leu Asn Ala Thr Ser Ile His Ala Asp Leu Ala Lys Arg Leu Leu
   145 150 155 160
   His Phe Tyr Pro Lys Leu Val Asn Asp Ile Tyr Met Ile Asp Glu Tyr
   165 170 175
   Tyr Gly Glu Ser Val Leu His Met Ala Ile Val Asn Glu Asp Pro Ala
   180 185 190
   Met Val Lys Val Leu Met Asp Ser Gly Ala Asn Leu Asn Glu Arg Cys
   195 200 205
   Phe Gly Asn Phe Met Ser Thr Glu Asp Gln Lys Ala Ser Arg Ser Asp
   210 215 220
   Ser Leu Asp His Glu Trp Val Asn Leu Cys Pro Asp Thr Asn Tyr Glu
   225 230 235 240
   Gly Tyr Val Tyr Trp Gly Glu Tyr Pro Leu Ser Phe Ala Ala Cys Leu
   245 250 255
   Gly Gln Glu Glu Ser Tyr Arg Leu Met Leu Ala Arg Gly Ala Asp Pro
   260 265 270
   Asn Asn Gln Asp Thr Asn Gly Asn Thr Val Leu His Met Leu Val Ile
   275 280 285
   Tyr Glu Lys Ile Ser Thr Phe Asp Met Ala Tyr Glu Ile Gly Ala Glu
   290 295 300
   Leu Asn Ile Arg Asn Val Gln Asn Leu Thr Pro Leu Thr Leu Ala Ala
   305 310 315 320
   Lys Leu Ala Arg Ile Gln Met Phe Phe His Ile Leu Lys Ile Glu Arg
   325 330 335
   Glu Ile Tyr Trp Gln Ile Gly Ser Ile Thr Cys Ala Ala Tyr Pro Leu
   340 345 350
   Thr Gln Ile Asp Thr Ile Asp Asn Asp Thr Gly Gln Ile Ser Arg Asn
   355 360 365
   Ser Ala Leu Asn Leu Val Val Phe Gly Glu Ser Pro Glu His Leu Glu
   370 375 380
   Met Met Asp Gly Val Leu Val Asp Leu Leu Asn Ala Lys Trp Asn Ala
   385 390 395 400
   Phe Val Lys Phe Arg Phe Tyr Arg Gln Phe Ile Leu Phe Ala Phe Tyr
   405 410 415
   Phe Ser Ile Ser Met Val Cys Phe Val Leu Arg Pro Gly Pro Pro Pro
   420 425 430
   Thr Gly Leu Lys Pro Pro Leu Ile Asn Tyr Thr Ser Thr Ser Thr Thr
   435 440 445
   Val Ala Thr Thr Pro Phe Asp Phe Met Asp Asn Glu Ser Met Leu Pro
   450 455 460
   Asp Met Tyr Asp Glu Met Met Ile Gly Gly Gly Gly Pro Thr Glu Phe
   465 470 475 480
   Glu Thr Val Gln Val Thr Thr Glu Met Ala Thr Thr Ser Asp Asn Ala
   485 490 495
   Thr Thr Thr Asn Asn Gly Ser Gly Ile Gly Thr Pro Leu Ile Tyr Glu
   500 505 510
   Asp Gly Gly Gly Gly Gly Gly Gly Gly Gly Thr Val Ser Leu His Gly
   515 520 525
   Lys Ser Ser Gly Ser Asp Arg Gly Lys His Arg Ser Thr Trp Ser Pro
   530 535 540
   Ala Asn His Thr Phe Lys Thr Asn Tyr Thr Tyr Asn Tyr Asn Lys Ser
   545 550 555 560
   Arg Lys Phe Trp Trp Asn Lys Arg Gln Lys Val Cys Arg Leu Met Ala
   565 570 575
   Val His Ser Thr Asp Asp Leu Ile Arg Met Gly Ser Glu Val Gly Met
   580 585 590
   Phe Met Gly Ala Phe Leu Tyr Leu Leu Ala Ala Val Arg Glu Ala Gly
   595 600 605
   Phe Leu Gly Thr Gln Met Phe Ile Glu Asn Leu Ala Ile Ala Pro Ala
   610 615 620
   Arg Val Met Phe Leu Phe Ser Cys Leu Leu Met Met Thr Ile Pro Pro
   625 630 635 640
   Leu Arg Leu Thr Cys Tyr Asp Lys Ala Glu Asp Ile Ile Ala Val Ile
   645 650 655
   Ile Met Leu Thr Thr Cys Pro Tyr Phe Leu Phe Phe Cys Arg Gly Phe
   660 665 670
   Lys Thr Val Gly Pro Phe Val Val Met Ile Tyr Arg Met Val Met Gly
   675 680 685
   Asp Leu Leu Arg Phe Val Ser Ile Tyr Met Val Phe Val Met Gly Phe
   690 695 700
   Ser Gln Ala Tyr Tyr Val Ile Phe Leu Ser Tyr Asp Asn Pro Leu Thr
   705 710 715 720
   Pro Glu Gly Ile Asp Asp Ser Val Leu Asn Pro Met Pro Thr Pro Thr
   725 730 735
   Glu Ser Ile Met Ala Met Phe Leu Met Ser Val Asn Thr Phe Thr Asp
   740 745 750
   Tyr Tyr Thr Ala Phe Asp Lys Thr Ser His Thr Leu Val Ala Lys Phe
   755 760 765
   Cys Phe Ile Val Phe Met Val Ile Val Ala Ile Leu Leu Val Asn Met
   770 775 780
   Leu Ile Ala Met Met Gly Asn Thr Tyr Gln Lys Ile Ala Glu Thr Arg
   785 790 795 800
   Asn Glu Trp Gln Arg Gln Trp Ala Arg Ile Val Leu Val Val Glu Arg
   805 810 815
   Gly Val Ser Pro Ser Gln Arg Leu Ile Lys Leu Met Tyr Tyr Ser Gln
   820 825 830
   Pro Met Ser Asp Gly Arg Arg Ala Leu Val Leu Arg Leu Asn Gln Thr
   835 840 845
   Asp Glu Asp Lys Glu Gln Met Lys Glu Ile Leu Glu Met Lys Arg Ile
   850 855 860
   His Asn Arg Tyr Val Glu Arg Met Arg Ala Arg Asp Leu Ser Ser Lys
   865 870 875 880
   Leu Ser Ser Ser Cys Gly Lys Asn Ala Asn Asn Gly Thr Phe Asn Leu
   885 890 895
   Lys Ala Asn Glu
   900
<210> 65
   <211> 707
   <212> PRT
   <213> Dendroctonus ponderosae
<400> 65
   Met Gly Asn Thr Glu Ser Asn Val Thr Ser Gly Val Lys Lys Gln Ala
   1 5 10 15
   Gly Ala Ala Met Gln Pro Ile Tyr Lys Leu Cys Asp Leu Lys Gly Gly
   20 25 30
   Gly Leu Leu Val Glu Leu Met Lys Arg Ala Ala Gln Asn Lys Gln Tyr
   35 40 45
   Ala Glu Ile Asp His Ala Ile Lys Thr Lys Val Glu His Phe Leu Tyr
       50                  55                  60
   Asn Lys Gly Ala Gly Arg Tyr Ile Pro Ile Ser Gln Met Val Leu Leu
   65                  70                  75                  80
   Arg Asn Lys Glu Arg Gly Lys His Lys Leu Leu Pro Gln Leu Arg Gly
                   85                  90                  95
   Met Glu Asn Pro Glu Asp Phe Glu Val Asp Asp Glu Gly Pro Glu Ile
               100                 105                 110
   Thr Glu Glu Glu Tyr Asn Lys Asp Pro Ser Leu Tyr Arg His Val Cys
           115                 120                 125
   Trp Lys Leu Arg Glu Arg Gly Ala Val Gly Glu Thr Ile Leu His Leu
       130                 135                 140
   Cys Leu Leu Asn Ala Thr Ser Leu His Ala Asp Ile Ala Lys Arg Leu
   145                 150                 155                 160
   Leu Arg Phe Tyr Pro Lys Leu Ile Asn Asp Ile Tyr Ile Ser Asp Glu
                   165                 170                 175
   Tyr Tyr Gly Glu Asn Val Leu His Ile Ala Ile Val Asn Glu Asp Pro
               180                 185                 190
   Ser Met Val Lys Phe Leu Leu Ala Ala Asn Val Asn Phe Gln Glu Arg
           195                 200                 205
   Cys Phe Gly Asn Phe Met Cys Pro Glu Asp Gln Lys Ser Ser Arg Thr
       210                 215                 220
   Asp Ser Leu Asp His Glu Trp Val Asn Val Tyr His Glu Thr Asn Tyr
   225                 230                 235                 240
   Asp Gly Tyr Val Tyr Trp Gly Glu Tyr Pro Leu Ser Phe Ala Ala Cys
                   245                 250                 255
   Leu Gly Gln Glu Glu Ser Phe Arg Leu Ile Leu Ala Lys Gly Ala Asn
               260                 265                 270
   Leu Asp Ala Gln Asp Thr Asn Gly Asn Thr Val Leu His Leu Leu Val
           275                 280                 285
   Ile Tyr Ser Lys Val Glu Ala Phe Asp Met Ala Tyr Glu Leu Gly Ala
       290                 295                 300
   His Leu Ser Ile Arg Asn Ile Leu Lys Met Thr Pro Leu Thr Leu Ala
   305 310 315 320
   Ala Lys Leu Ala Arg Met Asp Ile Phe Phe His Ile Leu Lys Leu Glu
   325 330 335
   Arg Glu Ile Tyr Trp Gln Ile Gly Ser Ile Thr Cys Ala Ala Tyr Pro
   340 345 350
   Leu Ser Gln Ile Asp Thr Ile Asp Val Lys Thr Gly Gln Ile Ser Lys
   355 360 365
   Met Ser Ala Leu Asn Leu Val Val Phe Gly Asp Lys Asp Glu His Leu
   370 375 380
   Glu Leu Leu Asp Gly Val Leu Val Asp Leu Leu Asn Ala Lys Trp Asn
   385 390 395 400
   Thr Phe Val Lys Ser Lys Phe Tyr Arg Gln Phe Phe Thr Phe Ala Phe
   405 410 415
   Tyr Phe Thr Val Ser Leu Val Asp Thr Ser Asn Asp Asp Tyr Asp Val
   420 425 430
   Glu Glu Trp Trp Asp Asn Leu Arg Glu Glu Cys Arg Leu Met Asn Leu
   435 440 445
   Asp Thr Thr Glu Ser Leu Val Arg Phe Thr Ala Glu Leu Gly Ile Thr
   450 455 460
   Phe Gly Ala Phe Leu Tyr Leu Ala Ala Ala Met Thr Ala Pro Ser Arg
   465 470 475 480
   Val Met Phe Leu Phe Ser Cys Ile Leu Val Phe Gly Val Ile Pro Ser
   485 490 495
   Leu Arg Leu Val Cys Met Asp Glu Val Glu Asp Ile Ile Ala Val Val
   500 505 510
   Val Met Leu Thr Thr Ala Pro Tyr Phe Leu Phe Phe Cys Arg Gly Phe
   515 520 525
   Lys Thr Val Gly Pro Phe Val Val Met Ile Tyr Arg Met Val Leu Gly
   530 535 540
   Asp Leu Leu Arg Phe Ala Thr Ile Tyr Met Val Phe Val Met Gly Phe
   545 550 555 560
   Ser Gln Ala Tyr Tyr Ile Ile Phe Leu Ser Phe Asp Asn Pro Leu Thr
   565 570 575
   Pro Glu Asp Val Asp Asp Ser Ala Thr Asn Pro Met Ser Thr Pro Ser
   580 585 590
   Glu Ser Ile Met Ala Met Phe Leu Met Ser Met Thr Asn Phe Gly Asp
   595 600 605
   Tyr Tyr Thr Ala Phe Ala Arg Thr Glu His Glu Tyr Glu Ala Lys Ile
   610 615 620
   Leu Phe Val Ile Phe Met Ala Ile Val Ala Ile Leu Leu Ile Asn Met
   625 630 635 640
   Leu Ile Ala Met Met Gly Asn Thr Tyr Gln Lys Ile Ala Glu Thr Arg
   645 650 655
   Asn Glu Trp Gln Arg Gln Trp Ala Arg Ile Val Leu Val Val Glu Arg
   660 665 670
   Gly Val Ser Pro Pro Glu Arg Leu Lys Gln Leu Asn Val Tyr Ser Gln
   675 680 685
   Pro Met Ser Asp Gly Lys Arg Ala Leu Val Leu Arg Leu Asn Gln Ser
   690 695 700
   Val Arg Tyr
   705
<210> 66
   <211> 905
   <212> PRT
   <213> Harpegnathos saltator
<400> 66
   Met Gly Asn Thr Glu Ser Asn Val Thr Ser Gly Val Lys Lys Gln Thr
   1 5 10 15
   Asp Thr Ser Ser Ile Met Leu Tyr Lys Leu Val Asp Leu Lys Gly Gly
   20 25 30
   Gly Leu Phe Val Asp Met Met Lys Arg Ala Ala Gln Thr Lys Gln Phe
   35 40 45
   Ala Glu Leu Asp His Ala Val Arg Thr Lys Val Glu Pro Tyr Leu Tyr
   50 55 60
   Asn Lys Gly Lys Gly Lys Trp Ile Pro Val Asp Lys Leu Val Leu Leu
   65 70 75 80
   Arg Asn Lys Asp Arg Pro Arg His Lys Met Leu Ala Pro Leu Lys Ala
   85 90 95
   Met Glu Asn Pro Ala Asp Tyr Asp Ile Asp Lys Asp Met Gly Asp Glu
   100 105 110
   Asp Val Asp Glu Thr Lys Ile Asp Lys Ser Lys Tyr Arg Leu Val Cys
   115 120 125
   Trp Ser Leu Ser Glu Arg Gly Ala Val Gly Glu Thr Ile Leu His Leu
   130 135 140
   Cys Met Leu Asn Ala Thr Ala Leu His Ala Asp Leu Ala Lys Arg Leu
   145 150 155 160
   Leu Arg Phe Tyr Pro Asn Leu Ile Asn Asp Ile Tyr Ile Ser Asp Glu
   165 170 175
   Tyr Tyr Gly Glu Asn Val Leu His Ile Ala Ile Val Asn Glu Asp Pro
   180 185 190
   Ala Met Val Lys Tyr Leu Leu Asp Ser Gly Ala Asp Val His Glu Arg
   195 200 205
   Cys Phe Gly Asn Phe Met Cys Pro Glu Asp Gln Lys Ala Ser Arg Val
   210 215 220
   Asp Ser Leu Asp His Glu Trp Val Cys Val Ala Pro Glu Thr Asn Tyr
   225 230 235 240
   Ser Gly Tyr Val Tyr Trp Gly Glu Tyr Pro Leu Ser Phe Ala Ala Cys
   245 250 255
   Leu Gly Gln Glu Glu Cys Tyr Arg Leu Met Leu Ala Arg Gly Ala Asp
   260 265 270
   Pro Asp Lys Gln Asp Thr Asn Gly Asn Thr Val Leu His Met Leu Val
   275 280 285
   Ile Tyr Glu Lys Leu Thr Thr Phe Asp Met Ala Tyr Glu Val Gly Ala
   290 295 300
   Ser Leu Ala Ile Arg Asn Val Gln His Leu Thr Pro Leu Thr Leu Ser
   305 310 315 320
   Ala Lys Leu Ala Lys Ile Glu Met Phe Phe His Ile Leu Asn Ile Glu
   325 330 335
   Arg Glu Ile Tyr Trp Gln Ile Gly Ser Ile Thr Cys Ala Ala Tyr Pro
   340 345 350
   Leu Ser Gln Ile Asp Thr Ile Asp Ile Asn Ile Gly Thr Ile Asn Lys
   355 360 365
   Asn Ser Ala Leu Asn Leu Val Val Phe Gly Glu Lys Asp Glu His Leu
   370 375 380
   Glu Leu Met Asp Gly Met Leu Val Asp Leu Leu Asn Ala Lys Trp Asn
   385 390 395 400
   Thr Phe Val Lys Ser Arg Phe Tyr Arg Gln Phe Phe Leu Phe Cys Phe
   405 410 415
   Tyr Phe Val Leu Ser Leu Ile Ser Phe Thr Leu Arg Pro Gly Pro Leu
   420 425 430
   Pro Gln Glu Thr Glu Asn Ala Asn Asp Thr Arg Thr Asn Ser Ser Asn
   435 440 445
   Ala Thr Ala Met Glu Ile Leu Arg Leu Phe Gln Ser Ser Asp Leu Ser
   450 455 460
   Asn Leu Val Thr Asn Ser Leu Ala Ala Ala Phe Ala Ser Asn Leu Lys
   465 470 475 480
   Ser Ser Leu Asn Val Thr Gln Glu Ser Leu Glu Lys Ile Gln Leu Gln
   485 490 495
   Val Thr Ser Asn Ile Thr Ser Ala Leu Lys Asn Ile Leu Pro Ser Leu
   500 505 510
   Ser Asp Asn Glu Gly Ile Leu Ser Ala Pro Asp Glu Ala Ala Ile Arg
   515 520 525
   Thr Leu Trp Tyr Thr Ser Ser Pro Tyr Asn Glu Ser Asp Tyr Leu Thr
   530 535 540
   Asp Ala Tyr Asn Glu Asn Val Thr Ser Asp Ala Asn Ser Thr Ala Gly
   545 550 555 560
   Thr Ile Ile Gly Ser Ile Glu Val Ser Lys Ser Asn Asp Lys Asp Asn
   565 570 575
   Trp Trp Glu Asp Leu Thr Glu Glu Cys Arg Leu Met Gln Leu Thr Thr
   580 585 590
   Ile Ser Ala Lys Ile Arg Leu Thr Ala Glu Val Leu Met Glu Ile Ala
   595 600 605
   Ala Val Leu Tyr Ile Leu Ala Ala Leu Arg Glu Ala Arg Phe Leu Gly
   610 615 620
   Leu Asn Met Phe Val Glu Asn Leu Met Thr Ala Pro Ser Arg Val Met
   625 630 635 640
   Phe Leu Phe Ser Cys Cys Ile Leu Leu Ser Phe Pro Phe Leu Arg Leu
   645 650 655
   Ala Cys Ala Asp Glu Val Glu Asp Met Leu Ala Val Val Val Met Leu
   660 665 670
   Thr Thr Ala Pro Tyr Phe Leu Phe Phe Cys Arg Gly Phe Lys Thr Val
   675 680 685
   Gly Pro Phe Val Val Met Ile Tyr Arg Met Ile Met Gly Asp Leu Leu
   690 695 700
   Arg Phe Val Ser Ile Tyr Leu Val Phe Val Met Gly Phe Ser Gln Ala
   705 710 715 720
   Tyr Tyr Ile Ile Phe Leu Ser Phe Asp Asn Pro Asn Thr Pro Glu Gly
   725 730 735
   Val Asp Asp Ser Val Ser Asn Pro Met Pro Ser Pro Met Glu Ser Ile
   740 745 750
   Met Ala Met Phe Leu Met Ser Met Thr Asn Phe Gly Asp Tyr Tyr Gly
   755 760 765
   Ala Phe Glu Arg Thr Glu His Glu Ser Glu Ala Lys Leu Leu Phe Val
   770 775 780
   Leu Tyr Met Ala Ile Val Ala Ile Leu Leu Val Asn Met Leu Ile Ala
   785 790 795 800
   Met Met Gly Asn Thr Tyr Gln Lys Ile Ala Glu Thr Arg Asn Glu Trp
   805 810 815
   Gln Arg Gln Trp Ala Arg Ile Val Leu Val Val Glu Arg Gly Val Ser
               820                 825                 830
   Pro Ala Glu Arg Leu Lys Lys Leu Met Asp Tyr Ser Gln Pro Met Ser
           835                 840                 845
   Gly Gly Arg Arg Ala Leu Val Leu Arg Leu Asn Gln Ser Glu Glu Asp
       850                 855                 860
   Lys Glu Glu Met Lys Glu Ile Leu Glu Met Lys Arg Thr His Asp Arg
   865                 870                 875                 880
   Leu Leu Lys Lys Arg Gln Gly Arg Met Ser Lys Glu Lys Val Leu Ala
                   885                 890                 895
   Glu Ser Glu Ile Ile Ile Thr Arg Asn
               900                 905
<210> 67
   <211> 903
   <212> PRT
   <213> Nasonia vitripennis
<400> 67
   Met Gly Asn Ala Glu Ser Asn Val Thr Ser Gly Val Lys Lys Gln Thr
   1               5                   10                  15
   Asp Ala Gly Ala Ile Ala Leu Tyr Lys Leu Val Asp Leu Lys Gly Gly
               20                  25                  30
   Gly Leu Leu Val Asp Met Met Lys Arg Ala Ser Gln Thr Lys Gln Tyr
           35                  40                  45
   Ala Glu Ile Asp His Ala Ile Arg Thr Lys Val Glu Pro Phe Leu Tyr
       50                  55                  60
   Asn Gly Gly Lys Gly Lys Trp Ile Pro Ile Ala Lys Leu Val Leu Leu
   65                  70                  75                  80
   Arg Asn Lys Glu Arg Gly Arg His Lys Met Leu Pro Val Leu Lys Ala
                   85                  90                  95
   Met Glu Lys Pro Glu Asp Tyr Asp Ile Asp Lys Asp Met Ala Asn Asp
               100                 105                 110
   Pro Glu Pro Asp Glu Asn Thr Ile Asp Lys Ser Lys Tyr Lys Leu Val
           115                 120                 125
   Cys Trp Thr Leu Ser Asp Arg Gly Ala Val Gly Glu Thr Ile Leu His
   130 135 140
   Leu Cys Met Leu Asn Ala Thr Gln Leu His Ala Asp Leu Ala Lys Arg
   145 150 155 160
   Leu Leu Arg Phe Tyr Pro Asn Leu Ile Asn Asp Ile Tyr Ile Asp Asp
   165 170 175
   Glu Tyr Tyr Gly Glu Asn Val Leu His Ile Ala Ile Val Asn Glu Asp
   180 185 190
   Pro Ser Met Val Lys Phe Leu Leu Asp Ser Gly Ala Asn Val Asn Glu
   195 200 205
   Arg Cys Cys Gly Asn Phe Met Cys Pro Glu Asp Gln Lys Ala Ser Arg
   210 215 220
   Asn Asp Ser Val Glu His Glu Trp Val Cys Val Cys Ser Glu Thr Asn
   225 230 235 240
   Tyr Asp Gly Tyr Val Tyr Trp Gly Glu Tyr Pro Leu Ser Phe Ala Ala
   245 250 255
   Cys Leu Asp Gln Glu Glu Cys Tyr Arg Leu Ile Leu Ala Lys Gly Ala
   260 265 270
   Asp Pro Asp Ser Gln Asp Thr Asn Gly Asn Thr Val Leu His Met Leu
   275 280 285
   Val Ile Tyr Glu Lys Leu Glu Thr Phe Asp Met Ala Tyr Glu Val Gly
   290 295 300
   Ser Ser Leu Ser Ile Arg Asn Val Leu Gln Leu Thr Pro Leu Thr Leu
   305 310 315 320
   Ala Ala Lys Leu Ala Arg Val Glu Met Phe Phe His Ile Leu Asn Ile
   325 330 335
   Glu Arg Glu Ile Tyr Trp Gln Ile Gly Ser Ile Thr Cys Ala Ala Tyr
   340 345 350
   Pro Leu Ser Gln Ile Asp Thr Ile Asp Val Asp Thr Gly Lys Ile Ser
   355 360 365
   Asn Asn Ser Ala Leu Asn Leu Val Val Phe Gly Asp Lys Glu Glu His
   370 375 380
   Leu Lys Leu Leu Glu Gly Val Leu Ile Asp Leu Leu Asn Ala Lys Trp
   385                 390                 395                 400
   Asn Thr Phe Val Lys Ser Arg Phe Tyr Gln Gln Phe Tyr Leu Phe Phe
                   405                 410                 415
   Cys Tyr Phe Ile Leu Ser Leu Ile Ser Cys Thr Leu Arg Pro Gly Pro
               420                 425                 430
   Ile Thr Lys Thr Asp Ala Pro Thr Thr Thr Pro His Pro Met Asn Asp
           435                 440                 445
   Thr Ser Ile Glu Ser Ile Thr Pro Thr Ser Glu Pro Leu Glu Leu Gln
       450                 455                 460
   Thr Leu Ser Ser Leu Leu Ala Lys Asn Leu Ala Glu Gln Leu Val Met
   465                 470                 475                 480
   Gly Met Lys Met Tyr Gly Asn Val Ser Glu Asp Ser Leu Ala Arg Ile
                   485                 490                 495
   Ser Leu Gln Val Ala Ser Asn Ile Ser Gly Ser Ile Lys Asp Ile Trp
               500                 505                 510
   Thr Arg Gln Asn Leu Thr Ser Asn Asp Thr Asp Tyr Val Asp Ile Ala
           515                 520                 525
   Gly Gly Leu Tyr Ala Asp Val Asn Glu Thr Ser Ala Ser Asp Pro Phe
       530                 535                 540
   Asp Leu Asn Ala Ser Asp Ile Val Ile Leu Lys Lys Gly Lys Lys Glu
   545                 550                 555                 560
   Leu Asp Asp Trp Trp Asp Asp Leu Thr Glu Asp Cys Arg Leu Met Gln
                   565                 570                 575
   Met Asn Ser Thr Ser Ala Lys Ile Arg Leu Thr Ala Glu Ile Phe Met
               580                 585                 590
   Glu Phe Gly Ala Ile Leu Tyr Ile Cys Ala Ala Leu Arg Glu Ala Arg
           595                 600                 605
   Phe Leu Gly Leu Asn Met Phe Ile Glu Asn Leu Met Thr Ala Pro Ser
       610                 615                 620
   Arg Val Met Phe Leu Phe Ser Cys Cys Ile Leu Leu Ser Phe Pro Phe
   625                 630                 635                 640
   Leu Arg Met Ser Cys Ala Asp Glu Val Glu Asp Ile Leu Ala Val Val
   645 650 655
   Val Met Leu Thr Thr Ala Pro Tyr Phe Leu Phe Phe Cys Arg Gly Phe
   660 665 670
   Lys Thr Val Gly Pro Phe Val Val Met Ile Tyr Arg Met Ile Met Gly
   675 680 685
   Asp Leu Leu Arg Phe Val Ser Ile Tyr Leu Val Phe Val Met Gly Phe
   690 695 700
   Ser Gln Ala Tyr Tyr Ile Ile Phe Leu Ser Phe Asp Asn Pro Ile Thr
   705 710 715 720
   Pro Glu Gly Val Asp Asp Ser Lys Ala Asn Pro Leu Pro Ser Ala Met
   725 730 735
   Glu Ser Ile Met Ala Met Phe Leu Met Ser Met Thr Asn Phe Gly Asp
   740 745 750
   Tyr Tyr Asp Ala Phe Glu Asn Thr Glu His Glu Met Leu Ala Lys Cys
   755 760 765
   Leu Phe Val Val Tyr Met Ala Ile Val Ser Val Leu Leu Val Asn Met
   770 775 780
   Leu Ile Ala Met Met Gly Asn Thr Tyr Gln Lys Ile Ala Glu Thr Arg
   785 790 795 800
   Asn Glu Trp Gln Arg Gln Trp Ala Arg Ile Val Leu Val Val Glu Arg
   805 810 815
   Gly Val Ala Pro Ala Glu Arg Leu Lys Lys Leu Asn Val Tyr Ser Gln
   820 825 830
   Pro Met Ser Asp Gly Arg Lys Ala Leu Val Leu Arg Leu Asn Gln Thr
   835 840 845
   Asp Lys Asp Lys Glu Glu Met Lys Asp Ile Leu Glu Met Lys Arg Ile
   850 855 860
   His Ile Lys Ser Ile Lys Arg Arg Lys Asp Lys Leu Gln Lys Ala Lys
   865 870 875 880
   Glu Asp Ala Glu Lys Leu Gln Gln Gln Met Ala Asp Lys Asp Asn Lys
   885 890 895
   Asp Lys Ala Val Ile Ile Gln
   900
<210> 68
   <211> 903
   <212> PRT
   <213> Megachile rotundata
<400> 68
   Met Gly Asn Thr Glu Ser Asn Val Thr Ser Gly Val Lys Lys Gln Thr
   1 5 10 15
   Asp Ser Ser Ser Ile Leu Leu Tyr Lys Leu Val Asp Leu Lys Gly Gly
   20 25 30
   Gly Leu Leu Val Asp Met Met Lys Arg Ala Thr Gln Thr Lys Gln Tyr
   35 40 45
   Ala Glu Leu Asp His Ala Leu Arg Thr Lys Val Glu Pro Tyr Leu Tyr
   50 55 60
   Asn Lys Gly Lys Gly Lys Trp Ile Pro Ile Glu Lys Leu Val Leu Leu
   65 70 75 80
   Arg Asn Lys Asp Arg Pro Lys His Lys Met Leu Pro Pro Leu Arg Ala
   85 90 95
   Met Glu Asn Pro Ala Asp Tyr Asp Ile Asp Lys Asp Met Gly Glu Glu
   100 105 110
   Glu Val Asp Glu Thr Lys Ile Asp Lys Ser Lys Tyr Arg Leu Val Cys
   115 120 125
   Trp Ser Leu Ser Gln Arg Gly Ala Val Gly Glu Thr Ile Met His Leu
   130 135 140
   Cys Met Leu His Ala Thr Ala Ile His Thr Asp Leu Ala Lys Arg Leu
   145 150 155 160
   Leu Arg Phe Tyr Pro Lys Leu Ile Asn Asp Ile Tyr Ile Ser Asp Glu
   165 170 175
   Tyr Tyr Gly Glu Asn Ala Leu His Ile Ala Ile Val Asn Glu Asp Pro
   180 185 190
   Ala Met Val Lys Phe Leu Leu Asp Ser Gly Ala Asp Val His Glu Arg
   195 200 205
   Cys Val Gly Asn Phe Met Cys Pro Glu Asp Gln Lys Ala Ser Arg Thr
   210 215 220
   Asp Ser Val Asp His Glu Trp Val Cys Val Ala Pro Glu Thr Asn Tyr
   225 230 235 240
   Ser Gly Tyr Val Tyr Trp Gly Glu Tyr Pro Leu Asn Phe Ala Ala Cys
   245 250 255
   Leu Gly Gln Glu Glu Cys Tyr Arg Leu Ile Leu Ala Arg Gly Ala Asp
   260 265 270
   Pro Asp Lys Gln Asp Thr Asn Gly Asn Thr Val Leu His Met Leu Val
   275 280 285
   Ile Tyr Glu Lys Met Ala Thr Phe Asp Met Ala Tyr Glu Val Gly Ala
   290 295 300
   Ser Leu Ser Ile Arg Asn Ala Gln His Leu Thr Pro Leu Thr Leu Ser
   305 310 315 320
   Ala Lys Leu Ala Arg Ile Glu Met Phe Phe His Ile Leu Asn Ile Glu
   325 330 335
   Arg Glu Ile Tyr Trp Gln Ile Gly Ser Ile Thr Cys Ala Ala Tyr Pro
   340 345 350
   Leu Ser Gln Val Asp Thr Ile Asp Val Asn Thr Gly Ser Ile Ser His
   355 360 365
   Thr Ser Ala Leu Asn Leu Val Val Phe Gly Glu Lys Asp Glu His Leu
   370 375 380
   Glu Leu Met Asp Gly Thr Met Val Asp Leu Leu Asn Ala Lys Trp Asn
   385 390 395 400
   Thr Phe Val Lys Ser Arg Phe Tyr Arg Gln Phe Phe Leu Phe Cys Phe
   405 410 415
   Tyr Phe Ile Leu Tyr Leu Ile Ser Phe Thr Leu Arg Pro Gly Pro Ser
   420 425 430
   Val Thr Ser Thr Ala Thr Thr Ala Pro Thr Thr Glu Ser Thr Thr Pro
   435 440 445
   Lys Leu Pro Asp Pro Pro Pro Asn Phe Ile Pro Val Thr Asn Gln Lys
   450 455 460
   Thr Phe Leu Glu Ala Ser Leu Ser Ser Ser Leu Asp Glu Ile Val Thr
   465                 470                 475                 480
   Asn Ala Leu Asn Leu Asn Phe Pro Ser Asn Ala Thr Ala Arg Phe Asp
                   485                 490                 495
   Asp Leu Lys Leu Asp Leu Val Thr His Ile Met Ser Asn Leu Lys Asn
               500                 505                 510
   Ile Leu Asn Gly Asn Glu Thr Lys Asn Glu Pro Phe Asn Ser Ser Gln
           515                 520                 525
   Leu Ala Arg Phe Asp Lys His Leu Phe Arg Glu Asn Asp Thr Asp Tyr
       530                 535                 540
   Ser Thr Ser Ile Leu Asn Glu Thr Ala Asp Val Asp Ser Thr Thr Lys
   545                 550                 555                 560
   Phe Ser Phe Asp Asn Ser Val Leu Phe Glu Ala Asp Asn Lys Ser Asp
                   565                 570                 575
   Trp Trp Thr Asp Leu Thr Thr Glu Cys Arg Leu Met Gln Leu Thr Thr
               580                 585                 590
   Ala Ser Ala Lys Ile Arg Leu Ile Ala Glu Ile Phe Met Glu Ile Ala
           595                 600                 605
   Ala Ile Leu Tyr Ile Leu Ala Ala Leu Arg Glu Ala Arg Phe Leu Gly
       610                 615                 620
   Leu Asn Met Phe Ile Glu Asn Leu Met Thr Val Pro Ser Arg Val Met
   625                 630                 635                 640
   Phe Leu Phe Ser Cys Cys Ile Leu Leu Ser Phe Pro Phe Leu Arg Leu
                   645                 650                 655
   Ser Cys Ala Asp Glu Val Glu Asp Val Leu Ala Val Val Val Met Leu
               660                 665                 670
   Thr Thr Ala Pro Tyr Phe Leu Phe Phe Cys Arg Gly Phe Lys Thr Val
           675                 680                 685
   Gly Pro Phe Val Val Met Ile Tyr Arg Met Ile Met Gly Asp Leu Leu
       690                 695                 700
   Arg Phe Val Ser Ile Tyr Leu Val Phe Val Ile Gly Phe Ser Gln Ala
   705 710 715 720
   Tyr Tyr Ile Ile Phe Leu Ser Phe Asp Asn Pro Asn Thr Pro Glu Gly
   725 730 735
   Val Asp Asp Ser Val Ser Asn Pro Met Pro Ser Pro Ile Glu Ser Ile
   740 745 750
   Met Ala Met Phe Leu Met Ser Met Thr Asn Phe Gly Asp Tyr Tyr Gly
   755 760 765
   Ala Phe Glu Arg Thr Arg His Glu Met Glu Ala Lys Phe Leu Phe Val
   770 775 780
   Val Tyr Met Ala Ile Val Ala Ile Leu Leu Val Asn Met Leu Ile Ala
   785 790 795 800
   Met Met Gly Asn Thr Tyr Gln Lys Ile Ala Glu Thr Arg Asn Glu Trp
   805 810 815
   Gln Arg Gln Trp Ala Arg Ile Val Leu Val Val Glu Arg Gly Val Ser
   820 825 830
   Pro Gln Glu Arg Leu Lys Lys Leu Met Asp Tyr Ser Gln Pro Met Ser
   835 840 845
   Asp Gly Arg Arg Ala Leu Val Leu Arg Leu Asn Gln Ser Glu Glu Asp
   850 855 860
   Lys Glu Glu Met Lys Glu Ile Leu Glu Met Lys Arg Thr His Glu Arg
   865 870 875 880
   Leu Tyr Lys Lys Arg Gln Leu Lys Thr Thr Lys Glu Lys Thr Thr Ile
   885 890 895
   Thr Glu Glu Asn Val Thr Leu
   900
<210> 69
   <211> 887
   <212> PRT
   <213> Apis mellifera
<400> 69
   Met Gly Asn Thr Glu Ser Asn Val Ala Ser Gly Val Lys Lys Gln Thr
   1               5                   10                  15
   Asp Ala Ser Ser Ile Leu Leu Tyr Lys Leu Val Asp Leu Lys Gly Gly
               20                  25                  30
   Gly Leu Leu Val Asp Met Met Lys Arg Ala Thr Gln Thr Lys Gln Tyr
           35                  40                  45
   Ala Glu Leu Asp His Ala Leu Arg Thr Lys Val Glu Pro Tyr Leu Tyr
       50                  55                  60
   Asn Lys Gly Lys Gly Lys Trp Ile Pro Ile Glu Lys Leu Val Leu Leu
   65                  70                  75                  80
   Arg Asn Lys Asp Arg Pro Lys His Lys Met Leu Pro Pro Leu Arg Ala
                   85                  90                  95
   Met Glu Asn Pro Ala Asp Tyr Asp Ile Asp Lys Asp Met Gly Glu Asp
               100                 105                 110
   Glu Val Asp Glu Thr Lys Ile Asp Lys Ser Lys Tyr Arg Leu Val Cys
           115                 120                 125
   Trp Ser Leu Ser Glu Arg Gly Ala Val Gly Glu Thr Ile Leu His Leu
       130                 135                 140
   Cys Met Leu His Ala Thr Ala Ile His Ile Asp Leu Ala Lys Arg Leu
   145                 150                 155                 160
   Leu Arg Phe Tyr Pro Lys Leu Ile Asn Asp Val Tyr Ile Ser Asp Glu
                   165                 170                 175
   Tyr Tyr Gly Glu Ser Ala Leu His Ile Ala Ile Val Asn Glu Asp Pro
               180                 185                 190
   Ser Met Val Lys Phe Leu Leu Asp Ser Gly Ala Asp Val His Glu Arg
           195                 200                 205
   Cys Ile Gly Asn Phe Met Cys Pro Glu Asp Gln Lys Ala Ser Arg Ala
       210                 215                 220
   Asp Ser Leu Asp His Glu Trp Val Cys Val Thr Pro Glu Thr Asn Tyr
   225                 230                 235                 240
   Asn Gly Tyr Val Tyr Trp Gly Glu Tyr Pro Leu Asn Phe Ala Ala Cys
                   245                 250                 255
   Leu Gly Gln Glu Glu Cys Tyr Arg Leu Ile Leu Ala Arg Gly Ala Asp
               260                 265                 270
   Pro Asp Lys Gln Asp Thr Asn Gly Asn Thr Val Leu His Met Leu Val
           275                 280                 285
   Ile Tyr Glu Lys Leu Ala Thr Phe Asp Met Ala Tyr Glu Val Gly Ala
       290                 295                 300
   Ser Leu Ala Ile Arg Asn Ala Gln His Leu Thr Pro Leu Thr Leu Ser
   305                 310                 315                 320
   Ala Lys Leu Ala Lys Ile Glu Met Phe Phe His Ile Leu Asn Ile Glu
                   325                 330                 335
   Arg Glu Ile Tyr Trp Gln Ile Gly Ser Ile Thr Cys Ala Ala Tyr Pro
               340                 345                 350
   Leu Ser Gln Val Asp Thr Ile Asp Val Asp Thr Gly Ser Ile Ser His
           355                 360                 365
   Asn Ser Ala Leu Asn Leu Val Val Phe Gly Glu Lys Asp Glu His Leu
       370                 375                 380
   Glu Leu Met Asp Gly Ile Leu Val Asp Leu Leu Asn Ala Lys Trp Asn
   385                 390                 395                 400
   Thr Phe Val Lys Phe Arg Phe Tyr Arg Gln Phe Phe Leu Phe Cys Phe
                   405                 410                 415
   Tyr Phe Val Leu Ser Leu Ile Ser Phe Thr Leu Arg Pro Gly Pro Ala
               420                 425                 430
   Thr Thr Ser Ser Val Ser Asn Pro Gln Ile Thr Ser Thr Glu Leu Pro
           435                 440                 445
   Thr Ile Pro Pro Lys Met Asp Pro Pro Val His Asn Ser Asp Leu Pro
       450                 455                 460
   Leu Leu Leu Asp Lys Ile Leu Ser Thr Ala Leu Val Ser Lys Arg Tyr
   465                 470                 475                 480
   Pro Trp Asn Leu Thr Arg Thr Arg Leu Asp Lys Leu Lys Leu Asp Ile
                   485                 490                 495
   Val Glu Asn Leu Thr Ser Ala Leu Ser Asp Met Leu Gly Thr Tyr Arg
               500                 505                 510
   Asn Glu Ile Lys Leu Phe Asn Arg Ser Asp Lys Thr Ser Ile Tyr Ser
           515                 520                 525
   Ser Arg Asn Asp Ala Lys Pro Ile Asp Glu Thr Val Leu Arg Ile Asn
       530                 535                 540
   Ser Thr Ile Leu Phe Asn Gly Asp Gly Ile Ser Phe Thr Ser Lys Tyr
   545                 550                 555                 560
   Asn Trp Trp Asn Asn Leu Thr Glu Glu Cys Arg Leu Met His Leu Asp
                   565                 570                 575
   Thr Leu Ser Thr Lys Ile Arg Leu Thr Ala Glu Val Leu Met Glu Ile
               580                 585                 590
   Ala Ala Thr Leu Tyr Ile Phe Ala Ala Leu Arg Glu Ala Arg Phe Leu
           595                 600                 605
   Gly Leu Asn Met Phe Ile Glu Asn Leu Met Thr Ala Pro Ser Arg Val
       610                 615                 620
   Met Phe Leu Phe Ser Cys Cys Ile Leu Leu Thr Phe Pro Phe Leu Arg
   625                 630                 635                 640
   Leu Ile Cys Ala Asp Glu Ile Glu Asp Met Leu Ala Val Val Val Met
                   645                 650                 655
   Leu Thr Thr Ala Pro Tyr Phe Leu Phe Phe Cys Arg Gly Phe Lys Thr
               660                 665                 670
   Val Gly Pro Phe Val Val Met Ile Tyr Arg Met Ile Met Gly Asp Leu
           675                 680                 685
   Leu Arg Phe Val Ser Ile Tyr Leu Val Phe Val Met Gly Phe Ser Gln
       690                 695                 700
   Ala Tyr Tyr Ile Ile Phe Leu Ser Phe Asp Asn Pro Asn Thr Pro Glu
   705                 710                 715                 720
   Gly Val Asp Asp Ser Met Ser Asn Pro Met Pro Ser Pro Ile Glu Ser
                   725                 730                 735
   Ile Met Ala Met Phe Leu Met Ser Met Thr Asn Phe Gly Asp Tyr Tyr
               740                 745                 750
   Gly Ala Phe Glu Arg Thr Gln His Glu Met Glu Ala Lys Phe Leu Phe
           755                 760                 765
   Val Val Tyr Met Ala Ile Val Ala Ile Leu Leu Val Asn Met Leu Ile
       770                 775                 780
   Ala Met Met Gly Asn Thr Tyr Gln Lys Ile Ala Glu Thr Arg Asn Glu
   785 790 795 800
   Trp Gln Arg Gln Trp Ala Arg Ile Val Leu Val Val Glu Arg Gly Val
   805 810 815
   Ser Pro Asp Glu Arg Leu Lys Lys Leu Met Asp Tyr Ser Gln Pro Met
   820 825 830
   Ser Asp Gly Arg Arg Ala Leu Val Leu Arg Leu Asn Gln Ser Glu Glu
   835 840 845
   Asp Lys Glu Glu Met Lys Glu Ile Leu Glu Met Lys Arg Thr His Asp
   850 855 860
   Lys Leu Tyr Lys Arg Arg Gln Ser Lys Thr Met Lys Lys Ile Leu Thr
   865 870 875 880
   Ser Glu Asp Asn Val Ile Leu
   885
<210> 70
   <211> 912
   <212> PRT
   <213> Apis florea
<220>
   <221> MOD_RES
   <222> (414)..(414)
   <223> Any amino acid
<400> 70
   Met Gly Asn Thr Glu Ser Asn Val Ala Ser Gly Val Lys Lys Gln Thr
   1               5                   10                  15
   Asp Ala Ser Ser Ile Leu Leu Tyr Lys Leu Val Asp Leu Lys Gly Gly
               20                  25                  30
   Gly Leu Leu Val Asp Met Met Lys Arg Ala Thr Gln Thr Lys Gln Tyr
           35                  40                  45
   Ala Glu Leu Asp His Ala Leu Arg Thr Lys Val Glu Pro Tyr Leu Tyr
       50                  55                  60
   Asn Lys Gly Lys Gly Lys Trp Ile Pro Ile Glu Lys Leu Val Leu Leu
   65                  70                  75                  80
   Arg Asn Lys Asp Arg Pro Lys His Lys Met Leu Pro Pro Leu Arg Ala
                   85                  90                  95
   Met Glu Asn Pro Ala Asp Tyr Asp Ile Asp Lys Asp Thr Gly Glu Asp
               100                 105                 110
   Glu Val Arg Asp Glu Thr Arg Ile Asp Lys Ser Lys Tyr Arg Leu Val
           115                 120                 125
   Cys Trp Ser Leu Ser Glu Arg Gly Ala Val Gly Glu Thr Ile Leu His
       130                 135                 140
   Leu Cys Met Leu His Ala Thr Ala Ile His Ile Asp Leu Ala Lys Arg
   145                 150                 155                 160
   Leu Leu Arg Phe Tyr Pro Lys Leu Ile Asn Asp Val Tyr Ile Ser Asp
                   165                 170                 175
   Glu Tyr Tyr Gly Glu Ser Ala Leu His Ile Ala Ile Val Asn Glu Asp
               180                 185                 190
   Pro Ser Met Val Lys Phe Leu Leu Asp Ser Gly Ala Asp Val His Glu
           195                 200                 205
   Arg Cys Ile Gly Asn Phe Met Cys Pro Glu Asp Gln Lys Ala Ser Arg
       210                 215                 220
   Ala Asp Ser Leu Asp His Glu Trp Val Cys Val Thr Pro Glu Thr Asn
   225                 230                 235                 240
   Tyr Asn Gly Tyr Val Tyr Trp Gly Glu Tyr Pro Leu Asn Phe Ala Ala
                   245                 250                 255
   Cys Leu Gly Gln Glu Glu Cys Tyr Arg Leu Ile Leu Ala Arg Gly Ala
               260                 265                 270
   Asp Pro Asp Lys Gln Asp Thr Asn Gly Asn Thr Val Leu His Met Leu
           275                 280                 285
   Val Ile Tyr Glu Lys Leu Ala Thr Phe Asp Met Ala Tyr Glu Val Gly
       290                 295                 300
   Ala Ser Leu Ala Ile Arg Asn Ala Gln His Leu Thr Pro Leu Thr Leu
   305                 310                 315                 320
   Ser Ala Lys Leu Ala Lys Ile Glu Met Phe Phe His Ile Leu Asn Ile
                   325                 330                 335
   Glu Arg Glu Ile Tyr Trp Gln Ile Gly Ser Ile Thr Cys Ala Ala Tyr
   340 345 350
   Pro Leu Ser Gln Val Asp Thr Ile Asp Val Asp Thr Gly Ser Ile Ser
   355 360 365
   His Asn Ser Ala Leu Asn Leu Val Val Phe Gly Glu Lys Asp Glu His
   370 375 380
   Leu Glu Leu Met Asp Gly Ile Leu Val Asp Leu Leu Asn Ala Lys Trp
   385 390 395 400
   Asn Thr Phe Val Lys Phe Arg Phe Tyr Arg Gln Phe Phe Xaa Phe Cys
   405 410 415
   Phe Tyr Phe Val Leu Ser Leu Ile Ser Phe Thr Leu Arg Pro Gly Pro
   420 425 430
   Ala Thr Thr Ser Ser Val Pro Asn Pro Val Ala Gln Thr Pro Leu Thr
   435 440 445
   Ser Thr Glu Leu Ser Thr Ile Ser Pro Lys Val Asp Pro Pro Val Ser
   450 455 460
   Asn Val Thr Val Asn Arg Ser Ser Asn Ala Phe Gln His Asp Ser Asn
   465 470 475 480
   Leu Ser Leu Leu Leu Asp Lys Ile Leu Ser Thr Ala Ser Val Lys Tyr
   485 490 495
   Pro Trp Asn Val Thr Arg Thr Arg Leu Asp Lys Leu Lys Leu Asp Ile
   500 505 510
   Ile Glu Asn Leu Thr Thr Ala Leu Asn Asn Leu Leu Ser Thr His Arg
   515 520 525
   Asp Glu Ile Glu Phe Phe Asn His Ser Tyr Lys Thr Thr Glu Tyr Phe
   530 535 540
   His Ser Thr Arg Asn Asp Thr Thr Pro Ile Asp Asn Leu Lys Ile Thr
   545 550 555 560
   Ile Asn Glu Thr Val Leu Ser Val Asn Ser Thr Ile Leu Phe Asn Asp
   565 570 575
   Gly Ile Ser Phe Ala Thr Gly Lys Tyr Asn Trp Trp Asn Asn Leu Thr
   580 585 590
   Glu Glu Cys Arg Leu Met His Val Asp Thr Leu Ser Thr Lys Ile Arg
           595                 600                 605
   Leu Thr Ala Glu Val Leu Met Glu Ile Ala Ala Thr Leu Tyr Ile Phe
       610                 615                 620
   Ala Ala Leu Arg Glu Ala Arg Phe Leu Gly Leu Asn Met Phe Ile Glu
   625                 630                 635                 640
   Asn Leu Met Thr Ala Pro Ser Arg Val Met Phe Leu Phe Ser Cys Cys
                   645                 650                 655
   Ile Leu Leu Thr Phe Pro Phe Leu Arg Leu Ile Cys Ala Asp Gln Val
               660                 665                 670
   Glu Asp Met Leu Ala Val Val Val Met Leu Thr Thr Ala Pro Tyr Phe
           675                 680                 685
   Leu Phe Phe Cys Arg Gly Phe Lys Thr Val Gly Pro Phe Val Val Met
       690                 695                 700
   Ile Tyr Arg Met Ile Met Gly Asp Leu Leu Arg Phe Val Ser Ile Tyr
   705                 710                 715                 720
   Leu Val Phe Val Met Gly Phe Ser Gln Ala Tyr Tyr Ile Ile Phe Leu
                   725                 730                 735
   Ser Phe Asp Asn Pro Asn Thr Pro Glu Gly Val Asp Asp Ser Met Ser
               740                 745                 750
   Asn Pro Met Pro Ser Pro Ile Glu Ser Ile Met Ala Met Phe Leu Met
           755                 760                 765
   Ser Met Thr Asn Phe Gly Asp Tyr Tyr Gly Ala Phe Glu Arg Thr Gln
       770                 775                 780
   His Glu Ile Glu Ala Lys Phe Leu Phe Val Val Tyr Met Ala Ile Val
   785                 790                 795                 800
   Ala Ile Leu Leu Val Asn Met Leu Ile Ala Met Met Gly Asn Thr Tyr
                   805                 810                 815
   Gln Lys Ile Ala Glu Thr Arg Asn Glu Trp Gln Arg Gln Trp Ala Arg
               820                 825                 830
   Ile Val Leu Val Val Glu Arg Gly Val Ser Pro Glu Glu Arg Leu Lys
           835                 840                 845
   Lys Leu Met Asp Tyr Ser Gln Pro Met Ser Asp Gly Arg Arg Ala Leu
       850                 855                 860
   Val Leu Arg Leu Asn Gln Ser Glu Glu Asp Lys Glu Glu Met Lys Glu
   865                 870                 875                 880
   Ile Leu Glu Met Lys Arg Thr His Asp Lys Leu Tyr Lys Lys Arg Gln
                   885                 890                 895
   Ser Lys Ile Met Lys Lys Ile Leu Thr Ser Glu Asp Asn Val Ile Leu
               900                 905                 910
<210> 71
   <211> 823
   <212> PRT
   <213> Pediculus humanus
<220>
   <221> source
   <223> /note="Pediculus humanus corporis"
<400> 71
   Met Gly Asn Thr Glu Ser Asn Val Thr Ser Gly Val Lys Lys Gln Ala
   1               5                   10                  15
   Gly Thr Ser Glu Gln Pro Leu Tyr Lys Leu Val Gly Leu Lys Gly Gly
               20                  25                  30
   Gly Leu Leu Val Asp Met Met Lys Arg Ala Thr Gln Thr Lys Gln Tyr
           35                  40                  45
   Ala Glu Leu Asp His Ala Ile Lys Thr Lys Ile Glu Pro Phe Leu Tyr
       50                  55                  60
   Asn Lys Gly Lys Gly Arg Leu Ile Pro Ile Ser Gln Leu Val Leu Leu
   65                  70                  75                  80
   Arg Asn Cys Glu Arg Ser Arg Thr Lys Leu Leu Pro Leu Leu Lys Asn
                   85                  90                  95
   Met Glu Asp Pro Glu Ser Glu Phe Asp Met Asp Arg Asp Gly Asn Pro
               100                 105                 110
   Gly Asp Phe Gln Pro Ala Pro Asp Gly Val Gln Glu Asp Phe His Asp
           115                 120                 125
   Tyr Asn Lys Lys Tyr Arg Asp Val Cys Trp Asp Leu Lys Lys Arg Gly
       130                 135                 140
   Ala Val Gly Glu Thr Cys Leu His Leu Cys Met Leu Asn Ala Thr Ser
   145                 150                 155                 160
   Ile His Ala Asp Leu Ala Lys Arg Leu Leu Lys Phe Tyr Pro Lys Leu
                   165                 170                 175
   Ile Asn Asp Ile Tyr Met Ser Asp Glu Tyr Tyr Gly Glu Ser Val Leu
               180                 185                 190
   His Val Ala Ile Val Asn Glu Asp Pro Ala Met Val Lys Tyr Leu Leu
           195                 200                 205
   Asp Ser Gly Ser Asn Phe His Glu Arg Cys Phe Gly Asn Phe Met Cys
       210                 215                 220
   Pro Glu Asp Gln Lys Ala Thr Arg Thr Asp Ser Leu Asp His Glu Tyr
   225                 230                 235                 240
   Val Asn Leu Ser Thr Glu Thr Asn Tyr Glu Gly Tyr Val Tyr Trp Gly
                   245                 250                 255
   Glu Tyr Pro Leu Ser Phe Ala Ala Cys Leu Gly Gln Glu Glu Ser Tyr
               260                 265                 270
   Arg Leu Met Leu Ala Lys Gly Ala Asn Pro Asp Asn Gln Asp Thr Asn
           275                 280                 285
   Gly Asn Thr Val Leu His Met Leu Val Ile His Asn Lys Leu Pro Met
       290                 295                 300
   Phe Asp Val Ala Tyr Glu Val Gly Ala Asn Leu Ser Leu Lys Asn Ala
   305                 310                 315                 320
   Lys Cys Leu Thr Pro Leu Thr Leu Ala Ala Lys Leu Ala Arg Val Glu
                   325                 330                 335
   Leu Phe Phe His Ile Leu Asn Ile Glu Arg Glu Ile Tyr Trp Gln Ile
               340                 345                 350
   Gly Ser Ile Thr Cys Ala Ala Tyr Pro Leu Ser Gln Ile Asp Thr Ile
           355                 360                 365
   Asp Ile Glu Thr Gly Asn Ile Ser Lys Asn Ser Ala Leu Asn Leu Val
       370                 375                 380
   Val Phe Gly Glu Lys Asp Glu His Leu Glu Leu Met Asp Gly Val Leu
   385                 390                 395                 400
   Val Asp Leu Leu Val Ala Lys Trp Asn Thr Tyr Val Lys Phe Arg Phe
   405 410 415
   Tyr Arg Gln Phe Ile Ser Phe Phe Phe Tyr Phe Leu Ile Ser Val Ile
   420 425 430
   Cys Phe Thr Leu Arg Pro Gly Pro Pro Thr Gly Thr Thr Thr Thr Thr
   435 440 445
   Asn Gly Ile Val Val Leu Asn Asn Asn Asn Asn Asn Ile Thr Ser Val
   450 455 460
   Val Asp Asn Ser Asn Asn Val Thr Thr Thr Thr Ala Thr Thr Thr Ala
   465 470 475 480
   Thr Ser Tyr Ser Phe Pro Gly His Val Ile Val Thr Leu Leu Ser Tyr
   485 490 495
   Asp Lys Tyr Glu Asp Lys Ile Arg Ile Ile Ser Glu Leu Ala Met Glu
   500 505 510
   Ile Gly Ser Val Leu Tyr Leu Leu Gly Ala Leu Arg Glu Ala Lys Phe
   515 520 525
   Leu Gly Ile His Met Phe Ile Glu Asn Leu Met Thr Ala Pro Ser Arg
   530 535 540
   Val Leu Phe Leu Gly Ser Cys Cys Ile Met Gln Phe Val Pro Trp Leu
   545 550 555 560
   Arg Ile Thr Cys Lys Glu Glu Thr Glu Asp Ile Val Ala Val Ile Ile
   565 570 575
   Met Leu Thr Thr Ala Pro Tyr Phe Leu Phe Phe Cys Arg Lys Met Asn
   580 585 590
   Asp Pro Val Phe Leu Phe Leu Phe Phe Leu Ile Lys Lys Ile Phe Phe
   595 600 605
   Leu Ile Phe Phe Phe Phe Phe Phe Thr Lys Pro Thr Tyr Tyr Ile Ile
   610 615 620
   Phe Leu Ser Phe Asp Asn Pro Lys Thr Pro Asp Gly Val Asp Asp Ser
   625 630 635 640
   Gly Thr Asn Pro Met Gln Ser Pro Val Glu Ser Ile Met Ala Met Phe
   645 650 655
   Leu Met Ser Leu Thr Asn Phe Gly Glu Tyr Tyr Gly Ala Phe Glu Lys
   660 665 670
   Thr Glu His Glu Phe Val Ala Lys Leu Met Phe Val Val Tyr Met Ala
   675 680 685
   Ile Val Ala Ile Leu Leu Val Asn Met Leu Ile Ala Met Met Gly Asn
   690 695 700
   Thr Tyr Gln Lys Ile Ala Glu Thr Arg Asn Glu Trp Gln Arg Gln Trp
   705 710 715 720
   Ala Arg Ile Val Leu Val Val Glu Arg Gly Val Ser Pro Gln Glu Arg
   725 730 735
   Leu Lys Lys Leu Met Leu Tyr Ser Gln Pro Met Ser Asp Gly Arg Arg
   740 745 750
   Ala Leu Val Leu Arg Leu His Gln Thr Pro Glu Asp Lys Glu Glu Met
   755 760 765
   Lys Glu Ile Leu Asp Met Lys Arg Ile His Asn Arg Thr Val Gln Arg
   770 775 780
   Arg Lys Ala Lys Glu Asn Lys Asn Val Thr Phe Ala Gly Thr Pro Lys
   785 790 795 800
   Asn Gly Ile Lys Asn Arg Leu Leu Gly Pro Val Pro Pro Leu Lys Ser
   805 810 815
   Ile Pro Ser Lys Pro Lys Ile
   820
<210> 72
   <211> 355
   <212> PRT
   <213> Danaus plexippus
<400> 72
   Met Leu Glu Ser Gly Gly Gly Leu Leu Val Asp Met Met Lys Arg Ala
   1 5 10 15
   Val Gln Asn Lys Gln Tyr Ala Glu Ile Asp His Ala Ile Lys Thr Lys
   20 25 30
   Val Glu Pro Phe Leu Tyr Asn Lys Gly Lys Gly Arg His Ile Pro Ile
   35 40 45
   Ser His Leu Val Leu Leu Arg Asn Lys Glu Arg Gly Arg His Lys Leu
       50                  55                  60
   Leu Pro Pro Leu Arg Gly Met Glu Asn Pro Asp Glu Glu Phe Asp Val
   65                  70                  75                  80
   Glu Lys Asp Trp Pro Val Val Thr Gln Glu Glu Tyr Glu Ala Asn Pro
                   85                  90                  95
   Ser Ala Tyr Arg Glu Leu Cys Trp Asp Leu Lys Glu Arg Gly Ala Val
               100                 105                 110
   Gly Glu Thr Ile Leu His Leu Cys Leu Leu Asn Ala Thr Ser Leu Leu
           115                 120                 125
   Ala Asp Leu Ala Lys Arg Leu Leu Arg Phe Tyr Pro Lys Leu Ile Asn
       130                 135                 140
   Asp Ile Tyr Met Ser Asp Glu Tyr Tyr Gly Glu Ser Val Leu His Met
   145                 150                 155                 160
   Thr Ile Val Asn Glu Asp Pro Thr Met Val Lys Phe Leu Leu Asp Ser
                   165                 170                 175
   Gly Ala Asp Tyr His Glu Arg Cys Phe Gly Asn Phe Met Cys Pro Glu
               180                 185                 190
   Asp Gln Lys Ala Ser Arg Thr Asp Ser Phe Asp His Glu Trp Val Asn
           195                 200                 205
   Val Gln Pro Asp Thr Asn Tyr Asp Gly Tyr Val Tyr Trp Gly Glu Tyr
       210                 215                 220
   Pro Leu Ser Phe Ala Ala Cys Leu Gly Gln Glu Glu Cys Tyr Arg Leu
   225                 230                 235                 240
   Ile Leu Ala Arg Gly Ala Asn Pro Asp Lys Gln Asp Thr Asn Gly Asn
                   245                 250                 255
   Thr Val Leu His Met Leu Ile Ile Tyr Asp Lys Ile Ser Thr Phe Asp
               260                 265                 270
   Met Ala Tyr Glu Val Gly Ala Ser Leu Asn Ile Arg Asn Val Gln Asn
           275                 280                 285
   Leu Thr Pro Leu Thr Leu Ala Ala Lys Leu Ala Arg Val Glu Leu Phe
       290                 295                 300
   Phe His Ile Leu Asn Ile Glu Arg Glu Ile Tyr Trp Gln Ile Gly Ala
   305 310 315 320
   Thr Thr Cys Ala Ala Tyr Pro Leu Gly Gln Val Asp Thr Ile Asp Thr
   325 330 335
   Glu Thr Gly Arg Ile Ser Lys Asp Ser Ala Leu Asn Leu Val Val Phe
   340 345 350
   Gly Val Ser
   355
<210> 73
   <211> 861
   <212> PRT
   <213> Solenopsis invicta
<400> 73
   Gly Gly Leu Leu Val Asp Met Met Lys Arg Ala Ser Gln Thr Lys Gln
   1 5 10 15
   Tyr Ala Glu Leu Asp His Ala Met Arg Thr Lys Val Glu Pro Phe Leu
   20 25 30
   Tyr Asn Lys Gly Lys Gly Lys Trp Ile Pro Ile Glu Lys Leu Val Leu
   35 40 45
   Leu Arg Asn Lys Asp Arg Pro Arg His Lys Met Leu Pro Pro Leu Lys
   50 55 60
   Ala Met Glu Asn Pro Ala Asp Tyr Asp Ile Asp Lys Asp Met Gly Asp
   65 70 75 80
   Glu Glu Val Asp Glu Ala Lys Ile Asp Lys Ser Lys Tyr Arg Leu Val
   85 90 95
   Cys Trp Asn Leu Ser Asp Arg Gly Ala Val Gly Glu Thr Ile Leu His
   100 105 110
   Leu Cys Met Leu Asn Ala Thr Ala Ile His Ala Asp Leu Ala Lys Arg
   115 120 125
   Leu Leu Arg Phe Tyr Pro Leu Leu Ile Tyr Asp Ile Tyr Leu Cys Asp
   130 135 140
   Glu Tyr Tyr Gly Glu Asn Val Leu His Ile Ala Ile Val Asn Glu Asp
   145 150 155 160
   Pro Ser Leu Val Lys Phe Leu Leu Asp Ser Gly Ala Asp Asp Pro Arg
                   165                 170                 175
   Ile Ile His Glu Arg Cys Phe Gly Thr Phe Met Cys Pro Glu Asp Gln
               180                 185                 190
   Lys Ala Ser Arg Phe Asp Ser Met Asp His Glu Trp Val Cys Val Thr
           195                 200                 205
   Pro Glu Thr Asp Tyr Asn Gly Tyr Val Tyr Trp Gly Glu Tyr Pro Leu
       210                 215                 220
   Ser Phe Ala Ala Cys Leu Gly Gln Glu Glu Cys Tyr Arg Leu Ile Leu
   225                 230                 235                 240
   Ala Arg Gly Ala Asp Pro Asp Lys Gln Asp Thr Asn Gly Asn Thr Val
                   245                 250                 255
   Leu His Met Leu Ala Thr Phe Asp Met Ala Tyr Glu Val Gly Ala Ser
               260                 265                 270
   Leu Asp Ile Arg Asn Val Gln His Leu Thr Pro Leu Thr Leu Ala Ala
           275                 280                 285
   Lys Leu Ala Arg Ile Glu Met Phe Phe His Ile Leu Asn Ile Glu Arg
       290                 295                 300
   Glu Ile Tyr Trp Gln Ile Gly Ser Ile Thr Cys Ala Ala Tyr Pro Leu
   305                 310                 315                 320
   Ser Gln Ile Asp Thr Ile Asp Val Asn Thr Gly Thr Ile Asn Lys Thr
                   325                 330                 335
   Ser Ala Leu Asn Leu Val Val Phe Gly Asp Lys Asp Glu His Leu Glu
               340                 345                 350
   Leu Met Asp Gly Met Leu Val Asp Leu Leu Asn Ala Lys Trp Asn Thr
           355                 360                 365
   Phe Val Lys Ser Arg Phe Tyr Arg Gln Phe Phe Leu Phe Cys Phe His
       370                 375                 380
   Phe Val Leu Ser Leu Ile Ser Phe Thr Leu Arg Pro Gly Pro Ser Thr
   385                 390                 395                 400
   Met Glu Thr Asp Glu Asn Ala Asn Asp Thr Ser Thr Lys Thr Asn Ser
                   405                 410                 415
   Ser Asn Ile Thr Glu Pro Ile Ile Leu Lys Pro Leu Gln Ser Ser Asp
               420                 425                 430
   Leu Ser Glu Leu Val Thr Asn Ser Phe Val Ala Ala Phe Ala Ser Asn
           435                 440                 445
   Leu Lys Ser Ser Leu Asn Met Thr Gln Glu Ser Leu Glu Lys Ile Gln
       450                 455                 460
   Leu Gln Val Thr Ser Asn Ile Thr Ser Ala Leu Lys Ser Ile Leu Leu
   465                 470                 475                 480
   Leu Thr Leu Asn Glu Asn Glu Gly Ile Leu Ser Pro Pro Asp Glu Ala
                   485                 490                 495
   Thr Ile His Thr Pro Trp Tyr Thr Asp Ser Ser Pro Asn Thr Ser Asp
               500                 505                 510
   Tyr Phe Thr Asp Leu Tyr Asn Lys Thr Val Val Ala Glu Asn Ser Thr
           515                 520                 525
   Val Lys Ala Val Val Asp Asp Val Ala Gln Thr Asp Tyr Trp Trp Asp
       530                 535                 540
   Asp Phe Ala Glu Glu Cys Arg Leu Met Gln Leu Thr Glu Thr Ser Ala
   545                 550                 555                 560
   Lys Ile Arg Leu Thr Ala Glu Thr Phe Met Tyr Ile Ala Ala Val Leu
                   565                 570                 575
   Tyr Ile Leu Ala Ala Leu Arg Glu Ala Arg Phe Leu Gly Leu Asn Met
               580                 585                 590
   Phe Ile Glu Asn Leu Ala Thr Ala Pro Ser Arg Val Met Phe Leu Phe
           595                 600                 605
   Ser Cys Cys Ile Leu Leu Ser Phe Pro Phe Leu Arg Phe Val Cys Ala
       610                 615                 620
   Asp Glu Ile Glu Asp Ile Leu Ala Val Val Val Met Leu Thr Thr Ala
   625                 630                 635                 640
   Pro Tyr Phe Leu Phe Phe Cys Arg Gly Phe Lys Thr Val Gly Pro Phe
                   645                 650                 655
   Val Val Met Ile Tyr Arg Met Ile Met Gly Asp Leu Leu Arg Phe Val
   660 665 670
   Cys Ile Tyr Leu Val Phe Val Met Gly Phe Ser Gln Ala Tyr Tyr Ile
   675 680 685
   Ile Phe Leu Ser Phe Asp Asn Pro Asn Thr Pro Glu Gly Thr Asp Asp
   690 695 700
   Ser Ile Thr Asn Pro Met Pro Ser Pro Met Glu Ser Val Met Ala Met
   705 710 715 720
   Phe Leu Met Ser Met Thr Asn Phe Gly Asp Tyr Phe Gly Ala Phe Glu
   725 730 735
   Arg Thr Glu His Glu Phe Glu Ala Lys Leu Leu Phe Val Leu Tyr Met
   740 745 750
   Val Ile Val Ala Ile Leu Leu Val Asn Met Leu Ile Ala Met Met Gly
   755 760 765
   Asn Thr Tyr Gln Lys Ile Ala Glu Thr Arg Asn Glu Trp Gln Arg Gln
   770 775 780
   Trp Ala Arg Ile Val Leu Val Val Glu Arg Gly Val Ser Pro Ala Glu
   785 790 795 800
   Arg Leu Lys Lys Leu Thr Asp Tyr Ser Gln Pro Met Ser Asp Gly Arg
   805 810 815
   Pro Ala Leu Val Leu Arg Leu His Gln Thr Glu Glu Asp Lys Glu Glu
   820 825 830
   Met Lys Glu Ile Leu Glu Met Lys Arg Thr His Glu Lys Leu Leu Lys
   835 840 845
   Lys Arg Gln Cys Arg Met Ser Lys Glu Lys Ile Leu Ser
   850 855 860
<210> 74
   <211> 861
   <212> PRT
   <213> Acromyrmex echinatior
<400> 74
   Met Gly Asn Thr Glu Ser Asn Val Ala Ser Ser Val Lys Lys Gln Thr
   1               5                   10                  15
   Asp Thr Ser Ser Ile Leu Leu Tyr Lys Leu Val Asp Leu Lys Gly Gly
               20                  25                  30
   Gly Leu Leu Val Asp Met Met Lys Arg Ala Thr Gln Thr Lys Gln Phe
   35 40 45
   Ala Glu Leu Asp His Ala Met Arg Thr Lys Val Glu Pro Phe Leu Tyr
   50 55 60
   Asn Lys Gly Lys Gly Lys Trp Ile Pro Val Asp Lys Leu Val Leu Leu
   65 70 75 80
   Arg Asn Lys Asp Arg Pro Arg His Lys Met Leu Pro Pro Leu Lys Ala
   85 90 95
   Met Glu Asn Pro Ala Asp Tyr Asp Ile Asp Lys Asp Met Gly Asp Glu
   100 105 110
   Glu Val Asp Glu Ala Lys Ile Asp Lys Ser Lys Tyr Arg Leu Val Cys
   115 120 125
   Trp Asn Leu Ser Asp Arg Gly Ala Val Gly Glu Thr Ile Leu His Leu
   130 135 140
   Cys Met Leu His Ala Thr Ala Ile His Ala Asp Leu Ala Lys Arg Leu
   145 150 155 160
   Leu Arg Phe Tyr Pro Lys Leu Ile Asn Asp Ile Tyr Leu Ser Asp Glu
   165 170 175
   Tyr Tyr Gly Glu Asn Val Leu His Ile Ala Ile Val Asn Glu Asp Pro
   180 185 190
   Ser Met Val Lys Phe Leu Leu Asp Ser Gly Ala Asp Asp Pro Cys Ile
   195 200 205
   Ile His Glu Arg Cys Phe Gly Asn Phe Met Cys Pro Glu Asp Gln Lys
   210 215 220
   Ala Ser Arg Phe Asp Asn Met Asp His Glu Trp Val Cys Val Ser Pro
   225 230 235 240
   Glu Thr Asn Tyr Asn Gly Tyr Val Tyr Trp Gly Glu Tyr Pro Leu Ser
   245 250 255
   Phe Ala Ala Cys Leu Gly Gln Glu Glu Cys Tyr Arg Leu Ile Leu Ala
   260 265 270
   Arg Gly Ala Asp Pro Asp Lys Gln Asp Thr Asn Gly Asn Thr Val Met
   275 280 285
   His Met Leu Ala Thr Phe Asp Met Ala Tyr Glu Val Gly Ala Ser Leu
   290 295 300
   Gly Ile Arg Asn Val Gln His Leu Thr Pro Leu Thr Leu Ala Ala Lys
   305 310 315 320
   Leu Ala Arg Ile Glu Ile Phe Phe His Ile Met Asn Ile Glu Arg Glu
   325 330 335
   Ile Tyr Trp Gln Ile Gly Ser Ile Thr Cys Ala Ala Tyr Pro Leu Ser
   340 345 350
   Gln Ile Asp Thr Ile Asp Val Asn Thr Gly Thr Ile Asn Asn Asn Ser
   355 360 365
   Ala Leu Asn Leu Val Val Phe Gly Asp Lys Asp Glu His Leu Glu Leu
   370 375 380
   Met Asp Gly Val Leu Val Asp Leu Leu Asn Ala Lys Trp Asn Thr Phe
   385 390 395 400
   Val Lys Ser Arg Phe Tyr Asn Gln Phe Phe Leu Phe Cys Phe Tyr Phe
   405 410 415
   Val Leu Ser Leu Ile Ser Phe Thr Leu Arg Pro Gly Pro Ser Thr Ile
   420 425 430
   Glu Thr Asp Glu Asn Ala Asn Asp Thr Ser Thr Arg Thr Asn Ser Ser
   435 440 445
   Asn Ile Thr Glu Pro Ile Ile His Lys Thr Leu Gln Ser Ser Asp Leu
   450 455 460
   Ser Glu Leu Val Thr Asn Ser Leu Val Ala Ala Phe Thr Ser Asn Phe
   465 470 475 480
   Lys Ser Ser Leu Asn Met Thr Gln Glu Ser Leu Glu Lys Ile Gln Leu
   485 490 495
   Gln Val Thr Ser Asn Ile Thr Ser Ala Leu Lys Ser Ile Leu Leu Leu
   500 505 510
   Thr Leu Asn Glu Asn Glu Gly Ile Leu Ser Pro Pro Asp Glu Ala Thr
   515 520 525
   Ile His Thr Leu Trp Tyr Thr Asp Ser Pro Pro Asn Ala Ser Asp Tyr
       530                 535                 540
   Phe Met Asn Thr Tyr Asn Lys Thr Asp Ala Asn Ser Thr Val Lys Thr
   545                 550                 555                 560
   Ile Ile Asp Gly Val Ala Arg Pro Lys Phe Lys Ser Asn Tyr Trp Trp
                   565                 570                 575
   Asp Asp Phe Ser Glu Glu Cys Arg Leu Met Gln Leu Ser Glu Met Ser
               580                 585                 590
   Ala Lys Ile Arg Leu Thr Ala Glu Ile Phe Met Phe Ile Ala Ala Val
           595                 600                 605
   Leu Tyr Ile Leu Ala Ala Leu Arg Glu Ala Lys Phe Leu Gly Leu His
       610                 615                 620
   Met Phe Ile Glu Asn Leu Met Thr Val Pro Ser Arg Val Met Phe Leu
   625                 630                 635                 640
   Ile Ser Cys Cys Ile Leu Met Trp Phe Pro Val Leu Arg Phe Thr Cys
                   645                 650                 655
   Ser Asp Glu Val Glu Asp Met Leu Ala Val Val Val Met Leu Thr Thr
               660                 665                 670
   Ala Pro Tyr Phe Leu Phe Phe Cys Arg Gly Phe Lys Thr Val Gly Pro
           675                 680                 685
   Phe Val Val Met Ile Tyr Arg Met Ile Met Gly Asp Leu Ile Arg Phe
       690                 695                 700
   Val Ser Ile Tyr Leu Val Phe Val Met Gly Phe Ser Gln Ala Tyr Tyr
   705                 710                 715                 720
   Ile Ile Phe Leu Ser Phe Asp Asn Pro Asn Thr Pro Glu Gly Ile Asp
                   725                 730                 735
   Asp Ser Ile Thr Asn Pro Met Pro Ser Pro Met Glu Ser Val Met Ala
               740                 745                 750
   Met Phe Leu Met Ser Met Thr Asn Phe Gly Asp Tyr Phe Gly Ala Phe
           755                 760                 765
   Asp Arg Thr Glu His Glu Phe Glu Ala Lys Leu Leu Phe Val Leu Tyr
       770                 775                 780
   Met Ala Ile Val Ala Ile Leu Leu Val Asn Met Leu Ile Ala Met Met
   785 790 795 800
   Gly Asn Thr Tyr Gln Lys Ile Ala Glu Thr Arg Asn Glu Trp Gln Arg
   805 810 815
   Gln Glu Glu Asp Lys Glu Glu Met Lys Glu Ile Leu Glu Met Lys Arg
   820 825 830
   Thr His Asp Arg Leu Leu Lys Lys Arg Gln Asn Arg Met Ser Lys Glu
   835 840 845
   Lys Ile Ile Phe Glu Thr Glu Pro Ile Ile Thr Lys Asn
   850 855 860
<210> 75
   <211> 904
   <212> PRT
   <213> Camponotus floridanus
<400> 75
   Met Gly Asn Thr Glu Ser Asn Val Thr Ser Gly Val Lys Lys Gln Thr
   1 5 10 15
   Asp Thr Ser Ser Ile Val Leu Tyr Lys Leu Val Asp Leu Lys Gly Gly
   20 25 30
   Gly Leu Leu Val Asp Met Met Lys Arg Ala Thr Gln Thr Lys Gln Phe
   35 40 45
   Ala Glu Leu Asp His Ala Met Arg Thr Lys Val Glu Pro Phe Leu Tyr
   50 55 60
   Asn Lys Gly Glu Gly Lys Trp Ile Pro Ile Asp Lys Leu Val Leu Leu
   65 70 75 80
   Arg Asn Lys Asp Arg Pro Arg His Lys Met Leu Ala Pro Leu Lys Ala
   85 90 95
   Met Glu Asn Pro Ala Asp Tyr Asp Ile Asp Lys Asp Met Gly Asp Glu
   100 105 110
   Glu Val Asp Glu Thr Lys Ile Asp Lys Ser Lys Tyr Arg Leu Val Cys
   115 120 125
   Trp Asn Leu Ser Glu Arg Gly Ala Val Gly Glu Thr Ile Leu His Leu
   130 135 140
   Cys Met Leu Asn Ala Thr Ala Leu His Ala Asp Leu Ala Lys Arg Leu
   145 150 155 160
   Leu Arg Phe Tyr Pro Lys Leu Ile Asn Asp Ile Tyr Leu Cys Asp Glu
   165 170 175
   Tyr Tyr Gly Glu Asn Val Leu His Ile Ala Ile Val Asn Glu Asp Pro
   180 185 190
   Ser Met Val Lys Tyr Leu Leu Asp Ser Gly Ala Asp Asp Pro Arg Ile
   195 200 205
   Ile His Glu Arg Cys Phe Gly Asn Phe Met Cys Pro Glu Asp Gln Lys
   210 215 220
   Ala Ser Arg Phe Asp Ser Gln Asp His Glu Trp Val Cys Val Thr Pro
   225 230 235 240
   Glu Thr Asn Tyr Asn Gly Tyr Val Tyr Trp Gly Glu Tyr Pro Leu Ser
   245 250 255
   Phe Ala Ala Cys Leu Gly Gln Glu Glu Cys Tyr Arg Leu Ile Leu Ala
   260 265 270
   Arg Gly Ala Asp Pro Asp Lys Gln Asp Thr Asn Gly Asn Thr Val Leu
   275 280 285
   His Met Leu Ala Thr Phe Asp Met Ala Tyr Glu Val Gly Ala Ser Leu
   290 295 300
   Asp Ile Arg Asn Val Gln His Leu Thr Pro Leu Thr Leu Ala Ala Lys
   305 310 315 320
   Leu Ala Arg Ile Glu Met Phe Phe His Ile Leu Asn Ile Glu Arg Glu
   325 330 335
   Ile Tyr Trp Gln Ile Gly Ser Ile Thr Cys Ala Ala Tyr Pro Leu Ser
   340 345 350
   Gln Ile Asp Thr Ile Asp Val Asn Ala Gly Thr Ile Asn Asn Asn Ser
   355 360 365
   Ala Leu Asn Leu Val Val Phe Gly Glu Lys Asp Glu His Leu Glu Leu
   370 375 380
   Met Asp Gly Val Leu Ile Asp Leu Leu Asn Ala Lys Trp Asn Thr Phe
   385 390 395 400
   Val Lys Phe Arg Phe Tyr Arg Gln Phe Ile Leu Phe Tyr Phe Tyr Phe
   405 410 415
   Val Leu Ser Leu Ile Ser Phe Thr Leu Arg Pro Gly Pro Gln Thr Asp
   420 425 430
   Glu Asn Ala Asn Asp Thr Leu Thr Asn Ser Phe Asn Thr Thr Arg Glu
   435 440 445
   Ser Met Ile Leu Lys Ser Leu His Ser Ser Asp Leu Ser Glu Leu Val
   450 455 460
   Thr Asn Ser Phe Thr Ala Ala Phe Thr Ser Asn Phe Lys Ser Ser Leu
   465 470 475 480
   Asn Val Thr Gln Glu Ser Leu Glu Lys Ile Gln Leu Gln Val Thr Ser
   485 490 495
   Asn Val Thr Ser Ala Leu Arg Ser Ile Leu Leu Leu Thr Leu Ser Asn
   500 505 510
   Asn Glu Asp Leu Leu Ser Ser Pro Asp Glu Ala Ala Ile His Thr Leu
   515 520 525
   Trp Tyr Thr Asp Asn Pro Gln Asn Ala Ser Asp Tyr Phe Ile Asn Met
   530 535 540
   Tyr Asn Lys Thr Val Thr Ile Asp Thr Asn Ser Thr Ala Lys Thr Thr
   545 550 555 560
   Ile Asp Asp Val Thr Leu Leu Gly Lys Phe Asn Asn His Glu Lys Trp
   565 570 575
   Trp Asp Asp Leu Thr Glu Glu Cys Arg Leu Met Gln Leu Thr Thr Met
   580 585 590
   Ser Ala Lys Ile Arg Leu Thr Ala Glu Ile Val Met Tyr Phe Ala Ala
   595 600 605
   Val Leu Tyr Ile Leu Ala Ala Leu Arg Glu Ala Arg Phe Leu Gly Leu
   610 615 620
   Asn Met Phe Ile Glu Asn Leu Met Thr Ala Pro Ser Arg Val Met Phe
   625 630 635 640
   Leu Phe Ser Cys Cys Ile Leu Met Ser Phe Pro Val Leu Arg Leu Ser
   645 650 655
   Cys Ser Asp Glu Val Glu Asp Val Leu Ala Val Val Val Met Leu Thr
   660 665 670
   Thr Ala Pro Tyr Phe Leu Phe Phe Cys Arg Gly Phe Lys Thr Val Gly
   675 680 685
   Pro Phe Val Val Met Ile Tyr Arg Met Ile Thr Gly Asp Leu Leu Arg
   690 695 700
   Phe Val Ser Ile Tyr Leu Val Phe Val Met Gly Phe Ser Gln Ala Tyr
   705 710 715 720
   Tyr Ile Ile Phe Leu Ser Phe Asp Asn Pro Asn Thr Pro Glu Gly Val
   725 730 735
   Asp Asp Ser Ile Thr Asn Pro Met Pro Ser Pro Met Glu Ser Val Met
   740 745 750
   Ala Met Phe Leu Met Ser Met Thr Asn Phe Gly Asp Tyr Tyr Ala Thr
   755 760 765
   Phe Asp Arg Thr Glu His Glu Phe Glu Ala Lys Leu Leu Phe Val Leu
   770 775 780
   Tyr Met Ala Ile Val Ala Ile Leu Leu Val Asn Met Leu Ile Ala Met
   785 790 795 800
   Met Gly Asn Thr Tyr Gln Lys Ile Ala Glu Thr Arg Asn Glu Trp Gln
   805 810 815
   Arg Gln Trp Ala Arg Ile Val Leu Val Val Glu Arg Gly Val Ser Pro
   820 825 830
   Ala Glu Arg Leu Lys Lys Leu Met Asp Tyr Ser Gln Pro Met Ser Asp
   835 840 845
   Gly Arg Lys Ala Leu Val Leu Arg Leu Asn Gln Thr Glu Glu Asp Lys
   850 855 860
   Glu Glu Met Lys Glu Ile Leu Glu Met Lys Arg Thr His Asp Lys Leu
   865 870 875 880
   Ile Lys Lys Arg Gln Asp Lys Ile Leu Lys Glu Lys Ala Leu Ala Glu
   885 890 895
   Thr Glu Ala Val Ile Thr Arg Lys
   900
<210> 76
   <211> 849
   <212> PRT
   <213> Myzus persicae
<220>
   <221> MOD_RES
   <222> (70)..(213)
   <223> Any amino acid
<220>
   <221> MOD_RES
   <222> (300)..(328)
   <223> Any amino acid
<220>
   <221> MOD_RES
   <222> (621)..(628)
   <223> Any amino acid
<400> 76
   Ala Ile Lys Thr Gln Val Glu Pro Met Leu Tyr Asn Lys Gly Glu Gly
   1               5                   10                  15
   Arg Met Ile Pro Ile Ala Lys Leu Val Leu Leu Arg Asn Arg Asp Arg
               20                  25                  30
   Ser Arg Ala Arg Trp Leu Pro Pro Leu Lys Asn Met Glu Asp Ala Asn
           35                  40                  45
   Glu Tyr Asp Ile Glu Lys Asp Ile Pro Thr Ile Glu Glu Leu Glu Lys
       50                  55                  60
   Leu Gly Glu Val Gln Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa Xaa Xaa Xaa Xaa Xaa Xaa Xaa Xaa Xaa Xaa Xaa Xaa
           115                 120                 125
   Xaa Xaa Xaa Xaa Xaa Xaa Xaa Xaa Xaa Xaa Xaa Xaa Xaa Xaa Xaa Xaa
       130                 135                 140
   Xaa Xaa Xaa Xaa Xaa Xaa Xaa Xaa Xaa Xaa Xaa Xaa Xaa Xaa Xaa Xaa
   145                 150                 155                 160
   Xaa Xaa Xaa Xaa Xaa Xaa Xaa Xaa Xaa Xaa Xaa Xaa Xaa Xaa Xaa Xaa
                   165                 170                 175
   Xaa Xaa Xaa Xaa Xaa Xaa Xaa Xaa Xaa Xaa Xaa Xaa Xaa Xaa Xaa Xaa
               180                 185                 190
   Xaa Xaa Xaa Xaa Xaa Xaa Xaa Xaa Xaa Xaa Xaa Xaa Xaa Xaa Xaa Xaa
           195                 200                 205
   Xaa Xaa Xaa Xaa Xaa Ala Arg Gly Ala Asp Pro Asn Asn Gln Asp Thr
       210                 215                 220
   Asn Gly Asn Thr Val Leu His Met Leu Val Ile Tyr Glu Lys Ile Ser
   225                 230                 235                 240
   Thr Phe Asp Met Ala Tyr Glu Ile Gly Ala Glu Leu Asn Ile Arg Asn
                   245                 250                 255
   Val Gln Asn Leu Thr Pro Leu Thr Leu Ala Ala Lys Leu Ala Arg Ile
               260                 265                 270
   Gln Met Phe Phe His Ile Leu Lys Ile Glu Arg Glu Ile Tyr Trp Gln
           275                 280                 285
   Ile Gly Ser Ile Thr Cys Ala Ala Tyr Pro Leu Xaa Xaa Xaa Xaa Xaa
       290                 295                 300
   Xaa Xaa Xaa Xaa Xaa Xaa Xaa Xaa Xaa Xaa Xaa Xaa Xaa Xaa Xaa Xaa
   305                 310                 315                 320
   Xaa Xaa Xaa Xaa Xaa Xaa Xaa Xaa His Leu Glu Met Met Asp Gly Val
                   325                 330                 335
   Leu Val Asp Leu Leu Asn Ala Lys Trp Asn Ala Phe Val Lys Phe Arg
               340                 345                 350
   Phe Tyr Arg Gln Phe Ile Leu Phe Ala Phe Tyr Phe Ser Ile Ser Met
           355                 360                 365
   Val Cys Phe Val Leu Arg Pro Gly Pro Pro Pro Thr Gly Leu Lys Pro
       370                 375                 380
   Pro Leu Ile Asn Tyr Thr Ser Thr Ser Thr Thr Val Ala Thr Thr Pro
   385                 390                 395                 400
   Phe Asp Phe Met Asp Asn Glu Ser Met Leu Pro Asp Asn Val Met Tyr
   405 410 415
   Asp Glu Met Met Ile Gly Gly Gly Gly Pro Thr Glu Phe Glu Thr Pro
   420 425 430
   Val Val Thr Thr Glu Met Ala Thr Val Asn Asp Asn Ala Thr Thr Asn
   435 440 445
   Asn Gly Thr Gly Ile Ile Arg Thr Pro Leu Ile Tyr Glu Asp Gly Ala
   450 455 460
   Gly Gly Gly Gly Gly Ser Gly Thr Val Ser Leu His Gly Lys Ser Ser
   465 470 475 480
   Gly Ser Asp Arg Gly Lys His Arg Ser Thr Trp Asn Pro Ala Asn His
   485 490 495
   Thr Phe Lys Thr Asn Tyr Thr Tyr Asn Tyr Asn Lys Ser Arg Lys Phe
   500 505 510
   Trp Trp Asn Lys Arg Gln Lys Val Cys Arg Leu Met Ala Val His Ser
   515 520 525
   Thr Asp Asp Leu Ile Arg Met Gly Ser Glu Val Gly Met Phe Val Gly
   530 535 540
   Ala Phe Leu Tyr Leu Leu Ala Ala Val Arg Glu Ala Gly Phe Leu Gly
   545 550 555 560
   Ser Gln Met Phe Ile Glu Asn Leu Ala Ile Ala Pro Ala Arg Val Met
   565 570 575
   Phe Leu Phe Ser Cys Leu Leu Met Met Thr Ile Pro Pro Leu Arg Leu
   580 585 590
   Thr Cys Tyr Asp Lys Ala Glu Asp Ile Ile Ala Val Ile Ile Met Leu
   595 600 605
   Thr Thr Cys Pro Tyr Phe Leu Phe Phe Cys Arg Gly Xaa Xaa Xaa Xaa
   610 615 620
   Xaa Xaa Xaa Xaa Val Met Ile Tyr Arg Leu Val Met Gly Asp Leu Leu
   625 630 635 640
   Arg Phe Val Ser Ile Tyr Met Val Phe Val Met Gly Phe Ser Gln Ala
   645 650 655
   Tyr Tyr Val Ile Phe Leu Ser Tyr Asp Asn Pro Leu Thr Pro Glu Gly
   660 665 670
   Ile Asp Asp Ser Val Leu Asn Pro Met Pro Thr Pro Thr Glu Ser Ile
   675 680 685
   Met Ala Met Phe Leu Met Ser Val Asn Thr Phe Thr Asp Tyr Tyr Thr
   690 695 700
   Ala Phe Asp Lys Thr Ser His Thr Leu Val Ala Lys Phe Cys Phe Ile
   705 710 715 720
   Val Phe Met Val Ile Val Ala Ile Leu Pro Val Asn Met Leu Ile Ala
   725 730 735
   Met Met Gly Asp Thr Tyr Gln Lys Ile Ala Glu Thr Arg Asn Glu Trp
   740 745 750
   Gln Arg Gln Trp Ala Arg Ile Val Leu Val Val Glu Arg Gly Val Ser
   755 760 765
   Pro Ser Gln Arg Leu Lys Lys Leu Met Tyr Tyr Ser Gln Pro Met Ser
   770 775 780
   Asp Gly Arg Arg Ala Leu Val Leu Arg Leu Asn Gln Thr Asp Glu Asp
   785 790 795 800
   Lys Glu Gln Met Lys Glu Ile Leu Glu Met Lys Arg Ile His Asn Arg
   805 810 815
   Tyr Val Glu Arg Met Arg Ala Arg Asp Leu Ser Ser Lys Leu Ser Ser
   820 825 830
   Ser Cys Gly Lys Asn Ala Asn Asp Gly Thr Ile Asn Leu Lys Ala Asn
   835 840 845
   Lys
<210> 77
   <211> 833
   <212> PRT
   <213> Bemisia tabaci
<220>
   <221> MOD_RES
   <222> (293)..(573)
   <223> Any amino acid
<400> 77
   Asp Pro Glu Lys Ile Asp Pro Lys Lys Cys Arg Glu Val Cys Trp Asp
   1               5                   10                  15
   Leu Met Glu Arg Gly Ala Val Gly Glu Thr Ile Leu His Leu Cys Leu
               20                  25                  30
   Leu Asn Ala Thr Ser Ile His Ala Asp Leu Ala Lys Arg Leu Leu Arg
           35                  40                  45
   Phe Tyr Pro Lys Leu Ile Asn Asp Ile Tyr Met Cys Asp Glu Tyr Tyr
       50                  55                  60
   Gly Glu Ser Val Leu His Ile Ala Ile Val Asn Glu Asp Pro Ala Met
   65                  70                  75                  80
   Val Lys Phe Leu Leu Asp Ser Gly Ala Asp Tyr His Glu Arg Cys Thr
                   85                  90                  95
   Gly Asn Phe Met Cys Pro Glu Asp Gln Lys Ala Thr Arg Gln Asp Thr
               100                 105                 110
   Val Glu His Glu Trp Val Asn Leu Ser Pro Leu Thr Asn Tyr Glu Gly
           115                 120                 125
   Tyr Val Tyr Trp Gly Glu Tyr Pro Leu Ser Phe Ala Ala Cys Leu Gly
       130                 135                 140
   Gln Glu Glu Cys Phe Arg Leu Met Leu Ala Arg Gly Ala Asn Pro Asp
   145                 150                 155                 160
   Asn Gln Asp Thr Asn Gly Asn Thr Val Leu His Met Leu Val Ile Tyr
                   165                 170                 175
   Glu Lys Leu Glu Thr Phe Asp Met Ala Tyr Glu Val Gly Ala Asn Ile
               180                 185                 190
   Ser Val Arg Asn Val Gln Asn Leu Thr Pro Leu Thr Leu Ala Ala Lys
           195                 200                 205
   Leu Ala Arg Ile Glu Met Phe Phe His Ile Leu Asn Ile Glu Arg Glu
       210                 215                 220
   Ile Tyr Trp Gln Ile Gly Ser Ile Ala Cys Ala Ala Tyr Pro Leu Gly
   225                 230                 235                 240
   Gln Ile Asp Thr Ile Asp Ile Glu Thr Gly His Ile Ser Lys Asn Ser
                   245                 250                 255
   Ala Leu Asn Leu Val Val Phe Gly Asp Lys Asp Glu His Leu Glu Leu
   260 265 270
   Met Asp Gly Val Leu Val Asp Leu Leu Asn Ala Lys Trp Asn Ala Phe
   275 280 285
   Val Lys Leu Arg Xaa Xaa Xaa Xaa Xaa Xaa Xaa Xaa Xaa Xaa Xaa Xaa
   290 295 300
   Xaa Xaa Xaa Xaa Xaa Xaa Xaa Xaa Xaa Xaa Xaa Xaa Xaa Xaa Xaa Xaa
   305 310 315 320
   Xaa Xaa Xaa Xaa Xaa Xaa Xaa Xaa Xaa Xaa Xaa Xaa Xaa Xaa Xaa Xaa
   325 330 335
   Xaa Xaa Xaa Xaa Xaa Xaa Xaa Xaa Xaa Xaa Xaa Xaa Xaa Xaa Xaa Xaa
   340 345 350
   Xaa Xaa Xaa Xaa Xaa Xaa Xaa Xaa Xaa Xaa Xaa Xaa Xaa Xaa Xaa Xaa
   355 360 365
   Xaa Xaa Xaa Xaa Xaa Xaa Xaa Xaa Xaa Xaa Xaa Xaa Xaa Xaa Xaa Xaa
   370 375 380
   Xaa Xaa Xaa Xaa Xaa Xaa Xaa Xaa Xaa Xaa Xaa Xaa Xaa Xaa Xaa Xaa
   385 390 395 400
   Xaa Xaa Xaa Xaa Xaa Xaa Xaa Xaa Xaa Xaa Xaa Xaa Xaa Xaa Xaa Xaa
   405 410 415
   Xaa Xaa Xaa Xaa Xaa Xaa Xaa Xaa Xaa Xaa Xaa Xaa Xaa Xaa Xaa Xaa
   420 425 430
   Xaa Xaa Xaa Xaa Xaa Xaa Xaa Xaa Xaa Xaa Xaa Xaa Xaa Xaa Xaa Xaa
   435 440 445
   Xaa Xaa Xaa Xaa Xaa Xaa Xaa Xaa Xaa Xaa Xaa Xaa Xaa Xaa Xaa Xaa
   450 455 460
   Xaa Xaa Xaa Xaa Xaa Xaa Xaa Xaa Xaa Xaa Xaa Xaa Xaa Xaa Xaa Xaa
   465 470 475 480
   Xaa Xaa Xaa Xaa Xaa Xaa Xaa Xaa Xaa Xaa Xaa Xaa Xaa Xaa Xaa Xaa
   485 490 495
   Xaa Xaa Xaa Xaa Xaa Xaa Xaa Xaa Xaa Xaa Xaa Xaa Xaa Xaa Xaa Xaa
   500 505 510
   Xaa Xaa Xaa Xaa Xaa Xaa Xaa Xaa Xaa Xaa Xaa Xaa Xaa Xaa Xaa Xaa
   515 520 525
   Xaa Xaa Xaa Xaa Xaa Xaa Xaa Xaa Xaa Xaa Xaa Xaa Xaa Xaa Xaa Xaa
   530 535 540
   Xaa Xaa Xaa Xaa Xaa Xaa Xaa Xaa Xaa Xaa Xaa Xaa Xaa Xaa Xaa Xaa
   545 550 555 560
   Xaa Xaa Xaa Xaa Xaa Xaa Xaa Xaa Xaa Xaa Xaa Xaa Xaa Ile Met Leu
   565 570 575
   Leu Thr Ser Pro Tyr Phe Leu Phe Phe Cys Arg Gly Phe Lys Thr Val
   580 585 590
   Gly Pro Phe Val Val Met Ile Tyr Arg Met Val Met Gly Asp Leu Leu
   595 600 605
   Arg Phe Val Ser Ile Tyr Met Val Phe Val Met Gly Phe Ser Gln Ala
   610 615 620
   Tyr Tyr Ile Ile Phe Leu Ser Phe Asp Asn Pro Ser Thr Pro Asp Gly
   625 630 635 640
   Val Asp Asp Thr Val Ser Asn Pro Met Pro Asn Pro Val Glu Ala Gly
   645 650 655
   Met Ala Met Phe Leu Met Ser Leu Asn Thr Phe Gly Asp Tyr Phe Ser
   660 665 670
   Ala Phe Glu Lys Thr Glu His Glu Leu Glu Ala Lys Val Cys Phe Val
   675 680 685
   Ile Phe Met Gly Ile Val Ala Ile Leu Leu Val Asn Met Leu Ile Ala
   690 695 700
   Met Met Gly Asn Thr Tyr Gln Lys Ile Ala Glu Thr Arg Asn Glu Trp
   705 710 715 720
   Gln Arg Gln Trp Ala Arg Ile Val Leu Val Val Glu Arg Gly Val Pro
   725 730 735
   Pro Lys Glu Arg Leu Thr Lys Leu Met Trp Tyr Ser Gln Pro Met Ser
   740 745 750
   Asp Gly Arg Arg Ala Leu Val Leu Arg Leu Asn Gln Thr Glu Gln Asp
   755 760 765
   Lys Glu Glu Met Lys Glu Ile Leu Glu Met Lys Arg Val His Asn Arg
   770 775 780
   Ile Val Glu Lys Arg Gln Lys Arg Leu Ala Lys Leu Ser Asn Asn Ala
   785 790 795 800
   Ser Pro Ser Asn Lys Pro Leu Val Asp Leu Pro Asn Leu Ser Asn His
   805 810 815
   Asp Ser Pro Thr Lys Pro Leu Leu Asp Leu Ala Ser Asn Leu Ser Lys
   820 825 830
   Phe
<210> 78
   <211> 748
   <212> PRT
   <213> Euschistus heros
<220>
   <221> MOD_RES
   <222> (102)..(102)
   <223> Any amino acid
<400> 78
   Leu Arg Phe Tyr Pro Lys Leu Ile Asn Asp Val Tyr Met Ser Asp Glu
   1 5 10 15
   Tyr Tyr Gly Glu Ser Val Leu His Ile Ala Ile Val Asn Glu Asp Pro
   20 25 30
   Ala Met Val Lys Phe Leu Leu Asp Ser Gly Val Asn Val Asn Glu Arg
   35 40 45
   Cys Phe Gly Asn Phe Met Cys Pro Glu Asp Gln Lys Ala Ser Arg Thr
   50 55 60
   Asp Ser Leu Asp His Glu Trp Val Asn Leu Gln Ser Phe Thr Thr Tyr
   65 70 75 80
   Glu Gly Tyr Val Tyr Trp Gly Glu Tyr Pro Leu Ser Phe Ala Ala Cys
   85 90 95
   Leu Gly Gln Glu Glu Xaa Tyr Arg Leu Met Leu Ala Arg Gly Ala Asn
   100 105 110
   Pro Asp Asn Gln Asp Thr Asn Gly Asn Thr Val Leu His Met Leu Val
           115                 120                 125
   Ile Tyr Cys Lys Ile Gln Thr Phe Asp Met Ala Tyr Glu Val Gly Gly
       130                 135                 140
   Asp Leu Ser Ile Arg Asn Val Gln Tyr Leu Thr Pro Leu Thr Leu Ala
   145                 150                 155                 160
   Ala Lys Leu Ala Arg Ile Glu Leu Phe Phe His Ile Leu Asn Ile Glu
                   165                 170                 175
   Arg Glu Ile Tyr Trp Gln Ile Gly Ser Ile Thr Cys Ala Ala Tyr Pro
               180                 185                 190
   Leu Ser Gln Ile Asp Thr Ile Asp Ile Val Thr Gly Asn Ile Ser Lys
           195                 200                 205
   Asn Ser Ala Leu Asn Leu Val Val Phe Gly Glu Lys Asp Glu His Leu
       210                 215                 220
   Glu Leu Met Asp Gly Val Leu Ile Asp Leu Leu Asn Ala Lys Trp Asn
   225                 230                 235                 240
   Ala Phe Val Lys Phe Arg Phe Tyr Arg Gln Phe Phe Leu Phe Leu Phe
                   245                 250                 255
   Tyr Phe Leu Ile Ser Leu Ile Cys Phe Thr Leu Arg Pro Gly Pro Pro
               260                 265                 270
   Pro Val Lys Pro Ile Ser Ser Phe Asn Ser Thr Lys Pro Ser Gly Asn
           275                 280                 285
   Asn Ile Thr Ala Glu Leu Ser Pro Glu Ser Asn Ala Thr Thr Val Pro
       290                 295                 300
   Lys Trp Ile Tyr Lys Thr Glu Ile Lys Lys Tyr Asp Pro Glu Asp Asn
   305                 310                 315                 320
   Ser Ser Gln Asn Ser Arg Leu Asn Val Ile Leu Arg Glu Val Ile Leu
                   325                 330                 335
   Thr Ser Leu Lys Val Glu Asp Ile Asp Lys Val Val Ser Phe Ser Asn
               340                 345                 350
   Ala Glu Pro Gln Asp Asn Ser Arg Glu Asp Ala Asp Gly Leu Val Cys
           355                 360                 365
   Asn Gly Asp Ser Val His Ser Cys Leu Gly Asn Gly Ser Asp Val Val
   370 375 380
   Ala Val Phe Gly Lys Gly Lys Ser Pro Lys Val Pro Lys Pro Gly Ala
   385 390 395 400
   Ser Ala Asp Asp Phe Asp Tyr Asp Asp Thr Trp Trp Glu Glu Phe Gly
   405 410 415
   Gln Cys Arg Leu Leu Gln Val Thr Ser Tyr Ile Glu Met Thr Arg Leu
   420 425 430
   Ile Ser Glu Val Leu Leu Asp Ile Gly Ala Leu Leu Tyr Ile Leu Ala
   435 440 445
   Ala Leu Arg Glu Ala Arg Phe Leu Gly Trp Ser Met Phe Val Glu Asn
   450 455 460
   Leu Met Thr Ala Pro Ser Arg Val Met Phe Leu Phe Ser Cys Cys Leu
   465 470 475 480
   Met Leu Thr Met Pro Phe Leu Arg Phe Thr Cys Asn Glu Glu Ile Glu
   485 490 495
   Asp Met Met Ala Val Ile Ile Met Leu Thr Thr Ala Pro Tyr Phe Leu
   500 505 510
   Phe Phe Cys Arg Gly Phe Lys Thr Val Gly Pro Phe Val Val Met Ile
   515 520 525
   Tyr Arg Met Ile Met Gly Asp Leu Leu Arg Phe Ala Thr Ile Tyr Leu
   530 535 540
   Val Phe Val Met Gly Phe Ala Gln Ala Tyr Tyr Ile Ile Phe Leu Ser
   545 550 555 560
   Phe Asp Asn Pro Leu Thr Pro Glu Gly Val Asp Asp Ser Val Ser Asn
   565 570 575
   Pro Ile Pro Asn Pro Met Glu Ala Val Met Ala Met Phe Phe Met Ser
   580 585 590
   Met Thr Ser Phe Gly Asp Tyr Tyr Pro Ala Leu Glu Arg Thr Ala His
   595 600 605
   Glu Phe Cys Ala Lys Leu Cys Phe Val Ile Tyr Met Ala Ile Val Ala
   610 615 620
   Ile Leu Leu Val Asn Met Leu Ile Ala Met Met Gly Asn Thr Tyr Gln
   625 630 635 640
   Lys Ile Ala Glu Thr Arg Asn Glu Trp Gln Arg Gln Trp Ala Arg Ile
   645 650 655
   Val Leu Val Val Glu Arg Gly Val Ser Pro Ser Glu Arg Leu Thr Lys
   660 665 670
   Leu Met Trp Tyr Ser Gln Pro Met Ser Asp Gly Arg Arg Ala Leu Val
   675 680 685
   Leu Arg Leu Asn Gln Ser Glu Glu Asp Lys Glu Glu Met Lys Glu Ile
   690 695 700
   Leu Glu Met Lys Arg Ile His Asn Arg Met Val Gln Lys Arg Lys Glu
   705 710 715 720
   Arg Glu Lys Ser Met Asn Lys Ser Pro Asn Leu Ile Asn Leu Asn Ser
   725 730 735
   Pro Ala Glu Asn Val Lys Asn Thr Asn Pro Phe Ser
   740 745
<210> 79
   <211> 734
   <212> PRT
   <213> Nilaparvata lugens
<220>
   <221> MOD_RES
   <222> (183)..(183)
   <223> Any amino acid
<220>
   <221> MOD_RES
   <222> (322)..(322)
   <223> Any amino acid
<400> 79
   Leu His Met Ala Ile Val Asn Glu Asp Pro Gly Met Val Lys Phe Leu
   1               5                   10                  15
   Leu Asn Ser Gly Ala Asp Phe His Glu Arg Cys Phe Gly Asn Phe Met
               20                  25                  30
   Cys Pro Glu Asp Gln Lys Ala Ser Arg Ser Asp Ser Phe Asp His Glu
           35                  40                  45
   Trp Val Asn Leu Trp Pro Val Thr Asn Tyr Glu Gly Tyr Val Tyr Trp
   50 55 60
   Gly Glu Tyr Pro Leu Ser Phe Ala Ala Cys Leu Gly Gln Glu Glu Cys
   65 70 75 80
   Tyr Arg Leu Met Leu Ala Arg Gly Ala Asn Pro Asp Asn Gln Asp Thr
   85 90 95
   Asn Gly Asn Thr Val Leu His Met Leu Val Ile Tyr Glu Lys Met Gly
   100 105 110
   Thr Phe Asp Thr Ala Tyr Glu Leu Gly Ala Asn Val Ser Val Arg Asn
   115 120 125
   Ile Gln Asn Leu Thr Pro Leu Thr Leu Ala Ala Lys Leu Ala Arg Ile
   130 135 140
   Glu Met Phe Phe His Ile Met Asn Ile Glu Arg Glu Ile Tyr Trp Gln
   145 150 155 160
   Ile Gly Ser Ile Thr Cys Ala Ala Tyr Pro Leu Met Leu Ile Asp Thr
   165 170 175
   Ile Asp Ile Glu Thr Gly Xaa Ile Ser Lys Asp Ser Ala Leu Asn Leu
   180 185 190
   Val Val Phe Gly Asp Lys Asp Glu His Leu Asp Leu Met Glu Gly Val
   195 200 205
   Leu Val Asp Leu Leu His Ala Lys Trp Asn Ala Phe Val Lys Phe Arg
   210 215 220
   Phe Tyr Arg Gln Phe Ile Met Phe Phe Gln Tyr Phe Leu Ile Ser Cys
   225 230 235 240
   Val Cys Phe Ile Leu Arg Pro Gly Pro Pro Glu Ala His Asp Gly Met
   245 250 255
   Asn Ile Thr Ala Asn Ala Thr Leu Asn Leu Ser Phe Gly Val Leu Glu
   260 265 270
   Val Arg Pro Glu Ala Leu Gln Ala Leu Leu Tyr Arg Leu Glu Lys Leu
   275 280 285
   Ser Leu Pro Gln Ser Gly Asp Leu Glu Arg Phe Ile Leu Glu Ser Leu
   290 295 300
   Ala Asp Glu Lys Ser Ile Ser Glu Ala Ala Leu Glu Asn Asp Glu Asn
   305 310 315 320
   Arg Xaa Pro Gly Trp Asn Asn Ser Val Phe Ser Thr Tyr Glu Thr Leu
   325 330 335
   Ala Arg Asp Thr Gly Tyr Gln Thr Asp Glu Ser Pro His Asp Gly Leu
   340 345 350
   Ser Ser Ser Met Asp Tyr Ala Glu Val Ala Glu Arg Leu Thr Lys Leu
   355 360 365
   Met Ile Asp Val Asn Leu Thr Glu Arg Ser Gly Ala Val Asn Glu Ile
   370 375 380
   Ile Leu Lys Ala Leu Pro Ser Leu Glu Glu Lys Glu Asp Gly Leu Glu
   385 390 395 400
   Glu Trp Trp Ser Gly Ile Ala Gly Asp Cys Ile Leu Leu Gln Val Asn
   405 410 415
   Asp Leu Tyr Asp Leu Ala Arg Leu Cys Ala Glu Val Ala Leu Glu Ile
   420 425 430
   Gly Ala Phe Leu Tyr Leu Leu Ala Ala Leu Arg Glu Ala Arg Phe Leu
   435 440 445
   Gly Leu Lys Met Met Ile Glu Asn Leu Met Thr Ala Pro Ser Arg Val
   450 455 460
   Met Phe Leu Phe Ser Cys Cys Leu Met Met Thr Met Pro Phe Phe Arg
   465 470 475 480
   Phe Thr Cys Gln Asp Glu Thr Glu Asp Ile Leu Ala Val Val Ile Met
   485 490 495
   Leu Thr Thr Gly Pro Tyr Phe Leu Phe Phe Cys Arg Gly Phe Lys Thr
   500 505 510
   Val Gly Pro Phe Val Val Met Ile Tyr Arg Met Val Met Gly Asp Leu
   515 520 525
   Leu Arg Phe Val Ser Ile Tyr Met Val Phe Val Met Gly Phe Ser Gln
   530 535 540
   Ala Tyr Tyr Ile Ile Phe Leu Thr Tyr Asp Asn Pro Thr Thr Pro Glu
   545 550 555 560
   Gly Thr Asp Asp Thr Thr Ser Asn Pro Leu Pro Ser Pro Ile Glu Ala
   565 570 575
   Ile Met Ala Met Phe Phe Met Ser Leu Thr Asn Phe Gly Asp Tyr Tyr
   580 585 590
   Ser Ala Phe Glu Asn Thr Glu His Glu Ile Glu Ala Lys Ala Phe Phe
   595 600 605
   Val Ile Tyr Met Val Ile Val Ala Ile Leu Leu Ile Asn Met Leu Ile
   610 615 620
   Ala Met Met Gly Asn Thr Tyr Gln Lys Ile Ala Glu Thr Lys Asn Glu
   625 630 635 640
   Trp Gln Arg Gln Trp Ala Arg Ile Val Leu Val Val Glu Arg Gly Val
   645 650 655
   Ser Pro Lys Glu Arg Leu Lys Lys Leu Met Ser Tyr Ser Gln Pro Met
   660 665 670
   Ser Asp Gly Gly Arg Ala Leu Val Leu Arg Leu Asn Met Thr Glu Glu
   675 680 685
   Asp Lys Glu Glu Met Lys Glu Ile Leu Glu Met Lys Arg Val Arg Asp
   690 695 700
   Lys Ile Val Met Lys Arg Lys Lys Gly Leu Lys Leu Leu Gly Thr Thr
   705 710 715 720
   Thr Pro Ser Pro Lys Arg Met Lys Gly Arg Glu Thr Pro Ile
   725 730
<210> 80
   <211> 162
   <212> PRT
   <213> Schistocerca americana
<400> 80
   Leu His Met Ala Ile Val Asn Glu Asp Pro Ala Met Val Lys Phe Leu
   1               5                   10                  15
   Leu Asp Ser Gly Ala Asn Val His Glu Arg Cys Phe Gly Thr Phe Met
               20                  25                  30
   Ser Pro Glu Asp Gln Lys Ala Gln Arg Ser Asp Ser Leu Asp His Glu
           35                  40                  45
   Trp Val Asn Val Asn Ser Asp Thr Asn Tyr Glu Gly Tyr Val Tyr Trp
       50                  55                  60
   Gly Glu Tyr Pro Leu Ser Phe Ala Ala Cys Leu Gly Gln Glu Glu Cys
   65                  70                  75                  80
   Tyr Arg Leu Val Leu Ala Lys Gly Ala Asn Pro Asp Ser Gln Asp Thr
                   85                  90                  95
   Asn Gly Asn Thr Thr Leu His Met Leu Val Ile His Arg Lys Met Thr
               100                 105                 110
   Gln Phe Asp Met Ala Tyr Glu Val Gly Ser Ser Leu Asp Ile Lys Asn
           115                 120                 125
   Asn Leu Asn Leu Thr Pro Leu Thr Leu Ala Ala Lys Leu Ala Arg Ile
       130                 135                 140
   Glu Met Phe Phe His Ile Leu Asn Ile Glu Arg Glu Ile Tyr Trp Gln
   145                 150                 155                 160
   Ile Gly
<210> 81
   <211> 1123
   <212> PRT
   <213> Drosophila melanogaster
<400> 81
   Met Lys Phe Leu Leu Lys Lys Cys Leu Arg Lys Lys Ala Pro Glu Met
   1               5                   10                  15
   Lys Pro Gly Ala Ile Leu Asp Ala Val Ile Ser Gln Ser Ser Ala Thr
               20                  25                  30
   Ala Cys Lys Cys Leu Leu Tyr Lys Leu Ala Asp Tyr Lys Arg Gly Gly
           35                  40                  45
   Asp Leu Ile Asp Ala Ile Asn Ser Gly Gly Leu Ile Ala Val Glu Gln
       50                  55                  60
   Leu Ile Arg Glu Gln Phe Gly Val Phe Met Tyr Asn Asp Gly Lys Gly
   65                  70                  75                  80
   Gln Val Ile Asn Arg Ala Glu Phe Leu Arg Trp Lys Tyr Arg Asp His
                   85                  90                  95
   Thr Glu Val Thr Ile Pro Ile Glu Ala Ser Leu Ser Ile His Asp Pro
   100 105 110
   Leu Gly Lys Trp Glu Asp His Lys Ala Cys Trp Gln Met Gln Tyr Arg
   115 120 125
   Gly Ala Leu Gly Glu Ser Leu Leu His Val Leu Ile Ile Cys Asp Ser
   130 135 140
   Lys Val His Thr Lys Leu Ala Arg Val Leu Leu Arg Val Phe Pro Asn
   145 150 155 160
   Leu Ala Leu Asp Val Met Glu Gly Glu Glu Tyr Leu Gly Ala Ser Ala
   165 170 175
   Leu His Leu Ser Ile Ala Tyr Ser Asn Asn Glu Leu Val Ala Asp Leu
   180 185 190
   Ile Glu Ala Gly Ala Asp Ile His Gln Arg Ala Ile Gly Ser Phe Phe
   195 200 205
   Leu Pro Arg Asp Gln Gln Arg Ala Asn Pro Ala Lys Ser Thr Asp Tyr
   210 215 220
   Glu Gly Leu Ala Tyr Met Gly Glu Tyr Pro Leu Ala Trp Ala Ala Cys
   225 230 235 240
   Cys Ala Asn Glu Ser Val Tyr Asn Leu Leu Val Asp Cys Gly Ser Asp
   245 250 255
   Pro Asp Ala Gln Asp Ser Phe Gly Asn Met Ile Leu His Met Val Val
   260 265 270
   Val Cys Asp Lys Leu Asp Met Phe Gly Tyr Ala Leu Arg His Pro Lys
   275 280 285
   Thr Pro Ala Lys Asn Gly Ile Val Asn Gln Thr Gly Leu Thr Pro Leu
   290 295 300
   Thr Leu Ala Cys Lys Leu Gly Arg Ala Glu Val Phe Arg Glu Met Leu
   305 310 315 320
   Glu Leu Ser Ala Arg Glu Phe Trp Arg Tyr Ser Asn Ile Thr Cys Ser
   325 330 335
   Gly Tyr Pro Leu Asn Ala Leu Asp Thr Leu Leu Pro Asp Gly Arg Thr
   340 345 350
   Asn Trp Asn Ser Ala Leu Phe Ile Ile Leu Asn Gly Thr Lys Pro Glu
   355 360 365
   His Leu Asp Met Leu Asp Gly Gly Ile Ile Gln Arg Leu Leu Glu Glu
   370 375 380
   Lys Trp Lys Thr Phe Ala Gln Asn Gln Phe Leu Lys Arg Leu Leu Ile
   385 390 395 400
   Leu Ser Thr His Leu Leu Cys Leu Ser Val Ser Val Tyr Leu Arg Pro
   405 410 415
   Ala His Asp Gly Glu Ala Glu Asp Glu Asp Ser Glu Gly Ser Asp Ala
   420 425 430
   Ser Ala Ala Ala Leu Leu Asp Ile Gln Ser Asp Glu Gly Asp Ser Gly
   435 440 445
   Gly Gly Asp Tyr Asn Ala Gln Thr Val Ala Arg Tyr Cys Ala Glu Phe
   450 455 460
   Ala Thr Leu Val Gly Val Leu Ser Tyr Val Ile Phe Gln Gln Gly Asp
   465 470 475 480
   Glu Ile Lys Asn Gln Gly Leu Ser Ala Phe Leu Lys Gln Leu Ser His
   485 490 495
   Ala Pro Ala Lys Ala Ile Phe Leu Phe Ser Asn Leu Leu Ile Leu Ala
   500 505 510
   Cys Ile Pro Phe Arg Leu Ile Gly Asp Thr Asp Thr Glu Glu Ala Ile
   515 520 525
   Leu Ile Phe Ala Val Pro Gly Ser Trp Phe Leu Leu Met Phe Phe Ala
   530 535 540
   Gly Ala Ile Arg Leu Thr Gly Pro Phe Val Thr Met Ile Tyr Ser Met
   545 550 555 560
   Ile Thr Gly Asp Met Phe Thr Phe Gly Ile Ile Tyr Cys Ile Val Leu
   565 570 575
   Cys Gly Phe Ser Gln Ala Phe Tyr Phe Leu Tyr Lys Gly His Pro Gln
   580 585 590
   Val Gln Ser Thr Met Phe Asn Thr Tyr Thr Ser Thr Trp Met Ala Leu
   595 600 605
   Phe Gln Thr Thr Leu Gly Asp Tyr Asn Tyr Pro Asp Leu Asn Gln Thr
   610 615 620
   Thr Tyr Pro Asn Leu Ser Lys Thr Val Phe Val Ile Phe Met Ile Phe
   625 630 635 640
   Val Pro Ile Leu Leu Leu Asn Met Leu Ile Ala Met Met Gly Asn Thr
   645 650 655
   Tyr Val Thr Val Ile Glu Gln Ser Glu Lys Glu Trp Met Lys Gln Trp
   660 665 670
   Ala Lys Ile Val Val Thr Leu Glu Arg Ala Val Pro Gln Ala Asp Ala
   675 680 685
   Lys Gly Tyr Leu Glu Ala Tyr Ser Ile Pro Leu Gly Pro Ser Asp Asp
   690 695 700
   Ser Gly Phe Glu Val Arg Gly Val Met Val Ile Lys Ser Lys Ser Lys
   705 710 715 720
   Thr Arg Ala Lys Gln Arg Lys Gly Ala Val Ser Asn Trp Lys Arg Val
   725 730 735
   Gly Arg Val Thr Leu Thr Ala Leu Lys Lys Arg Gly Met Thr Gly Glu
   740 745 750
   Glu Met Arg Arg Leu Met Trp Gly Arg Ala Ser Ile Ser Ser Pro Val
   755 760 765
   Lys Val Thr Lys Gln Lys Leu Lys Asp Pro Tyr Asn Leu His Thr Asp
   770 775 780
   Ser Asp Phe Thr Asn Ala Met Asp Met Leu Thr Phe Ala Ser Asn Pro
   785 790 795 800
   Ala Ser Ser Asn Gly Val Thr Leu Arg Ser Val Thr Ala Pro Pro Pro
   805 810 815
   Ala Pro Pro Ala Pro Asp Pro Phe Arg Glu Leu Ile Met Met Ser Asp
   820 825 830
   Gln Arg Pro Glu Thr His Asp Pro His Tyr Phe Ala Gly Leu Gln Gln
   835 840 845
   Leu Ala Asn Lys Ala Phe Asp Leu Val Glu Gln Thr Met Lys Thr Gln
       850                 855                 860
   Pro Gln Ala Pro Val Ala Lys Lys Val Asp Pro Leu Pro Val Ala Ser
   865                 870                 875                 880
   Val Ala Lys Ala Ser Pro Ala Ala Pro Ala Thr Gln Ala Thr Ala Thr
                   885                 890                 895
   Ala Ala Ala Ala Ser Asp Leu Met Ala Met Pro Leu Pro Ile Ser Asn
               900                 905                 910
   Leu Ser Asn Leu Phe Gln Asp Pro Lys Asp Ile Val Asp Pro Lys Lys
           915                 920                 925
   Leu Glu Glu Phe Met Ala Met Leu Ala Glu Val Glu Thr Glu Glu Ser
       930                 935                 940
   Asp Ser Gly Gly Pro Ile Leu Gly Lys Leu Ser Leu Ala Lys Arg Thr
   945                 950                 955                 960
   His Asn Ala Leu Ser Lys Ala Glu Ile Arg Arg Asp Gln Gln Gly Phe
                   965                 970                 975
   Glu Gly His Ser His Gly Gln Phe Gln Pro Met Ser Ser Val Trp Ala
               980                 985                 990
   Pro Pro Gly Leu Asp Val Asp Thr  Gly Phe His Phe Asp  Glu Ala Val
           995                 1000                 1005
   Ala Glu  Glu Val Leu Thr Ile  Glu Gln Glu Ala Glu  Val Glu Thr
       1010                 1015                 1020
   Glu Asp  Gly Asn Gly Gly Gln  Asp Ser Glu Asp Ile  Pro Thr Ala
       1025                 1030                 1035
   Glu Glu  Val His Ala Thr Met  Lys Gln Phe His Leu  Arg Lys Cys
       1040                 1045                 1050
   Gln Pro  Ala Gln Asp Glu Ala  Ala Arg Arg Ala Lys  Ser Ala Arg
       1055                 1060                 1065
   Val Arg  Arg Arg Asn Lys Val  Ser Pro Glu Gln Ser  Asp Asp Pro
       1070                 1075                 1080
   Asp Glu  Arg Ser Gln Arg Gly  Arg Ser Ala Tyr Thr  Arg Arg Thr
       1085                 1090                 1095
   Gln Ser Pro Pro Asp Pro Leu Glu Pro Trp Ser Thr Arg Glu Leu
   1100 1105 1110
   Gln Asp Ile Asn Lys Ile Leu Ala Arg Lys
   1115 1120
<210> 82
   <211> 1128
   <212> PRT
   <213> Musca domestica
<400> 82
   Met Arg Leu Phe Met Lys Lys Cys Leu Arg Lys Lys Pro Gln Glu Leu
   1 5 10 15
   Lys Pro Gly Ala Ile Leu Asp Ala Val Ile Ser Gln Ser Ser Ala Thr
   20 25 30
   Ala Ser Lys Cys Leu Leu Tyr Lys Leu Ala Asp Tyr Lys Arg Gly Gly
   35 40 45
   Asp Leu Ile Asp Ala Ile Asn Thr Gly Gly Leu Val Ala Val Glu Gln
   50 55 60
   Leu Ile Arg Glu Gln Phe Gly Ile Phe Met Tyr Asn Asp Gly Lys Gly
   65 70 75 80
   Gln Val Ile Asn Arg Ala Glu Phe Leu Arg Trp Lys Tyr Arg Asp His
   85 90 95
   Thr Glu Val Thr Ile Pro Ile Glu Ala Ser Leu Ser Ile His Asp Pro
   100 105 110
   Leu Gly Lys Trp Lys Asp His Ser Ala Cys Trp Gln Met Gln Tyr Arg
   115 120 125
   Gly Ala Leu Gly Glu Ser Leu Leu His Val Leu Ile Ile Cys Asp Ser
   130 135 140
   Lys Met His Thr Lys Leu Ala Arg Ile Leu Leu Arg Val Phe Pro Arg
   145 150 155 160
   Leu Ala Leu Asp Val Ile Glu Gly Glu Glu Tyr Leu Gly Ala Ser Ala
   165 170 175
   Leu His Leu Ala Ile Ala Tyr Ser Asn Asn Glu Leu Val Ala Asp Leu
   180 185 190
   Ile Glu Ala Gly Ala Asp Ile Asn Gln Arg Ala Ile Gly Ser Phe Phe
           195                 200                 205
   Leu Pro Lys Asp Gln Gln Arg Ala Asn Pro Ala Lys Ser Thr Asp Tyr
       210                 215                 220
   Glu Gly Leu Ala Tyr Met Gly Glu Tyr Pro Leu Ser Trp Ala Ala Cys
   225                 230                 235                 240
   Cys Ala Asn Glu Ser Val Tyr Asn Leu Leu Ile Asp His Asp Ala Asp
                   245                 250                 255
   Pro Asp Ala Gln Asp Ser Phe Gly Asn Met Ile Leu His Met Val Val
               260                 265                 270
   Val Cys Asp Lys Leu Asp Met Phe Gly Tyr Ala Leu Arg His Pro Lys
           275                 280                 285
   Thr Pro Ala Lys Asn Gly Ile Val Asn His Ser Gly Leu Thr Pro Leu
       290                 295                 300
   Thr Leu Ala Cys Lys Leu Gly Arg Ala Glu Val Phe Arg Glu Met Leu
   305                 310                 315                 320
   Glu Leu Ser Ala Arg Glu Phe Trp Arg Tyr Ser Asn Ile Thr Cys Ser
                   325                 330                 335
   Gly Tyr Pro Leu Asn Ala Leu Asp Thr Leu Leu Pro Asp Gly Arg Thr
               340                 345                 350
   Asn Trp Asn Ser Ala Leu Phe Ile Ile Leu Asn Gly Thr Lys Glu Glu
           355                 360                 365
   His Leu Asp Met Leu Asp Gly Gly Ile Ile Gln Arg Leu Leu Glu Glu
       370                 375                 380
   Lys Trp Lys Thr Phe Ala Gln Asn Gln Phe Leu Lys Arg Leu Leu Ile
   385                 390                 395                 400
   Leu Val Val His Leu Leu Cys Leu Ser Thr Ser Val Tyr Leu Arg Pro
                   405                 410                 415
   Ala His Asp Gly Asp Asp Asp Glu Glu Asp Glu Thr Ala Gln Ala Asn
               420                 425                 430
   Ala Gly Leu Gln Thr Asn Asp Pro Glu Asn Glu Glu Tyr Asp Ile Gln
           435                 440                 445
   Thr Ile Val Arg Tyr Cys Ala Glu Leu Cys Thr Ile Ala Gly Val Leu
       450                 455                 460
   Ser Phe Leu Ile Phe Gln Gln Gly Asp Glu Ile Lys Asn Gln Gly Leu
   465                 470                 475                 480
   Ser Ala Phe Leu Lys Gln Leu Ser His Ala Pro Ala Lys Val Ile Phe
                   485                 490                 495
   Leu Val Ser Asn Leu Met Ile Leu Ala Cys Ile Pro Phe Arg Leu Met
               500                 505                 510
   Gly Asp Thr Asp Thr Glu Glu Ala Ile Leu Ile Phe Ala Val Pro Gly
           515                 520                 525
   Ser Trp Phe Leu Leu Met Phe Phe Ala Gly Ala Ile Arg Leu Thr Gly
       530                 535                 540
   Pro Phe Val Thr Met Ile Tyr Ser Met Ile Thr Gly Asp Met Phe Thr
   545                 550                 555                 560
   Phe Gly Ile Ile Tyr Ser Ile Val Leu Cys Gly Phe Ser Gln Ala Phe
                   565                 570                 575
   Tyr Phe Leu Tyr Lys Gly His Pro Gln Leu Gln Ser Thr Met Phe Asp
               580                 585                 590
   Thr Phe Pro Ser Thr Trp Met Ala Leu Phe Gln Thr Thr Leu Gly Asp
           595                 600                 605
   Tyr Asn Tyr Pro Asp Leu Asn Gln Thr Thr Tyr Pro Asn Leu Ser Lys
       610                 615                 620
   Thr Val Phe Val Ile Phe Met Ile Phe Val Pro Ile Leu Leu Leu Asn
   625                 630                 635                 640
   Met Leu Ile Ala Met Met Gly Asn Thr Tyr Ala His Val Ile Glu Gln
                   645                 650                 655
   Ser Glu Lys Glu Trp Met Lys Gln Trp Ala Lys Ile Val Val Thr Leu
               660                 665                 670
   Glu Arg Ala Val Pro Gln Ala Asp Ala Lys Asn Tyr Leu Glu Ala Tyr
           675                 680                 685
   Ser Ile Pro Leu Gly Pro Ser Asp Asp Ser Gly Phe Glu Val Arg Gly
       690                 695                 700
   Val Met Val Ile Lys Ser Lys Ser Lys Thr Arg Ala Lys Gln Arg Lys
   705 710 715 720
   Gly Ala Val Ser Asn Trp Lys Arg Val Gly Arg Val Thr Leu Asn Ala
   725 730 735
   Leu Lys Lys Arg Gly Met Thr Gly Glu Glu Met Arg Arg Leu Met Trp
   740 745 750
   Gly Arg Ala Ser Ile Ser Ser Pro Ile Lys Val Ala Lys Lys Lys Leu
   755 760 765
   Lys Asp Pro Tyr Asn Leu Asn Gln Glu Pro Gly Gly Gln Ser Asn Leu
   770 775 780
   Thr Ser Ala Met Asp Met Leu Asn Phe Ala Asn Asp Pro Ala Ser Ser
   785 790 795 800
   Ser Gly Leu Ala Phe Arg Thr Ser Gly Thr Gln Asp Pro Lys Glu Lys
   805 810 815
   Lys Asp Val Pro His Ile Pro Ala Pro Asp Pro Leu Arg Asp Leu Val
   820 825 830
   Leu Leu Ala Glu Glu Lys Pro Glu Val His Asp Pro Gln Phe Tyr Ser
   835 840 845
   Ala Leu Ile Gln Leu Ala Asn Lys Ala Tyr Asp Leu Val Lys Lys Thr
   850 855 860
   Leu Glu Thr Gln His Gln Pro Gln Val Glu Glu Pro Ile Pro Leu Thr
   865 870 875 880
   Glu Ala Thr Ser Leu Lys Asp Val Thr Leu Thr Lys Gln Pro Asp Pro
   885 890 895
   Val Ala Ala Asp Ala Gly Leu Leu Gly Asp Met Ala Met Gly Ala Asn
   900 905 910
   Leu Thr Asn Leu Phe Gln Asp Pro Lys Asp Ile Val Asp Pro Lys Lys
   915 920 925
   Leu Glu Gln Phe Leu Lys Met Val Ala Asp Val Glu Thr Glu Glu Ser
   930 935 940
   Asp Gly Gly Gly Pro Ile Leu Gly Lys Leu Ser Leu Ala Arg Arg Thr
   945 950 955 960
   Arg Asn Ala Leu Phe Lys Val Pro Ile Pro Lys Ser Thr Leu Gly Asp
   965 970 975
   Ser Ser Asp Lys Leu Ile Asn Ser Val Trp Asp Lys Val Pro Ala Asp
   980 985 990
   Asn Asp Glu Asp Ser Ile Pro Ala Phe Asp Phe Gln Glu Ala Leu Ala
   995 1000 1005
   Glu His Val Leu Thr Val Glu Gln Glu Ala Asp Val Glu Thr Glu
   1010 1015 1020
   Asp Ala Asn Asp Asp Ser Gly Glu Thr Met Asp Asp Ile Pro Thr
   1025 1030 1035
   Ala Glu Glu Val His Ala Thr Met Lys Gln Phe His Leu Arg Lys
   1040 1045 1050
   Arg Gln Tyr Gln Gln Asp Asp Ala Ala Arg Arg Ala Lys Thr Ala
   1055 1060 1065
   Arg Ile Arg Arg Lys Asn Lys Val Ala Pro Asp Tyr Ala Gly Ser
   1070 1075 1080
   Glu Glu Gln Tyr Gly Arg Gly Arg Ser Ala Pro Met Lys Lys Lys
   1085 1090 1095
   His Asp Glu Asp Glu Met Ser Pro Pro Asp Pro Leu Glu Pro Trp
   1100 1105 1110
   Ser Thr Arg Glu Leu Gln Asn Ile Asn Lys Ile Leu Ala Lys Lys
   1115 1120 1125
<210> 83
   <211> 1144
   <212> PRT
   <213> Ceratitis capitata
<400> 83
   Met Lys Phe Phe Leu Lys Lys Cys Leu Arg Lys Lys Pro Glu Glu Met
   1               5                   10                  15
   Lys Pro Gly Ala Ile Leu Asp Ala Val Ile Ser Gln Ser Ser Pro Met
               20                  25                  30
   Ala Asn Lys Cys Leu Leu Tyr Lys Leu Ala Asp Tyr Met Arg Gly Gly
           35                  40                  45
   Asp Leu Ile Asp Ala Leu Asn Thr Gly Gly Leu Val Ala Val Glu Gln
       50                  55                  60
   Leu Ile Arg Glu Gln Phe Gly Ile Phe Met Tyr Asn Asp Gly Lys Gly
   65                  70                  75                  80
   Gln Val Ile Asn Arg Ala Glu Phe Leu Arg Trp Lys Tyr Arg Asp His
                   85                  90                  95
   Thr Glu Val Thr Ile Pro Ile Glu Ala Ser Leu Ser Arg His Asp Pro
               100                 105                 110
   Leu Gly Lys Trp Glu Asp His Lys Ala Cys Trp Gln Met Gln Phe Arg
           115                 120                 125
   Gly Ala Leu Gly Glu Ser Leu Leu His Val Leu Ile Ile Cys Asp Ser
       130                 135                 140
   Lys Ile His Thr Lys Leu Ala Arg Val Leu Ile Arg Val Phe Pro Asn
   145                 150                 155                 160
   Leu Ala Gln Asp Val Met Glu Gly Glu Glu Tyr Leu Gly Ala Ser Ala
                   165                 170                 175
   Leu His Leu Ala Ile Ala Tyr Ser Asn Asn Glu Leu Val Ala Asp Leu
               180                 185                 190
   Ile Glu Ala Gly Ala Asp Ile Asn Gln Arg Ala Ile Gly Ser Phe Phe
           195                 200                 205
   Leu Pro Arg Asp Gln Gln Arg Arg Asn Pro Ala Lys Ser Thr Asp Tyr
       210                 215                 220
   Glu Gly Leu Ala Tyr Leu Gly Glu Tyr Pro Leu Ala Trp Ala Ala Cys
   225                 230                 235                 240
   Cys Ala Asn Glu Ser Val Tyr Asn Leu Leu Val Asp Cys Gly Ala Asp
                   245                 250                 255
   Pro Asp Ala Gln Asp Ser Phe Gly Asn Met Ile Leu His Met Val Val
               260                 265                 270
   Val Cys Asp Lys Leu Asp Met Phe Gly Tyr Ala Leu Arg His Pro Lys
           275                 280                 285
   Thr Pro Ala Lys Asn Gly Ile Ala Asn His Ser Gly Leu Thr Pro Leu
   290 295 300
   Thr Leu Ser Cys Lys Leu Gly Arg Ala Glu Val Phe Arg Glu Met Leu
   305 310 315 320
   Glu Leu Ser Ala Arg Glu Phe Trp Arg Tyr Ser Asn Ile Thr Cys Ser
   325 330 335
   Gly Tyr Pro Leu Asn Ala Leu Asp Thr Leu Leu Pro Asp Gly Arg Thr
   340 345 350
   Asn Trp Asn Ser Ala Leu Phe Ile Ile Leu Asn Gly Thr Lys Glu Glu
   355 360 365
   His Leu Asp Met Leu Asp Gly Gly Ile Ile Gln Arg Leu Leu Glu Glu
   370 375 380
   Lys Trp Lys Thr Phe Ala Gln Asn Gln Phe Leu Lys Arg Leu Leu Ile
   385 390 395 400
   Leu Val Ile His Leu Val Phe Leu Ser Ile Ser Val Tyr Met Arg Pro
   405 410 415
   Ala Arg Val Ala Asp Asp Asp Asp Glu Asp Glu Ile Asp Ala Ser Ser
   420 425 430
   Lys Asp Thr Lys Ala Leu Glu Leu Ala Thr Glu Leu Asp Glu Asp Glu
   435 440 445
   Tyr Asp Val Gln Thr Ile Val Arg Tyr Cys Ala Glu Phe Gly Thr Ile
   450 455 460
   Val Gly Val Leu Ser Tyr Val Ile Phe Gln Gln Gly Asp Glu Ile Lys
   465 470 475 480
   Asn Gln Gly Leu Pro Ala Phe Leu Lys Gln Leu Thr His Ala Pro Ala
   485 490 495
   Lys Ala Ile Phe Leu Val Ser Asn Leu Leu Ile Leu Ala Cys Ile Pro
   500 505 510
   Cys Arg Leu Met Gly Asp Thr Asp Ala Glu Glu Ala Ile Leu Ile Phe
   515 520 525
   Ala Val Pro Gly Ser Trp Phe Leu Leu Met Phe Phe Ala Gly Ala Ile
   530 535 540
   Arg Leu Thr Gly Pro Phe Val Thr Met Ile Tyr Ser Met Ile Thr Gly
   545                 550                 555                 560
   Asp Met Phe Thr Phe Gly Ile Ile Tyr Cys Ile Val Leu Cys Gly Phe
                   565                 570                 575
   Ser Gln Ala Phe Tyr Phe Leu Tyr Lys Gly His Pro Gln Ile Gln Ser
               580                 585                 590
   Thr Met Phe Asn Thr Phe Pro Ser Thr Trp Met Ala Leu Phe Gln Thr
           595                 600                 605
   Thr Leu Gly Asp Tyr Asn Tyr Pro Asp Leu Asn Asn Thr Thr Tyr Pro
       610                 615                 620
   Asn Leu Ser Lys Thr Val Phe Val Ile Phe Met Ile Phe Val Pro Ile
   625                 630                 635                 640
   Leu Leu Leu Asn Met Leu Ile Ala Met Met Gly Asn Thr Tyr Ala Gln
                   645                 650                 655
   Val Ile Glu Arg Ser Glu Lys Glu Trp Met Lys Gln Trp Ala Lys Ile
               660                 665                 670
   Val Val Thr Leu Glu Arg Ala Val Pro Gln Ala Asp Ala Lys Asn Tyr
           675                 680                 685
   Leu Glu Ala Tyr Ser Ile Pro Leu Gly Pro Ile Asp Asp Ser Gly Tyr
       690                 695                 700
   Glu Val Arg Gly Val Met Val Ile Lys Ser Lys Ala Lys Thr Arg Ala
   705                 710                 715                 720
   Lys Gln Arg Lys Gly Ala Val Ser Asn Trp Lys Arg Val Gly Arg Val
                   725                 730                 735
   Thr Leu Asn Ala Leu Lys Lys Arg Gly Met Thr Gly Glu Gln Met Arg
               740                 745                 750
   Arg Leu Met Trp Gly Arg Ala Ser Ile Ser Ser Pro Ile Lys Ile Thr
           755                 760                 765
   Lys Lys Lys Leu Lys Asp Pro Tyr Asn Leu Asn Pro Gln Asn Asp Leu
       770                 775                 780
   Thr Ser Ala Met Asp Met Leu Thr Phe Ala Asn Asp Pro Ala Ala Ser
   785                 790                 795                 800
   Ala Gly Leu Gln Leu Arg Ser Ser Leu Met Asp Ser Gly Ala Glu Gly
   805 810 815
   Lys Ala Ala Lys Val Gln Glu Glu Asn Gln Gln Pro Ala Pro Asp Pro
   820 825 830
   Leu Arg Asp Leu Ile Phe Leu Ala Asp Arg Arg Pro Glu Met His Asp
   835 840 845
   Pro Gln Tyr Phe Val Gly Leu Gln Gln Leu Ala Asn Gln Ala Leu Asp
   850 855 860
   Leu Val Glu Gln Thr Met Gly Ile His Pro Pro Pro Thr Pro Ala Thr
   865 870 875 880
   Asp Ala Leu Leu Leu Ser Ala Ala Ala Ala Thr Thr Thr Ile Gly Ala
   885 890 895
   Val Thr Met Gln Thr Ala Thr Thr Val Ala Ala Pro Ser Ala Asp Leu
   900 905 910
   Ile Thr Pro Leu Gly Ala Asn Leu Thr Thr Leu Phe Gln Asp Asn Lys
   915 920 925
   Asp Val Val Asp Pro Gln Lys Leu Gln Glu Phe Leu Lys Met Leu Ala
   930 935 940
   Glu Ile Glu Thr Glu Glu Ser Asp Gly Gly Gly Arg Pro Ile Leu Gly
   945 950 955 960
   Lys Leu Ser Leu Ser Arg Arg Thr Lys Ser Ala Leu Ser Lys Ala Gln
   965 970 975
   Ile Lys Lys Asp Thr Val Gly Gly Ser Pro Asp Lys Leu Ile Pro Ser
   980 985 990
   Val Trp Ser His Asn Leu Pro Gln Pro Asp Glu Glu Pro Glu Phe Asn
   995 1000 1005
   Phe Glu Ala Ala Leu Ala Glu Glu His Thr Leu Thr Ile Glu Gln
   1010 1015 1020
   Glu Ala Glu Val Glu Thr Glu Thr Gly Asn Glu Arg Glu Asp Ser
   1025 1030 1035
   Glu Asp Cys Pro Thr Val Glu Glu Val His Ala Thr Met Lys Gln
   1040 1045 1050
   Phe His Leu Arg Lys Arg Gln Tyr Gln Gln Asp Asp Ala Ala Arg
   1055 1060 1065
   Arg Ala Lys Ser Ala Arg Ile Lys Arg Lys Asn Arg Ile Ser Pro
   1070 1075 1080
   Glu Gln Ser His Glu Cys Asp Asp Gly Ser Ser Gly Gly Gly Gly
   1085 1090 1095
   Gly Lys Ser Thr His Pro Arg Gly Ala Ser Ala Pro Gly Arg Lys
   1100 1105 1110
   His Thr Val Ser Glu Arg Leu Ser Pro Pro Asp Pro Leu Glu Pro
   1115 1120 1125
   Trp Ser Thr Arg Glu Leu Gln Asn Ile Asn Lys Ile Leu Ala Arg
   1130 1135 1140
   Lys
<210> 84
   <211> 1120
   <212> PRT
   <213> Aedes aegypti
<400> 84
   Met Val Gln Phe Asp Trp Arg Arg Met Cys Arg Lys Lys Arg Lys Val
   1 5 10 15
   Pro Gln Gly Gly Ala Ile Leu Asp Gln Val Ile Ser Gln Ser Ala Asn
   20 25 30
   Ala Ser Asn Gln Cys Leu Leu Tyr Lys Met Ala Asn Tyr Lys Arg Gly
   35 40 45
   Gly Asp Leu Ile Asp Ala Phe Gln Val Gly Gly Gln Lys Ala Val Glu
   50 55 60
   Gln Leu Ile Arg Glu Gln Phe Gly Val Phe Met Tyr Asn Asn Gly Arg
   65 70 75 80
   Gly Gln Ile Ile Asn Arg Ala Glu Tyr Leu Arg Trp Lys Tyr Met Asp
   85 90 95
   Asn His Glu Val Val Ile Pro Ile Glu Ala Ser Leu Ser Pro His Asp
   100 105 110
   Pro Leu Gly Lys Trp Val Asp His Lys Ala Cys Trp Gln Met Gln Tyr
   115 120 125
   Arg Gly Leu Leu Gly Glu Ser Leu Leu His Val Leu Ile Ile Cys Asp
   130 135 140
   Thr Lys Ile His Thr Lys Leu Ala Arg Ile Leu Leu Arg Val Phe Pro
   145 150 155 160
   Glu Leu Ser Ile Asp Val Met Glu Gly Glu Glu Tyr Leu Gly Ala Ser
   165 170 175
   Ala Leu His Leu Ala Ile Ala Tyr Ser Asn Asn Glu Leu Val Gly Asp
   180 185 190
   Leu Ile Asp Ala Gly Ala Asp Val Ser Gln Arg Ala Ile Gly Arg Phe
   195 200 205
   Phe Leu Pro Arg Asp Gln Gln Gly Leu Lys Pro Ala Lys Asn Thr Asp
   210 215 220
   Tyr Glu Gly Leu Ala Tyr Leu Gly Glu Tyr Pro Leu Ala Trp Ala Ala
   225 230 235 240
   Cys Cys Ala Asn Glu Ser Val Tyr Asn Leu Leu Leu Glu Cys Gly Ala
   245 250 255
   Asp Pro Asn Ala Gln Asp Ser Phe Gly Asn Met Ile Leu His Met Val
   260 265 270
   Val Val Cys Asp Lys Leu Asp Met Phe Gly Tyr Ala Leu Arg His Pro
   275 280 285
   Lys Leu Pro Cys Lys Asn Gly Ile Val Asn Glu Ser Gly Leu Thr Pro
   290 295 300
   Leu Thr Leu Ala Cys Arg Leu Gly Arg Asp Glu Val Phe Arg Glu Met
   305 310 315 320
   Leu Glu Leu Ser Ala Arg Glu Phe Trp Arg Tyr Ser Asn Ile Thr Cys
   325 330 335
   Ser Gly Tyr Pro Leu Asn Ala Leu Asp Thr Leu Met Pro Asp Gly Thr
   340 345 350
   Thr Asn Trp Asn Ser Ala Leu Phe Ile Ile Leu Asn Gly Thr Lys Glu
   355 360 365
   Glu His Leu Asn Met Leu Asp Gly Gly Ile Val Glu Arg Leu Leu Asp
       370                 375                 380
   Glu Lys Trp Lys Thr Phe Ala Arg Asn Gln Phe Ile Lys Arg Leu Leu
   385                 390                 395                 400
   Ile Leu Ala Leu His Leu Phe Cys Leu Ser Cys Ser Val Tyr Leu Arg
                   405                 410                 415
   Pro Val Arg Val Phe Lys Glu Glu Ser Glu Glu Gly Ser Asp Gly Gly
               420                 425                 430
   Asp Gly Ala Gly Thr Ala Ala Pro Asp Gly Val Asp Asp Asp Glu Asp
           435                 440                 445
   Ile Asp Leu Thr Thr Trp Phe Arg Tyr Gly Phe Glu Ile Ala Thr Val
       450                 455                 460
   Met Gly Val Leu Ser Tyr Val Val Leu Gln Gln Gly Asp Glu Ile Lys
   465                 470                 475                 480
   Asn Gln Gly Leu Ile Ser Phe Ile Lys Ser Leu Gly Asn Ala Pro Ala
                   485                 490                 495
   Lys Ala Ile Phe Leu Ile Ser Asn Leu Met Ile Leu Ala Cys Ile Pro
               500                 505                 510
   Phe Arg Met Met Gly Asp Val Glu His Glu Glu Ala Ile Leu Leu Phe
           515                 520                 525
   Ala Val Pro Gly Ser Trp Phe Leu Leu Met Phe Phe Ala Gly Ala Ile
       530                 535                 540
   Gly Leu Thr Gly Pro Phe Val Thr Met Ile Phe Ser Met Ile Thr Gly
   545                 550                 555                 560
   Asp Met Phe Thr Phe Gly Ile Ile Tyr Met Ile Val Leu Phe Gly Phe
                   565                 570                 575
   Ser Gln Ala Phe Tyr Phe Leu Tyr Lys Gly His Pro Glu Ala Glu Asp
               580                 585                 590
   Ser Pro Phe Gly Ser Tyr Phe Gly Thr Trp Met Gly Leu Phe Gln Thr
           595                 600                 605
   Thr Leu Gly Asp Tyr Asp Tyr Ala Asp Leu Asn Leu Thr Thr Tyr Pro
   610 615 620
   Asn Leu Ala Lys Thr Val Phe Ile Ile Phe Met Ile Phe Val Pro Ile
   625 630 635 640
   Leu Leu Leu Asn Met Leu Ile Ala Met Met Gly Asn Thr Tyr Ala Tyr
   645 650 655
   Val Ile Glu Gln Ala Glu Lys Glu Gly Met Lys Gln Trp Ala Lys Ile
   660 665 670
   Val Val Asn Leu Glu Arg Ala Val Lys Gln Glu Asp Ala Lys Lys Tyr
   675 680 685
   Leu Glu Glu Tyr Ser Ile Gly Leu Gly Pro Ser Asp Asp Pro Arg Tyr
   690 695 700
   Glu Ile Arg Gly Val Met Val Ile Lys Ser Lys Ser Lys Thr Arg Ala
   705 710 715 720
   Arg Gln Arg Lys Gly Ala Val Ser Asn Trp Lys Ser Val Leu Arg Val
   725 730 735
   Thr Leu Asn Glu Leu Lys Lys Arg Asn Met Thr Gly Glu Glu Leu Arg
   740 745 750
   Arg Ile Met Trp Gly Arg Ser Ser Ile Thr Ser Pro Ala Lys Ile Ser
   755 760 765
   Lys Lys Lys Lys Ile Tyr Glu Glu Glu Asp Pro Phe Ala Ile Thr Asn
   770 775 780
   Ala Ile Asp Val Met Ser Phe Thr Gln Asp Ile Val Met Val Ser Ser
   785 790 795 800
   Glu Pro Thr Gly Pro Pro Thr Ile Asp Pro Ser Lys Pro Ala Thr Ile
   805 810 815
   Ala Val Lys Pro Gln Thr Ala Pro Ala Ala Gln Pro Ile Pro Glu Ile
   820 825 830
   Pro Pro Gln Pro Pro Leu Val Lys Arg Gln Ser Val Val Ser Ala Pro
   835 840 845
   Pro Ala Tyr Asp Asp Phe Pro Pro Met Lys Asp Tyr Lys Asp Pro Leu
   850 855 860
   Arg Glu Leu Val Ile Ile Ser Glu Ser Pro Ser Val Asp Asp His Tyr
   865 870 875 880
   Val Gln Ser Cys Lys Thr Leu Ala Asn Asp Ala Ser Thr Leu Asp His
   885 890 895
   Asp Thr Leu Gly Gln Leu Asn Pro Phe Leu Glu Ala Lys Asp Val Val
   900 905 910
   Asp Pro Val Lys Glu Arg Glu Phe Leu Lys Thr Leu Glu Ala Leu Glu
   915 920 925
   Asp Thr Asp Ser Glu Ala Ala Glu Lys Pro Val Leu Gly Lys Ile Ser
   930 935 940
   Leu Ile Arg Arg Ala Lys Ser Ala Val Ser Arg Thr Thr Ser Arg Lys
   945 950 955 960
   Lys Lys Thr Asp Gln His Pro Leu Phe Thr Ile Ala Trp Asp Asp Lys
   965 970 975
   Asn Leu Thr Arg Thr His Pro Glu Asp Phe Gly Ala Leu Asn Thr Ala
   980 985 990
   Tyr Glu Tyr Ser Ala Glu Asp Phe Lys Gln Asp Val Asp Glu Glu Ala
   995 1000 1005
   Arg Glu Glu Glu Glu Asp Glu Gly Val Thr Val Glu Glu Val His
   1010 1015 1020
   Arg Arg Met Glu Glu Leu His His Arg Gly Arg Ala Ser Leu Glu
   1025 1030 1035
   Arg Asp Ser Asn Ser Thr Glu Ser Ser Lys Lys His Pro His Arg
   1040 1045 1050
   Lys Arg Gln Gly Met Gly Arg Gly Arg Asn Asn Lys Val Ser Pro
   1055 1060 1065
   Asp Thr Ser Asn Glu Ser Val Ser Gly Lys Lys Asp Lys Arg Met
   1070 1075 1080
   Lys Ser Ala Pro Thr Gly Gly Gly Thr Ser Pro Pro Asp Pro Leu
   1085 1090 1095
   Glu Pro Trp Ser Thr Arg Asp Ile Cys Asn Ile Asn Lys Leu Leu
   1100 1105 1110
   Asp Thr Asp Thr Gln Asp Glu
   1115 1120
<210> 85
   <211> 1137
   <212> PRT
   <213> Culex quinquefasciatus
<400> 85
   Met Val Lys Phe Asp Trp Arg Arg Met Cys Arg Lys Lys Lys Asn Val
   1 5 10 15
   Pro Gln Gly Gly Ala Ile Leu Asp Gln Val Ile Ser Gln Ser Thr Asn
   20 25 30
   Val Gln Gly Gln Cys Leu Leu Tyr Lys Met Ala Asn Tyr Lys Gly Gly
   35 40 45
   Gly Asp Leu Ile Asp Ala Phe Lys Met Gly Gly Gln Lys Ala Val Glu
   50 55 60
   Gln Leu Ile Arg Glu Gln Phe Gly Val Phe Met Tyr Asn Ala Gly Arg
   65 70 75 80
   Gly Gln Ile Ile Asn Arg Ala Glu Tyr Leu Arg Trp Lys Tyr Met Asp
   85 90 95
   Asn His Glu Val Val Ile Pro Ile Glu Ala Ser Leu Ser Pro His Asp
   100 105 110
   Pro Leu Gly Lys Trp Val Asp His Lys Ala Cys Trp Gln Met Gln Tyr
   115 120 125
   Arg Gly Leu Leu Gly Glu Ser Leu Leu His Val Leu Ile Ile Cys Asp
   130 135 140
   Thr Lys Ile His Thr Lys Leu Ser Arg Ile Leu Leu Arg Val Phe Pro
   145 150 155 160
   Glu Leu Ser Ile Asp Val Met Glu Gly Glu Glu Tyr Leu Gly Ala Ser
   165 170 175
   Ala Leu His Leu Ala Ile Ala Tyr Ser Asn Asn Glu Leu Val Gly Asp
   180 185 190
   Leu Ile Asp Ala Gly Ala Asn Val Ser Gln Arg Ala Ile Gly Arg Phe
   195 200 205
   Phe Leu Pro Arg Asp Gln Gln Lys Met His Pro Ala Lys Thr Thr Asp
       210                 215                 220
   Tyr Glu Gly Leu Ala Tyr Leu Gly Glu Tyr Pro Leu Ala Trp Ala Ala
   225                 230                 235                 240
   Cys Cys Ser Asn Glu Ser Val Tyr Asn Leu Leu Leu Glu Cys Gly Ala
                   245                 250                 255
   Asp Pro Asn Ala Gln Asp Ser Phe Gly Asn Met Ile Leu His Met Val
               260                 265                 270
   Val Val Cys Asp Lys Leu Asp Met Phe Gly Tyr Ala Leu Arg His Pro
           275                 280                 285
   Lys Leu Pro Cys Lys Asn Gly Ile Val Asn Glu Ala Gly Leu Thr Pro
       290                 295                 300
   Leu Thr Leu Ala Cys Arg Ile Gly Arg Asp Glu Val Phe Arg Glu Met
   305                 310                 315                 320
   Leu Glu Leu Ser Ala Arg Glu Phe Trp Arg Tyr Ser Asn Ile Thr Cys
                   325                 330                 335
   Ser Gly Tyr Pro Leu Asn Ala Leu Asp Thr Leu Met Pro Asp Gly Ser
               340                 345                 350
   Thr Asn Trp Asn Ser Ala Leu Phe Ile Ile Leu Asn Gly Thr Lys Glu
           355                 360                 365
   Glu His Leu Asn Met Leu Asp Gly Gly Ile Val Glu Arg Leu Leu Asp
       370                 375                 380
   Glu Lys Trp Lys Thr Phe Ala Arg Asn Gln Phe Leu Lys Arg Leu Leu
   385                 390                 395                 400
   Ile Leu Ala Leu His Leu Phe Cys Leu Ser Cys Ser Ile Tyr Leu Arg
                   405                 410                 415
   Pro Val His Val Phe Lys Asp Asp Asp Asp Ala Asp Asp Thr Asp Gly
               420                 425                 430
   Val Thr Asp Ser Pro Asp Thr Thr Asp Asp Glu Gly Ile Asp Leu Thr
           435                 440                 445
   Thr Trp Phe Arg Tyr Gly Phe Glu Val Ala Thr Val Met Gly Val Leu
       450                 455                 460
   Ser Tyr Val Val Val Gln Gln Gly Glu Glu Ile Lys Asn Gln Gly Phe
   465 470 475 480
   Ile Ser Phe Leu Lys Ser Leu Ala Gly Ala Pro Ala Lys Ala Ile Phe
   485 490 495
   Leu Ile Ser Asn Leu Met Ile Leu Cys Cys Ile Pro Leu Arg Ile Leu
   500 505 510
   Gly Asp Arg Glu Ala Glu Glu Ala Val Leu Leu Phe Ala Val Pro Gly
   515 520 525
   Ser Trp Phe Leu Leu Met Phe Phe Ala Gly Ala Ile Gly Leu Thr Gly
   530 535 540
   Pro Phe Val Thr Met Ile Phe Ser Met Ile Thr Gly Asp Met Phe Thr
   545 550 555 560
   Phe Gly Ile Ile Tyr Thr Ile Val Leu Phe Gly Phe Ser Gln Ala Phe
   565 570 575
   Tyr Phe Leu Tyr Lys Gly His Pro Asn Ala Asp Glu Ser Pro Phe Gly
   580 585 590
   Thr Tyr Phe Gly Thr Trp Met Gly Leu Phe Gln Thr Thr Leu Gly Asp
   595 600 605
   Tyr Asp Tyr Ala Asp Leu Asn Leu Thr Thr Tyr Pro Asn Leu Ala Lys
   610 615 620
   Thr Val Phe Ile Thr Phe Met Ile Phe Val Pro Ile Leu Leu Leu Asn
   625 630 635 640
   Met Leu Ile Ala Met Met Gly Asn Thr Tyr Ala Tyr Val Ile Glu Gln
   645 650 655
   Ala Glu Lys Glu Gly Met Lys Gln Trp Ala Lys Ile Val Val Asn Leu
   660 665 670
   Glu Arg Ala Val Lys Gln Glu Asp Ala Lys Lys Tyr Leu Glu Glu Tyr
   675 680 685
   Ser Ile Gly Leu Gly Pro Ser Asp Asp Pro Arg Phe Glu Ile Arg Gly
   690 695 700
   Val Met Val Ile Lys Ser Lys Ser Lys Thr Arg Ala Arg Gln Arg Lys
   705 710 715 720
   Gly Ala Val Ser Asn Trp Lys Ser Val Leu Arg Val Thr Leu Asn Glu
   725 730 735
   Leu Lys Lys Arg Ser Met Thr Gly Glu Glu Leu Arg Arg Ile Met Trp
   740 745 750
   Gly Arg Ser Ser Ile Thr Ser Pro Ala Lys Ile Ser Lys Lys Lys Lys
   755 760 765
   Ile Tyr Asp Glu Asp Glu Asp Pro Phe Ala Ile Thr Ala Ala Ile Asp
   770 775 780
   Val Met Ser Phe Thr Gln Asp Ile Val Met Val Ser Thr Glu Pro Val
   785 790 795 800
   Gly Pro Leu Met Ile Asp Pro Thr Lys Pro Pro Val Thr Thr Val Lys
   805 810 815
   Pro Thr Ala Pro Thr Val Pro Ser Ala Gln Pro Ala Pro Gly Val Pro
   820 825 830
   Met Thr Thr Ala Gln Thr Ala Ala Pro Thr Ala Pro Gln Leu Val Lys
   835 840 845
   Arg Gln Ser Val Val Ser Ala Pro Pro Ala Tyr Asp Asp Phe Leu Pro
   850 855 860
   Ala Lys Gly Phe Lys Asp Pro Leu Arg Glu Leu Leu Ile Leu Ser Glu
   865 870 875 880
   Ser Thr Ser Ile Asp Glu His Tyr Ala Gln Thr Cys Arg Ser Leu Ala
   885 890 895
   Thr Asp Ala Ser Thr Leu Asp His Glu Glu His Gln Gln Gln Gln Gln
   900 905 910
   Gln Thr Val Gly Gln Leu Asn Pro Phe Leu Asp Ala Lys Asp Val Val
   915 920 925
   Asp Pro Val Lys Glu Arg Glu Phe Leu Lys Thr Leu Glu Ala Leu Glu
   930 935 940
   Asp Thr Asp Ser Glu Ala Ala Glu Lys Pro Val Leu Gly Lys Ile Ser
   945 950 955 960
   Leu Ile Arg Arg Ala Arg Ser Ala Val Ser Arg Thr Thr Ser Arg Lys
   965 970 975
   Lys Lys Thr Asp Gln His Pro Leu Phe Met Ile Ala Trp Asp Asp Lys
   980 985 990
   Thr Thr Thr Ile Pro Gly Gly Gly Pro Gln Ala Val Ser Ala Thr Ala
   995 1000 1005
   Gly Thr Ala Ala Pro Glu Asp Asp Phe Gly Ala Leu Asn Thr Ala
   1010 1015 1020
   Tyr Glu Cys Ser Ala Glu Asp Leu Lys His Asp Val Asp Glu Glu
   1025 1030 1035
   Ala Arg Glu Asp Asp Pro Asp Glu Gly Val Thr Val Glu Glu Val
   1040 1045 1050
   His Arg Arg Met Glu Arg Phe His Gln Arg Pro Gly Arg Ser Ser
   1055 1060 1065
   Pro Glu Arg Asp Ser Asn Ser Thr Glu Ser Ser Lys Lys Gln Ala
   1070 1075 1080
   Lys Gly Asp Lys Arg Met Lys Ser Ala Pro Ser Gly His Gly Pro
   1085 1090 1095
   Arg His Arg Gln Met Met Asp Thr Gly Gly Gly Ser Pro Pro Asp
   1100 1105 1110
   Pro Leu Glu Pro Trp Ser Thr Lys Glu Ile Val Pro Ile Asn Lys
   1115 1120 1125
   Leu Leu Asp Thr Asp Thr Gln Asp Glu
   1130 1135
<210> 86
   <211> 1216
   <212> PRT
   <213> Anopheles gambiae
<400> 86
   Met Val His Leu Asp Pro Leu Arg Leu Cys Arg Lys Lys Arg Lys Leu
   1               5                   10                  15
   Pro Gln Gly Gly Ala Ile Leu Asp Gln Val Ile Ser Gln Ser Ala Ser
               20                  25                  30
   Ala Ser Asn Gln Cys Leu Leu Tyr Lys Met Ala Asn Tyr Lys Arg Gly
           35                  40                  45
   Gly Asp Leu Ile Asp Ala Phe Gln Ile Gly Gly Gln Lys Ala Val Glu
       50              55              60
   Gln Leu Ile Arg Glu Gln Phe Gly Val Phe Met Tyr Asn Lys Gly Arg
   65              70              75              80
   Gly Gln Ile Ile Asn Arg Ala Glu Tyr Leu Arg Trp Lys Tyr Met Asp
                   85              90              95
   Asn His Glu Val Ile Ile Pro Ile Glu Ala Ser Leu Ser Pro His Asp
               100             105             110
   Pro Leu Ser Lys Trp Asp Asp His Lys Ala Cys Trp Gln Met Gln Tyr
           115             120             125
   Arg Gly Leu Leu Gly Glu Ser Leu Leu His Val Leu Ile Ile Cys Asp
       130             135             140
   Thr Lys Val His Thr Lys Leu Ala Arg Ile Leu Leu Arg Val Phe Pro
   145             150             155             160
   Glu Gln Ser Ile Asp Val Met Glu Gly Glu Glu Tyr Leu Gly Ala Ser
                   165             170             175
   Ala Leu His Leu Ala Ile Ala Tyr Ser Asn Asn Glu Leu Val Gly Asp
               180             185             190
   Leu Ile Asp Ala Gly Ala Asp Val Ser Gln Arg Ala Thr Gly Arg Phe
           195             200             205
   Phe Leu Pro Arg Asp Gln Gln Gly Leu Arg Pro Ala Lys Thr Thr Asp
       210             215             220
   Tyr Glu Gly Leu Ala Tyr Leu Gly Glu Tyr Pro Leu Ala Trp Ala Ala
   225             230             235             240
   Cys Cys Ala Asn Glu Ser Val Tyr Asn Leu Leu Leu Glu Cys Gly Ala
                   245             250             255
   Asp Pro Asn Ala Gln Asp Ser Phe Gly Asn Met Ile Leu His Met Val
               260             265             270
   Val Val Cys Asp Lys Leu Asp Met Phe Gly Tyr Ala Leu Arg His Pro
           275             280             285
   Lys Leu Pro Cys Lys Asn Gly Ile Val Asn Ala Ala Gly Leu Thr Pro
       290                 295                 300
   Leu Thr Leu Ala Cys Arg Leu Gly Arg Asp Glu Val Phe Arg Glu Met
   305                 310                 315                 320
   Leu Glu Leu Ser Ala Arg Glu Phe Trp Arg Tyr Ser Asn Ile Thr Cys
                   325                 330                 335
   Ser Gly Tyr Pro Leu Asn Ala Leu Asp Thr Leu Met Pro Asp Gly Ser
               340                 345                 350
   Thr Asn Trp Asn Ser Ala Leu Phe Ile Ile Leu Asn Gly Thr Lys Glu
           355                 360                 365
   Glu His Leu Asn Met Leu Asp Gly Gly Ile Val Glu Arg Leu Leu Asp
       370                 375                 380
   Glu Lys Trp Lys Thr Phe Ala Arg Asn Gln Phe Ile Lys Arg Leu Leu
   385                 390                 395                 400
   Ile Leu Ala Ile His Leu Phe Cys Leu Ser Cys Ser Val Tyr Leu Arg
                   405                 410                 415
   Pro Val Arg Val Phe Ala Asp Asp Glu Gly Glu Glu Gly Gly Asp Ala
               420                 425                 430
   Thr Asp Asp Asp Gly Ala Asp Asp Gly Gly Pro Met Ala Val Asp Ala
           435                 440                 445
   Val Asp Asp Asp Ile Asp Leu Thr Thr Trp Val Arg Tyr Gly Phe Glu
       450                 455                 460
   Val Ala Thr Val Met Gly Val Leu Ser Tyr Val Val Leu Gln Gln Gly
   465                 470                 475                 480
   Asp Glu Ile Lys Asn Gln Gly Phe Phe Ser Phe Leu Lys Ser Leu Ser
                   485                 490                 495
   Gln Ala Pro Ala Lys Ala Ile Phe Leu Ile Ser Asn Ile Leu Ile Leu
               500                 505                 510
   Ala Cys Ile Pro Leu Arg Met Met Gly Asp Thr Glu Thr Glu Glu Ala
           515                 520                 525
   Ile Leu Leu Phe Ala Val Pro Gly Ser Trp Phe Leu Leu Met Phe Phe
       530                 535                 540
   Ala Gly Ala Ile Gly Leu Thr Gly Pro Phe Val Thr Met Ile Phe Ser
   545 550 555 560
   Met Ile Thr Gly Asp Met Phe Thr Phe Gly Ile Ile Tyr Met Ile Val
   565 570 575
   Leu Phe Gly Phe Ser Gln Ala Phe Tyr Phe Leu Tyr Lys Gly His Pro
   580 585 590
   Asn Ala Glu Asp Ser Pro Phe Gly Ser Tyr Phe Gly Thr Trp Met Ala
   595 600 605
   Leu Phe Gln Thr Thr Leu Gly Asp Tyr Asp Tyr Ala Asp Leu Asn Leu
   610 615 620
   Thr Thr Tyr Pro Asn Leu Ala Lys Thr Val Phe Val Ile Phe Met Ile
   625 630 635 640
   Phe Val Pro Ile Leu Leu Leu Asn Met Leu Ile Ala Met Met Gly Asn
   645 650 655
   Thr Tyr Ala Tyr Val Ile Glu Gln Ala Glu Lys Glu Gly Met Lys Gln
   660 665 670
   Trp Ala Lys Ile Val Val Asn Leu Glu Arg Ala Val Thr Gln Asp Asp
   675 680 685
   Ala Lys Arg Tyr Leu Glu Glu Tyr Ser Ile Gly Leu Gly Pro Ser Asp
   690 695 700
   Asp Pro Arg Tyr Glu Thr Arg Gly Val Met Val Ile Lys Ser Lys Ser
   705 710 715 720
   Lys Thr Arg Ala Arg Gln Arg Lys Gly Ala Val Ser Asn Trp Lys Ser
   725 730 735
   Val Leu Arg Val Thr Leu Asn Glu Leu Lys Lys Arg Ser Met Thr Gly
   740 745 750
   Glu Glu Leu Arg Arg Ile Met Trp Gly Arg Ser Ser Ile Thr Ser Pro
   755 760 765
   Ala Lys Ile Ala Lys Lys Lys Arg Pro Gly Glu Val Asp Asp Leu Leu
   770 775 780
   Gly Asp Pro Phe Ala Ile Thr Ala Ala Ile Asp Val Met Ser Phe Thr
   785 790 795 800
   Gln Asp Ile Val Met Val Ser Thr Asp Thr Val Cys Pro Ile Thr Ile
                   805                 810                 815
   Pro Pro Ala Ala Ala Pro Gly Thr Ala Ala Pro Lys Ala Gly Gly Thr
               820                 825                 830
   Lys Pro Val Val Val Ala Pro Pro Pro Pro Ala Ala Ala Thr Ala Val
           835                 840                 845
   Gln Gln Pro Pro Pro Pro Ala Thr Thr Arg Arg His Ser Thr Ala Ser
       850                 855                 860
   Ala Pro Ala Thr Met Gly Leu Gly Val Glu Ala Ala Leu Pro Gly Tyr
   865                 870                 875                 880
   Lys Asp Pro Leu Arg Glu Leu Val Ile Ile Ser Glu Ser Ala Ser Val
                   885                 890                 895
   Asp Glu Asn Tyr Ala Gln Asn Val Lys Thr Leu Ala Ile Asp Ala Ser
               900                 905                 910
   Thr Leu Asp His Val His Glu Ile Asp Ile Ser Gln Gln Gln Gln Gln
           915                 920                 925
   Gln Gln Gln Gln Gln Gln Gln Gln Pro Gln Gln Pro Gln Gln Gln Gln
       930                 935                 940
   Asn Leu Gly Gln Leu Thr Leu Phe Gln Asn Pro Lys Asp Val Val Asp
   945                 950                 955                 960
   Pro Val Arg Glu Arg Glu Phe Leu Lys Thr Leu Glu Ala Leu Glu Asp
                   965                 970                 975
   Thr Asp Ser Glu Ala Gly Glu Lys Pro Val Leu Gly Lys Ile Ser Leu
               980                 985                 990
   Ile Arg Arg Ala Lys Ser Ala Val  Ser Arg Ser Thr Ser  Arg Lys Arg
           995                 1000                1005
   Lys Thr  Asp Gln His Pro Leu  Phe Met Ile Ala Trp  Glu Asp Lys
       1010                1015                1020
   Gly Asp  Gln Gln Arg His Ser  Thr Ala Leu Tyr Asp  Gly Ala Thr
       1025                1030                1035
   Gly Ala  Ala Pro Ser Val Gly  Ala Pro Gly Glu Leu  Gly Gln Pro
       1040                1045                1050
   Glu Gln Pro Glu Asp Ala Val Thr Val Glu Glu Leu His Arg Arg
   1055 1060 1065
   Met Glu Gln Phe His Gln Arg Ala Ser Val Arg Glu Arg Asp Ala
   1070 1075 1080
   Asn Ser Ser Glu Ser Gly Ser Gly Lys Gln His Ala Gly Pro Lys
   1085 1090 1095
   Ala Arg Lys Pro Ser His His Arg Asp Gly Gly Gly Ser His His
   1100 1105 1110
   His His Gly Leu Gly Arg Gly Lys His Asn Lys Ile Ser Pro Asp
   1115 1120 1125
   Asn Ser Asn Glu Ser Thr Gly Gly Gly Gly Gly Gly Gly Ala Ser
   1130 1135 1140
   Gly His Gly Arg Lys Gln Asp Lys Arg Met Lys Ser Ala Pro Ile
   1145 1150 1155
   Leu Gly Ser Gly Gly Ala Gly Ser Ala Gly Ser Ser Ala Gly Gly
   1160 1165 1170
   Arg Thr Val Ala Pro Gly Gly Ala Arg Pro Asp Asp Gly Thr Gly
   1175 1180 1185
   Ser Pro Pro Asp Pro Leu Glu Pro Trp Ser Thr Lys Asn Ile Met
   1190 1195 1200
   Asn Ile Asn Lys Leu Leu Asp Gln Asp Thr Thr Glu Glu
   1205 1210 1215
<210> 87
   <211> 1086
   <212> PRT
   <213> Tribolium castaneum
<400> 87
   Met Gly Ala Lys Val Cys Lys Pro Cys Lys Lys Arg Lys Ala Asn Thr
   1 5 10 15
   Phe Gln Gly Gly Ser Ile Leu Asp Arg Val Ile Ser Gln Ala Ser Asn
   20 25 30
   Gln Asp Gln Cys Leu Leu Tyr Lys Leu Ala Asn Tyr Lys Lys Gly Gly
   35 40 45
   Glu Leu Ile Asp Ala Tyr Asn Gln Gly Gly Gln Ala Glu Val Glu Lys
       50                  55                  60
   Leu Ile Arg Glu Gln Phe Gly Gln Leu Met Tyr Gln Glu Gly Lys Gly
   65                  70                  75                  80
   Gln Ile Ile Asn Arg Ser Glu Tyr Leu Arg Trp Lys Phe Arg Asp His
                   85                  90                  95
   Glu Gln Val Ile Leu Pro Ile Glu Ala Ser Leu Ser Arg Tyr Asp Pro
               100                 105                 110
   Leu Ala Lys Trp Asn Asp His Glu Ala Cys Trp Gln Met Gln Phe Arg
           115                 120                 125
   Gly Ser Leu Gly Glu Ser Leu Leu His Val Leu Ile Ile Cys Asp Thr
       130                 135                 140
   Lys Ile His Thr Arg Leu Ala Arg Thr Leu Ile Lys Cys Phe Pro Lys
   145                 150                 155                 160
   Leu Ala Leu Asp Val Val Glu Gly Glu Glu Tyr Leu Gly Ala Ser Ala
                   165                 170                 175
   Leu His Leu Ala Ile Ala Tyr Asn Asn Asn Glu Leu Val Gln Asp Leu
               180                 185                 190
   Val Glu Ala Gly Ala Asn Val Asn Gln Arg Ala Ile Gly Ser Phe Phe
           195                 200                 205
   Leu Pro Arg Asp Gln Gln Arg Gln Lys Pro Ala Lys His Thr Asp Tyr
       210                 215                 220
   Glu Gly Leu Ala Tyr Leu Gly Glu Tyr Pro Leu Ala Trp Ala Ala Cys
   225                 230                 235                 240
   Cys Ala Asn Glu Ser Val Tyr Asn Leu Leu Leu Asp Ser Gly Ala His
                   245                 250                 255
   Pro Asp Tyr Gln Asp Asn Phe Gly Asn Met Ile Leu His Met Val Val
               260                 265                 270
   Val Cys Asp Lys Leu Asp Met Phe Gly Tyr Ala Leu Arg His Pro Lys
           275                 280                 285
   Leu Pro Ala Ser Asn Gly Ile Val Asn Lys Ala Gly Leu Thr Pro Leu
       290                 295                 300
   Thr Leu Ala Cys Lys Leu Gly Arg Ala Glu Val Phe Arg Glu Met Leu
   305 310 315 320
   Glu Leu Ser Ala Lys Glu Phe Trp Arg Tyr Ser Asn Ile Thr Cys Ser
   325 330 335
   Ala Tyr Phe Leu Asn Ala Leu Asp Thr Leu Leu Pro Asp Gly Arg Thr
   340 345 350
   Asn Trp Asn Ser Ala Leu Phe Ile Ile Leu Asn Gly Thr Lys Glu Glu
   355 360 365
   His Leu Ala Met Leu Asp Gly Gly Ile Ile Gln Arg Leu Leu Glu Glu
   370 375 380
   Lys Trp Lys Thr Phe Ala Arg Asn Gln Phe Leu Lys Arg Leu Leu Ile
   385 390 395 400
   Leu Val Val His Leu Leu Phe Leu Ser Leu Ala Val Tyr Leu Arg Pro
   405 410 415
   Asp Asp Pro Asp Glu Ser Leu Leu Thr Trp Ser Asp Asp Val Thr Leu
   420 425 430
   Ile Ala Arg Tyr Val Cys Glu Val Gly Thr Ile Leu Gly Val Leu Ser
   435 440 445
   Tyr Leu Val Leu Gln Gln Gly Asp Glu Ile Arg Asn Gln Gly Leu Thr
   450 455 460
   Ala Phe Leu Lys Gln Gln Leu Asn Ser Pro Pro Lys Leu Ile Phe Leu
   465 470 475 480
   Ile Ser Asn Phe Leu Ile Leu Ala Cys Ile Pro Cys Arg Leu Tyr Gly
   485 490 495
   Asp Lys Glu Thr Glu Glu Ala Ile Leu Cys Phe Ala Val Pro Gly Ser
   500 505 510
   Trp Phe Leu Leu Met Phe Phe Ala Gly Ala Val Arg Leu Thr Gly Pro
   515 520 525
   Phe Val Thr Met Ile Tyr Ser Met Ile Thr Gly Asp Met Leu Thr Phe
   530 535 540
   Gly Ile Ile Tyr Thr Val Phe Leu Phe Gly Phe Ser Gln Ser Phe Tyr
   545 550 555 560
   Phe Leu Tyr Lys Gly Phe Pro Gly Val Lys Thr Ser Leu Tyr Asn Thr
   565 570 575
   Tyr Met Ser Thr Trp Met Ala Leu Phe Gln Ile Thr Leu Gly Asn Tyr
   580 585 590
   Glu Tyr Ser Glu Leu Ser Ala Thr Thr Tyr Pro Ala Val Ser Lys Thr
   595 600 605
   Val Phe Ala Ile Phe Met Val Phe Val Pro Ile Leu Leu Leu Asn Met
   610 615 620
   Leu Ile Ala Met Met Gly Asn Thr Tyr Ala His Val Ile Glu Gln Ser
   625 630 635 640
   Glu Lys Glu Trp Val Lys Gln Trp Ala Lys Ile Val Ile Ala Leu Glu
   645 650 655
   Arg Ala Ile Pro Gln Ser Asp Ala Gln His Tyr Leu Gln Glu Tyr Ser
   660 665 670
   Ile Ser Leu Gly Pro Ser Glu Gln Asp Pro Ser Thr Glu Lys Arg Gly
   675 680 685
   Val Leu Val Ile Lys Ser Lys Ser Lys Thr Arg Ala Lys Gln Arg Lys
   690 695 700
   Gly Ala Val Ala Asn Trp Lys Arg Val Gly Lys Val Thr Ile Asn Ala
   705 710 715 720
   Leu Lys Lys Arg Gly Leu Thr Gly Glu Glu Met Arg Cys Leu Met Trp
   725 730 735
   Gly Arg Glu Ser Ile Asn Thr Pro Val Lys Thr Lys Lys Pro Val Lys
   740 745 750
   Asp Pro Leu Leu Asp Pro Gln Gly Pro Asn Leu Thr Gly Gly Phe Gly
   755 760 765
   Asp Ala Leu Thr Thr Ala Leu Asp Val Met Thr Phe Thr His Asp Leu
   770 775 780
   Asp Ile Val Gly Ala Ser Gln Gly Leu Asn Leu Ala Thr Asn Pro Lys
   785 790 795 800
   Pro Val Pro Pro Thr Thr Thr Ala Ser Ala Val Thr Val Asn Asn Gln
   805 810 815
   Ile Asn Ser Ala Leu Asn Ala Gln Gln Lys Ala Val Ala Gly Ala Gly
   820 825 830
   Val Gly Ala Leu Ala Met Ile Gly Thr Val Thr Gln Leu Thr Asp Ser
   835 840 845
   Gln Gly Tyr Ile Met Gln Asn Ser Val Lys Lys Glu Glu Lys Ile Val
   850 855 860
   Ala Thr Leu Glu Asp Pro Phe Arg Glu Leu Val Ile Asn Ala Asn Asp
   865 870 875 880
   Ser Asn Cys Asp Pro Glu Lys Leu Lys Met Leu Ala Leu Ser Ala Ala
   885 890 895
   Asn Leu Lys Asp Val Glu Glu Leu Ser Val Ala Lys Pro Gln Thr Lys
   900 905 910
   Ser Val Lys Ser Leu Ala Gly Ile Phe Ala Gly Thr Glu Thr Phe Val
   915 920 925
   Arg Lys Val Glu Glu Thr Ile Lys Lys Lys Tyr Ala Ala Leu Asp Pro
   930 935 940
   Ser Asp Ser Glu Gly Phe Gly Glu Pro Pro Leu Leu Gly Lys Ile Ser
   945 950 955 960
   Arg Thr Arg Arg Ala Lys Ser Ala Asn Leu Arg Asn Ser Ser Ala Arg
   965 970 975
   Ser Lys Ala Ser Asp Lys Lys Lys Leu Val Ala Gly Ser Gln Ser Ser
   980 985 990
   Ser Thr Asp Thr Val Asn Asn Glu Ile Asn Glu Lys Asn Ile Glu Asn
   995 1000 1005
   Ser Asp Leu Asp Tyr Ala Glu Glu Arg Ile Lys Leu Val Lys Glu
   1010 1015 1020
   Ser Leu Lys Gln Val Val Asp Val Ala Gln Ile Arg Pro Ile Asn
   1025 1030 1035
   Ile Asp Val Ala Leu Glu Glu Gln Val Ser Val Thr Ile Ser Glu
   1040 1045 1050
   Thr Met Arg Val Gln Gly Ser Gly Asp Gly Ala Glu Val Gln Ser
   1055 1060 1065
   Ser Asn Val Lys Pro Lys Arg Lys Lys Arg Ser Lys Thr Ala Lys
   1070 1075 1080
   Asn Asn Lys
   1085
<210> 88
   <211> 1101
   <212> PRT
   <213> Megachile rotundata
<400> 88
   Met Gly Gly Val Cys Ser Cys Arg Gly Leu Gly Ser Gln Val Asn Ala
   1 5 10 15
   Gly Ser Ile Leu Asp Arg Val Ile Ser Gln Ala Ser Asp Glu Asp Gln
   20 25 30
   Cys Leu Leu Tyr Arg Leu Ala Asn Tyr Lys Lys Ser Gly Glu Leu Ile
   35 40 45
   Asp Ala Tyr Asn Gln Gly Gly Gln Ala Glu Val Glu Lys Leu Ile Arg
   50 55 60
   Glu Gln Phe Gly Val Leu Met Tyr Ala Asp Gly Lys Gly Gln Met Ile
   65 70 75 80
   Asn Arg Ala Glu Tyr Leu Arg Trp Lys Phe Arg Asp Leu Glu Gln Val
   85 90 95
   Val Leu Pro Ile Glu Ala Ser Leu Ser Arg Phe Asp Pro Leu Ala Gln
   100 105 110
   Trp Asn Asp His Gln Ala Cys Trp Gln Met Gln Tyr Arg Gly Ser Leu
   115 120 125
   Gly Glu Thr Leu Leu His Val Leu Ile Ile Cys Asp Thr Arg Leu His
   130 135 140
   Thr Arg Leu Ala Arg Ile Leu Leu Lys Cys Phe Pro Arg Leu Ala Ile
   145 150 155 160
   Asp Val Val Glu Gly Glu Glu Tyr Leu Gly Ala Ser Ala Leu His Leu
   165 170 175
   Ala Ile Ala Tyr Asn Asn Asn Glu Leu Val Gln Asp Leu Val Glu Val
   180 185 190
   Gly Ala Ile Ile Ser Gln Arg Ala Ile Gly Ser Phe Phe Leu Pro Arg
   195 200 205
   Asp Gln Gln Arg Met Asn Pro Ala Lys Asn Thr Asp Tyr Glu Gly Leu
   210 215 220
   Ala Tyr Leu Gly Glu Tyr Pro Leu Ala Trp Ala Ala Cys Cys Ala Asn
   225 230 235 240
   Glu Ser Val Tyr Asn Leu Leu Leu Asp Ser Gly Ala Asp Pro Asp Glu
   245 250 255
   Gln Asp Ser Phe Gly Asn Met Ile Leu His Met Val Val Val Cys Asp
   260 265 270
   Lys Leu Asp Met Phe Gly Tyr Ala Leu Arg His Pro Lys Leu Pro Ala
   275 280 285
   Arg Asn Gly Ile Val Asn Ala Ala Gly Leu Thr Pro Leu Thr Leu Ala
   290 295 300
   Cys Gln Leu Gly Arg Ala Glu Val Phe Arg Glu Met Leu Glu Leu Ser
   305 310 315 320
   Ala Arg Glu Phe Trp Arg Tyr Ser Asn Ile Thr Cys Ser Ala Tyr Pro
   325 330 335
   Leu Asn Ala Leu Asp Thr Leu Leu Pro Asp Gly Arg Thr Asn Trp Asn
   340 345 350
   Ser Ala Leu Phe Ile Ile Leu Asn Gly Thr Lys Glu Glu His Leu Asn
   355 360 365
   Met Leu Asp Gly Gly Ile Ile Gln Arg Leu Leu Glu Glu Lys Trp Lys
   370 375 380
   Thr Phe Ala Arg Leu Gln Phe Leu Lys Arg Leu Ile Ile Leu Val Phe
   385 390 395 400
   His Leu Thr Ser Leu Ser Leu Ala Val Tyr Leu Arg Pro Ser Asn Leu
   405 410 415
   Asp Thr Val Leu Leu Lys Trp Pro Glu Glu Val Thr Glu Val Gly Arg
   420 425 430
   Thr Ile Ala Glu Cys Ile Thr Val Leu Gly Val Leu Ser Tyr Ile Leu
           435                 440                 445
   Val Gln Leu Gly Gly Glu Ile Ile Asn Ile Gly Val Leu Ser Phe Leu
       450                 455                 460
   Lys Gln Leu Ser His Glu Pro Ala Lys Leu Ile Phe Leu Ile Ser Asn
   465                 470                 475                 480
   Leu Leu Ile Leu Ala Cys Ile Pro Cys Arg Leu Ala Gly Asn Arg His
                   485                 490                 495
   Ala Glu Asp Ala Ile Leu Val Val Ala Val Pro Gly Ser Trp Phe Leu
               500                 505                 510
   Leu Met Phe Phe Ala Gly Ala Ile Arg Leu Thr Gly Pro Phe Val Thr
           515                 520                 525
   Met Val Tyr Ser Met Ile Thr Gly Asp Met Leu Thr Phe Gly Ile Ile
       530                 535                 540
   Tyr Met Val Val Leu Phe Gly Phe Cys Gln Ser Phe Tyr Phe Leu Tyr
   545                 550                 555                 560
   Lys Gly Phe Pro Gly Val Lys Ser Ser Leu Tyr Ser Ser Tyr His Ser
                   565                 570                 575
   Thr Trp Met Ala Leu Phe Gln Val Thr Leu Gly Asp Tyr Asn Tyr Thr
               580                 585                 590
   Asp Leu Ser Tyr Thr Thr Tyr Pro Asn Leu Ser Lys Met Val Phe Thr
           595                 600                 605
   Ile Phe Met Val Leu Val Pro Ile Leu Leu Leu Asn Met Leu Ile Ala
       610                 615                 620
   Met Met Gly Asn Thr Tyr Ala His Val Ile Glu Gln Ser Glu Lys Glu
   625                 630                 635                 640
   Trp Val Lys Gln Trp Ala Lys Ile Val Val Ser Leu Glu Arg Ala Val
                   645                 650                 655
   Ser Gln Lys Asp Ala Gln Asn Tyr Leu Gln Glu Tyr Ser Ile Lys Leu
               660                 665                 670
   Gly Pro Gly Asp Asp Pro Asn Asn Pro Ala Ser Glu Gln Arg Gly Val
           675                 680                 685
   Leu Val Ile Lys Ser Lys Ser Lys Thr Lys Ala Lys Gln Arg Lys Gly
       690                 695                 700
   Ala Val Ala Asn Trp Lys Arg Val Gly Lys Val Thr Ile Asn Glu Leu
   705                 710                 715                 720
   Arg Lys Arg Gly Met Thr Gly Glu Glu Leu Arg Arg Ile Met Trp Gly
                   725                 730                 735
   Arg Ala Ser Phe Ser Thr Pro Val Arg Val Ser Pro Asn Val Asp Glu
               740                 745                 750
   Pro Gln Val Ser Ala Val Thr Ala Gly Phe Gly Asp Ala Leu Thr Ala
           755                 760                 765
   Ala Leu Asp Val Met Ala Phe Ala His Asp Leu Asp Leu Ser Thr Ala
       770                 775                 780
   Thr Glu Ser Ile Pro Thr Asn Ile Asp Val Lys Gln Ser Lys Pro Lys
   785                 790                 795                 800
   Val Val Ser Ser Glu Gln Thr Lys Leu Thr Thr Asn Asn Gln Glu Lys
                   805                 810                 815
   Pro Ser Ile Thr Glu Lys Ala Ala Thr Glu Ile Ala Asn Lys Gln Val
               820                 825                 830
   Asn Ala Lys Asp Glu Thr Asn Lys Lys Pro Asn Glu Val Val Asp Val
           835                 840                 845
   Glu Lys Met Asn Val Lys Asn Ala Asn Asn Ser Ser Thr Val Glu Glu
       850                 855                 860
   Tyr His Asp Leu Phe Leu Glu Phe Val Ile Ala Ser Glu Ser Met Asn
   865                 870                 875                 880
   Asp Pro Glu Thr Leu Leu Lys Met Ala Glu Arg Val Ala Ala Glu Phe
                   885                 890                 895
   Asp Ser Ser Ala Asn Pro Lys Ile Asn Leu Gln Ile Leu Glu Gln Phe
               900                 905                 910
   Thr Met Thr Lys Ile Pro Met Glu Glu Gln Ala Ala Ala Val Lys Lys
           915                 920                 925
   Gln Tyr Phe Ile Glu Ser Ser Asp Asn Asp Phe Gly Gly Asp Asn Leu
       930                 935                 940
   Leu Gly Thr Val Ala Arg Leu Arg Arg Ile Arg Ser Ala Asn Ser Arg
   945                 950                 955                 960
   Phe Ile Thr Ala Arg Arg His Ser Arg His Val Asn Asp Asp Met Ser
                   965                 970                 975
   Ser Thr Ser Ser Ala Ser Gly Asp Gly Asn Pro Arg Tyr Gln Gln Leu
               980                 985                 990
   Leu Asn Asp Thr Glu Glu Val Pro  Ser Asn Gln Arg Thr  Glu Asn Arg
           995                 1000                 1005
   Asp Glu  Ser Ile Val Ala Asn  Lys Ser Glu Val Lys  Gln Asn Asp
       1010                 1015                 1020
   Ala Asn  Cys Gly Ser Arg Cys  Lys Ala Gln Lys Arg  Arg Pro Lys
       1025                 1030                 1035
   Thr Ala  Arg Asn Arg Val Ser  Pro Lys Glu Thr Glu  Asp Ser Gly
       1040                 1045                 1050
   Gln Arg  Arg Arg Glu Ser Met  Glu Arg Asn Lys Ala  Val Ser Pro
       1055                 1060                 1065
   Ala Thr  Ser Pro Thr Asp Pro  Leu Glu Pro Trp Ser  Thr Arg Gly
       1070                 1075                 1080
   Ile Lys  Asp Met Asn Thr Ile  Leu Ala Trp Glu Glu  Asn Val Pro
       1085                 1090                 1095
   Asp Ser  Pro
       1100
<210> 89
   <211> 1101
   <212> PRT
   <213> Apis mellifera
<400> 89
   Met Gly Gly Val Cys Ser Phe Arg Gly Arg Gly Ser Gln Val Asn Ala
   1               5                   10                  15
   Gly Ser Ile Leu Asp Arg Val Ile Ser Gln Ala Ser Asp Glu Asp Gln
               20                  25                  30
   Cys Leu Leu Tyr Arg Leu Ala Asn Tyr Lys Lys Gly Gly Glu Leu Ile
           35                  40                  45
   Glu Ser Tyr Asn Gln Gly Gly Gln Phe Glu Val Glu Lys Leu Ile Arg
   50 55 60
   Glu Gln Phe Gly Val Leu Met Tyr Ala Asp Gly Lys Gly Gln Val Ile
   65 70 75 80
   Asn Arg Ala Glu Tyr Leu Arg Trp Lys Phe Arg Asp Leu Glu Gln Val
   85 90 95
   Val Leu Pro Ile Glu Ala Ser Leu Ser Gln Phe Asp Pro Leu Ala Gln
   100 105 110
   Trp Asn Asp His Glu Ala Cys Trp Gln Met Gln Tyr Arg Gly Ser Leu
   115 120 125
   Gly Glu Thr Leu Leu His Val Leu Ile Ile Cys Asp Thr Arg Ile His
   130 135 140
   Thr Arg Val Ala Arg Ile Leu Leu Lys Cys Phe Pro Arg Leu Ala Ile
   145 150 155 160
   Asp Val Val Glu Gly Glu Glu Tyr Leu Gly Ala Ser Ala Leu His Leu
   165 170 175
   Ala Ile Ala Tyr Asn Asn Asn Glu Leu Val Gln Asp Leu Val Glu Ala
   180 185 190
   Gly Ala Ile Ile Ser Gln Arg Ala Ile Gly Ser Phe Phe Leu Pro Arg
   195 200 205
   Asp Gln Gln Arg Thr Asn Pro Ala Lys Asn Thr Asp Tyr Glu Gly Leu
   210 215 220
   Ala Tyr Leu Gly Glu Tyr Pro Leu Ala Trp Ala Ala Cys Cys Ala Asn
   225 230 235 240
   Glu Ser Val Tyr Asn Leu Leu Leu Asp Ser Gly Ala Asp Pro Asp Glu
   245 250 255
   Gln Asp Ser Phe Gly Asn Met Ile Leu His Met Val Val Val Cys Asp
   260 265 270
   Lys Leu Asp Met Phe Gly Tyr Ala Leu Arg His Pro Lys Leu Pro Ala
   275 280 285
   Arg Asn Gly Ile Val Asn Ala Ala Gly Leu Thr Pro Leu Thr Leu Ala
   290 295 300
   Cys Gln Leu Gly Arg Ala Glu Val Phe Arg Glu Met Leu Glu Leu Ser
   305 310 315 320
   Ala Arg Glu Phe Trp Arg Tyr Ser Asn Ile Thr Cys Ser Ala Tyr Pro
   325 330 335
   Leu Asn Ala Leu Asp Thr Leu Leu Pro Asp Gly Arg Thr Asn Trp Asn
   340 345 350
   Ser Ala Leu Phe Ile Ile Leu Asn Gly Thr Lys Glu Glu His Leu Asp
   355 360 365
   Met Leu Asp Gly Gly Ile Ile Gln Arg Leu Leu Glu Glu Lys Trp Lys
   370 375 380
   Thr Phe Ala Arg Leu Gln Phe Leu Lys Arg Leu Ile Ile Leu Ala Phe
   385 390 395 400
   His Leu Thr Ser Leu Ser Leu Ala Val Tyr Leu Arg Pro Ser Asn Thr
   405 410 415
   Asp Ala Gln Leu Leu Lys Trp Pro Glu Glu Ile Thr Glu Val Ala Arg
   420 425 430
   Thr Ile Ala Glu Cys Ile Thr Val Leu Gly Val Leu Ser Tyr Ile Leu
   435 440 445
   Val Gln Leu Gly Gly Glu Ile Ile Asn Ile Gly Leu Leu Ser Phe Met
   450 455 460
   Lys Gln Leu Ser His Glu Pro Ala Lys Leu Ile Phe Leu Ile Ser Asn
   465 470 475 480
   Leu Leu Ile Leu Ala Cys Ile Pro Cys Arg Leu Ala Gly Asn Arg His
   485 490 495
   Ala Glu Asp Ala Ile Leu Ile Val Ala Val Pro Gly Ser Trp Phe Leu
   500 505 510
   Leu Met Phe Phe Ala Gly Ala Val Arg Leu Thr Gly Pro Phe Val Thr
   515 520 525
   Met Val Tyr Ser Met Ile Thr Gly Asp Met Leu Thr Phe Gly Ile Ile
   530 535 540
   Tyr Met Val Val Leu Phe Gly Phe Cys Gln Ser Phe Tyr Phe Leu Tyr
   545 550 555 560
   Lys Gly Phe Pro Gly Val Lys Ser Ser Leu Tyr Ser Ser Tyr His Ser
   565 570 575
   Thr Trp Met Ala Leu Phe Gln Ile Thr Leu Gly Asp Tyr Asn Tyr Thr
   580 585 590
   Asp Leu Ser Tyr Thr Thr Tyr Pro Asn Leu Ser Lys Met Val Phe Ala
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Trp Val Lys Gln Trp Ala Lys Ile Val Val Ser Leu Glu Arg Ala Val
   645 650 655
   Ser Gln Lys Asp Ala Gln Asn Tyr Leu Gln Glu Tyr Ser Ile Lys Leu
   660 665 670
   Gly Pro Gly Asp Asp Pro Asn Asn Pro Ala Ala Glu Gln Arg Gly Val
   675 680 685
   Leu Ile Ile Lys Ser Lys Ser Lys Thr Lys Ala Lys Gln Arg Lys Gly
   690 695 700
   Ala Val Ala Asn Trp Lys Arg Val Gly Lys Val Thr Ile Asn Glu Leu
   705 710 715 720
   Lys Lys Arg Gly Leu Thr Gly Glu Glu Leu Arg Arg Ile Met Trp Gly
   725 730 735
   Arg Ala Ser Phe Ser Thr Pro Val Arg Val Ser Pro Lys Gly Val Glu
   740 745 750
   Pro Gln Val Ser Val Val Thr Ala Gly Phe Gly Asp Ala Leu Thr Thr
   755 760 765
   Ala Leu Asp Val Met Thr Phe Ala His Asp Leu Asp Leu Ser Thr Ala
   770 775 780
   Thr Glu Gly Ile Pro Thr Asn Ile Asp Ala Lys Gln Ser Lys Pro Lys
   785 790 795 800
   Ser Ala Thr Lys Glu Thr Lys Ser Thr Val Asn Asn Gln Gln Asn Val
   805 810 815
   Thr Thr Asn Ile Glu Pro Leu Lys Ser Thr Thr Glu Asn Val Asp Asp
   820 825 830
   Gln Ala Asn Asn Pro Arg Glu Lys Lys Val Thr Ser Gln Ala Ala Thr
   835 840 845
   Val His Glu Met Asn Leu Lys Asn Ala Asn His Ser Ser Val Thr Glu
   850 855 860
   Asp Phe Gln Asp Pro Leu Leu Glu Leu Val Ile Ala Ser Glu Asn Thr
   865 870 875 880
   Asn Asp Pro Glu Thr Leu Leu Glu Ile Ala Lys Arg Ala Ala Ala Gly
   885 890 895
   Phe Glu Thr Glu Thr Ser Ser Lys Ile Asn Leu Gln Ile Leu Glu Gln
   900 905 910
   Phe Thr Met Thr Lys Ile Pro Met Asp Glu Lys Val Asn Val Thr Arg
   915 920 925
   Lys Gln Tyr Phe Val Glu Ser Ser Asp Asn Asp Phe Gly Gly Asp Asn
   930 935 940
   Leu Leu Gly Thr Glu Ala Arg Leu Arg Arg Ile Arg Ser Ala Asn Asn
   945 950 955 960
   Arg Phe Ile Thr Thr Arg Arg Arg Ser Arg Asn Val Asp Asp Asp Leu
   965 970 975
   Ser Ser Thr Ser Ser Thr Ser Met Asp Arg Asn Pro Arg Tyr Gln Ser
   980 985 990
   Leu Leu Asn Gly His Glu Asn Ser Ile Asp Arg Pro Ile Glu Ser Arg
   995 1000 1005
   Glu Cys Ser Ile Glu Ala Ile Asn Ser Gln Ile Lys Gln Asn Gly
   1010 1015 1020
   Pro Cys Glu Thr Met Lys Ala Lys Val Gln Lys Lys Arg Pro Lys
   1025 1030 1035
   Thr Ala Arg Asn Arg Val Ser Pro Lys Glu Ile Glu Lys Ala Gly
   1040 1045 1050
   Pro Arg Arg Arg Glu Ser Ile Glu Arg Asn Lys Ala Val Ser Pro
   1055 1060 1065
   Ala Thr Ser Pro Thr Asp Pro Leu Glu Pro Trp Ser Thr Arg Gly
   1070 1075 1080
   Ile Lys Asp Met Asn Thr Ile Leu Ala Trp Glu Glu Thr Ala Pro
   1085 1090 1095
   Asp Ser Pro
   1100
<210> 90
   <211> 1105
   <212> PRT
   <213> Apis florea
<400> 90
   Met Gly Gly Val Cys Ser Phe Arg Gly Arg Gly Ser Gln Val Asn Ser
   1 5 10 15
   Gly Ser Ile Leu Asp Arg Val Ile Ser Gln Ala Ser Asp Glu Asp Gln
   20 25 30
   Cys Leu Leu Tyr Arg Leu Ala Asn Tyr Lys Lys Gly Gly Glu Leu Ile
   35 40 45
   Glu Ser Tyr Asn Gln Gly Gly Gln Phe Glu Val Glu Lys Leu Ile Arg
   50 55 60
   Glu Gln Phe Gly Val Leu Met Tyr Ala Asn Gly Lys Gly Gln Val Ile
   65 70 75 80
   Asn Arg Ala Glu Tyr Leu Arg Trp Lys Phe Arg Asp Leu Glu Gln Val
   85 90 95
   Val Leu Pro Ile Glu Ala Ser Leu Ser Gln Phe Asp Pro Leu Ala Gln
   100 105 110
   Trp Asn Asp His Glu Ala Cys Trp Gln Met Gln Tyr Arg Gly Ser Leu
   115 120 125
   Gly Glu Thr Leu Leu His Val Leu Ile Ile Cys Asp Thr Arg Ile His
   130 135 140
   Thr Arg Val Ala Arg Ile Leu Leu Lys Cys Phe Pro Arg Leu Ala Ile
   145 150 155 160
   Asp Val Val Glu Gly Glu Glu Tyr Leu Gly Ala Ser Ala Leu His Leu
                   165                 170                 175
   Ala Ile Ala Tyr Asn Asn Asn Glu Leu Val Gln Asp Leu Val Glu Ala
               180                 185                 190
   Gly Ala Ile Ile Ser Gln Arg Ala Ile Gly Ser Phe Phe Leu Pro Arg
           195                 200                 205
   Asp Gln Gln Arg Met Asn Pro Ala Lys Asn Thr Asp Tyr Glu Gly Leu
       210                 215                 220
   Ala Tyr Leu Gly Glu Tyr Pro Leu Ala Trp Ala Ala Cys Cys Ala Asn
   225                 230                 235                 240
   Glu Ser Val Tyr Asn Leu Leu Leu Asp Ser Gly Ala Asp Pro Asp Glu
                   245                 250                 255
   Gln Asp Ser Phe Gly Asn Met Ile Leu His Met Val Val Val Cys Asp
               260                 265                 270
   Lys Leu Asp Met Phe Gly Tyr Ala Leu Arg His Pro Lys Leu Pro Ala
           275                 280                 285
   Arg Asn Gly Ile Val Asn Ala Ala Gly Leu Thr Pro Leu Thr Leu Ala
       290                 295                 300
   Cys Gln Leu Gly Arg Ala Glu Val Phe Arg Glu Met Leu Glu Leu Ser
   305                 310                 315                 320
   Ala Arg Glu Phe Trp Arg Tyr Ser Asn Ile Thr Cys Ser Ala Tyr Pro
                   325                 330                 335
   Leu Asn Ala Leu Asp Thr Leu Leu Pro Asp Gly Arg Thr Asn Trp Asn
               340                 345                 350
   Ser Ala Leu Phe Ile Ile Leu Asn Gly Thr Lys Glu Glu His Leu Asp
           355                 360                 365
   Met Leu Asp Gly Gly Ile Ile Gln Arg Leu Leu Glu Glu Lys Trp Lys
       370                 375                 380
   Thr Phe Ala Arg Leu Gln Phe Leu Lys Arg Leu Ile Ile Leu Ala Phe
   385                 390                 395                 400
   His Leu Thr Ser Leu Ser Leu Ala Val Tyr Leu Arg Pro Ser Asn Thr
                   405                 410                 415
   Asp Ala Gln Leu Leu Lys Trp Pro Glu Glu Ile Thr Glu Val Ala Arg
   420 425 430
   Thr Ile Ala Glu Cys Ile Thr Val Leu Gly Val Leu Ser Tyr Ile Leu
   435 440 445
   Val Gln Leu Gly Gly Glu Ile Ile Asn Ile Gly Leu Leu Ser Phe Met
   450 455 460
   Lys Gln Leu Ser His Glu Pro Ala Lys Leu Ile Phe Leu Ile Ser Asn
   465 470 475 480
   Leu Leu Ile Leu Ala Cys Ile Pro Cys Arg Leu Ala Gly Asn Arg His
   485 490 495
   Ala Glu Asp Ala Ile Leu Ile Val Ala Val Pro Gly Ser Trp Phe Leu
   500 505 510
   Leu Met Phe Phe Ala Gly Ala Val Arg Leu Thr Gly Pro Phe Val Thr
   515 520 525
   Met Val Tyr Ser Met Ile Thr Gly Asp Met Leu Thr Phe Gly Ile Ile
   530 535 540
   Tyr Met Val Val Leu Phe Gly Phe Cys Gln Ser Phe Tyr Phe Leu Tyr
   545 550 555 560
   Lys Gly Phe Pro Gly Val Lys Ser Ser Leu Tyr Ser Ser Tyr His Ser
   565 570 575
   Thr Trp Met Ala Leu Phe Gln Ile Thr Leu Gly Asp Tyr Asn Tyr Thr
   580 585 590
   Asp Leu Ser Tyr Thr Thr Tyr Pro Asn Leu Ser Lys Met Val Phe Ala
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Trp Val Lys Gln Trp Ala Lys Ile Val Val Ser Leu Glu Arg Ala Val
   645 650 655
   Ser Gln Lys Asp Ala Gln Asn Tyr Leu Gln Glu Tyr Ser Ile Lys Leu
               660                 665                 670
   Gly Pro Gly Asp Asp Pro Asn Asn Pro Ala Ser Glu Gln Arg Gly Val
           675                 680                 685
   Leu Ile Ile Lys Ser Lys Ser Lys Thr Lys Ala Lys Gln Arg Lys Gly
       690                 695                 700
   Ala Val Ala Asn Trp Lys Arg Val Gly Lys Val Thr Ile Asn Glu Leu
   705                 710                 715                 720
   Lys Lys Arg Gly Leu Thr Gly Glu Glu Leu Arg Arg Ile Met Trp Gly
                   725                 730                 735
   Arg Ala Ser Phe Ser Thr Pro Val Arg Val Ser Pro Lys Gly Val Glu
               740                 745                 750
   Pro Gln Val Ser Val Val Thr Ala Gly Phe Gly Asp Ala Leu Thr Thr
           755                 760                 765
   Ala Leu Asp Val Met Ala Phe Ala His Asp Leu Asp Leu Ser Thr Ala
       770                 775                 780
   Thr Glu Gly Ile Pro Thr Asn Ile Asp Ala Lys Gln Ser Lys Pro Lys
   785                 790                 795                 800
   Ser Ala Ile Lys Glu Gln Met Lys Leu Thr Thr Asn Asn Gln Glu Asn
                   805                 810                 815
   Val Ser Thr Asn Val Glu Pro Gln Leu Lys Ser Thr Thr Glu Asn Ala
               820                 825                 830
   Asn Asp Gln Ala Asn Asn Pro Arg Glu Ile Lys Lys Val Thr Asn Gln
           835                 840                 845
   Val Thr Gly Ile His Glu Met Asn Leu Lys Asn Ala Asn His Ser Ser
       850                 855                 860
   Val Thr Glu Asp Phe Gln Asp Pro Leu Leu Glu Leu Val Ile Ala Ser
   865                 870                 875                 880
   Glu Asn Thr Asn Asp Pro Glu Thr Leu Leu Glu Ile Ala Lys Arg Ala
                   885                 890                 895
   Ala Ala Gly Phe Glu Thr Glu Ala Ser Ser Lys Ile Asn Leu Gln Ile
               900                 905                 910
   Leu Glu Gln Phe Thr Met Thr Lys Ile Pro Met Glu Glu Lys Val Ser
           915                 920                 925
   Val Thr Arg Lys Gln Tyr Phe Val Glu Ser Ser Asp Asn Asp Phe Gly
       930                 935                 940
   Gly Asp Asn Leu Leu Gly Thr Glu Ala Arg Leu Arg Arg Ile Arg Ser
   945                 950                 955                 960
   Ala Asn Asn Arg Phe Ile Thr Thr Arg Arg Arg Ser Arg Asn Val Asp
                   965                 970                 975
   Asp Asp Leu Ser Ser Thr Ser Ser Thr Ser Met Asp Arg Asn Pro Arg
               980                 985                 990
   Tyr Gln Ser Leu Phe Asn Gly His  Glu Asn Ser Ile Asp  Arg Pro Ile
           995                 1000                1005
   Glu Ser  Arg Glu Cys Ser Met  Glu Ala Ile Asn Ser  Gln Ile Lys
       1010                1015                1020
   Gln Asn  Gly Pro Pro Cys Glu  Thr Val Lys Ala Lys  Val Gln Lys
       1025                1030                1035
   Lys Arg  Pro Lys Thr Ala Lys  Asn Arg Val Ser Pro  Lys Glu Ile
       1040                1045                1050
   Glu Lys  Ala Gly Pro Arg Arg  Arg Glu Ser Ile Glu  Arg Asn Lys
       1055                1060                1065
   Ala Val  Ser Pro Ala Thr Ser  Pro Thr Asp Pro Leu  Glu Pro Trp
       1070                1075                1080
   Ser Thr  Arg Gly Ile Lys Asp  Met Asn Thr Ile Leu  Ala Trp Glu
       1085                1090                1095
   Glu Thr  Ala Pro Asp Ser Pro
       1100                1105
<210> 91
   <211> 1055
   <212> PRT
   <213> Pediculus humanus
<220>
   <221> source
   <223> /note="Pediculus humanus corporis"
<400> 91
   Met Gly Ala Gly Leu Cys Gly Thr Ser Gln Asp Pro Gln Asn Gln Gly
   1 5 10 15
   Ser Val Leu Asp Arg Val Ile Ser Gln Ala Ser Asn Lys Asp Asp Cys
   20 25 30
   Leu Leu Tyr Lys Leu Ala Asn Tyr Lys Asn Ser Gly Glu Leu Ile Glu
   35 40 45
   Ala Tyr Asn Ile Gly Gly Gln Ala Glu Val Glu Lys Leu Ile Lys Glu
   50 55 60
   Gln Phe Gly Val Leu Met Tyr Ala Asp Gly Lys Gly Glu Val Ile Lys
   65 70 75 80
   Arg Ala Glu Tyr Leu Arg Trp Lys Phe Arg Asp Gln Ala Gln Val Val
   85 90 95
   Leu Pro Ile Glu Ala Ser Leu Ser Ile Tyr Asp Pro Leu Ala Lys Trp
   100 105 110
   Glu Asp His Glu Ala Cys Trp Gln Met Gln Tyr Arg Gly Ser Leu Gly
   115 120 125
   Glu Thr Leu Leu His Val Leu Ile Ile Cys Asp Ser Lys Ile His Thr
   130 135 140
   Lys Leu Ala Arg Thr Leu Leu Lys Cys Phe Pro Lys Leu Ala Leu Asp
   145 150 155 160
   Ile Val Glu Gly Glu Glu Tyr Leu Gly Ala Ser Ala Leu His Leu Ala
   165 170 175
   Ile Ala Tyr Asn Asn Asn Glu Leu Val Glu Asp Leu Val Asp Ala Gly
   180 185 190
   Ala Asn Ile Asn Gln Arg Ala Val Gly Ser Phe Phe Leu Pro Lys Asp
   195 200 205
   Gln Gln Arg Ala Lys Pro Leu Lys Thr Thr Asp Tyr Glu Gly Leu Ala
   210 215 220
   Tyr Leu Gly Glu Tyr Pro Leu Ser Trp Ala Ala Cys Cys Ser Asn Glu
   225 230 235 240
   Ser Val Tyr Asn Leu Leu Leu Asp Val Gly Ala Asp Pro Asp Ser Gln
   245 250 255
   Asp Ser Phe Gly Asn Met Ile Leu His Met Val Val Val Cys Asp Lys
               260                 265                 270
   Leu Asp Met Phe Gly Tyr Ala Leu Arg His Pro Lys Val Pro Ala Ser
           275                 280                 285
   Asn Gly Ile Ile Asn Asn Glu Gly Leu Thr Pro Leu Thr Leu Ala Cys
       290                 295                 300
   Lys Leu Gly Arg Ala Asp Val Phe Lys Glu Met Leu Glu Leu Ser Ala
   305                 310                 315                 320
   Lys Glu Phe Trp Arg Tyr Ser Asn Ile Thr Cys Ser Ala Tyr Pro Leu
                   325                 330                 335
   Asn Ala Leu Asp Thr Leu Leu Pro Asp Gly Arg Thr Asn Trp Asn Ser
               340                 345                 350
   Ala Ile Phe Ile Ile Leu Asn Gly Thr Lys Glu Glu His Leu Asp Met
           355                 360                 365
   Leu Asp Gly Gly Ile Ile Gln Arg Leu Leu Glu Glu Lys Trp Lys Thr
       370                 375                 380
   Phe Ala Arg Asn Gln Phe Leu Lys Arg Leu Val Ile Phe Phe Leu His
   385                 390                 395                 400
   Ile Phe Cys Leu Ser Gly Ser Val Tyr Leu Arg Pro Asp Asp Arg Asn
                   405                 410                 415
   Lys Pro Leu Leu Gly Gly Thr Ser Val Gln Asp Val Val Arg Tyr Cys
               420                 425                 430
   Phe Glu Ile Gly Thr Ile Leu Gly Val Leu Cys Tyr Leu Cys Phe Gln
           435                 440                 445
   Gln Gly Asp Glu Ile Arg Asn Gln Gly Leu Ile Ser Phe Leu Lys Gln
       450                 455                 460
   Leu Pro His Asp Pro Ala Lys Phe Ile Phe Leu Ile Ser Asn Leu Leu
   465                 470                 475                 480
   Ile Leu Ala Cys Ile Pro Tyr Arg Val Ala Gly Asp Thr Asp Thr Glu
                   485                 490                 495
   Glu Ala Ile Leu Val Phe Ala Val Pro Ser Ser Trp Phe Leu Leu Met
               500                 505                 510
   Phe Phe Ala Gly Ala Ile Arg Leu Thr Gly Pro Phe Val Thr Met Ile
           515                 520                 525
   Tyr Ser Met Ile Thr Gly Asp Met Leu Thr Phe Gly Ile Ile Tyr Ser
       530                 535                 540
   Val Phe Leu Phe Gly Phe Ser Gln Ser Phe Phe Phe Leu Tyr Lys Gly
   545                 550                 555                 560
   Ser Lys Asn Val Ser Ser Ser Leu Phe Thr Ser Tyr Pro Ser Thr Trp
                   565                 570                 575
   Met Ala Leu Phe Gln Val Thr Met Gly Asp Tyr Asn Tyr Asn Asp Leu
               580                 585                 590
   Ser Leu Thr Ala Tyr Pro Ala Ile Ser Lys Met Val Phe Thr Ile Phe
           595                 600                 605
   Met Val Leu Val Pro Ile Leu Leu Leu Asn Met Leu Ile Ala Met Met
       610                 615                 620
   Gly Asn Thr Tyr Ala His Val Ile Glu Gln Ser Glu Lys Glu Trp Met
   625                 630                 635                 640
   Lys Gln Trp Ala Lys Ile Val Val Ala Leu Glu Arg Ala Val Asn Gln
                   645                 650                 655
   Glu Asp Cys His Arg Tyr Leu Gln Glu Tyr Ser Ile Lys Leu Gly Pro
               660                 665                 670
   Gly Asp Asp Pro Ser Thr Glu Gln Arg Gly Val Leu Val Ile Lys Ser
           675                 680                 685
   Lys Ser Lys Thr Arg Ala Lys Gln Arg Lys Gly Ala Leu Cys Asn Trp
       690                 695                 700
   Lys Arg Val Gly Lys Val Thr Ile Arg Glu Leu His Lys Arg Gly Met
   705                 710                 715                 720
   Thr Gly Glu Gln Leu Arg Arg Leu Met Trp Gly Arg Ser Ser Ile Ser
                   725                 730                 735
   Thr Pro Val Lys Pro Ala Pro Ile Lys Leu Gly His Val Thr Ser Ile
               740                 745                 750
   Ser Gly Val Ala Asp Ile Thr Val Pro Glu Thr Thr Gly Asn Thr Val
           755                 760                 765
   Gly Thr Gly Gly Gly Gly Leu Leu Ala Ala Leu Asp Val Met Ala Phe
       770                 775                 780
   Thr Asn Asp Leu Glu Phe Ser Ser Glu Asn Gln Thr Ser Thr Ser Phe
   785                 790                 795                 800
   Lys Thr Gln Pro Gln Pro Ser Glu Val Ser Cys Val Glu Ser Asp Ser
                   805                 810                 815
   Asn Ser Asp Pro Leu Tyr Asn Asp Pro Leu Arg Gln Leu Asn Glu Glu
               820                 825                 830
   Leu Asn Lys Thr Arg Glu Glu Ile Leu Thr Leu Ala Arg Val Ala Ala
           835                 840                 845
   Asn Asn Asp Gly Ile Glu Glu Ile Pro Pro Gln Ile Cys Gln Tyr Gly
       850                 855                 860
   Trp Ile Asn Asn Tyr Asn Phe Phe Gly Asn Thr Phe Ala Gln Asn Gln
   865                 870                 875                 880
   Lys Val Glu Asp Pro Val Gln Gln Pro Thr Phe Gln Ser Thr Pro Asn
                   885                 890                 895
   His Ser Asp Ser Asp Gly Leu Gly Asp Gly Leu Leu Leu Gly Ser Asn
               900                 905                 910
   Arg Arg Leu Lys Arg Thr Arg Ser Ala Asn Ile Lys Arg Lys Phe Ser
           915                 920                 925
   Gly Ser Ser Thr Asn Glu Lys Asn Leu Leu Glu Pro Glu Asp Ser Ser
       930                 935                 940
   Ser Thr Asn Ser Glu Asp Phe Ser Cys Leu Thr Glu Asn Tyr Ile Asn
   945                 950                 955                 960
   Lys Asn Ile Lys Val Asn Ala Asn Phe Ser Glu Asn Ser Leu Arg Ser
                   965                 970                 975
   Asn Arg Ser Ser Ala Asn Leu Val Val Lys Lys Phe Gly Arg Arg Ser
               980                 985                 990
   Gly Leu Lys Ser Ser Thr Asn Arg  Ile Ala Pro Ala Asp  Leu Ser Pro
           995                 1000                 1005
   Val Gly  Asn Val Ser Lys Tyr  Ser Glu Thr Leu Cys  Asn Ser Tyr
   1010 1015 1020
   Cys Ile Thr Ser Ser Ser Asp Val Leu Tyr Gln Trp Ser Ile Lys
   1025 1030 1035
   Gly Ile Thr Asn Met Asn Thr Leu Leu Gly Leu Glu Asn Glu Asp
   1040 1045 1050
   Ser Met
   1055
<210> 92
   <211> 1109
   <212> PRT
   <213> Acromyrmex echinatior
<400> 92
   Met Gly Gly Val Cys Ser Cys Arg Val Gly Arg Asn Ser Gln Val Asn
   1               5                   10                  15
   Ala Gly Ser Ile Leu Asp Arg Val Ile Ser Gln Ala Ser Asn Glu Asp
               20                  25                  30
   Gln Cys Leu Leu Tyr Arg Leu Ala Asn Tyr Lys Lys Gly Gly Glu Leu
           35                  40                  45
   Ile Glu Ala Tyr Asn Gln Gly Gly Gln Pro Glu Val Glu Lys Leu Ile
       50                  55                  60
   Arg Glu Gln Phe Gly Ile Leu Met Tyr Met Asp Gly Lys Gly Gln Ile
   65                  70                  75                  80
   Ile Asn Arg Ala Glu Tyr Leu Arg Trp Lys Phe Arg Asp Leu Glu Gln
                   85                  90                  95
   Val Val Leu Pro Ile Glu Ala Ser Leu Ser Arg Phe Asp Pro Leu Ala
               100                 105                 110
   Gln Trp Asn Asp His Glu Ala Cys Trp Gln Met Gln Tyr Arg Gly Ser
           115                 120                 125
   Leu Gly Glu Thr Leu Leu His Val Leu Ile Ile Cys Asp Thr Arg Met
       130                 135                 140
   His Thr Arg Ile Ala Arg Ile Leu Leu Lys Cys Phe Pro Arg Leu Ala
   145                 150                 155                 160
   Ile Asp Val Val Glu Gly Glu Glu Tyr Leu Gly Ala Ser Ala Leu His
                   165                 170                 175
   Leu Ala Ile Ala Tyr Ala Asn Asn Glu Leu Val Gln Asp Leu Val Glu
               180                 185                 190
   Ala Gly Ala Ile Ile Ser Gln Arg Ala Ile Gly Ser Phe Phe Leu Pro
           195                 200                 205
   Arg Asp Gln Gln Arg Met Asn Pro Ala Arg Asn Thr Asp Tyr Glu Gly
       210                 215                 220
   Leu Ala Tyr Leu Gly Glu Tyr Pro Leu Ala Trp Ala Ala Cys Cys Ala
   225                 230                 235                 240
   Asn Glu Ser Val Tyr Asn Leu Leu Leu Asp Ser Gly Ala Asp Pro Asp
                   245                 250                 255
   Glu Gln Asp Thr Phe Gly Asn Met Ile Leu His Met Val Val Val Cys
               260                 265                 270
   Asp Lys Leu Asp Met Phe Gly Tyr Ala Leu Arg His Pro Lys Leu Pro
           275                 280                 285
   Ala Arg Asn Gly Ile Val Asn Ala Ala Gly Leu Thr Pro Leu Thr Leu
       290                 295                 300
   Ala Cys Gln Leu Gly Arg Ala Glu Val Phe Arg Glu Met Leu Glu Leu
   305                 310                 315                 320
   Ser Ala Arg Glu Phe Trp Arg Tyr Ser Asn Ile Thr Cys Ser Ala Tyr
                   325                 330                 335
   Pro Leu Asn Ala Leu Asp Thr Leu Leu Pro Asp Gly Arg Thr Asn Trp
               340                 345                 350
   Asn Ser Ala Leu Phe Ile Ile Leu Asn Gly Thr Lys Glu Glu His Leu
           355                 360                 365
   Asp Met Leu Asp Gly Gly Ile Ile Gln Arg Leu Leu Glu Glu Lys Trp
       370                 375                 380
   Lys Thr Phe Ala Arg Leu Gln Phe Leu Lys Arg Leu Ile Ile Leu Val
   385                 390                 395                 400
   Phe His Leu Ala Ser Leu Ser Leu Ala Met Tyr Phe Arg Pro Ala Asp
                   405                 410                 415
   Met Glu Ala Val Leu Leu Gln Trp Pro Glu Glu Ile Thr Asp Val Val
   420 425 430
   Arg Thr Ala Ala Glu Cys Ile Thr Val Leu Gly Val Leu Asn Tyr Ile
   435 440 445
   Leu Val Gln Leu Gly Cys Glu Met Ile Asn Val Gly Pro Leu Ser Phe
   450 455 460
   Leu Lys Gln Leu Ser His Glu Pro Ala Lys Leu Ile Phe Val Ile Ser
   465 470 475 480
   Asn Leu Leu Ile Leu Ala Cys Ile Pro Cys Arg Ile Ala Gly Asp Arg
   485 490 495
   His Ala Glu Asp Ala Ile Leu Met Phe Ala Val Pro Gly Ser Trp Phe
   500 505 510
   Leu Leu Met Phe Phe Ala Gly Ala Ile Arg Leu Thr Gly Pro Phe Val
   515 520 525
   Thr Met Val Tyr Ser Met Ile Thr Gly Asp Met Leu Thr Phe Gly Ile
   530 535 540
   Ile Tyr Thr Val Met Leu Phe Gly Phe Ser Gln Ser Phe Tyr Phe Leu
   545 550 555 560
   Tyr Lys Gly Phe Pro Gly Val Lys Ser Ser Leu Tyr His Ser Tyr Pro
   565 570 575
   Ser Thr Trp Met Ala Leu Phe Gln Ile Thr Leu Gly Asp Tyr Asn Tyr
   580 585 590
   Ala Asp Leu Ser Asn Thr Thr Tyr Pro Asn Leu Ser Lys Thr Val Phe
   595 600 605
   Ala Ile Phe Met Val Leu Val Pro Ile Leu Leu Leu Asn Met Leu Ile
   610 615 620
   Ala Met Met Gly Asn Thr Tyr Ala His Val Ile Glu Gln Ser Glu Lys
   625 630 635 640
   Glu Phe Val Lys Gln Trp Ala Lys Ile Val Val Ser Leu Glu Arg Ala
   645 650 655
   Val Ser Gln Lys Asp Ala His Asn Tyr Leu Gln Glu Tyr Ser Ile Lys
   660 665 670
   Leu Gly Pro Gly Asp Asp Pro Asn Asn Pro Ala Ser Glu Gln Arg Gly
   675 680 685
   Val Leu Val Ile Lys Ser Lys Ser Lys Thr Arg Ala Lys Gln Arg Lys
   690 695 700
   Gly Ala Val Ser Asn Trp Lys Arg Val Gly Lys Val Thr Ile Leu Glu
   705 710 715 720
   Leu Arg Lys Arg Gly Met Thr Gly Glu Glu Leu Arg Arg Ile Met Trp
   725 730 735
   Gly Arg Ala Ser Phe Ser Thr Pro Val Arg Val Ser Pro Asn Ala Gly
   740 745 750
   Gln Pro Gln Val Ser Ala Val Thr Ala Gly Phe Asp Asp Ala Leu Thr
   755 760 765
   Ala Ala Leu Asp Val Met Ala Phe Ala His Asp Phe Asp Leu Ser Thr
   770 775 780
   Ala Thr Glu Gly Ile Pro Thr Asn Ile Asp Ala Lys Gln Ile Lys Thr
   785 790 795 800
   Ala Gln Ser Lys Ala Ala Leu Asn Asp Gln Gln Thr Lys Thr Thr Gln
   805 810 815
   Asn Asn Pro Glu Val Val Ile Asn Val Gln Pro His Asn Gln Pro Ile
   820 825 830
   Lys Glu Glu Thr Lys Leu Ser Gly Leu Thr Asp Leu Ile Asp Lys Ser
   835 840 845
   Ile Asp Pro Val Lys Asp Val Glu Ala Met Asn Leu Arg Asn Met Asn
   850 855 860
   Gln Thr Lys Ala Leu Glu Glu Thr Val Glu Asp Pro Phe Leu Glu Leu
   865 870 875 880
   Ala Ile Ala Ser Glu Thr Thr Thr Asp Tyr Lys Thr Leu Leu Gln Ile
   885 890 895
   Ala Lys Ser Ala Leu Ala Thr Ser Glu Ala Ser Met Lys Thr Thr Ile
   900 905 910
   Asp Pro Gln Ile Leu Ala His Phe Ala Met Val Ala Pro Pro Thr Ile
   915 920 925
   Glu Lys Ser Ser Ile Val Lys Lys Gln Tyr Phe Met Glu Ser Ser Asp
   930 935 940
   Asn Asp Leu Gly Gly Asp Asn Leu Leu Gly Thr Glu Ala Arg Leu Arg
   945 950 955 960
   Arg Ile Lys Ser Ala Asn Asn Arg Phe Thr Thr Ser Lys Arg Ser Arg
   965 970 975
   Arg Asp Asp Asp Gly Asp Ser Ser Ser Ser Thr Thr Ser Val Glu Gln
   980 985 990
   Ser Leu Arg Tyr Arg Pro Leu Leu Asn Asp Pro Glu Gln Arg Pro Met
   995 1000 1005
   Asn Arg Ile Glu Thr Glu Gly Arg Lys Thr Thr Val Glu Thr Ile
   1010 1015 1020
   Lys Pro Glu Val Lys Gln Asn Gly Ser Ile Pro Thr Lys Pro Leu
   1025 1030 1035
   Asp Lys Ile Gln Lys Arg Arg Pro Lys Thr Ala Lys Asn Arg Val
   1040 1045 1050
   Ser Pro Lys Glu Thr Glu Glu Pro Ala Arg Arg Arg Glu Ser Ile
   1055 1060 1065
   Glu Arg Asn Lys Ala Ala Ser Pro Pro Thr Ser Ser Ser Asp Pro
   1070 1075 1080
   Leu Glu Pro Trp Ser Thr Arg Gly Ile Thr Asp Met Asn Thr Ile
   1085 1090 1095
   Leu Ala Trp Arg Glu Asn Ala Pro Asp Ser Pro
   1100 1105
<210> 93
   <211> 1112
   <212> PRT
   <213> Harpegnathos saltator
<400> 93
   Met Gly Gly Val Cys Ser Cys Arg Gly Arg Gly Ser Gln Val Asn Ala
   1 5 10 15
   Gly Ser Ile Leu Asp Arg Val Ile Ser Gln Ala Ser Asn Glu Asp Gln
   20 25 30
   Cys Leu Leu Tyr Arg Leu Ala Asn Tyr Lys Lys Gly Gly Glu Leu Ile
           35                  40                  45
   Glu Ala Tyr Asn Gln Gly Gly Gln Pro Glu Val Glu Arg Leu Ile Arg
       50                  55                  60
   Glu Gln Phe Gly Ile Leu Met Tyr Ala Asp Gly Lys Gly Gln Val Ile
   65                  70                  75                  80
   Asn Arg Ala Glu Tyr Leu Arg Trp Lys Phe Arg Asp Leu Glu Gln Val
                   85                  90                  95
   Val Leu Pro Ile Glu Ala Ser Leu Ser Arg Phe Asp Pro Leu Ala Gln
               100                 105                 110
   Trp Tyr Asp His Glu Ala Cys Trp Gln Met Gln Tyr Arg Gly Ser Leu
           115                 120                 125
   Gly Glu Ser Leu Leu His Val Leu Ile Ile Cys Asp Thr Arg Met His
       130                 135                 140
   Thr Arg Ile Ala Arg Thr Leu Leu Lys Cys Phe Pro Arg Leu Ala Ile
   145                 150                 155                 160
   Asp Val Val Glu Gly Glu Glu Tyr Leu Gly Ala Ser Ala Leu His Leu
                   165                 170                 175
   Ala Ile Ala Tyr Asn Asn Asn Glu Leu Val Gln Asp Leu Val Glu Ala
               180                 185                 190
   Gly Ala Ile Ile Ser Gln Arg Ala Ile Gly Ser Phe Phe Leu Pro Arg
           195                 200                 205
   Asp Gln Gln Arg Met Asn Pro Val Arg Asn Thr Asp Tyr Glu Gly Leu
       210                 215                 220
   Ala Tyr Leu Gly Glu Tyr Pro Leu Ala Trp Ala Ala Cys Cys Ala Asn
   225                 230                 235                 240
   Glu Ser Val Tyr Asn Leu Leu Leu Asp Ser Gly Ala Asp Pro Asp Glu
                   245                 250                 255
   Gln Asp Ser Phe Gly Asn Met Ile Leu His Met Val Val Val Cys Asp
               260                 265                 270
   Lys Leu Asp Met Phe Gly Tyr Ala Leu Arg His Pro Lys Leu Pro Thr
           275                 280                 285
   Arg Asn Gly Ile Val Asn Ala Ala Gly Leu Thr Pro Leu Thr Leu Ala
       290                 295                 300
   Cys Gln Leu Gly Arg Ala Glu Val Phe Arg Glu Met Leu Glu Leu Ser
   305                 310                 315                 320
   Ala Arg Glu Phe Trp Arg Tyr Ser Asn Ile Thr Cys Ser Ala Tyr Pro
                   325                 330                 335
   Leu Asn Ala Leu Asp Thr Leu Leu Pro Asp Gly Arg Thr Asn Trp Asn
               340                 345                 350
   Ser Ala Leu Phe Ile Ile Leu Asn Gly Thr Lys Glu Glu His Leu Asp
           355                 360                 365
   Met Leu Asp Gly Gly Val Ile Gln Arg Leu Leu Glu Glu Lys Trp Lys
       370                 375                 380
   Thr Phe Ala Arg Leu Gln Phe Leu Lys Arg Leu Ile Ile Leu Val Phe
   385                 390                 395                 400
   His Leu Ala Ser Leu Ser Leu Ala Val Tyr Phe Arg Pro Ala Asp Met
                   405                 410                 415
   Asp Ala Glu Leu Leu Gln Trp Pro Asp Glu Ile Thr Asp Ala Ala Arg
               420                 425                 430
   Ile Ala Ala Glu Cys Ser Thr Val Leu Gly Val Leu Ser Tyr Ile Leu
           435                 440                 445
   Val Gln Leu Gly Gly Glu Met Ile Asn Ile Gly Pro Leu Ser Phe Leu
       450                 455                 460
   Lys Gln Leu Ser His Glu Pro Ala Lys Leu Ile Phe Val Ile Ser Asn
   465                 470                 475                 480
   Leu Leu Ile Leu Ala Cys Ile Pro Cys Arg Ile Ala Gly Asn Arg His
                   485                 490                 495
   Ala Glu Asp Ala Ile Leu Val Phe Ala Val Pro Gly Ser Trp Phe Leu
               500                 505                 510
   Leu Met Phe Phe Ala Gly Ala Ile Arg Leu Thr Gly Pro Phe Val Thr
           515                 520                 525
   Met Val Tyr Ser Met Ile Thr Gly Asp Met Leu Thr Phe Gly Ile Ile
       530                 535                 540
   Tyr Thr Val Met Leu Phe Gly Phe Ser Gln Ser Phe Tyr Phe Leu Tyr
   545 550 555 560
   Lys Gly Phe Pro Gly Val Lys Ser Ser Leu Tyr His Ser Tyr Pro Ser
   565 570 575
   Thr Trp Met Ala Leu Phe Gln Ile Thr Leu Gly Asp Tyr Asn Tyr Ala
   580 585 590
   Asp Leu Ser Asn Thr Thr Tyr Pro Asn Leu Ser Lys Thr Val Phe Ala
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Phe Val Lys Gln Trp Ala Lys Ile Val Val Ser Leu Glu Arg Ala Val
   645 650 655
   Ser Gln Lys Asp Ala His Asn Tyr Leu Gln Glu Tyr Ser Ile Lys Leu
   660 665 670
   Gly Pro Gly Asp Asp Pro Asn Asn Pro Ala Ser Glu Gln Arg Gly Val
   675 680 685
   Leu Ile Ile Lys Ser Lys Ser Lys Thr Arg Ala Lys Gln Arg Lys Gly
   690 695 700
   Ala Val Ala Asn Trp Lys Arg Val Gly Lys Val Thr Ile Asn Glu Leu
   705 710 715 720
   Arg Lys Arg Gly Met Thr Gly Glu Glu Leu Arg Arg Ile Met Trp Asp
   725 730 735
   Arg Ala Ser Ile Ser Thr Pro Val Arg Val Ser Pro Asn Val Asn Glu
   740 745 750
   Pro Gln Val Ser Ala Val Thr Ala Gly Phe Gly Asp Ala Leu Thr Ala
   755 760 765
   Ala Leu Asp Val Met Ala Phe Ala His Asp Phe Asp Leu Ser Thr Ala
   770 775 780
   Thr Glu Gly Val Pro Ser Asn Ile Asp Thr Arg Gln Thr Lys Ala Ile
   785 790 795 800
   Pro Asp Glu Gln Thr Arg Thr Ala Arg Asn Asn Pro Lys Ala Val Thr
   805 810 815
   Asp Val Gln Pro His Lys Gln Ala Ile Gly Gly Glu Glu Thr Lys Leu
   820 825 830
   Ser Gly Leu Thr Asp Leu Ile Asp Met Ser Ala Ala Glu Pro Ala Arg
   835 840 845
   Val Glu Ala Met Asn Leu Lys Asn Ala Ser Gln Ala Lys Ala His Glu
   850 855 860
   Gly Ala Val Glu Asp Pro Phe Leu Glu Leu Ala Ile Ala Ser Glu Thr
   865 870 875 880
   Thr Glu Asp Tyr Asp Thr Leu Leu Gln Met Ala Lys Ser Ala Leu Ala
   885 890 895
   Thr Ser Glu Ala Ser Thr Thr Val Asp Pro Gln Ile Leu Ala His Phe
   900 905 910
   Thr Met Ile Ala Pro Pro Pro Val Glu Lys Pro Ile Val Val Lys Lys
   915 920 925
   Gln Tyr Leu Val Glu Ser Ser Asp Asn Asp Leu Gly Gly Asp Asn Leu
   930 935 940
   Leu Gly Thr Glu Ala Arg Ile Arg Arg Ile Lys Ser Ala Asn Asp Arg
   945 950 955 960
   Phe Ile Ser Thr Ser Lys Arg Ser His His Asp Asp Asp Asn Asp Ser
   965 970 975
   Ser Ser Ser Val Thr Ser Val Asp Arg Ser Gln Pro Gln Tyr Arg Pro
   980 985 990
   Leu Leu Asn Asp Ser Glu Glu Ser Leu Ala Asn Arg Val Glu Asn Glu
   995 1000 1005
   Gly Arg Arg Thr Ser Asp Val Glu Thr Ile Lys Ser Glu Val Lys
   1010 1015 1020
   Gln Asn Gly Arg Gly Gly Gly Gly Gly Gly Val Ser Lys Pro Ser
   1025 1030 1035
   Asp Lys Arg Val Gln Lys Arg Arg Pro Lys Thr Ala Lys Asn Arg
   1040 1045 1050
   Val Ser Pro Lys Glu Ile Glu Glu Pro Ala Arg Arg Arg Glu Glu
   1055 1060 1065
   Ser Ile Glu Arg Asn Lys Ala Ala Leu Ser Pro Pro Ala Ser Pro
   1070 1075 1080
   Ser Asp Pro Leu Glu Pro Trp Ser Thr Arg Gly Ile Thr Asp Met
   1085 1090 1095
   Asn Thr Ile Leu Ala Trp Arg Glu Asn Ala Pro Asp Ser Pro
   1100 1105 1110
<210> 94
   <211> 1104
   <212> PRT
   <213> Bombus impatiens
<400> 94
   Met Gly Gly Val Cys Ser Cys Arg Gly Arg Gly Ser Gln Val Asn Ala
   1               5                   10                  15
   Gly Ser Ile Leu Asp Arg Val Ile Ser His Ala Ser Asp Glu Asp Gln
               20                  25                  30
   Cys Leu Leu Tyr Arg Leu Ala Asn Tyr Lys Lys Gly Gly Glu Leu Ile
           35                  40                  45
   Glu Ser Tyr Asn Gln Gly Gly Gln Ala Glu Val Glu Lys Leu Ile Arg
       50                  55                  60
   Glu Gln Phe Gly Val Leu Met Tyr Ala Asp Gly Lys Gly Gln Ala Ile
   65                  70                  75                  80
   Asn Arg Ala Glu Tyr Leu Arg Trp Lys Phe Arg Asp Leu Glu Gln Val
                   85                  90                  95
   Val Leu Pro Ile Glu Ala Ser Leu Ser Gln Phe Asp Pro Leu Ala Gln
               100                 105                 110
   Trp Asn Asp His Glu Ala Cys Trp Gln Met Gln Tyr Arg Gly Ser Leu
           115                 120                 125
   Gly Glu Thr Leu Leu His Val Leu Ile Ile Cys Asp Thr Arg Ile His
       130                 135                 140
   Thr Arg Leu Ala Arg Ile Leu Leu Lys Cys Phe Pro Arg Leu Ala Ile
   145 150 155 160
   Asp Val Val Glu Gly Glu Glu Tyr Leu Gly Ala Ser Ala Leu His Leu
   165 170 175
   Ala Ile Ala Tyr Asn Asn Asn Glu Leu Val Gln Asp Leu Val Glu Ala
   180 185 190
   Gly Ala Ile Ile Ser Gln Arg Ala Ile Gly Ser Phe Phe Leu Pro Arg
   195 200 205
   Asp Gln Gln Arg Thr Asn Pro Ala Lys Asn Thr Asp Tyr Glu Gly Leu
   210 215 220
   Ala Tyr Leu Gly Glu Tyr Pro Leu Ala Trp Ala Ala Cys Cys Ala Asn
   225 230 235 240
   Glu Ser Val Tyr Asn Leu Leu Leu Asp Ser Gly Ala Asp Pro Asp Glu
   245 250 255
   Gln Asp Ser Phe Gly Asn Met Ile Leu His Met Val Val Val Cys Asp
   260 265 270
   Lys Leu Asp Met Phe Gly Tyr Ala Leu Arg His Pro Lys Leu Pro Ala
   275 280 285
   Arg Asn Gly Ile Val Asn Ala Ala Gly Leu Thr Pro Leu Thr Leu Ala
   290 295 300
   Cys Gln Leu Gly Arg Ala Glu Val Phe Arg Glu Met Leu Glu Leu Ser
   305 310 315 320
   Ala Arg Glu Phe Trp Arg Tyr Ser Asn Ile Thr Cys Ser Ala Tyr Pro
   325 330 335
   Leu Asn Ala Leu Asp Thr Leu Leu Pro Asp Gly Arg Thr Asn Trp Asn
   340 345 350
   Ser Ala Leu Phe Ile Ile Leu Asn Gly Thr Lys Glu Glu His Leu Asp
   355 360 365
   Met Leu Asp Gly Gly Ile Ile Gln Arg Leu Leu Glu Glu Lys Trp Lys
   370 375 380
   Thr Phe Ala Arg Leu Gln Phe Leu Lys Arg Leu Ile Ile Leu Val Phe
   385 390 395 400
   His Leu Thr Ser Leu Ser Leu Ala Val Tyr Met Arg Pro Ser Asn Ile
   405 410 415
   Asp Ala Thr Leu Leu Glu Trp Pro Glu Glu Ile Thr Glu Val Gly Arg
   420 425 430
   Met Ile Ala Glu Cys Thr Thr Val Ile Gly Val Leu Ser Tyr Ile Leu
   435 440 445
   Val Gln Leu Gly Gly Glu Val Val Asn Ile Gly Leu Leu Ser Phe Met
   450 455 460
   Lys Gln Leu Ser His Glu Pro Ala Lys Leu Ile Phe Leu Ile Ser Asn
   465 470 475 480
   Leu Leu Ile Leu Ala Cys Ile Pro Cys Arg Leu Ala Gly Asn Arg His
   485 490 495
   Ala Glu Asp Ala Ile Leu Val Val Ala Val Pro Gly Ser Trp Phe Leu
   500 505 510
   Leu Met Phe Phe Ala Gly Ala Ile Arg Leu Thr Gly Pro Phe Val Thr
   515 520 525
   Met Val Tyr Ser Met Ile Thr Gly Asp Met Leu Thr Phe Gly Ile Ile
   530 535 540
   Tyr Met Val Val Leu Phe Gly Phe Cys Gln Ser Phe Tyr Phe Leu Tyr
   545 550 555 560
   Lys Gly Phe Pro Gly Val Lys Ser Ser Leu Tyr Ser Ser Tyr His Ser
   565 570 575
   Thr Trp Met Ala Leu Phe Gln Ile Thr Leu Gly Asp Tyr Asn Tyr Thr
   580 585 590
   Asp Leu Gly Tyr Thr Thr Tyr Pro Asn Leu Ser Lys Met Val Phe Ala
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Trp Val Lys Gln Trp Ala Lys Ile Val Val Ser Leu Glu Arg Ala Val
   645 650 655
   Ala Gln Lys Asp Ala Gln Asn Tyr Leu Gln Glu Tyr Ser Ile Lys Leu
               660                 665                 670
   Gly Pro Gly Asp Asp Pro Asn Asn Pro Ala Ser Glu Gln Arg Gly Val
           675                 680                 685
   Leu Ile Ile Lys Ser Lys Ser Lys Thr Lys Ala Lys Gln Arg Lys Gly
       690                 695                 700
   Ala Val Ala Asn Trp Lys Arg Val Gly Lys Val Thr Ile Asn Glu Leu
   705                 710                 715                 720
   Lys Lys Arg Gly Ile Thr Gly Glu Ala Leu Arg Arg Val Met Trp Gly
                   725                 730                 735
   Arg Ala Ser Phe Ser Thr Pro Val Arg Thr Ser Pro Asn Thr Ala Glu
               740                 745                 750
   Pro Gln Val Ser Ala Val Thr Ala Gly Phe Gly Asp Ala Leu Thr Ala
           755                 760                 765
   Ala Leu Asp Val Met Ala Phe Ala His Asp Leu Asp Leu Ser Thr Ala
       770                 775                 780
   Thr Glu Gly Ile Pro Thr Asn Ile Gly Ala Lys Gln Ser Lys Ser Lys
   785                 790                 795                 800
   Thr Ala Cys Lys Asp Gln Ala Lys Ser Ala Thr Asn Asn Gln Glu Lys
                   805                 810                 815
   Gly Ser Thr Asp Val Gln Gln Gln Arg Lys Leu Thr Thr Glu Asn Leu
               820                 825                 830
   Asp Asn Leu Ala Ser Ser His Asn Asp Ile Asp Lys Thr Ser Ser Glu
           835                 840                 845
   Phe Val Ala Val Arg Glu Met Asn Ser Lys Asn Ala Ser His Ser Ser
       850                 855                 860
   Met Ala Glu Asp Phe Gln Asp Pro Leu Leu Glu Leu Val Ile Ala Ser
   865                 870                 875                 880
   Glu Asn Thr Asp Asp Pro Glu Thr Leu Leu Lys Ile Ala Glu Arg Ala
                   885                 890                 895
   Ala Ala Asn Phe Glu Val Glu Ser Ser Ser Lys Ile Asn Leu Gln Ile
               900                 905                 910
   Leu Glu Gln Phe Thr Met Thr Lys Leu Pro Met Glu Glu Lys Val Ala
   915 920 925
   Ala Thr Lys Lys Gln Tyr Phe Val Glu Ser Ser Asp Asn Asp Phe Gly
   930 935 940
   Gly Asp Asn Leu Leu Gly Thr Glu Ala Arg Leu Arg Arg Ile Arg Ser
   945 950 955 960
   Ala Asn Asn Arg Phe Ile Thr Thr Arg Arg Arg Ser Arg His Thr Asp
   965 970 975
   Asp Asp Leu Ser Ser Thr Ser Ser Thr Ser Ala Asp Arg Asn Pro Gln
   980 985 990
   Tyr Gln Pro Leu Leu Asn Gly Pro Glu Asn Leu Ala Ser Arg Gln Ile
   995 1000 1005
   Glu Ser Arg Glu Ser Ser Ile Glu Ala Ile Lys Pro Gln Val Lys
   1010 1015 1020
   Gln Asn Gly Ser Cys Asp Ser Val Lys Ile Lys Val Gln Lys Arg
   1025 1030 1035
   Arg Pro Lys Thr Ala Lys Asn Arg Val Ser Pro Lys Glu Ile Glu
   1040 1045 1050
   Glu Ala Gly Pro Arg Arg Arg Glu Ser Ile Glu Arg Asn Lys Ala
   1055 1060 1065
   Val Ser Pro Ala Thr Ser Pro Thr Asp Pro Leu Glu Pro Trp Ser
   1070 1075 1080
   Thr Arg Gly Ile Lys Asp Met Asn Thr Ile Leu Ala Trp Glu Glu
   1085 1090 1095
   Asn Thr Pro Asp Ser Pro
   1100
<210> 95
   <211> 1024
   <212> PRT
   <213> Bombus terrestris
<400> 95
   Met Gly Gly Val Cys Ser Cys Arg Gly Arg Gly Ser Gln Val Asn Ala
   1 5 10 15
   Gly Ser Ile Leu Asp Arg Val Ile Ser His Ala Ser Asp Glu Asp Gln
               20                  25                  30
   Cys Leu Leu Tyr Arg Leu Ala Asn Tyr Lys Lys Gly Gly Glu Leu Ile
           35                  40                  45
   Glu Ser Tyr Asn Gln Gly Gly Gln Ala Glu Val Glu Lys Leu Ile Arg
       50                  55                  60
   Glu Gln Phe Gly Val Leu Met Tyr Ala Asp Gly Lys Gly Gln Ala Ile
   65                  70                  75                  80
   Asn Arg Ala Glu Tyr Leu Arg Trp Lys Phe Arg Asp Leu Glu Gln Val
                   85                  90                  95
   Val Leu Pro Ile Glu Ala Ser Leu Ser Gln Phe Asp Pro Leu Ala Gln
               100                 105                 110
   Trp Asn Asp His Glu Ala Cys Trp Gln Met Gln Tyr Arg Gly Ser Leu
           115                 120                 125
   Gly Glu Thr Leu Leu His Val Leu Ile Ile Cys Asp Thr Arg Ile His
       130                 135                 140
   Thr Arg Leu Ala Arg Ile Leu Leu Lys Cys Phe Pro Arg Leu Ala Ile
   145                 150                 155                 160
   Asp Val Val Glu Gly Glu Glu Tyr Leu Gly Ala Ser Ala Leu His Leu
                   165                 170                 175
   Ala Ile Ala Tyr Asn Asn Asn Glu Leu Val Gln Asp Leu Val Glu Ala
               180                 185                 190
   Gly Ala Ile Ile Ser Gln Arg Ala Ile Gly Ser Phe Phe Leu Pro Arg
           195                 200                 205
   Asp Gln Gln Arg Thr Asn Pro Ala Lys Asn Thr Asp Tyr Glu Gly Leu
       210                 215                 220
   Ala Tyr Leu Gly Glu Tyr Pro Leu Ala Trp Ala Ala Cys Cys Ala Asn
   225                 230                 235                 240
   Glu Ser Val Tyr Asn Leu Leu Leu Asp Ser Gly Ala Asp Pro Asp Glu
                   245                 250                 255
   Gln Asp Ser Phe Gly Asn Met Ile Leu His Met Val Val Val Cys Asp
               260                 265                 270
   Lys Leu Asp Met Phe Gly Tyr Ala Leu Arg His Pro Lys Leu Pro Ala
           275                 280                 285
   Arg Asn Gly Ile Val Asn Ala Ala Gly Leu Thr Pro Leu Thr Leu Ala
       290                 295                 300
   Cys Gln Leu Gly Arg Ala Glu Val Phe Arg Glu Met Leu Glu Leu Ser
   305                 310                 315                 320
   Ala Arg Glu Phe Trp Arg Tyr Ser Asn Ile Thr Cys Ser Ala Tyr Pro
                   325                 330                 335
   Leu Asn Ala Leu Asp Thr Leu Leu Pro Asp Gly Arg Thr Asn Trp Asn
               340                 345                 350
   Ser Ala Leu Phe Ile Ile Leu Asn Gly Thr Lys Glu Glu His Leu Asp
           355                 360                 365
   Met Leu Asp Gly Gly Ile Ile Gln Arg Leu Leu Glu Glu Lys Trp Lys
       370                 375                 380
   Thr Phe Ala Arg Leu Gln Phe Leu Lys Arg Leu Ile Ile Leu Val Phe
   385                 390                 395                 400
   His Leu Thr Ser Leu Ser Leu Ala Val Tyr Met Arg Pro Ser Asn Ile
                   405                 410                 415
   Asp Ala Thr Leu Leu Glu Trp Pro Glu Glu Ile Thr Glu Val Gly Arg
               420                 425                 430
   Met Ile Ala Glu Cys Thr Thr Val Ile Gly Val Leu Ser Tyr Ile Leu
           435                 440                 445
   Val Gln Leu Gly Gly Glu Val Val Asn Ile Gly Leu Leu Ser Phe Met
       450                 455                 460
   Lys Gln Leu Ser His Glu Pro Ala Lys Leu Ile Phe Leu Ile Ser Asn
   465                 470                 475                 480
   Leu Leu Ile Leu Ala Cys Ile Pro Cys Arg Leu Ala Gly Asn Arg His
                   485                 490                 495
   Ala Glu Asp Ala Ile Leu Val Val Ala Val Pro Gly Ser Trp Phe Leu
               500                 505                 510
   Leu Met Phe Phe Ala Gly Ala Ile Arg Leu Thr Gly Pro Phe Val Thr
   515 520 525
   Met Val Tyr Ser Met Ile Thr Gly Asp Met Leu Thr Phe Gly Ile Ile
   530 535 540
   Tyr Met Val Val Leu Phe Gly Phe Cys Gln Ser Phe Tyr Phe Leu Tyr
   545 550 555 560
   Lys Gly Phe Pro Gly Val Lys Ser Ser Leu Tyr Ser Ser Tyr His Ser
   565 570 575
   Thr Trp Met Ala Leu Phe Gln Ile Thr Leu Gly Asp Tyr Asn Tyr Thr
   580 585 590
   Asp Leu Gly Tyr Thr Thr Tyr Pro Asn Leu Ser Lys Met Val Phe Ala
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Trp Val Lys Gln Trp Ala Lys Ile Val Val Ser Leu Glu Arg Ala Val
   645 650 655
   Ala Gln Lys Asp Ala Gln Asn Tyr Leu Gln Glu Tyr Ser Ile Lys Leu
   660 665 670
   Gly Pro Gly Asp Asp Pro Asn Asn Pro Ala Ser Glu Gln Arg Gly Val
   675 680 685
   Leu Val Ile Lys Ser Lys Ser Lys Thr Lys Ala Lys Gln Arg Lys Gly
   690 695 700
   Ala Val Ala Asn Trp Lys Arg Val Gly Lys Val Thr Ile Asn Glu Leu
   705 710 715 720
   Lys Lys Arg Gly Ile Thr Gly Glu Ala Leu Arg Arg Val Met Trp Gly
   725 730 735
   Arg Ala Ser Phe Ser Thr Pro Arg Asp Thr Leu Arg Ala Leu Gly Ser
   740 745 750
   Val Gly Ile Val Arg Ala Val Gly Thr Leu Gly Pro Val Gly Thr Val
   755 760 765
   Arg Ala Val Gly Ala Leu Gly Pro Val Gly Thr Val Arg Asp Ser Ser
   770 775 780
   Met Ala Glu Asp Phe Gln Asp Pro Leu Leu Glu Leu Val Ile Ala Ser
   785 790 795 800
   Glu Asn Thr Asp Asp Pro Glu Thr Leu Leu Lys Ile Ala Glu Arg Ala
   805 810 815
   Ala Ala Asn Phe Glu Val Glu Ser Ser Ser Lys Ile Asn Leu Gln Ile
   820 825 830
   Leu Glu Gln Phe Thr Met Thr Lys Leu Pro Met Glu Glu Lys Val Ala
   835 840 845
   Ala Thr Lys Lys Gln Tyr Phe Val Glu Ser Ser Asp Asn Asp Phe Gly
   850 855 860
   Gly Asp Asn Leu Leu Gly Thr Glu Ala Arg Leu Arg Arg Ile Arg Ser
   865 870 875 880
   Ala Asn Asn Arg Phe Ile Thr Thr Arg Arg Arg Ser Arg His Thr Asp
   885 890 895
   Asp Asp Leu Ser Ser Thr Ser Ser Thr Ser Ala Asp Arg Asn Pro Gln
   900 905 910
   Tyr Gln Pro Leu Leu Asn Gly Pro Glu Asn Leu Val Ser Arg Gln Ile
   915 920 925
   Glu Ser Arg Glu Ser Ser Ile Glu Ala Ile Lys Pro Gln Val Lys Gln
   930 935 940
   Asn Gly Ser Cys Asp Ser Val Lys Ile Lys Val Gln Lys Arg Arg Pro
   945 950 955 960
   Lys Thr Ala Lys Asn Arg Val Ser Pro Lys Glu Ile Glu Glu Ala Gly
   965 970 975
   Pro Arg Arg Arg Glu Ser Ile Glu Arg Asn Lys Ala Val Ser Pro Ala
   980 985 990
   Thr Ser Pro Thr Asp Pro Leu Glu Pro Trp Ser Thr Arg Gly Ile Lys
   995 1000 1005
   Asp Met Asn Thr Ile Leu Ala Trp Glu Glu Asn Thr Pro Asp Ser
   1010 1015 1020
   Pro
<210> 96
   <211> 949
   <212> PRT
   <213> Dendroctonus ponderosae
<400> 96
   Met Gly Gly Cys Gln Cys Lys Pro Cys Cys Lys His Lys Lys Ser His
   1 5 10 15
   Tyr Thr Gly Gly Ser Ile Leu Asp Arg Val Ile Ser Gln Ser Ser Asn
   20 25 30
   Gln Asp Gln Cys Leu Leu Tyr Arg Leu Ala Asn Tyr Lys Lys Gly Gly
   35 40 45
   Glu Leu Ile Asp Ala Tyr Asn Ala Gly Gly Gln Ile Glu Cys Glu Lys
   50 55 60
   Leu Ile Arg Asp Gln Phe Ala Gln Leu Met Tyr Gln Asp Gly Lys Gly
   65 70 75 80
   Lys Ile Ile Asn Arg Ser Glu Tyr Leu Arg Trp Lys Phe Arg Asp His
   85 90 95
   Glu His Val Ile Leu Pro Ile Glu Ala Ser Leu Ser Arg Tyr Asp Pro
   100 105 110
   Leu Gly Lys Trp Thr Asp His Glu Ala Cys Trp Gln Met Gln Tyr Arg
   115 120 125
   Gly Ser Leu Gly Glu Ser Leu Leu His Leu Leu Ile Ile Cys Asp Ser
   130 135 140
   Lys Ile His Thr Arg Leu Ala Arg Thr Leu Ile Lys Cys Phe Pro Lys
   145 150 155 160
   Leu Ala Leu Asp Val Val Glu Gly Glu Glu Tyr Leu Gly Ala Ser Ala
   165 170 175
   Leu His Leu Ala Ile Ala Tyr Ser Asn Asn Glu Leu Val Gln Asp Leu
   180 185 190
   Val Glu Ala Gly Ala Asn Val Asn Gln Arg Ala Ile Gly Lys Gln Ile
   195 200 205
   Ala Asp Lys Thr Gly Lys Ile Asn Ala Lys Asn Arg Ser Phe Phe Leu
       210                 215                 220
   Pro Arg Asp Gln Gln Lys Gln Arg Pro Ala Lys His Thr Asp Tyr Glu
   225                 230                 235                 240
   Gly Leu Ala Tyr Leu Gly Glu Tyr Pro Leu Ala Trp Ala Ala Cys Cys
                   245                 250                 255
   Ala Asn Glu Ser Val Tyr Asn Leu Leu Leu Asp Ser Gly Ala His Pro
               260                 265                 270
   Asp Leu Gln Asp Asn Phe Gly Asn Met Ile Leu His Met Val Val Val
           275                 280                 285
   Cys Asp Lys Leu Asp Met Phe Gly Tyr Ala Leu Arg His Pro Lys Leu
       290                 295                 300
   Pro Ala Ser Asn Gly Ile Ile Asn Lys Ala Gly Leu Thr Pro Leu Thr
   305                 310                 315                 320
   Leu Ala Cys Lys Leu Gly Arg Ala Glu Val Phe Arg Glu Met Leu Glu
                   325                 330                 335
   Leu Ser Cys Lys Glu Phe Trp Arg Tyr Ser Asn Ile Thr Cys Ser Ala
               340                 345                 350
   Tyr Thr Leu Asn Ala Leu Asp Thr Leu Leu Pro Asp Gly Arg Thr Asn
           355                 360                 365
   Trp Asn Ser Ala Leu Phe Ile Ile Leu Asn Gly Thr Lys Glu Ala His
       370                 375                 380
   Leu Asp Met Leu Asp Gly Gly Ile Ile Gln Arg Leu Leu Glu Glu Lys
   385                 390                 395                 400
   Trp Lys Ala Phe Ala Arg Asn Gln Phe Leu Lys Arg Leu Ile Ile Leu
                   405                 410                 415
   Ile Val His Leu Ala Phe Met Ser Leu Ala Val Tyr Met Arg Pro Asp
               420                 425                 430
   Asp Pro Asp Glu Pro Leu Leu Glu Trp Ser Asp Asp Pro Thr Val Ile
           435                 440                 445
   Ile Arg Phe Thr Ala Glu Ile Gly Thr Leu Leu Asn Val Leu Ser Tyr
       450                 455                 460
   Val Val Val Gln Gln Gly Asp Glu Ile Arg Asn Gln Gly Phe Leu Ser
   465                 470                 475                 480
   Phe Leu Lys Gln Gln Thr Asn Ser Pro Pro Lys Cys Ile Phe Leu Val
                   485                 490                 495
   Ser Asn Leu Met Ile Leu Ala Cys Ile Pro Cys Arg Leu Ala Gly Asn
               500                 505                 510
   Arg Gly Leu Glu Glu Thr Ile Leu Ile Phe Ala Val Pro Gly Ser Trp
           515                 520                 525
   Phe Leu Leu Met Phe Phe Ala Gly Ala Val Arg Leu Thr Gly Pro Phe
       530                 535                 540
   Val Thr Met Ile Tyr Ser Met Ile Thr Gly Asp Met Leu Thr Phe Gly
   545                 550                 555                 560
   Ile Ile Tyr Thr Ile Phe Leu Phe Gly Phe Ser Gln Ser Leu Phe Phe
                   565                 570                 575
   Leu Tyr Lys Gly Asn Pro Ser Val Asn Gly Thr Leu Tyr His Ser Tyr
               580                 585                 590
   Ser Ser Thr Trp Met Ala Leu Phe Gln Val Thr Met Gly Asn Tyr Asp
           595                 600                 605
   Tyr Ser Glu Leu Ala Leu Thr Thr Tyr Pro Gly Val Ala Lys Ser Val
       610                 615                 620
   Phe Gly Leu Phe Met Val Phe Val Pro Ile Leu Leu Phe Asn Met Leu
   625                 630                 635                 640
   Ile Ala Met Met Gly Asn Thr Tyr Ala His Val Thr Glu Gln Ser Glu
                   645                 650                 655
   Lys Glu Trp Val Lys Gln Trp Ala Lys Ile Val Ile Ser Leu Glu Arg
               660                 665                 670
   Ala Ile Pro Gln Lys Glu Ala His Asn Tyr Leu Gln Glu Tyr Ser Ile
           675                 680                 685
   Ser Leu Gly Pro Ala Glu Asp Pro Ala Thr Glu Gln Arg Gly Val Met
       690                 695                 700
   Val Ile Lys Ser Lys Asn Lys Thr Arg Ala Lys Gln Arg Lys Gly Ala
   705                 710                 715                 720
   Val Ser Asn Trp Lys Arg Val Gly Lys Val Thr Ile Lys Ala Leu Lys
   725 730 735
   Lys Lys Gly Met Thr Gly Glu Glu Met Arg Cys Leu Met Trp Gly Arg
   740 745 750
   Glu Ser Ile Asn Thr Pro Val Lys Val Lys Gly Pro Lys Arg Asp Pro
   755 760 765
   Leu Ala Asn Pro Gln Gly Pro Asp Leu Ala Asn Gly Phe Gly Asp Ala
   770 775 780
   Leu Thr Ser Ala Leu Asp Val Met Ala Phe Thr His Asp Leu Asp Ile
   785 790 795 800
   Asn Ala Gln Gly Leu Asn Leu Ala Lys Gln Thr Leu Asn Pro Lys Pro
   805 810 815
   Phe Thr Asp Pro Leu Arg Thr Leu Val Leu Glu Ser Leu Val Glu Thr
   820 825 830
   Asn Ser Ala Arg Leu Ser Val Leu Ala Ala Gln Ala Ala Asn Leu Pro
   835 840 845
   Asp Thr Ala Glu Ile Val Ser Cys Pro Asn Thr Ala Thr Thr Ser Val
   850 855 860
   Lys Asn Leu Ala Gly Ile Phe Ala Gly Thr Gly Asn Phe Val Lys Lys
   865 870 875 880
   Ile Glu Glu Lys Val Lys Lys Lys Lys Tyr Ala Val Leu Glu Cys Ser
   885 890 895
   Asp Ser Glu Ser Phe Gly Gly Lys Cys His Gly Ile Ser Thr His Phe
   900 905 910
   Gln Ser Lys Thr Leu Lys Tyr Cys Arg Ser Ala Ala Gly Asp Asn Ile
   915 920 925
   Thr Gly Glu Glu Gly Gln Thr Asp Thr Tyr Ala Thr Phe Cys Lys Gln
   930 935 940
   Glu Lys Glu Phe Arg
   945
<210> 97
   <211> 1147
   <212> PRT
   <213> Nasonia vitripennis
<220>
   <221> MOD_RES
   <222> (351)..(351)
   <223> Any amino acid
<400> 97
   Met Gly Gly Ala Cys Ser Cys Gln Gly Arg Asp Ser Gln Val Asn Ala
   1               5                   10                  15
   Gly Ser Ile Leu Asp Arg Val Ile Ser Gln Ala Ser Asp Glu Asp Gln
               20                  25                  30
   Cys Leu Leu Tyr Arg Leu Ala Asn Tyr Lys Lys Gly Gly Glu Leu Ile
           35                  40                  45
   Glu Ala Tyr Asn Gln Gly Gly Gln Ala Glu Val Glu Arg Leu Ile Arg
       50                  55                  60
   Glu Gln Phe Gly Ile Leu Met Tyr Ala Asp Gly Lys Gly Gln Thr Ile
   65                  70                  75                  80
   Asn Arg Ala Glu Tyr Leu Arg Trp Lys Phe Arg Asp Leu Asp Gln Val
                   85                  90                  95
   Ile Leu Pro Ile Glu Ala Ser Leu Ser Arg Phe Asp Pro Leu Ala Gln
               100                 105                 110
   Trp Ser Asp His Glu Ala Cys Trp Gln Met Gln Tyr Arg Gly Ser Leu
           115                 120                 125
   Gly Glu Thr Leu Leu His Val Leu Ile Ile Cys Asp Thr Arg Thr His
       130                 135                 140
   Thr Arg Ile Ala Arg Thr Leu Leu Lys Cys Phe Pro Arg Leu Ala Ile
   145                 150                 155                 160
   Asp Val Val Glu Gly Glu Glu Tyr Leu Gly Ala Ser Ala Leu His Leu
                   165                 170                 175
   Ala Ile Ala Tyr Ala Asn Asn Glu Leu Val Gln Asp Leu Val Glu Ala
               180                 185                 190
   Gly Ala Ile Val Ser Gln Arg Ala Ile Gly Ser Phe Phe Leu Pro Arg
           195                 200                 205
   Asp Gln Gln Arg Pro Arg Pro Ala Lys Ser Thr Asp Tyr Glu Gly Leu
       210                 215                 220
   Ala Tyr Leu Gly Glu Tyr Pro Leu Ala Trp Ala Ala Cys Cys Ala Asn
   225                 230                 235                 240
   Glu Ser Val Tyr Asn Leu Leu Leu Asp Ser Gly Ala His Pro Asp Glu
                   245                 250                 255
   Gln Asp Thr Phe Gly Asn Ser Ile Leu His Met Val Val Val Cys Asp
               260                 265                 270
   Lys Leu Val Arg Phe Gly Tyr Ala Leu Arg His Pro Lys Leu Pro Ala
           275                 280                 285
   Ser Asn Gly Ile Ala Asn Ala Ala Gly Leu Thr Pro Leu Thr Leu Ala
       290                 295                 300
   Cys Gln Leu Gly Arg Ala Glu Val Phe Arg Glu Met Leu Glu Leu Ser
   305                 310                 315                 320
   Ala Arg Glu Phe Trp Arg Tyr Ser Asn Ile Thr Cys Ser Ala Tyr Pro
                   325                 330                 335
   Leu Asn Ala Leu Asp Thr Leu Leu Pro Asp Gly Arg Thr Ser Xaa Asn
               340                 345                 350
   Ser Ala Leu Phe Ile Ile Leu Asn Gly Thr Lys Glu Glu His Leu Asp
           355                 360                 365
   Met Leu Asp Gly Gly Ile Ile Gln Arg Leu Leu Glu Glu Lys Trp Lys
       370                 375                 380
   Thr Phe Ala Arg Leu Gln Phe Leu Lys Arg Leu Val Ile Leu Val Phe
   385                 390                 395                 400
   His Leu Val Ser Leu Ser Leu Ala Val Tyr Phe Arg Pro Ala Asp Thr
                   405                 410                 415
   Asp Ala Glu Leu Ala Gln Trp Pro Glu Glu Ile Thr Asp Val Val Arg
               420                 425                 430
   Val Ile Ala Glu Cys Val Thr Val Leu Gly Val Leu Gly Tyr Ile Leu
           435                 440                 445
   Leu Gln Leu Gly Gly Glu Ile Val Asn Ile Gly Phe Phe Ser Phe Phe
       450                 455                 460
   Lys Gln Leu Ser His Glu Pro Ala Lys Phe Ile Phe Leu Ile Ser Asn
   465 470 475 480
   Leu Leu Ile Leu Ala Cys Ile Pro Cys Arg Leu Ser Gly Asp Arg His
   485 490 495
   Thr Glu Asp Ala Ile Leu Val Val Ala Val Pro Gly Ser Trp Phe Leu
   500 505 510
   Leu Met Phe Phe Ala Gly Ala Val Arg Leu Thr Gly Pro Phe Val Thr
   515 520 525
   Met Val Tyr Ser Met Ile Leu Gly Asp Met Arg Thr Phe Gly Ile Ile
   530 535 540
   Tyr Met Ile Val Ile Cys Gly Phe Thr Gln Ala Phe Tyr Phe Leu Tyr
   545 550 555 560
   Lys Gly Tyr Pro Gly Val Lys Thr Thr Leu Phe His Ser Tyr Pro Ser
   565 570 575
   Thr Trp Met Ala Leu Phe Gln Ile Thr Leu Gly Asp Tyr Ser Tyr Ser
   580 585 590
   Asp Leu Ser Met Thr Thr Tyr Pro Asn Leu Ser Lys Ala Val Phe Thr
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Trp Met Lys Gln Trp Ala Lys Ile Val Val Ser Leu Glu Arg Ala Val
   645 650 655
   Ser Gln Asp Asp Ala His Asn Tyr Leu Gln Glu Tyr Ser Ile Lys Leu
   660 665 670
   Gly Pro Gly Asp Asp Pro Asn Asp Pro Ala Ser Glu Gln Arg Gly Val
   675 680 685
   Met Val Ile Lys Ser Lys Ser Lys Thr Arg Ala Arg Gln Arg Lys Gly
   690 695 700
   Ala Val Ala Asn Trp Lys Arg Val Gly Lys Val Thr Ile Ala Glu Leu
   705 710 715 720
   Lys Lys Arg Gly Met Thr Gly Glu Glu Leu Arg Arg Ile Met Trp Gly
   725 730 735
   Arg Ala Ser Phe Ser Thr Pro Val Arg Ser Ser Pro Ile Leu Met Glu
   740 745 750
   Pro Val Ser Ser Val Pro Gly Gly Phe Gly Asp Ala Leu Thr Ser Ala
   755 760 765
   Leu Asp Val Met Thr Phe Ala Ala Asp Val Pro Val Pro Ile Glu Gly
   770 775 780
   Ser Ile Pro Lys Pro Val Ala Val Ala Pro Gly Gln Gln Pro Thr Thr
   785 790 795 800
   Thr Leu Ala Leu Pro Asn Gly Ala Ala Lys Pro Gln Gln Pro Met Met
   805 810 815
   Thr Thr Ser Thr Thr Thr Thr Thr Ile Thr Thr Thr Val Gln Lys Thr
   820 825 830
   Leu Val Thr Gly Thr Ala Thr Thr Pro Ala Val Gly Val Thr Lys Thr
   835 840 845
   Val Ala Val Pro Ala Ala Thr Thr Val Ser Thr Thr Thr Thr Arg Ala
   850 855 860
   Ser Ala Ser Ala Val Pro Pro Thr Val Arg Pro Ser Glu Leu Gly Pro
   865 870 875 880
   Ala Ser Ala Pro Cys Ser Ala Asp Pro Leu Leu Glu Leu Met Leu Ala
   885 890 895
   Thr Glu Asp Pro Gly Ser Ser Glu Glu Thr Leu Gln Arg Leu Ala His
   900 905 910
   Ser Ala Arg Ile Val Gly Glu Ser Ala Thr Thr Ser Ala Lys Ser Asp
   915 920 925
   Ile Asp Ser Ser Phe Leu Glu Arg Leu Ala Leu Gly Gly Leu Gly Leu
   930 935 940
   Ala Ala Met Pro Glu Gln Pro Asp Lys Pro Val Glu Pro Lys Lys Lys
   945 950 955 960
   Gln Tyr Leu Val Glu Ser Ser Asp Asn Asp Ile Cys Ala Asn Asp Gly
   965 970 975
   Gln Leu Gly Thr Glu Ala Arg Leu Arg Arg Ile Arg Ser Ala Ser Ser
   980 985 990
   Arg Leu Ala Pro Thr Thr Thr Thr Ser Thr Ser Arg Arg Lys Ser Gly
   995 1000 1005
   Gln Gln Lys Gln Gln Arg Glu Ala Arg Asp Asp Glu Ser Thr Ser
   1010 1015 1020
   Ser Ala Ala Ser Leu Asp Asn Ile Ser Gly Tyr Gln Pro Leu Leu
   1025 1030 1035
   Asn Glu Pro Asp Thr Thr Ala Asp Gln Pro Ala Thr Gly Thr Glu
   1040 1045 1050
   Asp Asn Lys Pro Ala Asn Gly Arg Asn Leu Arg Lys Arg Pro Lys
   1055 1060 1065
   Thr Thr Ser Asn Glu Gly Lys Val Phe Cys Ser Arg Arg Thr Ile
   1070 1075 1080
   Val Val Leu Asn Lys Asn Lys Lys Asn Lys Lys Leu Glu Thr Ser
   1085 1090 1095
   Glu Ala Asn Met Asn Ala Arg Lys Lys Glu Thr Leu Leu Ala Leu
   1100 1105 1110
   His Asp Met Thr Ser Leu Ile Ser Gly Arg Val Leu Thr Leu Glu
   1115 1120 1125
   Gln Ala Asn Glu Asn Gly Tyr Glu Met Leu Glu Gln Leu Lys His
   1130 1135 1140
   Lys Leu Asp Pro
   1145
<210> 98
   <211> 1109
   <212> PRT
   <213> Camponotus floridanus
<400> 98
   Met Gly Gly Val Cys Ser Cys Arg Val Gly Arg Gly Asn Gln Leu Asn
   1               5                   10                  15
   Ala Gly Ser Ile Leu Asp Arg Val Ile Ser Gln Ala Ser Asn Glu Asp
               20                  25                  30
   Gln Cys Leu Leu Tyr Arg Leu Ala Asn Tyr Lys Lys Gly Gly Glu Leu
   35 40 45
   Ile Glu Ala Tyr Asn Gln Gly Gly Gln Pro Glu Val Glu Lys Leu Val
   50 55 60
   Arg Glu Gln Phe Gly Ile Leu Met Tyr Ala Asp Gly Lys Gly Gln Val
   65 70 75 80
   Ile Asn Arg Ala Glu Tyr Leu Arg Trp Lys Phe Arg Asp Leu Glu Gln
   85 90 95
   Val Val Leu Pro Ile Glu Ala Ser Leu Ser Arg Phe Asp Pro Leu Ala
   100 105 110
   Gln Trp Asn Asp His Glu Ala Cys Trp Gln Met Gln Tyr Arg Gly Ser
   115 120 125
   Leu Gly Glu Ala Leu Leu His Val Leu Ile Ile Cys Asp Thr Arg Ile
   130 135 140
   His Thr Arg Ile Ala Arg Ile Leu Leu Lys Cys Phe Pro Arg Leu Ala
   145 150 155 160
   Ile Asp Val Val Glu Gly Glu Glu Tyr Leu Gly Ala Ser Ala Leu His
   165 170 175
   Leu Ala Ile Ala Tyr Asn Asn Asn Glu Leu Val Gln Asp Leu Val Asp
   180 185 190
   Ala Gly Ala Ile Ile Ser Gln Arg Ala Ile Gly Ser Phe Phe Leu Pro
   195 200 205
   Arg Asp Gln Gln Gly Met Asn Pro Val Arg Asn Thr Asp Tyr Glu Gly
   210 215 220
   Leu Ala Tyr Leu Gly Glu Tyr Pro Leu Ala Trp Ala Ala Cys Cys Ala
   225 230 235 240
   Asn Glu Ser Val Tyr Asn Leu Leu Leu Asp Ser Gly Ala Asp Pro Asp
   245 250 255
   Glu Gln Asp Ser Phe Gly Asn Met Ile Leu His Met Val Val Val Gly
   260 265 270
   Asp Lys Leu Asp Met Phe Gly Tyr Ala Leu Arg His Pro Lys Leu Pro
   275 280 285
   Ala Arg Asn Gly Ile Ala Asn Ala Ala Gly Leu Thr Pro Leu Thr Leu
   290 295 300
   Ala Cys Gln Leu Gly Arg Ala Glu Val Phe Arg Glu Met Leu Glu Leu
   305 310 315 320
   Ser Ala Arg Glu Phe Trp Arg Tyr Ser Asn Ile Thr Cys Ser Ala Tyr
   325 330 335
   Pro Leu Asn Ala Leu Asp Thr Leu Leu Pro Asp Gly Arg Thr Asn Trp
   340 345 350
   Asn Ser Ala Leu Phe Ile Ile Leu Asn Gly Thr Lys Glu Glu His Leu
   355 360 365
   Asp Met Leu Asp Gly Gly Ile Ile Gln Arg Leu Leu Glu Glu Lys Trp
   370 375 380
   Lys Thr Phe Ala Arg Leu Gln Phe Leu Lys Arg Leu Ile Ile Leu Val
   385 390 395 400
   Phe His Leu Ala Ser Leu Ser Leu Ala Val Tyr Phe Arg Pro Ala Asp
   405 410 415
   Thr Asp Ala Val Leu Leu Gln Trp Pro Glu Glu Ile Thr Asp Val Ile
   420 425 430
   Arg Thr Ala Ala Glu Cys Ile Thr Val Leu Gly Val Leu Asn Tyr Ile
   435 440 445
   Leu Val Gln Leu Gly Gly Glu Met Ile Asn Val Gly Pro Leu Ser Phe
   450 455 460
   Leu Lys Gln Leu Ser His Glu Pro Ala Lys Leu Ile Phe Val Ile Ser
   465 470 475 480
   Asn Leu Leu Ile Leu Ala Cys Ile Pro Cys Arg Ile Ala Gly Asn Arg
   485 490 495
   His Ala Glu Asp Ala Ile Leu Val Phe Ala Val Pro Gly Ser Trp Phe
   500 505 510
   Leu Leu Met Phe Phe Ala Gly Ala Ile Arg Leu Thr Gly Pro Phe Val
   515 520 525
   Thr Met Val Tyr Ser Met Ile Thr Gly Asp Met Leu Thr Phe Gly Ile
   530 535 540
   Ile Tyr Thr Ile Met Leu Phe Gly Phe Ser Gln Ser Phe Tyr Phe Leu
   545 550 555 560
   Tyr Lys Gly Phe Pro Gly Val Lys Ser Ser Leu Tyr His Ser Tyr Val
   565 570 575
   Ser Thr Trp Met Ala Leu Phe Gln Ile Thr Leu Gly Asp Tyr Asn Tyr
   580 585 590
   Ala Glu Leu Asn Asn Thr Ile Tyr Pro Asn Leu Ser Lys Thr Val Phe
   595 600 605
   Ala Ile Phe Met Val Leu Val Pro Ile Leu Leu Leu Asn Met Leu Ile
   610 615 620
   Ala Met Met Gly Asn Thr Tyr Ala His Val Ile Glu Gln Ser Glu Lys
   625 630 635 640
   Glu Phe Val Lys Gln Trp Ala Lys Ile Val Val Ser Leu Glu Arg Ala
   645 650 655
   Val Ser Gln Lys Asp Ala His Asn Tyr Leu Gln Glu Tyr Ser Ile Lys
   660 665 670
   Leu Gly Pro Gly Asp Asp Pro Asn Asn Pro Ala Ser Glu Gln Arg Gly
   675 680 685
   Val Leu Val Ile Lys Ser Lys Ser Lys Thr Lys Ala Lys Gln Arg Lys
   690 695 700
   Gly Ala Val Ala Asn Trp Lys Arg Val Gly Lys Val Thr Ile Asn Glu
   705 710 715 720
   Leu Arg Lys Arg Gly Met Thr Gly Glu Glu Leu Arg His Ile Met Trp
   725 730 735
   Gly Arg Glu Ser Phe Ser Thr Pro Val Arg Val Ser Pro Asn Ala Gly
   740 745 750
   Gln Pro Gln Val Ser Ala Val Thr Ala Gly Phe Gly Asp Ala Leu Thr
   755 760 765
   Ala Ala Leu Asp Val Met Ala Phe Ala His Asp Phe Asp Leu Ser Ala
   770 775 780
   Ala Thr Glu Gly Ile Pro Thr Asn Ile Asp Ala Lys Gln Thr Lys Thr
   785 790 795 800
   Thr Gln Ser Lys Ala Ile Pro Asn Asp Gln Met Lys Thr Thr Gln Asn
   805 810 815
   Asn Pro Glu Thr Val Thr Asn Val Gln Pro His Lys Gln Pro Ile Gly
   820 825 830
   Glu Pro Thr Lys Leu Leu Gly Leu Thr Asp Leu Ile Asp Asn Ser Ile
   835 840 845
   Gly Pro Val Lys Asp Val Glu Ala Met Asn Leu Lys Asn Thr Asn Gln
   850 855 860
   Thr Lys Ala Leu Glu Ala Ser Met Glu Phe Glu Asp Pro Phe Leu Glu
   865 870 875 880
   Leu Ala Ile Ala Ser Glu Thr Thr Thr Asn Tyr Glu Ile Leu Leu Gln
   885 890 895
   Ile Ala Lys Ser Ala Leu Ala Ala Ser Glu Ala Ser Val Lys Thr Ala
   900 905 910
   Ile Asp Pro Gln Ile Leu Ala His Phe Ala Met Ile Pro Pro Pro Ile
   915 920 925
   Thr Glu Lys Phe Ile Val Lys Lys Gln Tyr Phe Met Glu Ser Ser Asp
   930 935 940
   Asn Asp Val Gly Gly Asp Asn Leu Leu Gly Thr Glu Ala Arg Leu Arg
   945 950 955 960
   Arg Ile Lys Ser Ala Asn Asn Arg Phe Ile Thr Thr Ser Lys Arg Pro
   965 970 975
   His Arg Asp Asp Asp Gly Asp Ser Ser Ser Ser Thr Ile Ser Ile Glu
   980 985 990
   Arg Asn Gln Arg Tyr Arg Pro Leu Phe Asn Asp Pro Glu Glu Arg Ser
   995 1000 1005
   Ile Asn His Val Glu Thr Glu Gly Gln Lys Thr Pro Met Glu Thr
   1010 1015 1020
   Ile Lys Ser Glu Ile Gln Gln Asn Gly Ser Ile Pro Leu Lys Pro
   1025 1030 1035
   Pro Asp Lys Ile Gln Lys Arg Arg Pro Lys Thr Ala Lys Asn Arg
   1040 1045 1050
   Val Ser Pro Lys Glu Ile Glu Glu Pro Arg Arg Arg Glu Ser Ile
   1055 1060 1065
   Glu Gln Asn Lys Ala Ala Ser Pro Pro Thr Ser Ser Ser Asp Pro
   1070 1075 1080
   Leu Glu Pro Trp Ser Thr Arg Gly Ile Thr Asp Met Asn Thr Ile
   1085 1090 1095
   Leu Ala Trp Arg Asp Asn Ala Pro Asp Ser Pro
   1100 1105
<210> 99
   <211> 998
   <212> PRT
   <213> Danaus plexippus
<400> 99
   Met Gly Val Pro Leu Ser Lys Leu Cys Ser Ala Thr Ser Val Pro Ala
   1 5 10 15
   Val Gly Ser Val Leu Asp Arg Val Ile Ser Gln Pro Ser Ser Glu Asp
   20 25 30
   His Thr Val Leu Tyr Lys Leu Ala Asp Tyr Lys Lys Gly Gly Leu Leu
   35 40 45
   Leu Glu Thr Tyr Thr Lys Gly Gly Val Val Ala Ala Glu Arg Leu Ile
   50 55 60
   Arg Glu Glu Phe Ser Ala Tyr Met Tyr Ala Gly Gly Arg Gly Arg Val
   65 70 75 80
   Ile Asn Arg Ala Glu Tyr Leu Arg Trp Lys Phe Arg Asp Gln Glu Gln
   85 90 95
   Val Val Leu Pro Ile Glu Ala Ser Leu Ser Pro His Asp Pro Leu Ala
   100 105 110
   Lys Trp Glu Asp His Thr Ala Cys Trp Gln Met Ser Tyr Arg Gly Ala
   115 120 125
   Leu Gly Glu Ser Leu Leu His Val Leu Ile Ile Cys Asp Thr Lys Ile
   130 135 140
   His Thr Arg Leu Ala Arg Thr Leu Val Lys Cys Phe Pro Lys Leu Ser
   145 150 155 160
   Leu Asp Val Val Glu Gly Glu Glu Tyr Leu Gly Ala Ser Ser Leu His
   165 170 175
   Leu Ala Ile Ala Tyr Ser Asn Asn Glu Leu Val Gln Asp Leu Val Glu
   180 185 190
   Ala Gly Ala Asp Val Ser Gln Lys Ala Ile Gly Ser Phe Phe Leu Pro
   195 200 205
   Arg Asp Gln Gln Lys Asn Pro Pro Ala Arg Gln Thr Asn Tyr Glu Gly
   210 215 220
   Leu Ala Tyr Leu Gly Glu Tyr Pro Leu Ala Trp Ala Ala Cys Cys Ala
   225 230 235 240
   Asn Glu Ala Val Tyr Asn Leu Leu Leu Asp Ser Gly Ala Asp Pro Asp
   245 250 255
   Ala Gln Asp Ser Phe Gly Asn Met Ile Leu His Met Val Val Val Cys
   260 265 270
   Asp Lys Leu Asp Met Phe Gly Tyr Ala Leu Arg His Pro Lys Val Pro
   275 280 285
   Ala Ser Asn Gly Arg Leu Asn Lys Ala Gly Phe Thr Pro Leu Thr Leu
   290 295 300
   Ala Cys Gln Leu Gly Arg Ala Ser Val Phe Arg Glu Met Leu Glu Leu
   305 310 315 320
   Ser Ser Arg Glu Phe Trp Arg Tyr Ser Asn Ile Thr Cys Ser Ala Tyr
   325 330 335
   Pro Leu Asn Ala Leu Asp Thr Leu Leu Pro Asp Gly Arg Thr Asn Trp
   340 345 350
   Asn Ser Ala Leu Phe Ile Ile Leu Asn Gly Thr Lys Gln Glu His Leu
   355 360 365
   Asn Met Leu Asp Gly Gly Ile Ile Gln Arg Leu Leu Glu Glu Lys Trp
   370 375 380
   Lys Thr Phe Ala Arg Thr Lys Phe Leu Lys Arg Leu Leu Ile Leu Met
   385 390 395 400
   Leu His Leu Ile Leu Leu Ser Ile Ser Val Tyr Leu Arg His Ser Ser
                   405                 410                 415
   Leu Glu Ala Asp Val Asp Pro Asp Trp Gly Leu Glu Val Asn Asp Ala
               420                 425                 430
   Arg Ser Gly Ile Arg Leu Ala Cys Glu Leu Gly Thr Ile Ile Ser Thr
           435                 440                 445
   Leu Cys Tyr Ile Ile Leu Gln Gln Gly Asp Glu Ile Lys Asn Gln Gly
       450                 455                 460
   Val Val Ala Tyr Phe Lys Gln Leu Ile His Glu Pro Ala Lys Phe Ile
   465                 470                 475                 480
   Phe Leu Ala Ser Asn Ile Leu Leu Leu Ala Cys Ile Pro Ala Arg Ile
                   485                 490                 495
   Ser Gln Arg Thr Thr Leu Glu Glu Ala Ile Leu Ile Phe Val Leu Pro
               500                 505                 510
   Gly Ser Trp Phe Leu Met Met Phe Phe Ala Gly Ala Val Lys Leu Thr
           515                 520                 525
   Gly Pro Phe Val Thr Met Ile Tyr Ser Met Ile Thr Gly Asp Met Phe
       530                 535                 540
   Thr Phe Gly Ile Ile Tyr Cys Ile Val Leu Phe Gly Phe Ser Gln Ser
   545                 550                 555                 560
   Phe Tyr Phe Leu Tyr Lys Gly Phe Pro Asn Val His Ser Thr Leu Tyr
                   565                 570                 575
   Ser Ser Tyr Pro Ser Thr Trp Met Ala Leu Phe Gln Ile Thr Leu Gly
               580                 585                 590
   Asp Tyr Ser Tyr Thr Asp Leu Gly Leu Thr Met Tyr Pro Asn Leu Ser
           595                 600                 605
   Lys Thr Val Phe Thr Val Phe Met Val Phe Val Pro Ile Leu Leu Leu
       610                 615                 620
   Asn Met Leu Ile Ala Met Met Gly Asn Thr Tyr Ala His Val Ile Glu
   625                 630                 635                 640
   Gln Ser Glu Lys Glu Trp Val Lys Gln Trp Ala Lys Ile Val Val Ser
   645 650 655
   Leu Glu Arg Ser Val Ser Gln Glu Asp Ala His Arg Tyr Leu Gln Glu
   660 665 670
   Tyr Ser Ile Gly Leu Gly Pro Ser Asp Asp Pro Arg Tyr Glu Gln Arg
   675 680 685
   Gly Val Met Val Ile Lys Ser Lys Ala Lys Thr Arg Ala Lys Gln Arg
   690 695 700
   Lys Gly Ala Leu Ser Asn Trp Lys Arg Val Gly Lys Val Thr Ile Ala
   705 710 715 720
   Glu Leu Arg Arg Arg Gly Ile Ser Gly Glu Glu Leu Arg Arg Leu Met
   725 730 735
   Trp Gly Arg Val Ser Ile Ser Thr Pro Thr Lys Ala Pro Leu Lys Cys
   740 745 750
   Val Pro Pro Pro Glu Leu Val Thr Ser Glu Ile Pro Gly Ser Gly Val
   755 760 765
   Gly Pro Ala Leu Ser Ser Ala Leu Asn Val Met Ala Tyr Thr Gln Asp
   770 775 780
   Leu Asp Leu Thr Asn Thr Gly Ser Glu Leu His Lys Gln Ile Thr Pro
   785 790 795 800
   Asp Leu Leu Val Asn Gly Lys Thr Pro Val Thr Ala Gln Asn Thr Ala
   805 810 815
   Thr Thr Pro Lys Val Pro Leu Val Asn Leu Pro Asn Lys Ala Gly Leu
   820 825 830
   Asp Val Gln Ser Thr Gly Ser Asp Gln Ser Val Pro Thr Ala Leu Thr
   835 840 845
   Lys Asn Met Asp Val Leu Gly Ile Asn Met Ser Thr Gln Asp Leu Leu
   850 855 860
   Lys Asn Gln Thr Ile Asn Asn Pro Ala Thr Ala Asn Glu Ile Val Phe
   865 870 875 880
   Lys Asp Tyr Leu Arg Asp Ile Ile Lys Ala Glu Gln Leu Gly Leu Asp
   885 890 895
   Asn Ile Asp Ile Lys Ala Leu Ala Glu Lys Ala Ala Asn Leu Thr Asp
   900 905 910
   Val Pro Glu Ile Asp Ile Asn Ile Ser Thr Ala Ala Arg Ser Ala Arg
   915 920 925
   Arg Val Val Ala Gly Ala Val Ser Gly Leu Phe Gly Val Thr Ala Glu
   930 935 940
   Thr Pro Arg Asp Ala Gly Trp Arg Arg Glu Arg His Glu His Thr Asp
   945 950 955 960
   Ser Asp Pro Val Pro Glu Cys Val Ile Leu Gly Arg Ala Ala Arg Ala
   965 970 975
   Arg Arg Ala Arg Ser Ala Ser Arg Arg Ala Pro Pro Pro Pro Pro His
   980 985 990
   Leu Tyr Val Pro Ala Arg
   995
<210> 100
   <211> 1179
   <212> PRT
   <213> Bombyx mori
<400> 100
   Met Gly Asn Ala Ile Gly Lys Phe Leu Thr Ala Gly Asn Val Gln Gly
   1               5                   10                  15
   Ala Gly Ser Val Leu Asp Arg Val Ile Ser Gln Pro Ser Ser Glu Asp
               20                  25                  30
   His Thr Val Leu Tyr Lys Leu Ala Asp Tyr Lys Lys Gly Gly Leu Leu
           35                  40                  45
   Leu Glu Thr Tyr Ala Lys Gly Gly Met Thr Ala Ala Glu Lys Leu Met
       50                  55                  60
   Arg Asp Glu Phe Ala Ala Tyr Met Tyr Gly Gly Gly Arg Gly Arg Val
   65                  70                  75                  80
   Ile Asn Arg Ala Glu Tyr Leu Arg Trp Lys Phe Arg Asp Gln Glu Gln
                   85                  90                  95
   Val Val Leu Pro Ile Glu Ala Ser Leu Ser Pro Tyr Asp Pro Leu Ala
               100                 105                 110
   Lys Trp Glu Asp His Thr Ala Cys Trp Gln Met Cys Tyr Arg Gly Ala
   115 120 125
   Leu Gly Glu Ser Leu Leu His Val Leu Ile Ile Cys Asp Thr Lys Ile
   130 135 140
   His Thr Arg Leu Ala Arg Thr Leu Val Lys Cys Phe Pro Lys Leu Ser
   145 150 155 160
   Leu Asp Val Val Glu Gly Glu Glu Tyr Leu Gly Ala Ser Ser Leu His
   165 170 175
   Leu Ala Ile Ala Tyr Ser Asn Asn Glu Leu Val Gln Asp Leu Val Glu
   180 185 190
   Ala Gly Ala Asp Val Asn Gln Arg Ala Ile Gly Ser Phe Phe Leu Pro
   195 200 205
   Arg Asp Gln Gln Arg Val Pro Pro Ala Arg Gln Thr Asn Tyr Glu Gly
   210 215 220
   Leu Ala Tyr Leu Gly Glu Tyr Pro Leu Ala Trp Thr Ala Cys Cys Ala
   225 230 235 240
   Asn Glu Ala Val Tyr Asn Leu Leu Leu Asp Ser Gly Ala Asp Pro Asp
   245 250 255
   Ala Gln Asp Ser Phe Gly Asn Met Ile Leu His Met Val Val Val Cys
   260 265 270
   Asp Lys Leu Asp Met Phe Gly Tyr Ala Leu Arg His Pro Lys Val Pro
   275 280 285
   Ala Ser Asn Gly Arg Met Asn Lys Ala Gly Phe Thr Pro Leu Thr Leu
   290 295 300
   Ala Cys Gln Leu Gly Arg Ala Ser Val Phe Arg Glu Met Leu Glu Leu
   305 310 315 320
   Ser Ala Arg Glu Phe Trp Arg Tyr Ser Asn Ile Thr Cys Ser Ala Tyr
   325 330 335
   Pro Leu Asn Ala Leu Asp Thr Leu Leu Pro Asp Gly Arg Thr Asn Trp
   340 345 350
   Asn Ser Ala Leu Phe Ile Ile Leu Asn Gly Thr Lys Gln Glu His Leu
   355 360 365
   Asn Met Leu Asp Gly Gly Ile Ile Gln Arg Leu Leu Glu Glu Lys Trp
       370                 375                 380
   Lys Thr Phe Ala Arg Thr Lys Phe Leu Lys Arg Leu Leu Ile Leu Met
   385                 390                 395                 400
   Leu His Leu Leu Leu Leu Ser Val Ser Val Tyr Leu Arg His Ser Ser
                   405                 410                 415
   Ala Glu Ala Asp Ala His Pro Asn Trp Gly Leu Glu Ile Asn Asp Ala
               420                 425                 430
   Arg Ser Gly Leu Arg Leu Ala Ser Glu Leu Gly Thr Ile Leu Ser Thr
           435                 440                 445
   Leu Cys Tyr Ile Ile Leu Gln Gln Gly Asp Glu Leu Lys Asn Gln Gly
       450                 455                 460
   Leu Val Ala Tyr Phe Lys Gln Leu Ile His Glu Pro Ala Lys Phe Ile
   465                 470                 475                 480
   Phe Leu Ala Ser Asn Ile Leu Val Leu Ala Cys Ile Pro Ala Arg Leu
                   485                 490                 495
   Leu Lys Glu Thr Asn Val Glu Glu Ala Ile Leu Leu Phe Leu Leu Pro
               500                 505                 510
   Gly Ser Trp Phe Leu Leu Met Phe Phe Ala Gly Ala Val Lys Leu Thr
           515                 520                 525
   Gly Pro Phe Val Thr Met Ile Tyr Ser Met Ile Thr Gly Asp Met Phe
       530                 535                 540
   Thr Phe Gly Ile Ile Tyr Cys Ile Val Leu Phe Gly Phe Ser Gln Ser
   545                 550                 555                 560
   Phe Tyr Phe Leu Tyr Arg Gly Phe Pro Asn Val Gln Ser Thr Leu Tyr
                   565                 570                 575
   Ser Ser Tyr Pro Ser Thr Trp Met Ala Leu Phe Gln Ile Thr Leu Gly
               580                 585                 590
   Asp Tyr Ser Tyr Ser Asp Leu Ser Gln Thr Thr Tyr Pro Asn Leu Ser
           595                 600                 605
   Lys Thr Val Phe Thr Val Phe Met Ile Phe Val Pro Ile Leu Leu Leu
       610                 615                 620
   Asn Met Leu Ile Ala Met Met Gly Asn Thr Tyr Ala His Val Ile Glu
   625 630 635 640
   Gln Ser Glu Lys Glu Trp Val Lys Gln Trp Ala Lys Ile Val Val Ser
   645 650 655
   Leu Glu Arg Ser Val Ala Gln Asp Asp Ala His Lys Tyr Leu Gln Glu
   660 665 670
   Tyr Ser Ile Gly Leu Gly Pro Ser Asp Asp Pro Arg Tyr Glu Gln Arg
   675 680 685
   Ala Val Met Val Ile Lys Ser Lys Ala Lys Thr Arg Ala Lys Gln Lys
   690 695 700
   Lys Asp Ala Leu Thr Asn Trp Lys His Val Gly Lys Val Thr Ile Ala
   705 710 715 720
   Glu Leu Arg Arg Arg Gly Ile Ser Gly Glu Glu Leu Arg Arg Leu Met
   725 730 735
   Trp Gly Arg Ile Ser Ile Ser Thr Pro Thr Lys Ala Pro Leu Pro Arg
   740 745 750
   Arg Val Pro Ala Pro Pro Pro Asp Cys Val Val Ser Ser Asp Val Gly
   755 760 765
   Leu Ala Ser Asp Val Gly Asn Gly Val Ala Pro Ala Leu Ser Ser Ala
   770 775 780
   Leu Asn Val Met Ala Phe Thr His Glu Leu Asp Ile Gly Thr Thr Gly
   785 790 795 800
   Ser Asp Gln Lys Gln Thr Thr Pro Asp Leu Leu Val Asn Gly Lys Thr
   805 810 815
   Ser Asn Ala Pro Ile Leu Thr Thr Gly Thr Gln Met Pro Lys Val Ser
   820 825 830
   Ser Lys Thr Leu Gly Thr Pro Val Ala Arg Ile Glu Ala Ser Ser Leu
   835 840 845
   Lys Thr Pro Leu Asp Val Lys Leu Asn Pro Thr Thr Leu Thr Gln Ser
   850 855 860
   Thr Val Gln Gly Asn Ile Gly Asn Val Asn Ile Pro Ile Ser Gly Val
   865 870 875 880
   Lys Ile Thr Thr Asn Val Gly Gln Asn Ser Ala Val Val Pro Glu Ile
                   885                 890                 895
   Gln Gln Ser Asn Glu Asn Gln Lys Pro Glu Gln Val His Gln Asp Tyr
               900                 905                 910
   Leu Arg Glu Leu Ile Val Leu Ala Glu Lys Pro Ala Thr Thr Asn Leu
           915                 920                 925
   Glu Leu Lys Gln Leu Ala Glu Lys Ala Ala Asp Leu Arg Asp Val Pro
       930                 935                 940
   Glu Ile Asp Ile Asn Ile Asn Met Ala Ala Lys Ser Ala Arg Lys Met
   945                 950                 955                 960
   Val Ala Gly Ala Val Ser Gly Leu Phe Gly Val Ala Ala Asp Thr Pro
                   965                 970                 975
   Ala Pro Asp Ala Gly Trp Arg Arg Asp Arg His Asp Asn Ser Asp Ser
               980                 985                 990
   Asp Pro Ile Ser Glu Ser Val Leu  Leu Ser Arg Ala Ser  Arg Ala Arg
           995                 1000                1005
   Arg Ala  Arg Ser Ala Ser Arg  Arg Ala Ala Pro Pro  Pro Pro His
       1010                 1015                1020
   Leu Tyr  Val Pro Ala Arg Ser  Met Tyr Leu Val Ala  Ser Glu Ser
       1025                 1030                1035
   Ser Ala  Ile Glu Ser Asp Ala  Pro Trp Asp Asp Gln  Pro Ser Ser
       1040                 1045                1050
   Glu Asn  Asn Ser Ala Val Gly  Gly Arg Arg Glu Glu  Ile Ala His
       1055                 1060                1065
   Ile Lys  Ala Ala Arg Pro Leu  Cys Ile Gln His Ala  Ala Ser Gly
       1070                 1075                1080
   Arg Thr  Ala Gln Val Glu His  Ser Leu Phe Val Val  Gly Pro Gly
       1085                 1090                1095
   Thr Gly  Ala Glu Val Glu Ser  Thr Pro Thr Val Gln  Lys Ser Glu
       1100                 1105                1110
   Lys Lys  Ala Arg Pro Lys Thr  Thr Arg Ser Arg Arg  Asn Arg Ile
   1115 1120 1125
   Ser Pro Thr Pro Val Gln Glu Ala Gly Ser Pro Leu Glu Pro Trp
   1130 1135 1140
   Ala Thr Ala Gln Leu Ser Lys Leu Ser Arg Leu Ile Arg Tyr Ser
   1145 1150 1155
   Ser Ser Ser Ala Ala Ser Ile Thr Ser Gln Thr Ser Thr Glu Gln
   1160 1165 1170
   Glu Gln Ser Pro Arg Tyr
   1175
<210> 101
   <211> 993
   <212> PRT
   <213> Acyrthosiphon pisum
<400> 101
   Met Gly Asn Ser Cys Ala Ser Cys Thr Asp Phe Ser Phe Ser Lys Thr
   1               5                   10                  15
   Ala Ser Gly Ser Val Leu Asp Arg Val Ile Ser Gln Ala Ser Asn Glu
               20                  25                  30
   Asp Asp Cys Leu Leu Tyr Lys Leu Ala Asp Tyr Lys Lys Gly Gly Glu
           35                  40                  45
   Leu Ile Asn Val Tyr Asn Asn Gly Gly Gln Asp Glu Ala Glu Arg Phe
       50                  55                  60
   Ile Val Glu Lys Leu Pro Gly Leu Met Tyr Asn Asn Gly Lys Gly Gln
   65                  70                  75                  80
   Val Ile Asn Arg Asn Asp Tyr Leu Arg Trp Lys Phe Gln Asn Arg Lys
                   85                  90                  95
   His Val Ala Val Asn Val Glu Glu Arg Pro Gly Pro His Asp Pro Leu
               100                 105                 110
   Thr Arg Trp Val Asp His Val Ala Cys Trp Gln Met Gln Tyr Arg Gly
           115                 120                 125
   Ser Leu Gly Glu Ser Leu Leu His Val Leu Ile Ile Cys Asp Thr Val
       130                 135                 140
   Ile His Thr Arg Leu Ser Arg Leu Leu Leu Lys His Tyr Pro Met Leu
   145                 150                 155                 160
   Ser Gln Asp Val Val Glu Gly Glu Glu Tyr Leu Gly Ala Ser Ala Leu
   165 170 175
   His Leu Ser Ile Ala Tyr Asn Asn Asn Asp Leu Ile Gln Asp Leu Val
   180 185 190
   Asp Ala Gly Ala Asn Ile Cys Gln Arg Ala Ile Gly Ser Phe Phe Leu
   195 200 205
   Pro Arg Asp Gln Gln Asn Lys Glu Ile Asn Asn Lys His Thr Asp Tyr
   210 215 220
   Glu Gly Leu Ala Tyr Leu Gly Glu Leu Pro Leu Ala Trp Ala Ala Cys
   225 230 235 240
   Cys Gly Asn Gln Thr Val Tyr Asn Leu Leu Ile Asp Ala Gly Ala Asn
   245 250 255
   Pro Asp Ala Gln Asp Ser Phe Gly Asn Met Ile Leu His Met Val Val
   260 265 270
   Val Cys Asp Lys Leu Ser Met Phe Gly Tyr Ala Leu Lys His Pro Lys
   275 280 285
   Val Lys Ala Ser Asn Gly Ile Met Asn Val Ala Gly Leu Thr Pro Leu
   290 295 300
   Thr Leu Ser Cys Lys Leu Ala Arg Thr Ser Val Phe Arg Glu Met Leu
   305 310 315 320
   Glu Leu Ser Ala Arg Glu Phe Trp Arg Tyr Ser Asn Ile Thr Cys Ser
   325 330 335
   Ala Tyr Pro Leu Ser Ala Leu Asp Thr Leu Leu Pro Asp Gly Arg Thr
   340 345 350
   Asn Trp Asn Ser Val Leu Phe Ile Ile Leu Asp Gly Thr Lys Glu Glu
   355 360 365
   His Leu Asp Met Leu Asp Gly Gly Ile Ile Gln Lys Leu Leu Glu Glu
   370 375 380
   Lys Trp Lys Thr Phe Ala Arg Lys Gln Phe Met Lys Arg Leu Val Ile
   385 390 395 400
   Leu Ser Ile His Leu Ile Met Leu Ser Ile Ser Ile Tyr Leu Arg Pro
   405 410 415
   Val Asp Gln Asp Lys Pro Leu Leu Gly Glu Ala Glu Asp Trp Gln Asp
   420 425 430
   Val Ala Arg Tyr Cys Phe Glu Gly Gly Thr Val Val Gly Val Leu Ser
   435 440 445
   Tyr Leu Ile Val Gln Gln Gly Gly Glu Ile Leu Asn Gln Gly Leu Val
   450 455 460
   Gly Phe Leu Lys Gln Thr Phe Lys Glu Pro Ala Lys Leu Ile Phe Leu
   465 470 475 480
   Ile Ser Asn Leu Leu Ile Leu Ala Cys Ile Pro Pro Arg Met Met Gly
   485 490 495
   Asp Lys Gln Thr Glu Glu Ala Ile Leu Val Phe Ala Val Pro Gly Ser
   500 505 510
   Trp Phe Leu Leu Met Phe Phe Ala Gly Ala Ile Arg Leu Thr Gly Pro
   515 520 525
   Phe Val Thr Met Val Tyr Leu Met Ile Thr Gly Asp Met Leu Thr Phe
   530 535 540
   Phe Val Ile Tyr Ser Val Ile Leu Ser Gly Phe Thr Gln Ser Phe Phe
   545 550 555 560
   Phe Leu Tyr Lys Gly Ser Pro Asp Val Ser Thr Ser Leu Tyr Lys Ser
   565 570 575
   Tyr Pro Ser Thr Trp Met Ala Leu Phe Gln Ile Thr Met Gly Asp Tyr
   580 585 590
   Asn Tyr Ala Asp Leu Ser Tyr Thr Val Tyr Pro Ala Leu Ser Lys Thr
   595 600 605
   Val Phe Thr Val Phe Met Val Leu Val Pro Ile Leu Leu Leu Asn Met
   610 615 620
   Leu Ile Ala Met Met Gly Asn Thr Tyr Ala His Val Ile Glu Gln Ser
   625 630 635 640
   Glu Lys Glu Trp Met Lys Gln Trp Ala Lys Ile Val Val Thr Leu Glu
   645 650 655
   Arg Ala Val Pro Gln Lys Ala Ala Arg Asn Tyr Leu Glu Glu Tyr Ser
               660                 665                 670
   Ile Gln Leu Ala Pro Gly Asp Asp Val Asn Pro Glu Gln Arg Gly Phe
           675                 680                 685
   Met Val Ile Lys Cys Lys Ser Gln Thr Arg Ala Lys Gln Arg Lys Gly
       690                 695                 700
   Ala Val Ile Asn Trp Lys Lys Ser Gly Lys Leu Val Ile Gln Glu Leu
   705                 710                 715                 720
   Arg Arg Leu Glu Gly Thr Gly Gln Asn Leu Arg Asp Met Ile Trp Lys
                   725                 730                 735
   Arg Ser Ser Leu Ser Ala Ser Ser Pro Val Ala Lys Ser Val Ser Ile
               740                 745                 750
   Thr Lys Asn Lys Ser Lys Ser Tyr Ser Glu Ala Lys Asp Asp Gln Ile
           755                 760                 765
   Arg Leu Ser Gly Ala Leu Gly Ala Ala Leu Asp Ala Ile Ala Val Ala
       770                 775                 780
   His Asp Leu Asp Met Thr Leu Thr Lys Asp Gln Ser Ile Asp Glu Leu
   785                 790                 795                 800
   His Asp Pro Leu Arg Gln Leu Val Ile Met Ser Glu Ser Asp Lys Asn
                   805                 810                 815
   Ile Asp Lys Ser Gln Val Glu Ile Val Ala Asn Ala Ala Ala Lys Ile
               820                 825                 830
   Ala Asn Gln Asp Ser Ile Lys Pro Ile Ser Ile Asn Leu Glu Gln Gln
           835                 840                 845
   Asn Asp Val Lys Asn Glu Lys Thr Lys Ile Pro Thr Ser Ser Asn Thr
       850                 855                 860
   Ile Thr Asn Arg Lys Leu Leu Asn Lys Gln Ser Lys Asn Leu Ser Met
   865                 870                 875                 880
   Tyr Gly Phe Glu Ser Gln Asp Met Ser Val Asp Lys Tyr Ser Lys Ala
                   885                 890                 895
   Ile Ala Ile Thr Arg Ser Ile Ser Thr Gln Thr Gly Gly Ile Gly Ile
               900                 905                 910
   Ile Glu Lys Asn Glu Arg Gln Ile Ile Arg Pro Lys Thr Ala Lys Ile
   915 920 925
   Asn Arg Val Thr Pro Thr Gln Asn Phe Ala Val Lys Arg Gly Gln Ser
   930 935 940
   Ala Gln Pro Asp Lys Arg Glu Asp His Arg Asp Asn Lys Glu Gln Gln
   945 950 955 960
   Cys Tyr Cys Ile Ser Gly Asn Asn Met Leu Arg Pro Trp Ser Thr Gln
   965 970 975
   Asp Leu Ala Pro Leu Asn Thr Ile Met Ala Trp Gly Pro Asn Asp Asp
   980 985 990
   Phe
<210> 102
   <211> 275
   <212> PRT
   <213> Solenopsis invicta
<400> 102
   Met Gly Gly Ala Cys Ser Cys Arg Phe Gly Arg Gly Ser Gln Val Asn
   1 5 10 15
   Pro Gly Ser Ile Leu Asp Arg Val Ile Ser Gln Ala Ser Asn Glu Asp
   20 25 30
   Gln Cys Leu Leu Tyr Arg Leu Ala Asn Tyr Lys Lys Gly Gly Glu Leu
   35 40 45
   Ile Glu Ala Tyr Asn Gln Gly Gly Gln Pro Glu Val Glu Lys Leu Ile
   50 55 60
   Arg Glu Gln Phe Gly Ile Leu Met Tyr Ala Asp Gly Lys Gly Gln Thr
   65 70 75 80
   Ile Asn Arg Ala Glu Tyr Leu Arg Trp Lys Phe Arg Asp Leu Glu Gln
   85 90 95
   Val Val Leu Pro Ile Glu Ala Ser Leu Thr Arg Phe Asp Pro Leu Ala
   100 105 110
   Gln Trp Asn Asp His Glu Ala Cys Trp Gln Met Gln Tyr Arg Gly Ser
   115 120 125
   Leu Gly Glu Thr Leu Leu His Val Leu Ile Ile Cys Asp Thr Arg Met
       130                 135                 140
   His Thr Arg Ile Ala Arg Ile Leu Leu Lys Cys Phe Pro Arg Leu Ser
   145                 150                 155                 160
   Ile Asp Val Val Glu Gly Glu Glu Tyr Leu Gly Ala Ser Ala Leu His
                   165                 170                 175
   Leu Ala Ile Ala Tyr Asn Asn Asn Glu Leu Val Gln Asp Leu Val Glu
               180                 185                 190
   Ala Gly Ala Ile Ile Ser Gln Arg Ala Ile Gly Ser Phe Phe Leu Pro
           195                 200                 205
   Arg Asp Gln Gln Arg Met Asn Pro Ala Arg Asn Thr Asp Tyr Glu Gly
       210                 215                 220
   Leu Ala Tyr Leu Gly Glu Tyr Pro Leu Ala Trp Ala Ala Cys Cys Ala
   225                 230                 235                 240
   Asn Glu Ser Val Tyr Asn Leu Leu Leu Asp Ser Gly Ala Asp Pro Asp
                   245                 250                 255
   Glu Gln Asp Ser Phe Gly Asn Met Ile Leu His Met Val Val Val Gly
               260                 265                 270
   Asp Lys Leu
           275
<210> 103
   <211> 142
   <212> PRT
   <213> Schistocerca americana
<220>
   <221> MOD_RES
   <222> (12)..(12)
   <223> Any amino acid
<400> 103
   Ser Trp Phe Leu Leu Met Phe Phe Ala Gly Ala Xaa Arg Leu Thr Gly
   1               5                   10                  15
   Pro Phe Val Thr Met Val Tyr Ser Met Ile Thr Gly Asp Met Leu Thr
               20                  25                  30
   Phe Gly Ile Ile Tyr Thr Val Val Leu Phe Gly Phe Ser Gln Ser Phe
           35                  40                  45
   Tyr Phe Leu Tyr Lys Gly Phe Pro Gly Val Lys Thr Ser Leu Tyr His
       50                  55                  60
   Ser Tyr Pro Thr Thr Trp Met Ala Leu Phe Gln Ile Thr Met Gly Asp
   65                  70                  75                  80
   Tyr Asn Tyr Pro Glu Leu Ser His Thr Thr Tyr Pro Thr Leu Ala Lys
                   85                  90                  95
   Thr Val Phe Ala Ile Phe Met Val Leu Val Pro Ile Leu Leu Leu Asn
               100                 105                 110
   Met Leu Ile Ala Met Met Gly Asn Thr Tyr Ala His Val Ile Glu Gln
           115                 120                 125
   Ser Glu Lys Glu Trp Met Lys Gln Trp Ala Lys Ile Val Val
       130                 135                 140
<210> 104
   <211> 889
   <212> PRT
   <213> Myzus persicae
<220>
   <221> MOD_RES
   <222> (289)..(296)
   <223> Any amino acid
<220>
   <221> MOD_RES
   <222> (371)..(436)
   <223> Any amino acid
<220>
   <221> MOD_RES
   <222> (745)..(759)
   <223> Any amino acid
<400> 104
   Glu Arg Pro Gly Pro His Asp Pro Leu Thr Arg Trp Val Asp His Val
   1               5                   10                  15
   Ala Cys Trp Gln Met Gln Tyr Arg Gly Ser Leu Gly Glu Ser Leu Leu
               20                  25                  30
   His Val Leu Ile Ile Cys Asp Thr Val Ile His Thr Arg Leu Ser Arg
           35                  40                  45
   Leu Leu Leu Lys His Tyr Pro Met Leu Ser Gln Asp Val Val Glu Gly
       50                  55                  60
   Glu Glu Tyr Leu Gly Ala Ser Ala Leu His Leu Ser Ile Ala Tyr Asn
   65                  70                  75                  80
   Asn Asn Asp Leu Ile Gln Asp Leu Val Asp Ala Gly Ala Asn Ile Cys
                   85                  90                  95
   Gln Arg Ala Ile Gly Ser Phe Phe Leu Pro Arg Asp Gln Gln Asn Lys
               100                 105                 110
   Glu Ile Asn Asn Lys His Thr Asp Tyr Glu Gly Leu Ala Tyr Leu Gly
           115                 120                 125
   Glu Leu Pro Leu Ala Trp Ala Ala Cys Cys Gly Asn Gln Thr Val Tyr
       130                 135                 140
   Asn Leu Leu Ile Asp Ala Gly Ala Asn Pro Asp Ala Gln Asp Ser Phe
   145                 150                 155                 160
   Gly Asn Met Ile Leu His Met Val Val Val Cys Asp Lys Leu Ser Met
                   165                 170                 175
   Phe Gly Tyr Ala Leu Lys His Pro Lys Val Pro Ala Ser Asn Gly Ile
               180                 185                 190
   Met Asn Val Ala Gly Leu Thr Pro Leu Thr Leu Ser Cys Lys Leu Ala
           195                 200                 205
   Arg Thr Ser Val Phe Arg Glu Met Leu Glu Leu Ser Ala Arg Glu Phe
       210                 215                 220
   Trp Arg Tyr Ser Asn Ile Thr Cys Ser Ala Tyr Pro Leu Ser Ala Leu
   225                 230                 235                 240
   Asp Thr Leu Leu Pro Asp Gly Arg Thr Asn Trp Asn Ser Val Leu Phe
                   245                 250                 255
   Ile Ile Leu Asp Gly Thr Lys Glu Glu His Leu Asp Met Leu Asp Gly
               260                 265                 270
   Gly Ile Ile Gln Lys Leu Leu Glu Glu Lys Trp Lys Thr Phe Ala Arg
           275                 280                 285
   Xaa Xaa Xaa Xaa Xaa Xaa Xaa Xaa Ile Leu Ser Ile His Leu Ile Met
       290                 295                 300
   Leu Ser Ile Ser Ile Tyr Leu Arg Pro Val Asp Gln Asp Lys Pro Leu
   305                 310                 315                 320
   Leu Gly Glu Ala Glu Asp Trp Gln Asp Val Ala Arg Tyr Cys Phe Glu
                   325                 330                 335
   Gly Gly Thr Val Val Gly Val Leu Ser Tyr Leu Ile Val Gln Gln Gly
               340                 345                 350
   Gly Glu Ile Leu Asn Gln Gly Leu Val Gly Phe Leu Lys Gln Thr Phe
           355                 360                 365
   Lys Glu Xaa Xaa Xaa Xaa Xaa Xaa Xaa Xaa Xaa Xaa Xaa Xaa Xaa Xaa
       370                 375                 380
   Xaa Xaa Xaa Xaa Xaa Xaa Xaa Xaa Xaa Xaa Xaa Xaa Xaa Xaa Xaa Xaa
   385                 390                 395                 400
   Xaa Xaa Xaa Xaa Xaa Xaa Xaa Xaa Xaa Xaa Xaa Xaa Xaa Xaa Xaa Xaa
                   405                 410                 415
   Xaa Xaa Xaa Xaa Xaa Xaa Xaa Xaa Xaa Xaa Xaa Xaa Xaa Xaa Xaa Xaa
               420                 425                 430
   Xaa Xaa Xaa Xaa Asp Met Leu Thr Phe Phe Val Ile Tyr Ser Val Ile
           435                 440                 445
   Leu Ser Gly Phe Thr Gln Ser Phe Phe Phe Leu Tyr Lys Gly Ser Pro
       450                 455                 460
   Asp Val Ser Thr Ser Leu Tyr Lys Ser Tyr Pro Ser Thr Trp Met Ala
   465                 470                 475                 480
   Leu Phe Gln Ile Thr Met Gly Asp Tyr Asn Tyr Ala Asp Leu Ser Tyr
                   485                 490                 495
   Thr Val Tyr Pro Ala Leu Ser Lys Thr Val Phe Thr Val Phe Met Val
               500                 505                 510
   Leu Val Pro Ile Leu Leu Leu Asn Met Leu Ile Ala Met Met Gly Asn
           515                 520                 525
   Thr Tyr Ala His Val Ile Glu Gln Ser Glu Lys Glu Trp Met Lys Gln
       530                 535                 540
   Trp Ala Lys Ile Val Val Thr Leu Glu Arg Ala Val Pro Gln Lys Ala
   545                 550                 555                 560
   Ala Arg Asn Tyr Leu Glu Glu Tyr Ser Ile Gln Leu Ala Pro Gly Asp
                   565                 570                 575
   Asp Val Asn Pro Glu Gln Arg Gly Phe Met Val Ile Lys Cys Lys Ser
   580 585 590
   Gln Thr Arg Ala Lys Gln Arg Lys Gly Ala Val Ile Asn Trp Lys Lys
   595 600 605
   Ser Gly Lys Leu Val Ile Gln Glu Leu Arg Arg Leu Glu Gly Thr Gly
   610 615 620
   Gln Asn Leu Arg Asp Met Ile Trp Lys Arg Ser Ser Leu Ser Ala Ser
   625 630 635 640
   Ser Pro Val Ala Lys Ser Val Ser Ile Thr Lys Asn Lys Ser Lys Ser
   645 650 655
   Tyr Ser Glu Ala Lys Asp Asp Gln Ile Arg Leu Ser Gly Ala Leu Gly
   660 665 670
   Ala Ala Leu Asp Ala Ile Ala Val Ala His Asp Leu Asp Met Thr Leu
   675 680 685
   Thr Lys Asp Gln Ser Ile Asp Glu Leu His Asp Pro Leu Arg Gln Leu
   690 695 700
   Val Ile Met Ser Glu Ser Asp Lys Asn Ile Asp Lys Ser Gln Val Glu
   705 710 715 720
   Ile Val Ala Asn Ala Ala Ala Lys Ile Ala Asn Gln Asp Ser Ile Lys
   725 730 735
   Pro Ile Ser Val Asn Leu Glu Gln Xaa Xaa Xaa Xaa Xaa Xaa Xaa Xaa
   740 745 750
   Xaa Xaa Xaa Xaa Xaa Xaa Xaa Thr Ile Thr Asn Arg Lys Leu Leu Asn
   755 760 765
   Lys Gln Ser Lys Asn Leu Ser Met Tyr Gly Phe Glu Ser Gln Asp Ile
   770 775 780
   Ser Val Asp Lys Tyr Ser Lys Glu Ile Val Ile Thr Arg Ser Ile Ser
   785 790 795 800
   Thr Gln Thr Gly Gly Ile Gly Ile Ile Glu Lys Asn Glu Arg Pro Ile
   805 810 815
   Ile Arg Pro Lys Thr Ala Lys Ile Asn Arg Val Thr Pro Thr Gln Asn
   820 825 830
   Phe Ala Val Lys Arg Gly Gln Ser Ala Gln Pro Asp Lys Arg Glu Asp
   835 840 845
   His Arg Asp Asn Lys Glu Gln Gln Cys Tyr Cys Ile Ser Gly Asn Asn
   850 855 860
   Met Leu Arg Pro Trp Ser Thr Gln Asp Leu Ala Pro Leu Asn Thr Ile
   865 870 875 880
   Met Ala Trp Gly Pro Asn Asp Asp Phe
   885
<210> 105
   <211> 967
   <212> PRT
   <213> Bemisia tabaci
<400> 105
   Met Tyr Asn Asp Gly Lys Gly Gln Ile Ile Lys Arg Ala Glu Tyr Leu
   1               5                   10                  15
   Arg Trp Lys Phe Arg Asp Gln Gln Gln Val Thr Leu Pro Ile Glu Ala
               20                  25                  30
   Ser Leu Ser Pro His Asp Pro Leu Ala Lys Trp Glu Asp His Glu Ala
           35                  40                  45
   Cys Trp Gln Met Gln Tyr Arg Gly Ser Leu Gly Glu Thr Leu Leu His
       50                  55                  60
   Val Leu Ile Ile Cys Asp Thr Lys Thr Asn Thr Arg Leu Ala Arg Thr
   65                  70                  75                      80
   Leu Leu Lys Cys Phe Pro Arg Leu Ala Gln Asp Ile Val Glu Gly Glu
                   85                  90                  95
   Glu Tyr Leu Gly Ala Ser Ala Leu His Leu Ala Ile Ala Tyr Asn Asn
               100                 105                 110
   Asn Glu Leu Val Gln Asp Leu Val Glu Ala Gly Ala Asn Val Asn Gln
           115                 120                 125
   Arg Ala Ile Gly Ser Phe Phe Leu Pro Lys Asp Gln Gln Arg Pro Arg
       130                 135                 140
   Pro Ala Lys His Thr Asp Tyr Glu Gly Leu Ala Tyr Leu Gly Glu Phe
   145                 150                 155                     160
   Pro Leu Ser Trp Ala Ala Cys Cys Ala Asn Glu Ser Val Tyr Asn Leu
   165 170 175
   Leu Leu Asp Ser Gly Ala His Pro Asp Met Gln Asp Thr Phe Gly Asn
   180 185 190
   Met Ile Leu His Met Val Val Val Cys Asp Lys Leu Asp Met Phe Gly
   195 200 205
   Tyr Ala Leu Arg His Pro Lys Leu Pro Ala Ser Asn Gly Ile Thr Asn
   210 215 220
   Thr Ala Gly Leu Thr Pro Leu Thr Leu Ala Cys Lys Leu Gly Arg Ala
   225 230 235 240
   Glu Val Phe Arg Glu Met Leu Glu Leu Ser Ala Arg Glu Phe Trp Arg
   245 250 255
   Tyr Ser Asn Ile Thr Cys Ser Ala Tyr Pro Leu Asn Ala Leu Asp Thr
   260 265 270
   Leu Leu Pro Asp Gly Arg Thr Asn Trp Asn Ser Ala Leu Phe Ile Ile
   275 280 285
   Leu Asn Gly Thr Lys Glu Glu His Leu Asp Met Leu Asp Gly Gly Ile
   290 295 300
   Ile Gln Arg Leu Leu Glu Glu Lys Trp Lys Thr Phe Ala Arg Arg Gln
   305 310 315 320
   Phe Leu Lys Arg Leu Ala Ile Leu Phe Leu His Leu Leu Met Leu Ser
   325 330 335
   Met Ala Val Tyr Leu Arg Pro Val Asp Arg Glu Gln Pro Leu Ile Gly
   340 345 350
   Gly Asp Asp Trp Ser Asp Ile Ala Arg Tyr Gly Phe Glu Val Gly Thr
   355 360 365
   Ile Met Gly Val Leu Ser Tyr Leu Val Val Gln Gln Gly Gly Glu Ile
   370 375 380
   Lys Asn Gln Gly Leu Gly Ser Phe Leu Lys Gln Leu Met Ala Glu Pro
   385 390 395 400
   Ala Lys Ala Ile Phe Leu Val Ser Asn Leu Leu Ile Leu Ala Cys Ile
   405 410 415
   Pro Phe Arg Met Ala Gly Asp Ser Gln Thr Glu Glu Ala Ile Leu Ile
   420 425 430
   Leu Ala Val Pro Gly Ser Trp Phe Leu Leu Met Phe Phe Ala Gly Ala
   435 440 445
   Val Arg Leu Thr Gly Pro Phe Val Thr Met Ile Tyr Ser Met Ile Thr
   450 455 460
   Gly Asp Met Phe Thr Phe Gly Ile Ile Tyr Ser Ile Leu Leu Phe Gly
   465 470 475 480
   Phe Ser Gln Ser Phe Tyr Phe Leu Tyr Lys Gly Ser Pro Asn Val Lys
   485 490 495
   Gln Thr Leu Phe His Ser Tyr Pro Thr Thr Trp Met Ala Leu Phe Gln
   500 505 510
   Ile Thr Leu Gly Asp Tyr Asn Tyr Pro Glu Leu Ala Phe Thr Val Tyr
   515 520 525
   Pro Thr Leu Ser Lys Ile Val Phe Thr Ser Phe Met Val Leu Val Pro
   530 535 540
   Ile Leu Leu Leu Asn Met Leu Ile Ala Met Met Gly Asn Thr Tyr Ala
   545 550 555 560
   His Val Ile Glu Arg Ser Glu Lys Glu Trp Met Lys Gln Trp Ala Lys
   565 570 575
   Ile Val Val Ala Leu Glu Arg Ala Val Pro Ala Ala Asp Ala His Asn
   580 585 590
   Tyr Leu Gln Glu Tyr Ser Ile Lys Leu Gly Pro Gly Asp Asp Pro Ser
   595 600 605
   Thr Glu Gln Arg Gly Val Met Val Ile Lys Ser Lys Ser Lys Thr Arg
   610 615 620
   Ala Lys Gln Arg Lys Gly Ala Val Ser Asn Trp Lys Arg Val Gly Lys
   625 630 635 640
   Val Thr Ile Asn Glu Leu Arg Lys Arg Gly Met Thr Gly Glu Gln Leu
   645 650 655
   Asn Arg Ile Met Trp Gly Arg Ala Ser Ile Ser Thr Pro Val Arg Gln
   660 665 670
   Pro Ala Met Ala Leu Leu Leu Glu Asp Ser Glu Glu Glu Asn Ala Ala
   675 680 685
   Asn Asn Thr Gly Pro Phe Gly Gly Ala Leu Thr Ala Ala Leu Asp Val
   690 695 700
   Met Ala Phe Thr His Asp Ile Asp Leu Asn Ile Gly Gln Thr Pro Val
   705 710 715 720
   Thr Val Ser Gly Leu Gly Gly Val Ser Val Arg Ser Gly Ala Glu Gly
   725 730 735
   Leu Pro Pro Glu Gln Pro Phe His Asp Pro Phe Arg Gln Leu Val Ile
   740 745 750
   Glu Ala Glu Asn Pro His Ile Asp Glu Ser Glu Leu Ala Ala Leu Ala
   755 760 765
   Asn Ala Ala Ala Met Ser Val Val Ala Pro Asp Asp Ile Pro Thr Pro
   770 775 780
   Val Val Thr Pro Glu Pro Val Ser Ala Leu Pro Arg Leu Pro Ser Leu
   785 790 795 800
   His Leu Glu Pro Ser Asp Ser Asp Gly Leu Gly Asp Gly Pro Leu Leu
   805 810 815
   Gly Arg Gly Ser Arg Val Arg Arg Val Arg Ser Ala His Leu Lys Gln
   820 825 830
   Gln Lys Ala Ser Ser Thr Asn Gly Asn Gly Tyr Ser Asp Arg Gln Arg
   835 840 845
   Leu Phe Ser Ser Lys Asn Glu Asp Ser Ser Ser Asn Thr Ser Leu Asp
   850 855 860
   Phe Asp Leu Thr Asn Pro Pro Ser Gln Leu Arg Leu Thr Pro Pro Pro
   865 870 875 880
   Arg Pro Val Thr Ala Glu Ile Val Ala Val Asn Pro Lys Lys Tyr Ile
   885 890 895
   Gly Gly Asn Ser Gln Glu Arg Leu Arg Pro Lys Thr Ala Lys Val Asn
   900 905 910
   Arg Val Ala Pro Thr Pro Asp Val Pro Val Arg Arg Gly Ile Ser Ala
   915 920 925
   Asn Gly Ile Pro Glu Ser Leu Pro Ser Pro Pro Asp Pro Leu Glu Pro
   930 935 940
   Trp Ser Thr Arg Asn Ile Val Asn Met Asn Ala Ile Leu Ala Trp Gln
   945 950 955 960
   Gln Ser Asp Gln Asp Ser Leu
   965
<210> 106
   <211> 984
   <212> PRT
   <213> Euschistus heros
<220>
   <221> MOD_RES
   <222> (383)..(383)
   <223> Any amino acid
<220>
   <221> MOD_RES
   <222> (466)..(466)
   <223> Any amino acid
<220>
   <221> MOD_RES
   <222> (649)..(649)
   <223> Any amino acid
<400> 106
   Met Gly Ile Ile Trp Gly Ser Gly Ala Ser Ser Val Asn Ala Gly Thr
   1 5 10 15
   Val Leu Asp Arg Val Ile Ser Gln Ala Ser Asn Lys Asp Glu Cys Leu
   20 25 30
   Leu Tyr Lys Leu Ala Asn Tyr Lys Lys Gly Gly Glu Leu Ile Asp Ala
   35 40 45
   Tyr Asn Ala Gly Gly Gln Ser Glu Val Glu Lys Leu Ile Arg Glu Gln
   50 55 60
   Phe Gly Gln Leu Met Tyr Asn Glu Gly Lys Gly Ala Leu Ile Asn Arg
   65 70 75 80
   Ala Glu Tyr Leu Arg Trp Lys Phe Arg Asp Met Gln Gln Val Gln Ile
   85 90 95
   Pro Ile Glu Ala Ser Leu Ser Ser Gln Asp Pro Leu Ser Lys Trp Glu
   100 105 110
   Asp His Gln Ala Cys Trp Gln Met Gln Tyr Arg Gly Ser Leu Gly Glu
   115 120 125
   Thr Leu Leu His Val Leu Ile Ile Cys Asp Thr Lys Ile His Thr Arg
   130 135 140
   Leu Ala Arg Thr Leu Leu Lys Cys Phe Pro Asn Leu Ala Ile Asp Val
   145 150 155 160
   Val Glu Gly Glu Glu Tyr Leu Gly Ala Ser Ala Leu His Leu Ala Ile
   165 170 175
   Ala Tyr Phe Asn Asn Glu Leu Val Gln Asp Leu Val Glu Ala Gly Ala
   180 185 190
   Asn Val Glu Gln Arg Ala Ile Gly Ser Phe Phe Leu Pro Arg Asp Gln
   195 200 205
   Gln Gly Gln Arg Pro Ser Lys His Thr Asp Tyr Glu Gly Leu Ala Tyr
   210 215 220
   Leu Gly Glu Tyr Pro Leu Ala Trp Ala Ala Cys Cys Ala Asn Glu Ser
   225 230 235 240
   Ile Tyr Asn Leu Leu Leu Asp Asn Gly Ala Asn Pro Asp His Arg Asp
   245 250 255
   Thr Phe Gly Asn Met Ile Leu His Met Val Val Val Cys Asp Lys Leu
   260 265 270
   Asp Met Phe Gly Tyr Ala Leu Arg His Pro Lys Met Pro Ala Ser Asn
   275 280 285
   Gly Ile Ala Asn Val Ala Gly Leu Thr Pro Leu Thr Leu Ala Cys Lys
   290 295 300
   Leu Gly Arg Ala Lys Val Phe Arg Glu Met Leu Glu Leu Ser Ala Arg
   305 310 315 320
   Glu Phe Trp Arg Tyr Ser Asn Ile Thr Cys Ser Ala Tyr Pro Leu Asn
   325 330 335
   Ala Leu Asp Thr Leu Leu Pro Asp Gly Arg Thr Asn Trp Asn Ser Ala
   340 345 350
   Leu Phe Ile Ile Leu Asn Gly Thr Lys Glu Glu His Leu Asp Met Leu
           355                 360                 365
   Asp Gly Gly Ile Ile Gln Arg Leu Leu Glu Glu Lys Trp Lys Xaa Phe
       370                 375                 380
   Ala Arg Arg Gln Phe Leu Lys Arg Leu Val Ile Leu Met Leu His Leu
   385                 390                 395                 400
   Leu Cys Leu Ser Gly Ala Val Tyr Leu Arg Pro Thr Asp Arg Thr Lys
                   405                 410                 415
   Pro Leu Leu Gly Gly Asp Asp Trp Lys Ser Val Ala Arg Gln Gly Phe
               420                 425                 430
   Glu Val Ala Thr Val Leu Gly Val Leu Ser Tyr Val Ile Val Gln Gln
           435                 440                 445
   Gly Gly Glu Ile Arg Asn Gln Gly Phe Ile Ser Phe Ile Lys Gln Leu
       450                 455                 460
   Asp Xaa Ala Lys Ala Ile Phe Leu Val Ser Asn Ile Leu Ile Leu Ile
   465                 470                 475                 480
   Cys Ile Pro Phe Arg Leu Met Asp Asp Lys Arg Thr Glu Glu Ala Ile
                   485                 490                 495
   Leu Val Phe Ala Val Pro Gly Ser Trp Phe Leu Leu Met Phe Phe Ala
               500                 505                 510
   Gly Ala Val Arg Leu Thr Gly Pro Phe Val Thr Met Val Tyr Ser Met
           515                 520                 525
   Ile Val Gly Asp Met Phe Thr Phe Gly Ile Ile Tyr Ser Ile Val Leu
       530                 535                 540
   Phe Gly Phe Ser Gln Ser Phe Tyr Phe Leu Tyr Lys Gly Phe Pro Gly
   545                 550                 555                 560
   Val Lys Asn Thr Leu Tyr Ser Ser Tyr His Ser Thr Trp Met Ala Leu
                   565                 570                 575
   Phe Gln Ile Thr Leu Gly Asp Tyr Asn Tyr Ala Glu Leu Ser His Thr
               580                 585                 590
   Ser Tyr Pro Thr Leu Ser Lys Thr Val Phe Ala Ile Phe Met Ile Leu
           595                 600                 605
   Val Pro Ile Leu Leu Leu Asn Met Leu Ile Ala Met Met Gly Asn Thr
   610 615 620
   Tyr Ala His Val Ile Glu Gln Ser Glu Lys Glu Trp Met Lys Gln Trp
   625 630 635 640
   Ala Lys Ile Val Val Ser Leu Glu Xaa Ala Val Asn Gln Glu Asp Cys
   645 650 655
   Lys Gln Tyr Leu Gln Glu Tyr Ser Ile Lys Leu Gly Pro Gly Asp Asp
   660 665 670
   Pro Ser Thr Glu Gln Arg Gly Val Met Val Ile Lys Ser Lys Ser Lys
   675 680 685
   Thr Arg Ala Lys Gln Arg Lys Gly Ala Val Ala Asn Trp Lys Arg Val
   690 695 700
   Gly Lys Val Thr Ile Asn Glu Leu Arg Lys Arg Gly Met Asn Gly Glu
   705 710 715 720
   Gln Leu Arg Arg Ile Met Trp Asp Arg Ala Ser Ile Ser Thr Pro Val
   725 730 735
   Lys Val Pro Gln Asn Pro Val Val Asp Val Leu Met Glu Asn Thr Ala
   740 745 750
   Glu Gln Gln Ala Gln Gln Thr Pro Gly Ser Phe Gly Gly Ala Leu Thr
   755 760 765
   Ala Ala Leu Asp Val Met Ala Phe Thr His Asp Leu Asp Leu Ser Ala
   770 775 780
   Ser Gly Ile Thr Ser Ser Gly Leu Asn Ala Lys Glu Leu Ile Val Ser
   785 790 795 800
   Asp Pro Phe Arg Asp Leu Val Leu Ser Ser Glu Ile Asp Val Gly Glu
   805 810 815
   Glu Glu Leu Ala Thr Leu Ala Glu Ala Ala Val Leu Ser Val Met Ser
   820 825 830
   Gly Gln Ser Asp Glu Ser Glu Lys Leu Lys Ile Ala Lys Phe Asn Lys
   835 840 845
   Gln Ile Ser Phe Glu Gln Asn Ala Val Leu Glu Val Ser Asp Ser Asp
   850 855 860
   Gly Phe Leu Asp Gly Gln Pro Leu Gly Gln Gly Ser Arg Ala Arg Lys
   865 870 875 880
   Val Lys Ser Ala Gln Glu Arg Gln Arg Ala Glu Arg Thr Trp Gly Gly
   885 890 895
   Thr Ser Ile Ser Ser Ile Glu Glu Pro Pro Pro Tyr Leu Pro Leu Pro
   900 905 910
   Pro Pro Pro Pro Leu His Ser His Gln Arg Pro Arg Pro Lys Thr Ala
   915 920 925
   Lys Pro Asn Arg Val Val Pro Glu Ala Val Val Ala Lys Arg Pro Gln
   930 935 940
   Ser Ser Ala Leu Gly Asp Arg Val Lys Ser Pro Pro Glu Leu Leu Glu
   945 950 955 960
   Pro Trp Ser Thr Arg Gly Ile Ala Thr Ile Asn Thr Ile Leu Ala Trp
   965 970 975
   Gln Pro Ser Asp Gln Asp Ser Met
   980
<210> 107
   <211> 8
   <212> PRT
   <213> Artificial Sequence
<220>
   <221> source
   <223> /note="Description of Artificial Sequence: Synthetic peptide"
<400> 107
   Asp Tyr Lys Asp Asp Asp Asp Lys
   1 5
<210> 108
   <211> 18
   <212> DNA
   <213> Artificial Sequence
<220>
   <221> source
   <223> /note="Description of Artificial Sequence: Synthetic primer"
<400> 108
   ggactttcca aaatgtcg 18
<210> 109
   <211> 56
   <212> DNA
   <213> Artificial Sequence
<220>
   <221> source
   <223> /note="Description of Artificial Sequence: Synthetic primer"
<400> 109
   ccggccgttt atcactacag aattcgaagc ttaaccttat cgtcgtcatc cttgta 56
<210> 110
   <211> 823
   <212> PRT
   <213> Apis mellifera
<400> 110
   Met Gly Asn Thr Glu Ser Asn Val Ala Ser Gly Val Lys Lys Gln Thr
   1               5                   10                  15
   Asp Ala Ser Ser Ile Leu Leu Tyr Lys Leu Val Asp Leu Lys Gly Gly
               20                  25                  30
   Gly Leu Leu Val Asp Met Met Lys Arg Ala Thr Gln Thr Lys Gln Tyr
           35                  40                  45
   Ala Glu Leu Asp His Ala Leu Arg Thr Lys Val Glu Pro Tyr Leu Tyr
       50                  55                  60
   Asn Lys Gly Lys Gly Lys Trp Ile Pro Ile Glu Lys Leu Val Leu Leu
   65                  70                  75                  80
   Arg Asn Lys Asp Arg Pro Lys His Lys Met Leu Pro Pro Leu Arg Ala
                   85                  90                  95
   Met Glu Asn Pro Ala Asp Tyr Asp Ile Asp Lys Asp Met Gly Glu Asp
               100                 105                 110
   Glu Val Asp Glu Thr Lys Ile Asp Lys Ser Lys Tyr Arg Leu Val Cys
           115                 120                 125
   Trp Ser Leu Ser Glu Arg Gly Ala Val Gly Glu Thr Ile Leu His Leu
       130                 135                 140
   Cys Met Leu His Ala Thr Ala Ile His Ile Asp Leu Ala Lys Arg Leu
   145                 150                 155                 160
   Leu Arg Phe Tyr Pro Lys Leu Ile Asn Asp Val Tyr Ile Ser Asp Glu
                   165                 170                 175
   Tyr Tyr Gly Glu Ser Ala Leu His Ile Ala Ile Val Asn Glu Asp Pro
   180 185 190
   Ser Met Val Lys Phe Leu Leu Asp Ser Gly Ala Asp Val His Glu Arg
   195 200 205
   Cys Ile Gly Asn Phe Met Cys Pro Glu Asp Gln Lys Ala Ser Arg Ala
   210 215 220
   Asp Ser Leu Asp His Glu Trp Val Cys Val Thr Pro Glu Thr Asn Tyr
   225 230 235 240
   Asn Gly Tyr Val Tyr Trp Gly Glu Tyr Pro Leu Asn Phe Ala Ala Cys
   245 250 255
   Leu Gly Gln Glu Glu Cys Tyr Arg Leu Ile Leu Ala Arg Gly Ala Asp
   260 265 270
   Pro Asp Lys Gln Asp Thr Asn Gly Asn Thr Val Leu His Met Leu Val
   275 280 285
   Ile Tyr Glu Lys Leu Ala Thr Phe Asp Met Ala Tyr Glu Val Gly Ala
   290 295 300
   Ser Leu Ala Ile Arg Asn Ala Gln His Leu Thr Pro Leu Thr Leu Ser
   305 310 315 320
   Ala Lys Leu Ala Lys Ile Glu Met Phe Phe His Ile Leu Asn Ile Glu
   325 330 335
   Arg Glu Ile Tyr Trp Gln Ile Gly Ser Ile Thr Cys Ala Ala Tyr Pro
   340 345 350
   Leu Ser Gln Val Asp Thr Ile Asp Val Asp Thr Gly Ser Ile Ser His
   355 360 365
   Asn Ser Ala Leu Asn Leu Val Val Phe Gly Glu Lys Asp Glu His Leu
   370 375 380
   Glu Leu Met Asp Gly Ile Leu Val Asp Leu Leu Asn Ala Lys Trp Asn
   385 390 395 400
   Thr Phe Val Lys Phe Arg Phe Tyr Arg Gln Phe Phe Leu Phe Cys Phe
   405 410 415
   Tyr Phe Val Leu Ser Leu Ile Ser Phe Thr Leu Arg Pro Gly Pro Ala
   420 425 430
   Thr Thr Ser Ser Leu Asp Ile Val Glu Asn Leu Thr Ser Ala Leu Ser
   435 440 445
   Asp Met Leu Gly Thr Tyr Arg Asn Glu Ile Lys Leu Phe Asn Arg Ser
   450 455 460
   Asp Lys Thr Ser Ile Tyr Ser Ser Arg Asn Asp Ala Lys Pro Ile Asp
   465 470 475 480
   Glu Thr Ile Phe Phe Pro Ser Tyr Phe Ser Lys Ser Leu Val Ser Asn
   485 490 495
   Val Arg Trp Asn Asn Leu Thr Glu Glu Cys Arg Leu Met His Leu Asp
   500 505 510
   Thr Leu Ser Thr Lys Ile Arg Leu Thr Ala Glu Val Leu Met Glu Ile
   515 520 525
   Ala Ala Thr Leu Tyr Ile Phe Ala Ala Leu Arg Glu Ala Arg Phe Leu
   530 535 540
   Gly Leu Asn Met Phe Ile Glu Asn Leu Met Thr Ala Pro Ser Arg Val
   545 550 555 560
   Met Phe Leu Phe Ser Cys Cys Ile Leu Leu Thr Phe Pro Phe Leu Arg
   565 570 575
   Leu Ile Cys Ala Asp Glu Ile Glu Asp Met Leu Ala Val Val Val Met
   580 585 590
   Leu Thr Thr Ala Pro Tyr Phe Leu Phe Phe Cys Arg Gly Phe Lys Thr
   595 600 605
   Val Gly Pro Phe Val Val Met Ile Tyr Arg Met Ile Met Gly Asp Leu
   610 615 620
   Leu Arg Phe Val Ser Ile Tyr Leu Val Phe Val Met Gly Phe Ser Gln
   625 630 635 640
   Ala Tyr Tyr Ile Ile Phe Leu Ser Phe Asp Asn Pro Asn Thr Pro Glu
   645 650 655
   Gly Val Asp Asp Ser Met Ser Asn Pro Met Pro Ser Pro Ile Glu Ser
   660 665 670
   Ile Met Ala Met Phe Leu Met Ser Met Thr Asn Phe Gly Asp Tyr Tyr
   675 680 685
   Gly Ala Phe Glu Arg Thr Gln His Glu Met Glu Ala Lys Phe Leu Phe
   690 695 700
   Val Val Tyr Met Ala Ile Val Ala Ile Leu Leu Val Asn Met Leu Ile
   705 710 715 720
   Ala Met Met Gly Asn Thr Tyr Gln Lys Ile Ala Glu Thr Arg Asn Glu
   725 730 735
   Trp Gln Arg Gln Trp Ala Arg Ile Val Leu Val Val Glu Arg Gly Val
   740 745 750
   Ser Pro Asp Glu Arg Leu Lys Lys Leu Met Asp Tyr Ser Gln Pro Met
   755 760 765
   Ser Asp Gly Arg Arg Ala Leu Val Leu Arg Leu Asn Gln Ser Glu Glu
   770 775 780
   Asp Lys Glu Glu Met Lys Glu Ile Leu Glu Met Lys Arg Thr His Asp
   785 790 795 800
   Lys Leu Tyr Lys Arg Arg Gln Ser Lys Thr Met Lys Lys Ile Leu Thr
   805 810 815
   Ser Glu Asp Asn Val Ile Leu
   820
<210> 111
   <211> 832
   <212> PRT
   <213> Nasonia vitripennis
<400> 111
   Met Gly Asn Ala Glu Ser Asn Val Thr Ser Gly Val Lys Lys Gln Thr
   1               5                   10                  15
   Asp Ala Gly Ala Ile Ala Leu Tyr Lys Leu Val Asp Leu Lys Gly Gly
               20                  25                  30
   Gly Leu Leu Val Asp Met Met Lys Arg Ala Ser Gln Thr Lys Gln Tyr
           35                  40                  45
   Ala Glu Ile Asp His Ala Ile Arg Thr Lys Val Glu Pro Phe Leu Tyr
       50                  55                  60
   Asn Gly Gly Lys Gly Lys Trp Ile Pro Ile Ala Lys Leu Val Leu Leu
   65 70 75 80
   Arg Asn Lys Glu Arg Gly Arg His Lys Met Leu Pro Val Leu Lys Ala
   85 90 95
   Met Glu Lys Pro Glu Asp Tyr Asp Ile Asp Lys Asp Met Ala Asn Asp
   100 105 110
   Pro Glu Pro Asp Glu Asn Thr Ile Gly Met Met Ile Met Lys Ile Leu
   115 120 125
   Ser Asn Lys Ser Lys Tyr Lys Leu Val Cys Trp Thr Leu Ser Asp Arg
   130 135 140
   Gly Ala Val Gly Glu Thr Ile Leu His Leu Cys Met Leu Asn Ala Thr
   145 150 155 160
   Gln Leu His Ala Asp Leu Ala Lys Arg Leu Leu Arg Phe Tyr Pro Asn
   165 170 175
   Leu Ile Asn Asp Ile Tyr Ile Asp Asp Glu Tyr Tyr Gly Glu Asn Val
   180 185 190
   Leu His Ile Ala Ile Val Asn Glu Asp Pro Ser Met Val Lys Phe Leu
   195 200 205
   Leu Asp Ser Gly Ala Asn Val Asn Glu Arg Cys Cys Gly Asn Phe Met
   210 215 220
   Cys Pro Glu Asp Gln Lys Ala Ser Arg Asn Asp Ser Val Glu His Glu
   225 230 235 240
   Trp Val Cys Val Cys Ser Glu Thr Asn Tyr Asp Gly Tyr Val Tyr Trp
   245 250 255
   Gly Glu Tyr Pro Leu Ser Phe Ala Ala Cys Leu Asp Gln Glu Glu Cys
   260 265 270
   Tyr Arg Leu Ile Leu Ala Lys Gly Ala Asp Pro Asp Ser Gln Asp Thr
   275 280 285
   Asn Gly Asn Thr Val Leu His Met Leu Val Ile Tyr Glu Lys Leu Glu
   290 295 300
   Thr Phe Asp Met Ala Tyr Glu Val Gly Ser Ser Leu Ser Ile Arg Asn
   305 310 315 320
   Val Leu Gln Leu Thr Pro Leu Thr Leu Ala Ala Lys Leu Ala Arg Val
   325 330 335
   Glu Met Phe Phe His Ile Leu Asn Ile Glu Arg Glu Ile Tyr Trp Gln
   340 345 350
   Ile Gly Ser Ile Thr Cys Ala Ala Tyr Pro Leu Ser Gln Ile Asp Thr
   355 360 365
   Ile Asp Val Asp Thr Gly Lys Ile Ser Asn Asn Ser Ala Leu Asn Leu
   370 375 380
   Val Val Phe Gly Asp Lys Glu Glu His Leu Lys Leu Leu Glu Gly Val
   385 390 395 400
   Leu Ile Asp Leu Leu Asn Ala Lys Trp Asn Thr Phe Val Lys Ser Arg
   405 410 415
   Phe Tyr Gln Gln Phe Tyr Leu Phe Phe Cys Tyr Phe Ile Leu Ser Leu
   420 425 430
   Ile Ser Cys Thr Leu Arg Pro Gly Pro Ile Thr Lys Thr Asp Ala Pro
   435 440 445
   Thr Thr Thr Pro His Pro Met Asn Asp Thr Ser Leu Tyr Ala Asp Val
   450 455 460
   Asn Glu Thr Ser Ala Ser Asp Pro Phe Asp Leu Asn Ala Ser Asp Ile
   465 470 475 480
   Val Ile Leu Lys Lys Gly Lys Lys Glu Leu Asp Asp Trp Trp Asp Asp
   485 490 495
   Leu Thr Glu Asp Cys Arg Leu Met Gln Met Asn Ser Thr Ser Ala Lys
   500 505 510
   Ile Arg Leu Thr Ala Glu Ile Phe Met Glu Phe Gly Ala Ile Leu Tyr
   515 520 525
   Ile Cys Ala Ala Leu Arg Glu Ala Arg Phe Leu Gly Leu Asn Met Phe
   530 535 540
   Ile Glu Asn Leu Met Thr Ala Pro Ser Arg Val Met Phe Leu Phe Ser
   545 550 555 560
   Cys Cys Ile Leu Leu Ser Phe Pro Phe Leu Arg Met Ser Cys Ala Asp
   565 570 575
   Glu Val Glu Asp Ile Leu Ala Val Val Val Met Leu Thr Thr Ala Pro
   580 585 590
   Tyr Phe Leu Phe Phe Cys Arg Gly Phe Lys Thr Val Gly Pro Phe Val
   595 600 605
   Val Met Ile Tyr Arg Met Ile Met Gly Asp Leu Leu Arg Phe Val Ser
   610 615 620
   Ile Tyr Leu Val Phe Val Met Gly Phe Ser Gln Ala Tyr Tyr Ile Ile
   625 630 635 640
   Phe Leu Ser Phe Asp Asn Pro Ile Thr Pro Glu Gly Val Asp Asp Ser
   645 650 655
   Lys Ala Asn Pro Leu Pro Ser Ala Met Glu Ser Ile Met Ala Met Phe
   660 665 670
   Leu Met Ser Met Thr Asn Phe Gly Asp Tyr Tyr Asp Ala Phe Glu Asn
   675 680 685
   Thr Glu His Glu Met Leu Ala Lys Cys Leu Phe Val Val Tyr Met Ala
   690 695 700
   Ile Val Ser Val Leu Leu Val Asn Met Leu Ile Ala Met Met Gly Asn
   705 710 715 720
   Thr Tyr Gln Lys Ile Ala Glu Thr Arg Asn Glu Trp Gln Arg Gln Trp
   725 730 735
   Ala Arg Ile Val Leu Val Val Glu Arg Gly Val Ala Pro Ala Glu Arg
   740 745 750
   Leu Lys Lys Leu Asn Val Tyr Ser Gln Pro Met Ser Asp Gly Arg Lys
   755 760 765
   Ala Leu Val Leu Arg Leu Asn Gln Thr Asp Lys Asp Lys Glu Glu Met
   770 775 780
   Lys Asp Ile Leu Glu Met Lys Arg Ile His Ile Lys Ser Ile Lys Arg
   785 790 795 800
   Arg Lys Asp Lys Leu Gln Lys Ala Lys Glu Asp Ala Glu Lys Leu Gln
   805 810 815
   Gln Gln Met Ala Asp Lys Asp Asn Lys Asp Lys Ala Val Ile Ile Gln
   820 825 830
<210> 112
   <211> 1435
   <212> PRT
   <213> Nasonia vitripennis
<220>
   <221> MOD_RES
   <222> (351)..(351)
   <223> Any amino acid
<400> 112
   Met Gly Gly Ala Cys Ser Cys Gln Gly Arg Asp Ser Gln Val Asn Ala
   1               5                   10                  15
   Gly Ser Ile Leu Asp Arg Val Ile Ser Gln Ala Ser Asp Glu Asp Gln
               20                  25                  30
   Cys Leu Leu Tyr Arg Leu Ala Asn Tyr Lys Lys Gly Gly Glu Leu Ile
           35                  40                  45
   Glu Ala Tyr Asn Gln Gly Gly Gln Ala Glu Val Glu Arg Leu Ile Arg
       50                  55                  60
   Glu Gln Phe Gly Ile Leu Met Tyr Ala Asp Gly Lys Gly Gln Thr Ile
   65                  70                  75                  80
   Asn Arg Ala Glu Tyr Leu Arg Trp Lys Phe Arg Asp Leu Asp Gln Val
                   85                  90                  95
   Ile Leu Pro Ile Glu Ala Ser Leu Ser Arg Phe Asp Pro Leu Ala Gln
               100                 105                 110
   Trp Ser Asp His Glu Ala Cys Trp Gln Met Gln Tyr Arg Gly Ser Leu
           115                 120                 125
   Gly Glu Thr Leu Leu His Val Leu Ile Ile Cys Asp Thr Arg Thr His
       130                 135                 140
   Thr Arg Ile Ala Arg Thr Leu Leu Lys Cys Phe Pro Arg Leu Ala Ile
   145                 150                 155                 160
   Asp Val Val Glu Gly Glu Glu Tyr Leu Gly Ala Ser Ala Leu His Leu
                   165                 170                 175
   Ala Ile Ala Tyr Ala Asn Asn Glu Leu Val Gln Asp Leu Val Glu Ala
               180                 185                 190
   Gly Ala Ile Val Ser Gln Arg Ala Ile Gly Ser Phe Phe Leu Pro Arg
   195 200 205
   Asp Gln Gln Arg Pro Arg Pro Ala Lys Ser Thr Asp Tyr Glu Gly Leu
   210 215 220
   Ala Tyr Leu Gly Glu Tyr Pro Leu Ala Trp Ala Ala Cys Cys Ala Asn
   225 230 235 240
   Glu Ser Val Tyr Asn Leu Leu Leu Asp Ser Gly Ala His Pro Asp Glu
   245 250 255
   Gln Asp Thr Phe Gly Asn Ser Ile Leu His Met Val Val Val Cys Asp
   260 265 270
   Lys Leu Val Arg Phe Gly Tyr Ala Leu Arg His Pro Lys Leu Pro Ala
   275 280 285
   Ser Asn Gly Ile Ala Asn Ala Ala Gly Leu Thr Pro Leu Thr Leu Ala
   290 295 300
   Cys Gln Leu Gly Arg Ala Glu Val Phe Arg Glu Met Leu Glu Leu Ser
   305 310 315 320
   Ala Arg Glu Phe Trp Arg Tyr Ser Asn Ile Thr Cys Ser Ala Tyr Pro
   325 330 335
   Leu Asn Ala Leu Asp Thr Leu Leu Pro Asp Gly Arg Thr Ser Xaa Asn
   340 345 350
   Ser Ala Leu Phe Ile Ile Leu Asn Gly Thr Lys Glu Glu His Leu Asp
   355 360 365
   Met Leu Asp Gly Gly Ile Ile Gln Arg Leu Leu Glu Glu Lys Trp Lys
   370 375 380
   Thr Phe Ala Arg Leu Gln Phe Leu Lys Arg Leu Val Ile Leu Val Phe
   385 390 395 400
   His Leu Val Ser Leu Ser Leu Ala Val Tyr Phe Arg Pro Ala Asp Thr
   405 410 415
   Asp Ala Glu Leu Ala Gln Trp Pro Glu Glu Ile Thr Asp Val Val Arg
   420 425 430
   Val Ile Ala Glu Cys Val Thr Val Leu Gly Val Leu Gly Tyr Ile Leu
   435 440 445
   Leu Gln Leu Gly Gly Glu Ile Val Asn Ile Gly Phe Phe Ser Phe Phe
   450 455 460
   Lys Gln Leu Ser His Glu Pro Ala Lys Phe Ile Phe Leu Ile Ser Asn
   465 470 475 480
   Leu Leu Ile Leu Ala Cys Ile Pro Cys Arg Leu Ser Gly Asp Arg His
   485 490 495
   Thr Glu Asp Ala Ile Leu Val Val Ala Val Pro Gly Ser Trp Phe Leu
   500 505 510
   Leu Met Phe Phe Ala Gly Ala Val Arg Leu Thr Gly Pro Phe Val Thr
   515 520 525
   Met Val Tyr Ser Met Ile Leu Gly Asp Met Arg Thr Phe Gly Ile Ile
   530 535 540
   Tyr Met Ile Val Ile Cys Gly Phe Thr Gln Ala Phe Tyr Phe Leu Tyr
   545 550 555 560
   Lys Gly Tyr Pro Gly Val Lys Thr Thr Leu Phe His Ser Tyr Pro Ser
   565 570 575
   Thr Trp Met Ala Leu Phe Gln Ile Thr Leu Gly Asp Tyr Ser Tyr Ser
   580 585 590
   Asp Leu Ser Met Thr Thr Tyr Pro Asn Leu Ser Lys Ala Val Phe Thr
   595 600 605
   Ile Phe Met Val Leu Val Pro Ile Leu Leu Leu Asn Met Leu Ile Ala
   610 615 620
   Met Met Gly Asn Thr Tyr Ala His Val Ile Glu Gln Ser Glu Lys Glu
   625 630 635 640
   Trp Met Lys Gln Trp Ala Lys Ile Val Val Ser Leu Glu Arg Ala Val
   645 650 655
   Ser Gln Asp Asp Ala His Asn Tyr Leu Gln Glu Tyr Ser Ile Lys Leu
   660 665 670
   Gly Pro Gly Asp Asp Pro Asn Asp Pro Ala Ser Glu Gln Arg Gly Val
   675 680 685
   Met Val Ile Lys Ser Lys Ser Lys Thr Arg Ala Arg Gln Arg Lys Gly
   690 695 700
   Ala Val Ala Asn Trp Lys Arg Val Gly Lys Val Thr Ile Ala Glu Leu
   705 710 715 720
   Lys Lys Arg Gly Met Thr Gly Glu Glu Leu Arg Arg Ile Met Trp Gly
   725 730 735
   Arg Ala Ser Phe Ser Thr Pro Val Arg Ser Ser Pro Ile Leu Met Glu
   740 745 750
   Pro Val Ser Ser Val Pro Gly Gly Phe Gly Asp Ala Leu Thr Ser Ala
   755 760 765
   Leu Asp Val Met Thr Phe Ala Ala Asp Val Pro Val Pro Ile Glu Gly
   770 775 780
   Ser Ile Pro Lys Pro Val Ala Val Ala Pro Gly Gln Gln Pro Thr Thr
   785 790 795 800
   Thr Leu Ala Leu Pro Asn Gly Ala Ala Lys Pro Gln Gln Pro Met Met
   805 810 815
   Thr Thr Ser Thr Thr Thr Thr Thr Ile Thr Thr Thr Val Gln Lys Thr
   820 825 830
   Leu Val Thr Gly Thr Ala Thr Thr Pro Ala Val Gly Val Thr Lys Thr
   835 840 845
   Val Ala Val Pro Ala Ala Thr Thr Val Ser Thr Thr Thr Thr Arg Ala
   850 855 860
   Ser Ala Ser Ala Val Pro Pro Thr Val Arg Pro Ser Glu Leu Gly Pro
   865 870 875 880
   Ala Ser Ala Pro Cys Ser Ala Asp Pro Leu Leu Glu Leu Met Leu Ala
   885 890 895
   Thr Glu Asp Pro Gly Ser Ser Glu Glu Thr Leu Gln Arg Leu Ala His
   900 905 910
   Ser Ala Arg Ile Val Gly Glu Ser Ala Thr Thr Ser Ala Lys Ser Asp
   915 920 925
   Ile Asp Ser Ser Phe Leu Glu Arg Leu Ala Leu Gly Gly Leu Gly Leu
   930 935 940
   Ala Ala Met Pro Glu Gln Pro Asp Lys Pro Val Glu Pro Lys Lys Lys
   945 950 955 960
   Gln Tyr Leu Ala Leu Lys Ser Ser Leu Lys Ser Asp Arg Ala Ser Leu
   965 970 975
   Gln His Pro Leu Ser His Asp Gly Gln Leu Gly Thr Glu Ala Arg Leu
   980 985 990
   Arg Arg Ile Arg Ser Ala Ser Ser Arg Leu Ala Pro Thr Thr Thr Thr
   995 1000 1005
   Ser Thr Ser Arg Arg Lys Ser Gly Gln Gln Lys Gln Gln Arg Glu
   1010 1015 1020
   Ala Arg Asp Asp Glu Ser Thr Ser Ser Ala Ala Ser Leu Asp Asn
   1025 1030 1035
   Ile Ser Gly Tyr Gln Pro Leu Leu Asn Glu Pro Asp Thr Thr Ala
   1040 1045 1050
   Asp Gln Pro Ala Thr Gly Thr Glu Asp Asn Lys Pro Ala Asn Gly
   1055 1060 1065
   Arg Asn Leu Arg Lys Arg Pro Lys Thr Thr Ser Asn Glu Gly Lys
   1070 1075 1080
   Val Phe Cys Ser Arg Arg Thr Ile Val Val Leu Asn Lys Asn Lys
   1085 1090 1095
   Lys Asn Lys Lys Leu Glu Thr Ser Glu Ala Asn Met Asn Ala Arg
   1100 1105 1110
   Lys Lys Glu Thr Leu Leu Ala Leu His Asp Met Thr Ser Leu Ile
   1115 1120 1125
   Ser Asp Asn Trp Ile Gln Ile Asn Ile Ala Arg Phe Gln Asn Asn
   1130 1135 1140
   Tyr Ser His Ser Lys Val Thr Lys Asn Leu Lys Lys Cys His Leu
   1145 1150 1155
   His Ile Glu Ser Asp Val Leu Leu Thr Ser Gln Gln Cys Cys Ile
   1160 1165 1170
   Asn Ile Ser Asn Ser Lys Ala Lys Met Asn Gln Ala Leu Leu Val
   1175 1180 1185
   Val Leu Ser Phe Phe Val Val Asn Glu Asp Gln Pro Val Ala Gly
   1190 1195 1200
   Trp Thr Trp Gly Lys Ser Ala Thr Ser Ser Ser Asn Asn Ser Ile
   1205 1210 1215
   Asn Asp Asp Arg Ala Arg Tyr Cys Arg Ile Cys Ala Ser His Thr
   1220 1225 1230
   Met Cys Leu Phe Pro Tyr Asp Asp Pro Gly Pro Lys Cys Ala Ala
   1235 1240 1245
   Val Glu Asn Gly Asp Leu Glu Pro Glu Glu Ile Glu Trp Ile Leu
   1250 1255 1260
   Gln Arg His Asn Ser Leu Arg Asp Gly Ile Ala Arg Ala Trp Lys
   1265 1270 1275
   Ser Gln Tyr Arg Pro Leu Pro Ala Arg Asp Met Met Gln Val Phe
   1280 1285 1290
   Trp Asp Glu Glu Leu Ala Lys Ile Ala Arg Arg Trp Ala Leu Gln
   1295 1300 1305
   Cys Asn Ile His Glu Lys Asp Gln Cys Arg Asp Val Glu Glu Phe
   1310 1315 1320
   Ser Val Ala Gln Ser Val Ser Ala Leu Asp Leu Gln Asp Ala Gly
   1325 1330 1335
   Asn Arg Ser Glu Met Glu Arg Leu Lys Phe His Leu Arg Ser Trp
   1340 1345 1350
   Tyr Ser Gln Leu Glu Pro Asp Phe Ser Asn Ser Ala Gly Val Gly
   1355 1360 1365
   Leu Ala Thr Pro Ser Val Thr Asn Val Gly Cys Gly Arg Ala Thr
   1370 1375 1380
   Tyr Ser Val Ala Ile Asp Asp Ser Asp Val Ala Ala Ala Val Glu
   1385 1390 1395
   Val Leu Val Cys Asn Tyr Gly Pro Ile Asp Asp Pro Glu Thr Glu
   1400 1405 1410
   Asp Asp Ser Gly Cys Glu Thr Arg Ser Arg Arg Tyr Ser Glu Leu
   1415 1420 1425
   Cys Gln  Leu Thr Arg Arg Glu
       1430                1435

## Description

### SUBMISSION OF SEQUENCE LISTING

The Sequence Listing associated with this application is filed in electronic format *via* EFS-Web. The name of the text file containing the Sequence Listing is PF78630_SEQLIST. The size of the text file is 619 KB, and the text file was created on June 18, 2015.

### BACKGROUND

The present disclosure generally relates to methods for determining agents that bind directly to one or more Nanchung proteins, transient receptor potential (TRP) protein complexes and/or channels and/or transient receptor potential V (TRPV) protein complexes and/or channels. The present disclosure also relates to compositions and methods of insect control as well as mode of action classification for an insecticide composition comprising a compound capable of binding directly to the one or more Nanchung proteins, transient receptor potential (TRP) protein complexes and/or channels and/or transient receptor potential V (TRPV) protein complexes and/or channels. The present disclosure further relates to compositions and methods of insect control, as well as mode of action classification for an insecticide composition comprising a compound capable of directly binding to the one or more Nanchung proteins, transient receptor potential (TRP) protein complexes and/or channels and/or transient receptor potential V (TRPV) protein complexes and/or channels. The present disclosure even further relates to compositions and methods of insect control as well as mode of action classification for an insecticide composition comprising a compound capable of directly binding to the one or more Nanchung proteins, transient receptor potential (TRP) protein complexes and/or channels and/or transient receptor potential V (TRPV) protein complexes and/or channels wherein the compound is also capable of modulating the one or more TRP and/or TRPV channels.

Almost all field crops, plants, and commercial farming areas are susceptible to attacks by plant insect pests. Plant insect pests are a major factor in the loss of the world's commercially important agricultural crops resulting both, in economic hardship to farmers and nutritional deprivation for local populations in many parts of the world. In other words, insect pests cause great loses and damages to human agriculture, food supply, post-harvest storage, horticulture, animal health and public health.

While advances have been made in the control of these insects, these insects have been able to adopt and evade the control measures, resulting in resistant insect populations. As a result, there remains a need for better understanding of the mechanisms that underlie feeding and other behaviors of insect pests. Such knowledge would allow for the design of agents and strategies for intervening or preventing attacks on field crops, plants, and commercial farming areas by the insect pests.

Broad spectrum chemical pesticides and insecticides have been used extensively to control or eradicate plant insect pests of agricultural importance. There is, however, substantial interest in identifying and/or developing effective alternative insecticides. As such, effective methods for screening of the new insecticides would be desirable.

Alexandre Nesterov et al in "TRP Channels in Insect Stretch Receptors as Insecticide Targets", Neuron, Vol. 86, no. 3, 1 May 2015 (2015-05-01), pages 665-671, discloses pymetrozine and pyrifluquinazon and their use as insecticides against aphids and whiteflies. It also teaches that these two molecules activate insect TRP channels and inhibit feeding behavior of these insects.

What are needed, then, are new methods that can be employed in screening for agents that modulate insect behavior, bind directly to one or more Nanchung proteins, transient receptor potential (TRP) and/or transient receptor potential V (TRPV) protein complexes and/or channels and in some cases, screening for agents that can act as insecticides.

### BRIEF SUMMARY OF THE INVENTION

The invention is as defined in the appended claims.

In one embodiment, the present disclosure relates to a method for determining whether or not a candidate compound binds directly to one or more Nanchung proteins,. The method may include providing one or more Nanchung proteins, that may be located in a cell, derived from a cell, produced by a cell or produced in a cell-free system, contacting the one or more Nanchung proteins, with a candidate compound, and assaying to determine the binding affinity of a candidate compound to the one or more Nanchung proteins, , wherein the binding affinity of the candidate compound is indicative of the candidate compounds ability to directly bind to one or more Nanchung proteins, ,.

For example, in certain embodiments, the binding assay step may include at least one of the following steps: (1) contacting the one or more Nanchung proteins, with a compound, wherein the compound may be labeled or unlabeled; (2) measuring the concentration of the compound which is bound to the one or more Nanchung proteins,; (3) calculating the equilibrium dissociation constant (K_{d}) for the one or more compounds; (4) wherein the K_{d} may be determined using one or more of the following methods: (I)(a) maximum binding (Bₘₐₓ) and (b) the concentration of labeled compound needed to reach 50% of maximum binding, (II) fluorescence polarization assay, (III) surface plasmon resonance, (IV) isothermal calorimetry, (V) Schild analysis; (5) contacting the one or more Nanchung proteins, with a labeled compound having high affinity to the one or more Nanchung proteins,; (6) contacting the one or more Nanchung proteins, with a candidate compound in the presence of the labeled compound of step 1; (7) increasing the amount of the candidate compound available to contact the one or more Nanchung proteins, until binding of the labeled compound is near 0%; (8) calculating the equilibrium inhibition constant (Kᵢ) and/or the equilibrium dissociation constant (K_{d}) for competition assay using one or more of the following methods: (I) (a) determining amount of bound labeled compound in the absence of the candidate compound and (b) determining concentration of the candidate compound that is required to reduce the amount of bound labeled compound by 50%, (II) fluorescent polarization assay, and/or (III) Schild analysis; (9) comparing the inhibition constant of the candidate compounds to other candidate compounds; (10) comparing the inhibition constant of the candidate compounds to the disassociation constant of the labeled compound.

In the methods set forth herein, the one or more insect TRP channels, and/or TRPV channels may comprise one or more Nanchung proteins, Inactive proteins, TRP proteins, TRPV proteins, Nanchung and Inactive protein complexes, Nanchung and TRPV protein complexes, and/or Nanchung and TRP protein complexes. For clarity, the TRP and/or TRPV channels may also comprise a Nanchung protein and any other TRP channel proteins found in cellular membranes, inserted in artificial membranes and/or a cell-free system. It is to be understood that the Nanchung, may be found in cellular membranes, in solution, or in a cell-free system. For purposes of determining whether a candidate compound directly binds to the insect TRP and/or TRPV channels, the TRP and/or TRPV channels may comprise one or more Nanchung, TRP and/or TRPV protein complexes.
It is to be understood that Nanchung and Inactive proteins are both insect TRPV proteins. However, Nanchung may also form a complex with TRP proteins. The ratio of the Nanchung to Inactive proteins co-expressed in the TRP and/or TRPV complex is, preferably, about 3:1 to about 1:3, more preferably about 1:1. The ratio of the Nanchung to TRP proteins co-expressed in the TRP and/or TRPV complex is, preferably, about 3:1 to about 1:3, more preferably about 1:1. The candidate compound may be a small organic molecule, small inorganic molecule, polysaccharides, peptides, proteins, nucleic acids, an extract made from biological materials, and any combination thereof.

In another embodiment, the methods set forth above may use one or more of the following compounds as the labeled compound: afidopyropen,

In yet another embodiment, the present disclosure relates to a candidate compound selected by the methods described above. The candidate compound may be a modulator that inhibits or increases the activity of the insect TRPV channel. The candidate compound may be a modulator that inhibits a feeding behavior of an insect. Even further, the candidate compound may modulate and/or bind directly to the one or more Nanchung proteins, , and in particular to the same site on the one or more Nanchung proteins,. The candidate compound may further modulate and/or bind directly to one or more insect Nanchung proteins, , and in particular to the same site on the one or more Nanchung proteins,. The candidate compound may further modulate and/or bind directly to one or more arthropod Nanchung proteins, arthropod TRP and/or arthropod TRPV proteins, protein complexes and/or channels, and in particular to the same site on the one or more Nanchung proteins,. The candidate compound may further modulate and/or bind directly to one or more Mollusk Nanchung proteins, , and in particular to the same site on the one or more Nanchung proteins. The candidate compound may further modulate and/or bind directly to one or more Nematode Nanchung proteins, , and in particular to the same site on the one or more Nanchung proteins,. The candidate compound may further modulate and/or bind directly to one or more pest Nanchung proteins. The candidate compound may further be unable to modulate and/or bind directly to one or more Mammalian Nanchung proteins and/or other non-target/non-pest species.

In another embodiment, the present invention relates to a composition containing the candidate compound identified by one or more of the methods set forth herein.

In another embodiment, the present disclosure relates to a method of insect control that includes applying to an insect or to an insects habitat, feeding source, and/or environment/habitat a composition comprising the candidate compound selected by one or more of the methods described herein or a compound selected by one or more of the methods described above. The compound may bind directly to the insect Nanchung protein,. The insect may be an agricultural/horticultural pest or a disease vector or a parasite.

### BRIEF DESCRIPTION OF THE DRAWINGS

Figure 1 depicts molecular structures of afidopyropen, pymetrozine (PM), pyrifluquinazon (PFQ) and its N-deacetylated form (dPFQ).
Figure 2 depicts the effects of afidopyorpen and pymetrozine (PM) in American grasshoppers *(Schistocerca Americana)*
Figures 3 depict activation of Ca²⁺ mobilization by afidopyropen, pymetrozine (PM), and N-deacetylated form of pyrifluquinazon (dPFQ) in mammalian cells co-expressing Nan and Iav, but not in parental hamster cells (cells are cells prior to infection with the genes or proteins of interest), nor in cells expressing Nanchung or Inactive alone.
Figure 4 depicts graphs demonstrating that afidopyropen, pymetrozine (PM), and N-deacetylated form of pyrifluquinazon (dPFQ) evoke membrane depolarization response in mammalian cells co-expressing Nanchung and Inactive, but not in parental hamster cells, nor in cells expressing Nanchung or Inactive alone.
Figure 4a depicts graphs demonstrating that N-deacetylated form of pyrifluquinazon (dPFQ) evokes current in frog oocytes co-expressing Nanchung and Inactive, but not in oocytes expressing Nanchung or Inactive alone.
Figure 5 depicts dose response relationships of calcium responses evoked by afidopyropen, pymetrozine (PM), and N-deacetylated form of pyrifluquinazon (dPFQ) in cells co-expressing Nanchung and Inactive.
Figure 6 depicts afidopyropen not activating mammalian TRRV4 channel.
Figure 7 depicts specific binding of [³H]-afidopyropen to membranes co-expressing Nan and Iav.
Figure 8A depicts [³H]-afidopyropen saturation binding to Nan protein and increased binding affinity in the presence of Iav
Figure 8B depicts Scatchard transformation of the saturation binding identified in Figure 8A.
Figure 9A. Both pymetrozine (PM) and N-deacetylated form of pyrifluquinazon (dPFQ) compete with [³H]-afidopyropen for binding to Nan + Iav membranes.
Figure 9B. Both pymetrozine (PM) and N-deacetylated form of pyrifluquinazon (dPFQ) compete with [³H]-afidopyropen from binding to Nan only membranes.
Figures 10-36 correspond to SEQ ID NOS: 1-27, respectively, and provide the nucleotide sequences of a Nanchung (Nan) gene from different species having the accession number specified in the Figure.
Figures 37-63 correspond to SEQ ID NOS: 54-80, respectively, and provide the amino acid sequences of a Nanchung (Nan) protein having the accession numbers specified in the Figure.
Figures 64-89 correspond to SEQ ID NOS: 28-53, respectively, and provide the nucleotide sequences of an Inactive (Iav) gene from different species having the accession numbers specified in the Figure.
Figures 90-115 correspond to SEQ ID NOS: 81-106, respectively, and provide the amino acid sequence of an Inactive (Iav) protein from different species having the accession numbers specified in the Figure.
Figure 116 depicts a sequence alignment of the Nan proteins (SEQ ID NOS 59, 60, 63, 58, 57, 56, 54, 74, 73, 75, 66, 70, 110, 68, and 111, residues 1-448 and 560-900 of SEQ ID NO: 64, and SEQ ID NOS 76, 79, 62, 77, 72, 78, 71, 80, 65, and 61, all respectively, in order of appearance).
Figure 117 depicts sequence alignment of the Iav proteins (SEQ ID NOS 84 and 85, residues 1-916, 954-1102, and 1167-1216 of SEQ ID NO: 86, and SEQ ID NOS 83, 82, 81, 101, 104, 100, 99, 92, 98, 93, 90, 89, 94, 95, 88, 102, 112, 103, 105, 106, 96, 87, and 91, all respectively, in order of appearance).
Figure 118 depicts an alignment tree for Nan proteins.
Figure 119 depicts an alignment tree for Iav proteins.

### detailed description of the DRAWINGS AND THE PRESENTLY PREFERRED EMBODIMENTS

### I. General Considerations

The transient receptor potential (TRP) channels constitute a large and important class of channels involved in modulating cellular homeostasis. The present disclosure provides methods and compositions that identify or include one or more compounds that bind to and/or modulate at least one TRP family member, including, but not limited to a TRPV channel in an insect. Specifically, the present disclosure provides methods for identifying candidate compounds capable of directly binding to one or more insect Nanchung proteins, TRP and/or TRPV proteins, protein complexes and/or channels and/or modulating one or more insect TRP and/or TRPV channel. Modulating an insect TRP and/or TRPV channel may modulate calcium homeostasis, sodium homeostasis, intracellular calcium levels, membrane polarization (resting membrane potential), and/or cation levels in a cell. Compounds that can modulate one or more insect TRP and/or TRPV channels are useful in many aspects including, but not limited to, insect control. Knowing that a compound binds directly to an insect TRP and/or TRPV channel is critical for insect management planning and preventing the development of resistant insect populations.

The present disclosure further identifies afidopyropen, PM, and/or dPFQ as compounds having high affinity to Nanchung protein, TRP, and/or TRPV proteins, protein complexes, and/or channels, such that one or more of the compounds above may be used in the methods presented herein as a labeled compound for purposes of the competition binding assay set forth herein.

The present disclosure also provides compositions comprising the candidate compounds identified herein for use as insecticides for treating pests. The present disclosure further comprises classifying the candidate compounds identified herein by their mode of action.

Without being bound by theory, the modulation of an insect TRP and/or TRPV elicits a signaling pathway that brings forth sensory neuron modulation which may increase or decrease feeding behavior of an insect, and such modulation (e.g., activation) of an insect TRP and/or TRPV channel in an insect may lead to a decrease in feeding behavior of such insect leading to the insect's death by starvation. Thus, again without being bound by theory, it is believed that compounds that modulate (e.g., activate) an insect TRP and/or TRPV channel may be used as insecticides.

TRP channels have been classified into at least seven groups: TRPC (canonical), TRPV (vanilloid), TRPM (melastatin), TRPP (polycystins), TRPML (mucolipins), TRPA (ankyrin), and TRPN (no mechanosensor potential C), which is not found in humans.

The TRPC group can be divided into 4 subfamilies (TRPC1, TRPC4,5, TRPC3,6,7 and TRPC2) based on sequence homology and functional similarities.

Currently, the mammalian TRPV family has 6 members. TRPV5 and TRPV6 are more closely related to each other than to TRPV1, TRPV2, TRPV3, or TRPV4. TRPA1 is most closely related to TRPV3, and is more closely related to TRPV1 and TRPV2 than to TRPV5 and TRPV6. The insects have only two members of TRPV family: Nanchung (Nan), and Inactive (Iav), which are most closely related to mammalian TRPV5 and TRPV6. The TRPV is expressed in a great number of organisms, including, e.g., insects *(Drosophila, Tribolium, Pediculus, Culex,* and *Anopheles),* crustaceans *(Daphnia pulex)* nematodes *(Caenorhabditis elegans),* molluscs *(Aplysia californica, Lottia gigantea)* and mammals (humans, mice, rats, monkeys and chimpanzee.

Mollusks, nemotodes, Arthropod, and more particularly insect, TRPV channels are represented by Nanchung and/or Inactive proteins (or homologues of thereof). Nanchung and Inactive proteins form a complex (Nesterov et al. 2015), and are implicated in insect hearing (Matsuura et al., 2009). However, Nanchung may form a complex with other TRP proteins

The TRPM family has 8 members. Constituents include the following: the founding member TRPM1 (Melastatin or LTRPC1), TRPM3 (KIAA1616 or LTRPC3), TRPM7 (TRP-PLIK, ChaK(1), LTRPC7), TRPM6 (ChaK2), TRPM2 (TRPC7 or LTRPC2), TRPM8 (Trp-p8 or CMR1), TRPM5 (Mtr1 or LTRPC5), and TRPM4 (FLJ20041 or LTRPC4).

The sole mammalian member of the TRPA family is TRPA1, whereas insects contain at least four members of this family: TRPA1, Painless, Pyrexia, and Waterwitch.

The TRPML family consists of the mucolipins, which include TRPML1 (mucolipins 1), TRPML2 (mucolipins 2), and TRPML3 (mucolipin3).

The TRPP family consists of two groups of channels: those predicted to have six transmembrane domains and those that have 11. TRPP2 (PKD2), TRPP3 (PKD2L1), TRPP5 (PKD2L2) are all predicted to have six transmembrane domains. TRPP1 (PKD1, PC1), PKD-REJ and PKD-1L1 are all thought to have 11 transmembrane domains. Insects have only one member of TRPP family, which is absent in some species.

The inventors have discovered that afidopyropen binds directly to the Nanchung protein alone or in complex with other TRP and/or TRPV proteins. Furthermore, the inventors have developed methods of identifying additional compounds that bind directly to the same site as afidopyropen on the insect Nanchung protein.

TRPV protein complex or related insect TRP protein complex having a Nanchung protein incorporated in its complex. Even further the inventors have identified that afidopyropen is a compound that is useful for identifying candidate compounds capable of binding directly to the insect Nanchung protein, TRPV protein complex or related insect TRP protein complex having a Nanchung protein incorporated in its complex. Identification of such compounds may lead to the identification of suitable chemical compounds that can function as insecticides and be useful in a pest management program.

Even further the inventors discovered that some compounds have increased binding affinity when both the Nanchung protein and the Inactive protein were present in the protein complex. Accordingly, it may be beneficial to identify compounds that directly bind to one or more TRP and or TRPV protein complexes comprising one or more Nanchung proteins. Additionally, it may be beneficial to identify compounds that bind directly to one or more TRP protein complexes comprising one or more Nanchung proteins and one or more Inactive proteins. It may also be beneficial to identify compounds that bind directly to one or more Nanchung proteins, and/or TRP and/or TRPV protein complexes and or channels. It may be beneficial to use the identified compounds as insecticides. It may be further beneficial to identify compound that directly bind to one or more Nanchung proteins, and/or TRP and/or TRPV proteins, protein complexes and/or channels and that may be found to modulate the TRP and/or TRPV channels of pest species while not binding directly to and/or modulating the one or more Nanchung proteins, and/or TRP and/or TRPV proteins, protein complexes and/or channels of mammalians and/or other non-pest species.

### II. Definitions:

Unless defined otherwise, all technical and scientific terms used herein have the same meaning as commonly understood to one of ordinary skill in the art. Although any methods, devices, and materials similar or equivalent to those described herein can be used in the practice or testing of the presently disclosed subject matter, representative methods, devices, and materials are now described.

The terms "a," "an," and "the" refer to "one or more" when used in this application, including the claims. Thus, for example, reference to "a cell" (e.g., "a mammalian cell") includes a plurality of such cells (e.g., a plurality of mammalian cells in culture).

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

The terms "insect," "insect pest," or "plant insect pest" refer to any one of the numerous usually small arthropod animals of the class Insecta that are known to associate with plants and which, as a result of that association, causes a detrimental effect on the plant's health and vigor. The term plant as used herein encompasses whole plants and parts of plants such as roots, stems, leaves and seed, as well as cells and tissues within the plants or plant parts. The terms "insect," "insect pest," or "plant insect pest" are used interchangeably throughout the instant application.

The terms "pests" and/or "pest species" refer to any one of numerous usually small arthropod animals of the class Insecta that are known to associate with plants and which, as a result of that association, causes a detrimental effect on the plant's health and vigor. Additionally, the terms refer to include mollusk, nematodes, other arthropod classes that are known to associate with plants and which, as a result of that association, causes a detrimental effect on the plant's health and vigor. The terms further includes arthropods, gastropods and nematodes including but not limited to: insects from the order of **Lepidoptera,** for example *Achroia grisella, Acleris* spp. such as *A. fimbriana, A. gloverana, A. variana; Acrolepiopsis assectella, Acronicta major, Adoxophyes* spp. such as *A. cyrtosema, A. orana; Aedia leucomelas, Agrotis* spp. such as *A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia)* spp. such as *A. gemmatalis; Apamea spp* , *Aproaerema modicella, Archips* spp. such as *A. argyrospila, A. fuscocupreanus, A. rosana, A. xyloseanus; Argyresthia conjugella, Argyroploce* spp., *Argyrotaenia* spp. such as *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola* spp., *Cacoecia* spp. such as C. *murinana, C. podana; Cactoblastis cactorum, Cadra cautella, Calingo braziliensis, Caloptilis theivora, Capua reticulana, Carposina* spp. such as C. *niponensis, C. sasakii; Cephus spp, Chaetocnema aridula, Cheimatobia brumata, Chilo* spp. such as C. *Indicus, C. suppressalis, C. partellus; Choreutis pariana, Choristoneura* spp. such as C. *conflictana, C. fumiferana, C. longicellana, C. murinana, C. occidentalis, C. rosaceana; Chrysodeixis (=Pseudoplusia) spp. such as C. eriosoma, C. includens; Cirphis unipuncta, Clysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (=Carpocapsa)* spp. such as *C. pomonella, C. latiferreana; Dalaca noctuides, Datana integerrima, Dasychira pinicola, Dendrolimus* spp. such as *D. pini, D. spectabilis, D. sibiricus; Desmiafuneralis, Diaphania* spp. such as *D. nitidalis, D. hyalinata; Diatraea grandiosella, Diatraea saccharalis, Diphthera festiva, Earias* spp. such as *E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia* spp. such as *E. cautella, E. elutella, E. kuehniella; Epinotia aporema, Epiphyas postvittana, Erannis tiliaria, Erionota thrax, Etiella* spp., *Eulia* spp., *Eupoecilia ambiguella, Euproctis chrysorrhoea, Euxoa* spp., *Evetria bouliana, Faronta albilinea, Feltia* spp. such as *F*. *subterranean; Galleria mellonella, Gracillaria* spp., *Grapholita* spp. such as *G. funebrana, G. molesta, G. inopinata; Halysidota* spp., *Harrisina americana, Hedylepta* spp., *Helicoverpa* spp. such as *H. armigera (=Heliothis armigera), H. zea (=Heliothis zea); Heliothis* spp. such as *H. assulta, H. subflexa, H. virescens; Hellula* spp. such as *H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. such as *L. coffeella, L. scitella; Leuminivora lycinivorella, Lithocolletis blancardella, Lithophane antennata, Llattia octo (=Amyna axis), Lobesia botrana, Lophocampa* spp., *Loxagrotis albicosta, Loxostege* spp. such as *L. sticticalis, L. cereralis; Lymantria* spp. such as *L. dispar, L. monacha; Lyonetia clerkella, Lyonetia prunifoliella, Malacosoma* spp. such as *M. americanum, M. californicum, M. constrictum, M. neustria; Mamestra* spp. such as *M. brassicae, M. configurata; Mamstra brassicae, Manduca* spp. such as *M. quinquemaculata, M. sexta; Marasmia spp, Marmara* spp., *Maruca testulalis, Megalopyge lanata, Melanchra picta, Melanitis leda, Mocis* spp. such as *M. lapites, M. repanda; Mocis latipes, Monochroa fragariae, Mythimna separata, Nemapogon cloacella, Neoleucinodes elegantalis, Nepytia* spp., *Nymphula* spp., *Oiketicus* spp., *Omiodes indicata, Omphisa anastomosalis, Operophtera brumata, Orgyia pseudotsugata, Oria* spp., *Orthaga thyrisalis, Ostrinia* spp. such as *O. nubilalis; Oulema oryzae, Paleacrita vernata, Panolis flammea, Parnara* spp., *Papaipema nebris, Papilio cresphontes, Paramyelois transitella, Paranthrene regalis, Paysandisia archon, Pectinophora* spp. such as *P. gossypiella; Peridroma saucia, Perileucoptera* spp., such as *P. coffeella; Phalera bucephala, Phryganidia californica, Phthorimaea* spp. such as *P. operculella; Phyllocnistis citrella, Phyllonorycter* spp. such as *P. blancardella, P. crataegella, P. issikii, P. ringoniella; Pieris* spp. such as *P. brassicae, P. rapae, P. napi; Pilocrocis tripunctata, Plathypena scabra, Platynota* spp. such as *P. flavedana, P. idaeusalis, P. stultana; Platyptilia carduidactyla, Plebejus argus, Plodia interpunctella, Plusia spp, Plutella maculipennis, Plutella xylostella, Pontia protodica, Prays* spp., *Prodenia* spp., *Proxenus lepigone, Pseudaletia* spp. such as *P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius* spp., *Schreckensteinia festaliella, Scirpophaga* spp. such as S. *incertulas, S. innotata; Scotia segetum, Sesamia spp.* such as *S. inferens, Seudyra subflava, Sitotroga cerealella, Sparganothis pilleriana, Spilonota lechriaspis, S. ocellana, Spodoptera (=Lamphygma)* spp. such as *S. eridania, S. exigua, S. frugiperda, S. latisfascia, S. littoralis, S. litura, S. omithogalli; Stigmella* spp., *Stomopteryx subsecivella, Strymon bazochii, Sylepta derogata, Synanthedon* spp. such as S. exitiosa, *Tecia solanivora, Telehin licus. Thaumatopoea pityocampa, Thaumatotibia (=Cryptophlebia) leucotreta, Thaumetopoea pityocampa, Thecla* spp., *Theresimima ampelophaga, Thyrinteina* spp, *Tildenia inconspicuella, Tinea* spp. such as *T. cloacella, T. pellionella; Tineola bisselliella, Tortrix* spp. such as *T. viridana; Trichophaga tapetzella, Trichoplusia* spp. such as *T. ni; Tuta (=Scrobipalpula) absoluta, Udea* spp. such as *U. rubigalis, U. rubigalis; Virachola* spp, *Yponomeuta padella, and Zeiraphera canadensis;*insects from the order of **Coleoptera,** for example *Acalymma vittatum, Acanthoscehdes obtectus, Adoretus* spp., *Agelastica alni, Agrilus* spp. such as *A. anxius, A. planipennis, A. sinuatus; Agriotes* spp. such as *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. such as *A. glabripennis; Anthonomus* spp. such as *A. eugenii, A. grandis, A. pomorum; Anthrenus* spp., *Aphthona euphoridae, Apion* spp., *Apogonia* spp., *Athous haemorrhoidalis, Atomaria* spp. such as *A. linearis; Attagenus* spp., *Aulacophora femoralis, Blastophagus piniperda, Blitophaga undata, Bruchidius obtectus, Bruchus* spp. such as *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. such as C. *assimilis, C. napi; Chaetocnema tibialis, Cleonus mendicus, Conoderus* spp. such as C. *vespertinus; Conotrachelus nenuphar, Cosmopolites* spp., *Costelytra zealandica, Crioceris asparagi, Cryptolestes ferrugineus, Cryptorhynchus lapathi, Ctenicera* spp. such as C. *destructor; Curculio* spp., *Cylindrocopturus* spp., *Cyclocephala spp, Dactylispa balyi, Dectes texanus, Dermestes* spp., *Diabrotica* spp. such as *D. undecimpunctata, D. speciosa, D. longicornis, D. semipunctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. such as *E. varivestis, E. vigintioctomaculata; Epitrix* spp. such as *E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera* spp. such as *H. brunneipennis, H. postica; Hypomeces squamosus, Hypothenemus* spp., *Ips typographus, Lachnosterna consanguinea, Lasioderma serricorne, Latheticus oryzae, Lathridius* spp., *Lema* spp. such as *L. bilineata, L. melanopus; Leptinotarsa* spp. such as *L. decemlineata; Leptispa pygmaea, Limonius californicus, Lissorhoptrus oryzophilus, Lixus* spp., *Luperodes* spp., *Lyctus* spp. such as *L. bruneus; Liogenys fuscus* , *Macrodactylus* spp. such as *M. subspinosus; Maladera matrida, Megaplatypus mutates, Megascelis* spp., *Melanotus communis, Meligethes* spp. such as *M. aeneus; Melolontha* spp. such as *M. hippocastani, M. melolontha; Metamasius hemipterus, Microtheca spp, Migdolus* spp. such as *M. fryanus, Monochamus* spp. such as *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp. such as *P. brassicae, P. cochleariae; Phoracantha recurva, Phyllobius pyri, Phyllopertha horticola, Phyllophaga* spp. such as *P. helleri; Phyllotreta* spp. such as P. *chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. such as *R. billineatus, R. ferrugineus, R. palmarum, R. phoenicis, R. vulneratus; Saperda candida, Scolytus schevyrewi, Scyphophorus acupunctatus, Sitona lineatus, Sitophilus* spp. such as S. *granaria, S. oryzae, S. zeamais; Sphenophorus* spp. such as *S. levis; Stegobium paniceum, Sternechus* spp. such as *S. subsignatus; Strophomorphus ctenotus, Symphyletes* spp., *Tanymecus* spp., *Tenebrio molitor, Tenebrioides mauretanicus, Tribolium* spp. such as *T. castaneum; Trogoderma* spp., *Tychius* spp., *Xylotrechus* spp. such as *X pyrrhoderus; and, Zabrus* spp. *such as Z. tenebrioides;* insects from the order of Diptera for example *Aedes* spp. such as *A. aegypti, A. albopictus, A. vexans; Anastrepha ludens, Anopheles* spp. such as *A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia* spp. such as C. *bezziana, C. hominivorax, C. macellaria; Chrysops atlanticus, Chrysops discalis, Chrysops silacea, Cochliomyia* spp. such as C. *hominivorax; Contarinia* spp. such as C. *sorghicola; Cordylobia anthropophaga, Culex* spp. such as C. *nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. such as *D. antique, D. coarctata, D. platura, D. radicum; Dermatobia hominis, Drosophila* spp. such as *D. suzukii, Fannia* spp. such as *F*. *canicularis; Gastraphilus* spp. such as G. *intestinalis; Geomyza tipunctata, Glossina* spp. such as *G. fuscipes, G. morsitans, G. palpalis, G. tachinoides; Haematobia irritans, Haplodiplosis equestris, Hippelates* spp., Hylemyia spp. such as *H. platura; Hypoderma* spp. such *as H. lineata; Hyppobosca* spp., *Hydrellia philippina, Leptoconops torrens, Liriomyza* spp. such as *L. sativae, L. trifolii; Lucilia* spp. such as *L. caprina, L. cuprina, L. sericata; Lycoria pectoralis, Mansonia titillanus, Mayetiola* spp. such as *M. destructor; Musca* spp. such as M. *autumnalis, M. domestica; Muscina stabulans, Oestrus* spp. such as *O*. *ovis; Opomyza florum, Oscinella* spp. such as *O. frit; Orseolia oryzae, Pegomya hysocyami, Phlebotomus argentipes, Phorbia* spp. such as *P. antiqua, P. brassicae, P. coarctata; Phytomyza gymnostoma, Prosimulium mixtum, Psila rosae, Psorophora columbiae, Psorophora discolor, Rhagoletis* spp. such as *R. cerasi, R. cingulate, R. indifferens, R. mendax, R. pomonella; Rivellia quadrifasciata, Sarcophaga* spp. such as S. *haemorrhoidalis; Simulium vittatum, Sitodiplosis mosellana, Stomoxys* spp. such as *S. calcitrans; Tabanus* spp. such as *T. atratus, T. bovinus, T. lineola, T. similis; Tannia spp., Thecodiplosis japonensis, Tipula oleracea, Tipula paludosa,* and *Wohlfahrtia* spp.; insects from the order of **Thysanoptera** for example, *Baliothrips biformis, Dichromothrips corbetti, Dichromothrips* ssp., *Echinothrips americanus, Enneothripsflavens, Frankliniella* spp. such as *F*. *fusca, F*. *occidentalis, F*. *tritici; Heliothrips* spp., *Hercinothrips femoralis, Kakothrips* spp., *Microcephalothrips abdominalis, Neohydatothrips samayunkur, Pezothrips kellyanus, Rhipiphorothrips cruentatus, Scirtothrips* spp. such as *S. citri, S. dorsalis, S. perseae; Stenchaetothrips spp, Taeniothrips cardamoni, Taeniothrips inconsequens, Thrips* spp. such as *T. imagines, T. hawaiiensis, T. oryzae, T. palmi, T. parvispinus, T. tabaci;* insects from the order of **Hemiptera** for example, *Acizzia jamatonica, Acrosternum* spp. such as *A. hilare;* Acyrthosipon spp. such as *A. onobrychis, A. pisum; Adelges laricis, Adelges tsugae, Adelphocoris* spp., such as *A. rapidus, A. superbus; Aeneolamia* spp., *Agonoscena* spp., *Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. such as *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli (Paratrioza cockerelli), Bemisia* spp. such as *B. argentifolii, B. tabaci (Aleurodes tabaci); Blissus* spp. such as *B. leucopterus; Brachycaudus* spp. such as *B. cardui, B. helichrysi, B. persicae, B. prunicola; Brachycolus* spp., *Brachycorynella asparagi, Brevicoryne brassicae, Cacopsylla* spp. such as *C. fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimex* spp. such as C. *hemipterus, C. lectularius; Coccomytilus halli, Coccus* spp. such as C. *hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes* spp. such as *D. citrifolii; Dalbulus maidis, Diaphorina* spp. such as *D. citri; Diaspis* spp. such as *D. bromeliae; Dichelops furcatus, Diconocoris hewetti, Doralis* spp., *Dreyfusia nordmannianae, Dreyfusia piceae, Drosicha* spp., *Dysaphis* spp. such as *D. plantaginea, D. pyri, D. radicola; Dysaulacorthum pseudosolani, Dysdercus* spp. such as *D. cingulatus, D. intermedius; Dysmicoccus* spp., *Edessa spp, Geocoris spp, Empoasca* spp. such as *E. fabae, E. solana; Epidiaspis leperii, Eriosoma* spp. such as *E. lanigerum, E. pyricola; Erythroneura* spp., *Eurygaster* spp. such as *E. integriceps; Euscelis bilobatus, Euschistus* spp. such as *E. heros, E. impictiventris, E. servus; Fiorinia theae, Geococcus coffeae, Glycaspis brimblecombei, Halyomorpha* spp. such as *H. halys; Heliopeltis* spp., *Homalodisca vitripennis (=H. coagulata), Horcias nobilellus, Hyalopterus pruni, Hyperomyzus lactucae, Icerya* spp. such as *I. purchase; Idiocerus* spp., *Idioscopus* spp., *Laodelphax striatellus, Lecanium* spp., *Lecanoideus floccissimus, Lepidosaphes* spp. such as *L. ulmi; Leptocorisa* spp., *Leptoglossus phyllopus, Lipaphis erysimi, Lygus* spp. such *as L. hesperus, L. lineolaris, L. pratensis; Maconellicoccus hirsutus, Marchalina hellenica, Macropes excavatus, Macrosiphum* spp. such as *M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia spp, Myzus* spp. such as M. *ascalonicus, M. cerasi, M. nicotianae, M. persicae, M. varians; Nasonovia ribis-nigri, Neotoxoptera formosana, Neomegalotomus spp, Nephotettix* spp. such as *N. malayanus, N. nigropictus, N. parvus, N. virescens; Nezara* spp. such as *N. viridula; Nilaparvata lugens, Nysius huttoni, Oebalus* spp. such as *O. pugnax; Oncometopia* spp., *Orthezia praelonga, Oxycaraenus hyalinipennis, Parabemisia myricae, Parlatoria* spp., *Parthenolecanium* spp. such as *P. corni, P. persicae; Pemphigus* spp. such as *P. bursarius, P. populivenae; Peregrinus maidis, Perkinsiella saccharicida, Phenacoccus* spp. such *as P. aceris, P. gossypii; Phloeomyzuspasserinii, Phorodon humuli, Phylloxera* spp. such as *P. devastatrix, Piesma quadrata, Piezodorus* spp. such as *P. guildinii; Pinnaspis aspidistrae, Planococcus* spp. such as *P. citri, P. ficus; Prosapia bicincta, Protopulvinaria pyriformis, Psallus seriatus, Pseudacysta persea, Pseudaulacaspis pentagona, Pseudococcus* spp. such as *P. comstocki; Psylla* spp. such as *P. mali; Pteromalus* spp., *Pulvinaria amygdali, Pyrilla* spp., *Quadraspidiotus* spp., such as *Q. perniciosus; Quesada gigas, Rastrococcus* spp., *Reduvius senilis, Rhizoecus americanus, Rhodnius* spp., *Rhopalomyzus ascalonicus, Rhopalosiphum* spp. such as *R. pseudobrassicas, R. insertum, R. maidis, R. padi; Sagatodes* spp., *Sahlbergella singularis, Saissetia* spp., *Sappaphis mala, Sappaphis mali, Scaptocoris spp, Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina); Stephanitis nashi, Stephanitis pyrioides, Stephanitis takeyai, Tenalaphara malayensis, Tetraleurodes perseae, Therioaphis maculate, Thyanta* spp. such as *T. accerra, T. perditor; Tibraca* spp., *Tomaspis* spp., *Toxoptera* spp. such as *T. aurantii; Trialeurodes* spp. such as *T. abutilonea, T. ricini, T. vaporariorum; Triatoma* spp., *Trioza* spp., *Typhlocyba* spp., *Unaspis* spp. such as *U. citri, U. yanonensis; and Viteus vitifolii;* insects from the order **Hymenoptera** for example *Acanthomyops interjectus, Athalia rosae, Atta spp such as A. capiguara, A. cephalotes, A. cephalotes, A. laevigata, A. robusta, A. sexdens, A. texana, Bombus spp., Brachymyrmex* spp., *Camponotus spp such as C. floridanus, C. pennsylvanicus, C. modoc; Cardiocondyla nuda, Chalibion sp, Crematogaster spp., Dasymutilla occidentalis, Diprion spp., Dolichovespula maculata, Dorymyrmex* spp, *Dryocosmus kuriphilus, Formica spp, Hoplocampa* spp. such *as H. minuta, H. testudinea; Iridomyrmex humilis,* Lasius spp. such as *L. niger, Linepithema humile, Liometopum spp, Leptocybe invasa, Monomorium spp such as M. pharaonis, Monomorium, Nylandria fulva, Pachycondyla chinensis, Paratrechina longicornis, Paravespula spp such as P. germanica, P. pennsylvanica, P. vulgaris; Pheidole spp such as P. megacephala; Pogonomyrmex spp such as P. barbatus, P. californicus, Polistes rubiginosa, Prenolepis impairs, Pseudomyrmex gracilis, Schelipron spp, Sirex cyaneus, Solenopsis spp such as S. geminata, S.invicta, S. molesta, S. richteri, S. xyloni, Sphecius speciosus, Sphex spp, Tapinoma spp such as T. melanocephalum, T. sessile; Tetramorium spp such as T. caespitum, T. bicarinatum,* Vespa spp. such as *V. crabro; Vespula spp such as V. squamosal; Wasmannia auropunctata, Xylocopa* sp.; insects from the order **Orthoptera** for example *Acheta domesticus, Calliptamus italicus, Chortoicetes terminifera, Ceuthophilus spp, Diastrammena asynamora, Dociostaurus maroccanus, Gryllotalpa spp such as G. africana, G. gryllotalpa; Gryllus spp, Hieroglyphus daganensis, Kraussaria angulifera, Locusta* spp. such as *L. migratoria, L. pardalina; Melanoplus spp such as M. bivittatus, M. femurrubrum, M. mexicanus, M. sanguinipes, M. spretus; Nomadacris septemfasciata, Oedaleus senegalensis, Scapteriscus spp, Schistocerca spp such as S. americana, S. gregaria, Stemopelmatus spp, Tachycines asynamorus, and Zonozerus variegatus;* pests from the Class **Arachnida** for example **Acari,** e.g. of the families Argasidae, Ixodidae and Sarcoptidae, such as Amblyomma spp. (e.g. *A. americanum, A. variegatum, A. maculatum),* Argas spp. such as *A. persicu), Boophilus* spp. such as *B. annulatus, B. decoloratus, B. microplus, Dermacentor spp such as D.silvarum, D. andersoni, D. variabilis, Hyalomma* spp. such as *H. truncatum, Ixodes* spp. such as *I. ricinus, I. rubicundus, I. scapularis, I. holocyclus, I. pacificus, Rhipicephalus sanguineus, Ornithodorus* spp. such as *O*. *moubata, O*. *hermsi, O*. *turicata), Ornithonyssus bacoti, Otobius megnini, Dermanyssus gallinae, Psoroptes* spp such as *P. ovis, Rhipicephalus* spp such as *R. sanguineus, R. appendiculatus, Rhipicephalus evertsi), Rhizoglyphus spp; Sarcoptes* spp. such asS. *Scabiei;* and Family **Eriophyidae** including *Aceria spp such as A. sheldoni, A. anthocoptes, Acallitus* spp; *Aculops* spp. such as *A. lycopersici, A. pelekassi; Aculus* spp such as *A. schlechtendali; Colomerus vitis, Epitrimerus pyri, Phyllocoptruta oleivora; Eriophytes ribis* and *Eriophyes* spp such as *Eriophyes sheldoni;* Family **Tarsonemidae** including *Hemitarsonemus spp., Phytonemuspallidus and Polyphagotarsonemus latus, Stenotarsonemus spp. Steneotarsonemus spinki;* Family **Tenuipalpidae** including Brevipalpus spp. such as *B. phoenicis;* Family **Tetranychidae** including *Eotetranychus spp., Eutetranychus spp., Oligonychus spp., Petrobia latens, Tetranychus spp such as T. cinnabarinus, T. evansi, T. kanzawai, T, pacificus, T. phaseulus, T. telarius* and *T. urticae; Bryobia praetiosa; Panonychus* spp. such as *P. ulmi, P. citri;, Metatetranychus spp.* and *Oligonychus* spp. such as *O*. *pratensis, O*. *perseae), Vasates lycopersici; Raoiella indica, Family* **Carpoglyphidae** including *Carpoglyphus spp; Penthaleidae* spp such as *Halotydeus destructor* ; Family **Demodicidae** with species such a *Demodex* spp; Family **Trombicidea** including *Trombicula spp:;* Family **Macronyssidae** including *Ornothonyssus* spp; Family **Pyemotidae** including *Pyemotes tritici; Tyrophagus putrescentiae;* Family Acaridae including *Acarus siro;* Family **Araneida** including *Latrodectus mactans, Tegenaria agrestis, Chiracanthium sp, Lycosa sp Achaearanea tepidariorum* and *Loxosceles reclusa;* pests from the Phylum **Nematoda,** for example, plant parasitic nematodes such as root-knot nematodes, *Meloidogyne* spp. such as *M. hapla, M. incognita, M. javanica;* cyst-forming nematodes, *Globodera* spp. such as *G. rostochiensis; Heterodera* spp. such as *H. avenae, H. glycines, H. schachtii, H. trifolii;* Seed gall nematodes, *Anguina* spp.; Stem and foliar nematodes, *Aphelenchoides* spp. such as *A. besseyi;* Sting nematodes, *Belonolaimus* spp. such as *B. longicaudatus;* Pine nematodes, *Bursaphelenchus* spp. such as *B. lignicolus, B. xylophilus;* Ring nematodes, *Criconema* spp.; *Criconemella* spp. such as *C. xenoplax* and *C. ornata;* and, *Criconemoides* spp. such as *Criconemoides informis; Mesocriconema* spp.; Stem and bulb nematodes, *Ditylenchus* spp. such as *D. destructor, D. dipsaci;* Awl nematodes, *Dolichodorus* spp.; Spiral nematodes, *Heliocotylenchus multicinctus;* Sheath and sheathoid nematodes, *Hemicycliophora* spp. and *Hemicriconemoides* spp.; *Hirshmanniella* spp.; Lance nematodes, *Hoploaimus* spp.; False rootknot nematodes, *Nacobbus* spp.; Needle nematodes, *Longidorus* spp. such as *L. elongatus;* Lesion nematodes, *Pratylenchus* spp. such as *P. brachyurus, P. neglectus, P. penetrans, P. curvitatus, P. goodeyi;* Burrowing nematodes, *Radopholus* spp. such as *R. similis; Rhadopholus* spp.; *Rhodopholus* spp.; Reniform nematodes, *Rotylenchus* spp. such as *R. robustus, R. reniformis; Scutellonema* spp.; Stubby-root nematode, *Trichodorus* spp. such as *T. obtusus, T. primitivus; Paratrichodorus* spp. such as *P. minor;* Stunt nematodes, *Tylenchorhynchus* spp. such as *T. claytoni, T. dubius;* Citrus nematodes, *Tylenchulus* spp. such as *T. semipenetrans;* Dagger nematodes, *Xiphinema* spp.; and other plant parasitic nematode species; insects from the order **Isoptera** for example *Calotermes flavicollis, Coptotermes spp such as C. formosanus, C. gestroi, C. acinaciformis; Cornitermes cumulans, Cryptotermes spp such as C. brevis, C. cavifrons; Globitermes sulfureus, Heterotermes spp such as H. aureus, H. longiceps, H. tenuis; Leucotermesflavipes, Odontotermes spp., Incisitermes spp such as I. minor, I. Snyder; Marginitermes hubbardi, Mastotermes spp such as M. darwiniensis Neocapritermes spp such as N. opacus, N. parvus; Neotermes spp, Procornitermes spp, Zootermopsis spp such as Z. angusticollis, Z. nevadensis, Reticulitermes* spp. such as *R. hesperus, R. tibialis, R. speratus, R. flavipes, R. grassei, R. lucifugus, R. santonensis, R. virginicus; Termes natalensis;* insects from the order **Blattaria** for example *Blatta spp such as B. orientalis, B. lateralis; Blattella spp such as B. asahinae, B. germanica; Leucophaea maderae, Panchlora nivea, Periplaneta spp such as P. americana, P. australasiae, P. brunnea, P. fuligginosa, P. japonica; Supella longipalpa,* **Parcoblatta pennsylvanica, Eurycotis floridana,** *Pycnoscelus surinamensis;* insects from the order **Siphonoptera** for example *Cediopsylla simples, Ceratophyllus spp., Ctenocephalides spp such as C. felis, C. canis, Xenopsylla cheopis, Pulex irritans, Trichodectes canis, Tunga penetrans,* and *Nosopsyllus fasciatus;* insects from the order **Thysanura** for example *Lepisma saccharina, Ctenolepisma urbana,* and *Thermobia domestica;* pests from the class **Chilopoda** for example *Geophilus spp.,* Scutigera spp. such as *Scutigera coleoptrata;* pests from the class **Diplopoda** for example *Blaniulus guttulatus, Julus spp, Narceus spp.;* pests from the class **Symphyla** for example *Scutigerella immaculata;* insects from the order **Dermaptera,** for example *Forficula auricularia;* insects from the order **Collembola,** for example *Onychiurus* spp. such as *Onychiurus armatus;* pests from the order **Isopoda** for example, *Armadillidium vulgare, Oniscus asellus, Porcellio scaber;* insects from the order **Phthiraptera,** for example *Damalinia spp.,* Pediculus spp. such as *Pediculus humanus capitis, Pediculus humanus corporis, Pediculus humanus humanus; Pthirus pubis,* Haematopinus spp. such as *Haematopinus eurysternus, Haematopinus suis;* Linognathus spp. such as *Linognathus vituli; Bovicola bovis, Menopon gallinae, Menacanthus stramineus* and *Solenopotes capillatus, Trichodectes spp..* Examples of further pest species which may be controlled by the compounds and/or compositions identified herein include: from the Phylum **Mollusca,** class **Bivalvia,** for example, *Dreissena spp.;* class **Gastropoda,** for example, *Arion spp., Biomphalaria spp., Bulinus spp., Deroceras spp., Galba spp., Lymnaea spp., Oncomelania spp., Pomacea canaliclata, Succinea spp.;* from the class of the ***helminths,*** for example, *Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp.,* Haemonchus spp. such as *Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*

The terms "non-pest" and/or "non-target" species refers to all other animals that would not be classified as "pests" and/or "pest species" as defined above.
The term "nucleic acid" refers to deoxyribonucleotides or ribonucleotides and polymers thereof in either single- or double-stranded form, composed of monomers (nucleotides) containing a sugar, phosphate and a base that is either a purine or pyrimidine. Unless specifically limited, the term encompasses nucleic acids containing known analogs of natural nucleotides, conservatively modified variants thereof, complementary sequences, and degenerate codon substitutions that have similar binding properties as the reference nucleic acid and are metabolized in a manner similar to naturally occurring nucleotides.
The terms "nucleic acid" or "nucleic acid molecule" can also be used interchangeably with gene, open reading frame (ORF), cDNA, and mRNA encoded by a gene, nucleic acid molecule, nucleic acid fragment, nucleic acid segment, or polynucleotide.
The term "gene" is used broadly to refer to any segment of nucleic acid associated with a biological function. Thus, genes include coding sequences and/or the regulatory sequences required for their expression. For example, "gene" refers to a nucleic acid fragment that expresses mRNA, functional RNA, or specific protein, including regulatory sequences. "Genes" also include non-expressed DNA segments that, for example, form recognition sequences for other proteins. "Genes" can be obtained from a variety of sources, including cloning from a source of interest or synthesizing from known or predicted sequence information, and may include sequences designed to have desired parameters.
The term "gene delivery" or "gene transfer" refers to methods or systems for reliably introducing foreign DNA into target cells and include injection, transduction, transfection, transformation and electroporation. Such methods can result in transient or long term expression of genes.
The term "transduction" refers to the delivery of a DNA molecule to a recipient cell either *in vivo* or *in vitro,* via a replication-defective viral vector, such as, e.g., adenoviral vector.
The term "transfection" is used to refer to the uptake of foreign DNA by a mammalian cell. A cell has been "transfected" when exogenous DNA has been introduced across the cell plasma membrane. Transfection can be used to introduce one or more exogenous DNA moieties, such as a plasmid vector and other nucleic acid molecules, into suitable cells. The term refers to both stable and transient uptake of the genetic material.
The term "transformation" and/or "transfection" refers to a process for introducing heterologous DNA into a cell. Transformed cells are understood to encompass not only the end product of a transformation/transfection process, but also transgenic progeny thereof.
The term "injection" refers to a process for introducing heterologous DNA or RNA into a cell using a syringe and a needle.
The term "vector" refers to any genetic element, such as a plasmid, phage, transposon, cosmid, chromosome, virus, virion, etc., which is capable of replication when associated with the proper control elements, such as a helper virus, and which can transfer gene sequences between cells. Thus, the term includes cloning and expression vehicles, as well as replication-defective viral vectors.

The terms "expression vector" and "expression cassette" refer to a nucleic acid molecule capable of directing expression of a particular nucleotide sequence in an appropriate host cell, typically comprising a promoter operatively linked to the nucleotide sequence of interest which is operatively linked to termination signals. It also typically comprises sequences required for proper translation of the nucleotide sequence. The coding region usually encodes a polypeptide(s) of interest but can also encode a functional RNA of interest, for example antisense RNA or a non-translated RNA, in the sense or antisense direction. The expression cassette comprising the nucleotide sequence of interest can be chimeric, meaning that at least one of its components is heterologous with respect to at least one of its other components. The expression cassette can also be one that is naturally occurring but has been obtained in a recombinant form useful for heterologous expression. Typically, however, the expression cassette is heterologous with respect to the host; i.e., the particular DNA sequence of the expression cassette does not occur naturally in the host cell and was introduced into the host cell or an ancestor of the host cell by a transformation event. The expression of the nucleotide sequence in the expression cassette can be under the control of a constitutive promoter or of an inducible promoter that initiates transcription only when the host cell is exposed to some particular external stimulus. In the case of a multicellular organism such as a plant, the promoter can also be specific to a particular tissue, organ, or stage of development.
The term "adenovirus" refers to a vector derived from an adenovirus serotype. Adenoviruses are double-stranded DNA viruses capable of infecting broad range of mammalian cells, both dividing and non-dividing. The adenovirus DNA is linear of approximately 36,000 bp. The most widely used adenoviral vectors for gene delivery are the replication-deficient vectors, in which the E1 and E3 regions of the adenoviral genome are deleted. E1-deleted viruses are propagated in complementing cells, such as HEK-293, which provide E1-encoded proteins in trans. E1/E3-deleted adenoviruses can accommodate up to 7.5 kb of foreign DNA. The classical method of incorporating foreign DNA into adenovirus genome involves homologous recombination in mammalian cells. More efficient method, as described in the present examples involves homologous recombination in *Escherichia coli* between a large plasmid containing most of the adenovirus genome and a small shuttle plasmid. The shuttle plasmid contains the expression cassette flanked by sequences homologous to the region to be targeted in the viral genome. The recombinant Ad genome is then linearized by restriction digestion and used to transfect E1-complementing mammalian cells to produce viral particles (Douglas JT., Methods Mol Biol. 2004; V246:3-14) To produce adenoviruses containing toxic gene, special adenoviral vectors have been developed where expression of foreign gene is suppressed during virus production. One of these systems, named, SpeAd Ad-teto system is offered by, for example, Welgen, Inc.
The term "tetracycline-controlled transactivator" (tTA) refers to a fusion protein used to control nucleic acid expression in the presence or absence of doxyclycline, tetracycline and related compounds. The tTA includes a Tet repressor (TetR) fused to any domain capable of activating transcription. The tTA may include a TetR fused to a C-terminal portion of adenovirus.
The term "operably linked" refers to two nucleic acid sequences that are related physically or functionally. For example, a promoter or regulatory DNA sequence is said to be "operably linked to" a DNA sequence that encodes an RNA or a polypeptide if the two sequences are situated such that the regulatory DNA sequence will affect the expression level of the coding or structural DNA sequence. A promoter is also said to be operably linked to a nucleotide sequence if when an RNA polymerase binds to the promoter under conditions sufficient for transcription, the nucleotide sequence is transcribed.
The term "isolated," when applied to a nucleic acid or polypeptide, denotes that the nucleic acid or polypeptide is essentially free of other cellular components with which it is associated in the natural state. It can be in a homogeneous state although it can be in either a dry or aqueous solution. Homogeneity and whether a molecule is isolated can be determined using analytical chemistry techniques such as polyacrylamide gel electrophoresis or high performance liquid chromatography. A polypeptide that is the predominant species present in a preparation is substantially isolated. The term "isolated" denotes that a nucleic acid or polypeptide gives rise to essentially one band in an electrophoretic gel. Particularly, it means that the nucleic acid or polypeptide is in some embodiments at least about 50% pure, in some embodiments at least about 85% pure, and in some embodiments at least about 99% pure.
The terms "label" and "labeled" refer to the attachment of a moiety, capable of detection by spectroscopic, radiologic, or other methods, to a molecule. Thus, the terms "label" or "labeled" refer to incorporation or attachment, optionally covalently or non-covalently, of a detectable marker into a molecule, such as a biomolecule. Various methods of labeling biomolecules are known in the art and can be used. Examples of labels for biomolecules include, but are not limited to, the following: radioisotopes, fluorescent labels, heavy atoms, enzymatic labels or reporter genes, chemiluminescent groups, and biotinyl groups. In some embodiments, labels are attached by spacer arms of various lengths to reduce potential steric hindrance. Fluorescent probe that can be utilized include, but are not limited to fluorescein isothiocyanate; fluorescein dichlorotriazine and fluorinated analogs of fluorescein; naphthofluorescein carboxylic acid and its succinimidyl ester; carboxyrhodamine 6G; pyridyloxazole derivatives; Cy2, 3, 3.5, 5, 5.5, and 7; phycoerythrin; phycoerythrin-Cy conjugates; fluorescent species of succinimidyl esters, carboxylic acids, isothiocyanates, sulfonyl chlorides, and dansyl chlorides, including propionic acid succinimidyl esters, and pentanoic acid succinimidyl esters; succinimidyl esters of carboxytetramethylrhodamine; rhodamine Red-X succinimidyl ester; Texas Red sulfonyl chloride; Texas Red-X succinimidyl ester; Texas Red-X sodium tetrafluorophenol ester; Red-X; Texas Red dyes; tetramethylrhodamine; lissamine rhodamine B; tetramethylrhodamine; tetramethylrhodamine isothiocyanate; naphthofluoresceins; coumarin derivatives (e.g., hydroxycoumarin, aminocoumarin, and methoxycoumarin); pyrenes; pyridyloxazole derivatives; dapoxyl dyes; Cascade Blue and Yellow dyes; benzofuran isothiocyanates; sodium tetrafluorophenols; 4,4-difluoro-4-bora-3a,4a-diaza-s-indacene; Alexa fluors (e.g., 350, 430, 488, 532, 546, 555, 568, 594, 633, 647, 660, 680, 700, and 750); isotope of hydrogen (e.g., tritium isotope, 3H), isotope of carbon (e.g., 14C) green fluorescent protein; yellow fluorescent protein or red fluorescent protein. The peak excitation and emission wavelengths will vary for these compounds and selection of a particular fluorescent probe for a particular application can be made in part based on excitation and/or emission wavelengths.
As used herein, the terms "candidate compound" or "test compound" refer to the collection of compounds that are to be screened for their ability to bind to Nanchung proteins, Nanchung and TRP protein complexes, Nanchung and Iav protein complexes, and/or modulate insect TRPV channels and/or insect TRP channels expressing a Nanchung protein. The candidate compounds may encompass numerous classes of chemical molecules, e.g., small organic or inorganic molecules, polysaccharides, biological macromolecules, e.g., peptides, proteins, peptide analogs and derivatives, peptidomimetics, nucleic acids, nucleic acid analogs and derivatives, an extract made from biological materials such as bacteria, plants, fungi, or animal cells or tissues, naturally occurring or synthetic compositions, or combinations thereof. Generally, the candidate compounds can have a molecular weight of about 50 to 500,000, but is not limited thereto.
As used herein, the term "small molecule" refers to a compound that is "natural product-like," however, the term "small molecule" is not limited to a "natural product-like" compound. Rather, a small molecule is typically characterized in that it contains several carbon-carbon bonds, and has a molecular weight more than about 50, but less than about 5000 Daltons (5 kD). Preferably the small molecule has a molecular weight of less than 3 kD, still more preferably less than 2 kD, and most preferably less than 1 kD. In some cases it is preferred that a small molecule have a molecular mass equal to or less than 700 Daltons.
In some embodiments, the candidate compound may be a synthetic molecule. The term "synthetic molecule" refers to a molecule that does not occur in nature.

In certain embodiments, the candidate compound may be a naturally-occurring molecule. Such a naturally-occurring molecule may be used in a purified or unpurified form, i.e., as obtained from the biological source. The term "naturally occurring" refers to an entity (e.g., a cell, biomolecule, etc.) that is found in nature as distinct from being artificially produced by man. For example, a polypeptide or nucleotide sequence that is present in an organism in its natural state, which has not been intentionally modified or isolated by man in the laboratory, is naturally occurring. As such, a polypeptide or nucleotide sequence is considered "non-naturally occurring" if it is encoded by or present within a recombinant molecule, even if the amino acid or nucleic acid sequence is identical to an amino acid or nucleic acid sequence found in nature.
Depending upon the particular embodiment being practiced, the candidate compounds may be provided free in solution, or may be attached to a carrier, or a solid support, e.g., beads. A number of suitable solid supports may be employed for immobilization of the candidate compounds. Examples of suitable solid supports include agarose, cellulose, dextran (commercially available as, i.e., Sephadex, Sepharose) carboxymethyl cellulose, polystyrene, polyethylene glycol (PEG), filter paper, nitrocellulose, ion exchange resins, plastic films, polyaminemethylvinylether maleic acid copolymer, glass beads, amino acid copolymer, ethylene-maleic acid copolymer, nylon, silk, etc. Additionally, for the methods described herein, the candidate compounds may be screened individually, or in groups. Group screening is particularly useful where hit rates for effective candidate compounds are expected to be low such that one would not expect more than one positive result for a given group.
There are millions of possible candidate compounds. Methods for developing small molecule, polymeric and genome based libraries are known and described, for example, in Ding, et al. J. Am. Chem. Soc. 124: 1594-1596 (2002) and Lynn, et al., J. Am. Chem. Soc. 123: 8155-8156 (2001). A number of small molecule libraries are known in the art and commercially available. Commercially available compound libraries can be obtained from, e.g., ArQule, Pharmacopia, graffinity, Panvera, Vitas-M Lab, Biomol International and Oxford. These libraries can be screened using the screening methods described herein. Chemical compound libraries such as those from of 10,000 compounds and 86,000 compounds from NIH Roadmap, Molecular Libraries Screening Centers Network (MLSCN) can also be used. A comprehensive list of compound libraries can be found at www.broad.harvard.edu/chembio/platform/screening/compound_libraries/index.htm. A chemical library or compound library is a collection of stored chemicals usually used ultimately in high-throughput screening or industrial manufacture. The chemical library can consist in simple terms of a series of stored chemicals. Each chemical has associated information stored in some kind of database with information such as the chemical structure, purity, quantity, and physiochemical characteristics of the compound.

The term "modulate" refers to an increase, decrease, or other alteration of any, or all, chemical and/or biological activities and/or properties of a biomolecule, such as the TRPV (e.g., insect TRPV) channel of the present disclosure. The term "modulation" as used herein thus refers to both upregulation (i.e., activation or stimulation) and downregulation (i.e., inhibition or suppression) of such an activity or property. As would be understood by one of ordinary skill in the art, a modulation of a chemical and/or biological activity and/or property of a biomolecule, such as TRPV channel, can result from an increase or decrease in the expression of the biomolecule in a cell. Accordingly, the terms "modulate" and grammatical variants thereof are intended to encompass both direct modulation (e.g., inhibition of a chemical and/or biological activity and/or property of a polypeptide via binding of an inhibitor to the polypeptide) as well as indirect modulation (e.g., upregulation or downregulation of expression of a protein, such as a TRPV channel or inhibition or stimulation of a biomolecule that acts together with a biomolecule of the presently disclosed subject matter to produce a biological effect).

The terms "polypeptide," "protein," and "peptide," which are used interchangeably herein, refer to a polymer of the 20 protein amino acids, or amino acid analogs, regardless of its size or function. Although "protein" is often used in reference to relatively large polypeptides, and "peptide" is often used in reference to small polypeptides, usage of these terms in the art overlaps and varies. The term "polypeptide" as used herein refers to peptides, polypeptides, and proteins, unless otherwise noted. The terms "protein," "polypeptide," and "peptide" are used interchangeably herein when referring to a gene product. Thus, exemplary polypeptides include gene products, naturally occurring proteins, homologs, orthologs, paralogs, fragments and other equivalents, variants, and analogs of the foregoing.

The term TRP channel refers to a channel and/or a protein complex formed from one or more of the following: Nanchung proteins, TRP proteins and/or Inactive protein. It is to be understood that the TRPV channel and/or TRPV protein complex is a type of TRP channel and/or TRP protein complex. Nanchung proteins and Inactive proteins are a type of TRPV proteins which are a type of TRP proteins. TRP proteins may be found in insect cellular membranes; mammalian cellular membranes; derived from a free cell system; produced by a cell culture; and/or produced by cells, including but not limited to a frog egg, insect cell, and/or mammalian cell, that have been genetically modified (including either or both DNA and/or RNA) to include the genes coding for one or more of the Nanchung proteins, TRP proteins and/or the Inactive proteins. It is to be understood that the TRP proteins disclosed herein may be mammalian TRP proteins and/or arthropod TRP proteins and/or Nematode TRP proteins and/or Mollusk TRP proteins. It is to be further understood that Nanchung and Inactive proteins may be found in arthropods, including, but not limited to insects, as well as Mollusks and/or Nematodes. It is to be understood that TRP and/or TRPV channels may be formed from one or more TRP and/or TRPV protein complexes; from two or more TRP and/or TRPV protein complexes; from three or more TRP and/or TRPV protein complexes, and/or from four or more TRP and/or TRPV protein complexes.

The term "binding affinity" refers to the ability of compounds to form a bond with a receptor. For purposes of this invention, an equilibrium constant is used to determine the binding affinity of one or more compounds with the one or more Nanchung proteins, TRP channels and or TRPV channels. A dissociation constant (Kd) is a specific type of equilibrium constant measured in a labeled compound saturation binding assay. It is the molar concentration of labeled compound that binds to half the receptor sites at equilibrium. The lower the dissociation constant the higher the binding affinity of the candidate compound for the Nanchung protein, a Nanchung TRP protein complex, and or a Nanchung Inactive protein complex. An inhibition constant (Ki) is a specific type of equilibrium constant that is measured using competition binding of an unlabeled compound with a labeled compound. It is the molar concentration of the competing unlabeled compound that would occupy 50% of the receptors at equilibrium, if no labeled compound was present. It can be calculated using the Cheng and Prusoff equation: Ki=IC₅₀/(1+[L]/K_{d}) where IC₅₀ is the molar concentration of competing unlabeled compound which reduces the specific binding of a labeled compound by 50%. In this equation [L] is the molar concentration of a labeled compound in the assay; K_{d} is equilibrium dissociation constant for a labeled compound. In addition to saturation binding assay, equilibrium dissociation constant, Kd can be measured in multiple ways, including but not limited to:
1. Using the association and dissociation rates constants where Kd=Koff/Kon
2. Using a fluorescence polarization assay
3. Using surface plasmon resonance
4. Using isothermal calorimetry
5. Using Schild analysis

Surface plasmon resonance and isothermal calorimetry do not require labeled compound to measure K_{d}. In addition, for the competition assay, Kᵢ and/or K_{d} (for competition assay) may be calculated using one or more of the following: Cheng and Prusoff equation, fluorescence polarization, and/or Schild analysis. It is to be understood that the K_{d} and Kᵢ values calculated for a compound should be similar, but due to experimental error the values may have less than a two-fold difference between the two values or less than a three-fold difference between the two-values depending upon the extent of the experimental error.

The term "species specific binding" for purposes of this application refers to preferential binding of a compound to insect TRP and/or TRPV proteins versus vertebrate TRP and/or TRPV proteins. The term "high affinity" and/or "high binding affinity" refers to a compound whose equilibrium dissociation constant (K_{d}) and/or equilibrium inhibition constant (Kᵢ) is calculated as being less than or equal to 3x10⁻⁷ M, less than or equal to 3x10⁻⁸ M, and/or less than or equal to 30x10⁻¹² M. The lower the K_{d} or Kᵢ the higher the binding affinity for the compound.

The term "mode of action" refers to the classification of a compound and/or a composition according to the Insecticide Resistance Action Committee (IRAC) into the appropriate group based on the compound and/or composition (a) exhibiting the same function as other insecticides in that group and (b) binding directly to the same site on particular proteins, protein complexes and/or protein channels as other compounds and/or compositions in that group.

### III. Screening Methods

The inventors have discovered that insect TRP and/or TRPV channels may be used to identify compounds that block insect feeding behavior and thus be useful as insecticides. As such, in one embodiment, the present disclosure relates to a method for determining whether or not a candidate compound is a modulator of an insect TRP and/or TRPV channel. It is to be understood herein that insect TRP channels comprise one or more Nanchung proteins in a complex with one or more TRP proteins such that they form an ion channel. It is to be further understood that insect TRPV channels comprise one or more Nanchung proteins in a complex with one or more TRPV proteins, including but not limited to Inactive proteins, such that they form an ion channel. The inventors have further discovered methods of identifying compounds that bind directly to one or more Nanchung proteins,. Furthermore the inventors have determined that a compound, afidopyropen, binds directly to one or more Nanchung proteins,. It is to be understood that Arthropod, Mollusk and/or Nematode TRP and/or TRPV channels comprise one or more Nanchung proteins in a complex with one or more TRP and/or TRPV proteins such that they form an ion channel.

The methods identified herein includes providing one or more Nanchung proteins,; contacting the first cell with a candidate compound; and assaying for modulation of the insect TRP and/or TRPV channel, wherein the modulation of the insect TRP and/or TRPV channel identifies the candidate compound as a modulator of the insect TRP and/or TRPV channel. Modulation of the TRP and/or TRPV channel may be assayed using conventional *in vitro* and *in vivo* methods well known to the skilled artisan. For example, in certain embodiments, the assaying step may include (1) detecting calcium ion mobilization in the first cell in response to the candidate compound, (2) detecting a membrane potential in the first cell in response to the candidate compound, (3) comparing calcium ion mobilization in the first cell in the absence of the candidate compound with calcium ion mobilization in the first cell in the presence of the candidate compound. For example, calcium response may be measured by assessment of the uptake of Ca²⁺ or by using fluorescent dyes, such as Fluo-4 or Fura-2 or fluorescent proteins, such as Cameleon , (4) comparing a membrane potential in the first cell in the absence of the candidate compound with a membrane potential in the first cell in the presence of the candidate compound, (5) comparing calcium ion mobilization in the first cell in the presence of the candidate compound with calcium ion mobilization reference level indicative of no modulation of the TPRV channel, and/or (6) comparing a membrane potential in the first cell in the presence of the candidate compound with a membrane potential reference level indicative of no modulation of the TPRV channel, (7) comparing ion currents in the first cell in the absence of the candidate compound with a ion currents in the first cell in the presence of the candidate compound using standard electrophysiology methods, such as voltage clamp or patch clamp. It is to be understood that this same assay could be run on TRP and/or TRPV channels from vertebrate, arthropod, mollusks, nematodes or any other organisms. Such a test would allow one to determine whether or not the candidate compound worked as a modulator is those systems and thus, help one determine the potential environmental impact of using the candidate compound as an insecticide.
Preferably, the candidate compound may modulate the calcium ion mobilization or the membrane potential, or ion currents in the first cell by at least 10% relative to the reference level; more preferably at least 20% relative to the reference level; more preferably at least 30% relative to the reference level; more preferably at least 40% relative to the reference level; more preferably at least 50% relative to the reference level; more preferably at least 60% relative to the reference level; more preferably at least 70% relative to the reference level; more preferably at least 75% relative to the reference level; more preferably at least 80% relative to the reference level; more preferably at least 85% relative to the reference level; more preferably at least 90% relative to the reference level; and more preferably at least 95% relative to the reference level.
In addition, activation of TRPV channel may be assayed using a variety of other conventional assays that measure changes in ion fluxes including, but not limited to, patch clamp techniques, measurement of whole cell currents, radiolabeled ion flux assays, and fluorescence assays using voltage sensitive dyes (Zheng at al., 2004).
Modulation of an insect TRPV channel may also be assessed using a variety of other *in vitro* and *in vivo* assays to determine functional, chemical, and physical effects, e.g., measuring the binding of the insect TRPV to other molecules, including peptides, small organic molecules, and lipids; measuring insect TRPV protein and/or mRNA levels, or measuring other aspects of insect TRPV polypeptides, e.g., transcription levels, or physiological changes.

The inventors have further determined that it is beneficial to identify compounds that bind directly to one or more Nanchung proteins and thus be useful as insecticides. As such, in one embodiment, the present disclosure relates to a method for determining whether or not a candidate compound directly binds in a species specific manner to one or more Nanchung proteins. There are two variations of binding assays, a saturation assay and a competition assay. The saturation assay may include at least one of the following steps: (1) contacting the one or more Nanchung proteins, with a compound, wherein the compound may be labeled or unlabeled; (2) measuring the concentration of the labeled compound and/or unlabeled compound which is bound to the one or more Nanchung proteins,; (3) calculating the equilibrium disassociation constant (K_{d}) for the one or more labeled compounds and/or unlabeled compound (4) wherein the K_{d} may be determined using a (a) maximum binding (Bₘₐₓ) and (b) the concentration of the compound needed to reach 50% of maximum binding.

The competition assay may include one or more of the following steps: (1) contacting the one or more Nanchung proteins, with a compound, wherein the compound may be labeled or unlabeled; (2) measuring the concentration of the compound which is bound to the one or more Nanchung proteins, ; (3) calculating the equilibrium disassociation constant (K_{d}) for the one or more compounds; (4) wherein the K_{d} may be determined using one or more of the following methods: (I)(a) maximum binding (Bₘₐₓ) and (b) the concentration of labeled compound needed to reach 50% of maximum binding, (II) fluorescence polarization assay, (III) surface plasmon resonance, (IV) isothermal calorimetry, (V) Schild analysis; (5) contacting the one or more Nanchung proteins, with a labeled compound having high affinity to the one or more Nanchung proteins,; (6) contacting the one or more one or more Nanchung proteins, with a candidate compound in the presence of the labeled compound of step 1; (7) increasing the amount of the candidate compound available to contact the one or more Nanchung proteins, until binding of the labeled compound is near 0%; (8) calculating the equilibrium inhibition constant (Kᵢ) and/or the equilibrium dissociation constant (K_{d}) for competition assay using one or more of the following methods: (I) (a) determining amount of bound labeled compound in the absence of the candidate compound and (b) determining concentration of the candidate compound that is required to reduce the amount of bound labeled compound by 50%, (II) fluorescent polarization assay, and/or (III) Schild analysis; (9) comparing the inhibition constant of the candidate compounds to other candidate compounds; (10) comparing the inhibition constant of the candidate compounds to the disassociation constant of the labeled compound.

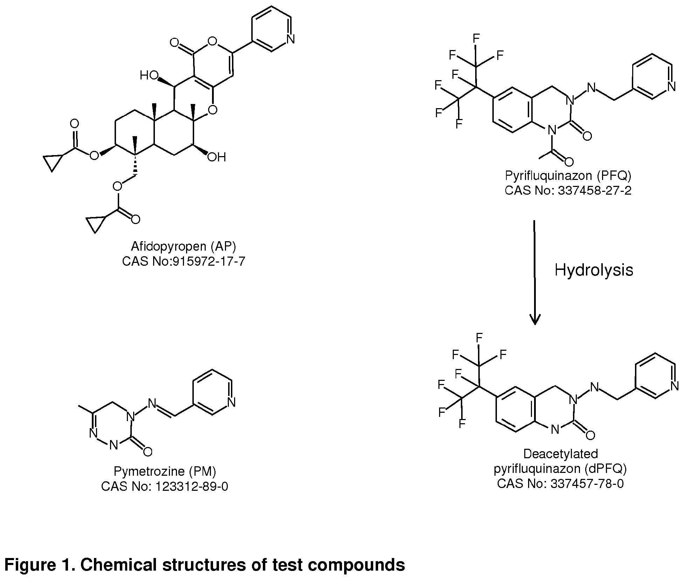

Binding to a one or more Nanchung proteins, may be assessed using a variety of *in vitro* and *in vivo* assays to determine whether the candidate compound binds directly to the one or more Nanchung proteins,. Identification of molecular targets of insecticides is essential for assessing their spectra, health and environmental impacts, as well as for insect resistance management. Pymetrozine (PM) and pyrifluquinazon (PFQ) are two structurally-related commercial insecticides (chemical structures are shown on Figure 1) which act by disrupting coordination and feeding of plant-sucking insects such as aphids and whiteflies (Casida and Durkin, 2013; Maienfisch, 2012). Pyrifluquinazon can spontaneously de-acetylate in aqueous solutions. The de-acetylated form of pyrifluquinazon (dPFQ) was shown to be significantly more potent than the parent compound, suggesting that pyrifluquinazon is a pro-drug, which is activated through de-acetylation (Nesterov et al., 2015). dPFQ was, therefore, used in the present disclosure however it is to be understood that pyrifluquinazon may be used as well.

Afidopyropen is a new insecticide compound with a previously unknown mode of action. Although afidopyropen and Pymetrozine have significantly different chemical structures (Figure 1), they have similar spectrum of insects they are toxic to, and produce similar behavioral symptoms, including feeding inhibition in aphids (Leichter et al., 2013). However, whether afidopyropen and Pymetrozine bind to the same location and thus would be classified as having the same mode of action was unknown.

TRP and/or TRPV (Transient Receptor Potential) proteins form hetero- and homotetrameric cation channels in various cell types (Venkatachalam and Montell, 2007). Pymetrozine and pyrifluquinazon disturbed *Drosophila* coordination and hearing by overstimulating, and ultimately silencing TRPV channels in chordotonal organs (Nesterov et al., 2015), which are serially arranged stretch receptors (Kavlie and Albert, 2013) which sense relative position of body parts in insects (Kamikouchi et al., 2009; Sun et al., 2009). While Pymetrozine and pyrifluquinazon were found to bind to the Nanchung protein alone, the modulation effect of the Pymetrozine and pyrifluquinazon required simultaneous presence of two TRPV proteins, Nanchung (Nan) and Inactive (Iav), which co-occur in chordotonal neurons and form functional ion channels. The current disclosure shows that afidopyropen, Pymetrozine and de-acetylated pyrifluquinazon. modulate TRP channels directly, by directly binding to the same site on these protein channels. Furthermore, the current disclosure identifies methods of determining whether or not a candidate compound binds directly to the same site on these proteins, protein complexes and/or protein channels. Determining the binding location and affinities for candidate compounds assists with the classification of a compounds mode of action and allows for better insect management and control that may result in decreased risk of developing resistant insect populations.

The current disclosure also found that there was increased binding affinity of Pymetrozine, deacetylated pyrifluquinazon, and afidopyropen in the presence of both the Nanchung protein and the Inactive protein. The current disclosure also found that afidopyropen exhibited modulation activity only in the presence of both Nanchung and Inactive proteins, protein complexes and/or channels. The current disclosure also found that afidopyropen exhibited modulation activity in the presence of both Nanchung and TRP proteins, protein complexes and/or channels. Furthermore the current disclosure identified afidopyropen as having a high binding affinity to the Nanchung protein, a Nanchung-TRP protein complex, a Nanchung-TRP protein channel, a Nanchung-TRPV protein complex, Nanchung-TRPV protein channel, a Nanchung-Inactive protein complex, and a Nanchung-Inactive protein channel. Accordingly, the current disclosure identifies a labeled compound having a high binding affinity to one or more Nanchung proteins, , such as afidopyropen, may be used to calculate the binding affinity of a candidate compound using the competition assay as described herein.

As noted previously, the candidate compound may be a small organic molecule, small inorganic molecule, polysaccharides, peptides, proteins, nucleic acids, an extract made from biological materials such as bacteria, plants, fungi, animal cells, animal tissues, and any combination thereof. Generally, a candidate compound can be tested at any concentration that can modulate the activity of a TRP and/or TRPV channel, and/or exhibit binding to TRP and/or TRPV proteins. In some embodiments, the candidate compound may be tested at a concentration in the range of about 0.01 nM to about 1000 mM. In certain other embodiments, the compound may be tested in the range of about 100 µM to about 1000 µM. In certain further embodiments, the candidate compound may be tested at greater than 0.05 mM, 0.1 mM, 0.2 mM, 0.3 mM, 0.4 mM, 0.5 mM, 0.6 mM, 0.7 mM, 0.8 mM, 0.9 mM, 1 mM, 1.1 mM, 1.2 mM, 1.3 mM, 1.4 mM, 1.5 mM, 1.6 mM, 1.7 mM, 1.8 mM, 1.0 mM, or 2 mM. Other ranges and concentrations of the candidate compound may also be suitable.

In some embodiments, the candidate compound may be tested at two or more different concentrations. Preferably the highest concentration tested is at least 2×, at least 3×, at least 4×, at least 5×, at least 6×, at least 7×, at least 8×, at least 9×, at least 10×, at least 15×, at least 20×, at least 25×, at least 50×, at least 75×, at least 100×, at least 200×, at least 250× higher than the lowest concentration employed. For example, the candidate compound may be tested at 0.1 mM, 0.5 mM, and 1 mM, where the highest concentration is at least 10X the concentration of the lowest concentration employed.
Generally, the one or more Nanchung proteins, s may be contacted with a candidate compound for any suitable length of time before measuring the binding affinity and/or activity of the insect TRP and/or TRPV channel and/or proteins. For example, the insect TRPV channel may be contacted with a candidate compound for at least 5 seconds, at least 10 seconds, at least 15 seconds, at least 30 seconds, at least 45 seconds, at least 1 minute, at least 2 minutes, at least 3 minutes, at least 4 minutes, at least 5 minutes, at least 10 minutes, at least 15 minutes, at least 30 minutes, at least 60 minutes, at least 2 hours, at least 3 hours or more before activity and/or binding of the insect TRP and/or TRPV is measured. In some embodiments, activity may be measured at the instant when the insect TRPV is contacted with a candidate compound.
In some embodiments, activity of insect TRPV channel may be measured over a period of time as well as binding of the candidate compound to the one or more Nanchung proteins, TRP and/or TRPV protein complexes and/or channels. For example, activity may be measured for a period of at least 5 seconds, at least 10 seconds, at least 15 seconds, at least 30 seconds, at least 45 seconds, at least 1 minute, at least 2 minutes, at least 3 minutes, at least 4 minutes, at least 5 minutes, at least 10 minutes, at least 15 minutes, at least 30 minutes, at least 60 minutes, at least 2 hours, at least 3 hours, at least 4 hours or more. The measurement period can start before the one or more Nanchung proteins, is contacted with a candidate compound, at the instant when the one or more Nanchung proteins, is first contacted with a candidate compound or start after a period of time after the one or more Nanchung proteinsls is first contacted with a candidate compound. The one or more Nanchung proteins, may be continuously contacted with the candidate compound while activity and/or binding is measured.
In some embodiments, the candidate compound has an effective concentration EC50 of less than or equal to 500 nM, less than or equal to 250 nM, less than or equal to 100 nM, less than or equal to 50 nM, less than or equal to 10 nM, less than or equal to 1 nM, less than or equal to 0.1 nM, less than or equal to 0.01 nM, or less than or equal to 0.001 nM for activating and/or binding an insect-specific TRPV channel.
In some embodiments, the candidate compound has an IC50 of less than or equal to 500 nM, less than or equal to 250 nM, less than or equal to 100 nM, less than or equal to 50 nM, less than or equal to 10 nM, less than or equal to 1 nM, less than or equal to 0.1 nM, less than or equal to 0.01 nM, or less than or equal to 0.001 nM for inhibiting and/or binding an insect-specific TRPV channel.

Preferably, the candidate compound is a modulator that inhibits an insect feeding behavior.
In certain embodiments the candidate compound binds directly to the Nanchung protein
In certain other embodiments the candidate compound binds directly to a complex consisting of the one or more Nanchung proteins and one or more TRP proteins.
Preferably the candidate compound binds directly to the one or more Nanchung and Inactive protein complexes.
By the term "preferential modulation" is meant that activity or other property of insect-specific TRPV and/or TRP channel is modulated by at least 5%, at least 10%, at least 20%, at least 30%, at least 40%, at least 50%, at least 60%, at least 70%, at least 80%, at least 90%, at least 95%, at least 100%, or more relative to a reference level or the mammalian-specific TRPV and/or TRP channel.
By the term "preferential binding" is meant that the equilibrium binding constant (K_{d} or Kᵢ) of the insect-specific TRPV and/or TRP channel is decreased by at least 5%, at least 10%, at least 20%, at least 30%, at least 40%, at least 50%, at least 60%, at least 70%, at least 80%, at least 90%, at least 95%, at least 100%, or more relative to a reference level or the mammalian-specific TRPV and/or TRP channel.
In certain other embodiments, non-selective modulators and/or binders of the TRPV and/or TRP channel may be identified. The term "non-selective" in connection with a modulator and/or binder of the TRPV and/or TRP proteins, protein complexes and/or channels means that a compound can modulate and/or bind directly to one or more TRPV and/or TRP proteins, protein complexes and/or channels in different organisms, e.g., a compound may modulate both, arthropod, such as insect, and mammalian TRPV and/or TRP protein complexes and/or channels.
In certain other embodiments, the candidate compound is an insect-specific TRP channel modulator and/or binder and does not modulate the activity of and/or bind to the mammalian-specific TRP, e.g., the tested compound has no significant effect on the mammalian-specific TRPV channel.
In certain embodiments, the one or more Nanchung protein, is in a biological cell The term "biological cell" or "cell" as used herein has its commonly understood meaning. Inside a cell, the Nanchung protein, may be expressed from an endogenous gene in the cell or from at least one vector that is transfected into the cell. It is to be understood that either the cultured cell, or a cell obtained directly from animal, such as frog oocyte, or whole tissue may be used as a source of the Nanchung,. Preferably, the TRPV channel proteins, Nanchung and Inactive proteins are co-expressed in the cell. The Nanchung and Inactive proteins may be co-expressed at varying rations, preferably, the ratio of the Nanchung to Inactive proteins co-expressed in the first cell is about 3:1 to about 1:3, more preferably about 1:1. It is to be understood that the Nanchung protein may be combined with other TRP channel proteins and that binding to the Nanchung protein in this scenario will have similar effects for purposes of the candidate compound acting as an insecticide. The Nanchung and TRP proteins may be co-expressed at varying rations, preferably, the ratio of the Nanchung to TRP proteins co-expressed in the first cell is about 3:1 to about 1:3, more preferably about 1:1.
In certain embodiments, the one or more Nanchung proteins TRPV and/or TRP protein complexes and/or channels are formed in a cell-free system. The term "cell-free system" as used herein means an in vitro system used to study biological reactions that happen within cells while reducing the complex interactions found in a whole cell. Subcellular fractions can be isolated by ultracentrifugation to provide molecular machinery that can be used in reactions in the absence of many of the other cellular components. Cell-free biosystems can be prepared by mixing one or more of the following: ribosomes; and/or DNA and/or RNA encoding for one or more proteins of interest; membranes; as well as a one or more enzymes and/or coenzymes. Cell-free biosystems have several advantages suitable in industrial applications._In vitro biosystems can be easily controlled. Cell-free systems provide an alternative choice for fast protein synthesis and very high product yields are usually accomplished without the formation of by-products or synthesis of cell mass.

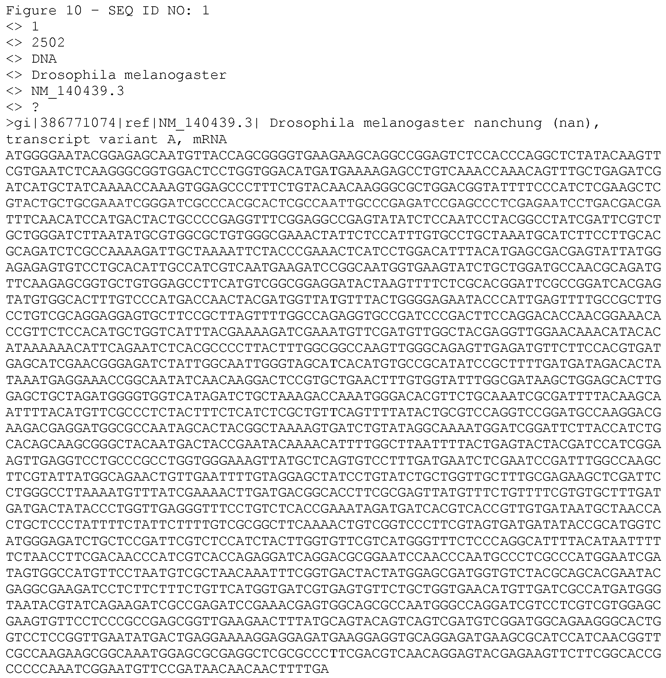

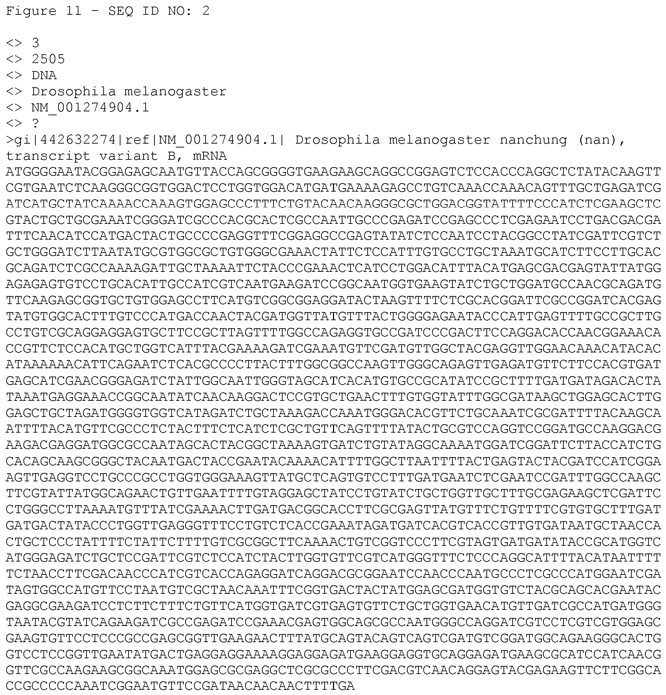

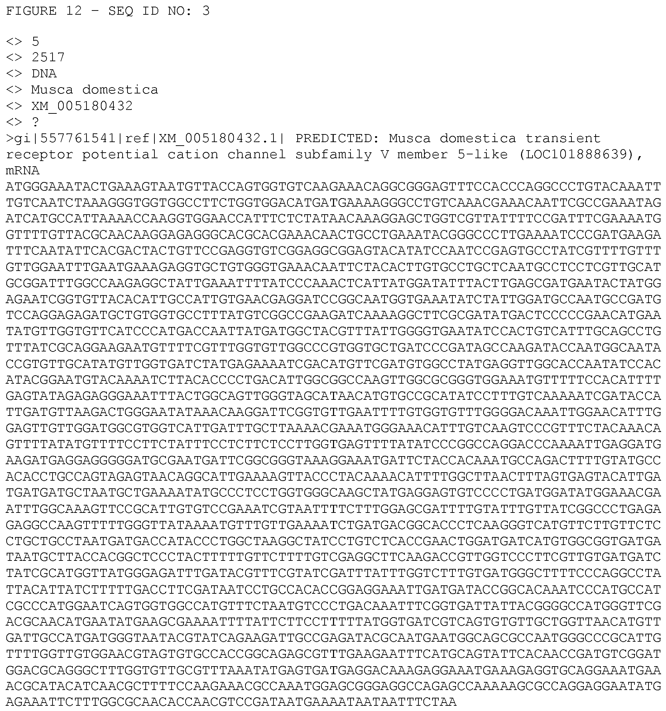

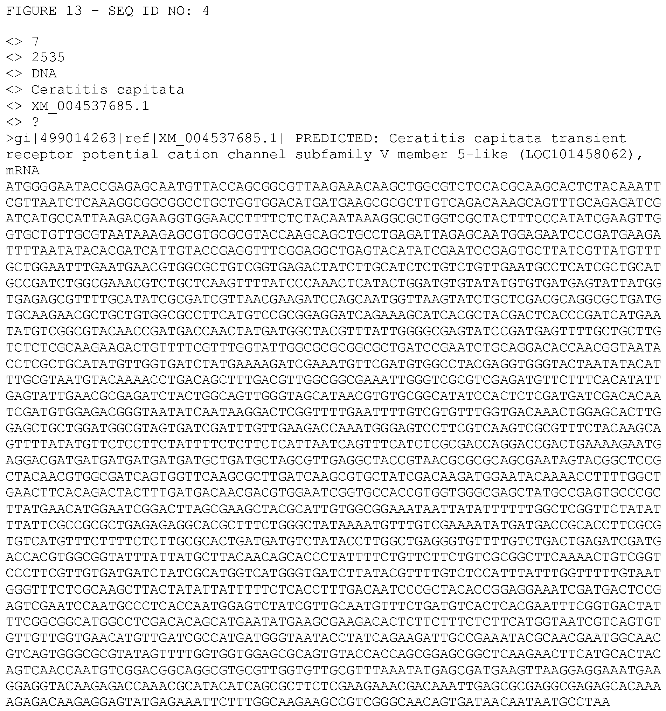

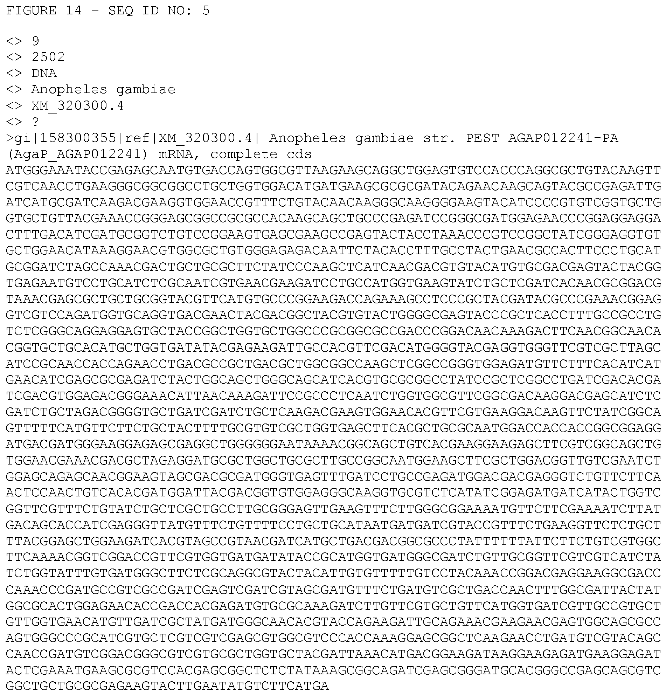

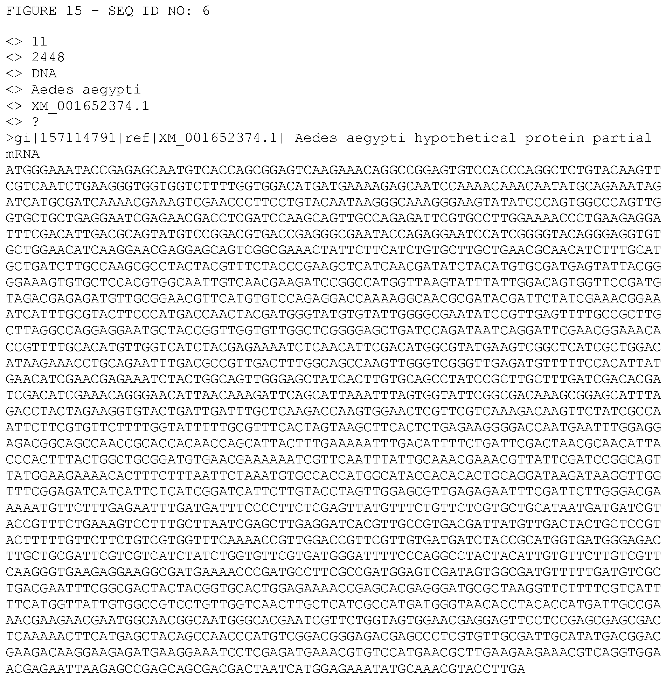

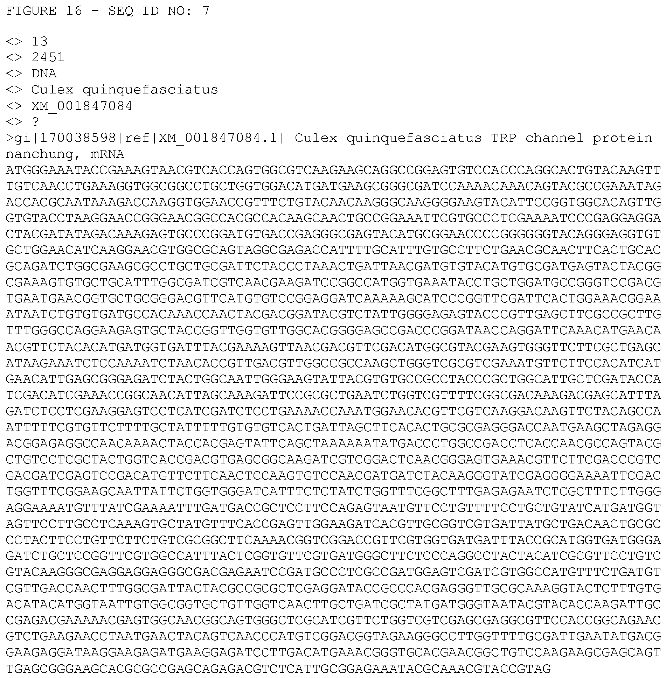

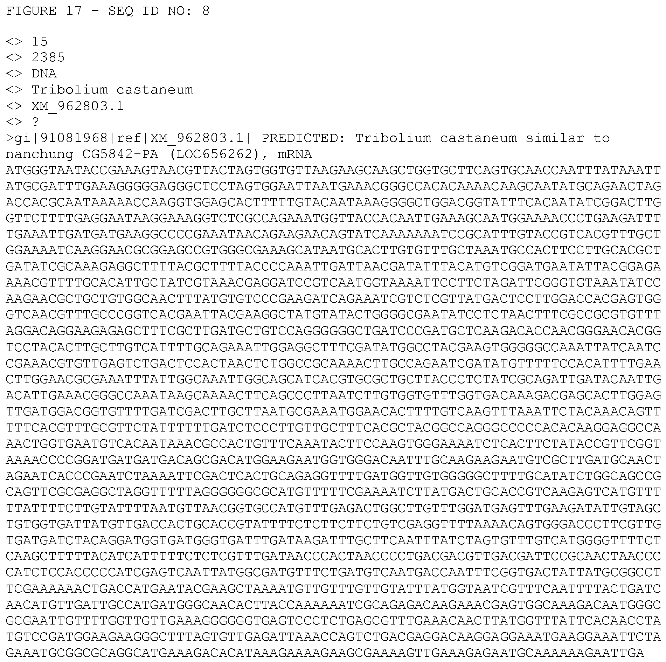

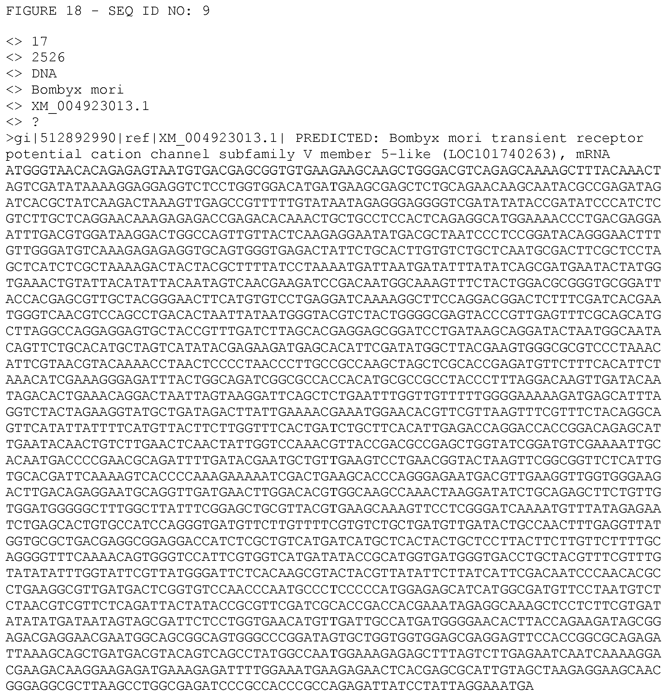

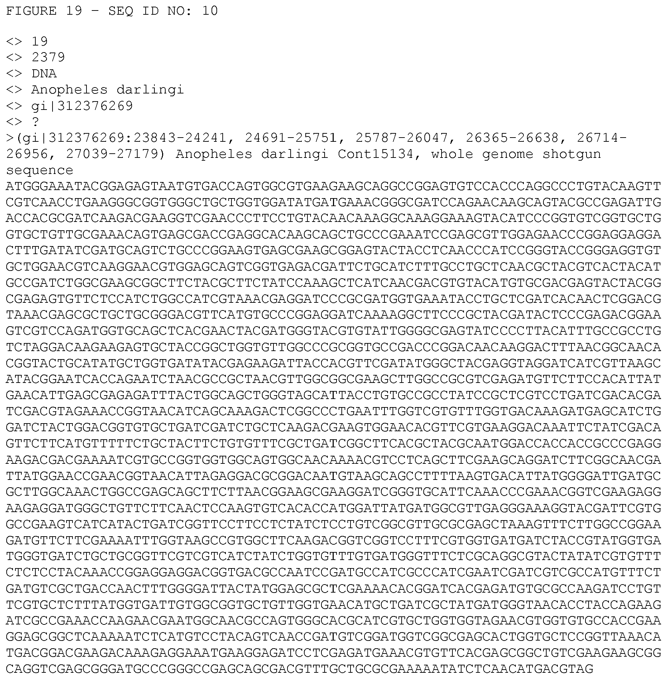

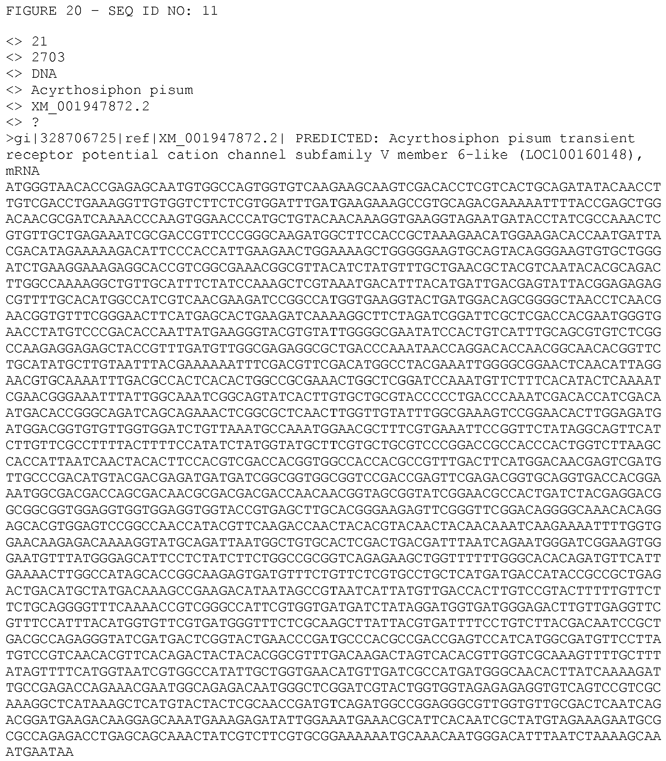

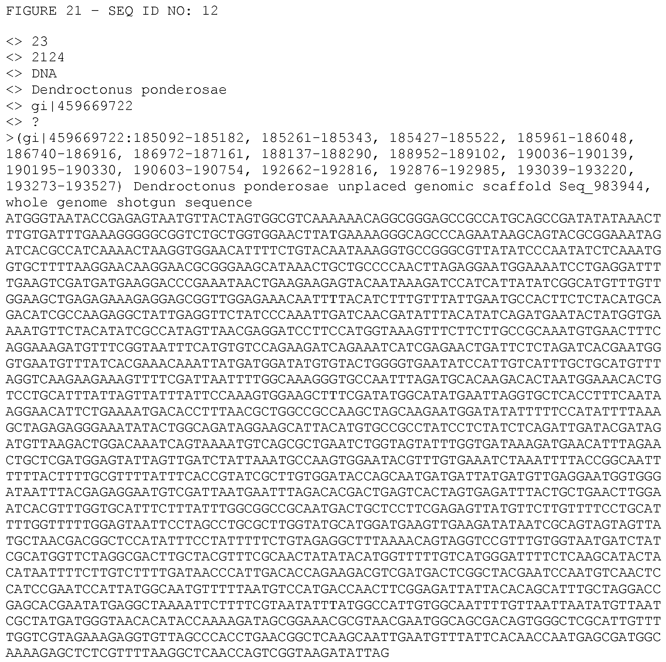

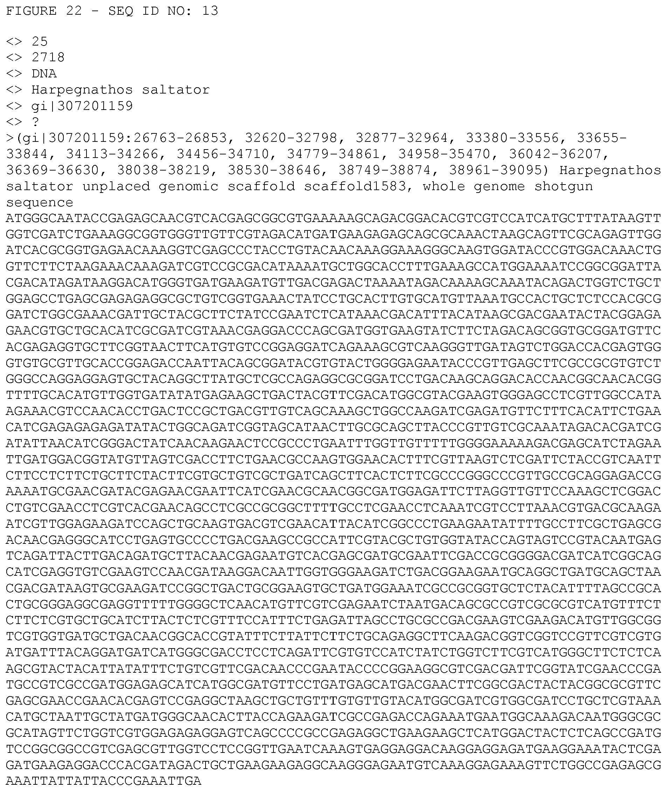

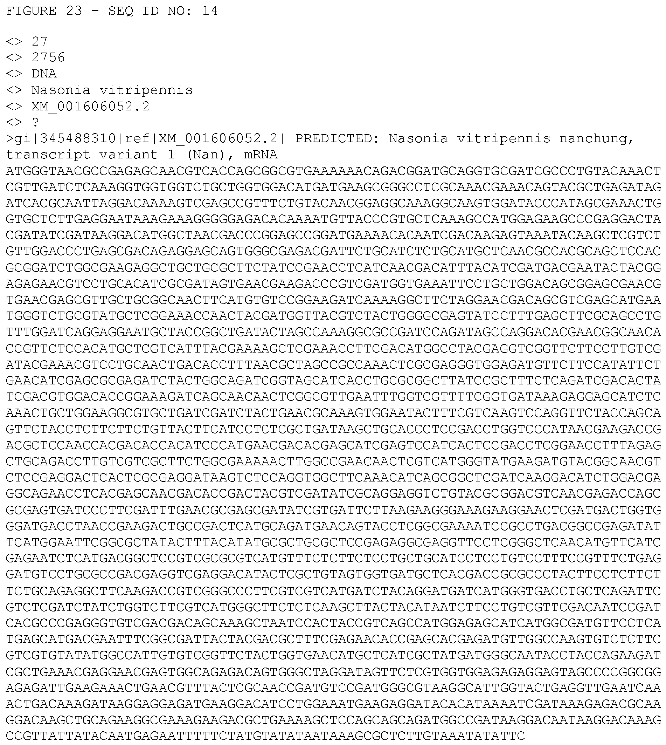

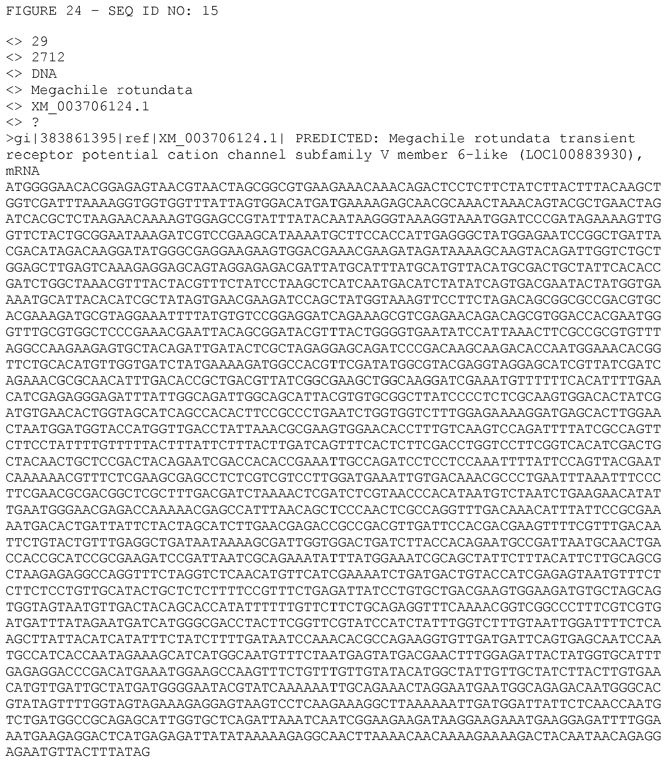

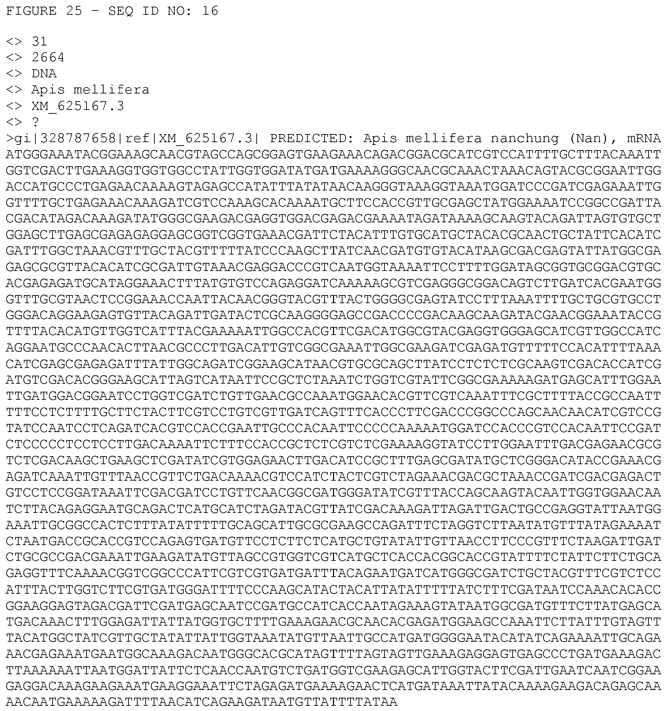

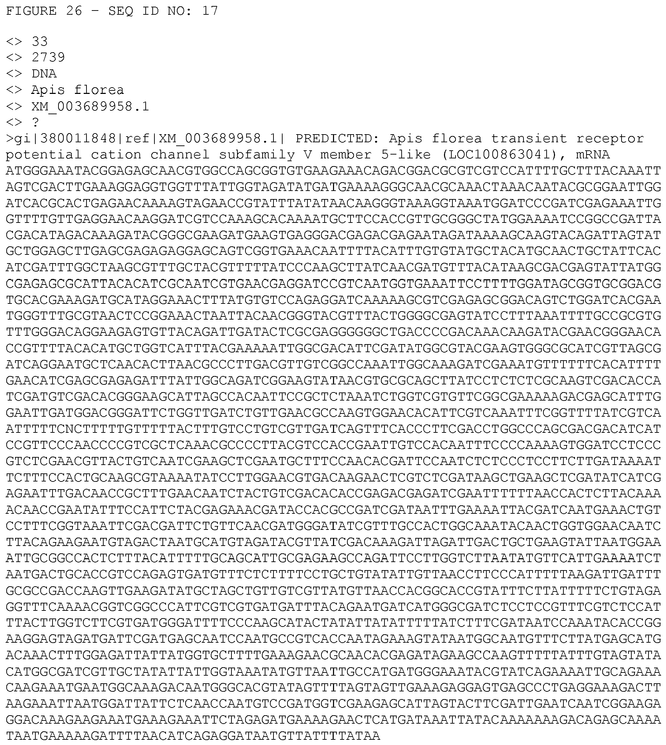

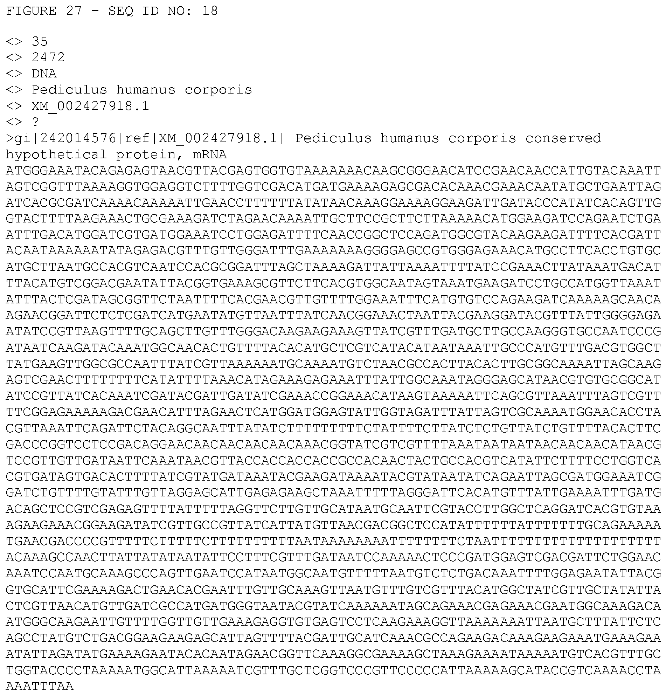

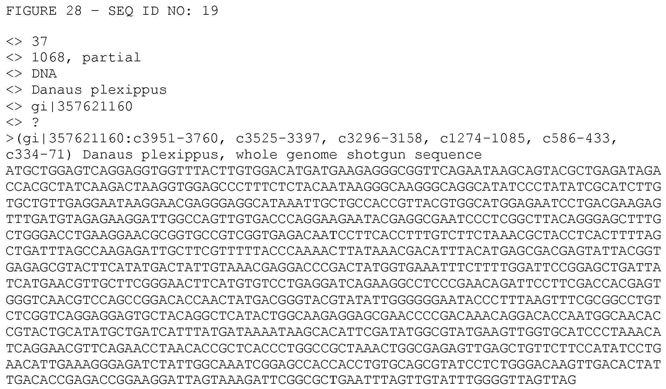

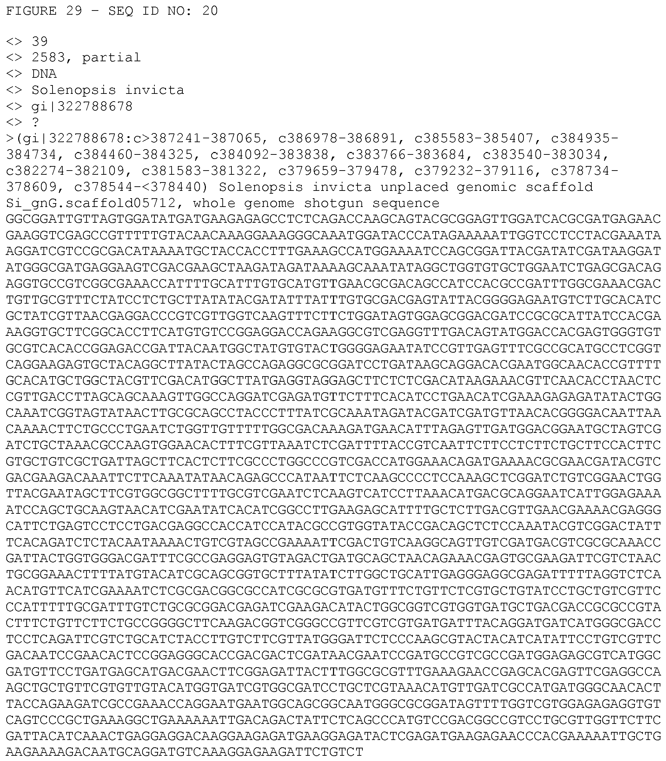

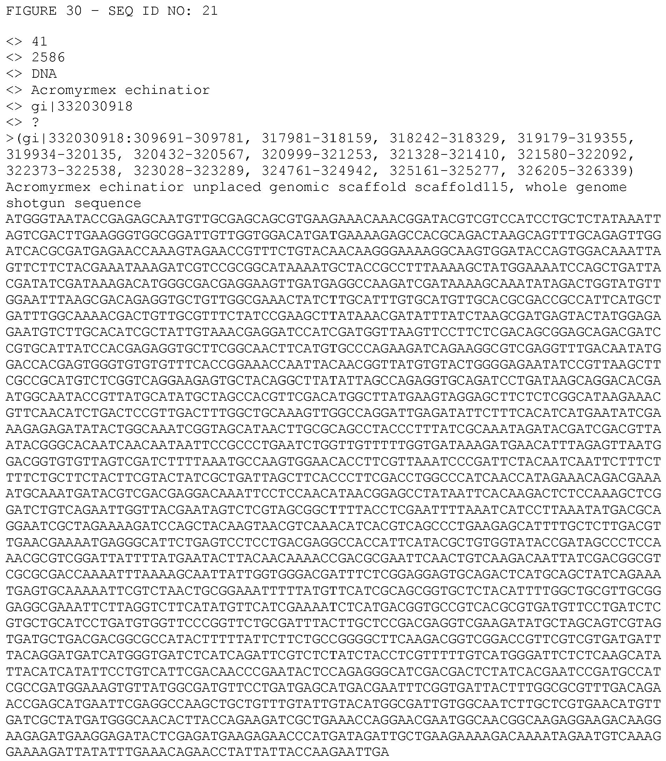

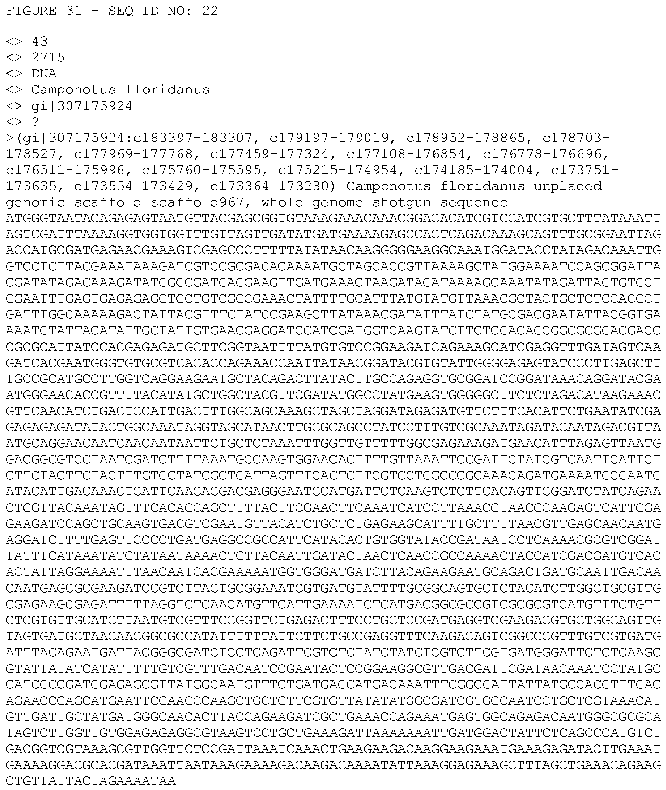

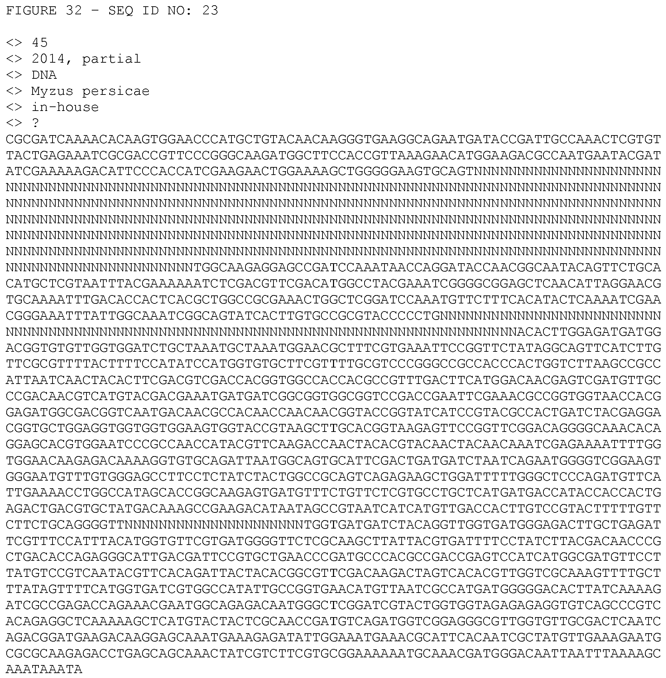

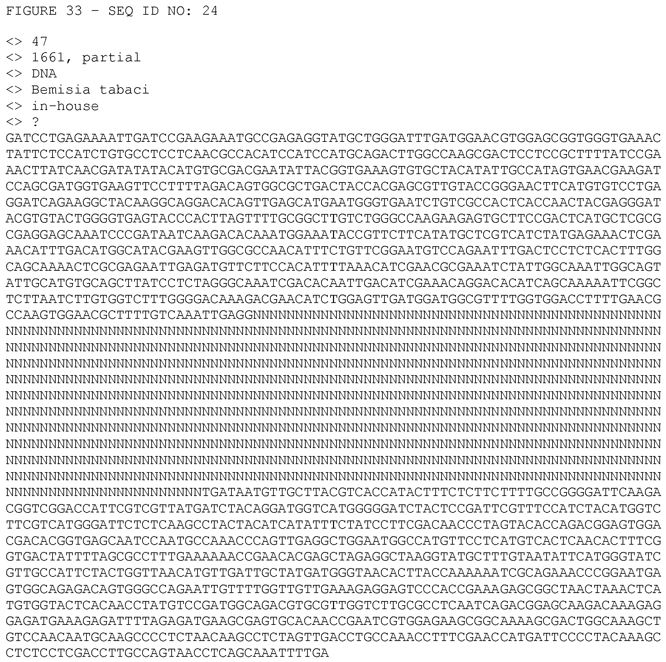

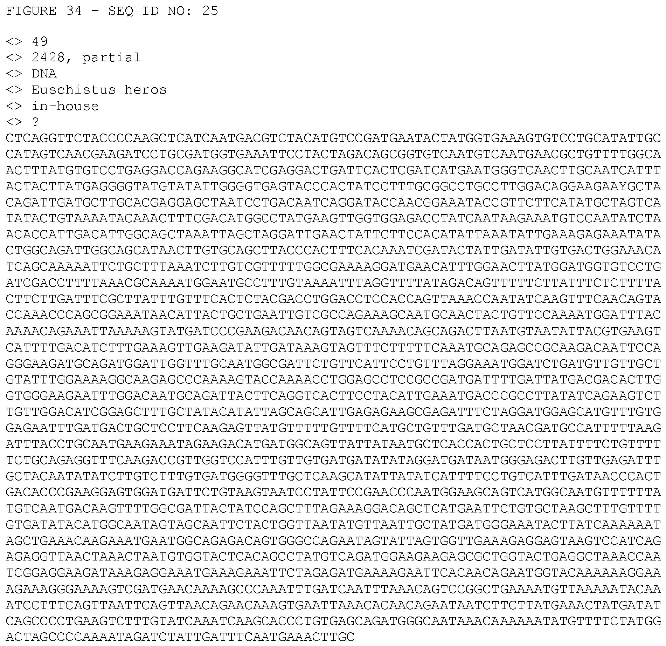

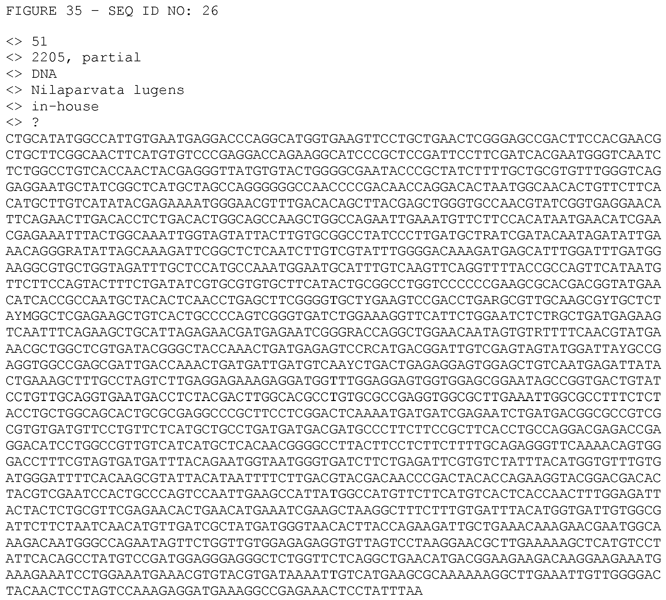

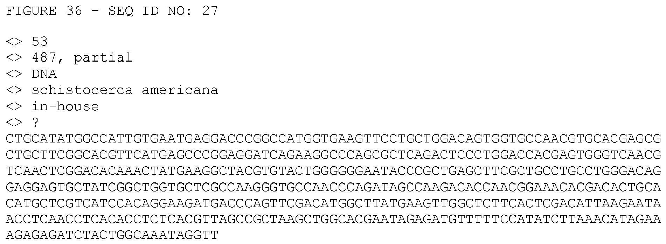

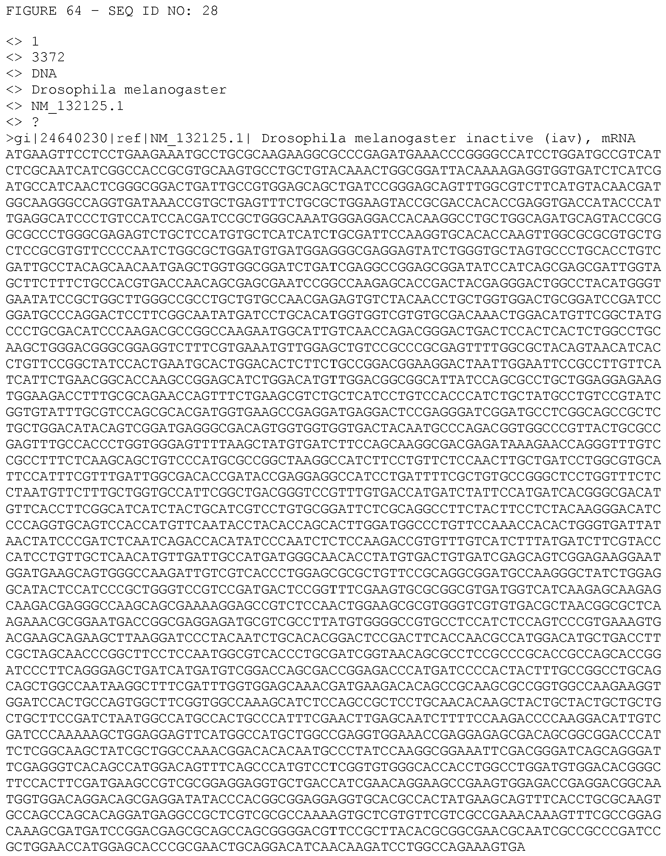

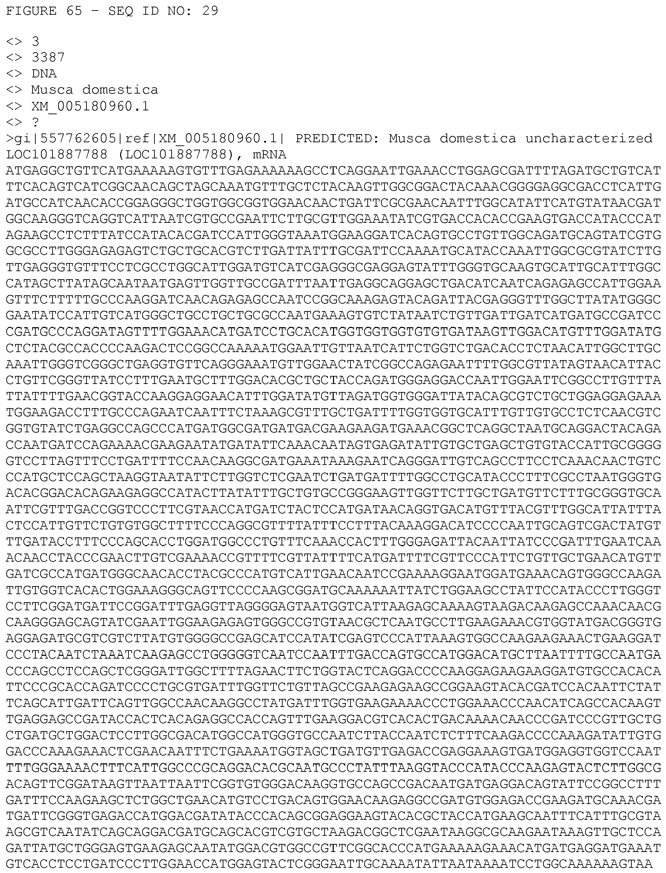

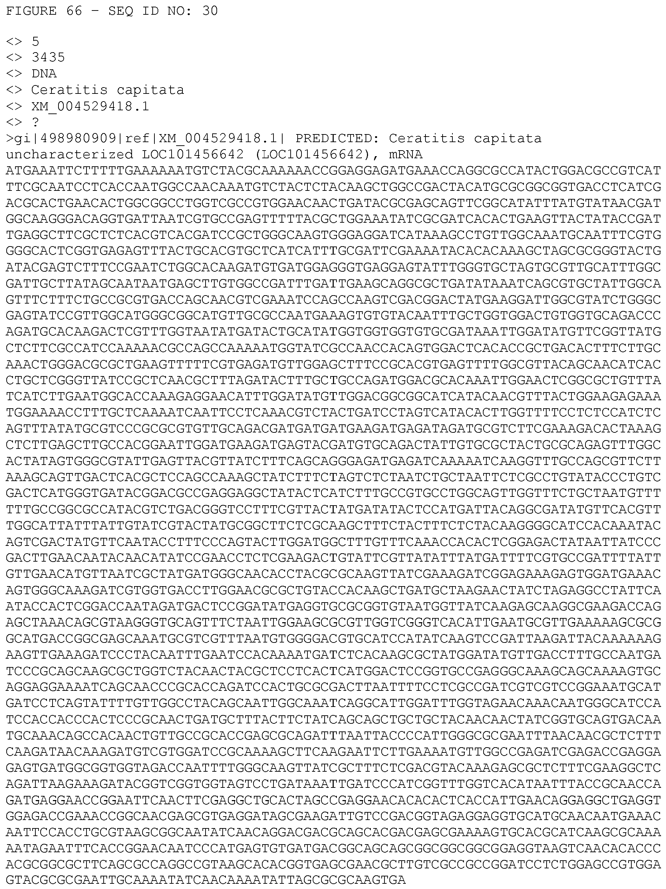

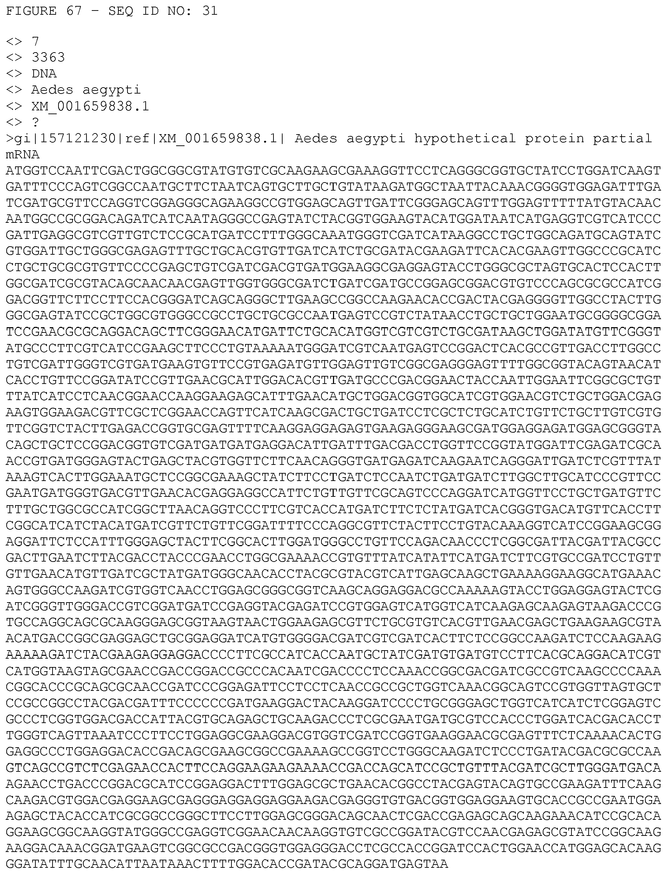

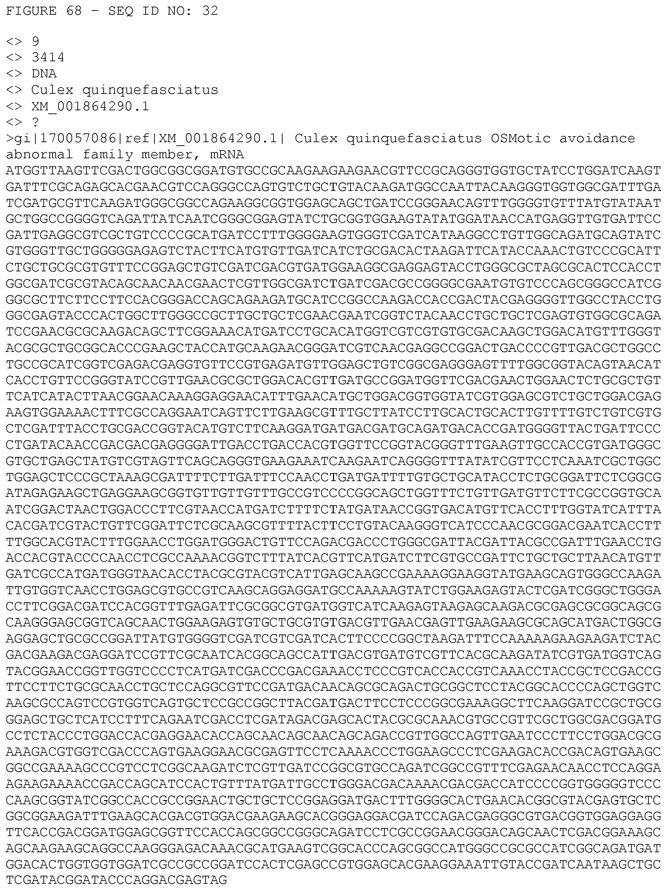

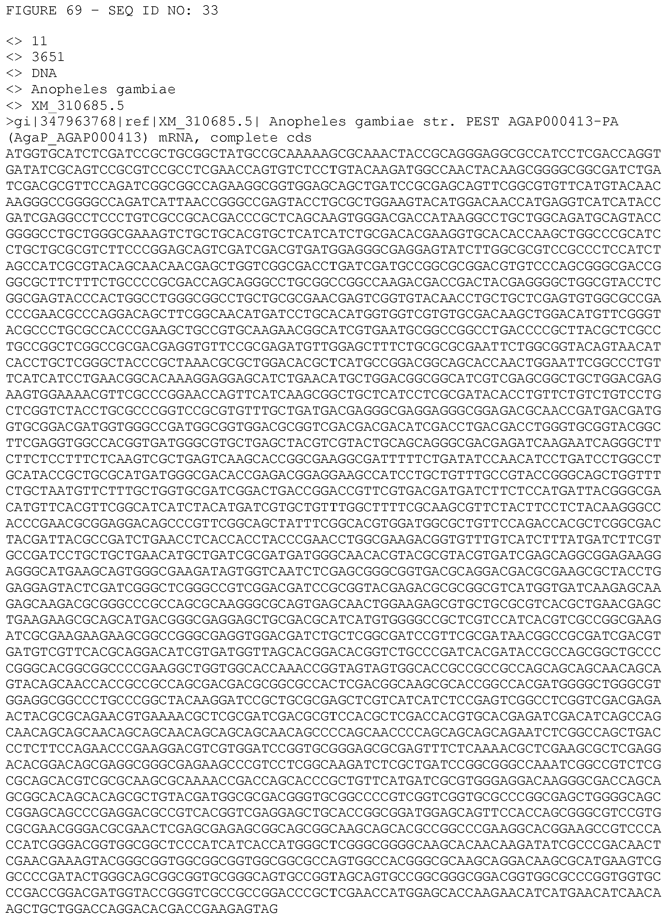

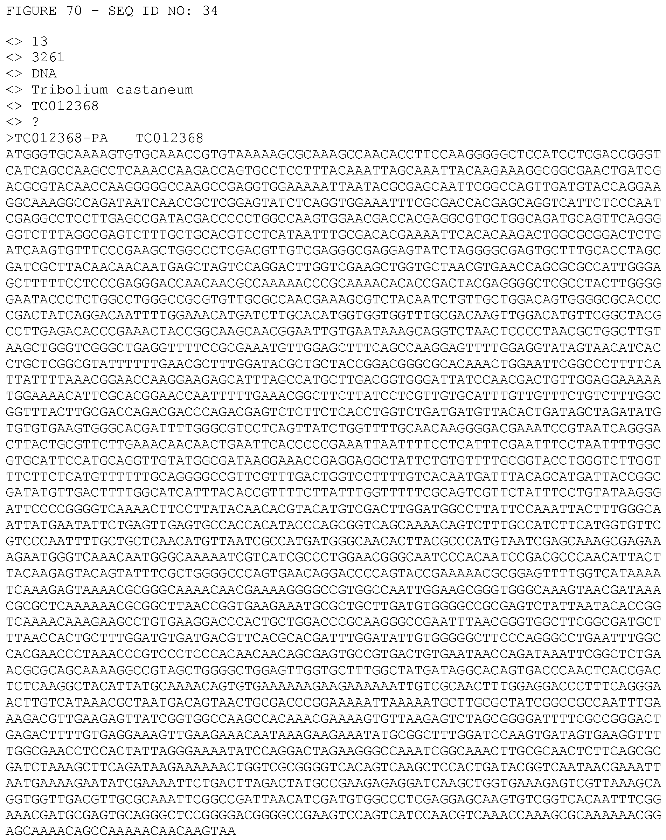

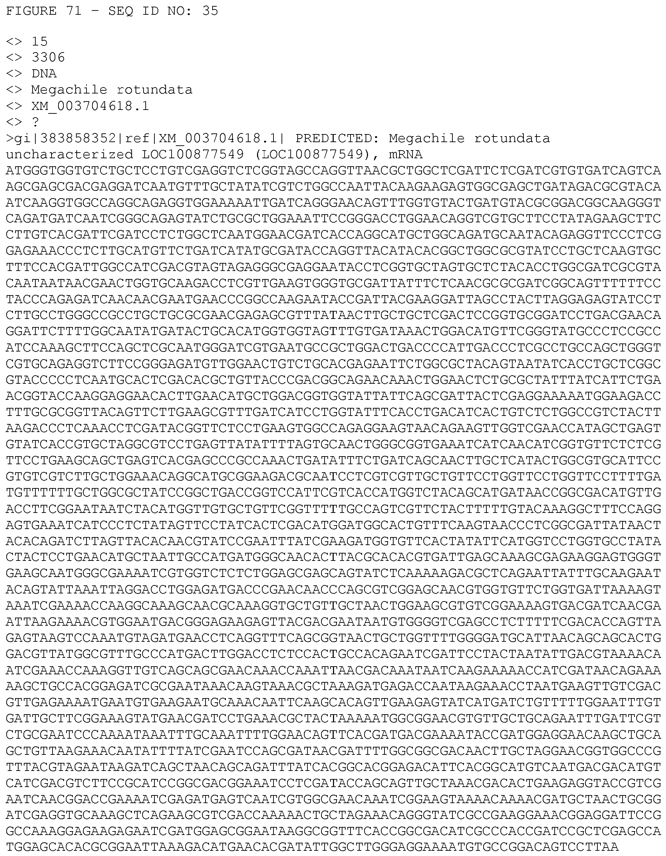

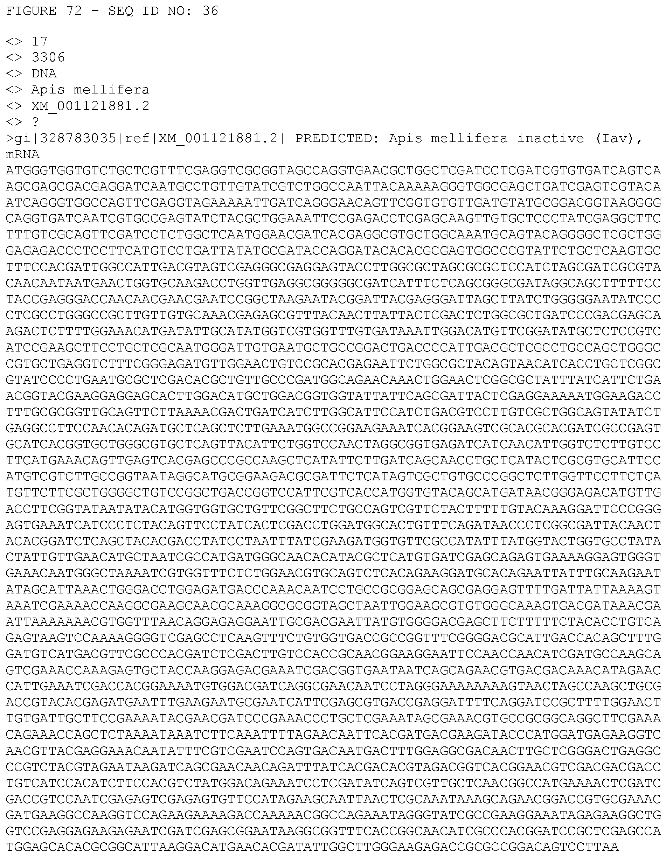

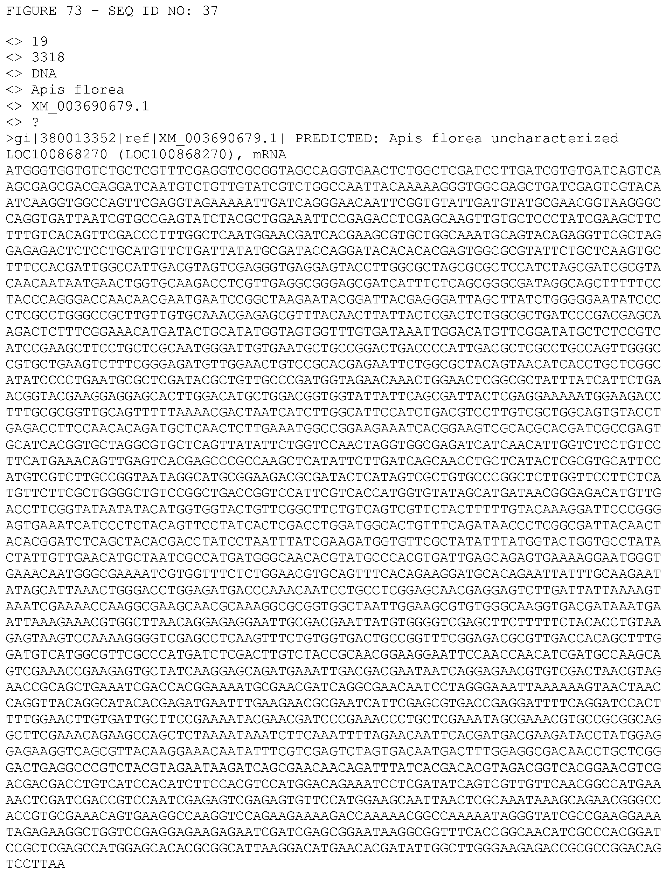

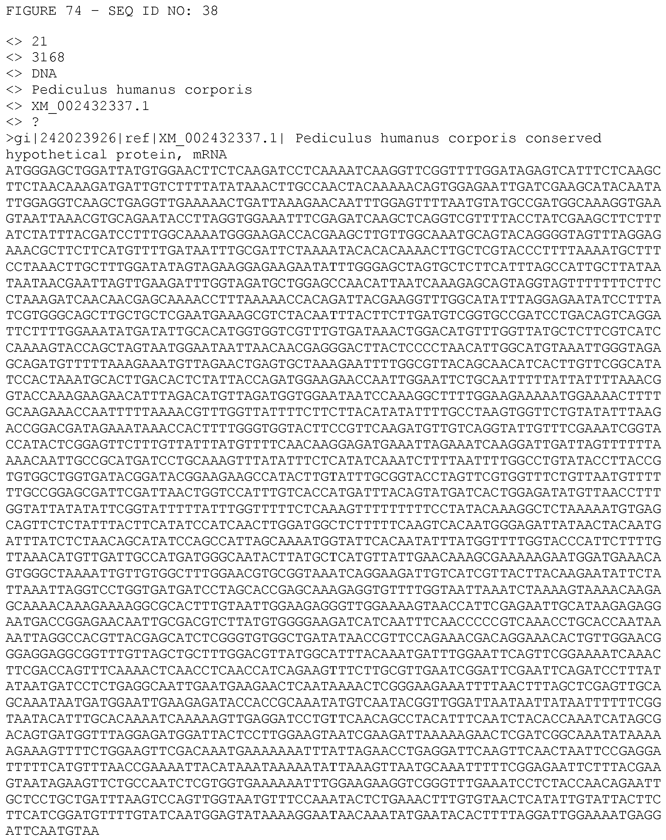

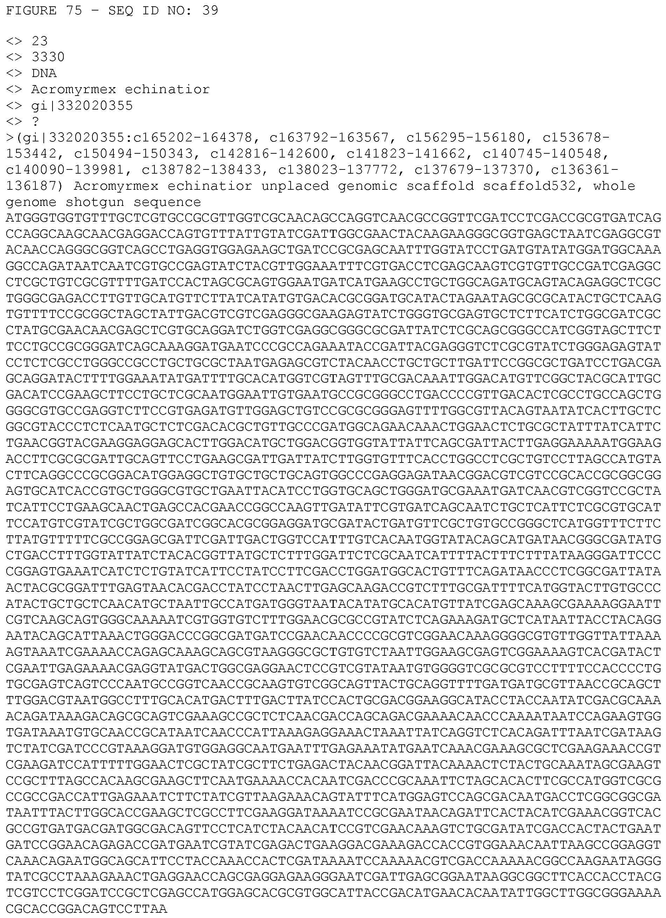

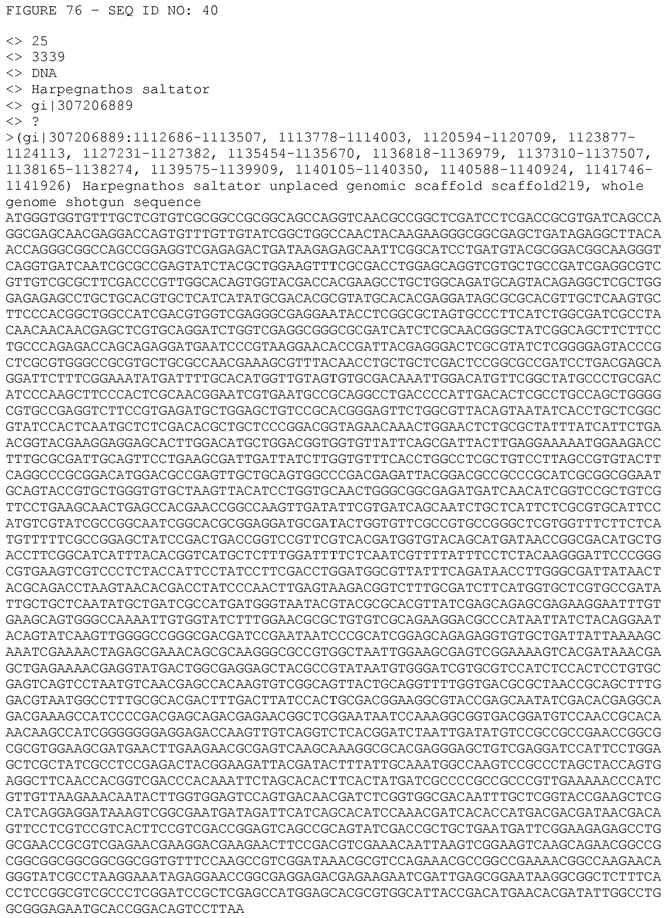

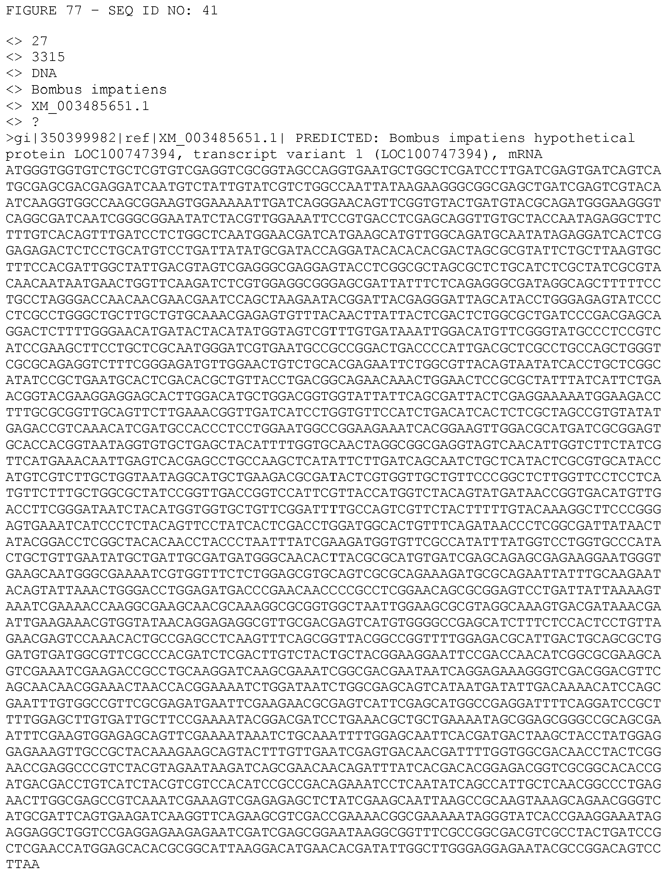

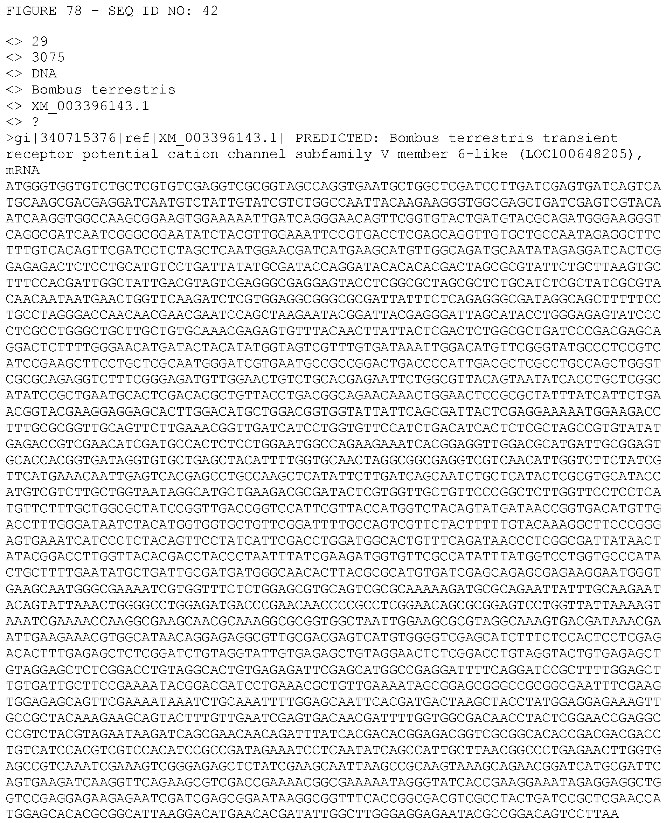

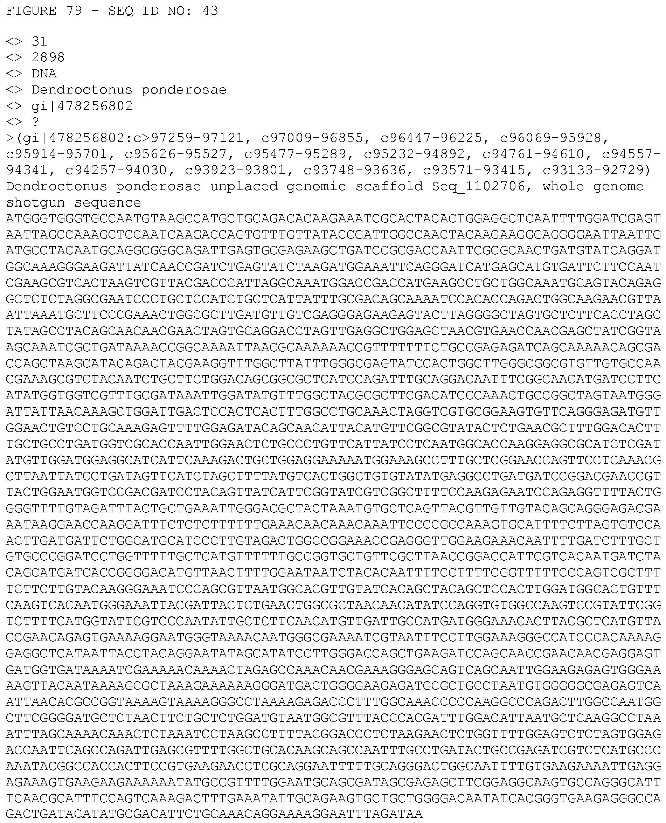

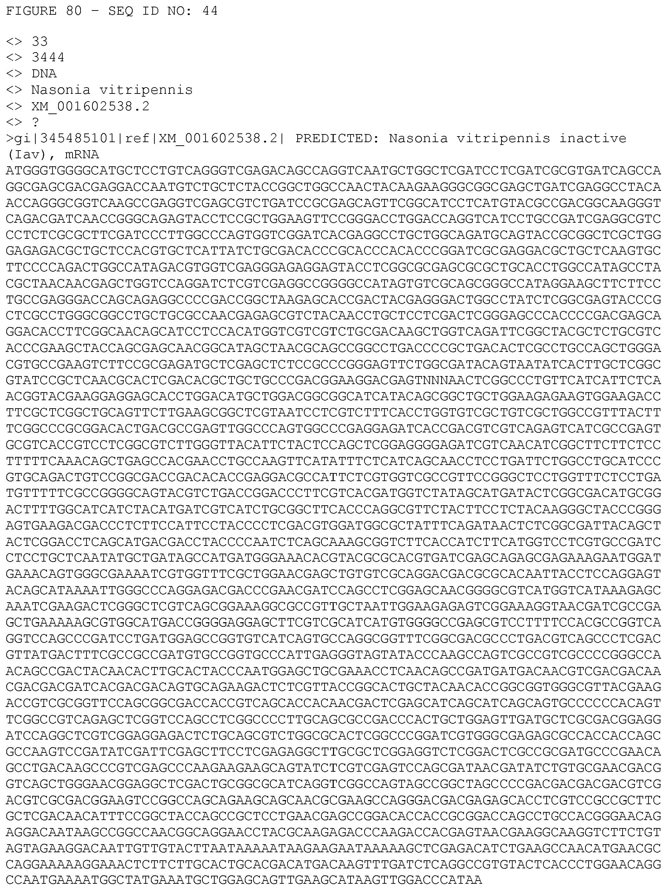

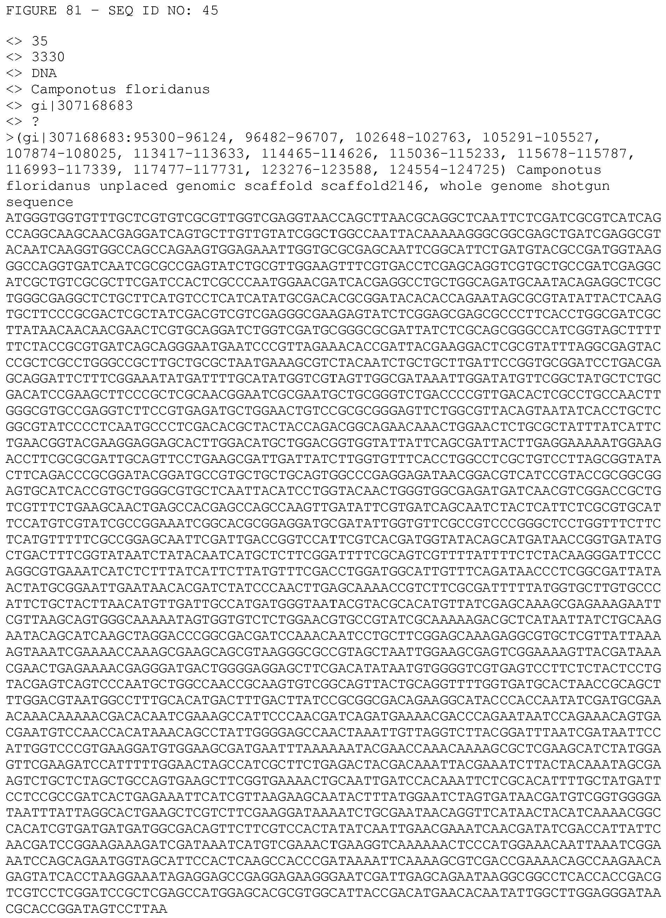

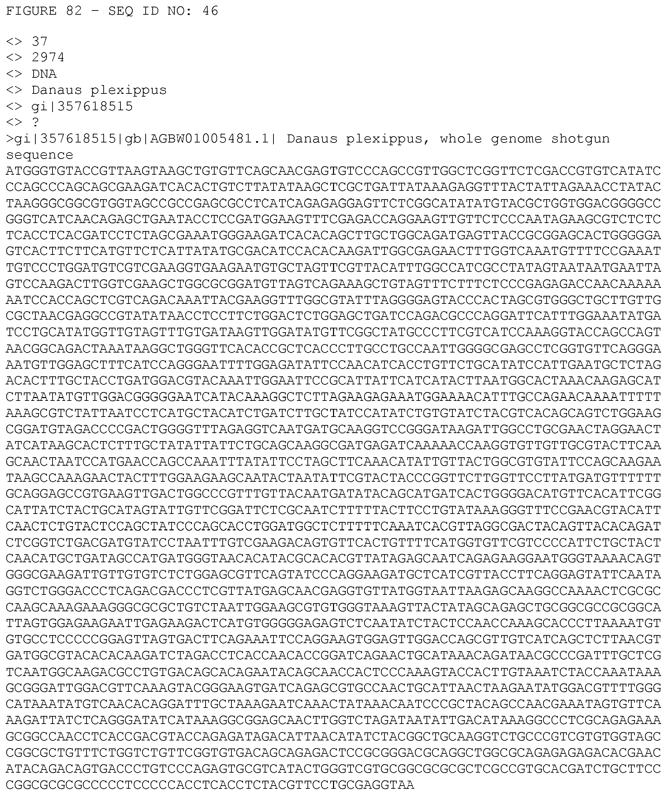

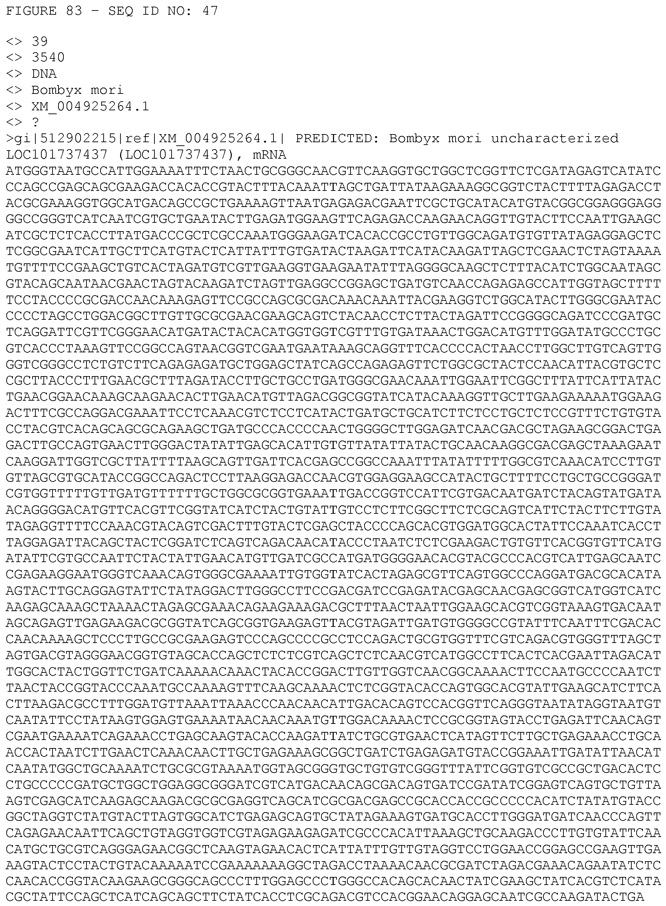

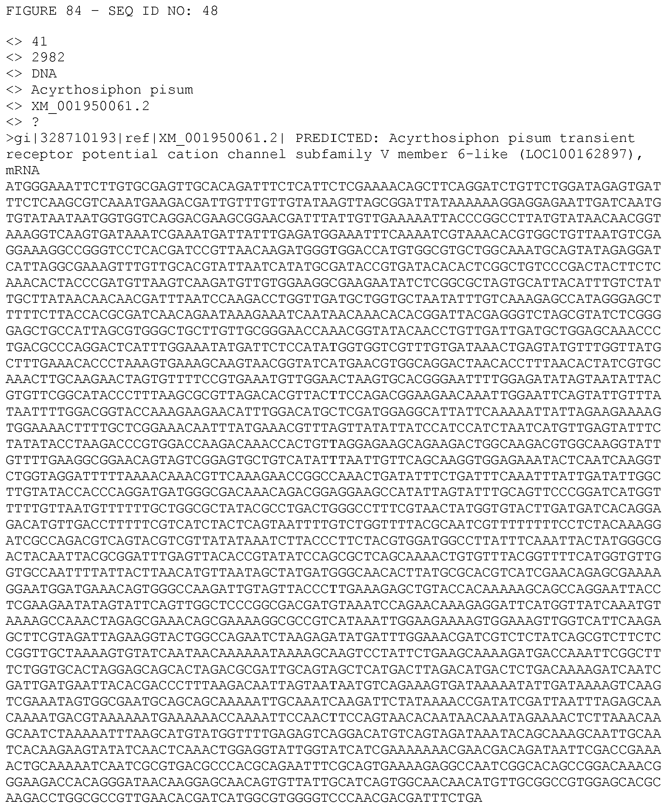

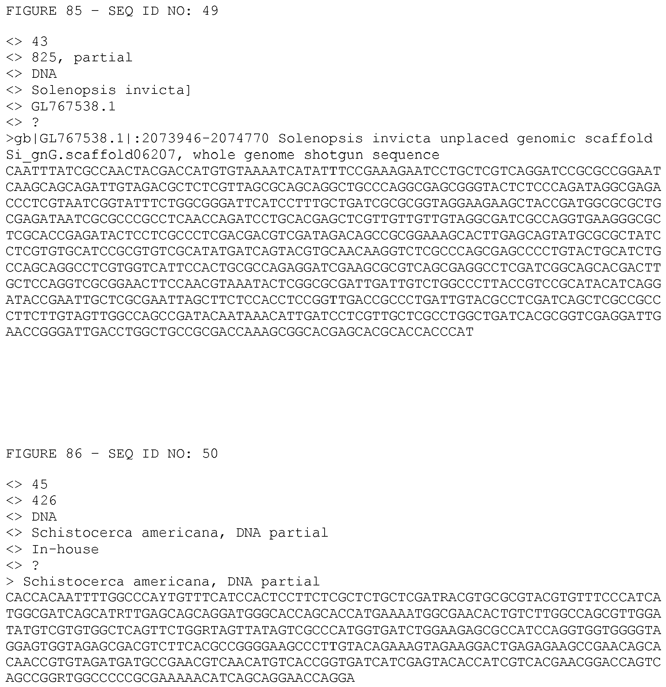

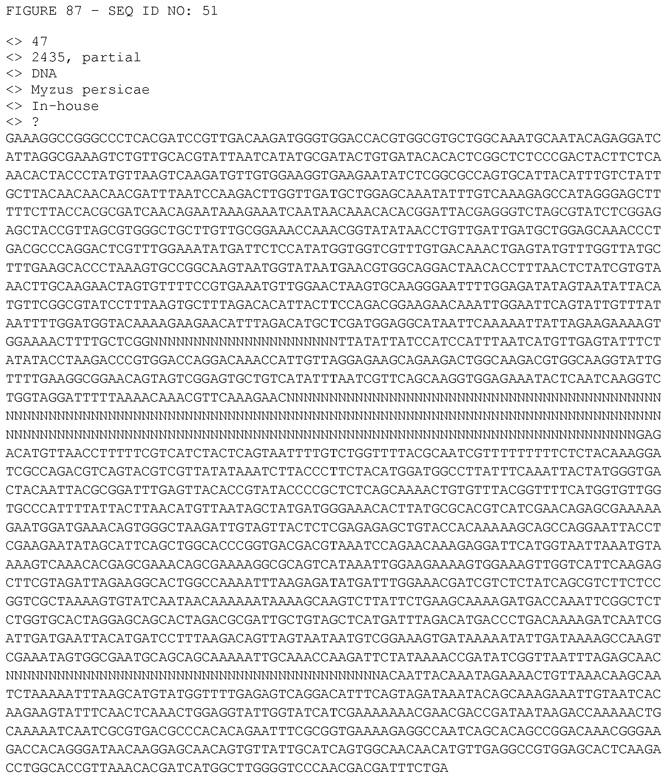

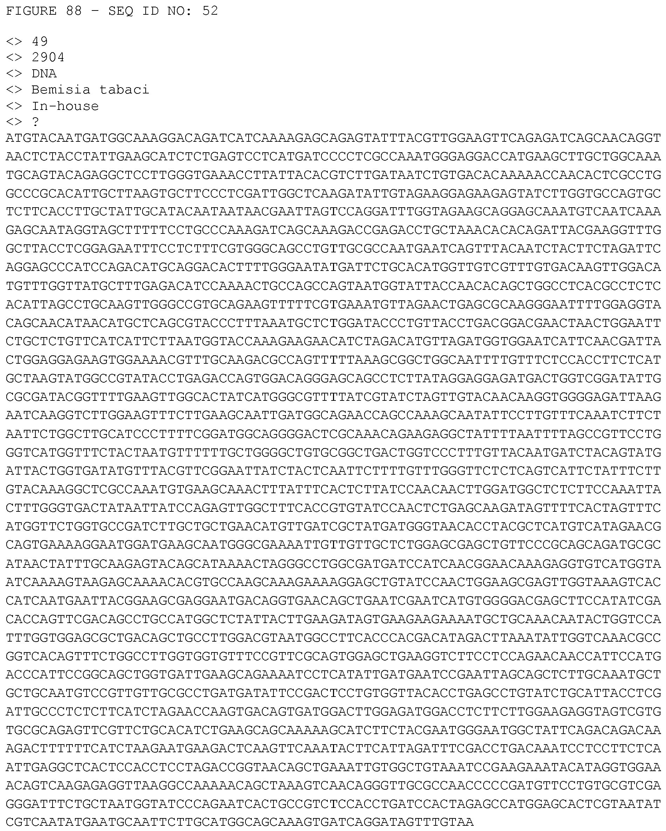

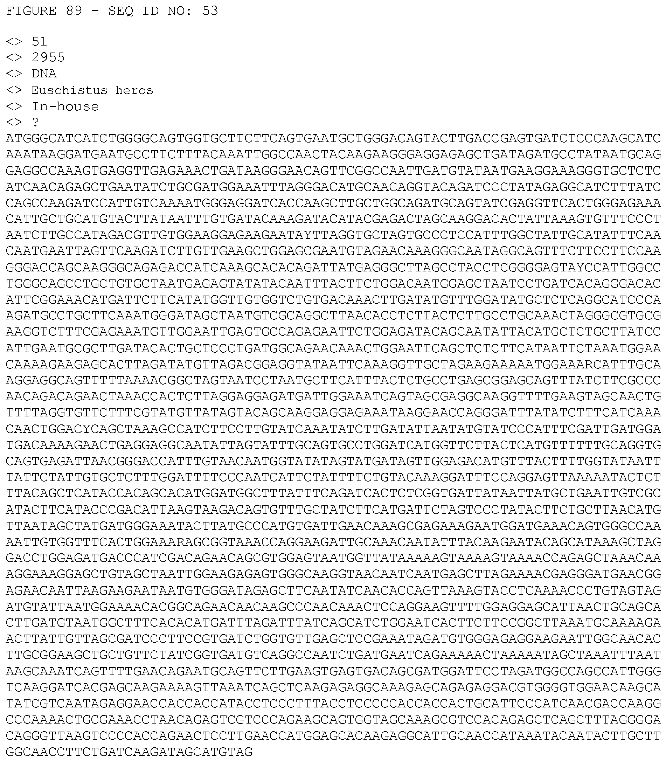

In some embodiments, the Nanchung nucleic acid sequences may be selected from the group consisting of a) a polynucleotide molecule comprising a nucleic acid molecule having a sequence selected from the group consisting of SEQ ID NOS: 1-27 (FIGS. 10-36); b) a polynucleotide molecule having at least about 70% sequence identity to the sequence of SEQ ID NOS: 1-27 (FIGS. 10-36); and c) a fragment of the polynucleotide molecule of a) or b). Such fragments can be a UTR, a core promoter, an intron, an enhancer, a cis-element, or any other regulatory element. In some embodiments, the Inactive nucleic acid sequences may be selected from the group consisting of a) a polynucleotide molecule comprising a nucleic acid molecule having a sequence selected from the group consisting of SEQ ID NOS: 28-53 (FIGS. 64-89); b) a polynucleotide molecule having at least about 70% sequence identity to the sequence of SEQ ID NOS: 28-53 (FIGS. 64-89); and c) a fragment of the polynucleotide molecule of a) or b). Such fragments can be a UTR, a core promoter, an intron, an enhancer, a cis-element, or any other regulatory element. Human, mouse, rat, or hamster TRPV1, TRPV2, TRPV3, TRPV4, TRPV5 or TRPV6
The disclosed polynucleotides are capable of providing for expression of Nanchung and Inactive proteins in the host cells.

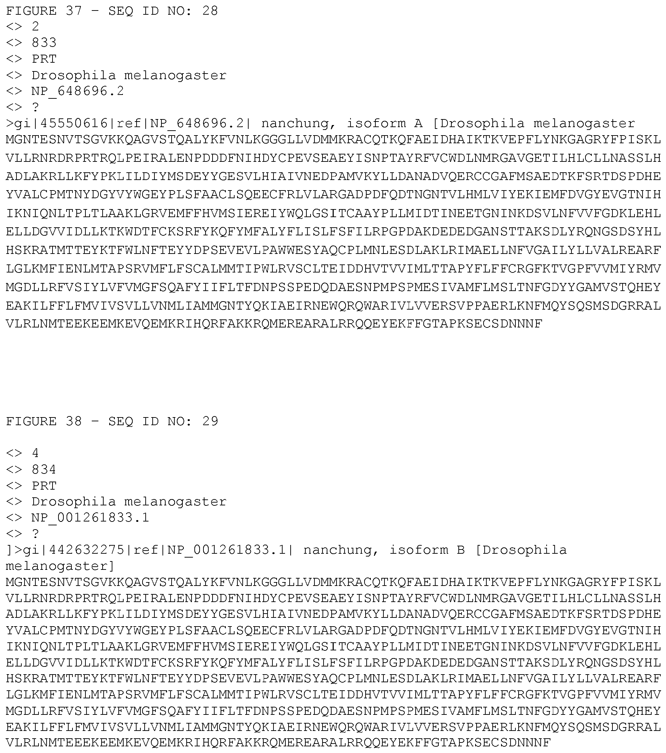

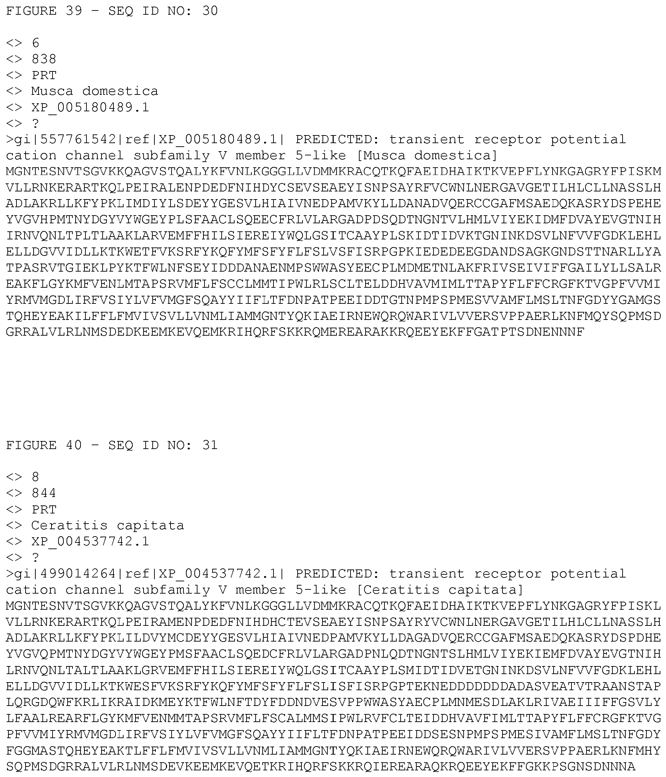

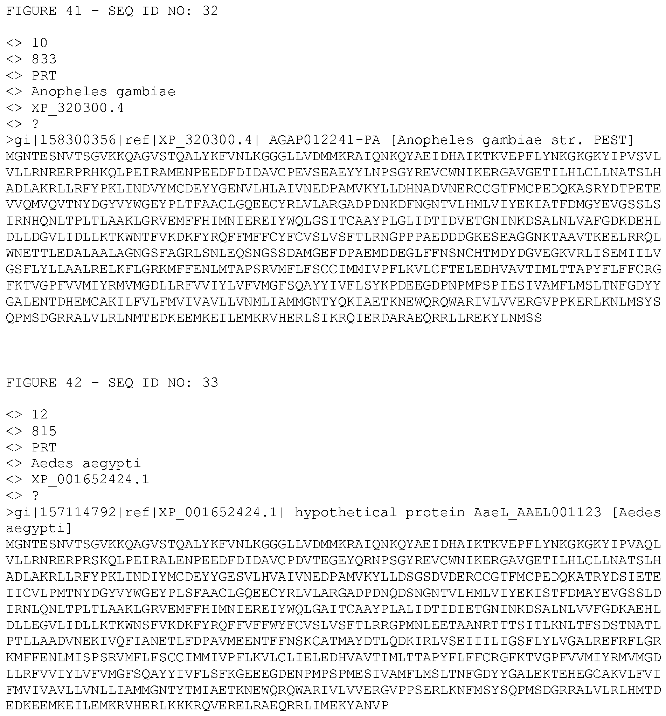

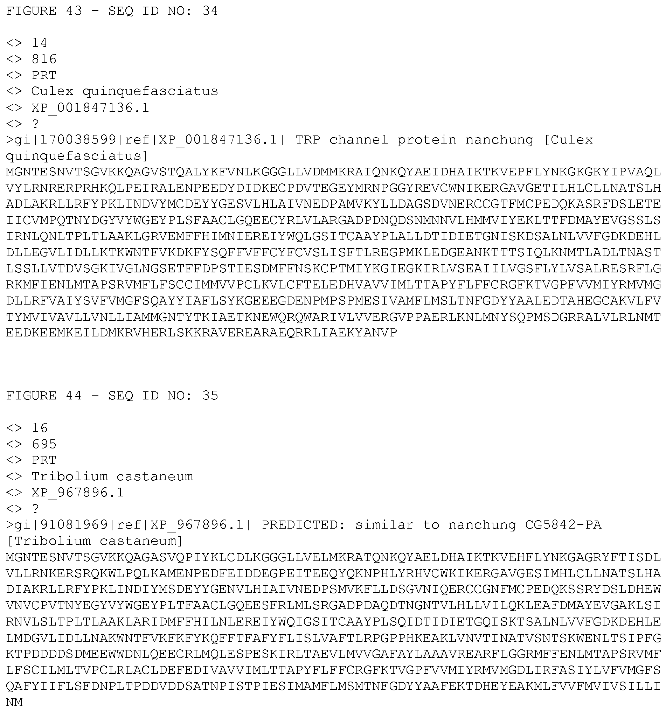

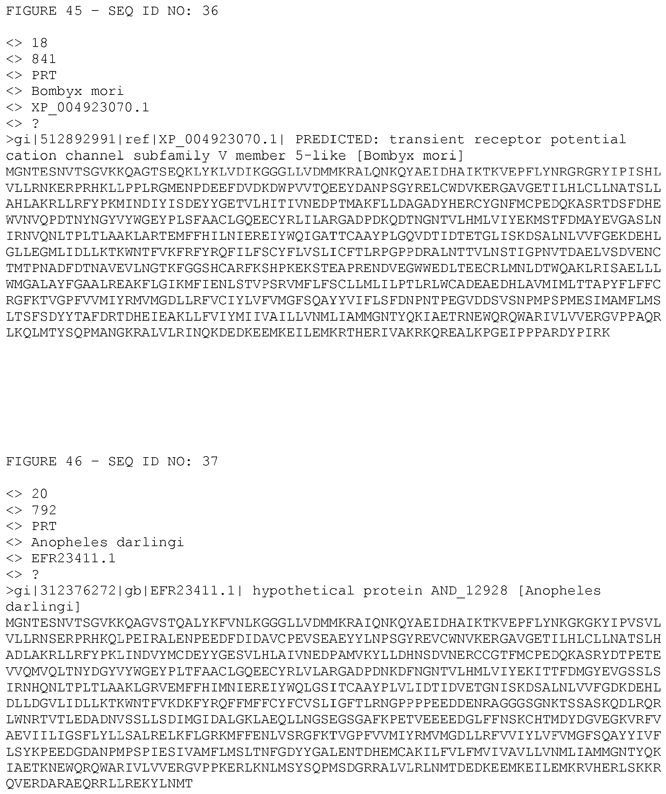

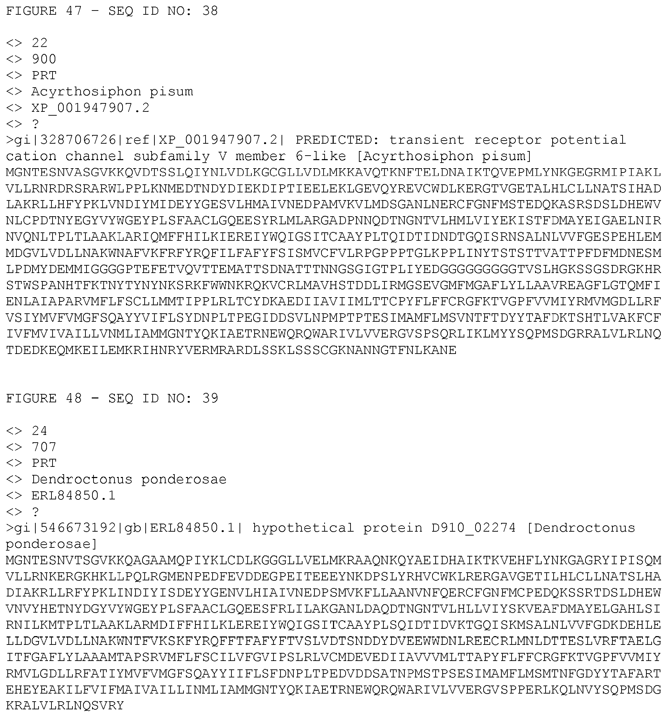

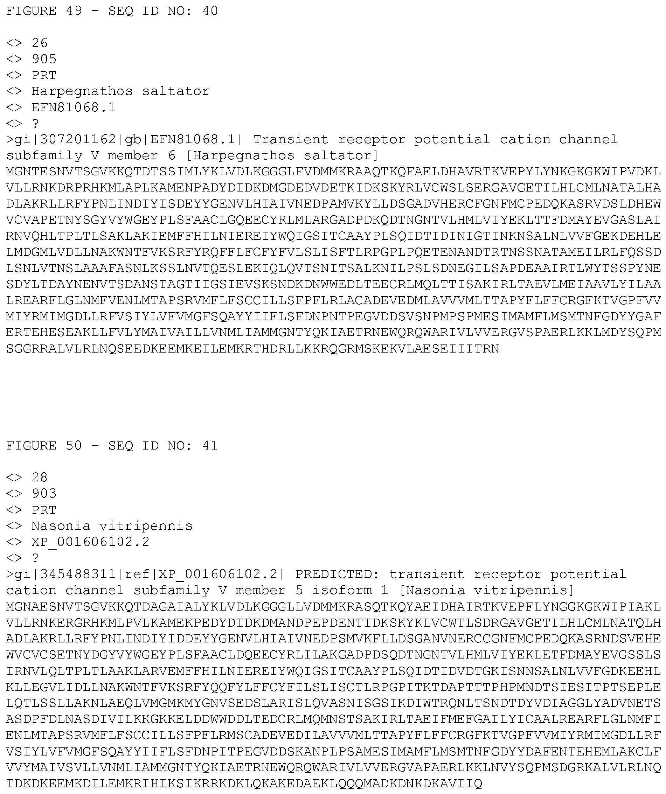

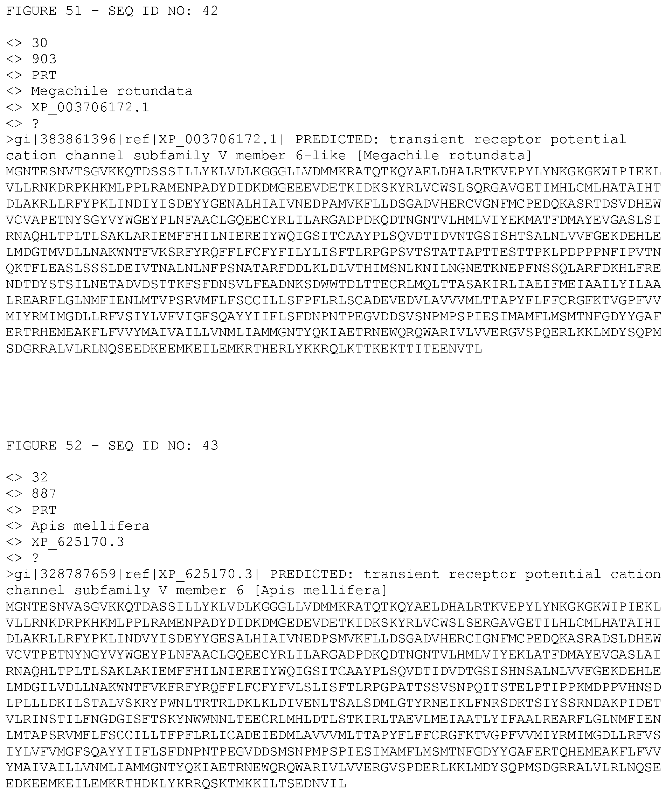

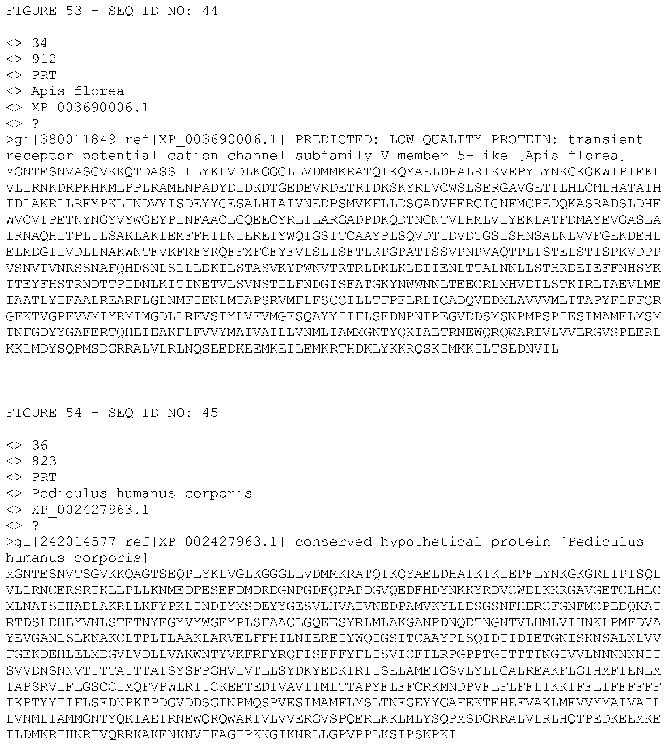

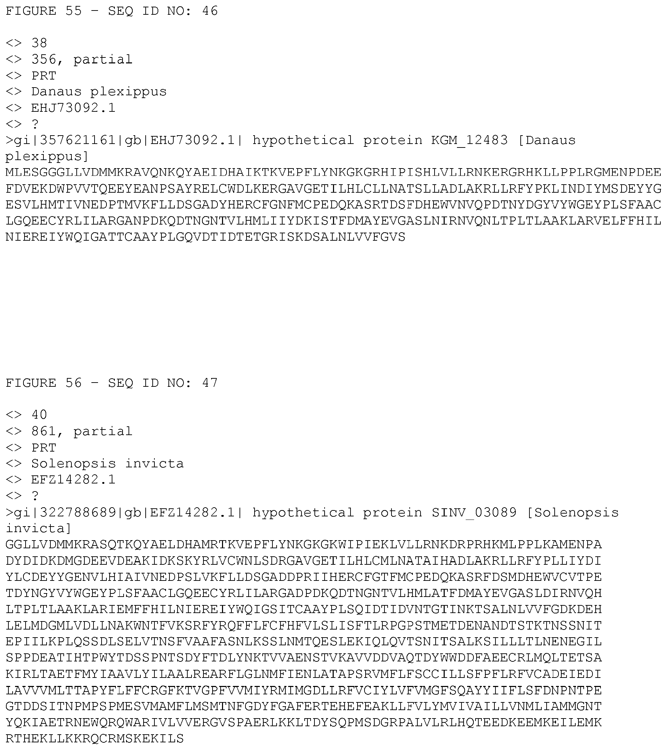

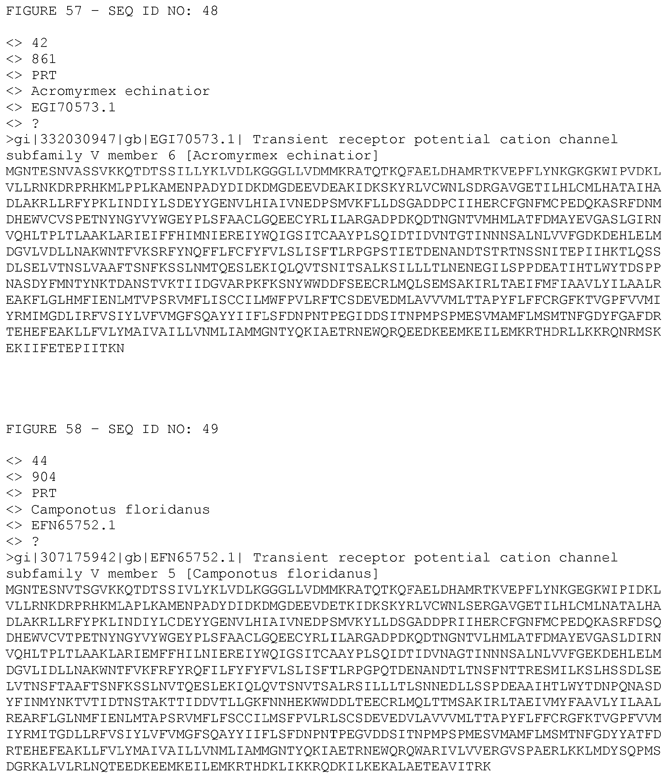

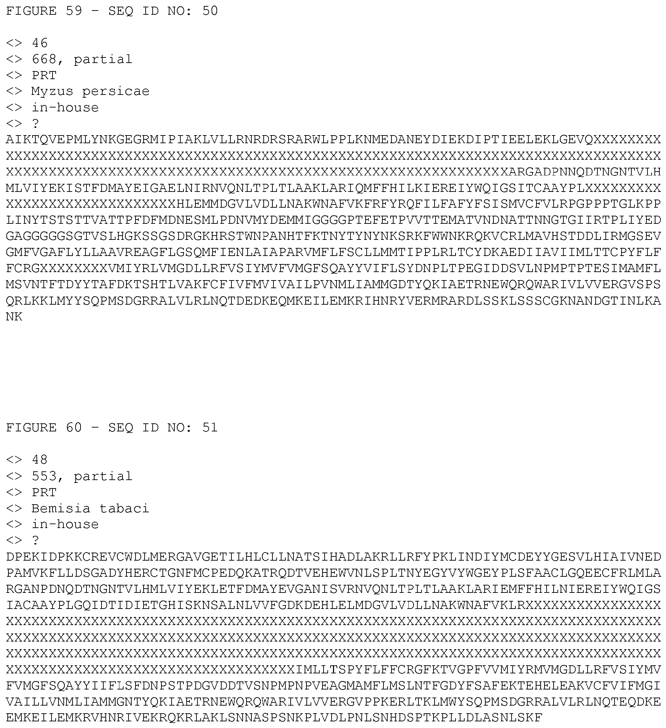

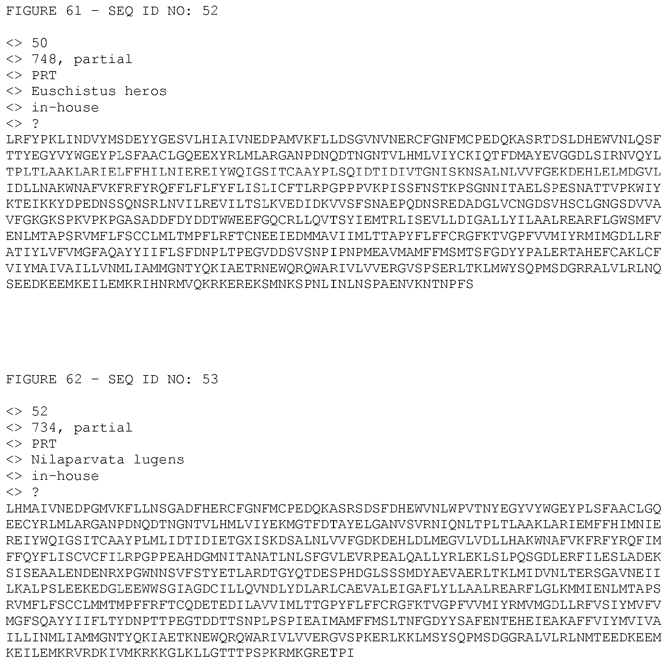

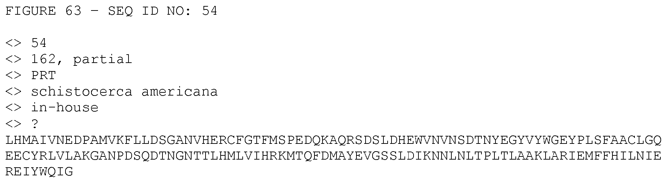

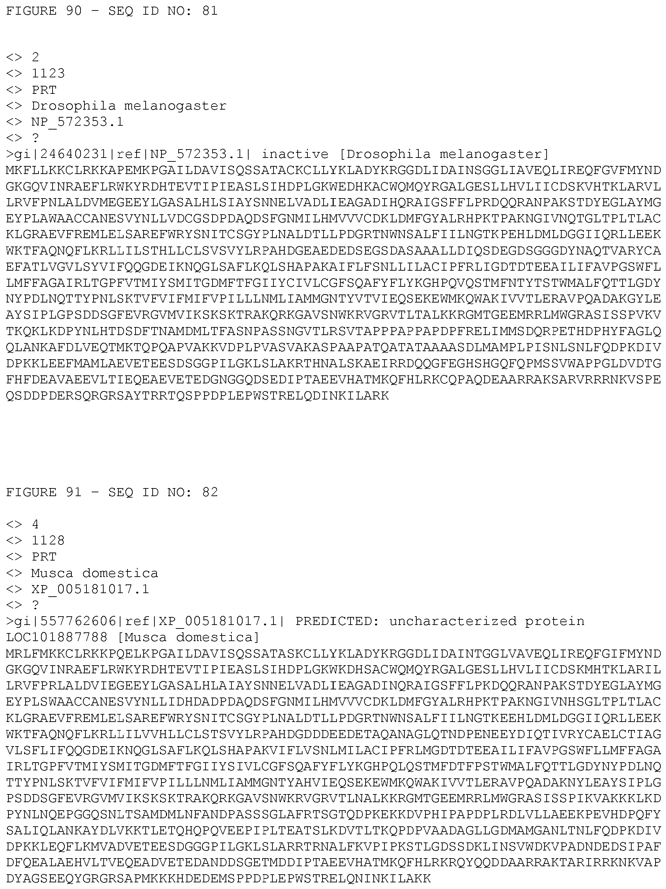

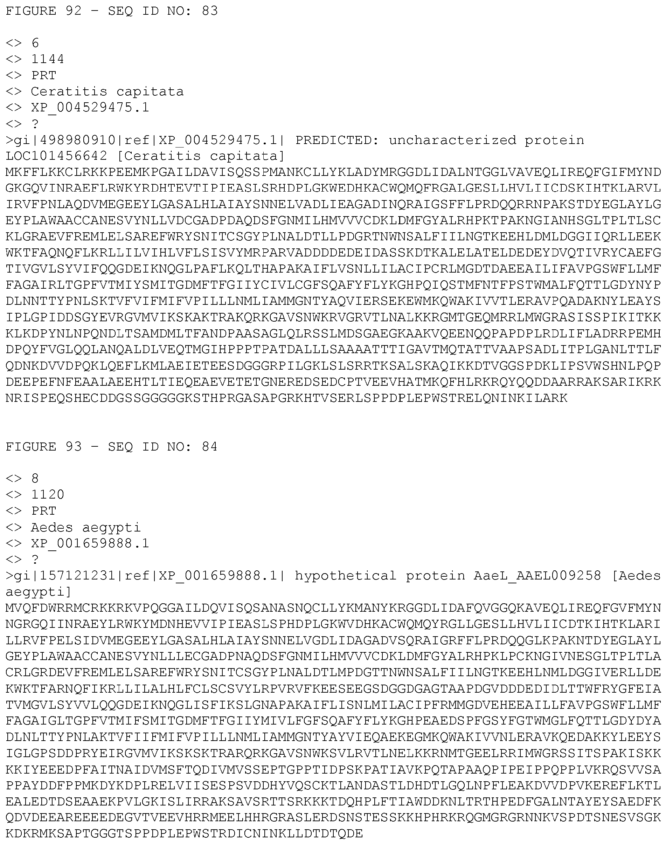

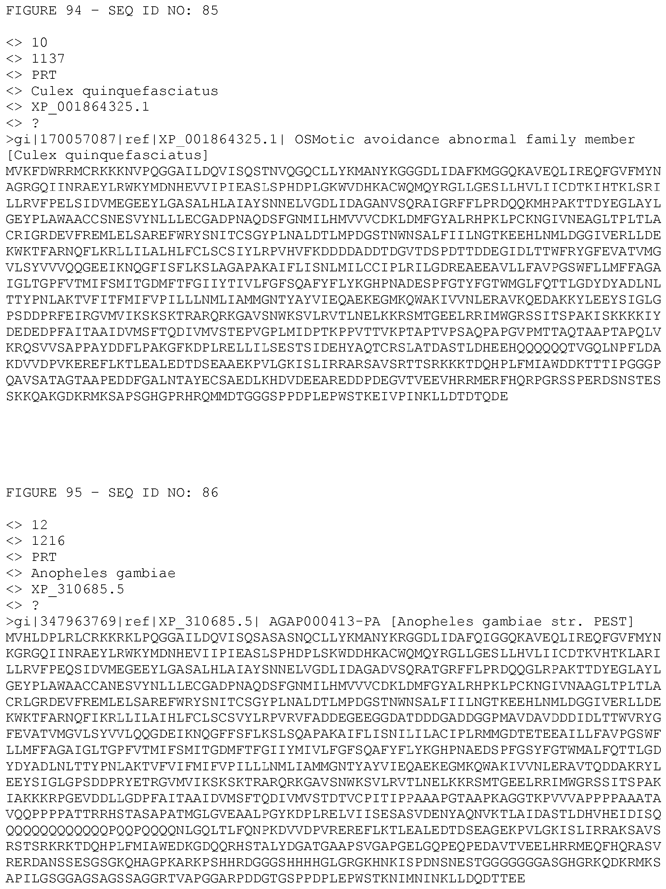

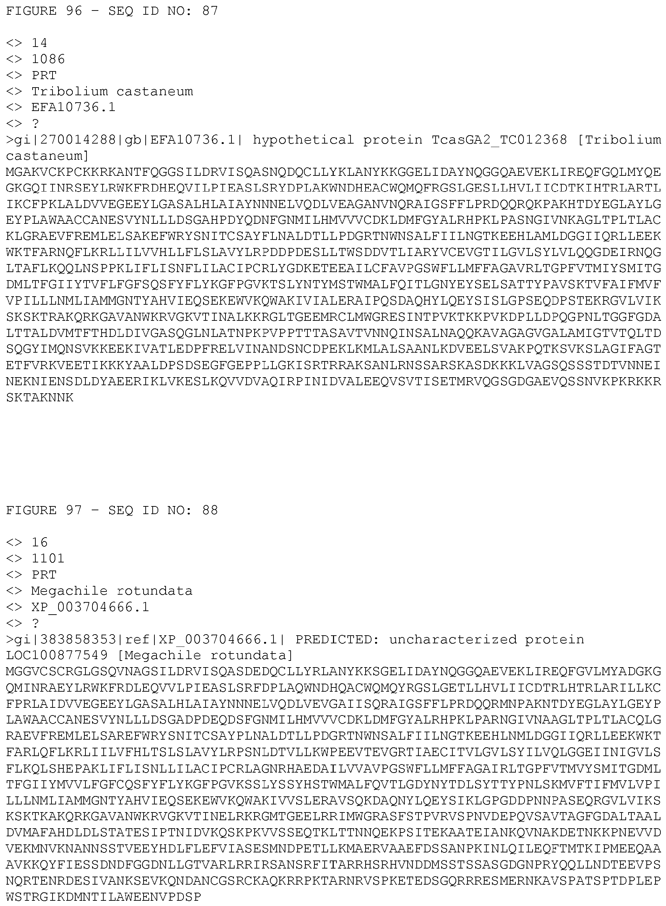

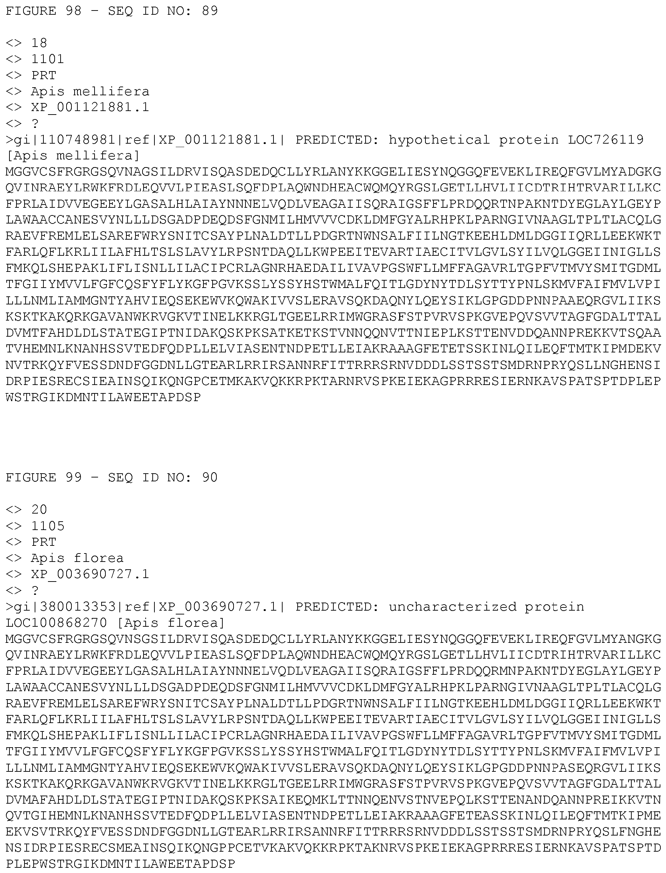

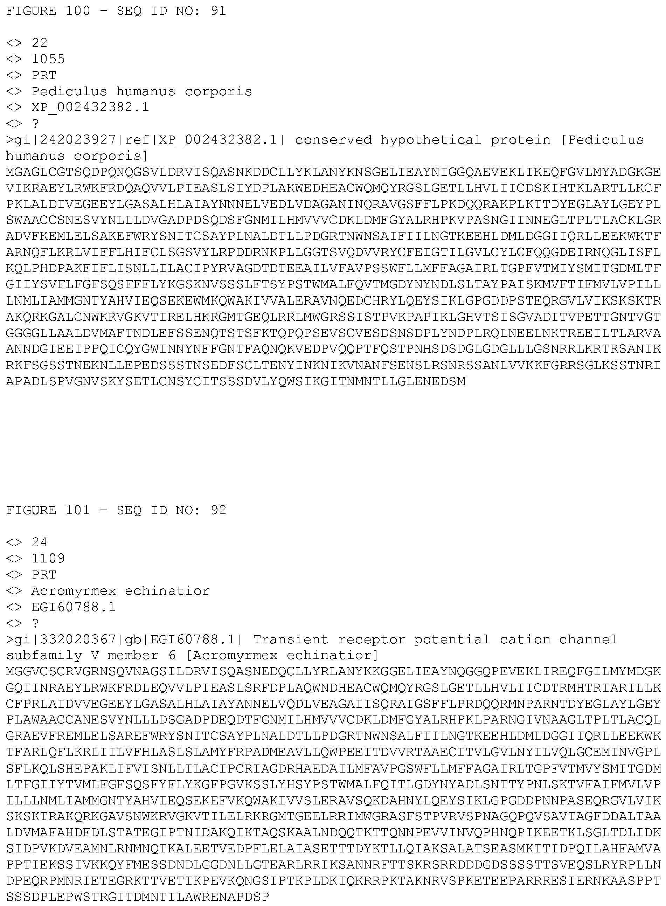

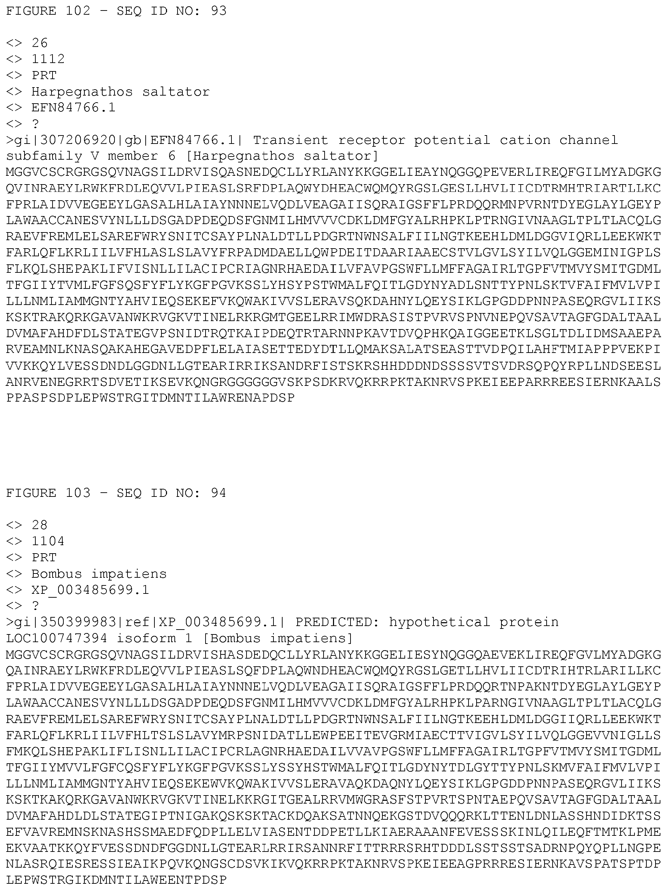

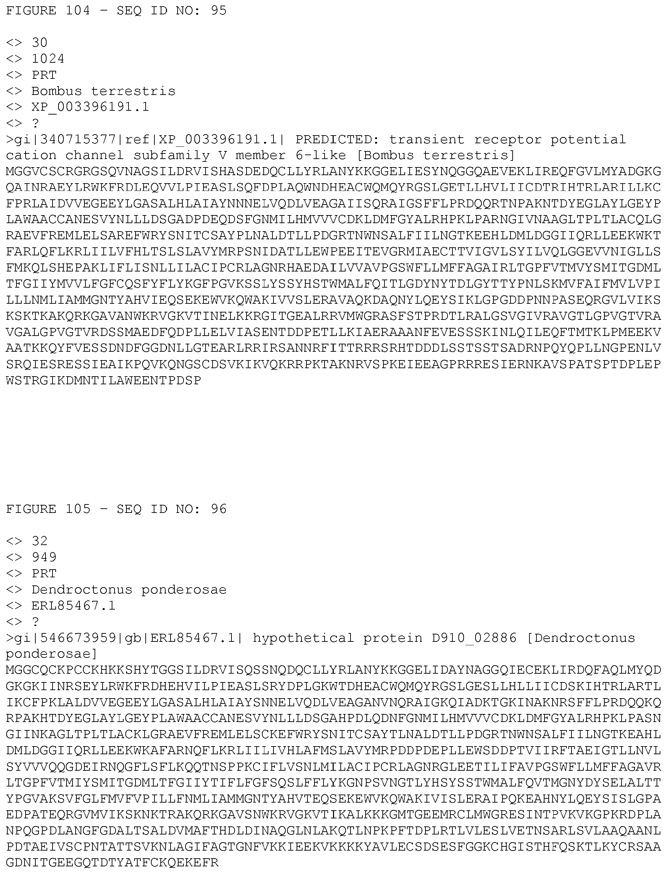

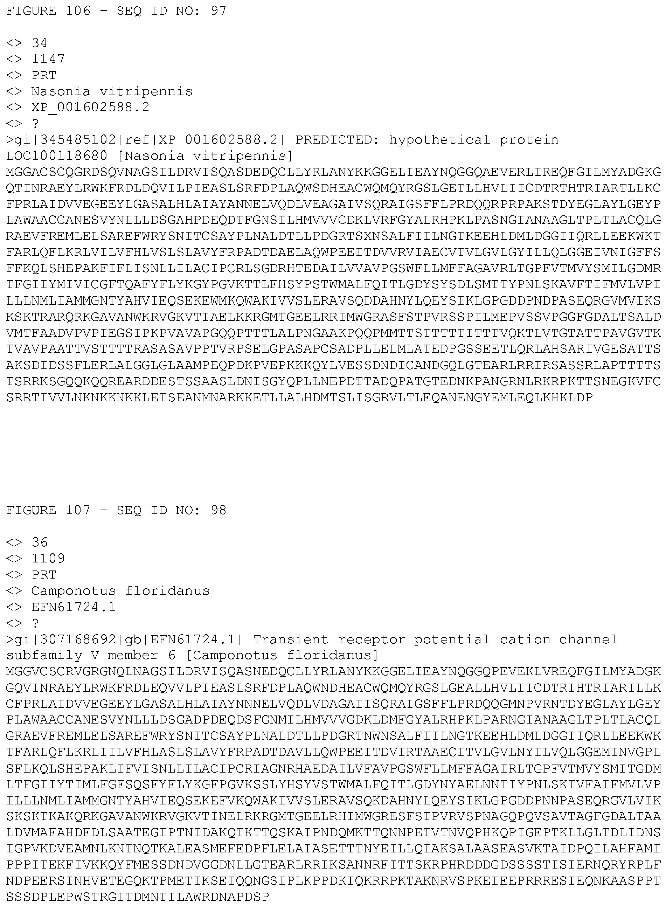

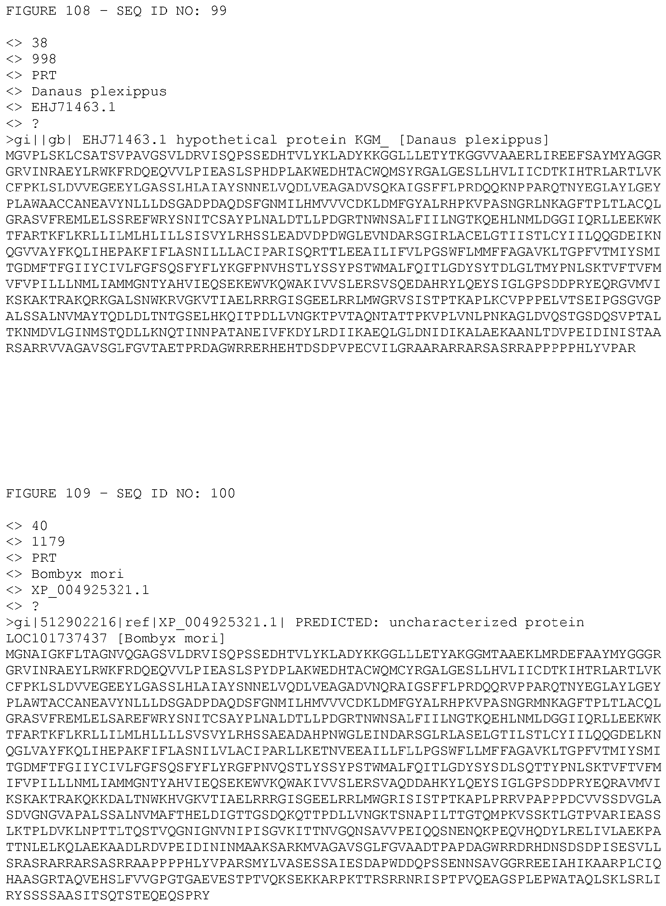

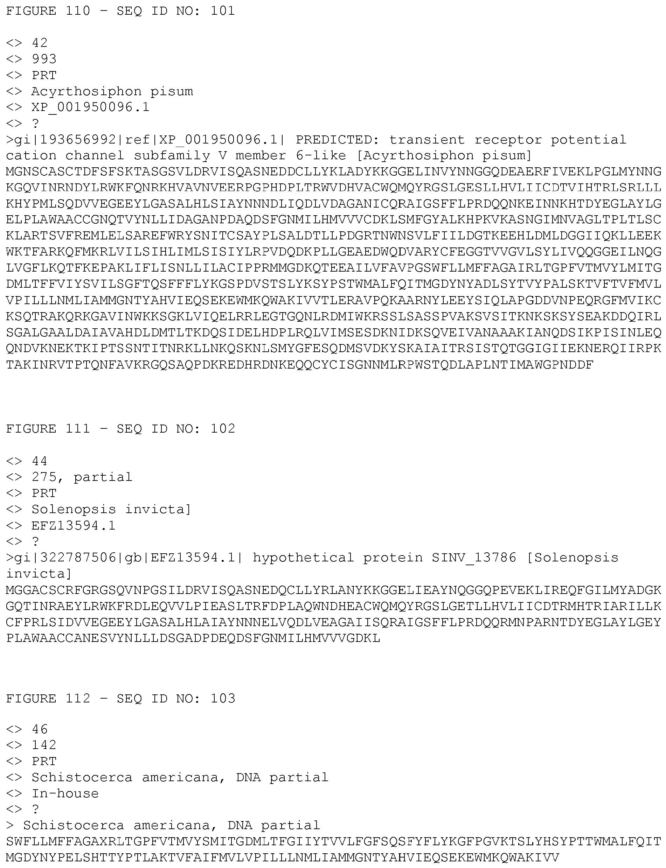

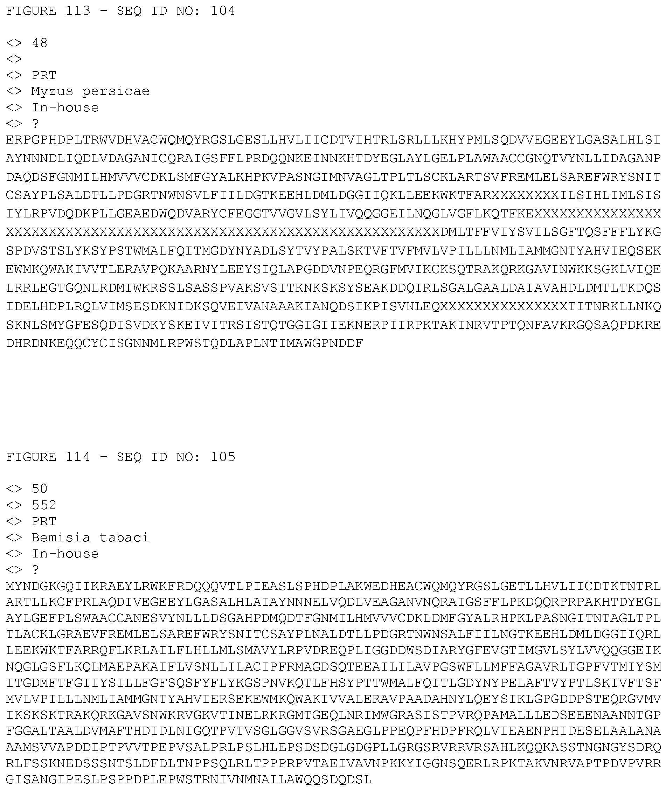

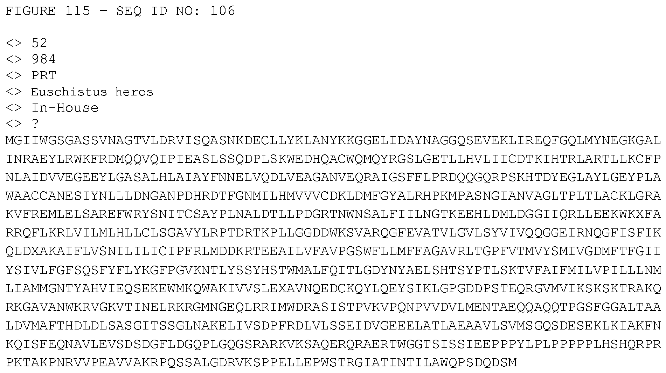

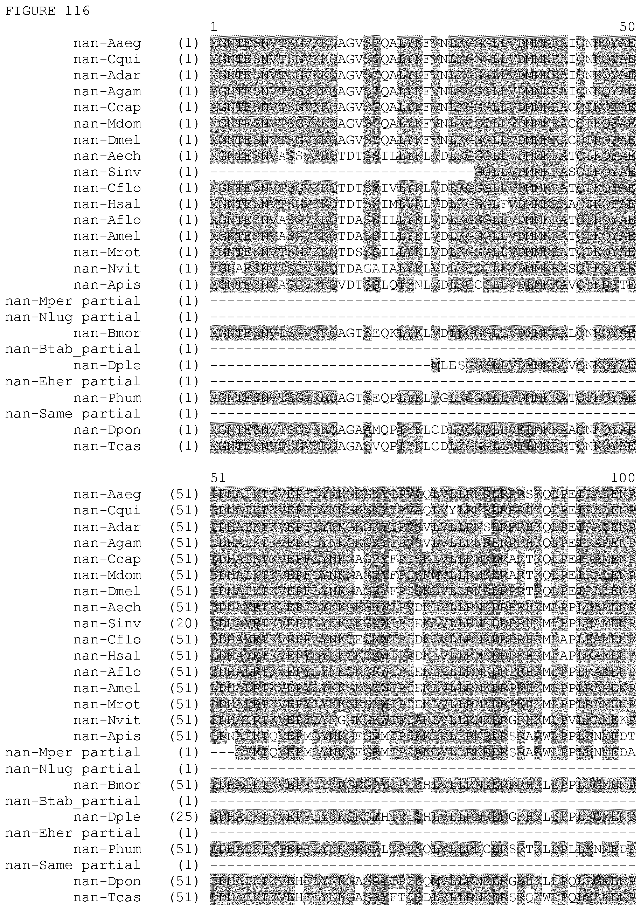

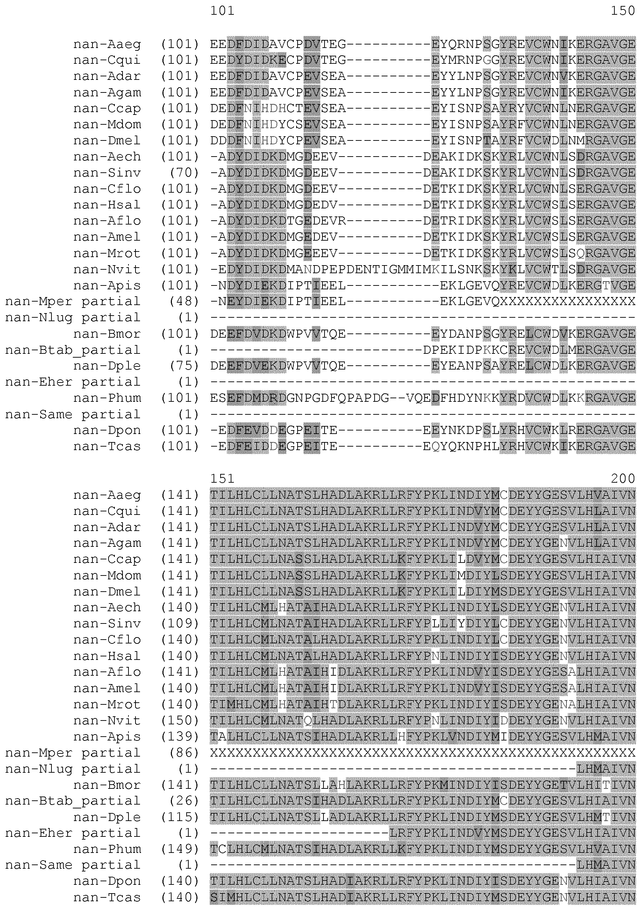

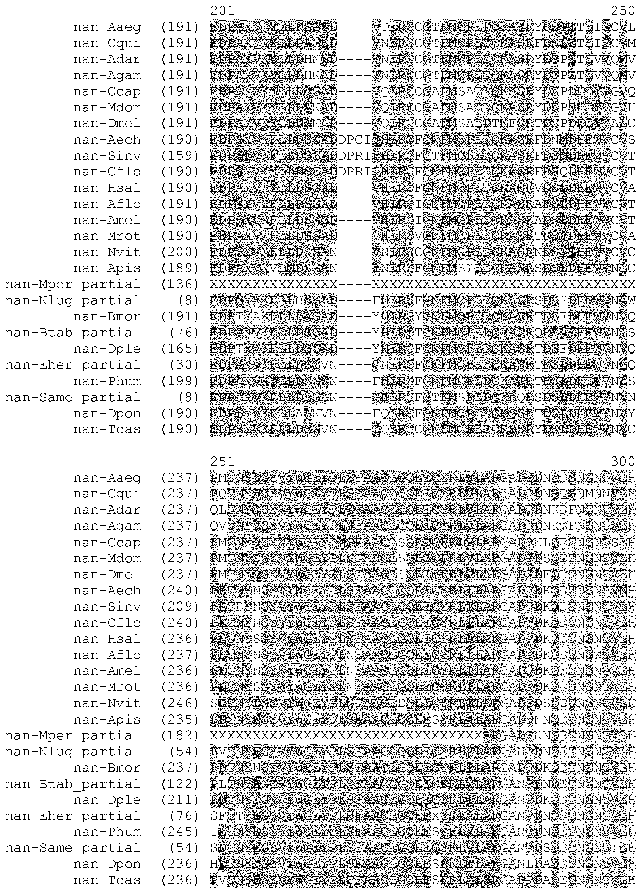

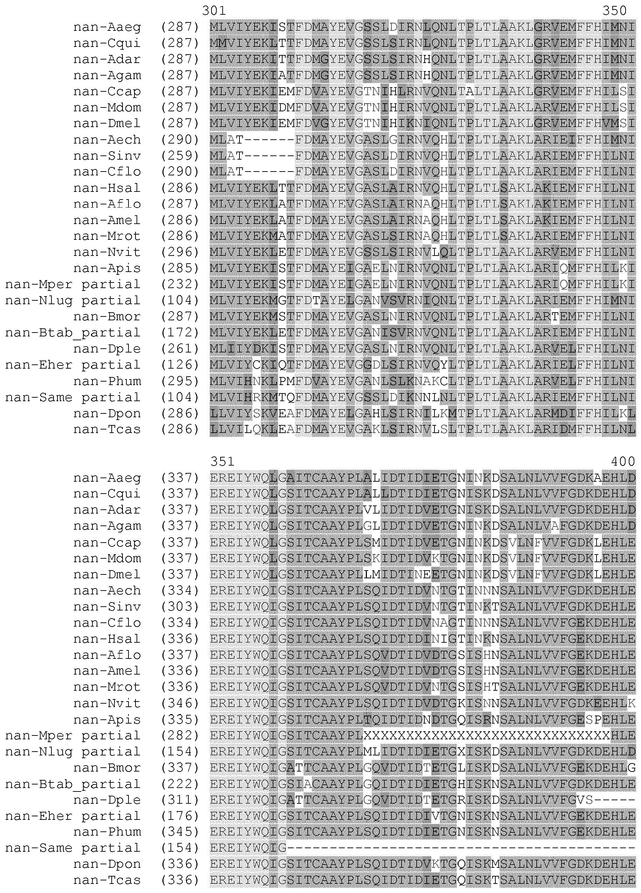

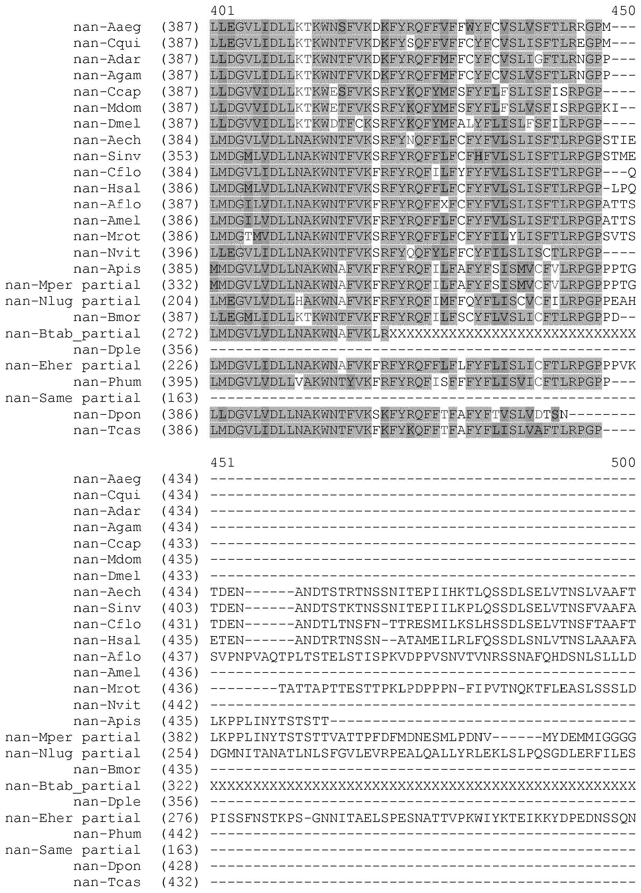

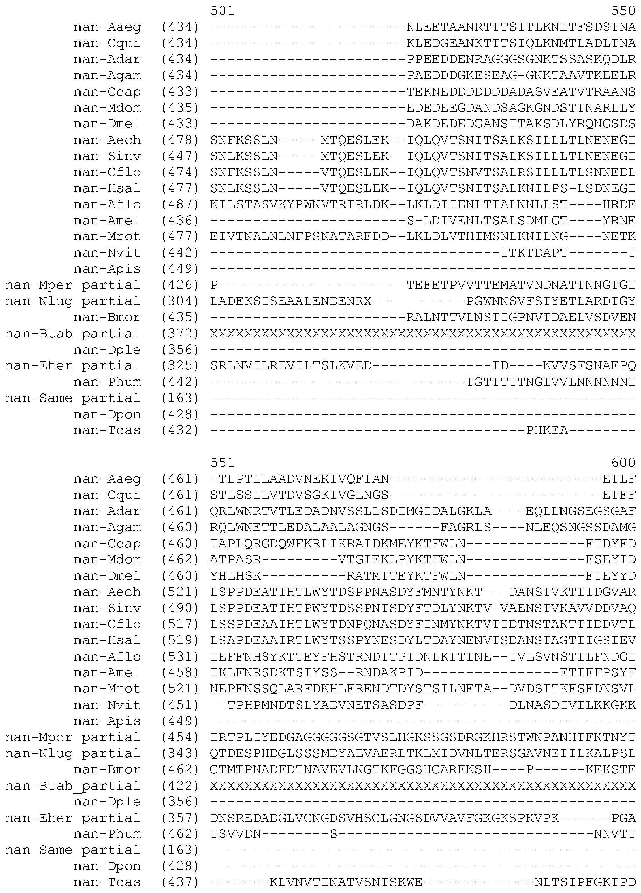

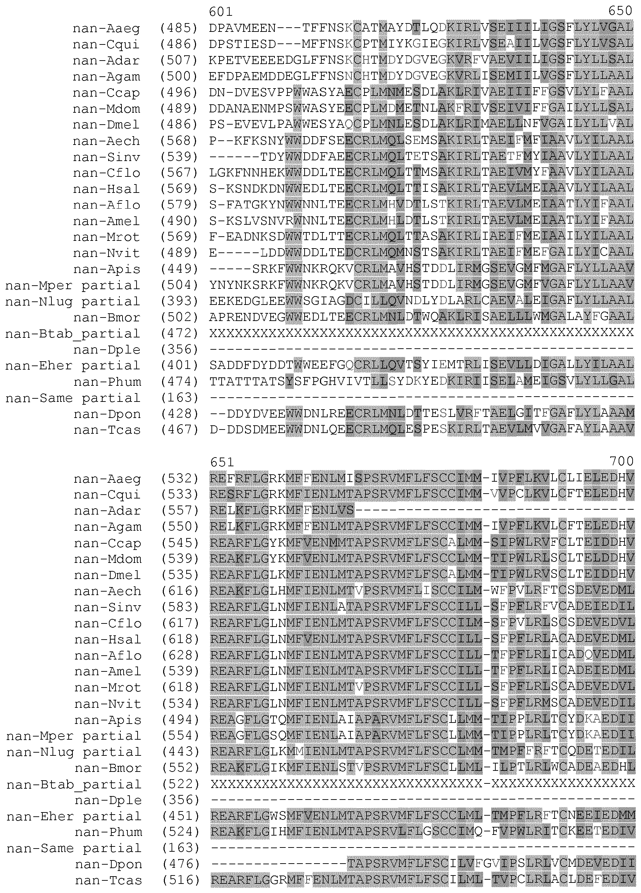

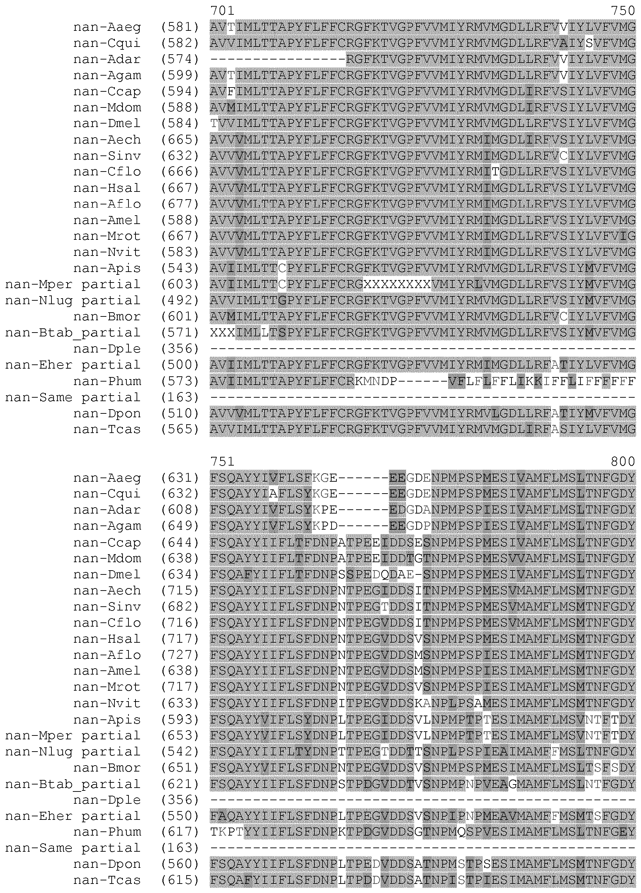

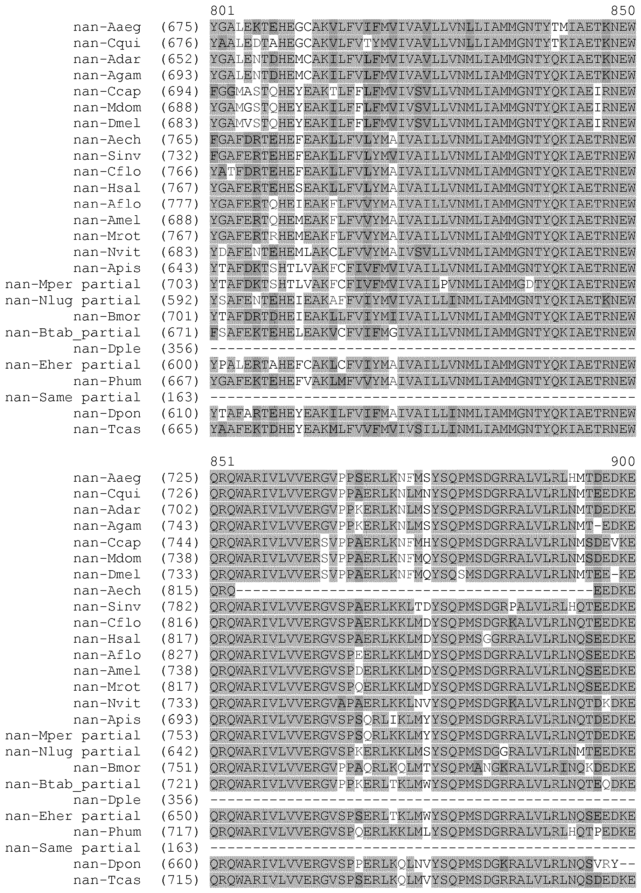

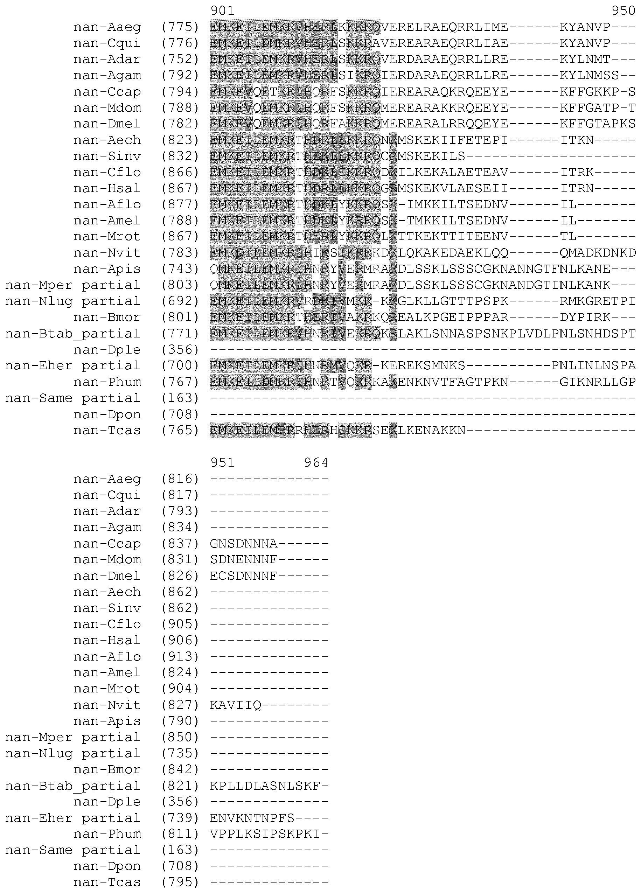

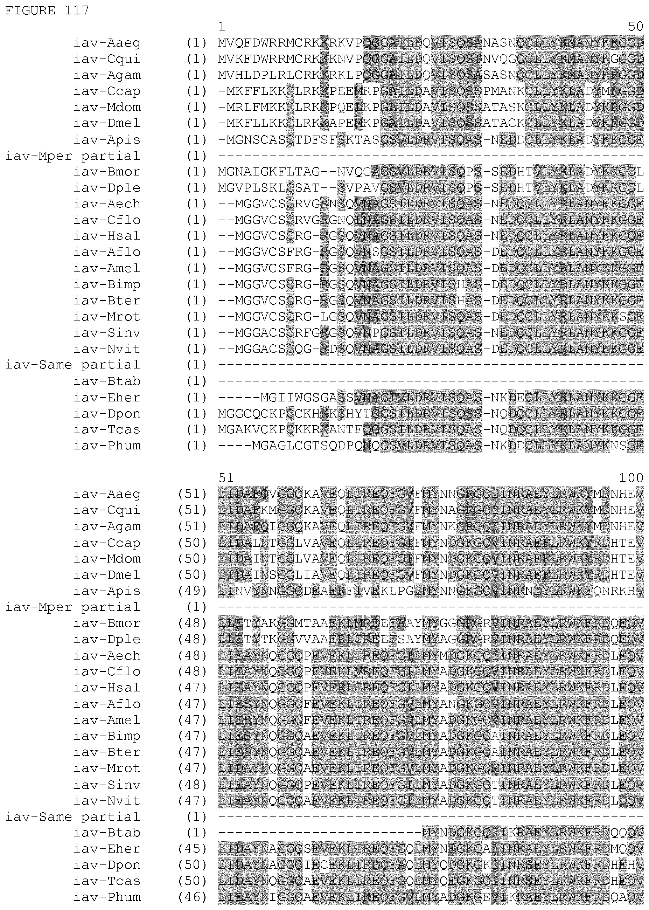

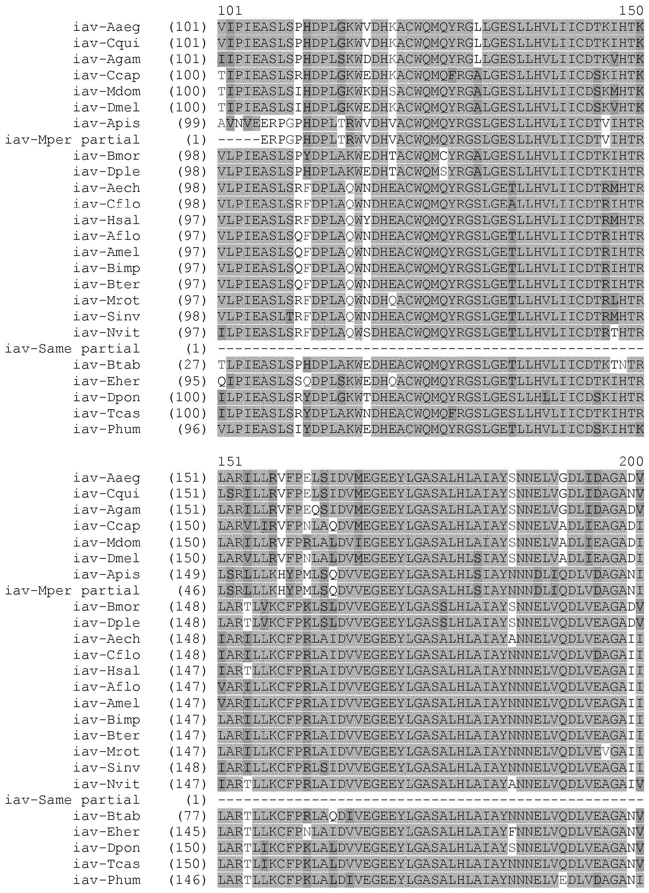

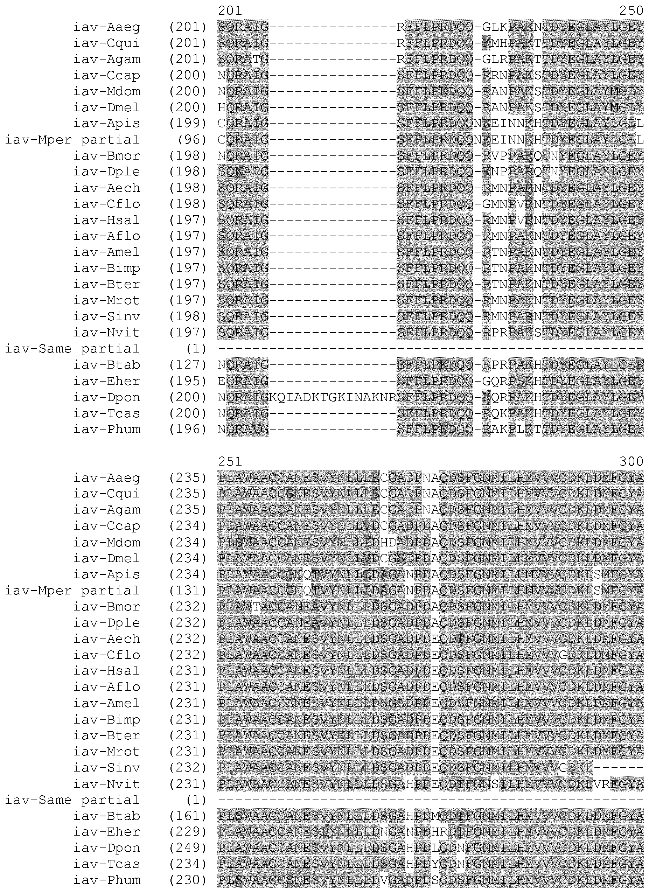

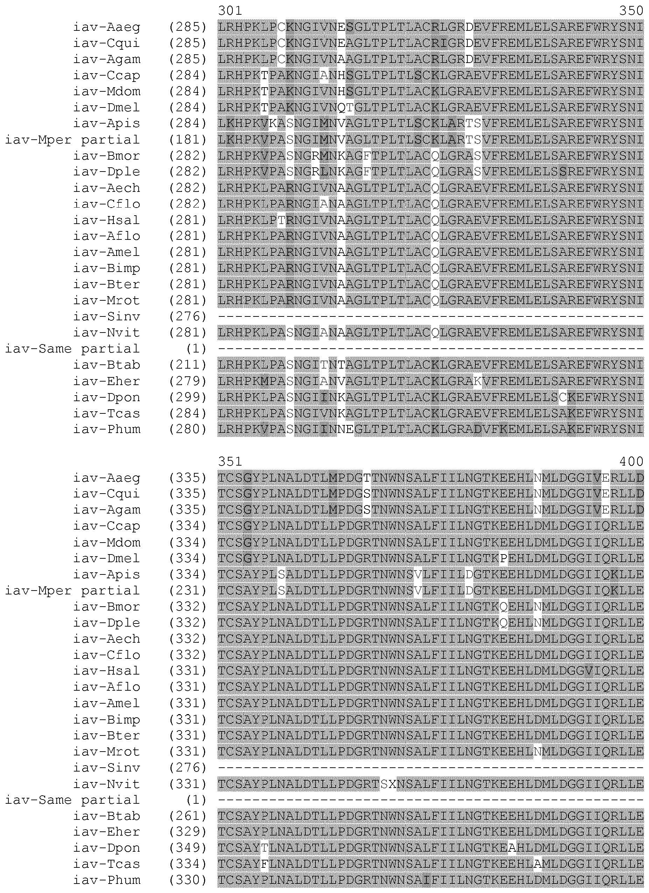

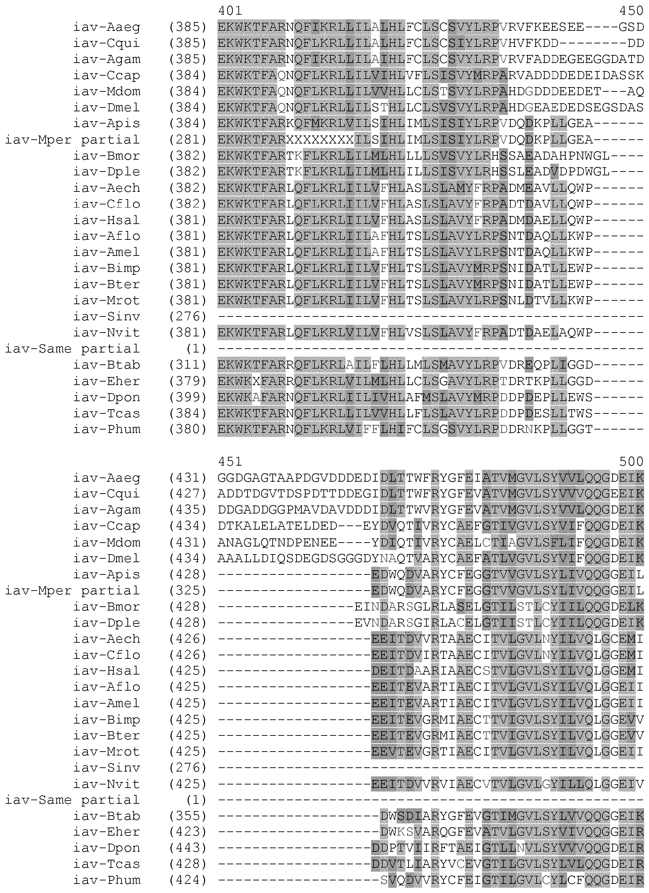

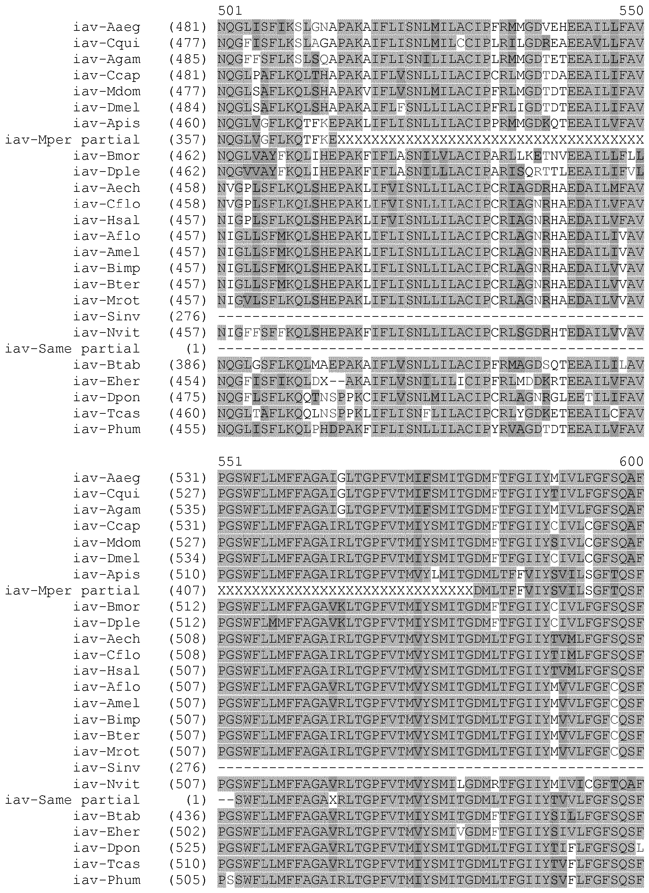

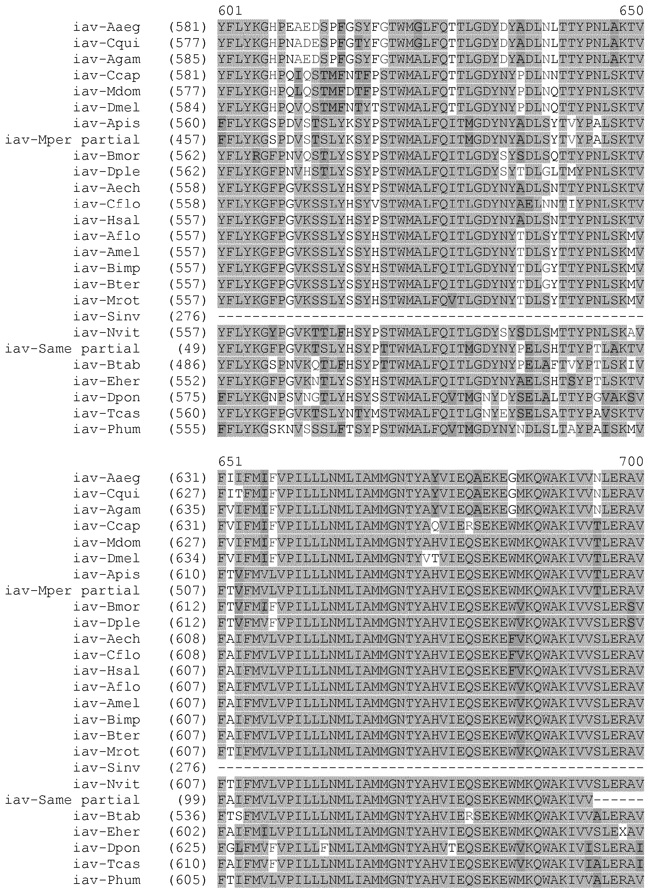

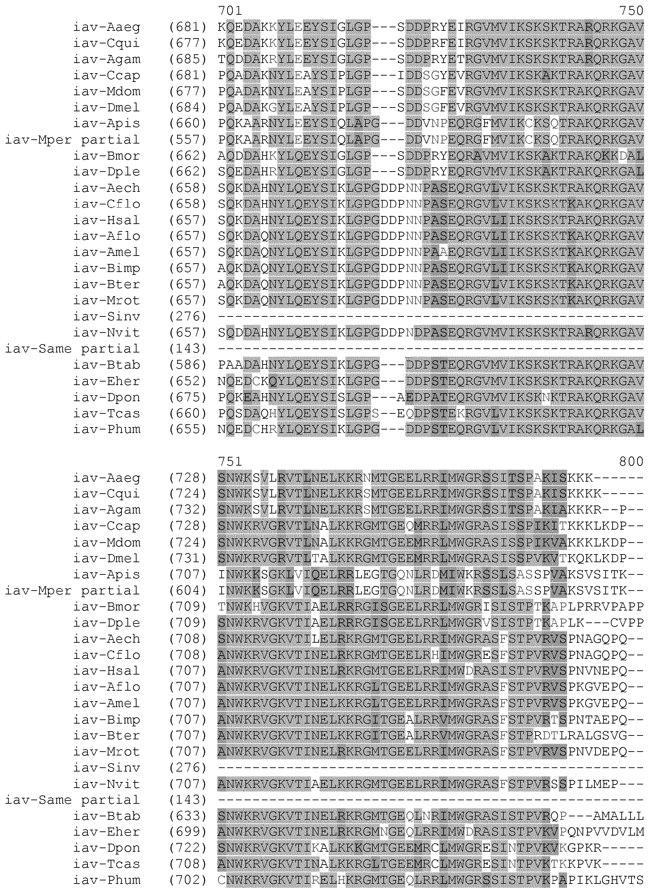

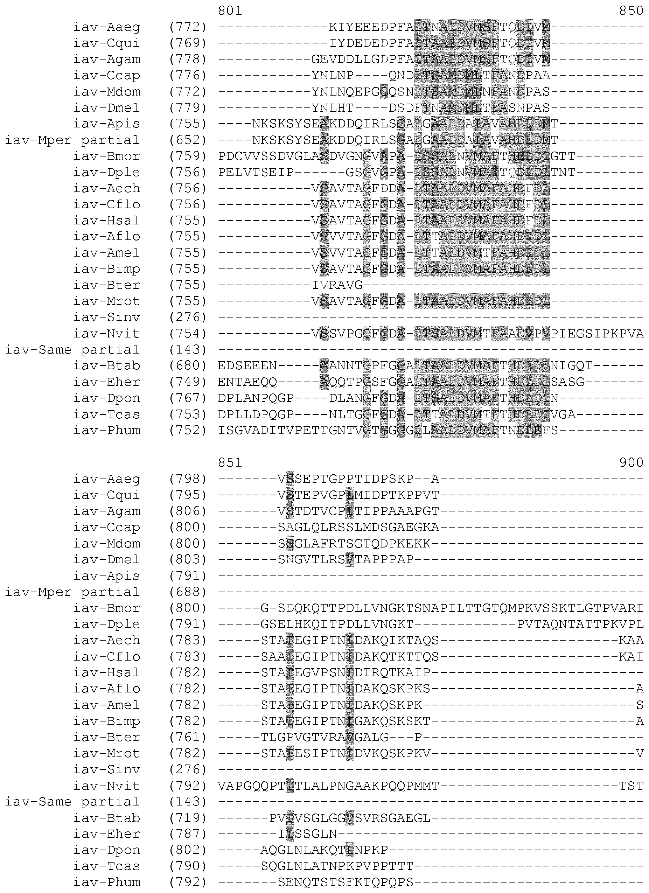

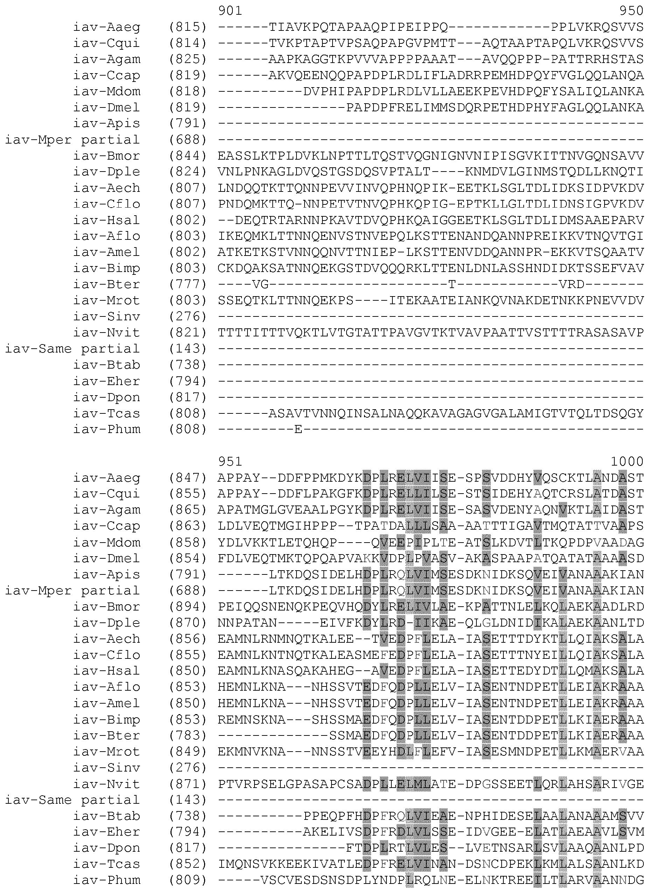

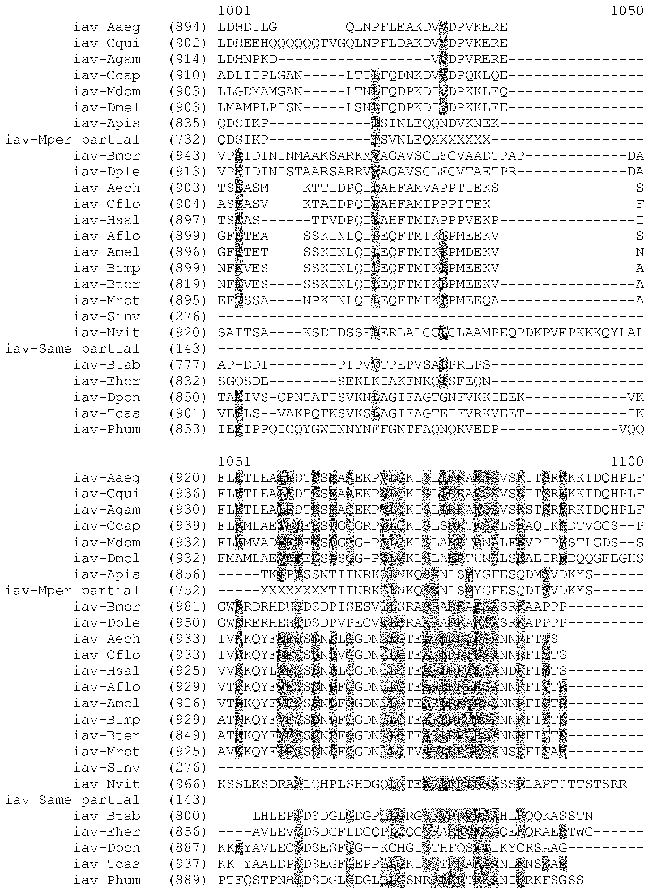

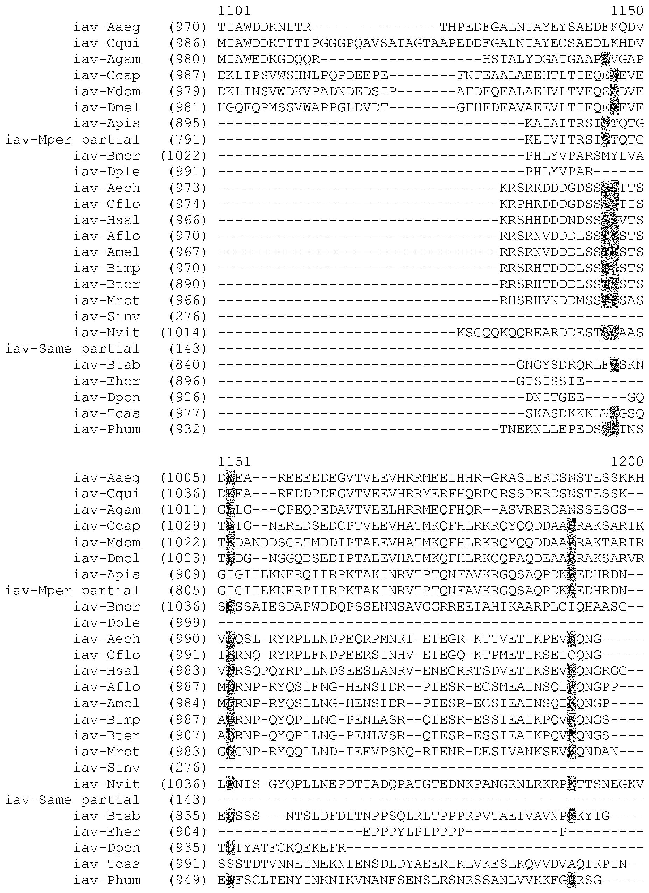

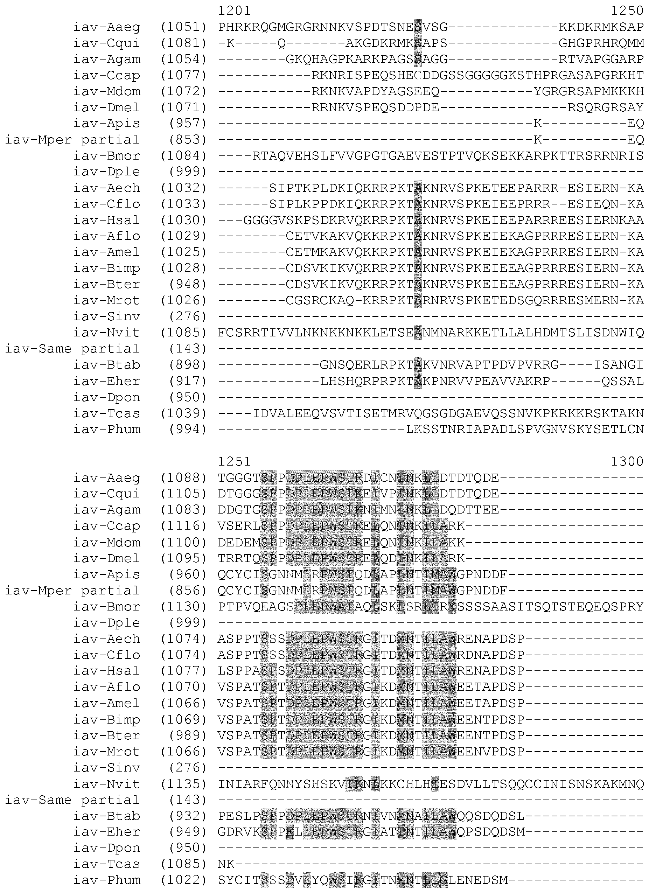

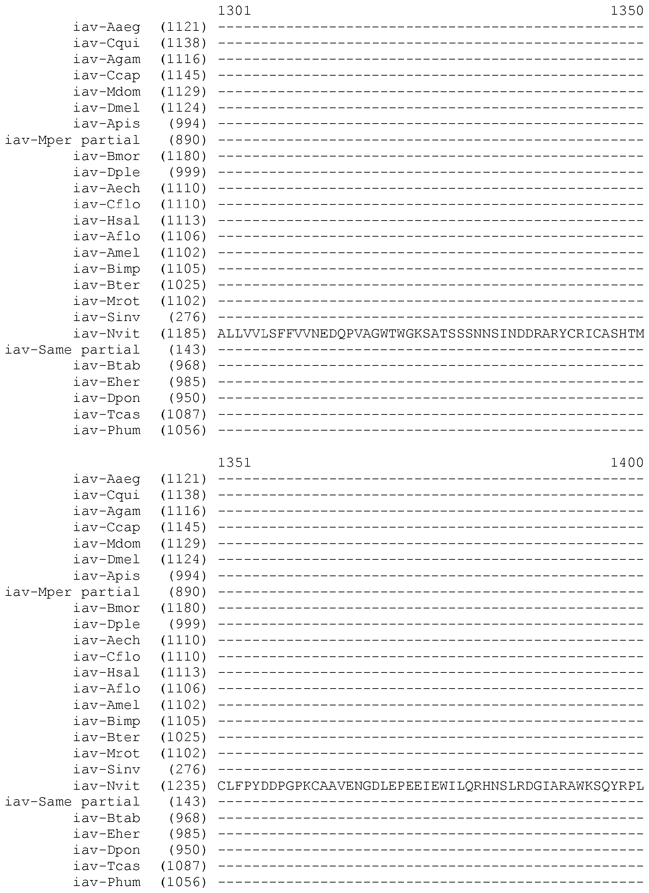

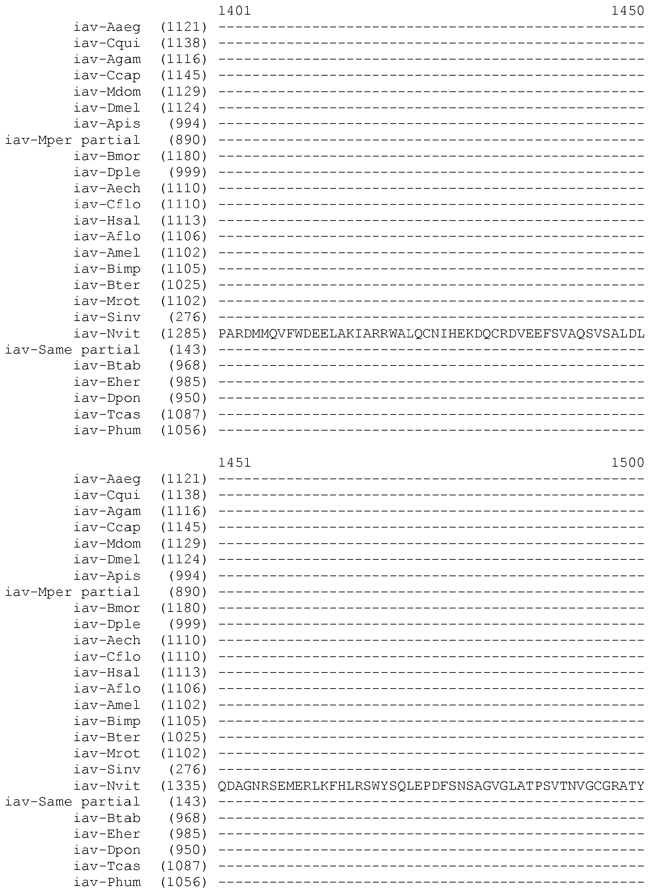

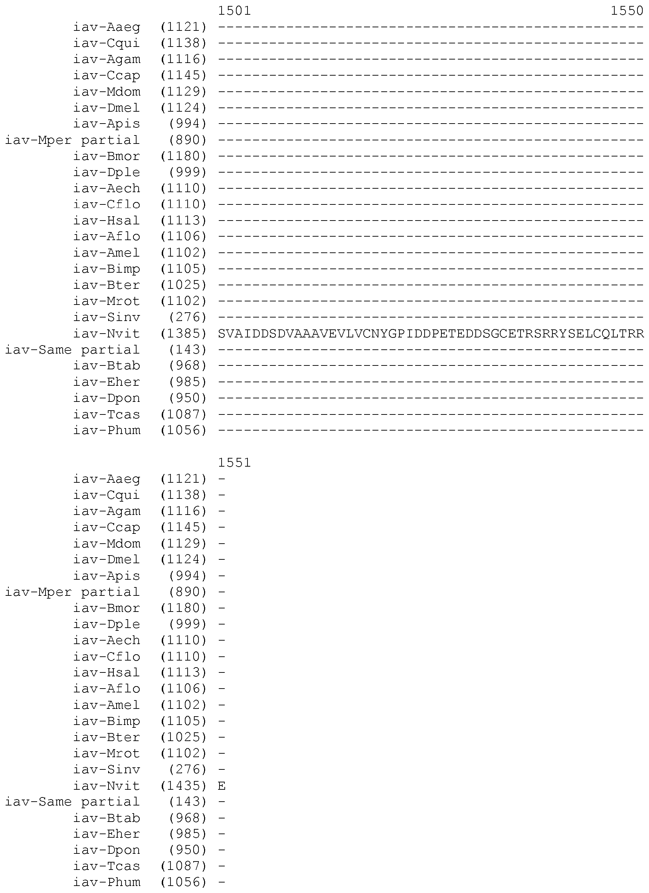

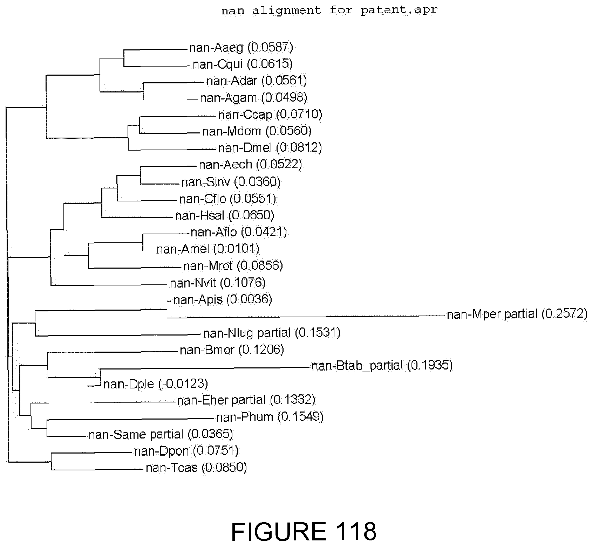

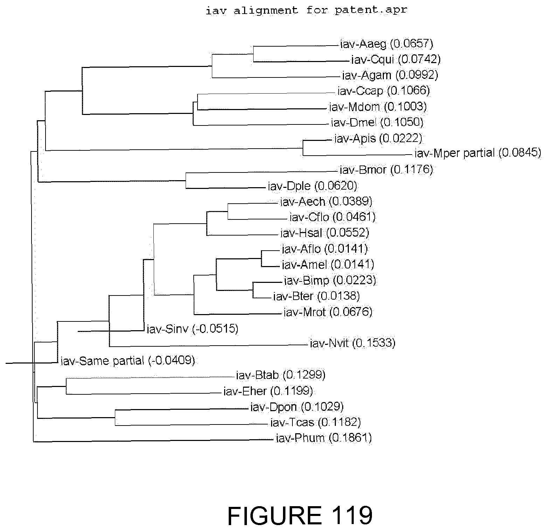

In certain embodiments, the Nanchung protein sequences may be selected from the group consisting of a) a polypeptide comprising an amino acid sequence having a sequence selected from the group consisting of SEQ ID NOS: 54-80 (FIGS. 37-63); b) a polypeptide having at least 70% sequence identity to the SEQ ID NOS: 54-80 (FIGS. 37-63); and c) a fragment or a conservative variant of the polypeptide of a) or b).
In certain embodiments, the Inactive protein sequences may be selected from the group consisting of a) a polypeptide comprising an amino acid sequence having a sequence selected from the group consisting of SEQ ID NOS: 81-106 (FIGS. 90-115); b) a polypeptide having at least 70% sequence identity to the SEQ ID NOS: 81-106 (FIGS. 90-115); and c) a fragment or a conservative variant of the polypeptide of a) or b).
The sequence alignment of the Nanchung and Inactive proteins is shown in Figures 116 and 117, respectively. The family distribution tree for the Nanchung and Inactive proteins is shown in Figures 118 and 119, respectively.
As used herein, the "percent sequence identity" is determined by comparing two optimally aligned sequences over a comparison window, where the portion of the polynucleotide or a polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, divided by the number of matched positions by the total number of positions in the window of comparison and multiplying the result by 100 to yield the percentage of sequence identity. Alignment for the purposes of determining the percentage identity can be achieved in various ways that are within the skill in the art, for example, using publicly available computer software such as BLAST. Those skilled in the art can determine appropriate parameters for measuring alignment, including any algorithms needed to achieve optimal alignment over the full length of the sequences being compared.
As used herein, the term a "conservative variant" refers to an amino acid sequence in which a first amino acid is replaced by a second amino acid or amino acid analog having at least one similar biochemical property, which can be, for example, similar size, charge, hydrophobicity or hydrogen bonding capacity. For example, a first hydrophobic amino acid can be conservatively substituted with a second (non-identical) hydrophobic amino acid such as alanine, valine, leucine, or isoleucine, or an analog thereof. Similarly, a first basic amino acid can be conservatively substituted with a second (non-identical) basic amino acid such as arginine or lysine, or an analog thereof. In the same way, a first acidic amino acid can be conservatively substituted with a second (non-identical) acidic amino acid such as aspartic acid or glutamic acid, or an analog thereof or an aromatic amino acid such as phenylalanine can be conservatively substituted with a second aromatic amino acid or amino acid analog, for example tyrosine. In some embodiments, the peptide comprises conservative variant substitution of at least one amino acid, e.g. 1, 2, 3, 4, 5, 6, 7, 8, 9, 10 or more amino acids. Typically, a conservative variant will retain at least 50%, 55%, 60%, 65%, 70%, 75%, 80%, 85%, 90%, 95%, 97%, 98%, 99% or more of the activity of the wild-type peptide sequence.
Exemplary conservative variant substitution include, but are not limited to, replacement of Alanine (A) with D-ala, Gly, Aib, β-Ala, Acp, L-Cys, or D-Cys; Arginine (R) with D-Arg, Lys, D-Lys, homo-Arg, D-homo-Arg, Met, Be, D-Met, or D-Ile; Asparagine (N) with D-Asn, Asp, D-Asp, Glu, D-Glu, Gln, or D-Gln; Aspartic acid (D) with D-Asp, D-Asn, Asn, Glu, D-Glu, Gln, or D-Gln; Cysteine (C) with D-Cys, S-Me-Cys, Met, D-Met, Thr, or D-Thr; Glutamine (Q) with D-Gln, Asn, D-Asn, Glu, D-Glu, Asp, or D-Asp; Glutamic Acid (E) with D-Glu, D-Asp, Asp, Asn, D-Asn, Gln, or D-Gln; Glycine (G) with Ala, D-Ala, Pro, D-Pro, Aib, β-Ala, or Acp; Isoleucine (I) with D-Ile, Val, D-Val, AdaA, AdaG, Leu, D-Leu, Met, or D-Met; Leucine (L) with D-Leu, Val, D-Val, AdaA, AdaG, Leu, D-Leu, Met, or D-Met; Lysine (K) with D-Lys, Arg, D-Arg, homo-Arg, D-homo-Arg, Met, D-Met, Ile, D-Ile, Orn, or D-Orn; Methionine (M) with D-Met, S-Me-Cys, Be, D-Ile, Leu, D-Leu, Val, or D-Val; Phenylalanine (F) with D-Phe, Tyr, D-Thr, L-Dopa, His, D-His, Trp, D-Trp, Trans-3,4 or 5-phenylproline, AdaA, AdaG, cis-3, 4 or 5-phenylproline, Bpa, or D-Bpa; Proline (P) with D-Pro, L-I-thioazolidine-4-carboxylic acid, or D- or -L-1-oxazolidine-4-carboxylic acid (U.S. Pat. No. 4,511,390); Serine (S) with D-Ser, Thr, D-Thr, allo-Thr, Met, D-Met, Met (O), D-Met (O), L-Cys, or D-Cys; Threonine (T) with D-Thr, Ser, D-Ser, allo-Thr, Met, D-Met, Met (O), D-Met (O), Val, or D-Val; Tyrosine (Y) with D-Tyr, Phe, D-Phe, L-Dopa, His, or D-His; and Valine (V) with D-Val, Leu, D-Leu, Ile, D-Ile, Met, D-Met, AdaA, or AdaG.
Conservative variants of the insect or mammalian TRPV channels can be prepared according to methods for altering peptide sequences known in the art, and include those that may be found in references, which compile such methods, e.g., Molecular Cloning: A Laboratory Manual, J. Sambrook, et al., eds., Second Edition, Cold Spring Harbor, or Current Protocols in Molecular Biology, F. M. Ausubel, et al., eds., John Wiley & Sons, Inc. New York.
Conservative variants of TRPV and/or TRP channel may also be made by alteration of a nucleic acid encoding the TRPV and/or TRP polypeptide.
In certain embodiments, the screening method of the present disclosure may be a high-throughput screening. High-throughput screening (HTS) refers to a method for scientific experimentation that uses robotics, data processing and control software, liquid handling devices, and sensitive detectors. High-Throughput Screening or HTS allows a researcher to quickly conduct millions of biochemical, genetic or pharmacological tests. HTS is well known in the art, including, for example, U.S. Pat. Nos. 5,976,813; 6,472,144; 6,692,856; 6,824,982; and 7,091,048.

HTS uses automation to run a screen of an assay against a library of candidate compounds. An assay is a test for specific activity: usually inhibition or stimulation of a biochemical or biological mechanism. Specifically, an assay would be screening for inhibition or stimulation of the insect TRPV channel. Typical HTS screening libraries or "decks" can contain from 100,000 to more than 2,000,000 compounds.

The key labware or testing vessel of HTS is the microtiter plate, which is a small container, usually disposable and made of plastic that features a grid of small, open divots called wells. Modern microplates for HTS generally have either 384, 1536, or 3456 wells. These are all multiples of 96, reflecting the original 96 well microplate with 8×129 mm spaced wells.

To prepare for an assay, a researcher fills each well of the plate with the appropriate reagents that he or she wishes to conduct the experiment with. After some incubation time has passed to allow the reagent to absorb, bind to, or otherwise react (or fail to react) with the compounds in the wells, measurements are taken across all the plate's wells, either manually or by a machine. Manual measurements are often necessary when the researcher is using microscopy to (for example) seek changes that a computer could not easily determine by itself. Otherwise, a specialized automated analysis machine can run a number of experiments on the wells such as current or voltage measurements, colorimetric measurements, radioactivity counting, etc. In this case, the machine outputs the result of each experiment as a grid of numeric values, with each number mapping to the value obtained from a single well. A high-capacity analysis machine can measure dozens of plates in the space of a few minutes like this, generating thousands of experimental data points very quickly.

### IV. Formulations, Applications and Uses

In another aspect, the disclosure provides a compound selected by the screening assay described herein. It is to be understood that analogs, derivatives, isomers, and pharmaceutically acceptable salts of the compounds selected by the screening assays described herein as well as any formulations including the selected compound are also included herein.

The compound or group of compounds being determined or identified by the method according to the disclosure can be used in methods of insect control, e.g., by modulating insect feeding behavior The identified compounds, analogs, derivatives, isomers, and pharmaceutically acceptable salts thereof are also referred to as "active agents" or "active ingredients" herein.

In some embodiments, the compound modulates the feeding behavior of an insect. As used herein, the term "feeding behavior" refers to the process by which organisms, such as insects obtain food. Without being bound by theory, the modulation of an insect TRPV elicits a signaling pathway that brings forth motor neuron modulation which may decrease feeding behavior of an insect, and such modulation of an insect TRPV channel in an insect may lead to a decrease in feeding behavior of such insect leading to the insect's death by starvation. Thus, again without being bound by theory, it is believed that compounds that modulate (e.g., activate or inhibit) an insect TRPV channel may be used as insecticides. This decrease in feeding behavior can be used to destroy insects in a particular location and thus control such insects. Thus, in some embodiments, the method comprises modulation of TRPV ion channel or family members in the insect with a compound identified by a screening method described herein.

Accordingly, in certain embodiments, the disclosure provides a method of insect control by modulating feeding behavior in an insect using a compound identified by the screening methods described herein. As used in context of methods of insect control, compounds identified by the screening methods described herein also include analogs, derivatives, isomers and pharmaceutically acceptable salts of such compounds.

In one embodiment, the methods and active agents described herein are applicable to insects that are agricultural or horticultural pest.

Examples of agricultural pests include, but are not limited to,Aphids (including *Aphis fabae, Aphis gossypii, Aphis pomi, Aulacorthum solani, Brevicoryne brassicae, Dysaphis plantaginea, Macrosiphum euphorbiae, Macrosiphum euphorbiae, Macrosiphum rosae, Myzus persicae, Nasonovia ribisnigri);* Whiteflies (including *Aleurodes spp., Bemisia spp., Dialeurodes spp., Trialeurodes spp.);* Planthoppers (including *Laodelphax striatellus, Nilaparvata lugens, Siphanta spp., Sogatella furcifera);* Leafhoppers (including *Amrasca spp., Empoasca spp.);* Scales (including *Aspidiotus spp., Chrysomphalus aonidum, Icerya purchase, Unaspis citri);* Mealybugs (including *Maconellicoccus spp., Paracoccus spp., Planococcus spp.);* Pollen beetles (including *Carpophilus spp., Meligethes spp.);* Thrips (including *Caliothrips spp., Frankliniella spp., Scirtothrips spp., Thrips spp.);* and Psyllids (including *Bactericera spp., Cacopsylla spp., Diaphorina citri, Paratrioza cockerelli).*

For example, vegetable and cole crops are sensitive to infestation by one or more of the following insect pests: aphids, brown plant hopper, alfalfa looper, armyworm, beet armyworm, artichoke plume moth, cabbage budworm, cabbage looper, cabbage webworm, corn earworm, celery leafeater, cross-striped cabbagewon, european corn borer, diamondback moth, green cloverworm, imported cabbageworm, melonworm, omnivorous leafroller, pickleworm, rindworm complex, saltmarsh caterpillar, soybean looper, tobacco budworm, tomato fruitworm, tomato homworrri, tomato pinworm, velvetbean caterpillar, and yellow-striped annyworm.

Likewise, pasture and hay crops such as alfalfa, pasture grasses and silage are often attacked by pests, such as annyworm, beef annyworm, alfalfa caterpillar, European skipper, a variety of loopers and webworms, as well as yellowstriped armyworms.

Fruit and vine crops are often susceptible to attack and defoliation by achema sphinx moth, amorbia, armyworm, citrus cutworm, banana skipper, blackheaded fireworm, blueberry leafroller, cankerworm, cherry fruitworm, citrus cutworm, cranberry girdler, eastern tent caterpillar, fall webworm, fall webworm, filbert leafroller, filbert webworm, fruit tree leafroller, grape berry moth, grape leaffolder, grapeleaf skeletonizer, green fruitworm, gummosos-batrachedra commosae, gypsy moth, hickory shuckworm, hornworms, loopers, navel orangeworm, obliquebanded leafroller, omnivorous leafroller, omnivorous looper, orange tortrix, orangedog, oriental fruit moth, pandemis leafroller, peach twig borer, pecan nut casebearer, redbanded leafroller, redhumped caterpillar, roughskinned cutworm, saltmarsh caterpillar, spanworm, tent caterpillar, thecla-thecla basillides, tobacco budworm, tortrix moth, tufted apple budmoth, variegated leafroller, walnut caterpillar, western tent caterpillar, and yellowstriped annyworm.

Field crops such as canola/rape seed, evening primrose, meadow foam, corn (field, sweet, popcorn), cotton, hops, jojoba, peanuts, rice, safflower, small grains (barley, oats, rye, wheat, etc.), sorghum, soybeans, sunflowers, and tobacco are often targets for infestation by insects, including: armyworm, asian and other corn borers, banded sunflower moth, beet annyworm, bollworm, cabbage looper, corn rootworm (including southern and western varieties), cotton leaf perforator, diamondback moth, european corn borer, green cloverworm, headmoth, headworm, imported cabbagewonn, loopers (including Anacamptodes spp.), obliquebanded leafroller, omnivorous leaftier, podworin, podworm, saltmarsh caterpillar, southwestern corn borer, soybean looper, spotted cutworm, sunflower moth, tobacco budworm, tobacco hornworm, velvetbean caterpillar, Bedding plants, flowers, ornamentals, vegetables and container stock are frequently fed upon by a host of insect pests such as armyworm, azalea moth, beet armyworm, diamondback moth, ello moth (hornworm), Florida fern caterpillar, lo moth, loopers, oleander moth, omnivorous leafroller, omnivorous looper, and tobacco Forests, fruit, ornamental, and nut-bearing trees, as well as shrubs and other nursery stock are often susceptible to attack from diverse insects such as bagworm, blackheaded budworm, browntail moth, california oakworm, douglas fir tussock moth, elm spanworm, fall webworm, fruit tree leafroller, greenstriped mapleworm, gypsy moth, Jack pine budworm, mimosa webworm, pine butterfly, redhumped caterpillar, saddleback caterpillar, saddle prominent caterpillar, spring and fall cankerworm, spruce budworm, tent caterpillar, tortrix, and western tussock moth.

Likewise, turf grasses are often attacked by pests such as armyworm, sod webworm, and tropical sod webworm.

It is also envisioned that the methods described herein are also applicable to pest control, wherein the pests are not insects but rather, e.g., nematodes, slugs or snails.

In one embodiment, the methods described herein are also applicable to insects that are parasites. Examples of some insect parasites are Braconid Wasps, family Braconidae; Ichneumonid Wasps, family Ichneumonidae; Chalcid Wasps, family Chalcidae; Tachinid Flies, family Tachonidae.

In certain other embodiments, the methods described herein may also be applicable to insects that are disease vectors. Vectors are organisms that can introduce a pathogen such as a bacterium or virus into a host organism to cause an infection or disease. Exemplary disease vector include, but are not limited to, mosquitoes, Ticks, Siphonaptera (fleas), Diptera (flies), Phthiraptera (lice) and Hemiptera (true bugs).

Once a compound suitable for insecticidal use is identified, the active ingredient, or formulations comprising them, may be applied directly to the target insects (i.e., larvae, pupae and/or adults), or to the locus of the insects. In one embodiment, the active ingredient or a formulation and/or composition containing the active ingredient is applied directly to the adult insect. In one embodiment, the active agent is applied directly to the larvae and/or pupae of the target insect. In another embodiment, the active ingredient is applied to the locus of the insects.

Because compounds incorporating hydrophobic moieties will penetrate the insect cuticle, active agents can be conjugated with hydrophobic moieties. Hydrophobic moieties include, but are not limited to, lipids and sterols.

In one embodiment, the active ingredient or a formulation including the active ingredient may be applied as a spray. For example, the active ingredient may be applied as an agricultural spray in aerial crop dusting, an environmental spray to control biting insects, or as a topical spray for localized control of biting insects. The active ingredient may be formulated for the purpose for spray application such as an aerosol formulation. Spray application may be accomplished with a spray pump. The active ingredient may be also encapsulated within materials such as starch, flour and gluten in granular formulations.

In certain embodiments, the active ingredient or a formulation including the active ingredient may be applied in conjunction with other insecticides and/or pesticides such as organo-phosphates, synthetic pyrethroids, carbamates, chlorinated hydrocarbons, when used in agricultural and/or environmental insect control.

The active ingredient may be administered in an amount effective to induce the desired response as determined by routine testing. The actual effective amount will of course vary with the specific active ingredient, the target insect and its stage of development, the application technique, the desired effect, and the duration of the effect, and may be readily determined by the practitioner skilled in the art. "An effective amount of active ingredient" refers to the amount of active ingredient that modulates (activates or inhibits) an insect TRPV channel, e.g., modulates feeding behavior of an insect to achieve the desired insect control.

Methods of formulation are well known to one skilled in the art and are also found in Knowles, D A (1998) Chemistry and technology of agricultural formulations. Kluwer Academic, London,. One skilled in the art will, of course, recognize that the formulation and mode of application may affect the activity of the active ingredient in a given application. Thus, for agricultural and/or horticultural use the TRPV inhibitors and/or agonists may be formulated as a granular of relatively large particle size (for example, 8/16 or 4/8 US Mesh), as water-soluble or water-dispersible granules, as powdery dusts, as wettable powders, as emulsifiable concentrates, as aqueous emulsions, as solutions, as suspension concentrate, as capsule suspensions, as soluble (liquid) concentrates, as soluble powders, or as any of other known types of agriculturally-useful formulations, depending on the desired mode of application. It is to be understood that the amounts specified in this specification are intended to be approximate only, as if the word "about" were placed in front of the amounts specified.

These formulations may be applied either as water-diluted sprays, or dusts, or granules in the areas in which insect control is desired. The formulations may contain as little as 0.1%, 0.2% or 0.5% to as much as 95% or more by weight of active ingredient, e.g. insect TRPV inhibitor.

"Dusts" are free flowing admixtures of the active ingredient with finely divided solids such as talc, natural clays, kieselguhr, flours such as walnut shell and cottonseed flours, and other organic and inorganic solids which act as dispersants and carriers for the toxicant; these finely divided solids have an average particle size of less than about 50 microns. A typical dust formulation useful herein is one containing 90 parts, 80 parts, 70 parts, 60 parts, 50 parts, 40 parts, 30 parts, 20 parts, preferably 10 parts, or less of the active ingredient, e.g., insect TRPV inhibitor or insect TRPV agonist. In one embodiment, the dust formulation may include 1 part or less of the active ingredient and 99 parts or more of talc.

Wettable powders, useful as formulations, are in the form of finely divided particles that disperse readily in water or other dispersant. The wettable powder is ultimately applied to the locus where insect control is needed either as a dry dust or as an emulsion in water or other liquid. Typical carriers for wettable powders include Fuller's earth, kaolin clays, silicas, and other highly absorbent, readily wet inorganic diluents. Wettable powders typically are prepared to contain about 5-80% of active ingredient, depending on the absorbency of the carrier, and usually also contain a small amount of a wetting, dispersing or emulsifying agent to facilitate dispersion. For example, a useful wettable powder formulation contains 80.0 parts of the active ingredient, 17.9 parts of Palmetto clay, and 1.0 part of sodium lignosulfonate and 0.3 part of sulfonated aliphatic polyester as wetting agents. Additional wetting agent and/or oil may be added to a tank mix for to facilitate dispersion on the foliage of the plant.

Other useful formulations are emulsifiable concentrates (ECs) which are homogeneous liquid compositions dispersible in water or other dispersant, and may consist entirely of the active ingredient, and a liquid or solid emulsifying agent, or may also contain a liquid carrier, such as xylene, heavy aromatic naphthas, isophorone, or other non-volatile organic solvents. For insecticidal application these concentrates are dispersed in water or other liquid carrier and normally applied as a spray to the area to be treated. The percentage by weight of the essential active ingredient may vary according to the manner in which the composition is to be applied, but in general comprises 0.5 to 95% of active ingredient by weight of the insecticidal composition.

Flowable formulations are similar to ECs, except that the active ingredient is suspended in a liquid carrier, generally water. Flowables, like ECs, may include a small amount of a surfactant, and will typically contain active ingredients in the range of 0.5 to 95%, frequently from 10 to 50%, by weight of the composition. For insecticidal application, flowables may be diluted in water or other liquid vehicle, and are typically applied as a spray.

Typical wetting, dispersing or emulsifying agents used in agricultural and/or horticultural formulations may include, but are not limited to, the alkyl and alkylaryl sulfonates and sulfates and their sodium salts; alkylaryl polyether alcohols; sulfated higher alcohols; polyethylene oxides; sulfonated animal and vegetable oils; sulfonated petroleum oils; fatty acid esters of polyhydric alcohols and the ethylene oxide addition products of such esters; and the addition product of long-chain mercaptans and ethylene oxide. Many other types of useful surface-active agents are available in commerce. Surface-active agents, when used, typically include 1 to 15% by weight of the composition.

Other useful formulations include suspensions of the active ingredient in a relatively non-volatile solvent such as water, corn oil, kerosene, propylene glycol, or other suitable solvents.

In certain embodiments, formulations for insecticidal applications may include simple solutions of the active ingredient in a solvent, in which it is completely soluble at the desired concentration, such as acetone, alkylated naphthalenes, xylene, or other organic solvents.

Granular formulations, wherein the active ingredient is carried on relative coarse particles, are of particular utility for aerial distribution or for penetration of cover crop canopy. Pressurized sprays, typically aerosols wherein the active ingredient is dispersed in finely divided form as a result of vaporization of a low-boiling dispersant solvent carrier may also be used. Water-soluble or water-dispersible granules are free flowing, non-dusty, and readily water-soluble or water-miscible. In use by the farmer on the field, the granular formulations, emulsifiable concentrates, flowable concentrates, aqueous emulsions, solutions, etc., may be diluted with water to give a concentration of active ingredient in the range of say 0.1% or 0.2% to 1.5% or 2%.

By far the most frequently used are water-miscible formulations for mixing with water then applying as sprays. Water miscible, older formulations include: emulsifiable concentrate, wettable powder, soluble (liquid) concentrate, and soluble powder. Newer, non-powdery formulations with reduced or no hazardous solvents and improved stability include: suspension concentrate, capsule suspensions, and water dispersible granules. Such formulations are preferably solutions and suspension, e.g., aqueous suspension and solutions, ethanolic suspension and solutions, aqueous/ethanolic suspension and solutions, saline solutions, and colloidal suspensions.

Alternatively, a sprayable wax emulsion formulation may be used. The formulation contains the active ingredient, in an amount from about 0.01% to 75% by weight. The aqueous wax emulsions are broadly described in U.S. Pat. No. 6,001,346,. The TRPV inhibitors of the methods described herein can have a viscosity appropriate for use in aerial or backpack spray applications.

The biodegradable wax carrier comprises at least about 10% by weight of the formulation. The biodegradable wax carrier is selected from the group consisting of paraffin, beeswax, vegetable based waxes such as soywax (soybean based), and hydrocarbon based waxes such as Gulf Wax Household Paraffin Wax; paraffin wax, avg. m.p. 53C (hexacosane), high molecular weight hydrocarbons), carnauba wax, lanolin, shellac wax, bayberry wax, sugar cane wax, microcrystalline, ozocerite, ceresin, montan, candelilla wax, and combinations thereof.

Formulations may also contain an emulsifier in an amount from about 1% to about 10% by weight. Suitable emulsifiers include lecithin and modified lecithins, mono- and diglycerides, sorbitan monopalmitate, sorbitan monooleate, sorbitan monolaurate, polyoxyethylene-sorbitan monooleate, fatty acids, lipids, etc. The emulsifiers provide or improve emulsification properties of the composition. The emulsifier can be selected from many products which are well known in the art, including, but not limited to, sorbitan monolaurate (anhydrosorbitol stearate, molecular formula C₂₄H₄₆O₆), ARLACEL 60, ARMOTAN MS, CRILL 3, CRILL K3, DREWSORB 60, DURTAN 60, EMSORB 2505, GLYCOMUL S, HODAG SMS, IONET S 60, LIPOSORB S, LIPOSORB S-20, MONTANE 60, MS 33, MS33F, NEWCOL 60, NIKKOL SS 30, NISSAN NONION SP 60, NONION SP 60, NONION SP 60R, RIKEMAL S 250, sorbitan c, sorbitan stearate, SORBON 60, SORGEN 50, SPAN 55, AND SPAN 60; other sorbitan fatty acid ester that may be used include sorbitan monopalmitate, sorbitan monostearate, sorbitan tristearate, sorbitan monooleate, sorbitan sesquioleate, sorbitan trioleate, sorbitan monooleate, sorbitan trioleate.

In certain embodiments, formulations can include a phagostimulant, such as corn oil, molasses, glycerol, or corn syrup, proteinaceous material (protein or hydrolyzed protein), sugars like sucrose, or food-based ingredients such as trimethylamine, putrescine, bacterial or yeast volatiles or metabolites, ammonium acetate, ammonium carbonate or other ammonia-emitting compounds. Acetic acid vapor can be provided by compounds that produce volatilized acetic acid, for example, aqueous acetic acid, glacial acetic acid, glacial (concentrated) acetic acid, or ammonium producing compounds such as but not restricted to ammonium hydroxide, ammonium carbonate, ammonium bicarbonate, ammonium acetate, etc.

In certain embodiments, the active ingredient may be formulated and/or applied with one or more second compounds. For example, various combinations of TRPV inhibitors and TRPV agonists may be used to obtain greater advantage. For example, both a TRPV inhibitor and TRPV agonist may be applied at the same time. As such in one embodiment, a formulation described herein may include both, a TRPV inhibitor and a TRPV agonist. In one embodiment, two or more active agents may be formulated together. In alternative embodiment, two or more active agents formulated together are all either TRPV inhibitors or are all TRPV agonists. Such combinations may provide certain advantages, such as, without limitation, exhibiting synergistic effects for greater control of insects or non-insect pests, reducing rates of application thereby minimizing any impact to the environment and to worker safety, controlling a broader spectrum of insects and non-insect pests, and improving tolerance by non-pest species, such as mammals and fish. Other second compounds include, without limitation, insecticides, pesticides, plant growth regulators, fertilizers, soil conditioners, or other agricultural and horticultural chemicals. The formulation may include such second compounds in an amount from about 0.002% to about 25% by weight of the composition.

In certain embodiments, the formulations of the present disclosure may contain visual attractants. e.g. food coloring.

A variety of additives may also be incorporated into the formulation. These additives typically change and/or enhance the physical characteristics of the carrier material and are, therefore, suitable for designing compositions having specific requirements as to the release rate and amount of the active ingredient, protection of the wax composition from weather conditions, etc. These additives are, among others, plasticizers, volatility suppressants, antioxidants, lipids, various ultraviolet blockers and absorbers, or antimicrobials, typically added in amounts from about 0.001% to about 10%, more typically between 1-6%, by weight.

Plasticizers, such as glycerin or soy oil affect physical properties of the composition and may extend its resistance to environmental destruction.

Antioxidants, such as vitamin E, BHA (butylated hydroxyanisole), BHT (butylated hydroxytoluene), and other antioxidants which protect the bioactive agent from degradation, may be added in amounts from about 0.1% to about 3%, by weight.

Ultraviolet blockers, such as beta-carotene, lignin or p-aminobenzoic acid protect the bioactive agents from light degradation may be added in amounts from about 1% to about 3%, by weight. Antimicrobials, such as potassium sorbate, nitrates, nitrites, and propylene oxide, protect the bioactive agents from microbial destruction may be added in amounts from 0.1% to about 2% by weight.

Adjuvants can also be added to the formulation. An "adjuvant" is broadly defined as any substance added to the spray tank, separate from the insecticide formulation that will improve the performance of the insecticide. These may include, but are not limited to: wetter-spreaders, stickers, penetrants, compatibility agents, buffers, and so on.

Other compounds and materials may also be added provided they do not substantially interfere with the activity of active ingredient. Whether or not an additive substantially interferes with the active ingredient's activity can be determined by standard test formats, involving direct comparisons of efficacy of the composition of the active ingredient without an added compound and the composition of the active ingredient with an added compound.

It was discovered that an insect will stop eating after ingesting a TRP and/or TRPV channel activating compound. For example ingesting a TRPV ion gated channel agonist can cause an insect to stop eating. Thus, in one embodiment, the compounds may be formulated with a food source for insects, e.g., formulated with compounds in insect diet. In another embodiment, the compounds may be formulated with sucrose. Without wishing to be bound by theory, the insect will feed on such mixtures and stop eating.

In certain embodiments, the active agent may be applied to feeding locus of insects. This inhibits insect feeding leading to starvation of insects. In one embodiment, the active agent is applied as a spray to locus of insects, e.g., feeding locus. The active agent may be applied to the insect and/or pest (mollusk, nematode and/or arthropod) feeding source, used in bait or traps, and or directly applied to the pest itself.

In one embodiment, the active agent may be applied to insect traps. For example, the trap may be coated with the active agent or trap may be loaded with insect food comprising an active agent.

The nucleic acid sequences of Nanchung and Inactive subunits of the TRPV channel as well as the corresponding amino acid sequences, their origins and accession numbers (if available) are summarized in Table 1 below. Upon co-expression of the Nanchung and Inactive proteins in a cell, a functional TRPV channel may be formed.

**Table 1.**

| **SEQ ID NO.** | **DESCRIPTION** | **ORGANISM** | **ACCESSION NO.** |
|---|---|---|---|
| 1 | Nanchung DNA | *Drosophila melanogaster* | NM_140439.3 |
| 2 | Nanchung DNA | *Drosophila melanogaster* | NM_001274904.1 |
| 3 | Nanchung DNA | *Musca domestica* | XM_005180432 |
| 4 | Nanchung DNA | *Ceratitis capitata* | XM_004537685.1 |
| 5 | Nanchung DNA | *Anopheles gambiae* | XM_320300.4 |
| 6 | Nanchung DNA | *Aedes aegypti* | XM_001652374.1 |
| 7 | Nanchung DNA | *Culex quinquefasciatus* | XM_001847084 |
| 8 | Nanchung DNA | *Tribolium castaneum* | XM_962803.1 |
| 9 | Nanchung DNA | *Bombyx mori* | XM_004923013.1 |
| 10 | Nanchung DNA | *Anopheles darlingi* | gi\|312376269 |
| 11 | Nanchung DNA | *Acyrthosiphon pisum* | XM_001947872.2 |
| 12 | Nanchung DNA | *Dendroctonus ponderosae* | gi\|459669722 |
| 13 | Nanchung DNA | *Harpegnathos saltator* | gi\|307201159 |
| 14 | Nanchung DNA | *Nasonia vitripennis* | XM_001606052.2 |
| 15 | Nanchung DNA | *Megachile rotundata* | XM_003706124.1 |
| 16 | Nanchung DNA | *Apis mellifera* | XM_625167.3 |
| 17 | Nanchung DNA | *Apis florea* | XM_003689958.1 |
| 18 | Nanchung DNA | *Pediculus humanus corporis* | XM_002427918.1 |
| 19 | Nanchung DNA | *Danaus plexippus* | gi\|357621160 |
| 20 | Nanchung DNA | *Solenopsis invicta* | gi\|322788678 |
| 21 | Nanchung DNA | *Acromyrmex echinatior* | gi\|332030918 |
| 22 | Nanchung DNA | *Camponotus floridanus* | gi\|307175924 |
| 23 | Nanchung DNA | *Myzus persicae* | In-house |
| 24 | Nanchung DNA | *Bemisia tabaci* | In-house |
| 25 | Nanchung DNA | *Euschistus heros* | In-house |
| 26 | Nanchung DNA | *Nilaparvata lugens* | In-house |
| 27 | Nanchung DNA | *Schistocerca americana* | In-house |
| 28 | Inactive DNA | *Drosophila melanogaster* | NM_132125.1 |
| 29 | Inactive DNA | *Musca domestica* | XM_005180960.1 |
| 30 | Inactive DNA | *Ceratitis capitata* | XM_004529418.1 |
| 31 | Inactive DNA | *Aedes aegypti* | XM_001659838.1 |
| 32 | Inactive DNA | *Culex quinquefasciatus* | XM_001864290.1 |
| 33 | Inactive DNA | *Anopheles gambiae* | XM_310685.5 |
| 34 | Inactive DNA | *Tribolium castaneum* | TC012368 |
| 35 | Inactive DNA | *Megachile rotundata* | XM_003704618.1 |
| 36 | Inactive DNA | *Apis mellifera* | XM_001121881.2 |
| 37 | Inactive DNA | *Apis florea* | XM_003690679.1 |
| 38 | Inactive DNA | *Pediculus humanus corporis* | XM_002432337.1 |
| 39 | Inactive DNA | *Acromyrmex echinatior* | gi\|332020355 |
| 40 | Inactive DNA | *Harpegnathos saltator* | gi307206889 |
| 41 | Inactive DNA | *Bombus impatiens* | XM_003485651.1 |
| 42 | Inactive DNA | *Bombus terrestris* | XM_003396143.1 |
| 43 | Inactive DNA | *Dendroctonus ponderosae* | gi\|478256802 |
| 44 | Inactive DNA | *Nasonia vitripennis* | XM_001602538.2 |
| 45 | Inactive DNA | *Camponotus floridanus* | gi\|307168683 |
| 46 | Inactive DNA | *Danaus plexippus* | gi\|357618515 |
| 47 | Inactive DNA | *Bombyx mori* | XM_004925264.1 |
| 48 | Inactive DNA | *Acyrthosiphon pisum* | XM_001950061.2 |
| 49 | Inactive DNA | *Solenopsis invicta* | GL767538.1 |
| 50 | Inactive DNA | *Schistocerca americana, DNA partial* | In-house |
| 51 | Inactive DNA | *Myzus persicae* | In-house |
| 52 | Inactive DNA | *Bemisia tabaci* | In-house |
| 53 | Inactive DNA | *Euschistus heros* | In-house |
| 54 | Nanchung PROT | *Drosophila melanogaster* | NP_648696.2 |
| 55 | Nanchung PROT | *Drosophila melanogaster* | NP_001261833.1 |
| 56 | Nanchung PROT | *Musca domestica* | XP_005180489.1 |
| 57 | Nanchung PROT | *Ceratitis capitata* | XP_004537742.1 |
| 58 | Nanchung PROT | *Anopheles gambiae* | XP_320300.4 |
| 59 | Nanchung PROT | *Aedes aegypti* | XP_001652424.1 |
| 60 | Nanchung PROT | *Culex quinquefasciatus* | XP_001847136.1 |
| 61 | Nanchung PROT | *Tribolium castaneum* | XP_967896.1 |
| 62 | Nanchung PROT | *Bombyx mori* | XP_004923070.1 |
| 63 | Nanchung PROT | *Anopheles darlingi* | EFR23411.1 |
| 64 | Nanchung PROT | *Acyrthosiphon pisum* | XP_001947907.2 |
| 65 | Nanchung PROT | *Dendroctonus ponderosae* | ERL84850.1 |
| 66 | Nanchung PROT | *Harpegnathos saltator* | EFN81068.1 |
| 67 | Nanchung PROT | *Nasonia vitripennis* | XP_001606102.2 |
| 68 | Nanchung PROT | *Megachile rotundata* | XP_003706172.1 |
| 69 | Nanchung PROT | *Apis mellifera* | XP_625170.3 |
| 70 | Nanchung PROT | *Apis florea* | XP_003690006.1 |
| 71 | Nanchung PROT | *Pediculus humanus corporis* | XP_002427963.1 |
| 72 | Nanchung PROT | *Danaus plexippus* | EHJ73092.1 |
| 73 | Nanchung PROT | *Solenopsis invicta* | EFZ14282.1 |
| 74 | Nanchung PROT | *Acromyrmex echinatior* | EGI70573.1 |
| 75 | Nanchung PROT | *Camponotus floridanus* | EFN65752.1 |
| 76 | Nanchung PROT | *Myzus persicae* | In-house |
| 77 | Nanchung PROT | *Bemisia tabaci* | In-house |
| 78 | Nanchung PROT | *Euschistus heros* | In-house |
| 79 | Nanchung PROT | *Nilaparvata lugens* | In-house |
| 80 | Nanchung PROT | *Schistocerca americana* | In-house |
| 81 | Inactive PROT | *Drosophila melanogaster* | NP_572353.1 |
| 82 | Inactive PROT | *Musca domestica* | XP_005181017.1 |
| 83 | Inactive PROT | *Ceratitis capitata* | XP_004529475.1 |
| 84 | Inactive PROT | *Aedes aegypti* | XP_001659888.1 |
| 85 | Inactive PROT | *Culex quinquefasciatus* | XP_001864325.1 |
| 86 | Inactive PROT | *Anopheles gambiae* | XP_310685.5 |
| 87 | Inactive PROT | *Tribolium castaneum* | EFA10736.1 |
| 88 | Inactive PROT | *Megachile rotundata* | XP_003704666.1 |
| 89 | Inactive PROT | *Apis mellifera* | XP_001121881.1 |
| 90 | Inactive PROT | *Apis florea* | XP_003690727.1 |
| 91 | Inactive PROT | *Pediculus humanus corporis* | XP_002432382.1 |
| 92 | Inactive PROT | *Acromyrmex echinatior* | EGI60788.1 |
| 93 | Inactive PROT | *Harpegnathos saltator* | EFN84766.1 |
| 94 | Inactive PROT | *Bombus impatiens* | XP_003485699.1 |
| 95 | Inactive PROT | *Bombus terrestris* | XP_003396191.1 |
| 96 | Inactive PROT | *Dendroctonus ponderosae* | ERL85467.1 |
| 97 | Inactive PROT | *Nasonia vitripennis* | XP_001602588.2 |
| 98 | Inactive PROT | *Camponotus floridanus* | EFN61724.1 |
| 99 | Inactive PROT | *Danaus plexippus* | EHJ71463.1 |
| 100 | Inactive PROT | *Bombyx mori* | XP_004925321.1 |
| 101 | Inactive PROT | *Acyrthosiphon pisum* | XP_001950096.1 |
| 102 | Inactive PROT | *Solenopsis invicta* | EFZ13594.1 |
| 103 | Inactive PROT | *Schistocerca americana, DNA partial* | In-house |
| 104 | Inactive PROT | *Myzus persicae* | In-house |
| 105 | Inactive PROT | *Bemisia tabaci* | In-house |
| 106 | Inactive PROT | *Euschistus heros* | In-house |

Adenovirions, which include the adenovirus expression vectors of the present disclosure, can be produced using the following methodology. Adenovirons may be used to generate cells used to express the proteins of interest, for example, Nanchung, Inactive, TRP and/or TRPV proteins.

The methods of creating an adenovirus generally involves the steps of introducing the vector containing the gene of interest (e.g., Nan or Iav) into a producer cell. The vector containing the gene of interest may be introduced into the producer cell using standard transfection techniques known to one of skill in the art (Zoltukhin et al., Gene Therapy, 6:973-985, 1999). The adenovirus produced by producer cells (packaging cells) are used to transduce a cell of interest. The cell of interest produces the desired proteins.

Preferably, the adenovirions are added to the cells at the appropriate multiplicity of infection according to standard transduction methods appropriate for the particular target cells. Titers of adenovirions to administer can vary, depending upon the target cell type and the particular viral vector, and may be determined by those of skill in the art without undue experimentation.

The cell line may be an insect cell line, such as Sf9 (ATCC# CRL-1711) or Schneider 2 (S2) cells (Life Technologies, #R690-07) (if needed, to introduce a gene of interest into an insect cell transfection techniques may be used among others), African clawed frog (Xenopus laevis) oocytes (to introduce a gene of interest injection techniques may be used), or a mammalian cell line, such as mouse, hamster, human cell line, (to introduce a gene of interest into a mammalian cell several techniques may be used, including but not limited to transfection, adenoviruses, retroviruses, and/or electroporation, etc...) or any other cell line that does not normally expresses Nanching and Inactive proteins. One example of a mammalian cell line suitable for use with the vector expression system of the present disclosure includes Chinese hamster ovary (CHO-K1) cells (ATCC# CCL-61).

In certain embodiments, the cell line co-expresses the Nanchung and Inactive proteins at a ratio of 3:1, 2:1, 1:1, 1:2, and 1:3. Preferably, the cell line co-expresses the Nanchung and Inactive proteins at a ratio of 1:1.

In some embodiments, a cell expressing a recombinant nucleic acid sequence encoding a TRPV channel is a cell that has been transformed with an expression vector comprising a nucleotide sequence encoding an insect TRPV channel such as, but not limited to the TRPV channel proteins discloses herein. Methods for transforming cells that would be known to one of ordinary skill in the art include, but are not limited to, infection using viral vectors, lipofection, electroporation, particle bombardment, and transfection. Detailed procedures for representative methods can be found in Sambrook & Russell, 2001, and references cited therein. Useful expression vectors and methods of introducing such vectors into cells or expression of the encoded polypeptide are also known to one of ordinary skill in the art. For example, a plasmid expression vector can be introduced into a cell by calcium-phosphate mediated transfection, DEAE-Dextran-mediated transfection, lipofection, polybrene- or polylysine-mediated transfection, electroporation, or by conjugation to an antibody, gramicidin S, artificial viral envelopes, or other intracellular carriers. A viral expression vector can be introduced into a cell in an expressible form by infection or transduction, for example, or by encapsulation in a liposome.

When a cell expressing a recombinant nucleic acid sequence encoding an insect TRPV channel gene product has been produced, these cells can then be employed in testing candidate compounds for an ability to modulate ion transport in the cell through the TRP and/or TRPV channel. The exemplary methods for testing ion transport in the cells were described above as well as presented in the Examples sections below. Other applicable methods would be known to those of skill in the art upon consideration of this disclosure.

### EXAMPLES

The following Examples provide illustrative embodiments. In light of the present disclosure and the general level of skill in the art, those of skill will appreciate that the following Examples are intended to be exemplary only and that numerous changes, modifications, and alterations can be employed without departing from the scope of the presently disclosed subject matter.

### MODULATION EXAMPLES FPR AFIDOPYROPEN

### Example I: Afidopyropen and pymetrozine elicit characteristic hind leg extension in grasshoppers.

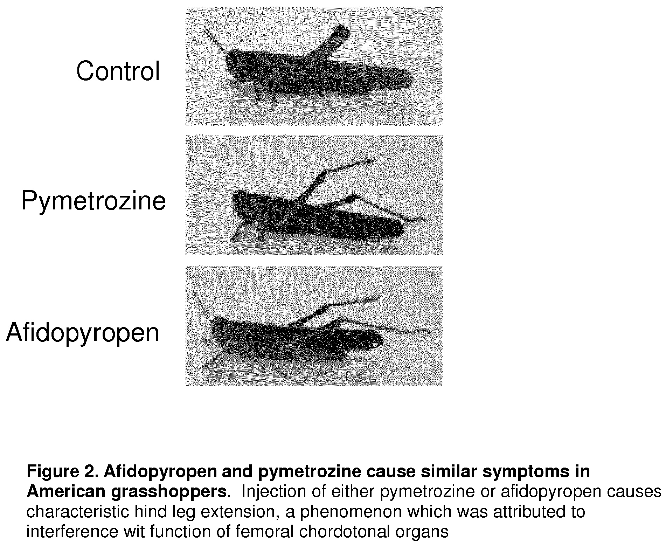

Injection of pymetrozine (PM), was shown to produce characteristic hind leg extension in locusts (*Locusta migratoria),* a phenomenon which was attributed to silencing mechanosensory neurons in femoral chordotonal organs (Ausborn et al., 2005) The Inventors tested whether afidopyropen targeted insect chordotonal organs in similar fashion, by injecting this compound into American grasshoppers (*Schistocerca americana),* and found than this injection elicited hind leg extension similarly to pymetrozine (Figure 2), which suggested that pymetrozine and afidopyropen may act on the same molecular target.

### Example II : Afidopyropen activates Drosophila TRPV channels.

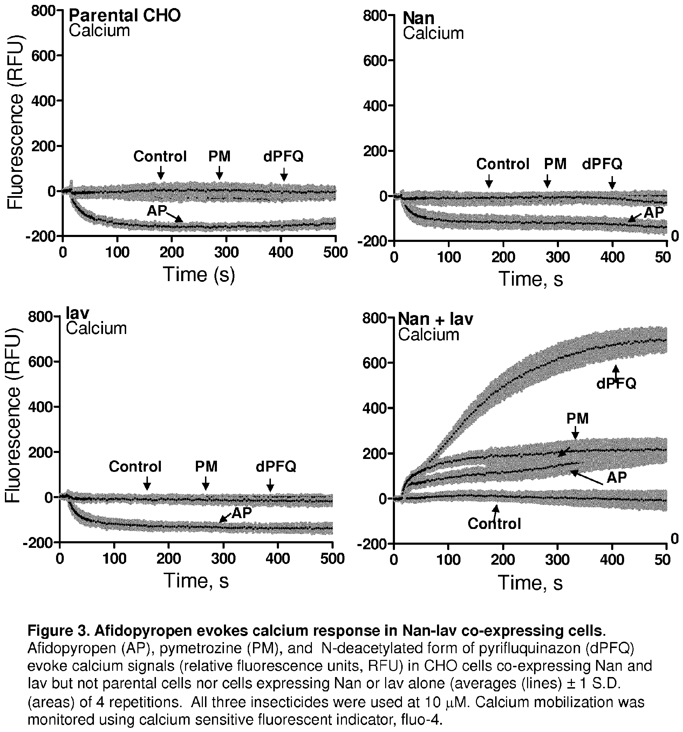

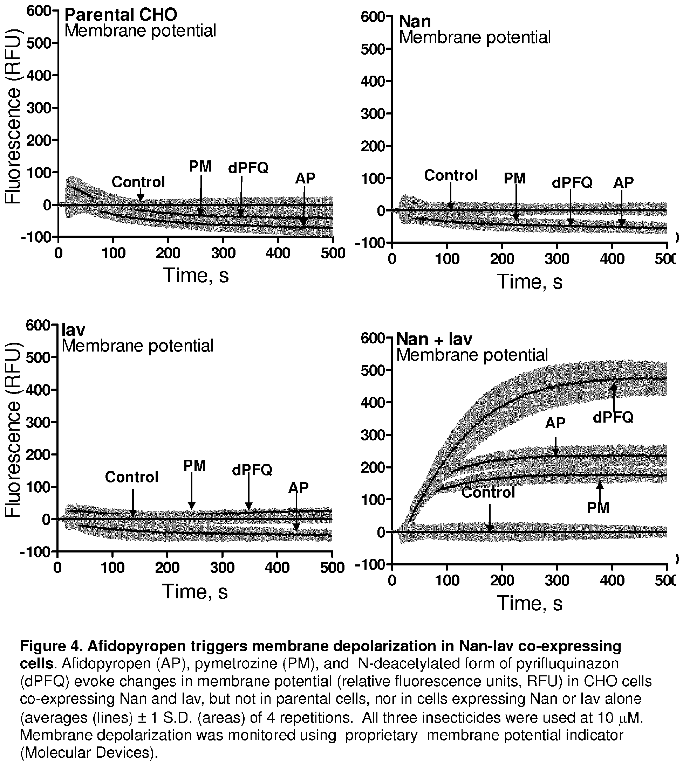

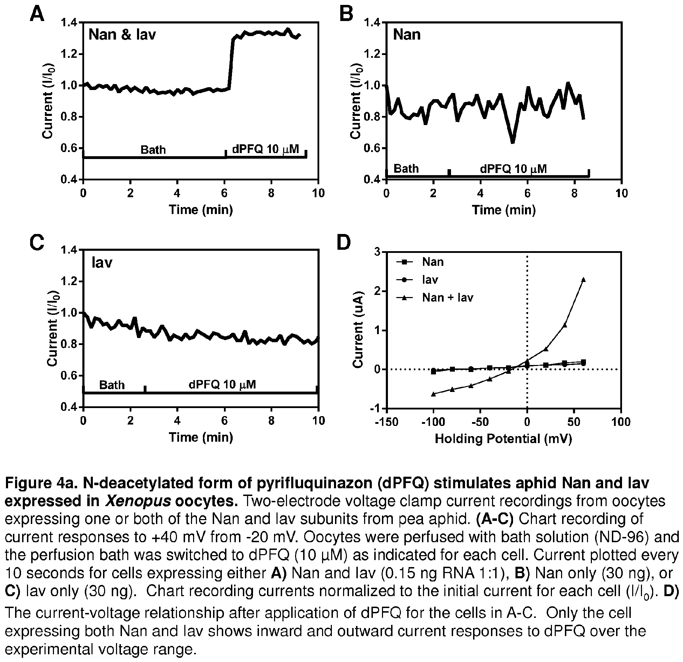

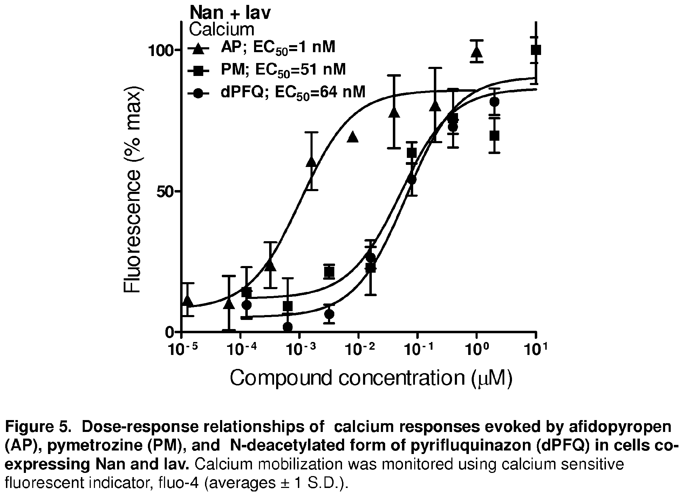

Pymetrozine (PM), pyrifluquinazon and N-deacetylated_pyrifluquinazon (dPFQ) elicited calcium mobilization and membrane depolarization in CHO cells (hamster cells) co-infected with adenoviruses expressing insect TRPV channels, Nan and Iav (Nesterov et al., 2013; Nesterov et al., 2015). Using the same approach, the inventors found afidopyropen evoked similar responses in cells co-infected with adenoviruses expressing insect TRPV channels, Nan and Iav. Two different assays were used in these studies: one (Figure 3) monitored calcium mobilization with calcium probe , fluo-4 (Marshall et al., 2013); whereas another one, (Figure 4), measured agonist-induced depolarization of plasma with florescent voltage indicator (Zheng et al., 2004). In both assays, afidopyropen, pymetrozine and N-deacetylated pyrifluquinazon triggered calcium mobilization and membrane depolarization responses only in cells co-infected with Nan and Iav viruses, but not in parental cells (e.g. the hamster cells that weren't infected with Nan and/or Iav), nor in cells expressing Nan and/or Iav alone. In the calcium mobilization assay, dose-dependence curves (Figure 5) yielded the half-maximal effective concentrations (EC₅₀) of 1.0 nM, 64 nM, and 51 nM for afidopyropen, N-deacetylated pyrifluquinazon, and pymetrozine, respectively.

### Example III : N-deacetylated pyrifluquinazon activates TRPV channels from pea aphid

To determine whether activation of TRPV channels from other insect species also requires simultaneous presence of Nan and Iav subunits atwo-electrode voltage clamp electrophysiology was used to measure channel opening in *Xenopus laevis* (frog) oocytes injected with Nan and Iav mRNA from pea aphid, *Acyrthosiphon pisum.* Current-voltage tests were performed, where currents were measured over a range of holding potentials. N-deacetylated pyrifluquinazon evoked both inward and outward current responses only in oocytes expressing both Nan and Iav (Figure 4a), but neither Nan no Iav alone.

### Example IV: Afidopyropen does not activate mammalian TRPV4 channel.

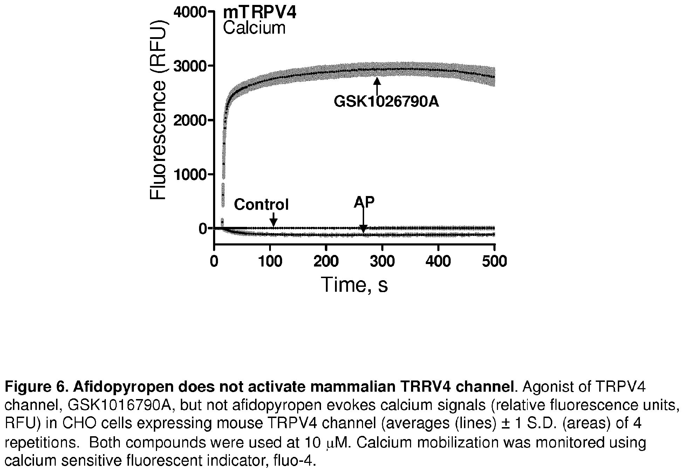

To gain insight into receptor species specificity of afidopyropen effects, CHO cells (hamster cells) were transduced with the adenovirus expressing mouse TRPV channel, mTRPV4. As shown on Figure 6, the TRPV4 agonist GSK1016790A (Thorneloe et al., 2008), evoked strong calcium response in these cells, whereas afidopyropen failed to activate mTRPV4-expressing cell. GSK1016790A is a known strong agonist of the TRPV4 channel. Accordingly, the inventors found that afidopyropen was a species-specific insecticide, in that it was a modulator of insect TRPV channels but did not modulate the mammalian TRPV4 channel. TRPV4 channel is one of the closest orthologues of the insect TRPV channel. It was chosen to be used based on sequence similarity and the availability of a known agonist.

### DIRECT BINDING EXAMPLES

### Example V: Afidopyropen directly binds insect TRPV channel

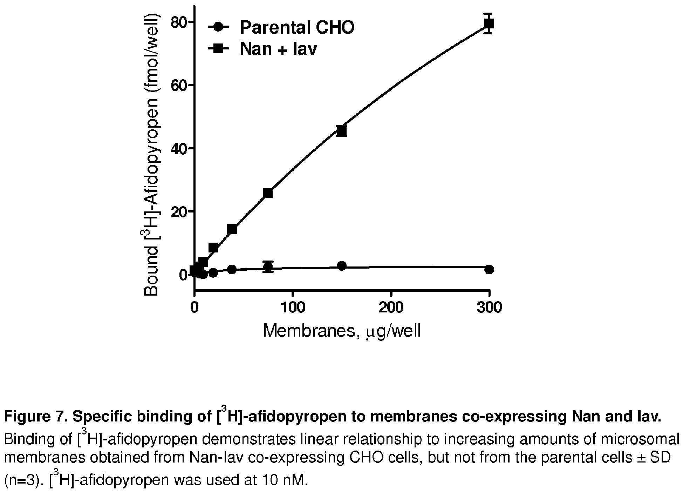

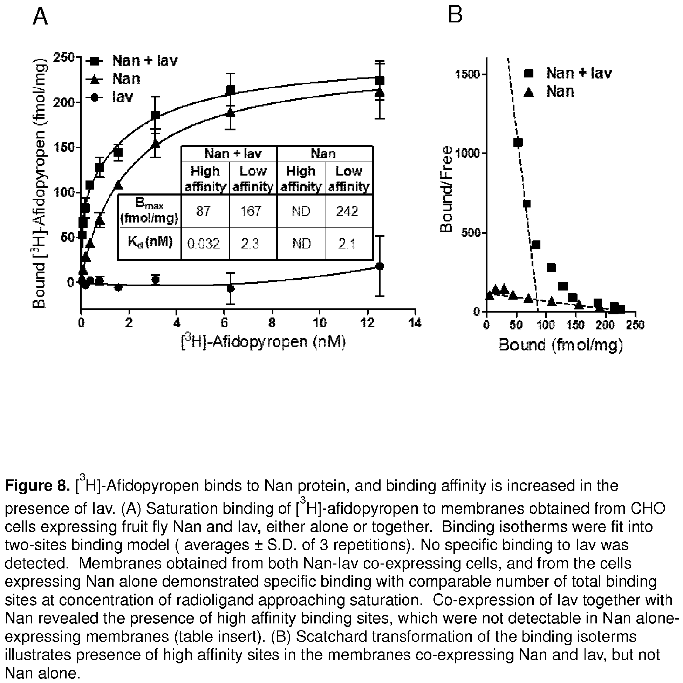

Specific binding of tritium-labeled afidopyropen to membranes prepared from Nan and Iav co-expressing cells exhibited a linear relationship to increasing amounts of membranes, whereas binding to parental cell membranes remained low regardless of the amount of membranes used (Figure 7). Saturation binding (Figure 8) to Nan-lav membranes revealed the presence of 2 binding sites: one of very high affinity (K_{d} = 0.032 nM, Bₘₐₓ = 87 fmol/mg of membrane proteins), and one of lower affinity (K_{d} = 2.3 nM, Bₘₐₓ = 167 fmol/mg of membrane proteins). In membranes prepared from Nan-only cells, only lower affinity sites were detected (K_{d} = 2.1 nM, Bₘₐₓ = 242 fmol/mg of membrane proteins),whereas no specific binding was observed in membranes prepared from Iav-only cells. From this data, the inventors found that the Nan protein forms the major binding interface for afidopyropen, whereas the presence of Iav may act as a facilitator to increase binding affinity.

### Example VI: Pymetrozine and N-deacetylated form of pyrifluquinazon compete with [³H]-Afidopyropen for binding.

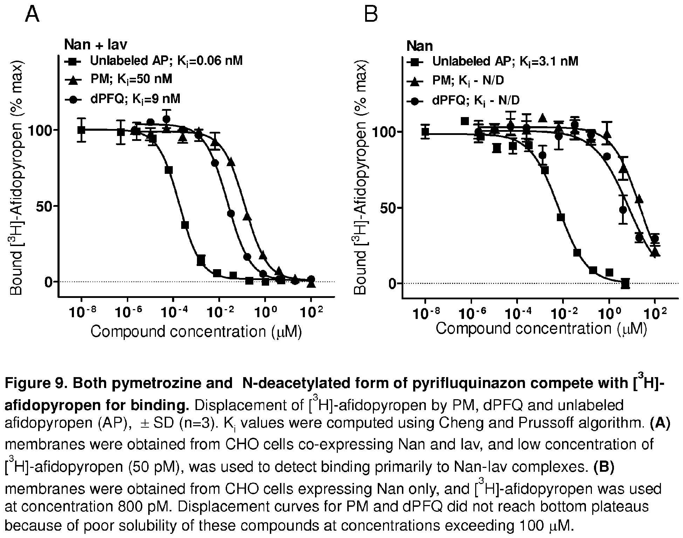

To assess the ability of pymetrozine and N-deacetylated form of pyrifluquinazon to displace [³H]-Afidopyropen from Nan-Iav complexes (Figure 9A), the experiment was performed at low concentration of [³H]- Afidopyropen (0.05 nM), i.e., under conditions where binding of [³H]- Afidopyropen to Nan-only membranes was negligible (Figure 8). Both pymetrozine, N-deacetylated pyrifluquinazon and unlabeled Afidopyropen, completely displaced [³H]-Afidopyropen from the high affinity sites with simple kinetics (Hill values ≈ -1), indicating that all three compounds tested share the same binding site. Noteworthy, for unlabeled Afidopyropen, Kᵢ value of 60 pM measured in displacement assay (Figure 9A) came close to K_{d} value of 32 pM obtained for [³H]- Afidopyropen in saturating binding experiments (Figure 8), with less than a two-fold difference, which illustrates close similarity for these two values. N-deacetylated pyrifluquinazon and pymetrozine exhibited significantly lower binding affinities than that of Afidopyropen, with Kᵢ values of 9 nM and 50 nM for N-deacetylated pyrifluquinazon and pymetrozine, respectively, which correlates with lower potencies of these compounds in the functional assay (Figure 5). Based on this data, the inventors found that afidopyropen directly binds to the same site as pymetrozine and dPFQ, which means these three compounds have the same mode of action.

Displacement of [³H]- Afidopyropen from Nan-only expressing membranes (Figure 9B) with unlabeled Afidopyropen yielded Kᵢ value of 3.1 nM, which is close to the K_{d} value of 2.1 nM calculated from saturation binding with [³H]- Afidopyropen (Figure 8). Although pymetrozine and N-deacetylated pyrifluquinazon clearly displaced [³H]- afidopyropen from Nan-only sites, poor solubility of these compounds at high concentrations, prevented competition binding curves from reaching the bottom plateaus, hampering accurate determination of their Kᵢ values. Nevertheless, all three compounds clearly exhibited the same potency ranking (Afidopyropen >_N-deacetylated pyrifluquinazon≥pymetrozine) for low affinity Nan-only sites as for high affinity Nan-Iav complexes. It appears, therefore, that co-expression of Iav protein increases binding affinities for all 3 insecticides, despite significant structural differences between Afidopyropen on one hand, and pymetrozine and N-deacetylated pyrifluquinazon on the other hand (Figure 1). The inventors found that the presence of Inactive protein increased binding affinity for N-deacetylated pyrifluquinazon and pymetrozine, in addition to afidopyropen.

In summary, using the combination of functional and binding assays in the examples above, the inventors demonstrated the following: i) the insect TRPV channel is a direct molecular target of the candidate insecticide, Afidopyropen; ii) Afidopyropen binding site in TRPV protein is shared with two commercial insecticides, pymetrozine and N-deacetylated pyrifluquinazon indicating the same mode of action of all three compounds; iii)binding affinities of all three compounds were measured; iv) Nanchung protein forms the main binding interface for all three compounds, whereas presence of Inactive protein increases binding affinity.(v) new methods were developed which can be used for identifying candidate compounds that may be used as insecticides which act by directly targeting insect TRPV channels (vi) afidopyropen may be used in these methods to assist in the identification of new candidate compounds having same mode of action.

Pymetrozine and GSK10116790A were obtained from Sigma, pyrifluquinazon from ChemService Inc., (West Chester, PA), N-deacetylated pyrifluquinazon from Wako Pure Chemical Industries, LTD (Richmond, VA), and Afidopyropen was synthesized at BASF. Tritium labeled Afidopyropen (19 Ci/mmol) was custom-made by RC TRITEC AG (Teufen, Germany).

### Leg extension assay

American grasshoppers (*Schistocerca americana)* were obtained from Dr. Hojun Song (University of Central Florida). Test compounds (10 mM in 100% DMSO), or vehicle (100% DMSO) were applied by injection through the abdominal intersegmental cuticle (3 µl per animal), using Hamilton syringe. The effects of compounds were assessed 40-60 minutes post-injection.

### Heterologous expression of Nan and Iav in CHO-K1 cells

To produce Nan, Iav and mTRPV4 with fused FLAG tag and AcGFP fluorescent protein at their carboxyl termini, complementary DNAs (cDNAs) encoding *Drosophila* Nan (NCBI NM_001274904.1) and Iav (NCBI NM_132125.1) were synthesized by Life Technologies (Grand Island, NY), with the addition of sequences encoding FLAG antibody tags at the C-termini. cDNA for mouse TRPV4 (NCBI NM_001274904.1) containing a C-terminal FLAG epitope sequence was purchased from Origene (Rockville, MD). Insect cDNAs were codon-optimized for expression in mammalian cells. The Nan-FLAG and Iav-FLAG cDNAs were subcloned into the Bgl II and HindIII sites of the modified pAcGFP1-Hyg-N1 vector (Clontech, Kyoto, Japan), which contained a FLAG tag at the C-terminus of the AcGFP moiety (pAcGFP1-Hyg-N1-FLAG). The mTRPV4-FLAG cDNA was PCR amplified using the primers 5'-GGACTTTCCAAAATGTCG-3' (SEQ ID NO:108) and 5'-CCGGCCGTTTATCACTACAGAATTCGAAGCTTAACCTTATCGTCGTCATCCTTGT A-3' (SEQ ID NO: 109), subsequently digested with BglII and HindIII, and sub-cloned into the pAcGFP1-Hyg-N1-FLAG vector. These cloning procedures added an AcGFP protein flanked by two FLAG epitope tags to the carboxyl termini of each Nan, Iav and mouse TRPV4.

To produce Nan with fused HA epitopes and mCherry fluorescent protein at its carboxyl terminus, codon-optimized *nan* cDNA was PCR amplified using the primers 5'-GCTGGTTTAGTGAACCGTCAG-3' and 5'-CCGCGGTACCGTCGACTGCAGAATTCGAAGCTTAGCGTAATCTGGAACATCGTA TGGGTAGAAGTTGTTGTTGTCGCTGCACTCGGACTTGGG-3', adding an HA epitope tag to the C-terminus of Nan. The PCR product was digested with XhoI and HindIII, and subcloned into the modified pmCherry-N1 vector (Clontech), which contained an HA tag at the C-terminus of the mCherry moiety. This cloning procedure added mCherry protein flanked by two HA epitope tags to the C-terminus of Nan.

To produce adenoviruses, epitope-tagged Nan and Iav were digested with XhoI and NotI and subcloned to the same sites of the adenovirus shuttle vector pENTCMV1-TetO (Welgen Inc., Worceter, MA). Epitope-tagged mTRPV4 was digested with BglII, blunt-ended with Klenow and digested with XbaI. The fragment was subcloned into PmeI and XbaI sites of the pENTCMV1-TetO. pENTCMV1-TetO vectors containing tagged Nan, Iav or mTRPV4 expression constructs were treated with LR Clonase II (Life Technologies) and ligated to a pAdREP plasmid (Welgen), which contains the remaining adenovirus genome. The pENTCMV1-TetO vector contains two Tet repressor binding sites within a modified CMV promoter, which repress transcription of the gene of interest in cells expressing the Tet repressor. The recombination products were transformed into *Escherichia coli* cells, positive clones were selected, and the cosmid DNA was isolated. The cosmid DNA was digested with Pac I and then transfected into HEK293-TetR cells, which produce the Tet repressor (Postle et al., 1984), preventing expression of TRP channels by the adenovirus packaging cells. Adenoviruses were purified from large-scale cultures and viral titers were calculated as viral particles/mL = Absorption at 260 nm x 1.1x10¹⁴.

### Heterologous expression of Nan and Iav in frog (Xenopus laevis) oocytes

Nan and Iav coding sequences were RT-PCR amplified from pea aphid (*Acyrthosiphon pisum)* antennal total RNA using the QIAGEN Onestep RT-PCR Kit (cat# 210212) and the primers nan-Ap start 5'-GCCACCATGGGTAACACCGAGAGCAATG-3', nan-Ap stop 5'-TTATTCATTTGCTTTTAGATTAAATGTCCC-3', iav-Ap start 5'-GCCACCATGGGAAATTCTTGTGCGAG-3' and iav-Ap stop 5'-TCAGAAATCGTCGTTGGG-3', and cloned into the oocyte expression vector pGH19. Linear cDNA templates for transcription were produced by PCR amplification from these constructs using the vector specific primers 5'-GTTGTAAAACGACGGCCAGTGAATTG-3' and 5'-GACACTATAGAATACTCAAGCTAGCCTCGAGG-3'. These primers were designed to include the T7 promoter and the 3'UTR sequence from the Xenopus globin-XI gene found in pGH19 in the PCR product. The PCR conditions were 1 cycle at 95°C for 4 minutes, 35 cycles with 95 °C for 30 seconds, 55 °C for 30 seconds and 65 °C for 4 minutes, and 1 cycle at 68 °C for 6 minutes.

*In vitro* transcription was performed with the T7 polymerase mMessage mMachine kit (Ambion). *Xenopus laevis* ovaries were obtained from Xenopus1, Inc. (Dexter, MI). The lobes were partially dissected and digested with collagenase type 1 (Alfa Aesar) dissolved in Oocyte Ringers solution (pH 7.5, in mM: NaCl 82.5, KCl 2, MgCl2 1, HEPES 5) until defolliculated. Defolliculated oocytes were washed with and incubated in ND-96⁺ (pH 7.5, in mM: NaCl 96, KCl 2, MgCl2 1, CaCl2 1.8, HEPES 5, Na Pyruvate 2.5 and Gentamycin 50 ug/mL) for two hours before RNA injection. Stage IV and V oocytes were injected with RNA from Nan, Iav or both using a Nanoject automatic injector (3-00-203-X, Drummond Scientific). For one to one expression of each subunit, 0.15 ng of RNA for each subunit was co-injected in 50 nL of RNAse free water (3 ng/uL). For single subunit RNA injections, 30 ng of RNA was injected in 50 nL (600 ng/uL) water to ensure maximal expression of the single subunit. Prior to any measure or comparison steps, oocytes were incubated at 17°C in ND-96⁺ and the media was changed daily. Further testing steps were made 3 days after RNA injection and/or after sufficient protein production by the oocytes.

### Calcium imaging and membrane depolarization assays

Titers of adenoviruses which allow for equal expression levels of recombinant TRP channels, and optimal cell responses were established previously (Nesterov et al., 2015).Hamster CHO-Kl cells (ATCC® CCL-61TM) were infected with adenoviruses expressing AcGFP-Iav, mCherry-Nan, 1:1 mixture of AcGFP-Iav and mCherry-Nan, (4000 viral particles per cell) or AcGFP-mTRPV4 (400 viral particles per cell). The cells were seeded on poly-D-lysine coated 96-well plates (Greiner Bio-One, Frickenhausen, Germany) in 100 µl of media, at a density of 40,000 cells per well. The cells were kept overnight at 37°C, followed by 3 days at 25°C. The medium was changed two days after seeding. Both Ca²⁺ mobilization and changes of membrane potential were measured using a FLIPR-TETRA instrument (Molecular Devices, Sunnyvale, CA). Ca²⁺ mobilization was measured using fluo-4AM (Life Technologies). The cells were loaded with 50 µl of Hank's buffered salt solution (HBSS) containing 4 µM fluo-4AM, 5mM probenecid, 20 mM CaCl₂, and 0.02% pluoronic for 2 hours at 25oC. The dye was then discarded, 50 µl of HBSS were added. The test compounds were dissolved in DMSO and added to the cells in 50 µl HBSS, yielding a final DMSO concentration of 0.2%. Fluorescence was monitored for 10 minutes at 1 second intervals at 470-495nm/515-575nm excitation/emission wavelengths. Changes of membrane potential were measured using a similar procedure, except that the cells were loaded for 2 hours with a proprietary membrane potential dye (Molecular Devices, Cat # R8042) dissolved in HBSS. The dye loading solution was discarded and replaced with 50 ul of 20mM HEPES, 11.1mM Glucose, 125mM NaCl, and 2.5mM KCl, and 20 mM CaCl₂. The test compounds dissolved in DMSO were added to the cells in 50 µl of the same solution, yielding a final DMSO concentration of 0.2%. Fluorescence was monitored for 10 minutes at 1 second intervals at 510-545 nm/565-625 nm excitation/emission.

EC₅₀ values were calculated using GraphPad Prism (GraphPad Software).

### Two-electrode voltage clamping of Xenopus laevis oocytes.

Oocytes were placed in a RC-8 recording chamber (Warner Instruments) filled with ND-96 bath solution (in mM: NaCl 96, KCl 2, MgCl2 1, CaCl2 1.8, and HEPES 5, pH 7.5,). Glass microelectrodes filled with 3 M KCl and with resistances between 0.9-1.4 MΩ were used to voltage clamp the oocytes through a Turbo Tec 10-C amplifier (npi) and ITC-16 computer interface (InstruTech) controlled by PatchMaster recording software v.2x73.5 (HEKA). Oocytes were perfused with ND-96 saline at 2 mL/min (gravity fed). N-deacetylated pyrifluquinazon (dPFQ) was delivered through the same perfusion line directly over the cell by switching out the bath solution with 10 uM dPFQ diluted in ND-96 and 0.1% DMSO. Between voltage steps, oocytes were held at -20 mV. Every 10 seconds, the voltage was stepped to +40 mV and the current, after equilibration, was measured and plotted over time as chart recordings (Figure 4a). Current voltage relationships for single and co-expressed subunits were determined by measuring currents elicited by stepping the voltage from the holding potential of -20 mV to voltages between -100 through +60 mV. Chart recordings and IV tests were exported from Patchmaster and analyzed and plotted with Prism 6 (GraphPad). Chart recording currents were normalized to the initial current for each cell (I/I₀). N-deacetylated pyrifluquinazon evoked both inward and outward current responses only in oocytes expressing both Nan and Iav (Figure 4a), but neither current responses inward or outward were evoked in oocytes expressing Nan or Iav alone. Accordingly, a method for determining whether or not a candidate compound may be a modulator of an insect TRPV channel may include providing a first cell expressing an insect TRPV channel, wherein that first cell may be a *Xenopus laevis* oocyte; contacting the first cell with a candidate compound, wherein the candidate compound may be dPFQ; and assaying for modulation of the insect TRPV channel, wherein the modulation identifies the candidate compound as the modulator of the insect TRPV channel and further wherein the assaying step comprises at least of one the following steps: comparing the electrical current between the first cell in the absence of the candidate compound with the electrical current in the first cell in the presence of the candidate compound; and/or comparing the electrical current between the first cell in the presence of the candidate compound and having one or more Nanchung proteins and one or more Inactive proteins with the electrical current of the first cell in the presence of the candidate compound and not having one or more Nanchung proteins and/or one or more Inactive proteins.

### Membrane preparation

CHO-K1 cells were transduced with adenoviruses expressing AcGFP-tagged Nan and Iav either alone, or as 1:1 mixture (4000 viral particles per cell). The adenovirus-transduced cells were seeded at 10 x 10⁶ cells per T175 flask (Greiner) and kept at 37°C 18 hrs followed by incubation at 25°C for 72 hrs. The media was changed at 24 hrs and 72 hrs post transduction. The cells were rinsed twice with PBS, and incubated in Versene solution (Life Technologies) for 10 min at room temperature. Detached cells were pelleted at 300 × g for 10 min and resuspended in 8 mL of ice-cold 20 mM Hepes (pH 7.3) containing 1% (wt/vol) protease inhibitors mixture (Halt; Thermo Fisher). After 10 min on ice, the cells were homogenized by sonication. The samples were centrifuged at 300 × g for 10 min, and supernatants were collected and centrifuged for 2 h at 50,000 × g. Membranes were resuspended in 0.5 mL of 50mM HEPES, 100mM NaCl, 1mM CaCl₂, 5mM MgCl₂, pH 7.4, and stored at -80°C. Protein content was determined using the Bradford Method.

### [³H]- Afidopyropen binding assay

Equilibrium binding assays were performed in the binding buffer (50mM HEPES, 100mM NaCl, ImM CaCl₂, 5mM MgCl₂, pH 7.4). The reactions were performed in 96-well U-bottom polypropylene plates. 100 µL membranes resuspended in the binding buffer containing Tween-20 (0.0025% final concentration in the assay), were combined with either 200 µl (saturation binding experiments), or 800 µl (ligand displacement experiments) of binding buffer containing [³H]- afidopyropen, dissolved in ethanol, and, where indicated, test compounds, dissolved in DMSO. The final concentrations of ethanol and DMSO were kept at 0.2% and 0.25% respectfully in both saturation binding and competition experiments. Typically, 60-100 µg of membranes were used per data point. Followed by 3 hours of incubation at room temperature, assay mixes were filtered through Multiscreen HTS+ 96 Hi Flow FC filters (EMD Millipore), pre-wetted with 0.1% polyethyleneimine. Filters were washed three times with 200 µl of ice-cold binding buffer, scintillation cocktail was added and radioactivity was measured using Wallac 1450 Microbeta counter. Non-specific binding was measured in the presence of 500-fold excess of unlabeled Afidopyropen. Typically, ligand depletion accounted for less than 10% of total ligand. In saturation binding studies, at the lowest concentrations of [³H]-afidopyropen, equal to 49 pM and 97.7 pM, ligand depletion was 19% and 14%, respectfully. That was corrected by assuming that concentration of free ligand was equal to total minus bound concentration. Nonlinear regression analysis was performed using GraphPad Prism (GraphPad Software), and Kᵢ values were calculated by Cheng-Prusoff method (Cheng and Prusoff, 1973).

### REFERENCES

Ausborn, J., Wolf, H., Mader, W., and Kayser, H. (2005). The insecticide pymetrozine selectively affects chordotonal mechanoreceptors. The Journal of experimental biology 208, 4451-4466.
Casida, J.E., and Durkin, K.A. (2013). Neuroactive insecticides: targets, selectivity, resistance, and secondary effects. Annual review of entomology 58, 99-117.
Cheng, Y., and Prusoff, W.H. (1973). Relationship between the inhibition constant (Kl) and the concentration of inhibitor which causes 50 per cent inhibition (150) of an enzymatic reaction. Biochemical pharmacology 22, 3099-3108.
Kamikouchi, A., Inagaki, H.K., Effertz, T., Hendrich, O., Fiala, A., Gopfert, M.C., and Ito, K. (2009). The neural basis of Drosophila gravity-sensing and hearing. Nature 458, 165-171. Kavlie, R.G., and Albert, J.T. (2013). Chordotonal organs. Current biology : CB 23, R334-335.
Leichter, C.A., Thompson, A., Johnson, B.R., and Scott, J.G. (2013). The high potency of ME-5343 to aphids is due to a unique mechanism of action. Pesticide Biochemistry and Physiology, 169-175.
Maienfisch, P. (2012). Selective feeding blockers: pymetrozine, flonicamid, and pyrifluquinanzon. In Modern Crop Protection Compounds W. Krämer, U. Schirmer, P. Jenschke, and M. Witschel, eds. (New York: John Wiley and Sons), pp. 1327-1346.
Marshall, I.C., Owen, D.E., and McNulty, S. (2013). Measuring Ca(2)(+) changes in multiwell format using the Fluorometric Imaging Plate Reader (FLIPR((R))). Methods in molecular biology 937, 103-109.
Nesterov, A., Kandasamy, R., Wedel, B., and Salgado, V.L. (2013). Methods for determining modulators of insect ransient receptor otential V (TRPV) channel.
Nesterov, A., Spalthoff, C., Kandasamy, R., Katana, R., Rankl, N.R., Andrés, M., Jähde, P., Dorsch, J.A., Stam, L.F., Braun, F., et al. (2015). TRP channels in insect stretch receptors as insecticide targets. Neuron 86, 665-671.
Postle, K., Nguyen, T.T., and Bertrand, K.P. (1984). Nucleotide sequence of the repressor gene of the TN10 tetracycline resistance determinant. Nucleic acids research 12, 4849-4863.
Sun, Y., Liu, L., Ben-Shahar, Y., Jacobs, J.S., Eberl, D.F., and Welsh, M.J. (2009). TRPA channels distinguish gravity sensing from hearing in Johnston's organ. Proceedings of the National Academy of Sciences of the United States of America 106, 13606-13611.
Thorneloe, K.S., Sulpizio, A.C., Lin, Z., Figueroa, D.J., Clouse, A.K., McCafferty, G.P., Chendrimada, T.P., Lashinger, E.S., Gordon, E., Evans, L., et al. (2008). N-((1S)-1-{[4-((2S)-2-{[(2,4-dichlorophenyl)sulfonyl]amino}-3-hydroxypropanoyl)-1-piperazinyl]carbonyl}-3-methylbutyl)-1-benzothiophene-2-carboxamide (GSK1016790A), a novel and potent transient receptor potential vanilloid 4 channel agonist induces urinary bladder contraction and hyperactivity: Part I. The Journal of pharmacology and experimental therapeutics (2008) Aug:326, 432-442.
Venkatachalam, K., and Montell, C. (2007). TRP channels. Annual review of biochemistry 76, 387-417.
Zheng, W., Spencer, R.H., and Kiss, L. (2004). High throughput assay technologies for ion channel drug discovery. Assay and drug development technologies 2, 543-552.
Kim J, Chung YD, Park DY, Choi S, Shin DW, Soh H, Lee HW, Son W, Yim J, Park CS, Kernan MJ, Kim C. A TRPV family ion channel required for hearing in Drosophila. Nature. 2003 Jul 3;424(6944):81-4. Epub 2003 Jun 18. PubMed PMID: 12819662.
Gong Z, Son W, Chung YD, Kim J, Shin DW, McClung CA, Lee Y, Lee HW, Chang DJ, Kaang BK, Cho H, Oh U, Hirsh J, Kernan MJ, Kim C. Two interdependent TRPV channel subunits, inactive and Nanchung, mediate hearing in Drosophila. J Neurosci. 2004 Oct 13;24(41):9059-66. PubMed PMID: 15483124.
Chang GC, Snyder WE. Pymetrozine causes a nontarget pest, the Colorado potato beetle (Coleoptera: Chrysomelidae), to leave potato plants. J Econ Entomol. 2008 Feb;101(1):74-80. PubMed PMID: 18330119.
Douglas JT. Adenovirus-mediated gene delivery: an overview. Methods Mol Biol. 2004; V246:3-14. Review. PubMed PMID: 14970581.

## Claims

1. A method for determining whether a compound binds directly to the same site as afidopyropen on one or more Nanchung proteins, comprising:
(a) providing one or more Nanchung proteins;
(b) contacting the one or more Nanchung proteins with, afidopyropen,
(c) contacting the one or more Nanchung proteins with one or more candidate compounds; and
(d) determining whether the one or more candidate compounds bind to the same site as afidopyropen and determining the binding affinity of the one or more candidate compounds to the one or more Nanchung proteins.

## Patentansprüche

1. Verfahren zur Bestimmung, ob eine Verbindung direkt an die gleiche Stelle wie Afidopyropen auf einem oder mehreren Nanchung-Proteinen bindet, umfassend:
(a) Bereitstellen eines oder mehrerer Nanchung-Proteine;
(b) In-Kontakt-Bringen des einen bzw. der mehreren Nanchung-Proteine mit Afidopyropen,
(c) In-Kontakt-Bringen des einen bzw. der mehreren Nanchung-Proteine mit einer oder mehreren Kandidatenverbindungen; und
(d) Bestimmen, ob die eine oder mehreren Kandidatenverbindungen an die gleiche Stelle wie Afidopyropen binden, und Bestimmen der Bindungsaffinität der einen oder mehreren Kandidatenverbindungen für das eine bzw. die mehreren Nanchung-Proteine.

## Revendications

1. Procédé pour la détermination si un composé se lie directement au même site que l'afidopyropène sur une ou plusieurs protéines Nanchung, comprenant :
(a) la mise à disposition d'une ou plusieurs protéines Nanchung ;
(b) la mise en contact de la ou des protéines Nanchung avec de l'afidopyropène,
(c) la mise en contact de la ou des protéines Nanchung avec un ou plusieurs composés candidats ; et
(d) la détermination si le ou les composés candidats se lie(nt) au même site que l'afidopyropène et la détermination de l'affinité de liaison du ou des composés candidats à la ou aux protéines Nanchung.
